(19) Europäisches Patentamt / European Patent Office / Office européen des brevets

(11) EP 4 737 459 A1

(12) **EUROPEAN PATENT APPLICATION**
published in accordance with Art. 153(4) EPC

(43) Date of publication:
**06.05.2026 Bulletin 2026/19**

(21) Application number: **24830986.6**

(22) Date of filing: **28.06.2024**

(51) International Patent Classification (IPC):
*C07D 491/22* (2006.01) *A61K 31/35* (2006.01) *A61K 31/519* (2006.01) *A61P 25/04* (2006.01) *A61P 29/00* (2006.01)

(52) Cooperative Patent Classification (CPC):
**A61K 31/35; A61K 31/519; A61P 25/04; A61P 29/00; C07D 491/22**

(86) International application number:
**PCT/CN2024/102404**

(87) International publication number:
**WO 2025/002357 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.06.2023 CN 202310787067**

(71) Applicant: **Vastpro (Shanghai) Pharmaceutical Technology Development Co., Ltd.**
**Shanghai 201210 (CN)**

(72) Inventors:
- **LEI, Lijun**
  **Shanghai 201210 (CN)**
- **YAN, Xuhui**
  **Shanghai 201210 (CN)**
- **WANG, Chengxi**
  **Shanghai 201210 (CN)**
- **GUO, Zhaoxiang**
  **Shanghai 201210 (CN)**
- **HUANG, Chengyang**
  **Shanghai 201210 (CN)**
- **LIU, Pi**
  **Shanghai 201210 (CN)**
- **ZHONG, Yun**
  **Shanghai 201210 (CN)**
- **JIANG, Biao**
  **Shanghai 201210 (CN)**
- **ZHU, Wenfeng**
  **Shanghai 201210 (CN)**

(74) Representative: **Müller, Christian Stefan Gerd**
**ZSP Patentanwälte PartG mbB**
**Hansastraße 32**
**80686 München (DE)**

# (54) TETRODOTOXIN DERIVATIVE COMPOUND AND USE THEREOF AS SODIUM CHANNEL BLOCKER

(57) The present invention relates to a compound having an inhibitory effect on a plurality of sodium ion channels or a pharmaceutically acceptable derivative thereof, a pharmaceutical composition thereof and use thereof as a pan sodium ion channel blocker.

EP 4 737 459 A1

## Description

### Technical Field

Provided is a compound having an inhibitory effect on a plurality of sodium ion channels or a pharmaceutically acceptable derivative thereof, a pharmaceutical composition thereof and use thereof as a pan sodium ion channel blocker.

### Background

Tetrodotoxin (TTX) is an amino perhydroquinazolin type compound having the following structure:

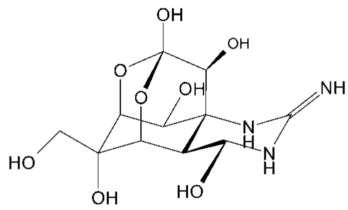

.

Tetrodotoxin has a local stimulation effect on the intestinal tract and rapidly acts on nerve terminals and nerve centers after absorption, which can block sodium ion channels on nerve excitation membranes with high selectivity and high affinity, and hinder nerve conduction, thereby causing nerve paralysis and death. Clinically, tetrodotoxin is mainly used for analgesia, local anesthesia, sedation, antispasmodic, hypotension, anti-arrhythmia, etc., but is greatly limited in application due to its strong toxicity.

Voltage-gated sodium channels (VGSC) are microporous transmembrane glycoproteins widely distributed on excitable cell membranes such as neurons, are mainly responsible for transmembrane transport of $Na^+$, and are the most important ion channels required for neurons to produce excitability and exert normal electrophysiological functions. The voltage-gated sodium channel consists of an $\alpha$ subunit and multiple $\beta$ subunits. $\alpha$ subunit is the main functional unit, and 9 subtypes (Nav1.1 to Nav1.9) have been found at present, which are surrounded by 4 highly similar homologous domains to form a central pore of an ion channel, and each domain has 6 $\alpha$ helical transmembrane fragments (S1-S6), wherein the amino acid sequence of S4 is highly conserved and is considered as a voltage receptor of a voltage-gated sodium channel. There are four subtypes of $\beta$ subunits ($\beta$1-$\beta$4), which play an auxiliary role in localization and stability of $\alpha$ subunit on the membrane, and participate in regulating the voltage sensitivity and inactivation process of $\alpha$ subunit. Voltage-gated sodium channels of different animals have wide homology, but they also have great differences. The sodium ion channels are divided into TTX sensitive (TTX-S) and TTX insensitive (TTX-R) according to the sensitivity blocked by tetrodotoxin (TTX). TTX-R-type sodium ion channels include Nav1.5, Nav1.8, and Nav1.9, and the rest are TTX-S-type sodium ion channels. Nav1.7 is a transmembrane protein encoded by SCN9A, specifically expressed on peripheral sensory nerve terminals and sympathetic ganglion neurons, and mainly expressed on large-diameter dorsal root ganglion (DRG) neurons and unmyelinated small-diameter DRG, that is, expressed on 85% of nociceptors, indicating that Nav1.7 has a very critical effect on pain conduction. Nav1.7 plays a central role in pain signaling and maintenance and has become a very important target for the development of an analgesic drug. The compound according to the present disclosure is structurally modified and optimized over tetrodotoxin, and the obtained tetrodotoxin derivative innovative molecules have significant inhibitory activity against TTX-S type sodium ion channels (including Nav1.1, Nav1.2, Nav1.3, Nav1.4, Nav1.6 and Nav1.7) and is a pan sodium ion channel blocker.

In the prior art, derivatives based on tetrodotoxin are mainly derived from separation and extraction from nature. For example, 4,9-anhydro TTX (Nakamura M, Yasumoto T. Tetrodotoxin years in puffer fish [J]. Toxicon, 1985, 232): 271-276.), 6-epi TTX, 11-deoxy TTX (Yasumoto T, Yotsu M, Murata M, et al. New tetradotoxin analogs from the nenops ensauda [J] .j.am.chem.soc, 1988, 110(7): 2344-2345.) and the like. Laboratory synthesis of some derivatives of tetrodotoxin are also reported. For example, 11-Deoxytetrodotoxin (J. AM. CHEM. SOC. 2002, 124, 7847-7852), 8,11-Dioxytetrodotoxin (Chem. Eur. J. 2004, 10, 452-462), and the like.

### Summary

In the first aspect, provided is a compound having a structure of formula (I) or a pharmaceutically acceptable derivative thereof:

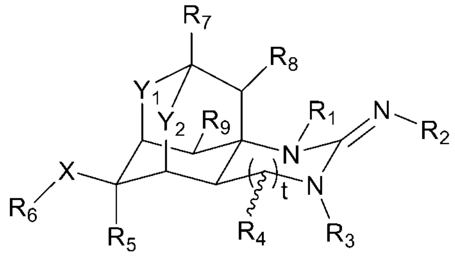

Formula (I)

wherein,

X is absent or selected from the group consisting of $-(CH_2)_m-$ and

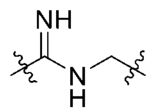

,

wherein $-CH_2-$ is optionally replaced by -O- or carbonyl or optionally substituted with at least one $R^X$;

$Y_1$ and $Y_2$ are each independently selected from the group consisting of methylene, O, S and NH, wherein methylene and NH are optionally substituted with at least one $R^X$;

$R_1$ and $R_3$ are each independently selected from the group consisting of hydrogen, hydroxyl, amino, cyano, $C_{1-8}$ alkyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl and $C_{1-8}$ alkyl-$C_{5-10}$ heteroaryl, wherein the alkyl, cycloalkyl, heterocyclyl, aryl and heteroaryl are optionally substituted with at least one $R^X$;

$R_2$ is selected from the group consisting of hydrogen, hydroxyl, amino, formyl, acetyl, $C_{1-8}$ alkyl, $C_{3-8}$ cycloalkyl, $C_{3-8}$ heterocyclyl, $-(C{=}O)OR_{10}$ and $-O(C{=}O)R_{11}$, wherein the formyl, acetyl, alkyl, cycloalkyl and heterocyclyl are optionally substituted with at least one $R^X$;

each $R_4$ is independently selected from the group consisting of hydrogen, deuterium, hydroxyl, halogen, $-OR_{12}$ and $C_{1-8}$ alkyl;

or $R_3$ and $R_4$ together with the atom(s) to which they are attached form a 5-12 membered heterocycle, wherein the heterocycle is optionally substituted with at least one $R^X$;

$R_5$ is selected from the group consisting of hydrogen, deuterium, tritium, hydroxyl, halogen, $C_{1-8}$ alkyl, $C_{3-8}$ cycloalkyl and $-OR_{13}$;

$R_6$ is selected from the group consisting of hydroxyl, mercapto, amino, azido, halogen, cyano, $C_{1-12}$ alkyl, $C_{2-8}$ alkenyl, $-O-C_{1-12}$ alkyl, $C_{3-8}$ cycloalkyl, $C_{3-8}$ heterocyclyl, $C_{6-10}$ aryl, $C_{5-10}$ heteroaryl, $-(C{=}O)-R_{14}$, guanidino, ureido, $-(OCH_2CH_2)_n-OH$,

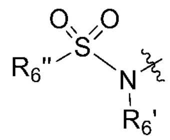 , 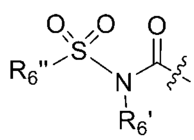 ,

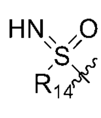 and 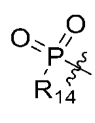 ,

wherein the alkyl, amino, cycloalkyl, heterocyclyl, aryl, heteroaryl, guanidino and ureido are optionally substituted with at least one $R^X$;

or $R_5$ and $R_6$ together with the atom(s) to which they are attached form a 3-12 membered heterocycle, wherein the heterocycle is optionally substituted with at least one $R^X$;

$R_7$ is selected from the group consisting of hydrogen, deuterium, tritium, hydroxyl, halogen, $C_{1-4}$ alkyl, $-O-C_{1-4}$ alkyl, $C_{3-8}$ cycloalkyl and $C_{3-8}$ heterocyclyl, wherein the alkyl, cycloalkyl and heterocyclyl are optionally substituted with at least one $R^X$;

$R_8$ is selected from the group consisting of hydrogen, deuterium, tritium, hydroxyl, halogen, $C_{1-8}$ alkyl, $C_{3-8}$ cycloalkyl and $-OR_{15}$;

or $R_4$ and $R_8$ together with the atom(s) to which they are attached form a 5-12 membered heterocycle, wherein the heterocycle is optionally substituted with at least one $R^X$;

$R_9$ is selected from the group consisting of hydrogen, deuterium, tritium, hydroxyl, $C_{1-8}$ alkyl, $C_{3-8}$ cycloalkyl and $-OR_{16}$;

$R_{10}$ and $R_{11}$ are each independently selected from the group consisting of $C_{1-8}$ alkyl, $C_{3-8}$ cycloalkyl, $C_{3-8}$ heterocyclyl, $C_{6-20}$ aryl, $C_{1-8}$ alkyl-$C_{6-20}$ aryl, $C_{5-20}$ heteroaryl and $C_{1-8}$ alkyl-$C_{5-20}$ heteroaryl, wherein the alkyl, cycloalkyl, heterocyclyl, aryl and heteroaryl are optionally substituted with at least one $R^X$;

each of $R_{12}$, $R_{13}$, $R_{14}$, $R_{15}$ and $R_{16}$ is independently selected from the group consisting of $C_{1-8}$ alkyl, $C_{2-8}$ alkenyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl, and $C_{1-8}$ alkyl-$C_{5-10}$ heteroaryl, wherein the alkyl, cycloalkyl, heterocyclyl, aryl, and heteroaryl are optionally substituted with at least one $R^X$;

$R_{6'}$ is selected from the group consisting of hydrogen, hydroxyl, amino, cyano, $C_{1-8}$ alkyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl and $C_{1-8}$ alkyl-$C_{5-10}$ heteroaryl, wherein the alkyl, cycloalkyl, heterocyclyl, aryl and heteroaryl are optionally substituted with at least one $R^X$;

$R_{6''}$ is selected from the group consisting of hydroxyl, mercapto, amino, halogen, $C_{1-12}$ alkyl, $C_{2-8}$ alkenyl, $-O-C_{1-12}$ alkyl, $C_{3-8}$ cycloalkyl, $C_{3-8}$ heterocyclyl, $C_{6-10}$ aryl and $C_{5-10}$ heteroaryl, wherein the alkyl, amino, cycloalkyl, heterocyclyl, aryl and heteroaryl are optionally substituted with at least one $R^X$;

each $R^X$ is independently selected from the group consisting of hydrogen, halogen, hydroxyl, carboxyl, amino, cyano, formyl, $C_{1-8}$ alkyl, $-O-C_{1-8}$ alkyl, $C_{2-8}$ alkenyl, $C_{3-20}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl, $C_{1-8}$ alkyl-$C_{5-10}$ heteroaryl,

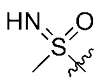, 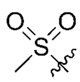 and 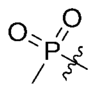,

wherein the amino, formyl, alkyl, cycloalkyl, heterocyclyl, aryl and heteroaryl are optionally substituted with at least one $R^Y$;

each $R^Y$ is independently selected from the group consisting of hydrogen, halogen, hydroxyl, amino, carboxyl, formyl, acetyl, $C_{1-8}$ alkyl, $-O-C_{1-8}$ alkyl, $C_{3-8}$ cycloalkyl, $-O-C_{3-8}$ cycloalkyl, $C_{3-8}$ heterocyclyl, $-O-C_{3-8}$ heterocyclyl, $C_{1-8}$ alkyl-$C_{3-8}$ heterocyclyl, $C_{6-10}$ aryl, $-O-C_{6-10}$ aryl, $C_{1-8}$ alkyl-$C_{6-10}$ aryl, $C_{5-10}$ heteroaryl, $-O-C_{5-10}$ heteroaryl and $C_{1-8}$ alkyl-$C_{5-10}$ heteroaryl, wherein the formyl, acetyl, alkyl, cycloalkyl, heterocyclyl, aryl, and heteroaryl are optionally substituted with at least one halogen, hydroxyl, or $R^{Y1}$;

each $R^{Y1}$ is independently selected from the group consisting of amino, $C_{1-8}$ alkyl, $C_{3-8}$ cycloalkyl, $C_{3-8}$ heterocyclyl, $C_{6-10}$ aryl, and $C_{5-10}$ heteroaryl, wherein the alkyl, cycloalkyl, heterocyclyl, aryl, and heteroaryl are optionally substituted with at least one $R^{Y2}$;

each $R^{Y2}$ is independently selected from the group consisting of amino, $C_{1-8}$ alkyl, $C_{3-8}$ cycloalkyl, $C_{3-8}$ heterocyclyl, $C_{6-10}$ aryl, and $C_{5-10}$ heteroaryl;

when X is methylene and $R_1$, $R_2$ and $R_3$ are hydrogen at the same time, $R_4$, $R_5$, $R_6$, $R_7$, $R_8$ and $R_9$ are not hydroxyl at the same time;

m is an integer selected from 0-6;

n is an integer selected from 1-12;

t is an integer selected from 0-4.

In the second aspect, provided is a pharmaceutical composition comprising a therapeutically effective amount of a compound of formula (I) or a pharmaceutically acceptable derivative thereof and one or more pharmaceutically acceptable carriers.

In the third aspect, provided is a method for treating a disease or condition associated with a sodium ion channel, comprising administering to a subject in need thereof a therapeutically effective amount of a compound of formula (I) or a pharmaceutically acceptable derivative thereof or a pharmaceutical composition thereof, optionally in combination with another therapeutical agent. Accordingly, provided also is use of the compound of formula (I) or a pharmaceutically acceptable derivative thereof or a pharmaceutical composition thereof in the manufacture of a medicament for treating a disease or condition associated with a sodium ion channel, optionally in combination with another therapeutical agent. Further provided is a compound of formula (I), or a pharmaceutically acceptable derivative thereof, or a pharmaceutical composition thereof for treating a disease or condition associated with a sodium ion channel, optionally in combination with another therapeutic agent. In an embodiment, the sodium ion channel is a TTX- S sodium ion channel. In an embodiment, the disease or condition associated with a sodium ion channel comprises pain. In an embodiment, the pain comprises neuropathic pain, inflammatory pain, visceral pain, cancer pain, chemotherapeutic pain, traumatic pain, surgical pain,

post-surgical pain, childbirth pain, labor pain, chronic pain, persistent pain, peripherally mediated pain, centrally mediated pain, chronic headache, migraine, sinus headache, tension headache, phantom limb pain, dental pain, pain associated with HIV, acute pain, pain associated with multiple sclerosis (MS), familial rectal pain, fibromyalgia, or comprises pain caused by depression, cardiovascular disease, neurogenic cystitis, ulcerative colitis, respiratory disease, psychiatric disease, peripheral nerve damage, HIV treatment-induced neuropathy, thermosensitivity, sarcoidosis, irritable bowel syndrome, Crohn's disease, amyotrophic lateral sclerosis (ALS), diabetic neuropathy, peripheral neuropathy, arthritis, rheumatoid arthritis, osteoarthritis, atherosclerosis, paroxysmal dystonia, myasthenic syndrome, myotonic, malignant hyperthermia, cystic fibrosis, pseudohyperaldosteronism, rhabdomyolysis, hypothyroidism, bipolar depression, anxiety, schizophrenia, sodium channel toxin related disease, familial erythromelalgia, primary erythromelalgia, epilepsy, epileptic encephalopathy, focal and generalized tonic seizure, restless leg syndrome, arrhythmia, tachyarrhythmia, atrial fibrillation, or ventricular fibrillation.

Therefore, provided also is a method for analgesia, comprising administering to a subject in need thereof a therapeutically effective amount of a compound of formula (I) or a pharmaceutically acceptable derivative thereof or a pharmaceutical composition thereof, optionally in combination with another therapeutic agent. Accordingly, provided is use of the compound of formula (I) or a pharmaceutically acceptable derivative thereof or a pharmaceutical composition thereof in the manufacture of a medicament for analgesia, optionally in combination with another therapeutic agent. Provided further is a compound of formula (I) or a pharmaceutically acceptable derivative thereof or a pharmaceutical composition thereof for use in analgesia, optionally in combination with another therapeutic agent. In an embodiment, the pain is as described above.

In the fourth aspect, provided is a compound of formula (II) having the following structure:

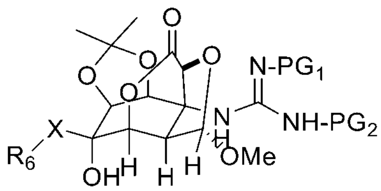

Formula (II)

wherein X and $R_6$ are as defined herein for the compound of formula (I), and $Pg_1$, $Pg_2$ is each independently selected from the group consisting of acetyl, trityl, tert-butoxycarbonyl (Boc), benzyloxycarbonyl (Cbz), cyclopentyloxycarbonyl and fluorenylmethoxycarbonyl (Fmoc).

## Brief Description of Drawings

FIG. 1 shows the inhibition results of analgesic efficacy test of the acetic acid writhing pain model in ICR mice. FIG. 1A shows the results of Compound TTX-S in the analgesic efficacy test of the acetic acid writhing pain model in ICR mice; FIG. 1B shows the results of Compound 73 in the analgesic efficacy test of the acetic acid writhing pain model in ICR mice; FIG. 1C shows the results of Compound 77 in the analgesic efficacy test of the acetic acid writhing pain model in ICR mice; FIG. 1D shows the results of Compound 199 in the analgesic efficacy test of the acetic acid writhing pain model in ICR mice; FIG. 1E shows the results of Compound 199-B in the analgesic efficacy test of the acetic acid writhing pain model in ICR mice; FIG. 1F shows the results of Compound 210 in the analgesic efficacy test of the acetic acid writhing pain model in ICR mice.

FIG. 2 shows the inhibition results of analgesic efficacy test of the rat formalin inflammatory pain model. FIG. 2A shows the results of Compound TTX in the analgesic efficacy test of the rat formalin inflammatory pain model; FIG. 2B shows the results of Compound 77 in the analgesic efficacy test of the rat formalin inflammatory pain model; FIG. 2C shows the results of Compound 199 in the analgesic efficacy test of the rat formalin inflammatory pain model; FIG. 2D shows the results of Compound 199-B in the analgesic efficacy test of the rat formalin inflammatory pain model; FIG. 2E shows the results of Compound 189 in the analgesic efficacy test of the rat formalin inflammatory pain model; FIG. 2F shows the results of Compound 223 in the analgesic efficacy test of the rat formalin inflammatory pain model; FIG. 2G shows the results of Compound 206 in the analgesic efficacy test of the rat formalin inflammatory pain model; FIG. 2H shows the results of Compound 209 in the analgesic efficacy test of the rat formalin inflammatory pain model.

## Detailed Description

Definition

Unless otherwise defined below, all technical and scientific terms used herein have the same meaning as commonly understood by those skilled in the art. References to technologies employed herein are intended to refer to the technologies commonly understood in the art, including variations on those technologies apparent to those skilled in the art, or substitutions of equivalents. While the following terms are believed to be well understood by those skilled in the art, the following definitions are set forth to better explain the present disclosure.

The terms "comprising", "including", "having", "containing", or "relating to", and other variations thereof herein, are inclusive or open-ended and do not exclude other elements or method steps which are not listed, although other elements or method steps which are not listed are not necessarily present (i.e., the terms also encompass the terms "consisting essentially of" and "consisting of").

The term "about" means within ± 10% of the stated numerical value, preferably within ± 5%, more preferably within ± 2%.

As used herein, the term "alkyl" is defined as a linear or branched saturated aliphatic hydrocarbon. In some embodiments, the alkyl has 1 to 12, for example, 1, 2, 3, 4, 5, 6, 7, 8, 9, 10, 11 or 12 carbon atoms, for another example, 1 to 4 carbon atoms. For example, as used herein, the term "$C_{1-4}$ alkyl" refers to a linear or branched aliphatic hydrocarbon chain having 1-4 carbon atoms (e.g., methyl, ethyl, n-propyl, isopropyl, n-butyl, isobutyl, sec-butyl, or tert-butyl) that is optionally substituted with one or more (such as 1 to 3) suitable substituents such as halogen (where the group is referred to as a "haloalkyl") (e.g., $CH_2F$, $CHF_2$, $CF_3$, $CCl_3$, $C_2F_5$, $C_2Cl_5$, $CH_2CF_3$, $CH_2Cl$ or $-CH_2CH_2CF_3$ or the like.). Accordingly, "alkoxy" refers to an alkyl group as described herein which is attached to rest of the molecule through an oxygen atom.

The term "alkenyl" refers to a linear or branched unsaturated aliphatic hydrocarbon group having at least one double bond composed of carbon atoms and hydrogen atoms. In some embodiments, the alkenyl has 2 to 8 carbon atoms. For example, 2, 3, 4, 5, 6, 7 or 8 carbon atoms. Non-limiting examples of alkenyl include, but are not limited to, ethenyl, 1-propenyl, 2-propenyl, 1-butenyl, isobutenyl, 1,3-butadienyl, and the like.

As used herein, the term "cycloalkyl" refers to a saturated or unsaturated non-aromatic monocyclic or fused polycyclic hydrocarbon ring group, including but not limited to monocyclic cycloalkyl (such as cyclopropyl, cyclobutyl, cyclopentyl, cyclohexyl, cycloheptyl, cyclooctyl, cyclononyl, etc.), fused cycloalkyl, bridged ring or spirocyclic ring structure. In the present disclosure, cycloalkyl is optionally substituted with one or more (such as 1 to 3) same or different substituents. The carbon atom on the cycloalkyl group is optionally substituted with an oxo group (i.e., forming C=O). The term "$C_{3-20}$ cycloalkyl" refers to a cycloalkyl group having 3 to 20 ring-forming carbon atoms, for example, a cycloalkyl group having 3, 4, 5, 6, 7, 8, 9, 10, 11, 12, 13, 14, 15, 16, 17, 18, 19 or 20 ring-forming carbon atoms. The term "$C_{3-8}$ cycloalkyl" refers to a cycloalkyl group having 3 to 8 ring-forming carbon atoms, for example, a cycloalkyl group having 3, 4, 5, 6, 7 or 8 ring-forming carbon atoms, such as $C_{3-6}$cycloalkyl, which may be monocyclic cycloalkyl, such as cyclopropyl, cyclobutyl, cyclopentyl, cyclohexyl, cycloheptyl or cyclooctyl, or fused cycloalkyl, such as $C_{5-8}$ fused cycloalkyl or $C_{5-6}$ fused cycloalkyl.

As used herein, the term "heterocyclyl" or "heterocycle" refers to an aliphatic monocyclic, fused polycyclic, bridged ring, or spiro ring group having 2 or more (e.g., 3, 4, 5, 6, 7, 8, 9, 10, 11, 12, 13 or 14) carbon atoms and one or more (e.g., 1, 2, 3, or 4) heteroatoms including, but not limited to, oxygen, nitrogen, and sulfur atoms, the carbon atoms and heteroatoms on the heterocyclyl being optionally substituted with an oxo group (e.g., forming C=O, S(=O), or $S(=O)_2$), the carbon atoms and heteroatoms on the heterocyclyl also being optionally substituted with a nitrogen-containing group (e.g., forming C=NH).

As used herein, the term "$C_{3-8}$ heterocyclyl" refers to a heterocyclyl containing 3-8 ring atoms, such as 3, 4, 5, 6, 7 or 8 ring atoms, including but not limited to 3-8 membered heterocyclyl, 3-7 membered heterocyclyl, 4-8 membered heterocyclyl, 4-7 membered heterocyclyl, 5-6 membered heterocyclyl, 3-6 membered heterocyclyl, 4-7 membered nitrogen-containing heterocyclyl, 4-7 membered oxygen-containing heterocyclyl, 4-7 membered sulfur-containing heterocyclyl, 5-6 membered nitrogen-containing heterocyclyl, 5-6 membered oxygen-containing heterocyclyl, 5-6 membered sulfur-containing heterocyclyl, and the like, each optionally further containing one or more other heteroatoms independently selected from oxygen, nitrogen, and sulfur. Examples of 3-8 membered heterocyclyl include, but are not limited to, oxiranyl, aziridinyl, azetidinyl, oxetanyl, tetrahydrofuranyl, pyrrolidinyl, pyrrolidinyl (e.g.,

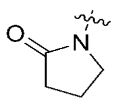

), imidazolidinyl, pyrazolidinyl, tetrahydropyranyl, piperidinyl, morpholinyl, dithianyl, thiomorpholinyl, piperazinyl, trithianyl,

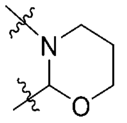, 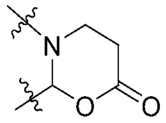,

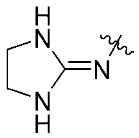, 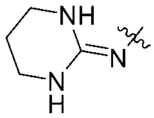, 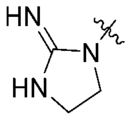, 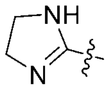, 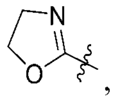, 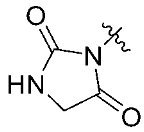, 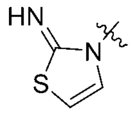, 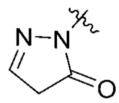,

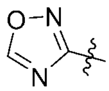, 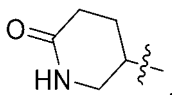, 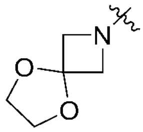, and 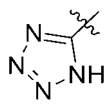.

In the present disclosure, the heterocyclyl may form a fused ring structure with the heterocyclyl or the cycloalkyl group, and the connecting point between the fused ring structure and other groups can be on any heterocyclyl or cycloalkyl group. Therefore, the heterocyclyl of the present disclosure also includes (but is not limited to) heterocyclyl-fused heterocyclic, heterocyclyl-fused cycloalkyl, monoheterocyclyl-fused monoheterocyclic, monoheterocyclyl-fused monocyclic cycloalkyl, such as 3-7 membered (mono) heterocyclic-fused 3-7 membered (mono) heterocyclic, 3-7 membered (mono) heterocyclic-fused (mono) cycloalkyl, 3-7 membered (mono) heterocyclic-fused $C_{4-6}$ (mono) cycloalkyl, examples of which include but are not limited to pyrrolidinyl-fused cyclopropyl, cyclopentyl-fused azacyclopropyl, pyrrolidinyl-fused cyclobutyl, pyrrolidinyl-fused pyrrolidinyl, pyrrolidinyl-fused piperidinyl, pyrrolidinyl-fused piperazinyl, piperidinyl-fused morpholinyl,

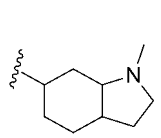, 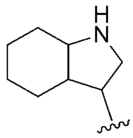,

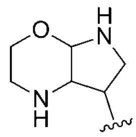 or 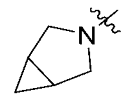.

The term "aryl" may contain 6-20 carbon atoms ($C_{6-20}$ aryl), for example, 6, 7, 8, 9, 10, 11, 12, 13, 14, 15, 16, 17, 18, 19 or 20 carbon atoms, for another example, 6-14 carbon atoms ($C_{6-14}$aryl) or 6-10 carbon atoms ($C_{6-10}$ aryl), for example, phenyl, naphthyl, indanyl, fluorenyl, or the like. "Aryl" includes 6-membered aromatic carbocyclic ring, such as phenyl; bicyclic ring having at least one aromatic carbocyclic ring, such as naphthyl, indane, and 1,2,3,4-tetrahydroquinoline; and tricyclic ring having at least one aromatic carbocyclic ring, such as fluorene. If the aryl substituent is bicyclic or tricyclic and at least one of the rings is non-aromatic, it is considered to be joined by an aromatic ring. For example, aryl includes 6-membered aromatic carbocyclic ring fused to 5-7 membered heterocycle containing one or more heteroatoms selected from N, O and S, provided that the connecting site is aromatic carbocyclic ring.

The term "heteroaryl" contains 5- to 10-membered aromatic monocyclic ring, such as 5, 6, 7, 8, 9 or 10-membered aromatic monocyclic ring, the ring contains 1 to 4 (e.g., 1, 2, 3 or 4) heteroatoms selected from N, O and S, and in some embodiments, 1 to 3 heteroatoms, the rest of which are carbon atoms; 8- to 12-membered bicyclic ring, such as 8, 9, 10, 11 or 12-membered bicyclic ring, the ring contains 1 to 4 (e.g., 1, 2, 3 or 4) heteroatoms selected from N, O and S, the rest of which are carbon atoms, and at least one heteroatom is present in the aromatic ring; and 11- to 14-membered tricyclic ring, such as 11, 12, 13 or 14-membered tricyclic ring, the ring contains 1 to 4 (e.g., 1, 2, 3 or 4) heteroatoms selected from N, O and S, the rest of which are carbon atoms, and at least one heteroatom is present in the aromatic ring. The term "$C_{5-20}$ heteroaryl" refers to heteroaryl containing 5-20 ring atoms, such as 5, 6, 7, 8, 9, 10, 11, 12, 13, 14, 15, 16, 17, 18, 19 or 20 ring atoms. Non-limiting examples of heteroaryl include, but are not limited to, pyrrolyl, furyl, thienyl, imidazolyl, oxazolyl, pyrazolyl, pyridyl, pyrimidinyl, pyrazinyl, quinolinyl, isoquinolinyl, tetrazolyl (e.g.,

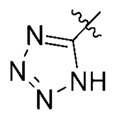

), triazolyl, triazinyl, benzofuranyl, benzothienyl, indolyl, isoindolyl, and the like.

As used herein, the term "halo" or "halogen" includes F, Cl, Br, or I.

The term "substituted" means that one or more (e.g., one, two, three, or four) hydrogens on the designated atom are replaced with a selection from the indicated group, provided that the normal valence of the designated atom in that case is not exceeded and the substitution forms a stable compound, e.g., two hydrogens on the designated carbon atom are replaced with the indicated group by a double bond. Combination of substituents and/or variables are permissible only when such a combination forms a stable compound.

If a substituent is described as "optionally substituted with at least one" or "optionally substituted with one or more", the substituent may be (1) unsubstituted or (2) substituted. If a carbon of a substituent is described as being optionally substituted with one or more of a list of substituents, one or more hydrogens on the carbon (to the extent of any hydrogens present) may be replaced, individually and/or together, by an independently selected optional substituent. If a nitrogen of a substituent is described as being optionally substituted with one or more of a list of substituents, one or more hydrogens on the nitrogen (to the extent of any hydrogens present) may each be replaced by an independently selected optional substituent.

If a substituent is described as being "independently selected from the group consisting of" a group, each substituent is selected independently of the other. Thus, each substituent can be the same as or different from another (other) substituent. The groups described herein may be optionally substituted with one or more substituents. Useful substituents include, but are not limited to, deuterium, tritium, hydroxyl, amino, cyano, mercapto, azido, guanido, ureido, halogen, alkyl, cycloalkyl, heterocyclyl, alkoxy, alkenyl, aryl, heteroaryl, heterocyclyl, and the like, as described herein.

As used herein, the term "at least one" or similar expression "one or more" refers to one or more than one under reasonable conditions, such as 2, 3, 4, 5, 6, 7, 8, 9, or 10.

Unless otherwise indicated, as used herein, the connecting point of a substituent can be from any suitable position of the substituent.

The present disclosure also includes all pharmaceutically acceptable isotopically labeled compounds that are the same as the compounds of the present disclosure, except that one or more atoms are replaced by atoms having the same atomic number but an atomic mass or mass number different from the atomic mass or mass number prevailing in nature. Examples of isotopes suitable for inclusion in the compounds of the present disclosure include, but are not limited to, isotopes of hydrogen (e.g., deuterium ( $^{2}H$), tritium $^{3}H$); isotopes of carbon (e.g., $^{11}C$, $^{13}C$, and $^{14}C$); isotopes of chlorine (e.g., $^{36}Cl$); isotopes of fluorine (e.g., $^{18}F$); isotopes of iodine (e.g., $^{123}I$ and $^{125}I$); isotopes of nitrogen (e.g., $^{13}N$ and $^{15}N$); isotopes of oxygen (e.g., $^{15}O$, $^{17}O$, and $^{18}O$); isotopes of phosphorus (e.g., $^{32}P$); and isotopes of sulfur (e.g., $^{35}S$). Certain isotopically labeled compounds of the present disclosure, such as those incorporated with radioisotopes, may be used in drug and/or substrate tissue distribution studies (e.g., assays). Radioisotope tritium (i.e., $^{3}H$) and carbon-14 (i.e., $^{14}C$) are particularly useful for this purpose due to the ease of incorporation and detection. Substitution with positron emission isotopes such as $^{11}C$, $^{18}F$, $^{15}O$, and $^{13}N$ can be used in positron emission tomography (PET) studies to examine substrate receptor occupancy. Isotopically labeled compounds of the present disclosure can be prepared by methods similar to those described in the provided routes and/or examples and preparations by using appropriate isotopically labeled reagents in place of the previously employed non-labeled reagents. Pharmaceutically acceptable solvates of the present disclosure include those in which the crystallization solvent may be isotopically substituted, for example, $D_2O$, acetone-$d_6$*or* DMSO-$d_6$.

The term "stereoisomer" refers to an isomer formed due to at least one asymmetric center. In compounds having one or more (e.g., one, two, three, or four) asymmetric centers, it is possible a racemic mixture, a single enantiomer, a diastereomeric mixture, and an individual diastereomer may be produced. Specific individual molecules may also be present as geometric isomers (cis/trans). Similarly, the compound of the present disclosure may be present as a mixture of two or more structurally distinct forms in rapid equilibrium, commonly referred to as tautomers. Representative examples of tautomers include keto-enol tautomers, phenol-ketone tautomers, nitroso-oxime tautomers, imine-enamine tautomers, and the like. For example, nitroso-oximes may be present in solution in the following tautomeric forms:

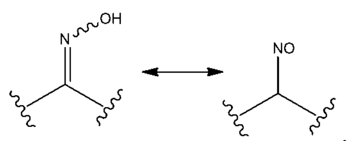

It should be understood that the scope of the present disclosure encompasses all such isomers or mixtures

thereof in any ratio (e.g., 60%, 65%, 70%, 75%, 80%, 85%, 90%, 95%, 96%, 97%, 98%, 99%).

The chemical bonds of the compound of the present disclosure may be depicted herein using solid ( 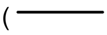 ), solid wedge ( 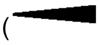 ), or dashed wedge ( 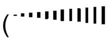 ). The use of a solid line to depict a bond to an asymmetric carbon atom is intended to indicate that all possible stereoisomers (e.g., particular enantiomers, racemic mixtures, etc.) at that carbon atom are included. The use of a real or imaginary wedge to depict a bond to an asymmetric carbon atom is intended to indicate the presence of the stereoisomers as shown. When present in a racemic mixture, real and imaginary wedges are used to define relative stereochemistry rather than absolute stereochemistry. Unless otherwise indicated, it is intended that the compounds of the present disclosure may exist in the form of stereoisomers, including cis and trans isomers, optical isomers (e.g., R and S enantiomers), diastereoisomers, geometric isomers, rotational isomers, conformational isomers, atropisomers, and mixtures thereof. The compounds of the present disclosure may exhibit more than one type of isomerism and consist of mixtures thereof, such as racemic mixtures and diastereomeric pairs.

It should also be understood that certain compounds of the present disclosure may exist in free form for treatment or, where appropriate, in the form of pharmaceutically acceptable derivatives thereof. In the present disclosure, a pharmaceutically acceptable derivative includes, but is not limited to, a pharmaceutically acceptable salt, ester, solvate, N-oxide, metabolite or prodrug, which can directly or indirectly provide the compound of the present disclosure or metabolite or residue thereof after administration to a patient in need thereof. Therefore, when "the compound of the present disclosure" is mentioned herein, it is also intended to encompass various derivative forms of the compound.

A pharmaceutically acceptable salt of the compound of the present disclosure includes an acid addition salt and a base addition salt thereof. For example, hexafluorophosphate, meglumine salt, or the like.

The compound of the present disclosure also encompasses that containing a protecting group. In any process of preparing the compound of the present disclosure, it may be necessary and/or desirable to protect a sensitizing or reactive group on any relevant molecule, thereby forming a chemically protected form of the compound of the present disclosure. This can be accomplished by a conventional protecting group, which can be removed at an appropriate subsequent stage using methods known in the art.

"Protecting group" (Pg) refers to a class of substituents used to block or protect a particular functional group by reacting with other functional groups on the compound. For example, an "amino protecting group" refers to a substituent attached to an amino group to block or protect an amino functional group on a compound. Suitable amino protecting groups include acetyl, trityl, tert-butoxycarbonyl (Boc), benzyloxycarbonyl (Cbz), cyclopentyloxycarbonyl, and fluorenylmethoxycarbonyl (Fmoc). Likewise, a "hydroxyl protecting group" refers to a class of hydroxyl substituents that can effectively block or protect hydroxyl functionality. Suitable protecting groups include acetyl and silyl. A "carboxyl protecting group" refers to a class of carboxyl substituents that can effectively block or protect carboxyl functionality. Commonly used carboxyl protecting groups include $-CH_2CH_2SO_2Ph$, cyanoethyl, 2-(trimethylsilyl)ethyl, 2-(trimethylsilyl)ethoxymethyl, 2-(p-toluenesulfonamide)ethyl, 2-(p-nitrophenylthio)ethyl, 2-(diphenylphosphine)-ethyl, nitroethyl, and the like. For general description and description of protecting groups, see: T. W. Greene, Protective Groups in Organic Synthesis, John Wiley & Sons, New York, 1991.

The term "pharmaceutically acceptable derivative" refers to a compound in the form of a pharmaceutically acceptable salt, hydrate, solvate, prodrug, stereoisomer, enantiomer, tautomer or isotopically labeled compound of the compound or a mixture thereof, or a pharmaceutically acceptable excipient. The "pharmaceutically acceptable salt" includes, but is not limited to, a salt made with pharmaceutically acceptable non-toxic base or acid, including an inorganic or organic base and an inorganic or organic acid. The salt of an inorganic base may be selected from, for example, aluminum, ammonium, calcium, copper, iron, ferrous, lithium, magnesium, manganese, divalent manganese, potassium, sodium, zinc salts. Further, the salt of a pharmaceutically acceptable inorganic base may be selected from ammonium, calcium, magnesium, potassium, and sodium salts. One or more crystal structures may be present in a solid salt, and a hydrate form may also be present. The salt of a pharmaceutically acceptable organic non-toxic base may be selected from, for example, primary, secondary and tertiary amine salt, substituted amine including naturally occurring substituted amine, cyclic amine, basic ion exchange resin such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucosamine, glucosamine, histidine, hydrabamine, isopropylamine, lysine, methylglucamine, morpholine, piperazine, piperidine, polyamine resin, procaine, purine, theobromine, triethylamine, trimethylamine, tripropylamine, tromethamine.

The term "effective amount" refers to a dose of a target compound or a pharmaceutically acceptable derivative thereof capable of causing a biological or medical response in a tissue, system, animal, or human that can be observed by a researcher, veterinarian, clinician, or other clinician.

The term "composition" includes: a product containing a specific amount of a specific ingredient, and any product directly or indirectly formed by combining these specific amounts of specific ingredients. A pharmaceutical composition comprises: a product comprising an active ingredient and an inert ingredient as a carrier, and a product made by combining, complexing or aggregating any two or more ingredients directly or indirectly, or a product made by decomposing one or more ingredients, or a product made by other types of reactions or interactions of one or more ingredients.

The term "pharmaceutically acceptable" refers to being compatible with the other components in the formulation and not unacceptably toxic to the user.

The term "subject" refers to an individual suffering from a disease, condition, or the like, including a mammal and a non-mammal. A mammal includes, but is not limited to, any member of mammals: human, non-human primate such as chimpanzees, and other apes and monkey; a farm animal such as cattle, horse, sheep, goat, pig; livestock such as a rabbit, dog, and cat; an lab animal including rodent such as rat, mouse, guinea pig, and the like. A non-mammalian animal includes, but is not limited to, bird, fish, etc. In an embodiment, the mammal is a human.

The term "treating" includes alleviating, reducing or ameliorating a disease or symptom, preventing other symptoms, ameliorating or preventing underlying metabolic factors of a symptom, inhibiting a disease or symptom, e.g., preventing the development of a disease or symptom, reducing a disease or symptom, promoting the alleviation of a disease or symptom, or arresting signs of a disease or symptom, extending to include prevention. "Treating" also includes achieving a therapeutic benefit and/or a prophylactic benefit. Therapeutic benefit refers to eradication or amelioration of the condition being treated. In addition, the therapeutic benefit is achieved by eradicating or improving one or more physiological signs associated with the underlying disease, although the patient may still have the underlying disease, but improvement in the patient's disease may be observed. The prophylactic benefit refers to a patient that takes a composition to prevent a certain disease risk, or a patient that takes a composition upon one or more physiological conditions of a disease, although the disease has not yet been diagnosed.

The "pharmaceutically acceptable carrier" in the present disclosure refers to a diluent, adjuvant, excipient or vehicle that is administered together with a therapeutic agent, and which, within the scope of reasonable medical judgment, is suitable for contacting the tissues of a human and/or other animal without excessive toxicity, irritation, allergic response or other problems or complications corresponding to a reasonable benefit/risk ratio.

As used herein, the term "therapeutic window" refers to a dose or concentration range of a drug that is effective in treating a disease without an unacceptable toxic effect.

Compounds of the Present disclosure

In some embodiments, provided is a compound having a structure of Formula (I) or a pharmaceutically acceptable derivative thereof:

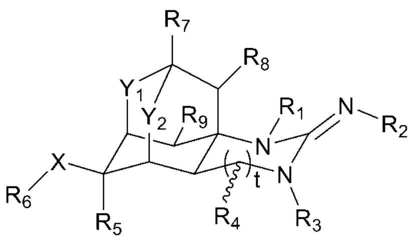

Formula (I)

wherein,

X is absent or selected from the group consisting of $-(CH_2)_m-$ and

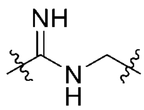

,

wherein $-CH_2-$ is optionally replaced by -O- or carbonyl or optionally substituted with at least one $R^X$;

$Y_1$ and $Y_2$ are each independently selected from the group consisting of methylene, O, S and NH, wherein methylene and NH are optionally substituted with at least one $R^X$;

$R_1$ and $R_3$ are each independently selected from the group consisting of hydrogen, hydroxyl, amino, cyano, $C_{1-8}$ alkyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl and $C_{1-8}$ alkyl-$C_{5-10}$ heteroaryl, wherein the alkyl, cycloalkyl, heterocyclyl, aryl and heteroaryl are optionally substituted with at least one $R^X$;

$R_2$ is selected from the group consisting of hydrogen, hydroxyl, amino, formyl, acetyl, $C_{1-8}$ alkyl, $C_{3-8}$ cycloalkyl, $C_{3-8}$ heterocyclyl, $-(C{=}O)OR_{10}$ and $-O(C{=}O)R_{11}$, wherein the formyl, acetyl, alkyl, cycloalkyl and heterocyclyl are optionally substituted with at least one $R^X$;

each $R_4$ is independently selected from the group consisting of hydrogen, deuterium, hydroxyl, halogen, $-OR_{12}$ and $C_{1-8}$ alkyl;

or $R_3$ and $R_4$ together with the atom(s) to which they are attached form a 5-12 membered heterocycle, wherein the heterocycle is optionally substituted with at least one $R^X$;

$R_5$ is selected from the group consisting of hydrogen, deuterium, tritium, hydroxyl, halogen, $C_{1-8}$ alkyl, $C_{3-8}$ cycloalkyl and $-OR_{13}$;

$R_6$ is selected from the group consisting of hydroxyl, mercapto, amino, azido, halogen, cyano, $C_{1-12}$ alkyl, $C_{2-8}$ alkenyl, $-O-C_{1-12}$ alkyl, $C_{3-8}$ cycloalkyl, $C_{3-8}$ heterocyclyl, $C_{6-10}$ aryl, $C_{5-10}$ heteroaryl, $-(C{=}O)-R_{14}$, guanidino, ureido, $-(OCH_2CH_2)_n-OH$,

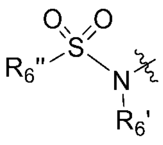 , 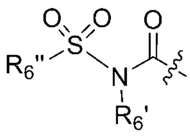 ,

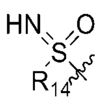 and 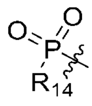 ,

wherein the alkyl, amino, cycloalkyl, heterocyclyl, aryl, heteroaryl, guanidino and ureido are optionally substituted with at least one $R^X$;

or $R_5$ and $R_6$ together with the atom(s) to which they are attached form a 3-12 membered heterocycle, wherein the heterocycle is optionally substituted with at least one $R^X$;

$R_7$ is selected from the group consisting of hydrogen, deuterium, tritium, hydroxyl, halogen, $C_{1-4}$ alkyl, $-O-C_{1-4}$ alkyl, $C_{3-8}$ cycloalkyl and $C_{3-8}$ heterocyclyl, wherein the alkyl, cycloalkyl and heterocyclyl are optionally substituted with at least one $R^X$;

$R_8$ is selected from the group consisting of hydrogen, deuterium, tritium, hydroxyl, halogen, $C_{1-8}$ alkyl, $C_{3-8}$ cycloalkyl and $-OR_{15}$;

or $R_4$ and $R_8$ together with the atom(s) to which they are attached form a 5-12 membered heterocycle, wherein the heterocycle is optionally substituted with at least one $R^X$;

$R_9$ is selected from the group consisting of hydrogen, deuterium, tritium, hydroxyl, $C_{1-8}$ alkyl, $C_{3-8}$ cycloalkyl and $-OR_{16}$;

$R_{10}$ and $R_{11}$ are each independently selected from the group consisting of $C_{1-8}$ alkyl, $C_{3-8}$ cycloalkyl, $C_{3-8}$ heterocyclyl, $C_{6-20}$ aryl, $C_{1-8}$ alkyl-$C_{6-20}$ aryl, $C_{5-20}$ heteroaryl and $C_{1-8}$ alkyl-$C_{5-20}$ heteroaryl, wherein the alkyl, cycloalkyl, heterocyclyl, aryl and heteroaryl are optionally substituted with at least one $R^X$;

each of $R_{12}$, $R_{13}$, $R_{14}$, $R_{15}$ and $R_{16}$ is independently selected from the group consisting of $C_{1-8}$ alkyl, $C_{2-8}$ alkenyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl, and $C_{1-8}$ alkyl-$C_{5-10}$ heteroaryl, wherein the alkyl, cycloalkyl, heterocyclyl, aryl, and heteroaryl are optionally substituted with at least one $R^X$;

$R_{6'}$ is selected from the group consisting of hydrogen, hydroxyl, amino, cyano, $C_{1-8}$ alkyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl and $C_{1-8}$ alkyl-$C_{5-10}$ heteroaryl, wherein the alkyl, cycloalkyl, heterocyclyl, aryl and heteroaryl are optionally substituted with at least one $R^X$;

$R_{6''}$ is selected from the group consisting of hydroxyl, mercapto, amino, halogen, $C_{1-12}$ alkyl, $C_{2-8}$ alkenyl, $-O-C_{1-12}$ alkyl, $C_{3-8}$ cycloalkyl, $C_{3-8}$ heterocyclyl, $C_{6-10}$ aryl and $C_{5-10}$ heteroaryl, wherein the alkyl, amino, cycloalkyl, heterocyclyl, aryl and heteroaryl are optionally substituted with at least one $R^X$;

each $R^X$ is independently selected from the group consisting of hydrogen, halogen, hydroxyl, carboxyl, amino, cyano, formyl, $C_{1-8}$ alkyl, $-O-C_{1-8}$ alkyl, $C_{2-8}$ alkenyl, $C_{3-20}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl, $C_{1-8}$ alkyl-$C_{5-10}$ heteroaryl,

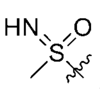 , 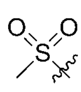 and 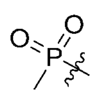 ,

wherein the amino, formyl, alkyl, cycloalkyl, heterocyclyl, aryl and heteroaryl are optionally substituted with at least one $R^Y$;

each $R^Y$ is independently selected from the group consisting of hydrogen, halogen, hydroxyl, amino, carboxyl, formyl, acetyl, $C_{1-8}$ alkyl, -O-$C_{1-8}$ alkyl, $C_{3-8}$ cycloalkyl, -O-$C_{3-8}$ cycloalkyl, $C_{3-8}$ heterocyclyl, -O-$C_{3-8}$ heterocyclyl, $C_{1-8}$ alkyl-$C_{3-8}$ heterocyclyl, $C_{6-10}$ aryl, -O-$C_{6-10}$ aryl, $C_{1-8}$ alkyl-$C_{6-10}$ aryl, $C_{5-10}$ heteroaryl, -O-$C_{5-10}$ heteroaryl and $C_{1-8}$ alkyl-$C_{5-10}$ heteroaryl, wherein the formyl, acetyl, alkyl, cycloalkyl, heterocyclyl, aryl, and heteroaryl are optionally substituted with at least one halogen, hydroxyl, or $R^{Y1}$;

each $R^{Y1}$ is independently selected from the group consisting of amino, $C_{1-8}$ alkyl, $C_{3-8}$ cycloalkyl, $C_{3-8}$ heterocyclyl, $C_{6-10}$ aryl, and $C_{5-10}$ heteroaryl, wherein the alkyl, cycloalkyl, heterocyclyl, aryl, and heteroaryl are optionally substituted with at least one $R^{Y2}$;

each $R^{Y2}$ is independently selected from the group consisting of amino, $C_{1-8}$ alkyl, $C_{3-8}$ cycloalkyl, $C_{3-8}$ heterocyclyl, $C_{6-10}$ aryl, and $C_{5-10}$ heteroaryl;

when X is methylene and $R_1$, $R_2$ and $R_3$ are hydrogen at the same time, $R_4$, $R_5$, $R_6$, $R_7$, $R_8$ and $R_9$ are not hydroxyl at the same time;

m is an integer selected from 0-6;

n is an integer selected from 1-12;

t is an integer selected from 0-4.

In some embodiments, X is absent or selected from the group consisting of methylene, carbonyl, and

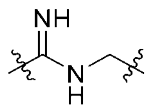.

In a specific embodiment, X is absent. It will be understood by those skilled in the art that the absence of X means that $R_6$ is directly connected to rest of the molecule. In a specific embodiment, X is methylene. In a specific embodiment, X is carbonyl. In an embodiment, X is methylene, which is further substituted with a $R^X$. In an embodiment, X is methylene, which is further substituted with a $R^X$, and $R^X$ is halogen. In a specific embodiment, X is methylene, which is further substituted with a $R^X$, and $R^X$ is fluorine.

In some embodiments, $Y_1$ is methylene. In some embodiments, $Y_1$ is O. In some embodiments, $Y_1$ is S. In some embodiments, $Y_1$ is NH.

In some embodiments, $Y_2$ is methylene. In some embodiments, $Y_2$ is O. In some embodiments, $Y_2$ is S. In some embodiments, $Y_2$ is NH.

In a specific embodiment, $R_1$ is hydrogen. In a specific embodiment, $R_1$ is hydroxyl. In a specific embodiment, $R_1$ is amino. In a specific embodiment, $R_1$ is cyano.

In some embodiments, $R_2$ is selected from the group consisting of hydrogen, hydroxyl, amino, formyl, acetyl, and -(C=O)$OR_{10}$. In some embodiments, $R_2$ is selected from the group consisting of hydrogen, acetyl, and -(C=O)$OR_{10}$. In a specific embodiment, $R_2$ is hydrogen. In a specific embodiment, $R_2$ is hydroxyl. In a specific embodiment, $R_2$ is amino. In a specific embodiment, $R_2$ is formyl. In a specific embodiment, $R_2$ is acetyl. In a specific embodiment, $R_2$ is -(C=O)$OR_{10}$, wherein $R_{10}$ is benzyl. In a specific embodiment, $R_2$ is -(C=O)$OR_{10}$, wherein $R_{10}$ is cyclopentyl.

In some embodiments, $R_3$ is selected from the group consisting of hydrogen, hydroxyl, amino, cyano, $C_{1-8}$ alkyl, $C_{3-8}$ cycloalkyl, $C_{1-8}$ alkyl-$C_{3-8}$ cycloalkyl, and $C_{1-8}$ alkyl-$C_{6-10}$ aryl, wherein the alkyl, cycloalkyl, and aryl are optionally substituted with at least one $R^X$. In some embodiments, $R_3$ is selected from the group consisting of hydrogen, $C_{1-8}$ alkyl, $C_{3-8}$ cycloalkyl, $C_{1-8}$ alkyl-$C_{3-8}$ cycloalkyl, and $C_{1-8}$ alkyl-$C_{6-10}$ aryl, wherein the alkyl, cycloalkyl, and aryl are optionally substituted with at least one $R^X$. In some embodiments, $R_3$ is selected from the group consisting of hydrogen, $C_{1-8}$ alkyl, $C_{3-8}$ cycloalkyl, $C_{1-8}$ alkyl-$C_{3-8}$ cycloalkyl, and $C_{1-8}$ alkyl-$C_{6-10}$ aryl, wherein the alkyl, cycloalkyl, and aryl are optionally substituted with at least one $R^X$, wherein $R^X$ is selected from the group consisting of hydroxyl and carboxyl. In a specific embodiment, $R_3$ is hydrogen. In a specific embodiment, $R_3$ is methyl. In a specific embodiment, $R_3$ is ethyl. In a specific embodiment, $R_3$ is isopropyl. In a specific embodiment, $R_3$ is

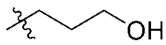.

In a specific embodiment, $R_3$ is

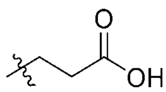.

In a specific embodiment, $R_3$ is

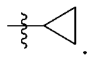

In a specific embodiment, $R_3$ is

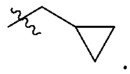

In a specific embodiment, $R_3$ is

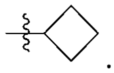

In a specific embodiment, $R_3$ is

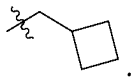

In a specific embodiment, $R_3$ is

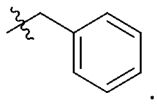

In a specific embodiment, $R_3$ is hydroxyl. In a specific embodiment, $R_3$ is amino. In a specific embodiment, $R_3$ is cyano.

In some embodiments, $R_4$ is selected from the group consisting of hydrogen, deuterium, and hydroxyl. In a specific embodiment, $R_4$ is hydrogen. In a specific embodiment, $R_4$ is deuterium. In a specific embodiment, $R_4$ is hydroxyl.

In some embodiments, $R_3$ and $R_4$ together with the atom(s) to which they are attached form a 6-membered heterocycle. In some embodiments, $R_3$ and $R_4$ together with the atom(s) to which they are attached form a heterocycle selected from the group consisting of

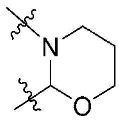

and

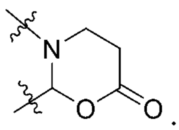

In a particular embodiment, $R_3$ and $R_4$ together with the atom(s) to which they are attached form a heterocycle of

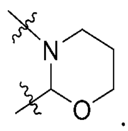

In a particular embodiment, $R_3$ and $R_4$ together with the atom(s) to which they are attached form a heterocycle of

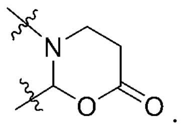

In a specific embodiment, $R_5$ is hydroxyl. In a specific embodiment, $R_5$ is hydrogen. In a specific embodiment, $R_5$ is deuterium.

In an embodiment, $R_5$ is halogen. In a specific embodiment, $R_5$ is fluorine.

In an embodiment, $R_5$ is $-OR_{13}$. In a specific embodiment, $R_5$ is methoxy. In a specific embodiment, $R_5$ is

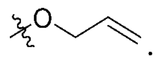

In some embodiments, $R_6$ is selected from the group consisting of hydroxy, mercapto, amino, azido, halogen, cyano, $C_{2-8}$ alkenyl, $-O-C_{1-12}$ alkyl, $C_{3-8}$ heterocyclyl, $C_{5-10}$ heteroaryl, guanidino, ureido, and

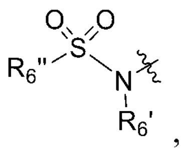

wherein the amino, alkyl, heterocyclyl, heteroaryl, guanidino, and ureido are optionally substituted with at least one $R^X$. In some embodiments, $R_6$ is selected from the group consisting of hydroxy, mercapto, amino, azido, halogen, cyano, $C_{2-8}$ alkenyl, $-O-C_{1-12}$ alkyl, $C_{3-8}$ heterocyclyl, $C_{5-10}$ heteroaryl, guanidino, ureido, and

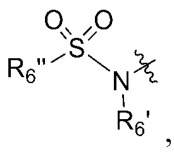

wherein the amino, alkyl, heterocyclyl, heteroaryl, guanidino, and ureido are optionally substituted with at least one $R^X$, wherein $R^X$ is selected from the group consisting of hydrogen, halogen, hydroxy, carboxy, amino, $C_{1-8}$ alkyl, $C_{1-8}$ alkyl-$C_{6-10}$ aryl, $C_{3-20}$ cycloalkyl, $C_{3-8}$ heterocyclyl, and $C_{5-10}$ heteroaryl. In some embodiments, $R^X$ is selected from the group consisting of halogen, hydroxyl, amino, formyl, $C_{1-8}$ alkyl, $C_{3-20}$ cycloalkyl, $C_{1-8}$ alkyl-$C_{6-10}$ aryl, $C_{3-8}$ heterocyclyl, and

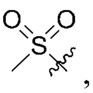

wherein the amino, formyl, alkyl, cycloalkyl, and heterocyclyl are optionally substituted with at least one $R^Y$. In other embodiments, $R^Y$ is selected from the group consisting of halogen, hydroxyl, carboxyl, formyl, acetyl, and $C_{1-8}$ alkyl, wherein the formyl is optionally substituted with hydroxyl or $R^{Y1}$. In yet other embodiments, $R^{Y1}$ is selected from the group consisting of amino, which is optionally substituted with $R^{Y2}$. In some embodiments, $R^{Y2}$ is selected from the group consisting of $C_{1-8}$ alkyl.

In some embodiments, $R_6$ is

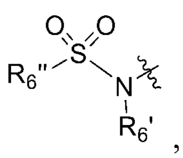

wherein $R_{6'}$ is selected from the group consisting of hydrogen and methyl. In some embodiments, $R_6$ is

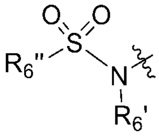 ,

wherein $R_{6''}$ is selected from the group consisting of $C_{1-12}$ alkyl, $C_{3-8}$ cycloalkyl, $C_{6-10}$ aryl, and $C_{5-10}$ heteroaryl, wherein the alkyl, cycloalkyl, aryl, and heteroaryl are optionally substituted with at least one $R^X$. In other embodiments, $R^X$ is selected from the group consisting of halogen, $C_{1-8}$ alkyl, -O-$C_{1-8}$ alkyl, $C_{3-20}$ cycloalkyl, and $C_{6-10}$ aryl, wherein the alkyl, cycloalkyl, and aryl are optionally substituted with at least one $R^Y$. In yet other embodiments, $R^Y$ is halogen, and the halogen is fluorine.

In some embodiments, $R_6$ is selected from the group consisting of hydroxyl, halogen, cyano, amino, azido, methoxy, methylamino, tert-butyl, propenyl, guanidino, ureido,

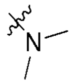 ,

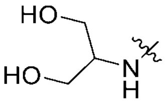 , 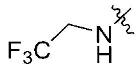 , 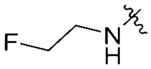 , 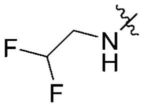 , 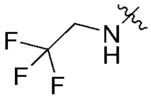 , 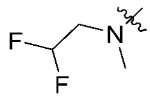 ,

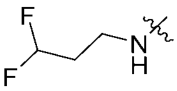 , 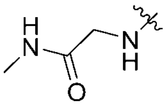 , 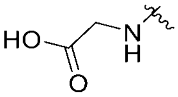 , 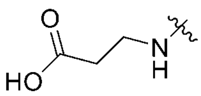 , 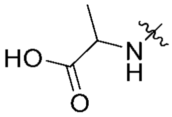 , 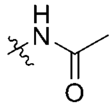 ,

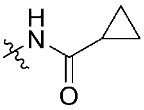 , 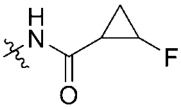 , 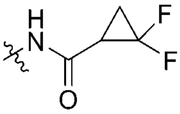 , 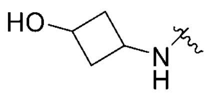 , 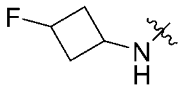 ,

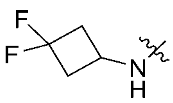 , 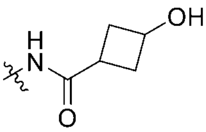 , 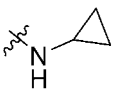 , 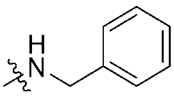 , 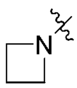 , 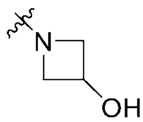 , 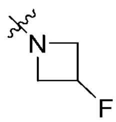 ,

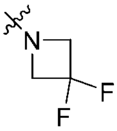 , 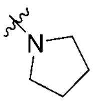 , 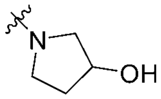 , 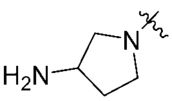 , 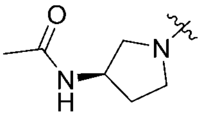 , 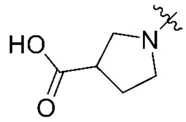 ,

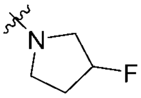 , 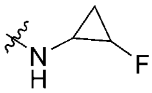 , 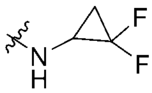 , 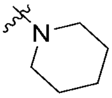 , 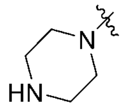 , 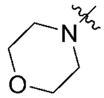 , 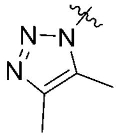 ,

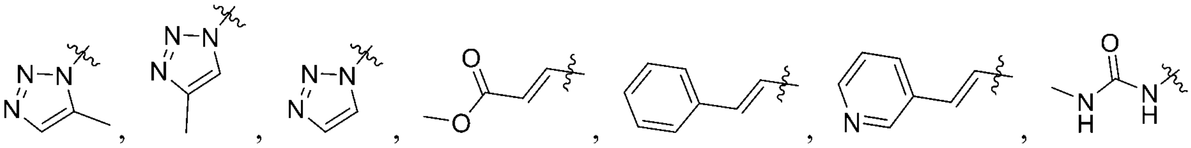 , , , , , , ,

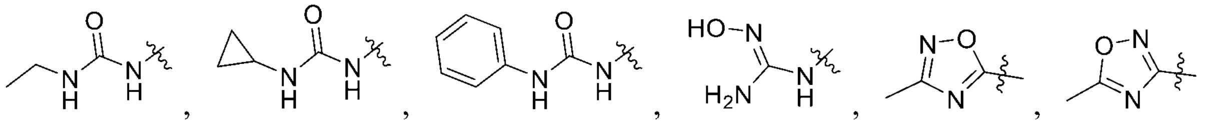
, , , , , ,

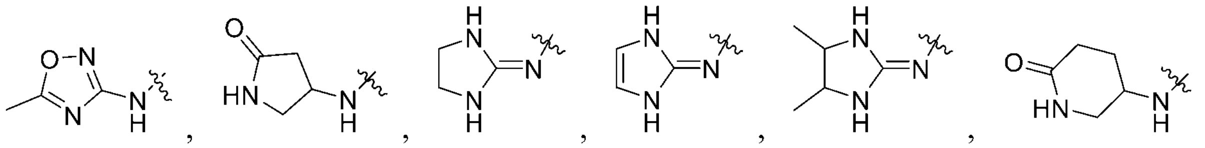
, , , , , ,

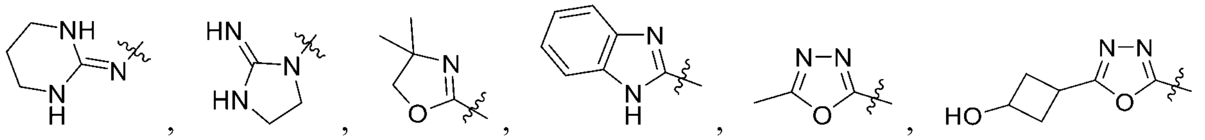
, , , , , ,

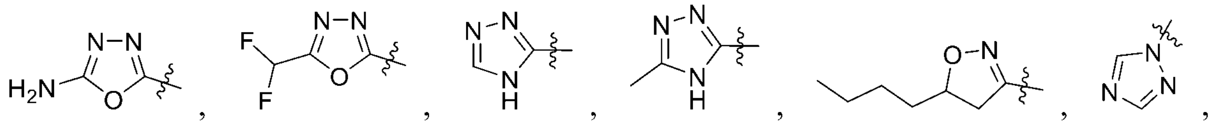
, , , , , ,

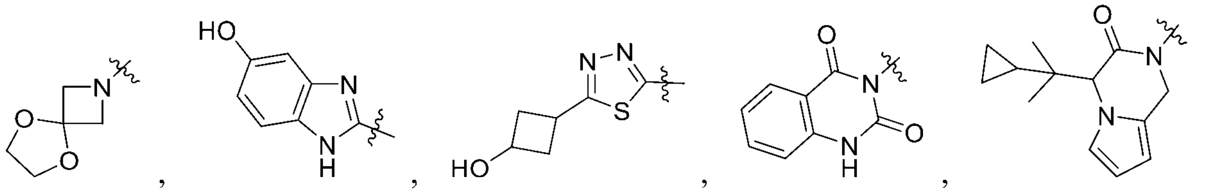
, , , , ,

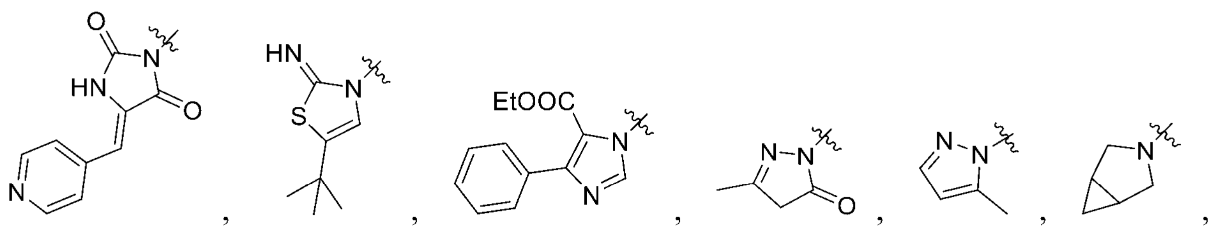
, , , , , ,

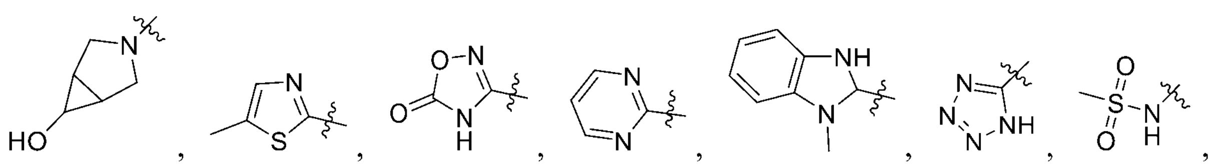
, , , , , , ,

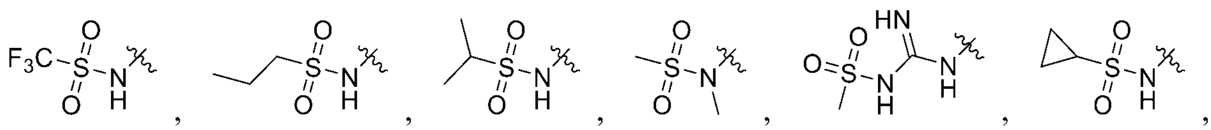
, , , , , ,

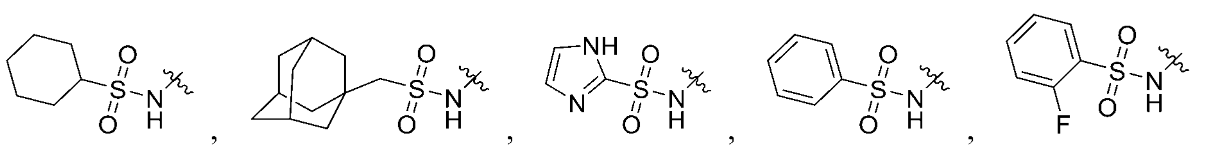
, , , , ,

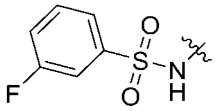, 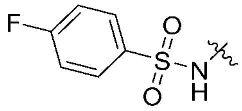, 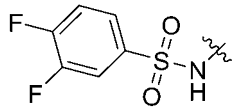, 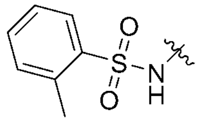, 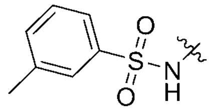,

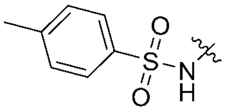, 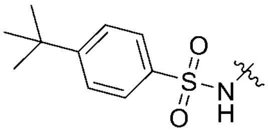, 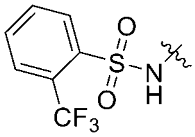, 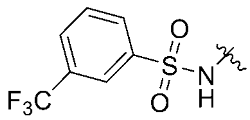,

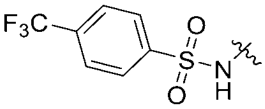, 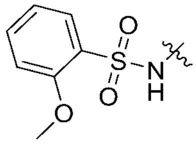, 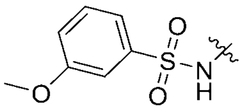, 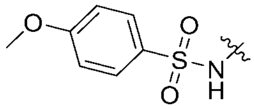,

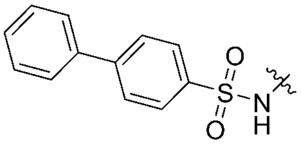, 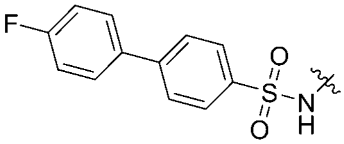, 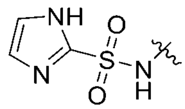, 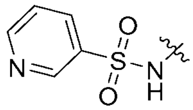,

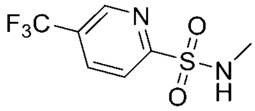, 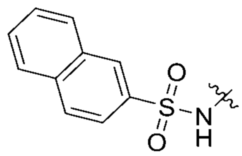 and 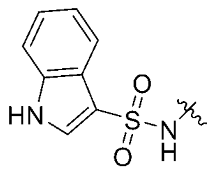.

In some preferable embodiments, $R_6$ is selected from the group consisting of hydroxyl, amino, methoxy, methylamino, guanidino, ureido,

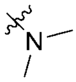, 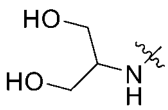, 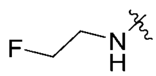,

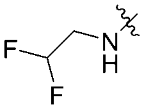, 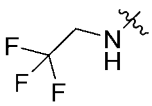, 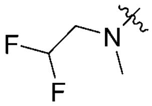, 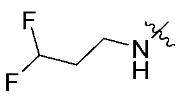, 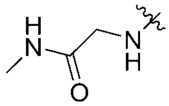, 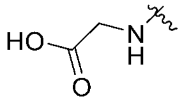,

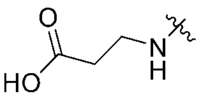, 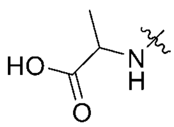, 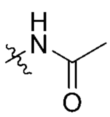, 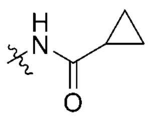, 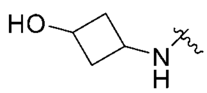, 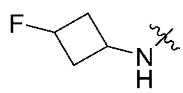,

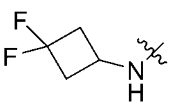, 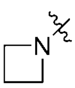, 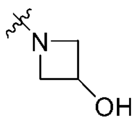, 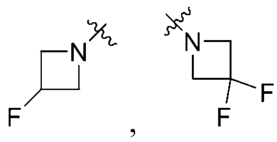, 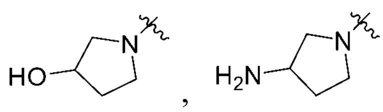,

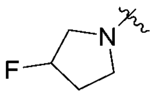, 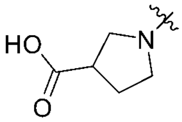, 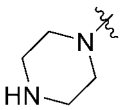, 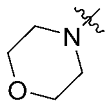, 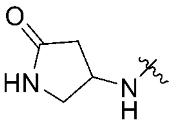, 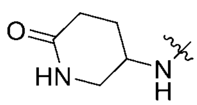,

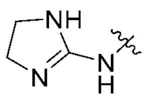, 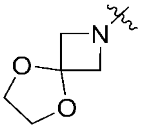, 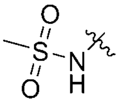, 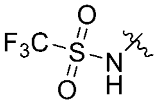, 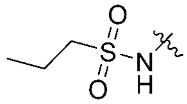, 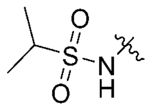, 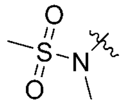,

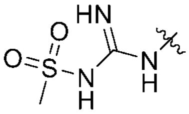, 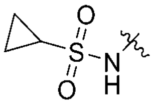, 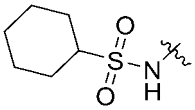, 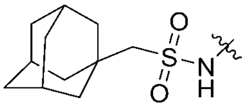, 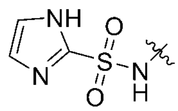,

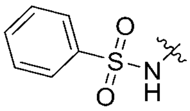, 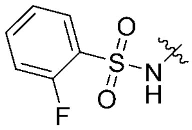, 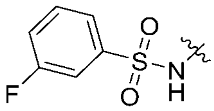, 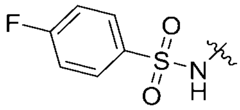, 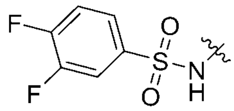,

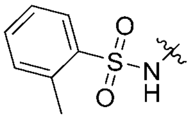, 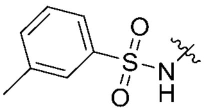, 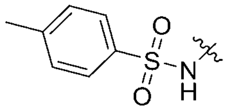, 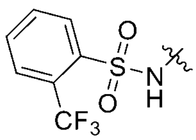, 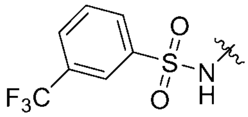,

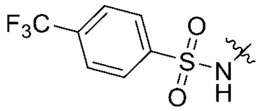, 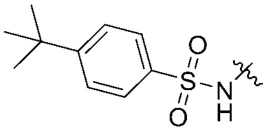, 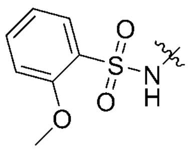, 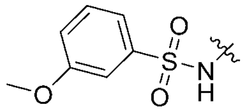,

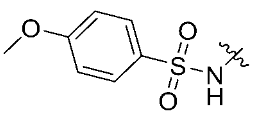, 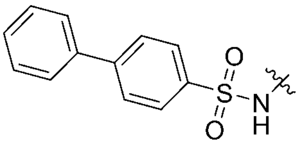, 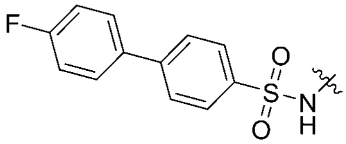, 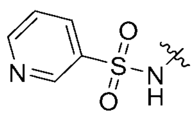,

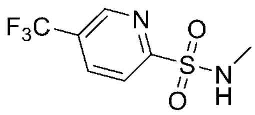, 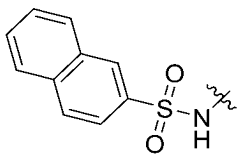 and 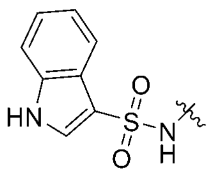.

In some more preferable embodiments, $R_6$ is selected from the group consisting of hydroxyl, amino, methylamino, guanidino, ureido,

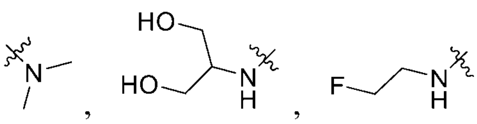, , ,

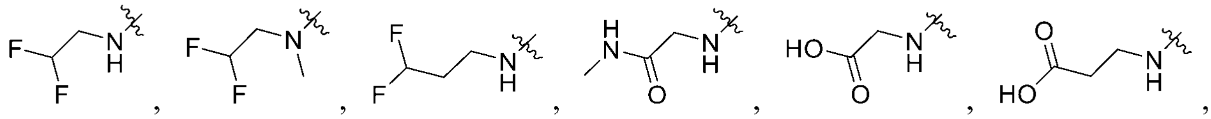 , , , , , ,

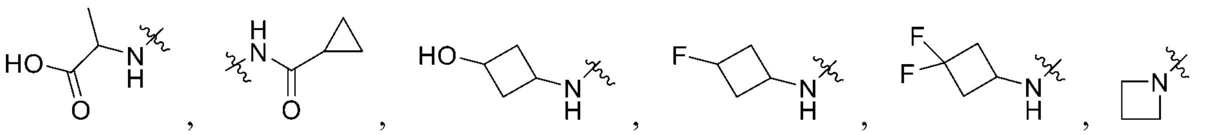 , , , , , ,

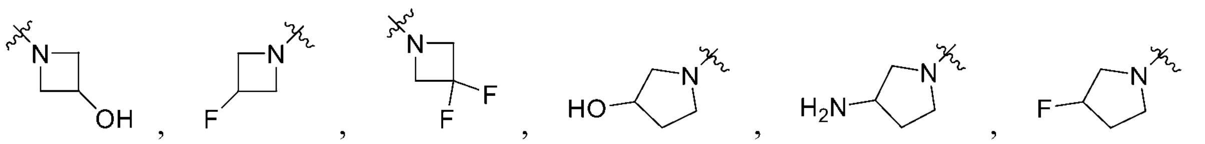 , , , , , ,

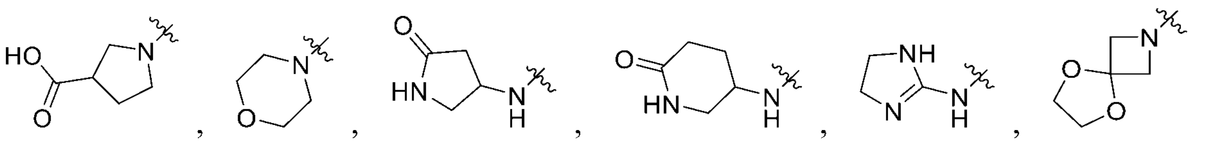 , , , , , ,

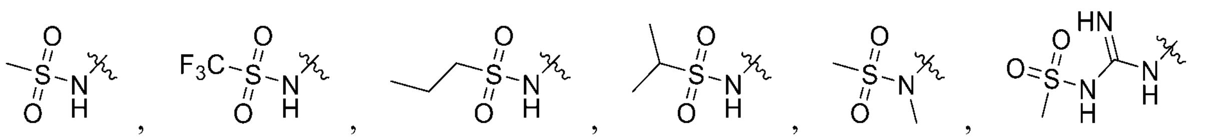 , , , , , ,

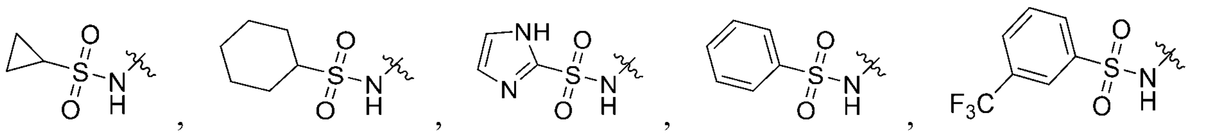 , , , , ,

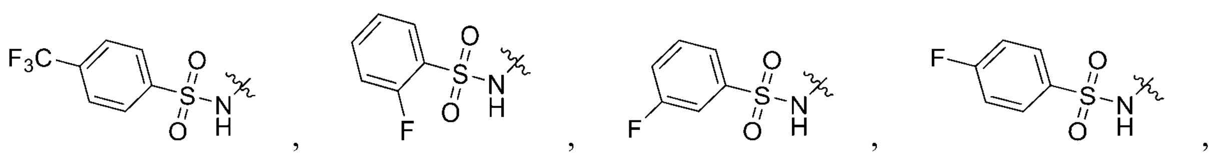 , , , ,

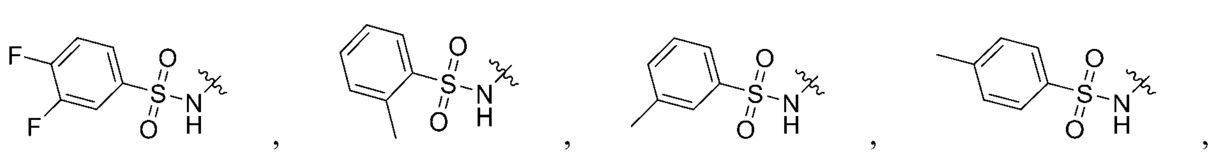 , , , ,

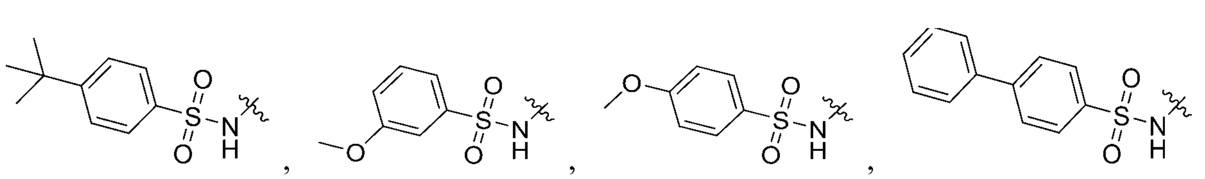 , , , ,

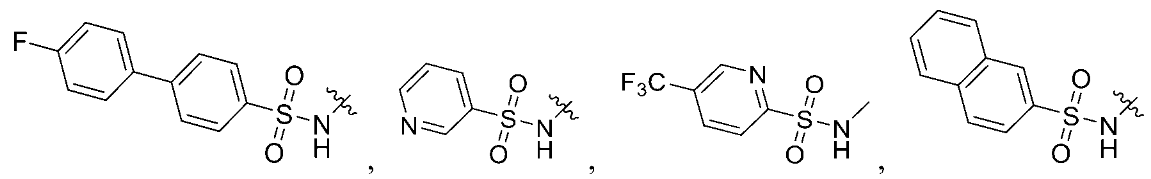 , , ,

and

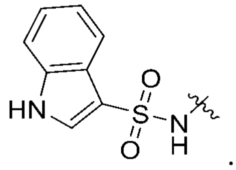

In a specific embodiment, $R_6$ is hydroxyl. In some embodiments, $R_6$ is selected from the group consisting of halogen. In some embodiments, $R_6$ is fluorine. In some embodiments, $R_6$ is cyano. In some embodiments, $R_6$ is azido. In a specific embodiment, $R_6$ is amino. In a specific embodiment, $R_6$ is methoxy. In a specific embodiment, $R_6$ is methylamino. In a specific embodiment, $R_6$ is tert-butyl. In a specific embodiment, $R_6$ is propenyl. In a specific embodiment, $R_6$ is guanidine. In a specific embodiment, $R_6$ is a urea group. In a specific embodiment, $R_6$ is

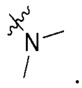

In a specific embodiment, $R_6$ is

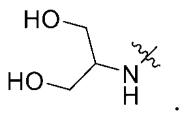

In a specific embodiment, $R_6$ is

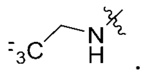

In a specific embodiment, $R_6$ is

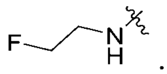

In a specific embodiment, $R_6$ is

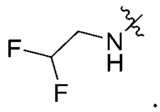

In a specific embodiment, $R_6$ is

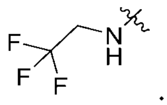

In a specific embodiment, $R_6$ is

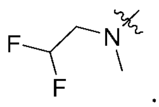

In a specific embodiment, $R_6$ is

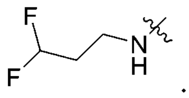

In a specific embodiment, $R_6$ is

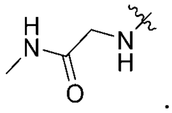

In a specific embodiment, $R_6$ is

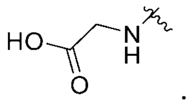

In a specific embodiment, $R_6$ is

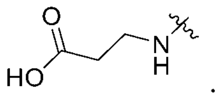

In a specific embodiment, $R_6$ is

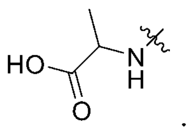

In a specific embodiment, $R_6$ is

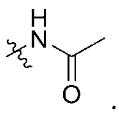

In a specific embodiment, $R_6$ is

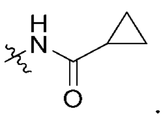

In a specific embodiment, $R_6$ is

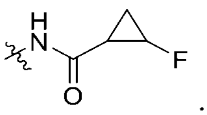

In a specific embodiment, $R_6$ is

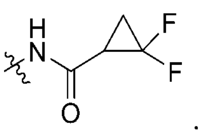

In a specific embodiment, $R_6$ is

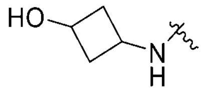.

In a specific embodiment, $R_6$ is

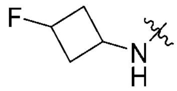.

In a specific embodiment, $R_6$ is

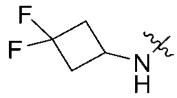.

In a specific embodiment, $R_6$ is

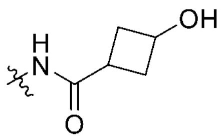.

In a specific embodiment, $R_6$ is

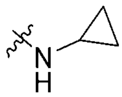.

In a specific embodiment, $R_6$ is

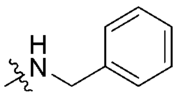.

In a specific embodiment, $R_6$ is

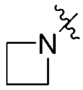.

In a specific embodiment, $R_6$ is

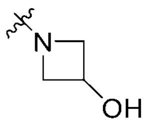.

In a specific embodiment, $R_6$ is

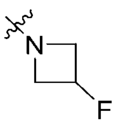.

In a specific embodiment, $R_6$ is

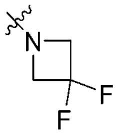
.

In a specific embodiment, $R_6$ is

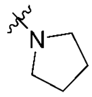
.

In a specific embodiment, $R_6$ is

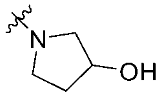
.

In a specific embodiment, $R_6$ is

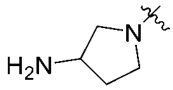
.

In a specific embodiment, $R_6$ is

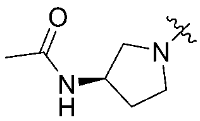
.

In a specific embodiment, $R_6$ is

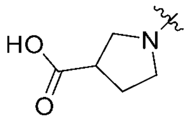
. ]

In a specific embodiment, $R_6$ is

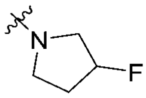
.

In a specific embodiment, $R_6$ is

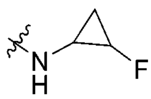
.

In a specific embodiment, $R_6$ is

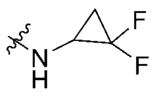
.

In a specific embodiment, $R_6$ is

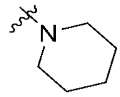 .

In a specific embodiment, $R_6$ is

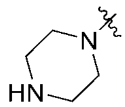 .

In a specific embodiment, $R_6$ is

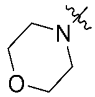 .

In a specific embodiment, $R_6$ is

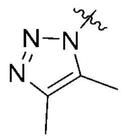 .

In a specific embodiment, $R_6$ is

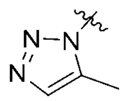 .

In a specific embodiment, $R_6$ is

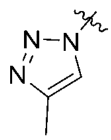 .

In a specific embodiment, $R_6$ is

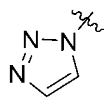 .

In a specific embodiment, $R_6$ is

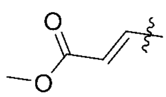 .

In a specific embodiment, $R_6$ is

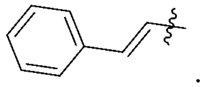

In a specific embodiment, $R_6$ is

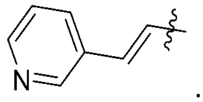

In a specific embodiment, $R_6$ is

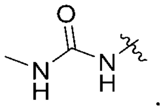

In a specific embodiment, $R_6$ is

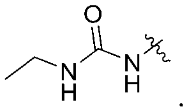

In a specific embodiment, $R_6$ is

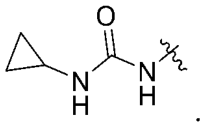

In a specific embodiment, $R_6$ is

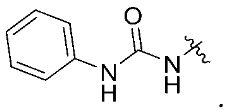

In a specific embodiment, $R_6$ is

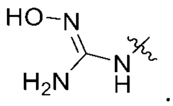

In a specific embodiment, $R_6$ is

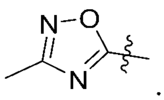

In a specific embodiment, $R_6$ is

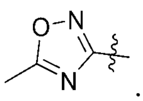

In a specific embodiment, $R_6$ is

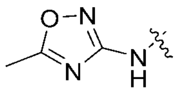.

In a specific embodiment, $R_6$ is

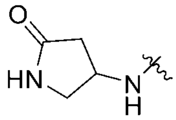.

In a specific embodiment, $R_6$ is

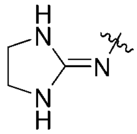.

In a specific embodiment, $R_6$ is

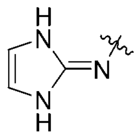.

In a specific embodiment, $R_6$ is

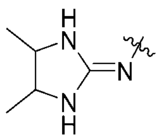.

In a specific embodiment, $R_6$ is

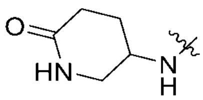.

In a specific embodiment, $R_6$ is

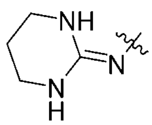.

In a specific embodiment, $R_6$ is

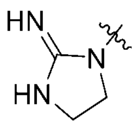.

In a specific embodiment, $R_6$ is

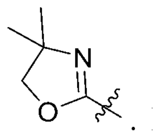

In a specific embodiment, $R_6$ is

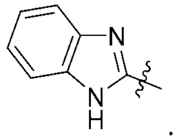

In a specific embodiment, $R_6$ is

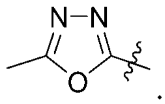

In a specific embodiment, $R_6$ is

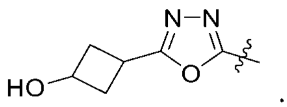

In a specific embodiment, $R_6$ is

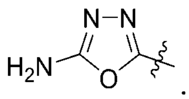

In a specific embodiment, $R_6$ is

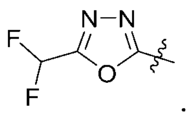

In a specific embodiment, $R_6$ is

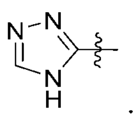

In a specific embodiment, $R_6$ is

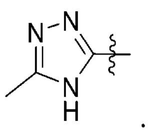

In a specific embodiment, $R_6$ is

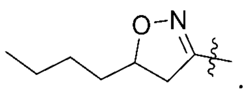

In a specific embodiment, $R_6$ is

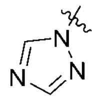.

In a specific embodiment, $R_6$ is

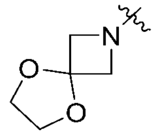.

In a specific embodiment, $R_6$ is

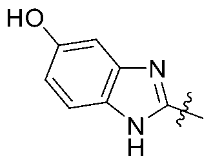.

In a specific embodiment, $R_6$ is

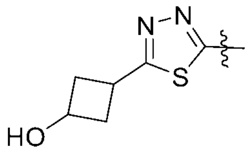.

In a specific embodiment, $R_6$ is

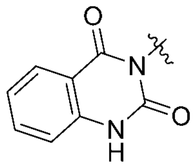.

In a specific embodiment, $R_6$ is

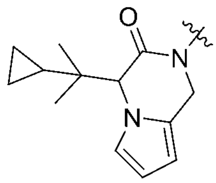.

In a specific embodiment, $R_6$ is

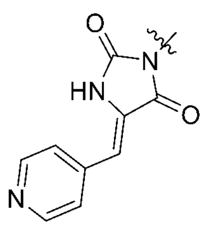.

In a specific embodiment, $R_6$ is

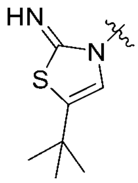
.

In a specific embodiment, $R_6$ is

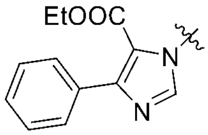
.

In a specific embodiment, $R_6$ is

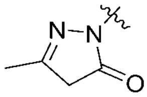
.

In a specific embodiment, $R_6$ is

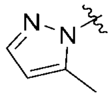
.

In a specific embodiment, $R_6$ is

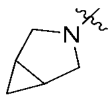
.

In a specific embodiment, $R_6$ is

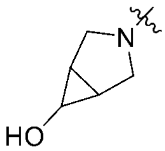
.

In a specific embodiment, $R_6$ is

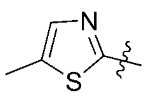
.

In a specific embodiment, $R_6$ is

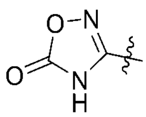
.

In a specific embodiment, $R_6$ is

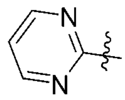
.

In a specific embodiment, $R_6$ is

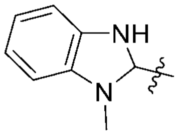
.

In a specific embodiment, $R_6$ is

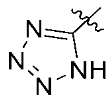
.

In a specific embodiment, $R_6$ is

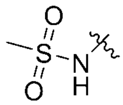
.

In a specific embodiment, $R_6$ is

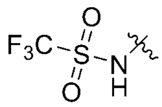
.

In a specific embodiment, $R_6$ is

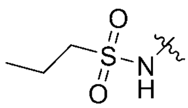
.

In a specific embodiment, $R_6$ is

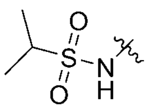
.

In a specific embodiment, $R_6$ is

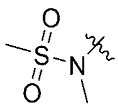
.

In a specific embodiment, $R_6$ is

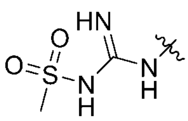
.

In a specific embodiment, $R_6$ is

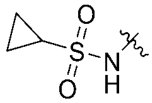

.

In a specific embodiment, $R_6$ is

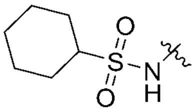

.

In a specific embodiment, $R_6$ is

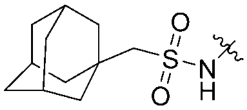

.

In a specific embodiment, $R_6$ is

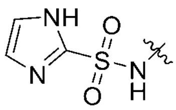

.

In a specific embodiment, $R_6$ is

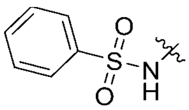

.

In a specific embodiment, $R_6$ is

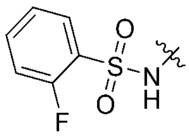

.

In a specific embodiment, $R_6$ is

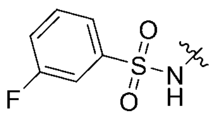

.

In a specific embodiment, $R_6$ is

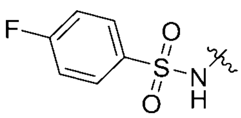

.

In a specific embodiment, $R_6$ is

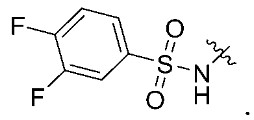

In a specific embodiment, $R_6$ is

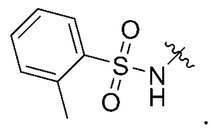

In a specific embodiment, $R_6$ is

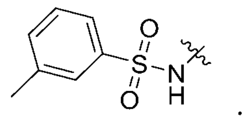

In a specific embodiment, $R_6$ is

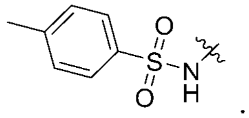

In a specific embodiment, $R_6$ is

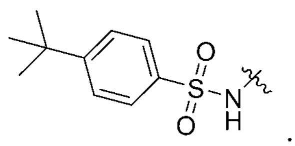

In a specific embodiment, $R_6$ is

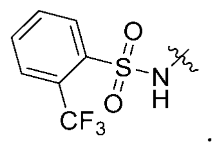

In a specific embodiment, $R_6$ is

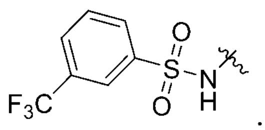

In a specific embodiment, $R_6$ is

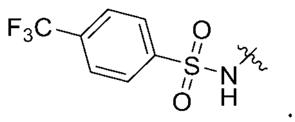

In a specific embodiment, $R_6$ is

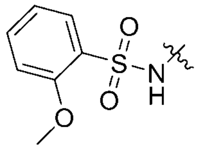.

In a specific embodiment, $R_6$ is

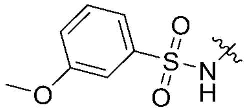.

In a specific embodiment, $R_6$ is

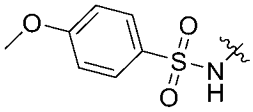.

In a specific embodiment, $R_6$ is

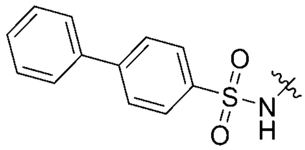.

In a specific embodiment, $R_6$ is

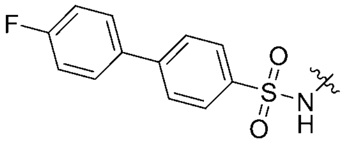.

In a specific embodiment, $R_6$ is

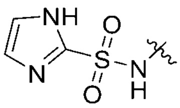.

In a specific embodiment, $R_6$ is

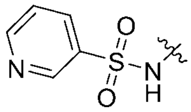.

In a specific embodiment, $R_6$ is

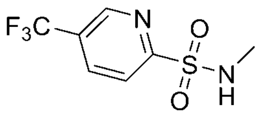.

In a specific embodiment, $R_6$ is

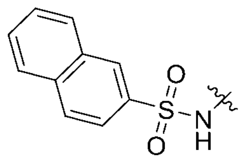.

In a specific embodiment, $R_6$ is

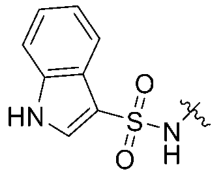.

In some embodiments, $R_5$ and $R_6$ together with the atom(s) to which they are attached form a 3-12 membered heterocycle. In a specific embodiment, $R_5$ and $R_6$ together with the atom(s) to which they are attached form a heterocycle selected from the group consisting of ethylene oxide,

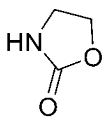, 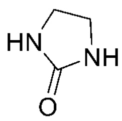, 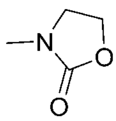, 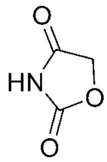 and 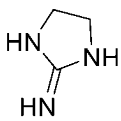.

In a specific embodiment, $R_5$ and $R_6$ together with the atom(s) to which they are attached form a heterocycle of ethylene oxide. In a particular embodiment, $R_5$ and $R_6$ together with the atom(s) to which they are attached form a heterocycle of

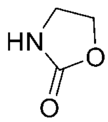.

In a particular embodiment, $R_5$ and $R_6$ together with the atom(s) to which they are attached form a heterocycle of

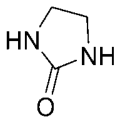.

In a particular embodiment, $R_5$ and $R_6$ together with the atom(s) to which they are attached form a heterocycle of

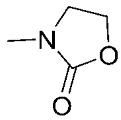.

In a particular embodiment, $R_5$ and $R_6$ together with the atom(s) to which they are attached form a heterocycle of

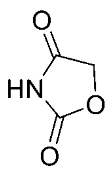.

In a particular embodiment, $R_5$ and $R_6$ together with the atom(s) to which they are attached form a heterocycle of

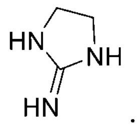

In some embodiments, $R_7$ is selected from the group consisting of hydrogen, hydroxyl, $C_{1-8}$ alkyl, and $-O-C_{1-8}$ alkyl, wherein the alkyl is optionally substituted with at least one $R^X$. In a specific embodiment, $R_7$ is hydrogen. In a specific embodiment, $R_7$ is hydroxyl. In a specific embodiment, $R_7$ is methoxy. In a specific embodiment, $R_7$ is trifluoromethoxy. $R_7$ is difluoromethoxy. In a specific embodiment, $R_7$ is methyl. In a specific embodiment, $R_7$ is trifluoromethyl. In a specific embodiment, $R_7$ is difluoromethyl.

In a specific embodiment, $R_8$ is hydroxyl.

In some embodiments, $R_4$ and $R_8$ together with the atom(s) to which they are attached form a heterocycle having the following structure:

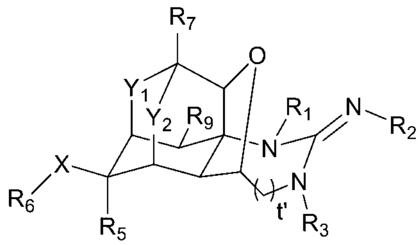

,

wherein X, $Y_1$, $Y_2$, $R_1$, $R_2$, $R_3$, $R_5$, $R_6$, $R_7$ and $R_9$ are as defined herein for formula (I), t' is an integer selected from 0-3, for example t' is 0, 1, 2 or 3.

In some embodiments, in the compound of formula (I) or the pharmaceutically acceptable derivative thereof as provided herein, $R_4$ and $R_8$ together with the atom(s) to which they are attached form a heterocycle, which has the following structure:

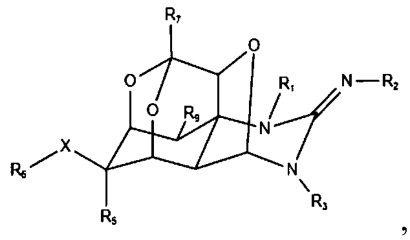

,

wherein X, $R_1$, $R_2$, $R_3$, $R_5$, $R_6$, $R_7$ and $R_9$ are as defined herein for formula (I).

In some embodiments, $R_9$ is selected from the group consisting of hydrogen, deuterium, hydroxyl, and $-OR_{16}$. In a specific embodiment, $R_9$ is hydrogen. In a specific embodiment, $R_9$ is deuterium. In a specific embodiment, $R_9$ is hydroxyl. In a specific embodiment, $R_9$ is methoxy. In a specific embodiment, $R_9$ is

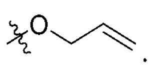

In some embodiments, m is 0. In some embodiments, m is 1. In some embodiments, m is 2. In some embodiments, m is 3. In some embodiments, m is 4. In some embodiments, m is 5. In some embodiments, m is 6.

In some embodiments, n is 1. In some embodiments, n is 2. In some embodiments, n is 3. In some embodiments, n is 4. In some embodiments, n is 5. In some embodiments, n is 6. In some embodiments, n is 7. In some embodiments, n is 8. In some embodiments, n is 9. In some embodiments, n is 10. In some embodiments, n is 11. In some embodiments, n is 12.

In some embodiments, t is 0. In some embodiments, t is 1. In some embodiments, t is 2. In some embodiments, t is 3. In some embodiments, t is 4.

The present disclosure encompasses any combination of the above embodiments.

In some embodiments, the compound of the present disclosure comprises, but is not limited to:

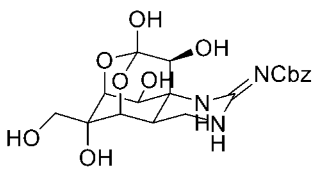
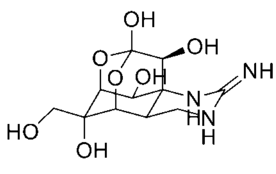
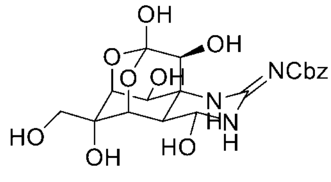

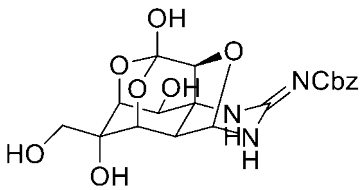
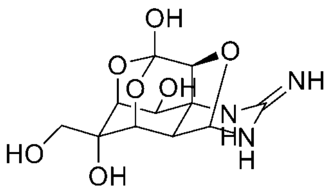
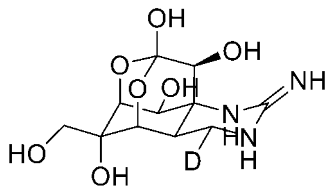

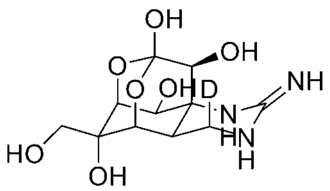
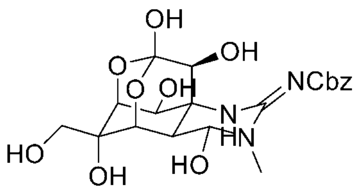
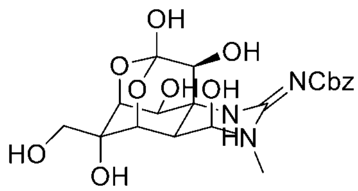

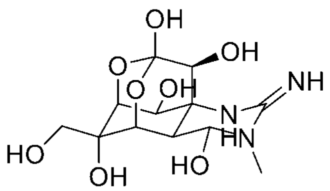
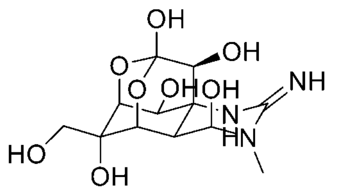
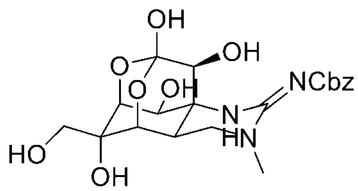

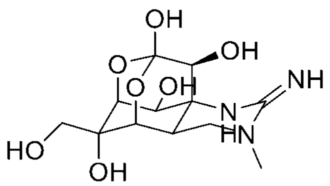
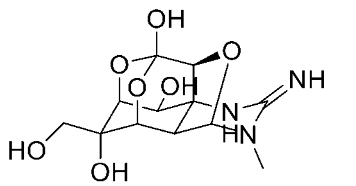
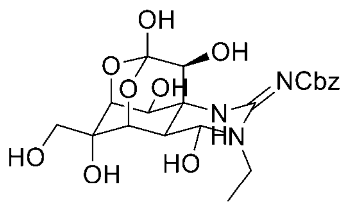

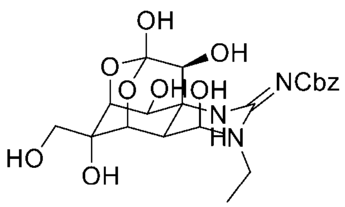
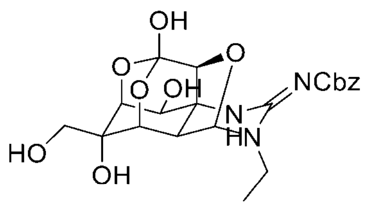
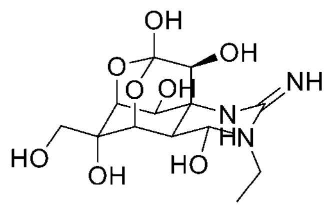

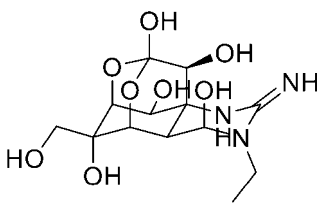
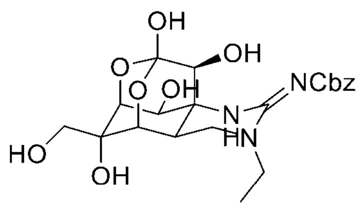
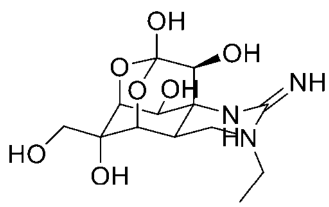

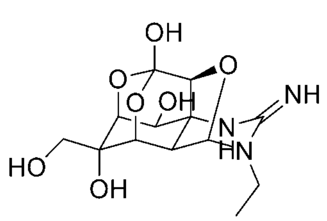
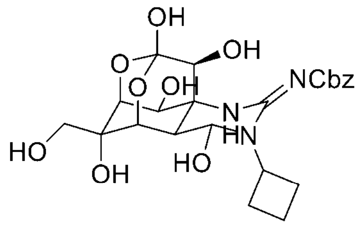
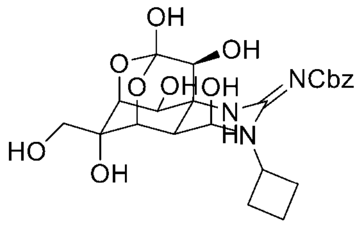

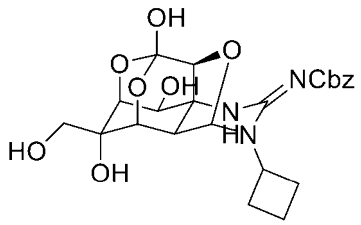
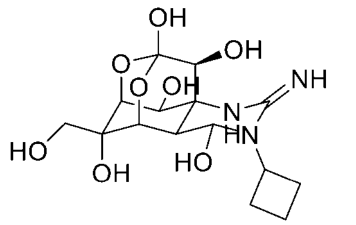
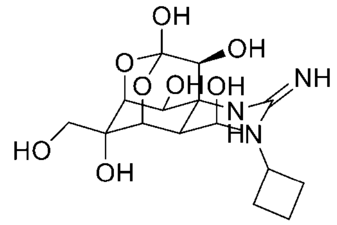

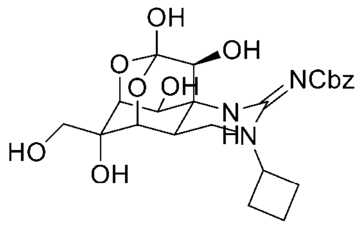
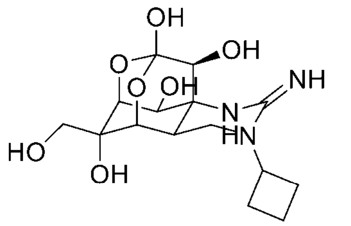
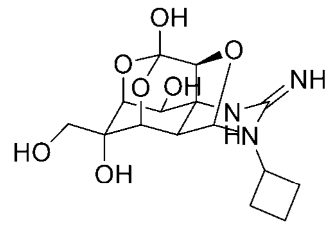

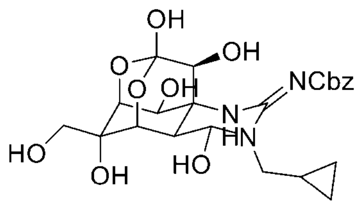
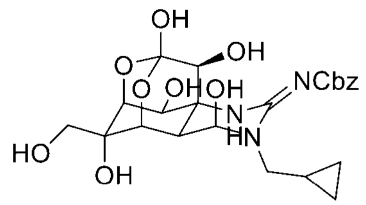
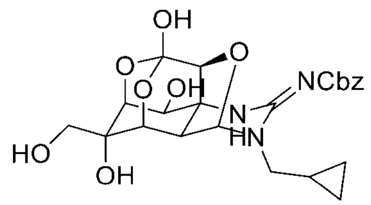

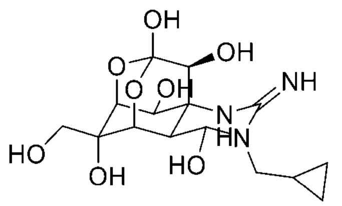
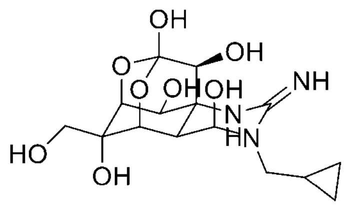
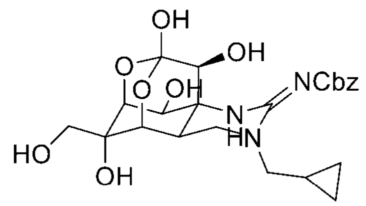

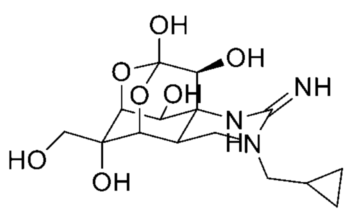
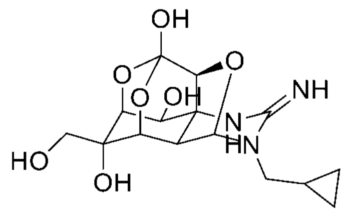
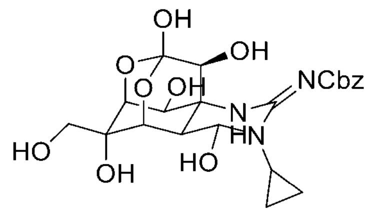

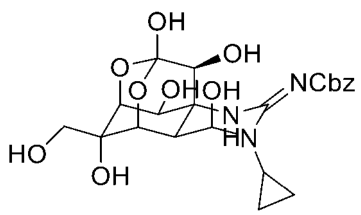
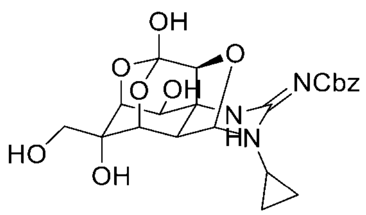
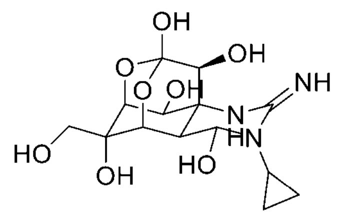

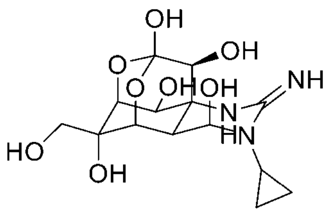
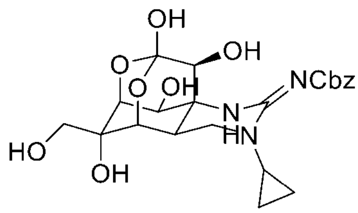
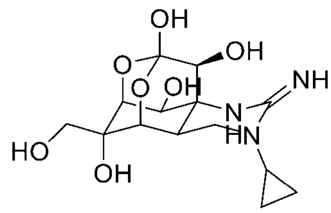
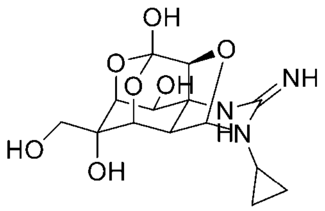
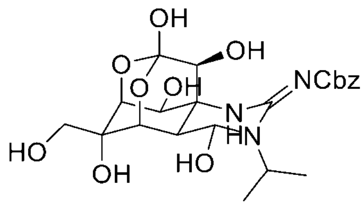
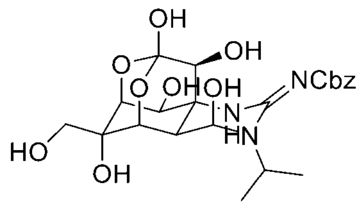
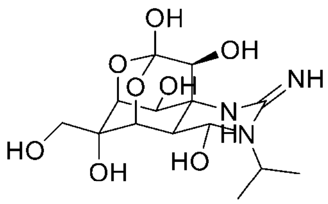
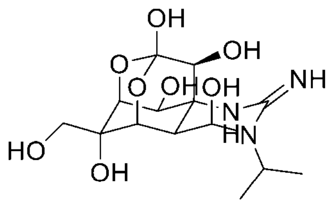
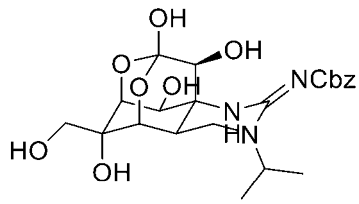
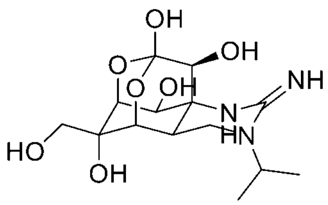
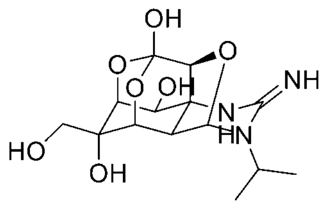
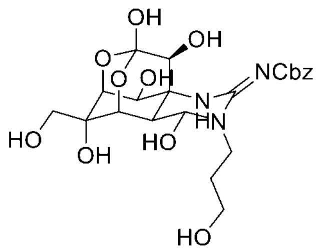
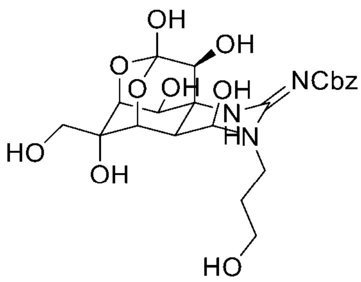
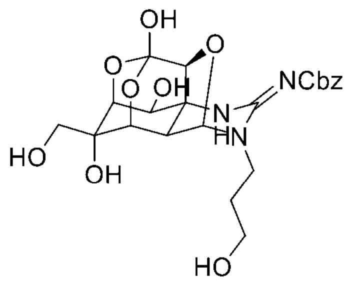
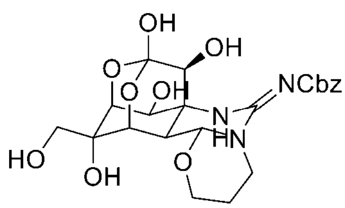
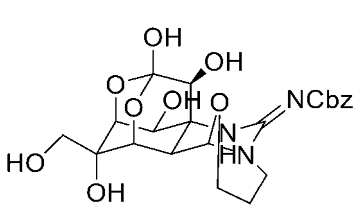
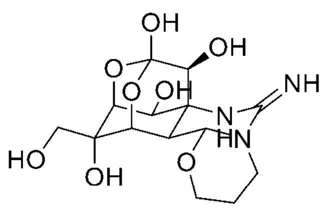
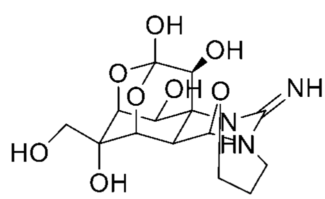

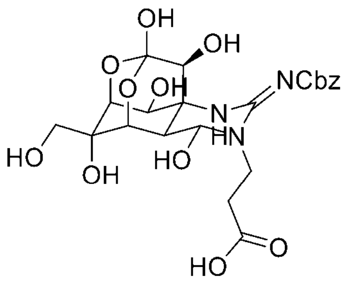
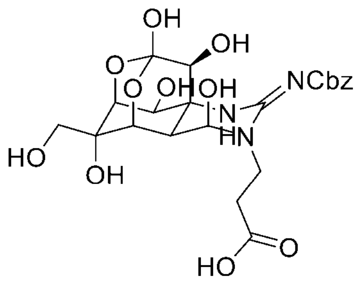
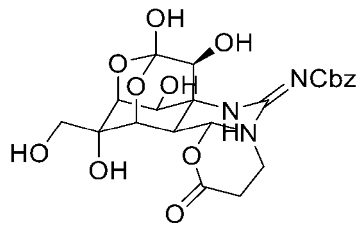

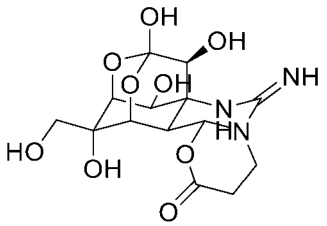
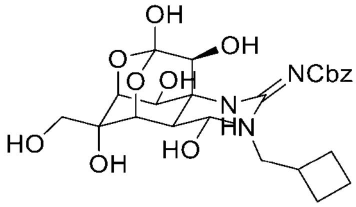
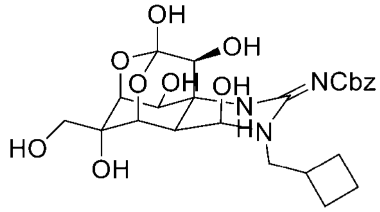

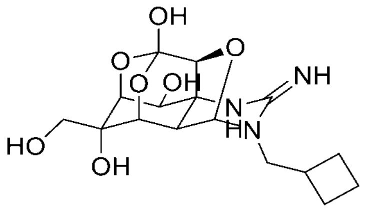
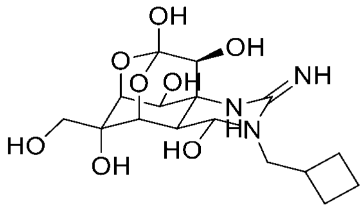
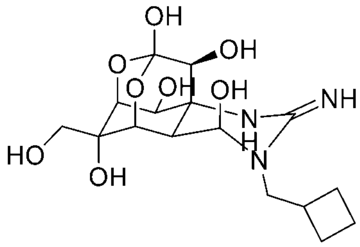

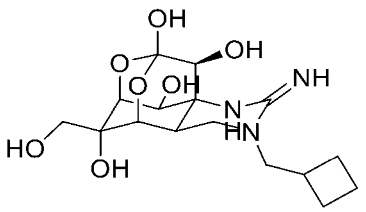
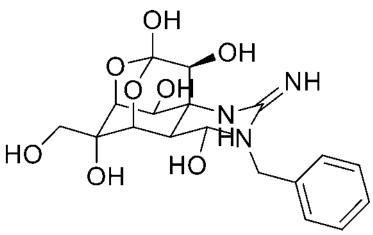
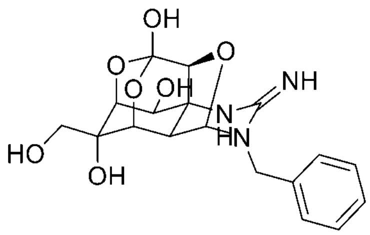

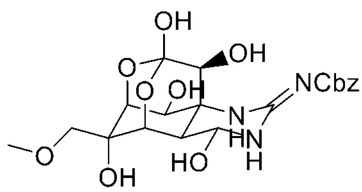
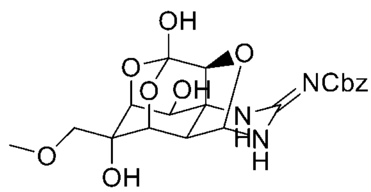
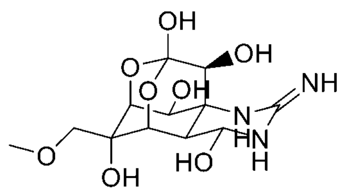

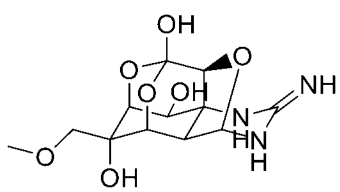
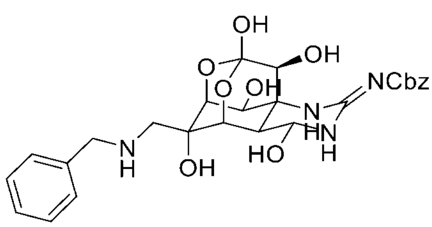
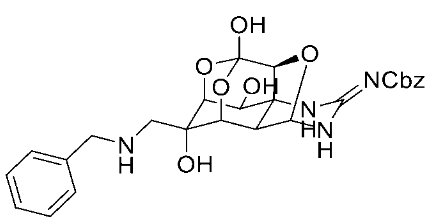

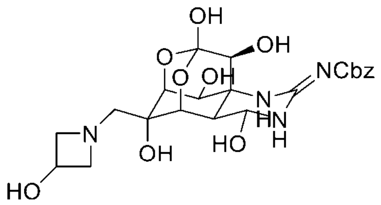 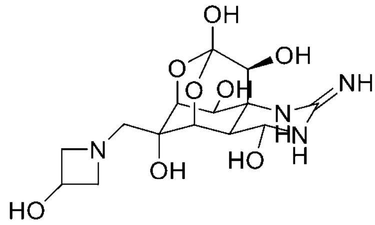 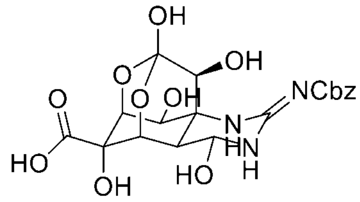

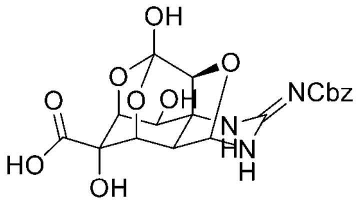 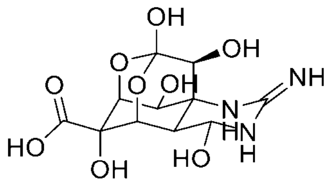 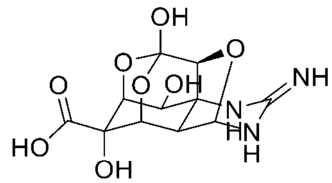

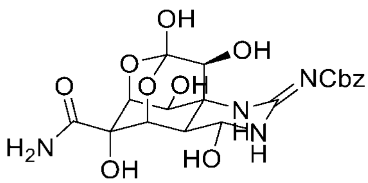 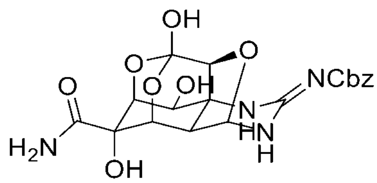 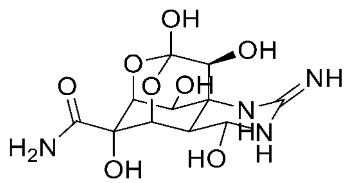

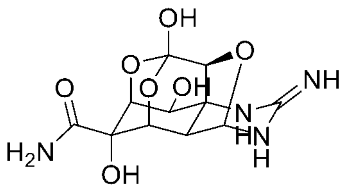 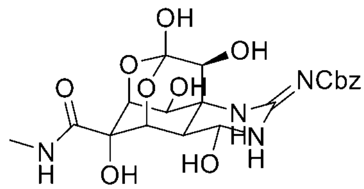 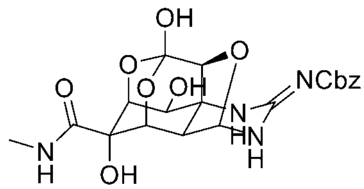

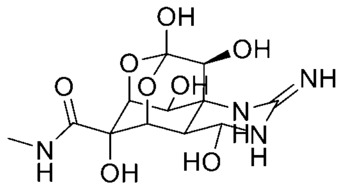 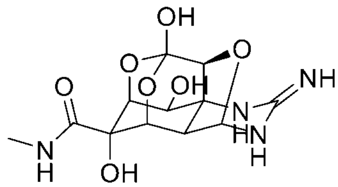 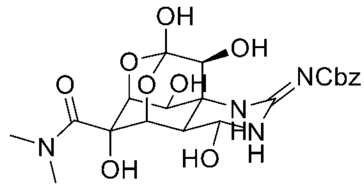

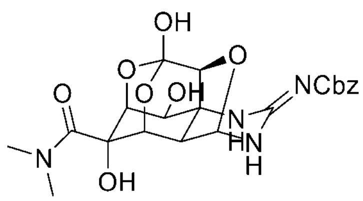 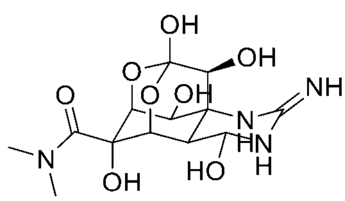 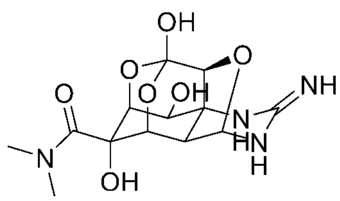

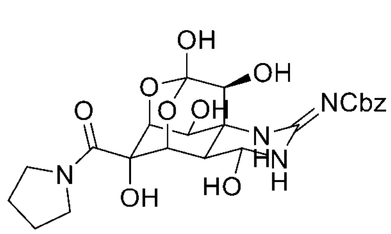 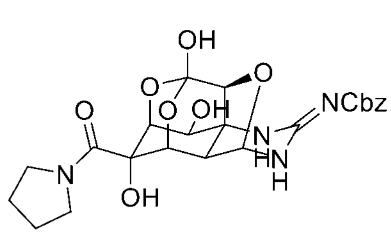 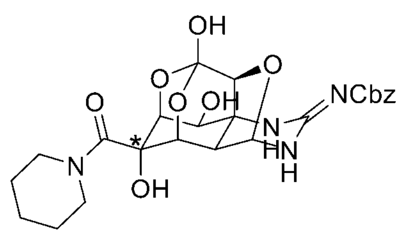

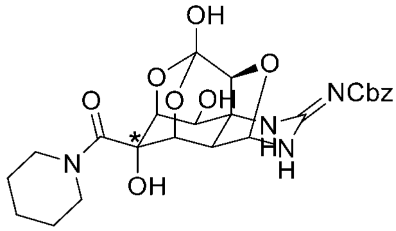 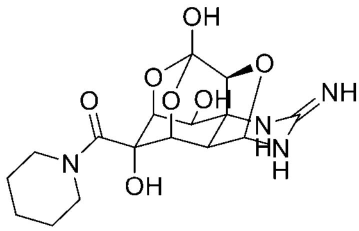 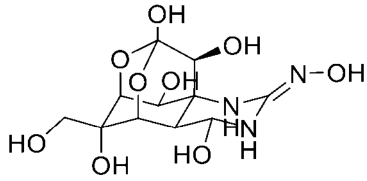

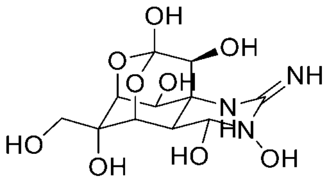 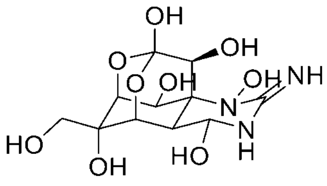 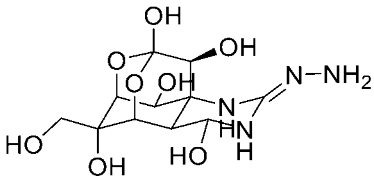

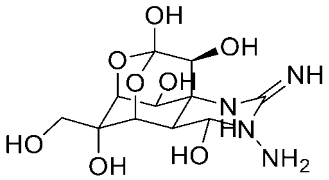 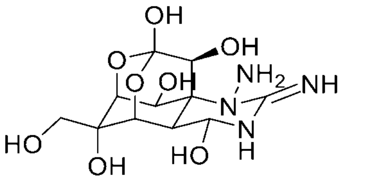 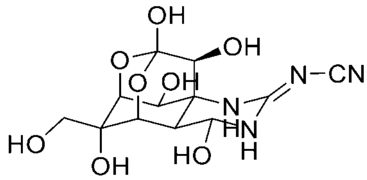

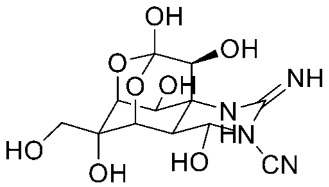 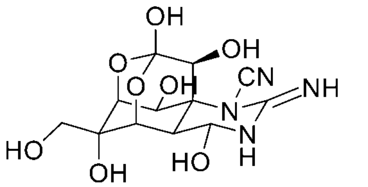 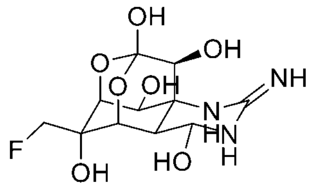

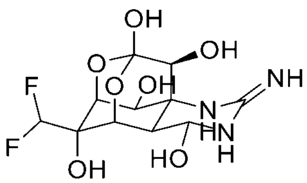 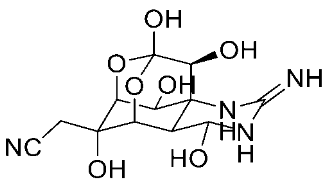 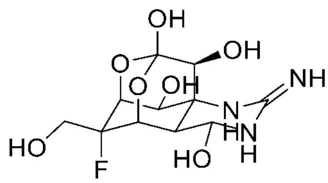

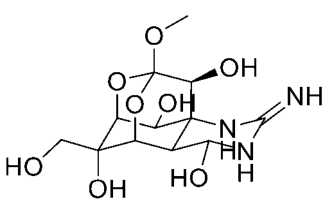 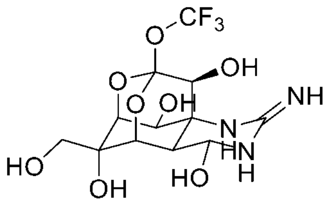 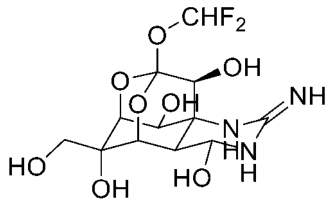

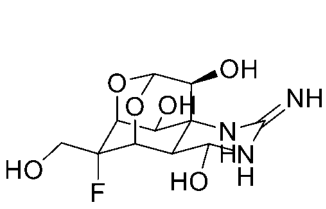 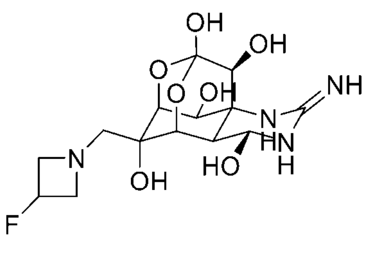 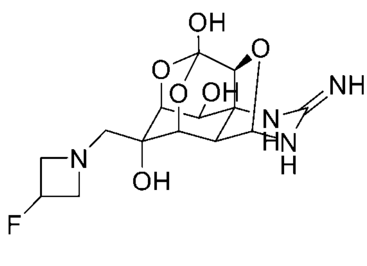

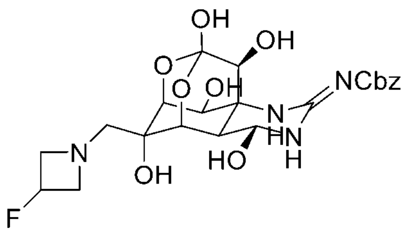
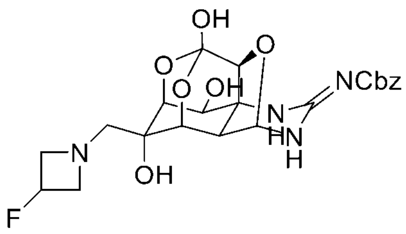
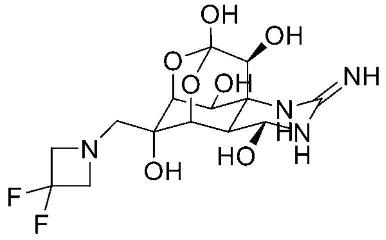

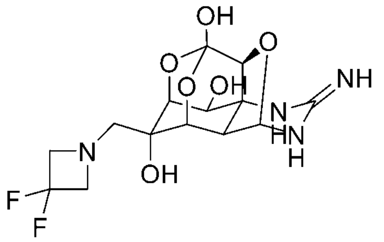
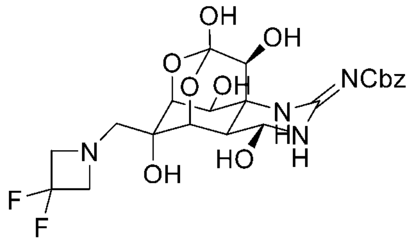
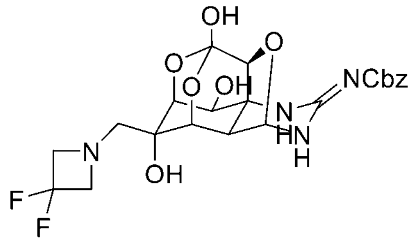

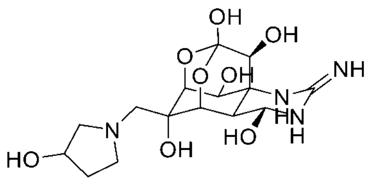
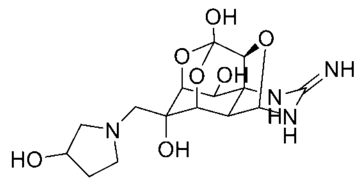
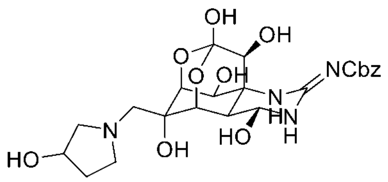

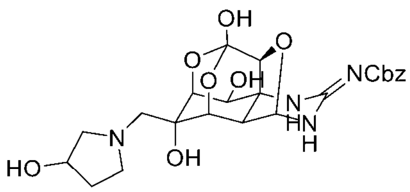
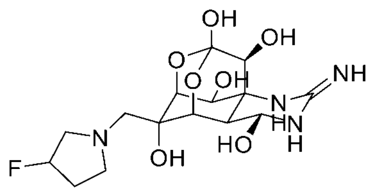
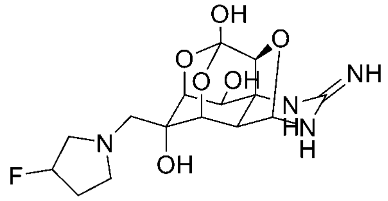

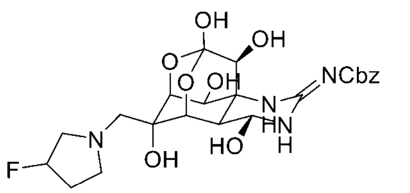
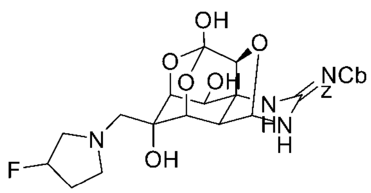
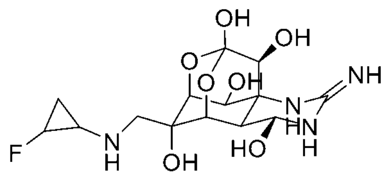

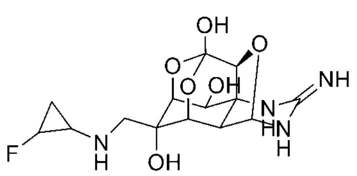
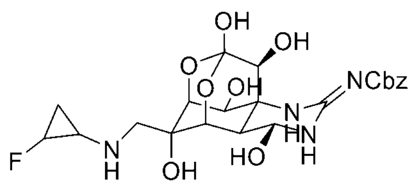
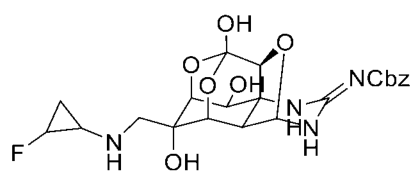

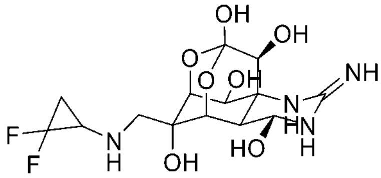
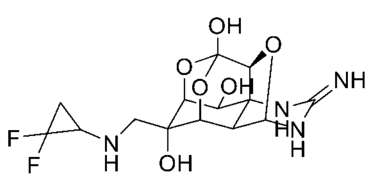
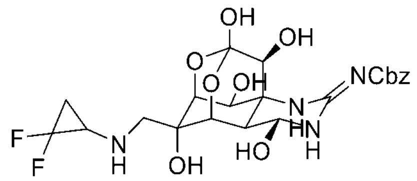

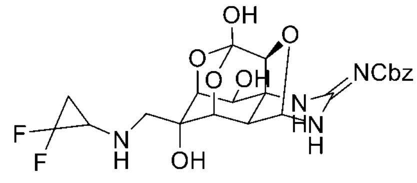
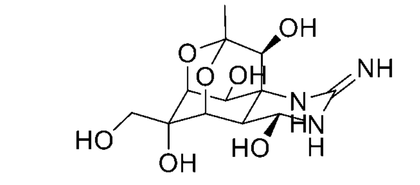
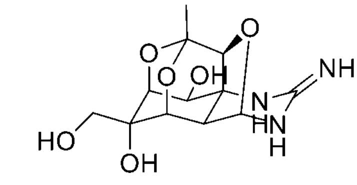
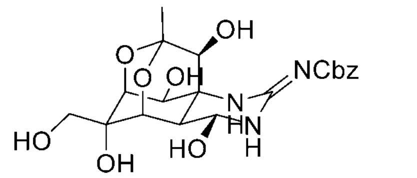
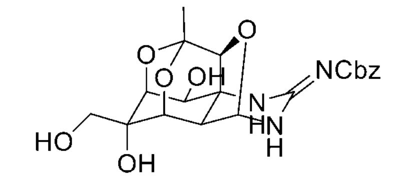
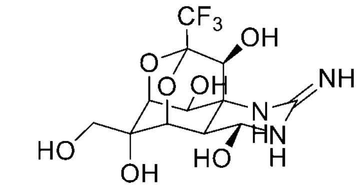
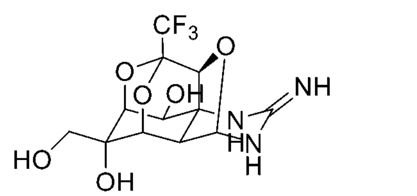
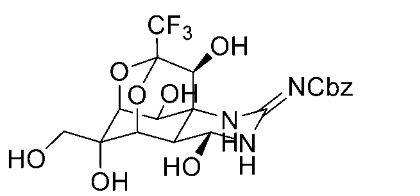
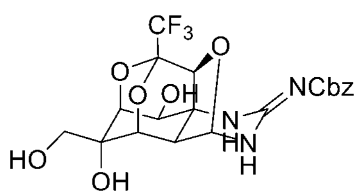
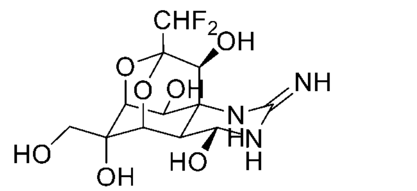
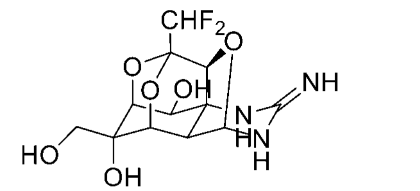
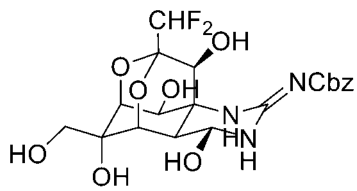
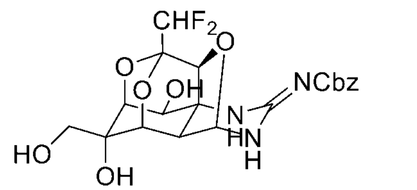
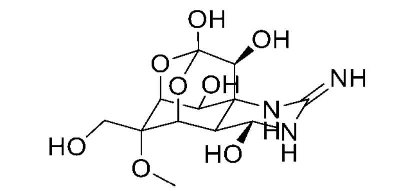
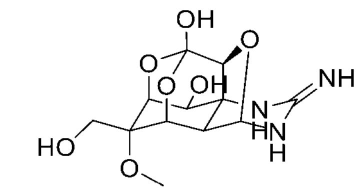
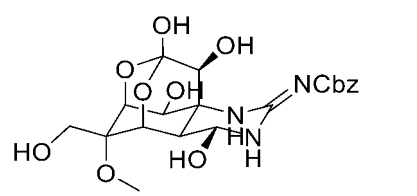
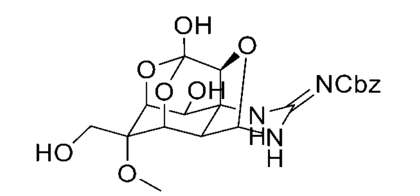
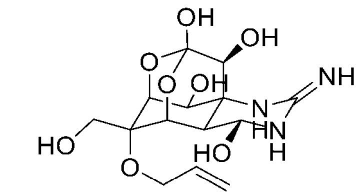
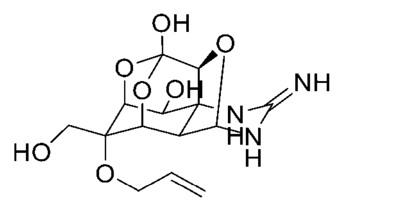
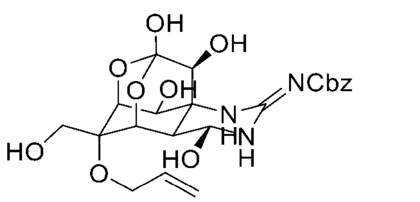
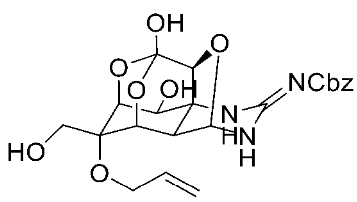
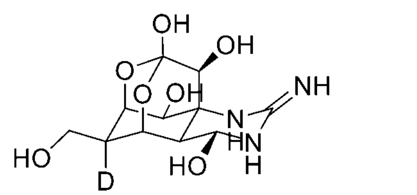
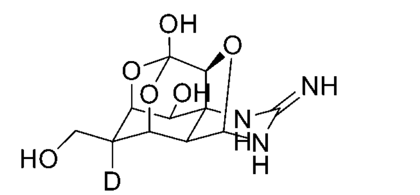
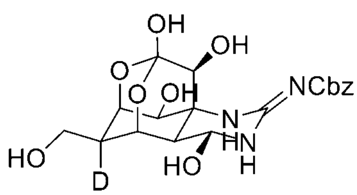

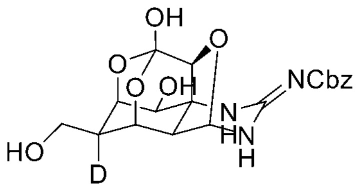
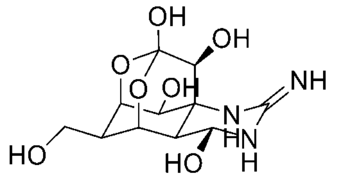
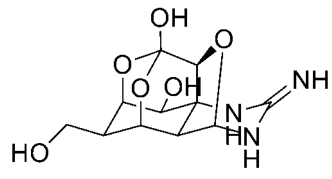
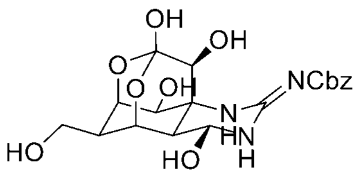
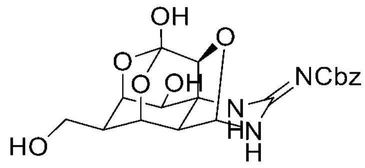
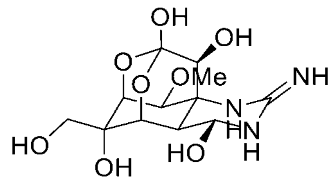
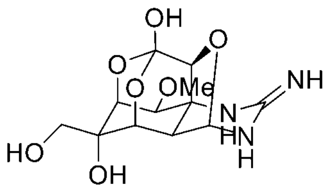
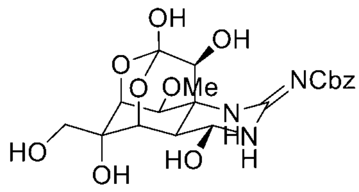
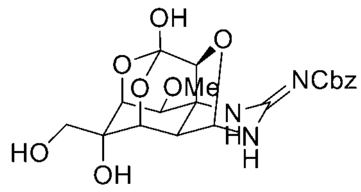
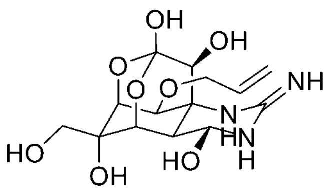
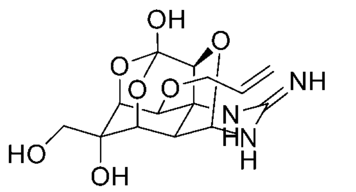
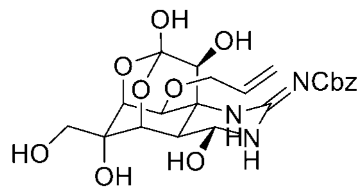
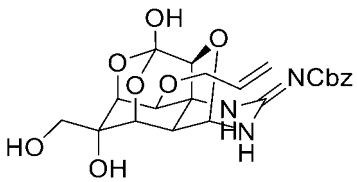
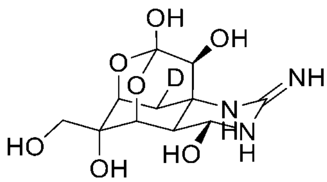
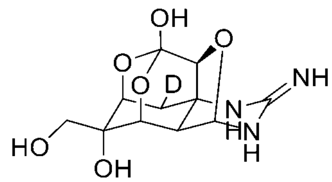
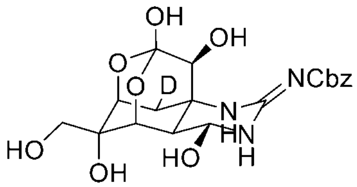
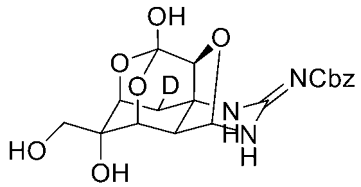
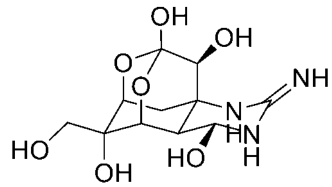
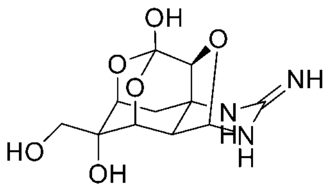
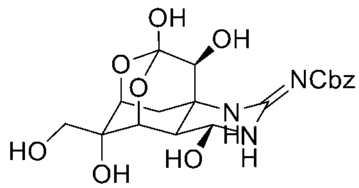
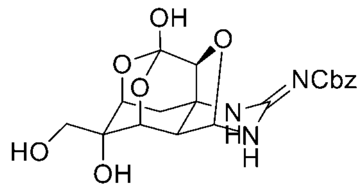
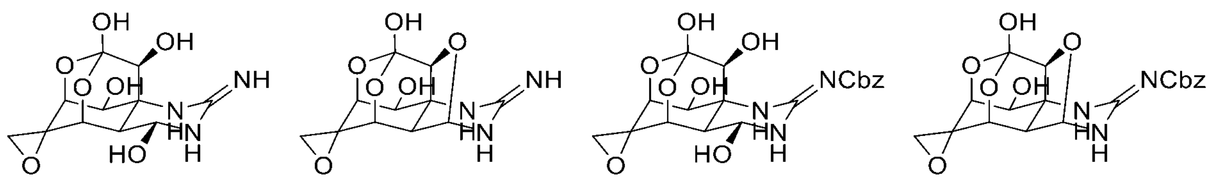

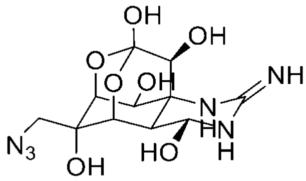
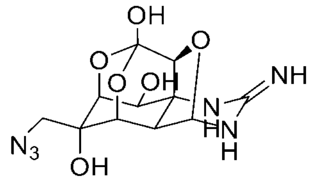
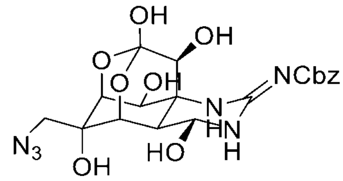
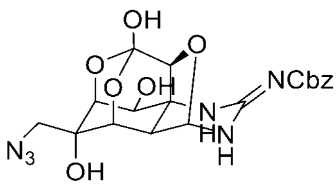
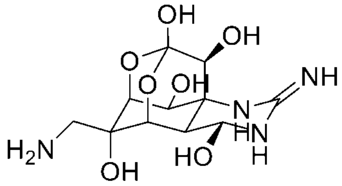
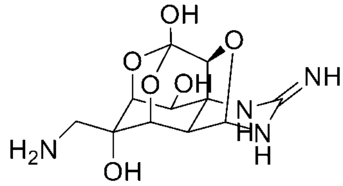
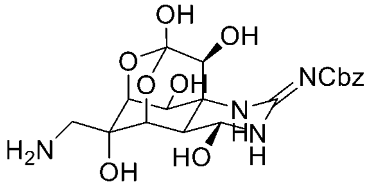
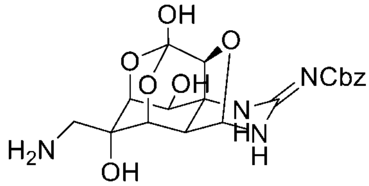
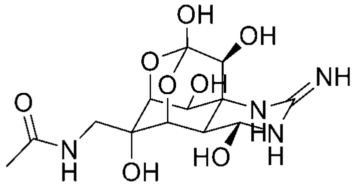
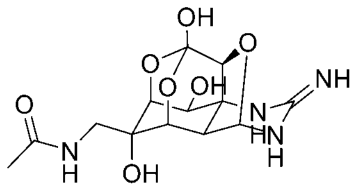
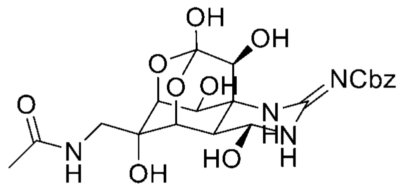
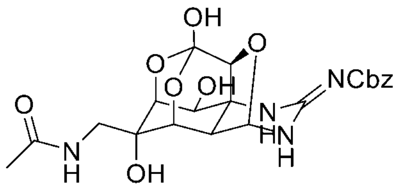
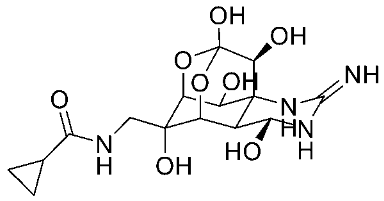
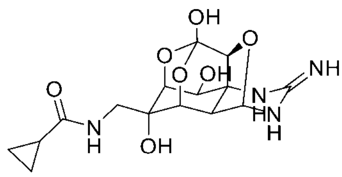
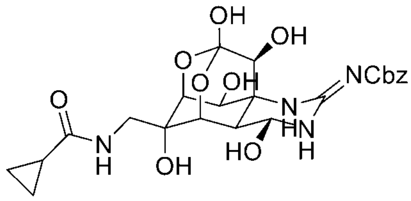
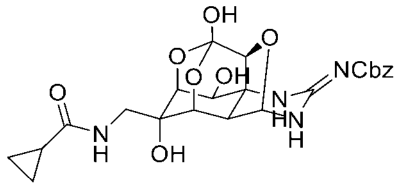
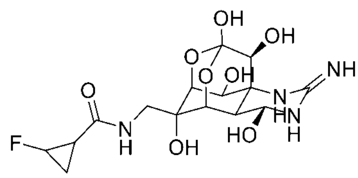
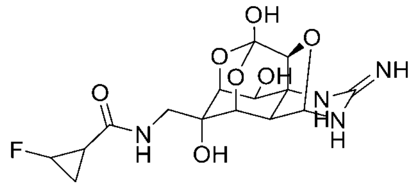
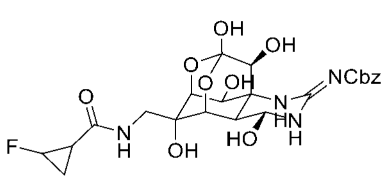
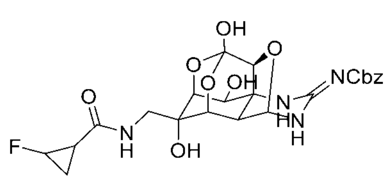
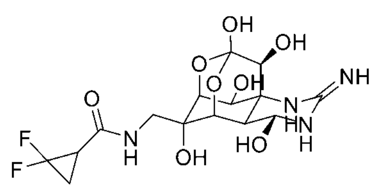
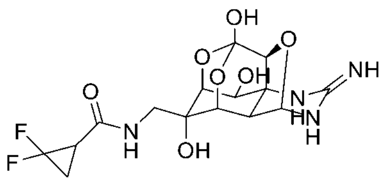
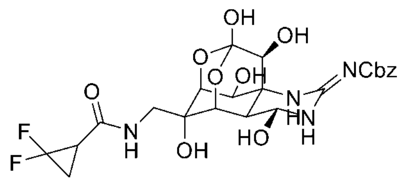
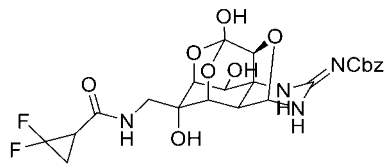

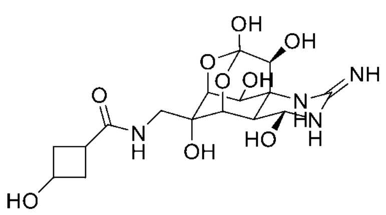
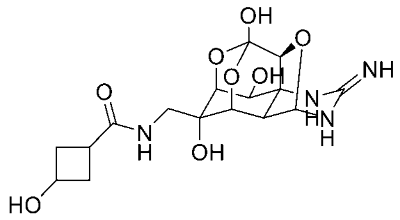
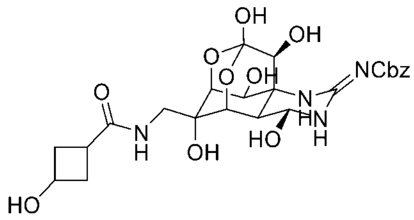

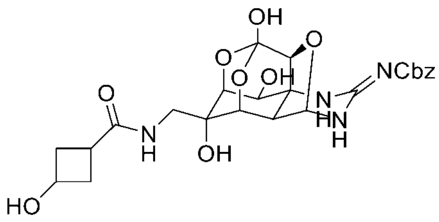
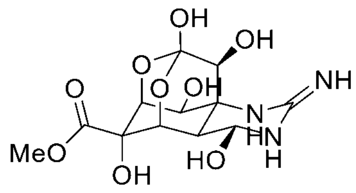
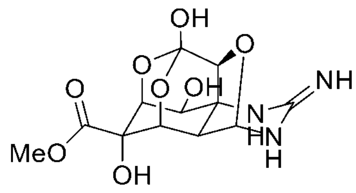

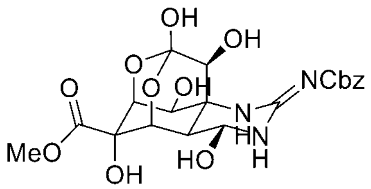
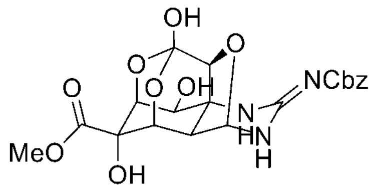
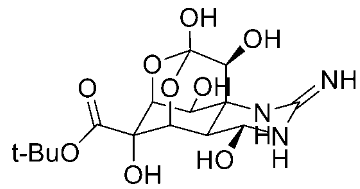

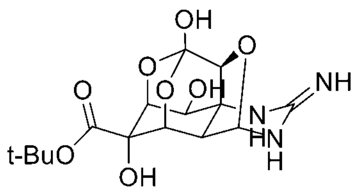
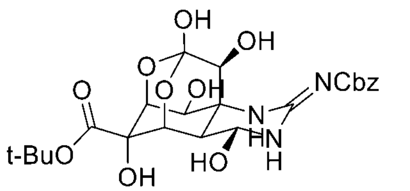
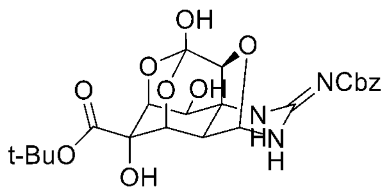

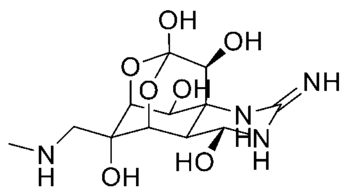
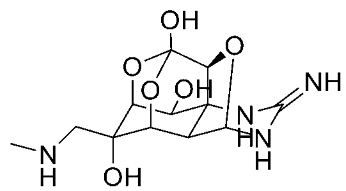
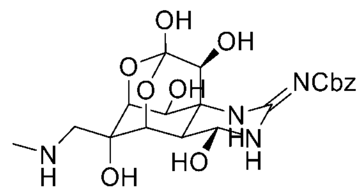

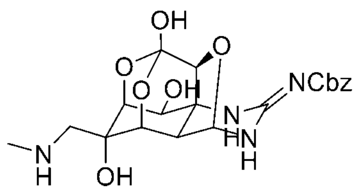
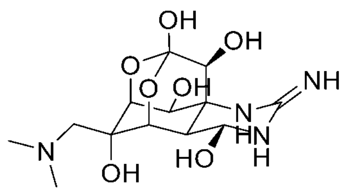
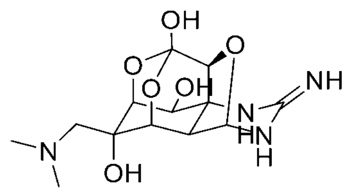

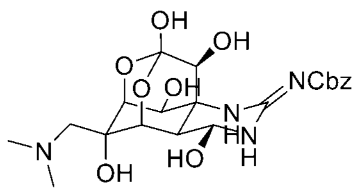
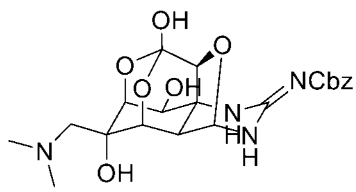
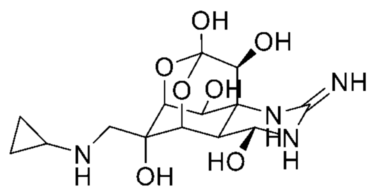

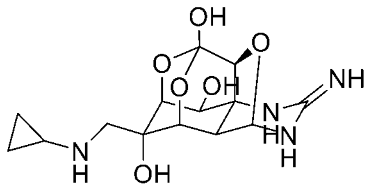
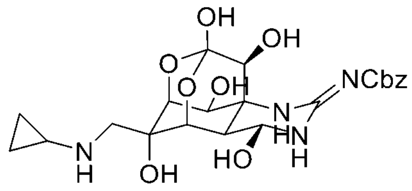
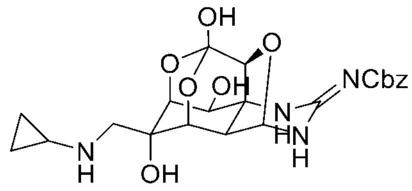

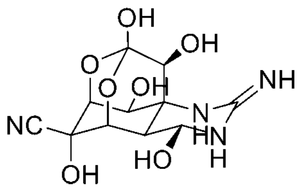
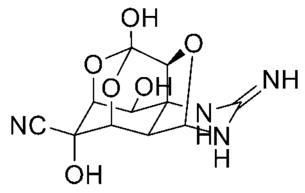
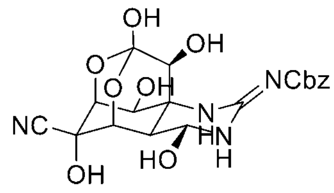

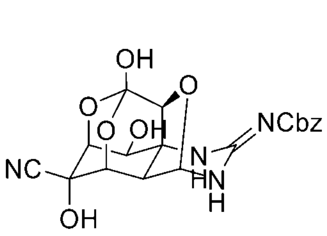
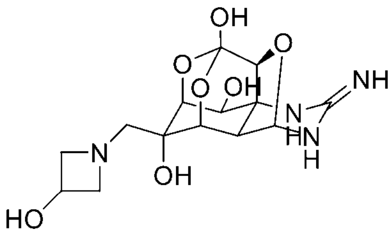
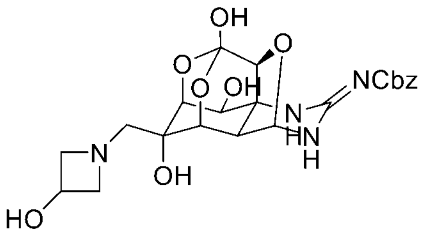

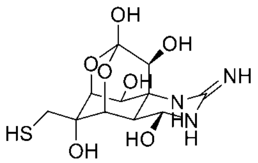
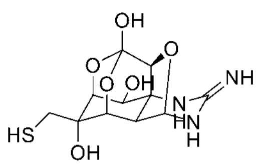
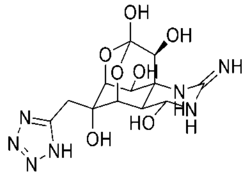
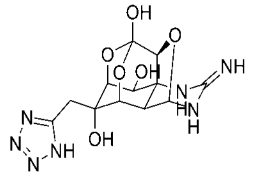

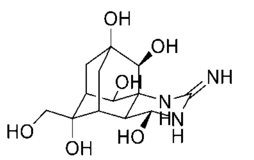
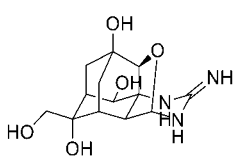
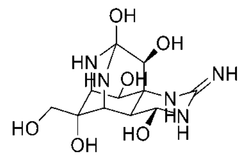
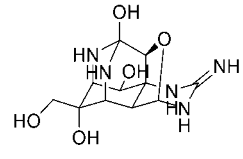

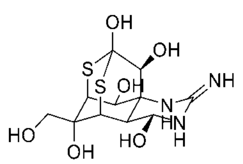
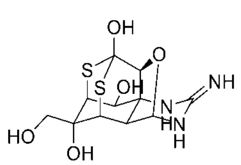
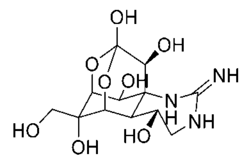
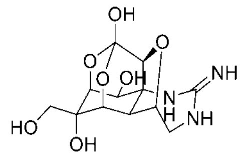

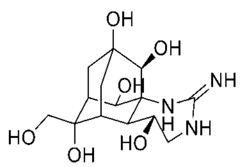
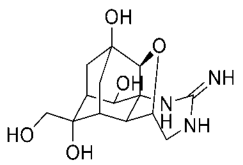
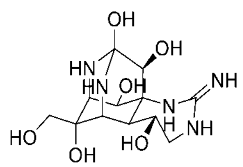
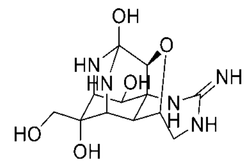

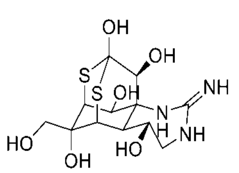
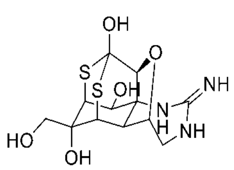
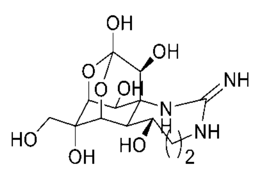
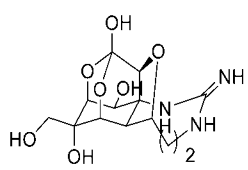

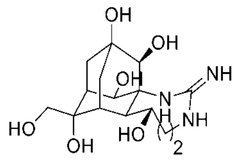 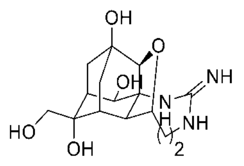 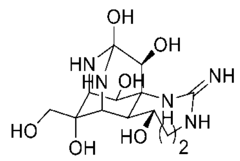 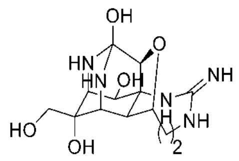

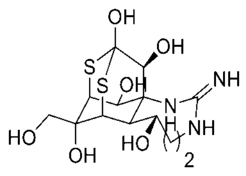 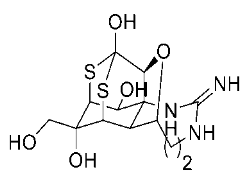 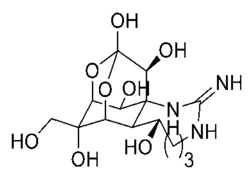 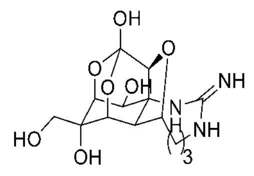

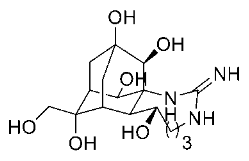 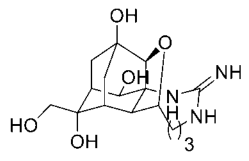 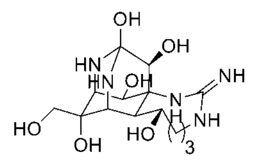 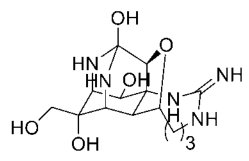

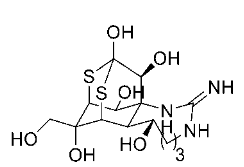 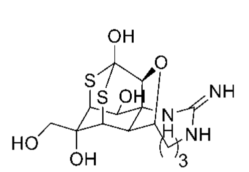 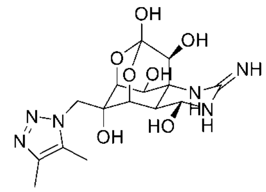 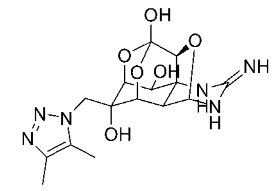

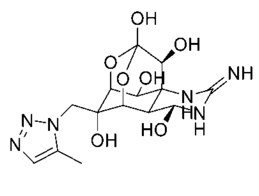 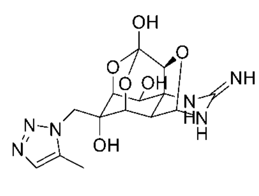 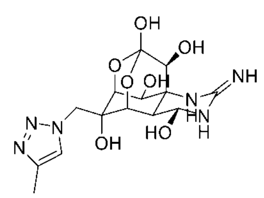 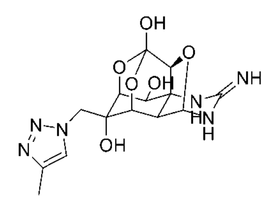

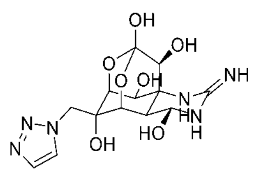 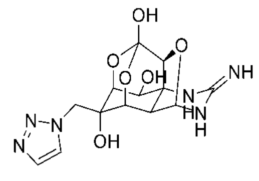 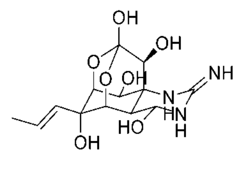 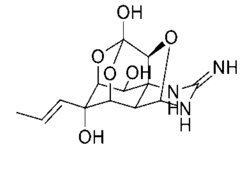

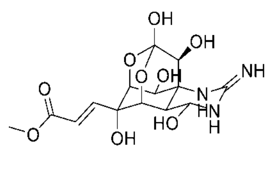 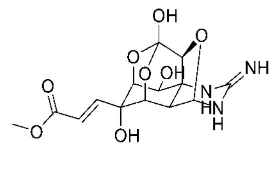 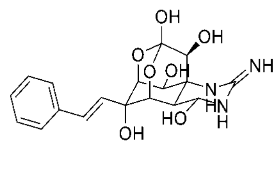 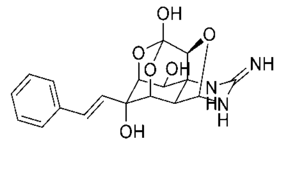

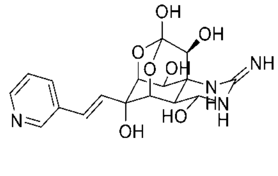 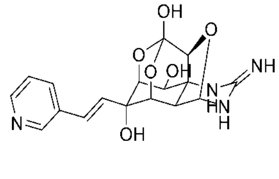 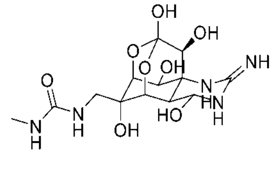 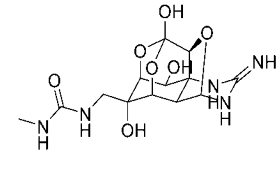

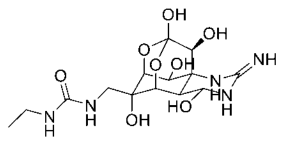 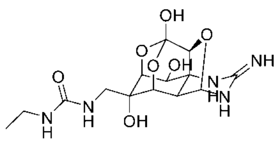 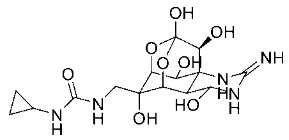 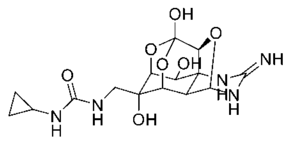

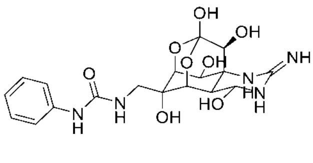 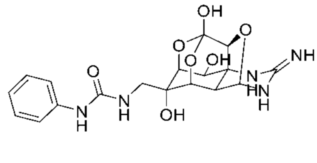 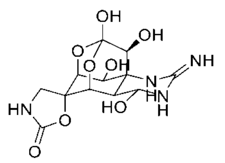 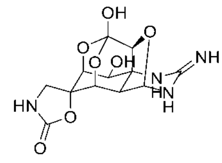

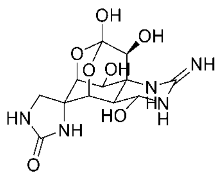 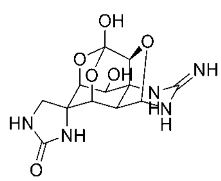 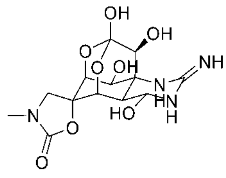 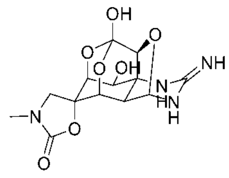

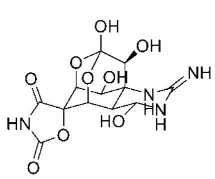 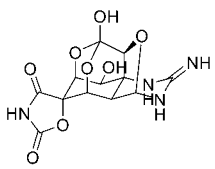 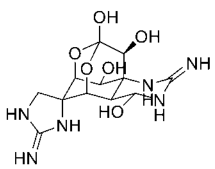 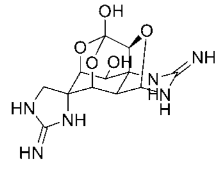

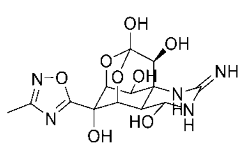 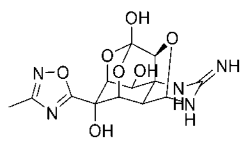 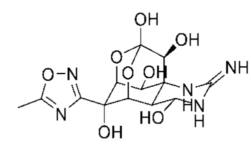 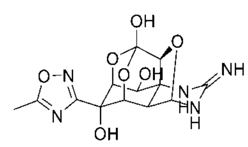

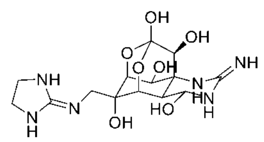 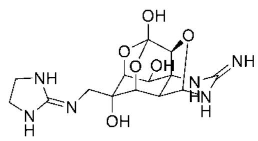 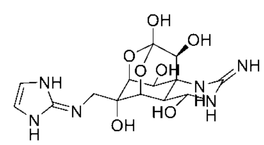 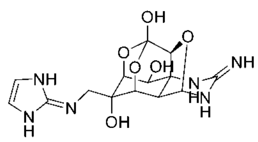

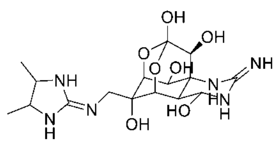 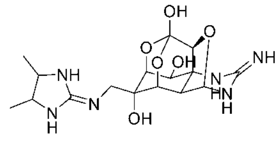 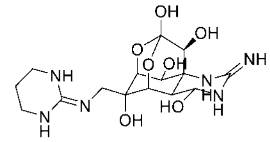 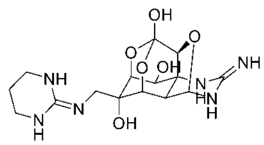

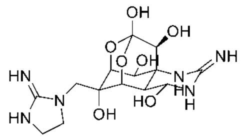 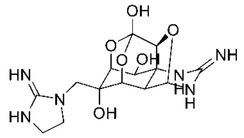 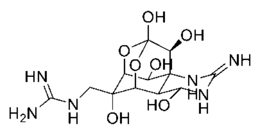 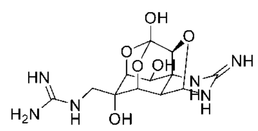

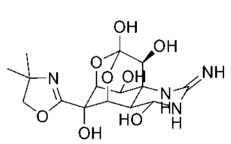 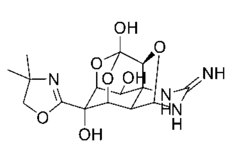 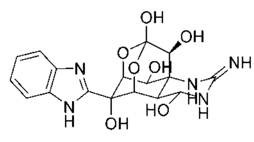 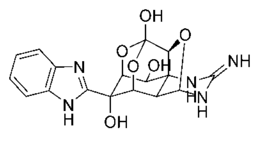

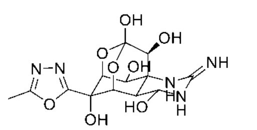
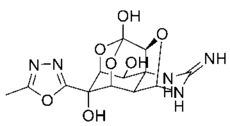
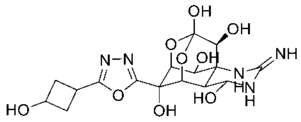
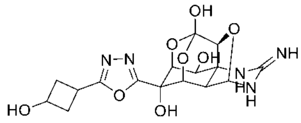

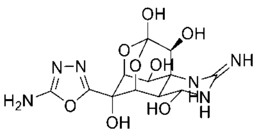
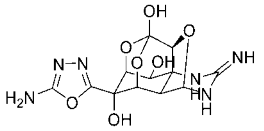
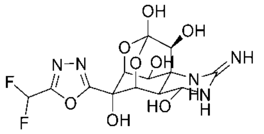
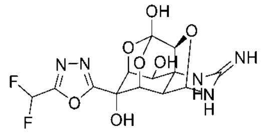

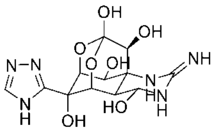
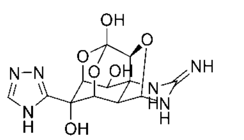
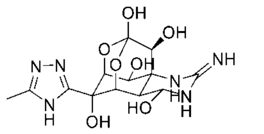
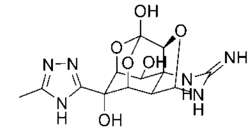

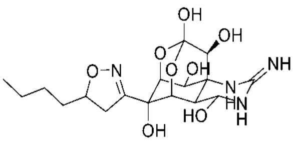
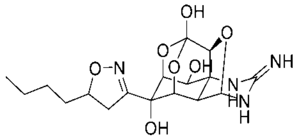
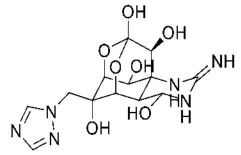
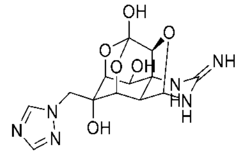

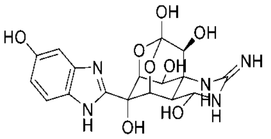
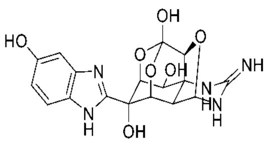
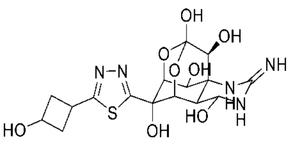
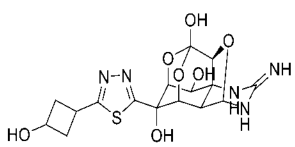

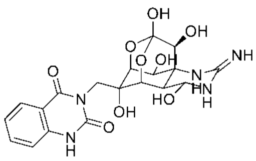
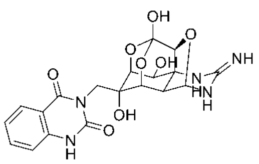
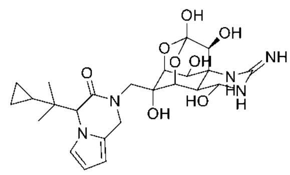
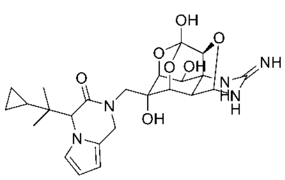

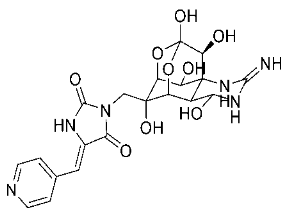
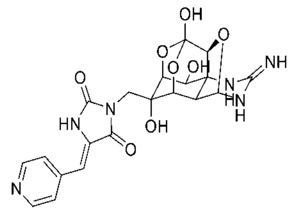
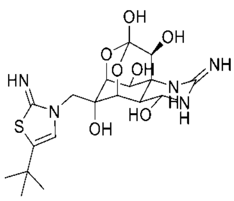
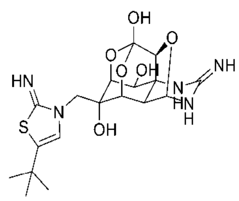

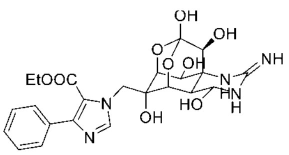
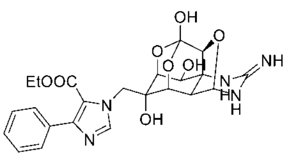
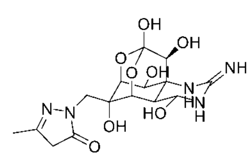
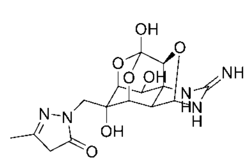

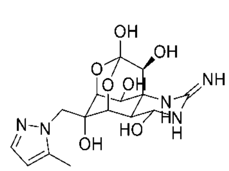
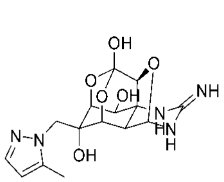
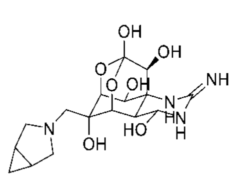
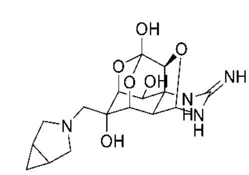

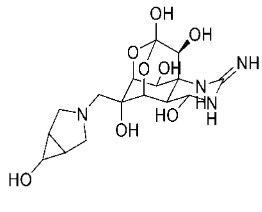 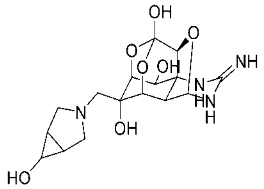 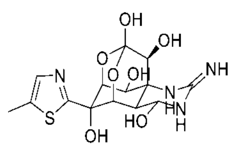 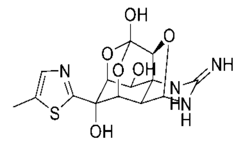

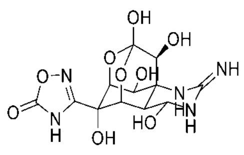 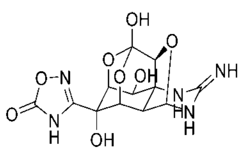 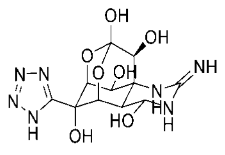 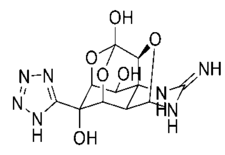

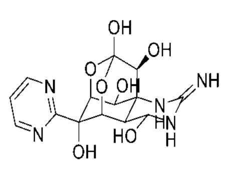 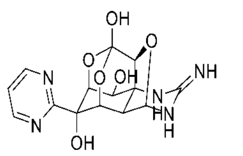 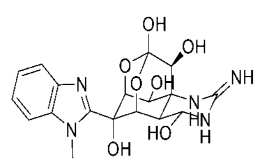 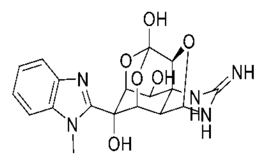

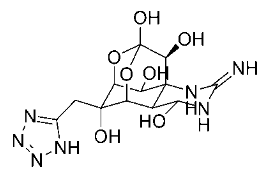 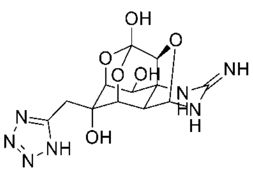 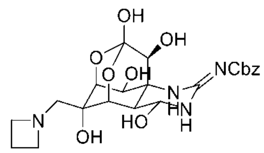 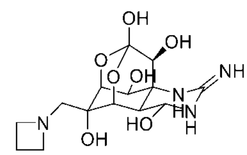

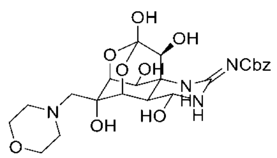 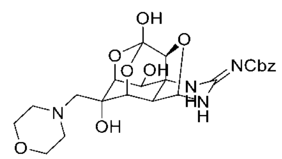 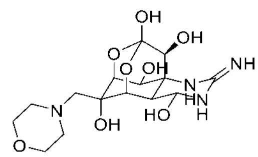 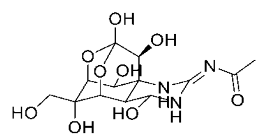

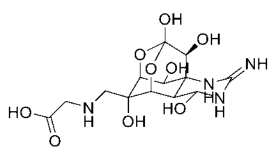 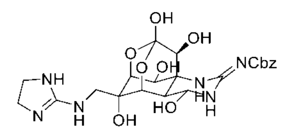 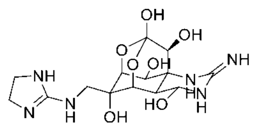 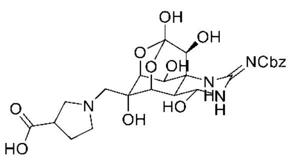

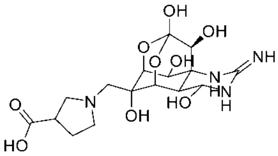 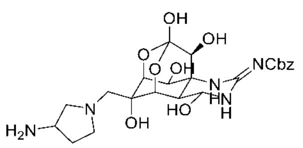 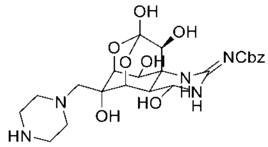 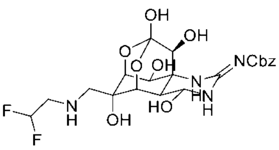

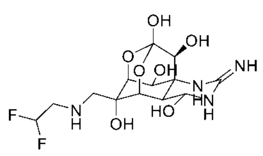 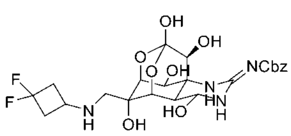 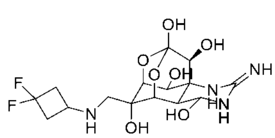 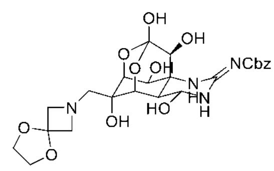

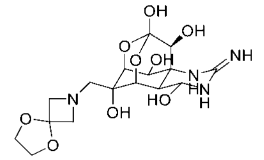
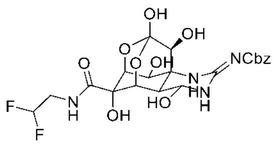
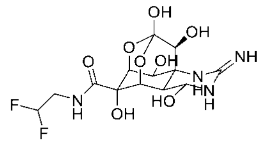
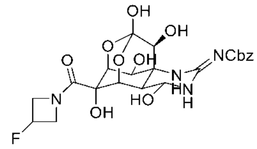
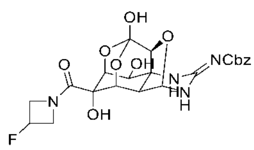
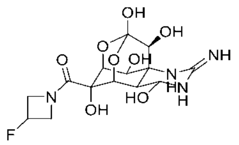
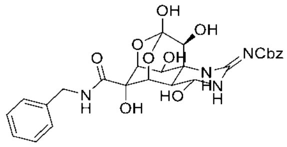
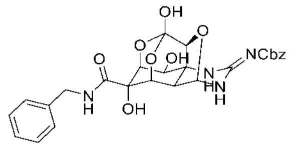
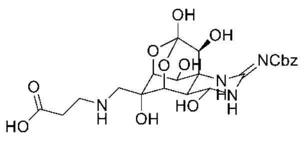
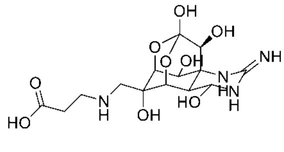
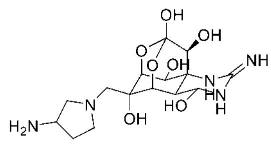
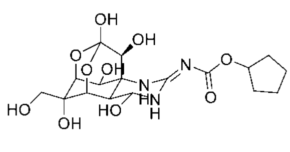
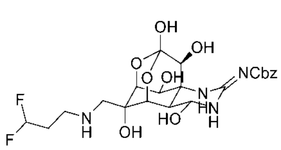
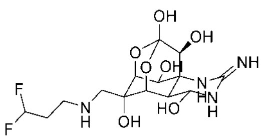
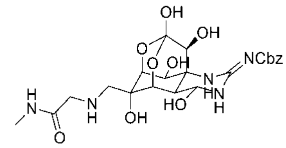
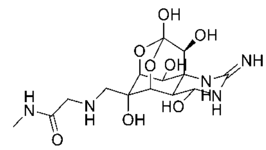
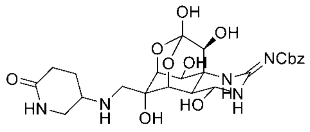
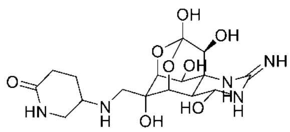
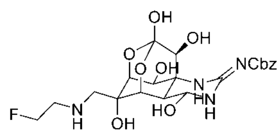
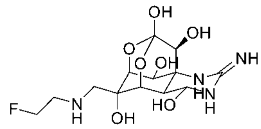
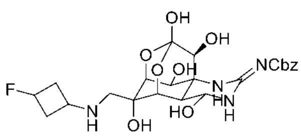
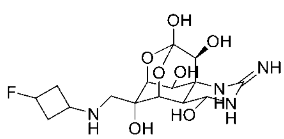
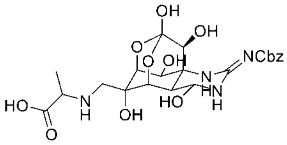
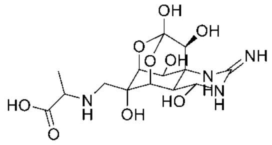
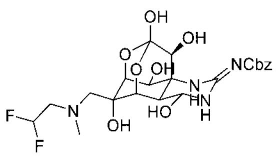
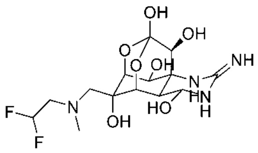
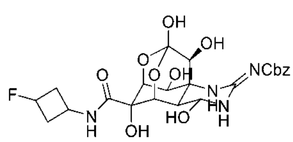
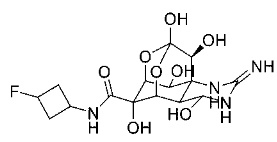
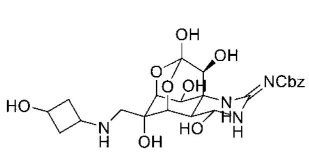
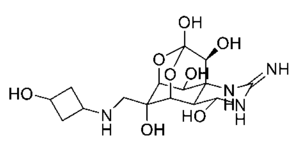
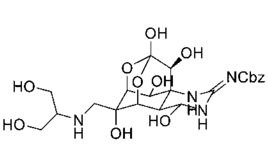
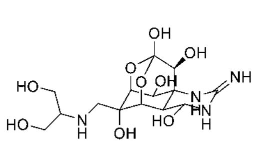
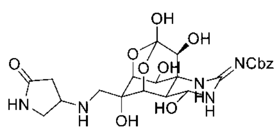
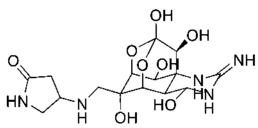
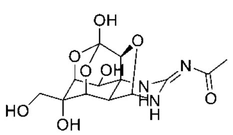
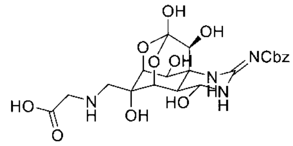

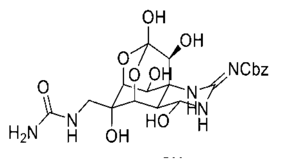 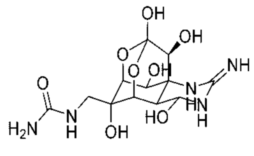 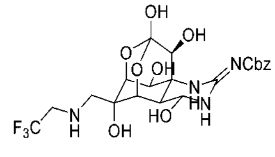 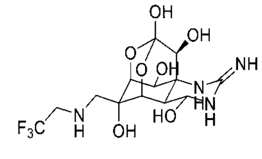

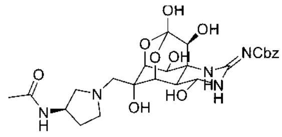 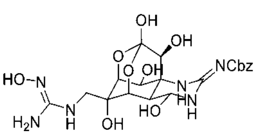 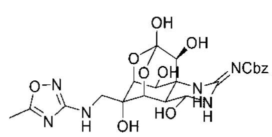

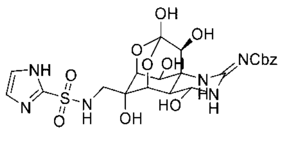 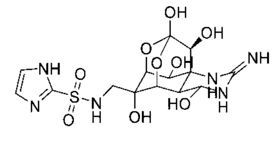 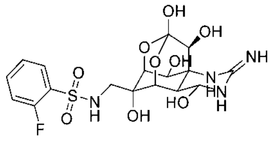 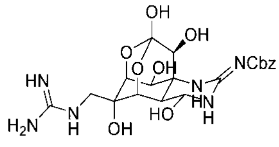

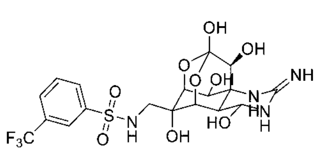 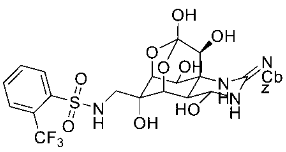 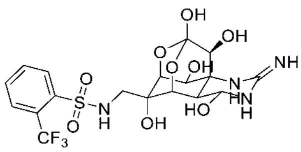

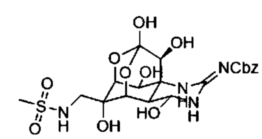 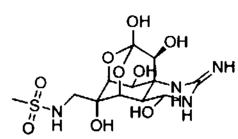 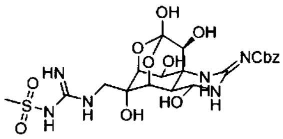 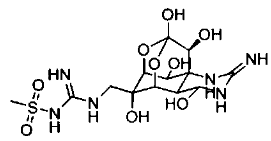

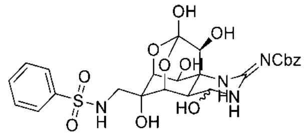 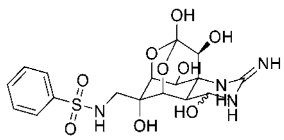 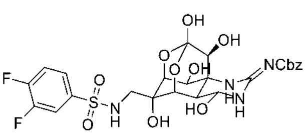 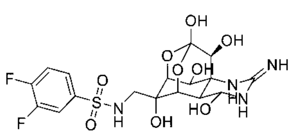

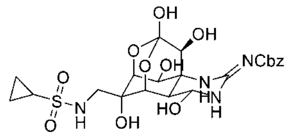 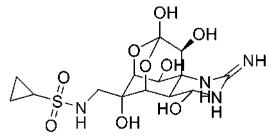 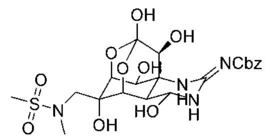 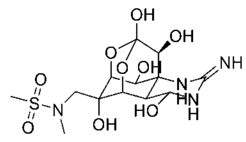

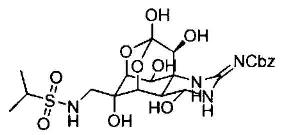 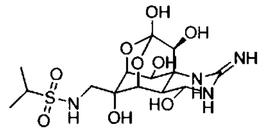 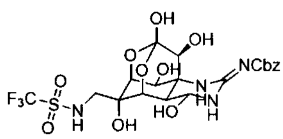 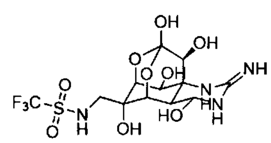

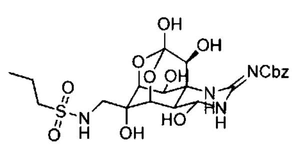 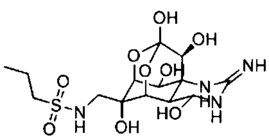 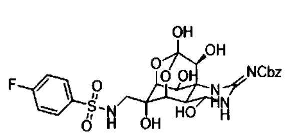 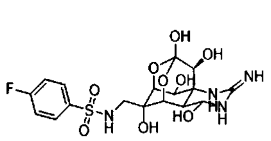

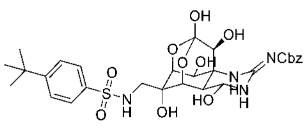 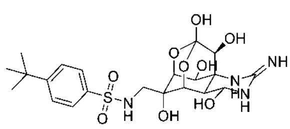 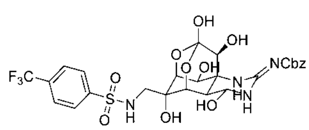 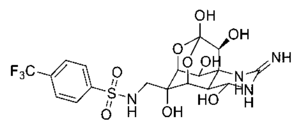

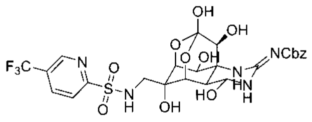 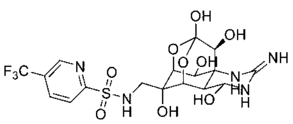 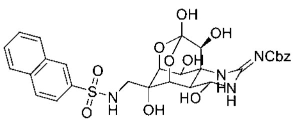 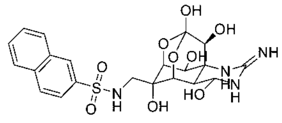

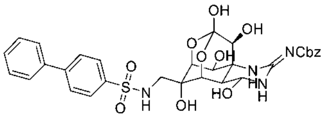 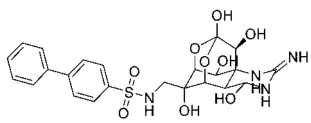 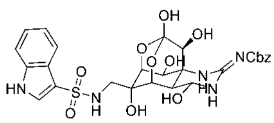 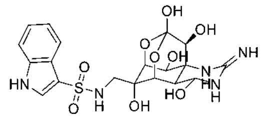

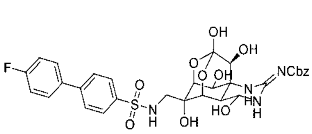 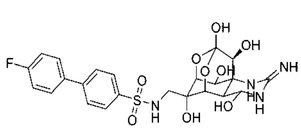 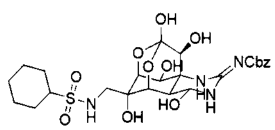 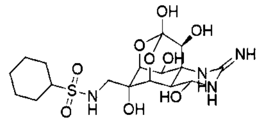

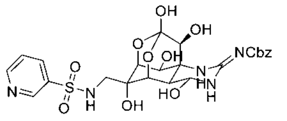 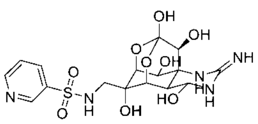 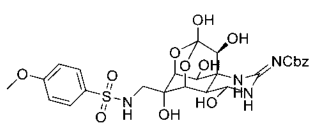 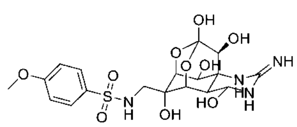

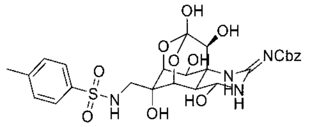 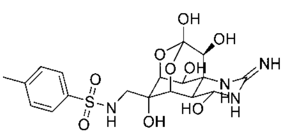 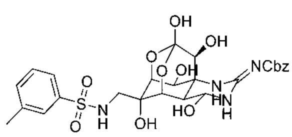 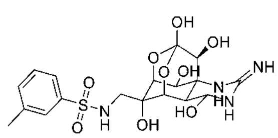

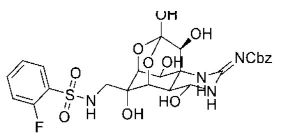 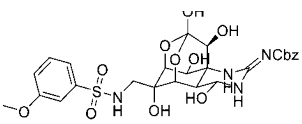 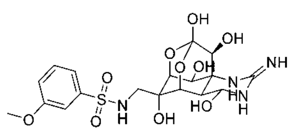 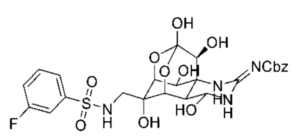

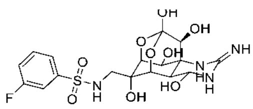 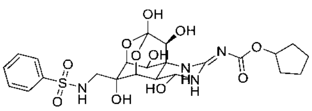 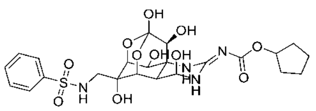 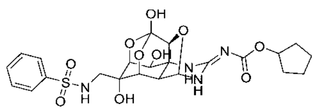

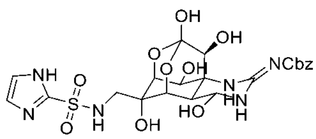 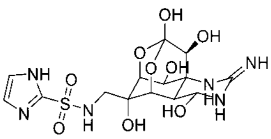 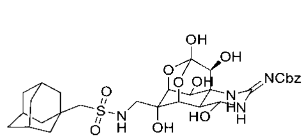 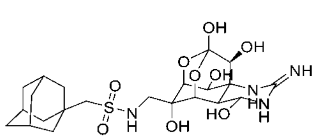

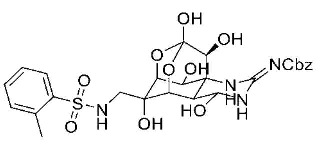 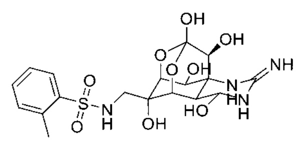 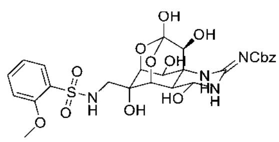 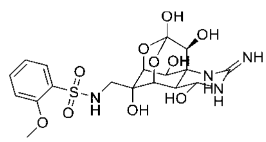

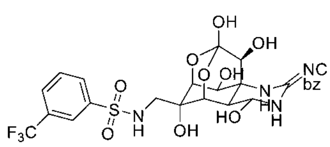 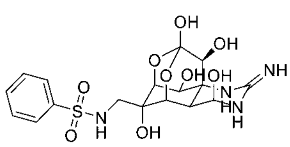 and 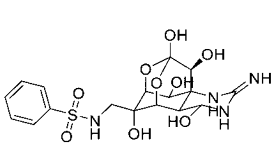 .

In some preferable embodiments, the compound of the present disclosure comprises the following structure:

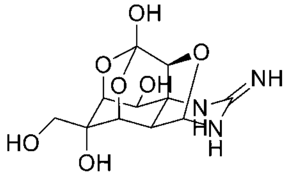
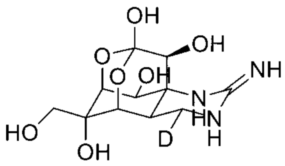
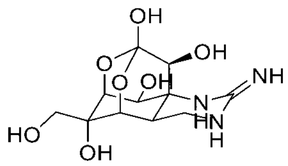
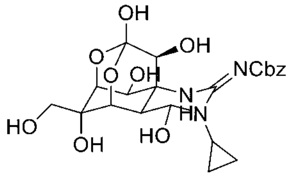

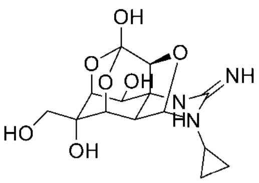
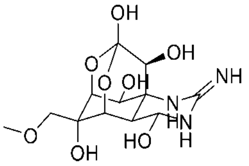
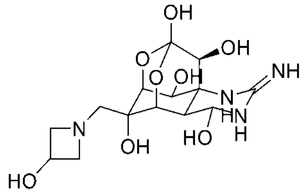
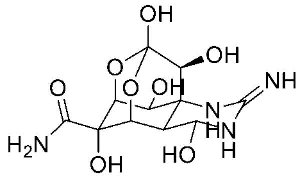

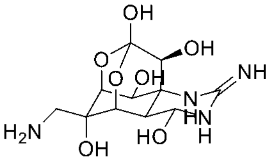
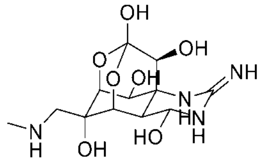
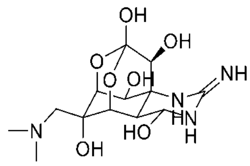
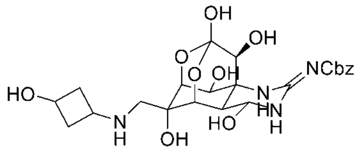

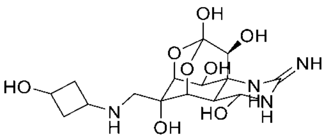
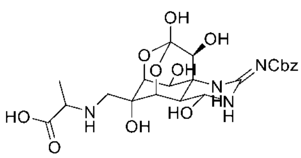
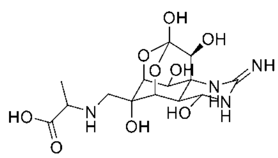
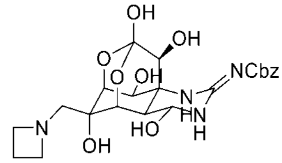

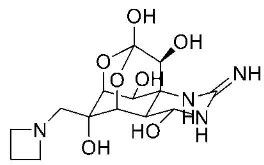
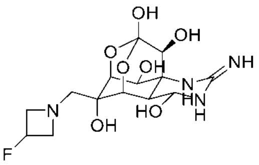
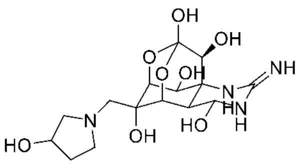
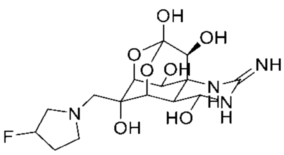

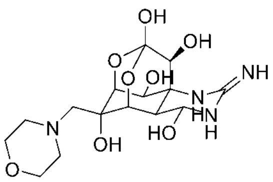
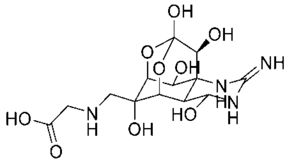
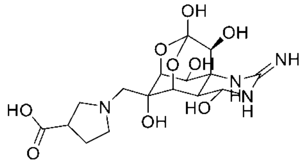
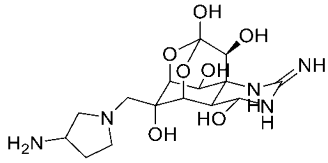

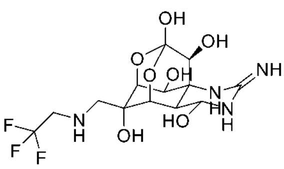
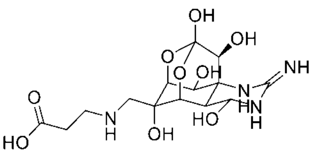
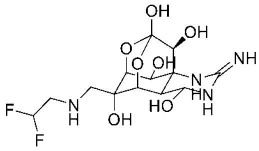
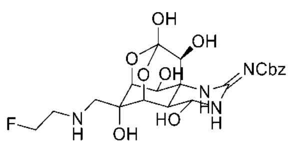

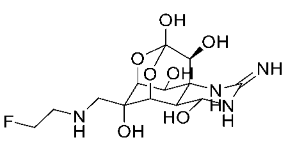
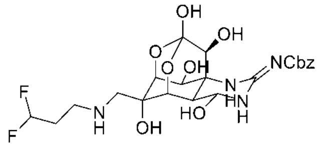
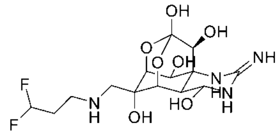
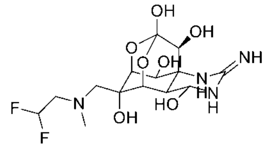

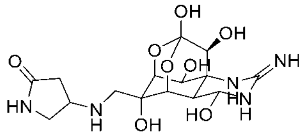 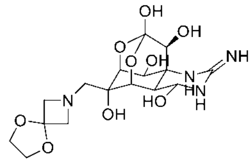 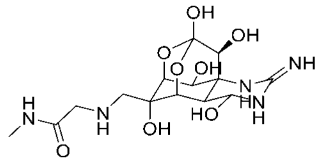 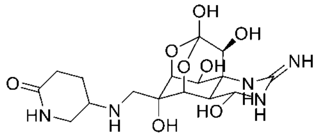

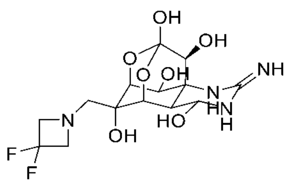 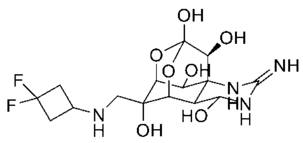 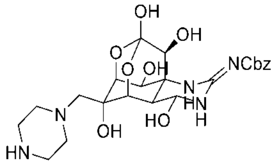 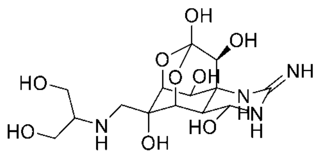

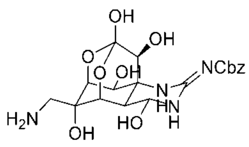 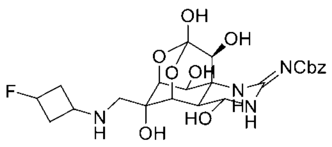 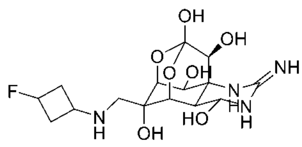 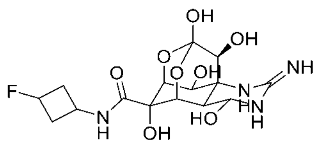

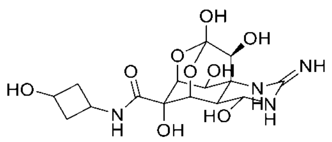 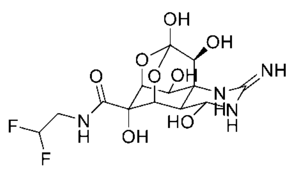 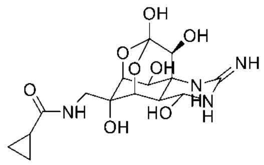 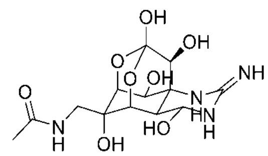

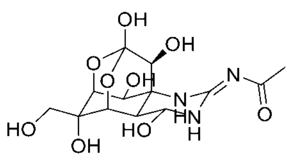 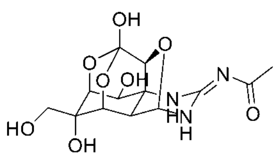 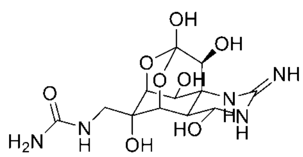 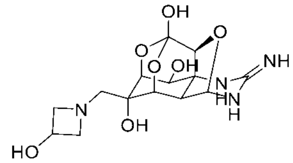

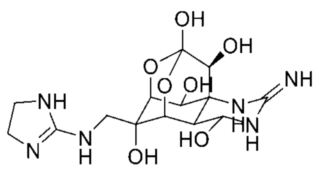 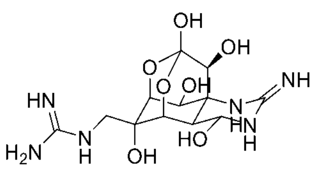 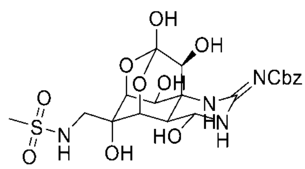 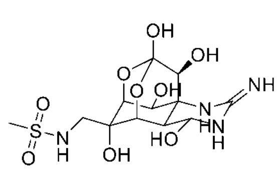

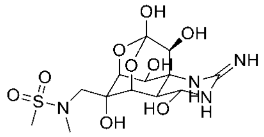 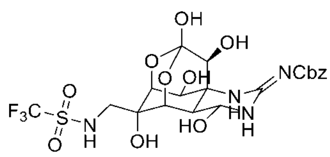 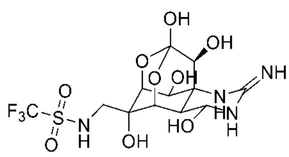 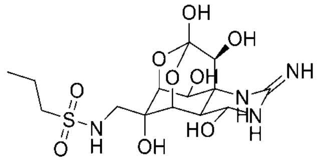

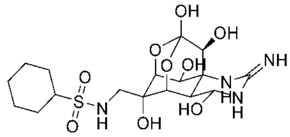 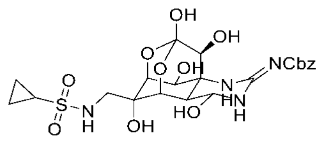 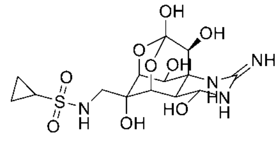 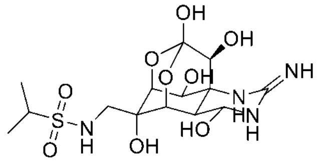

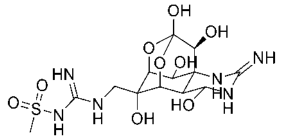 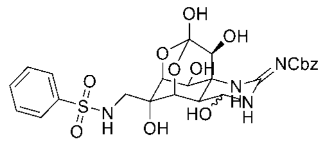 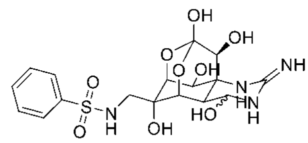 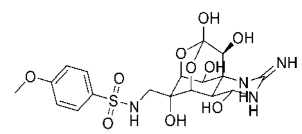

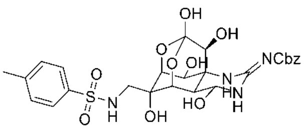 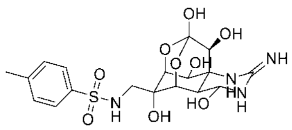 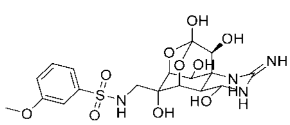 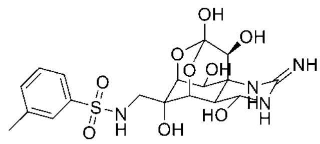

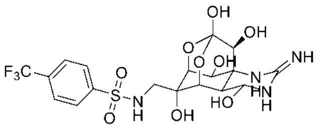 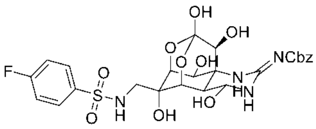 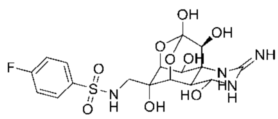 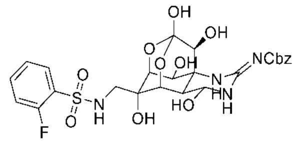

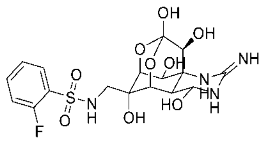 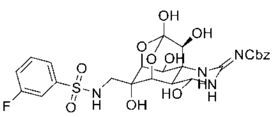 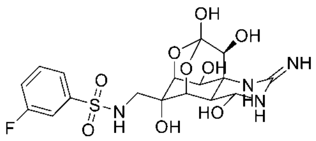 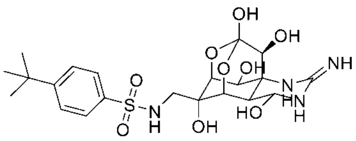

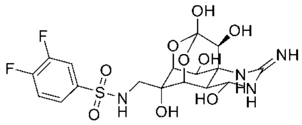 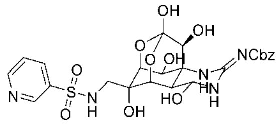 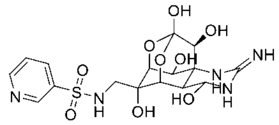 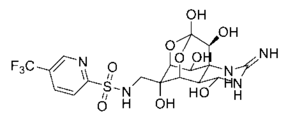

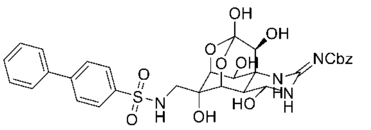 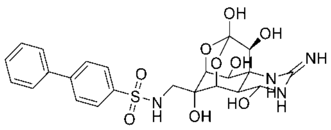 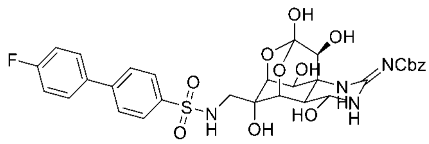

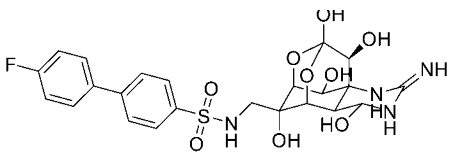 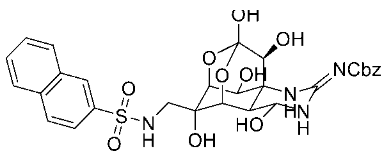 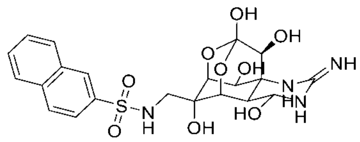

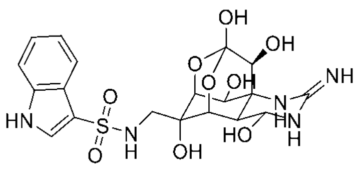 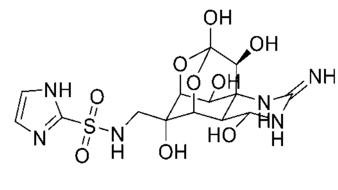 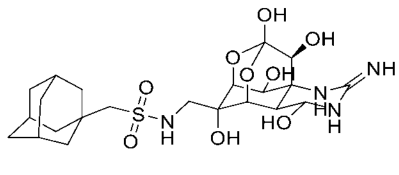

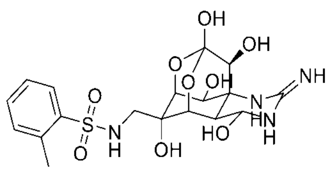 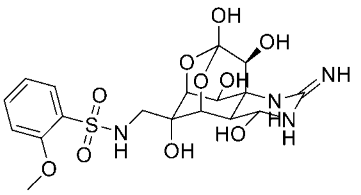 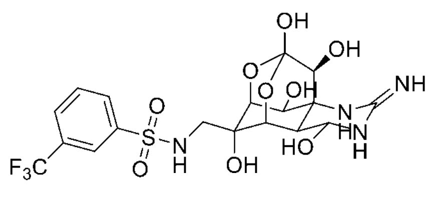

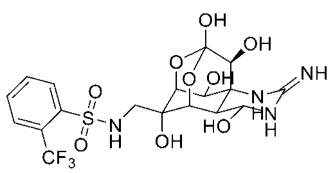 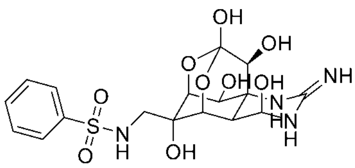 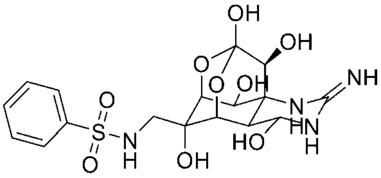

and

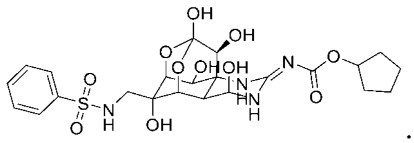

In some more preferable embodiments, the compound of the present disclosure comprises the following structure:

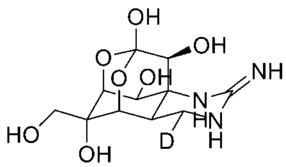
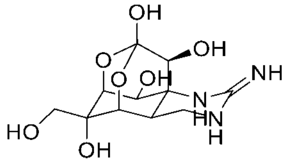
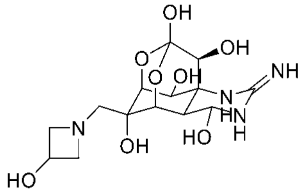
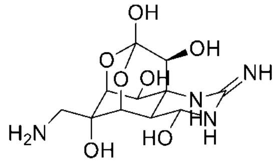

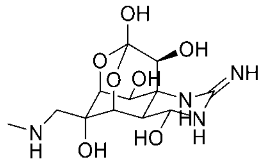
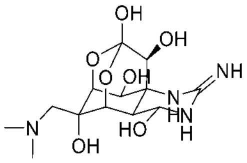
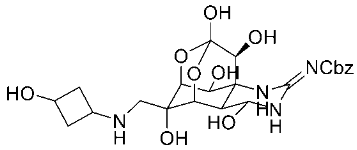
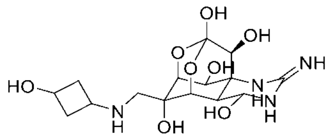

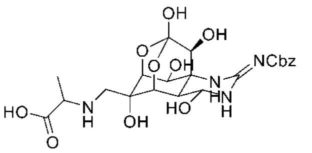
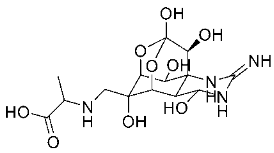
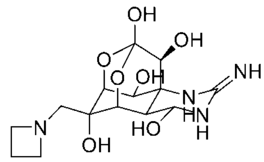
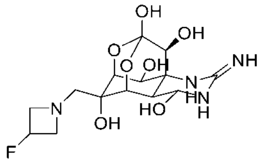

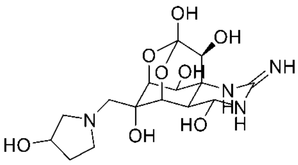
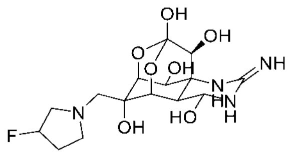
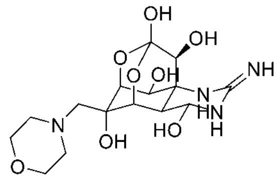
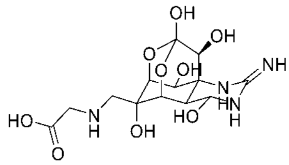

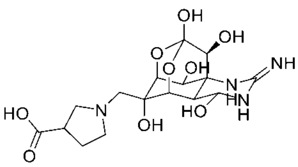
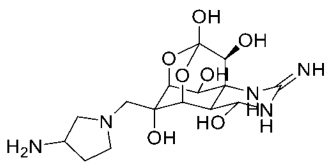
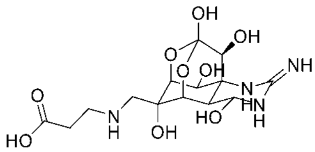
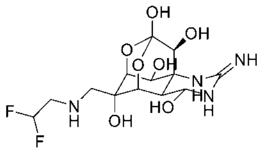

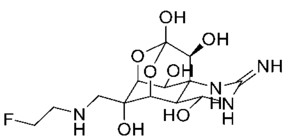
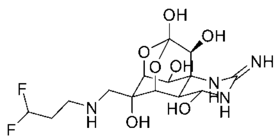
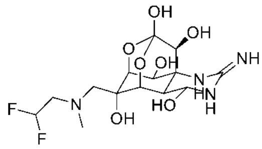
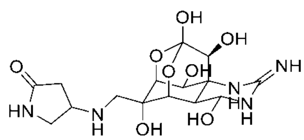

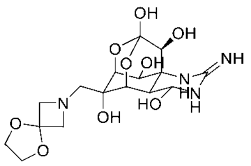
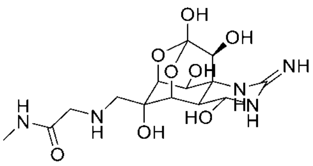
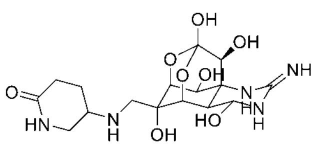
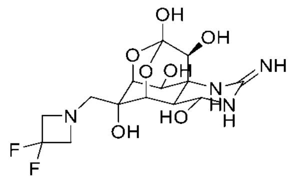

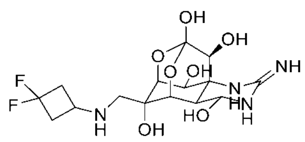
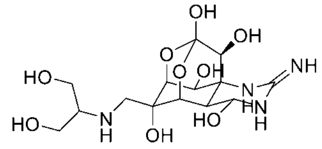
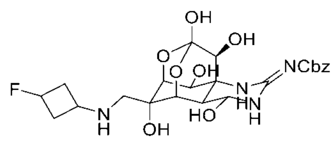

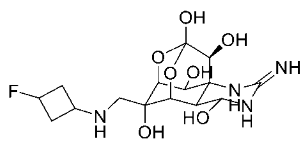
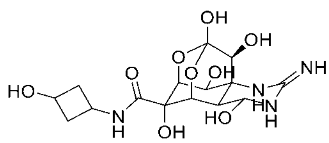
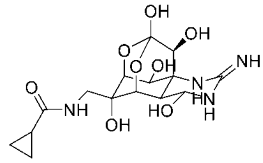
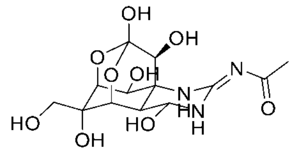

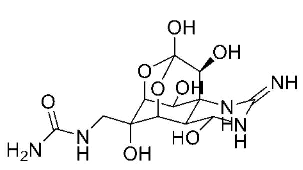
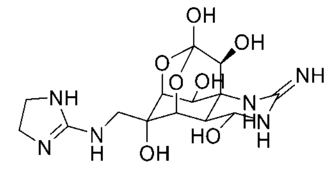
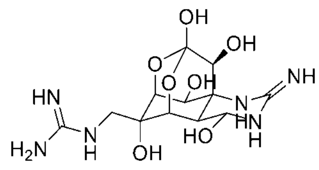

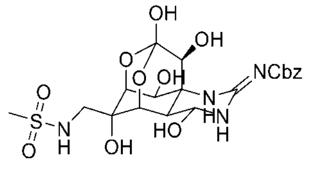
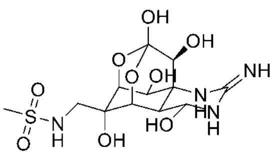
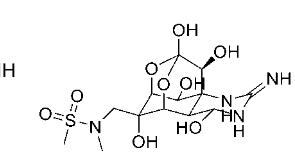
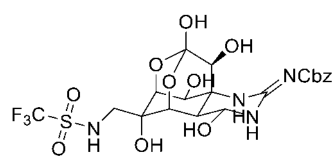

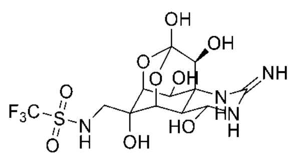
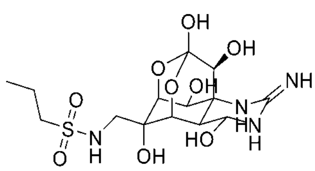
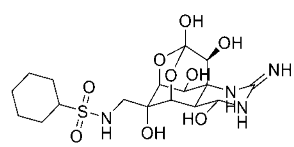
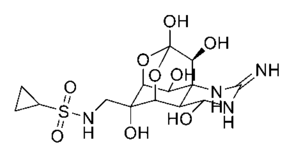

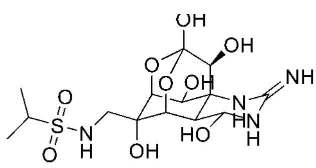
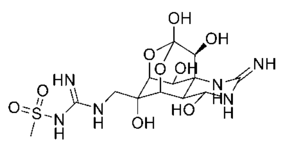
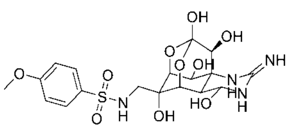
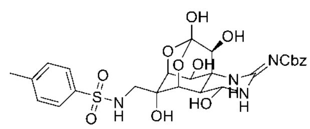

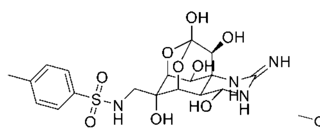
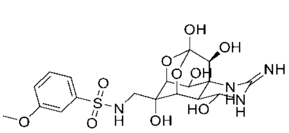
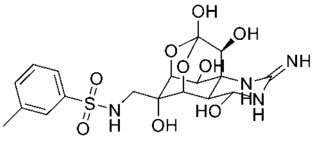
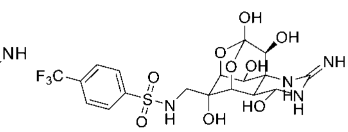

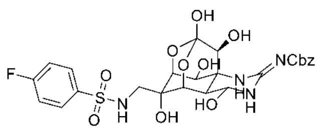
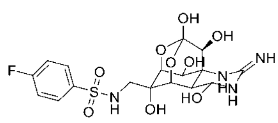
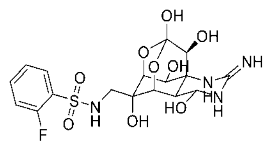
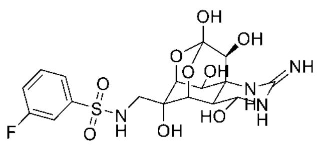

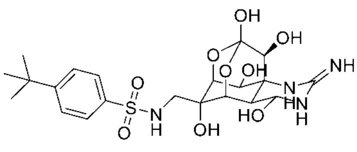
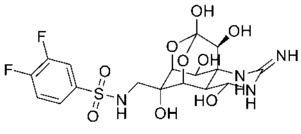
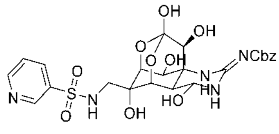
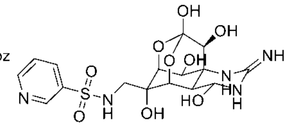

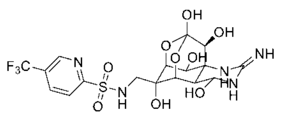

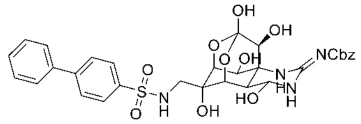

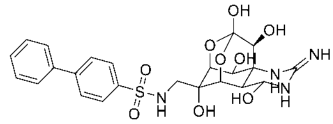

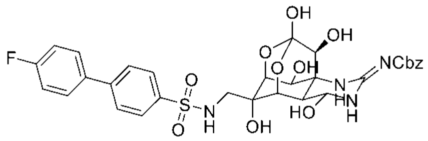

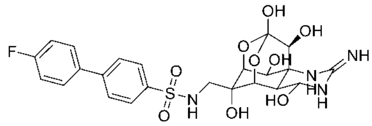

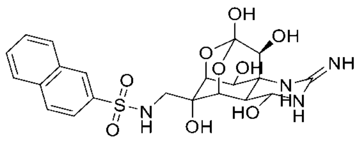

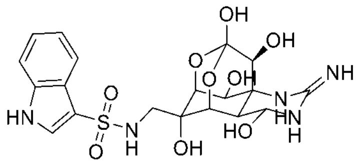

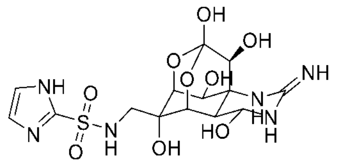

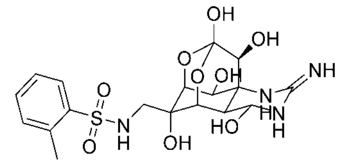

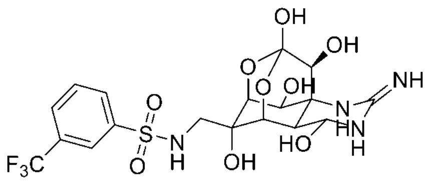

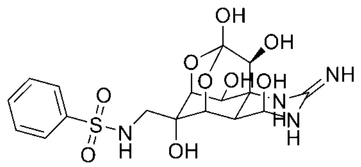

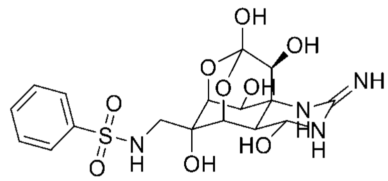

and

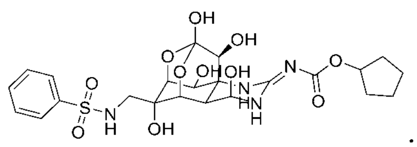

.

Pharmaceutical composition, formulation and methods of treatment of the present disclosure

In some embodiments, provided is a pharmaceutical composition comprising a therapeutically effective amount of a compound of formula (I) or a pharmaceutically acceptable derivative thereof and one or more pharmaceutically acceptable carriers.

In some embodiments, the pharmaceutical composition can be administered orally, intravenously, intraarterially, subcutaneously, intraperitoneally, intramuscularly, or transdermally.

The pharmaceutical compositions of the present disclosure may act systematically and/or locally. It may be administered in a suitable route for this purpose.

For these routes of administration, the pharmaceutical composition of the present disclosure can be administered in a suitable dosage form.

The dosing regimen may be adjusted to provide a best desired response. For example, a single bolus may be administered, several divided doses may be administered over time, or the dose may be proportionally decreased or increased as indicated by the acute need for treatment. It is noted that a dosage value may vary with the type and severity of condition to be alleviated, and may include single or multiple doses. It is further understood that for any particular individual, the specific dosing regimen should be adjusted over time according to the subject's needs and the professional judgment of the person administering the composition or supervising the administration of the composition.

The amount of a compound of the present disclosure administered will depend on the subject being treated, the severity of the disorder or condition, the rate of administration, the disposition of the compound, and the judgment of a prescribing physician.

In some embodiments, the pharmaceutical compositions of the present disclosure may further comprise one or more additional therapeutic agents (e.g., other drugs for use with sodium ion channels). In some embodiments, the treatment method of the present disclosure may further comprise administering one or more additional therapeutic agents (e.g., other drugs for use with sodium ion channels).

Process for synthesizing compound of formula (I)

Provided is a compound of formula (II) having the following structure:

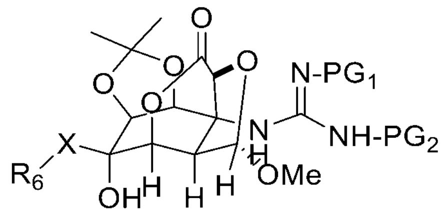

Formula (II)

wherein X and $R_6$ are as defined herein for formula (I), and $Pg_1$, $Pg_2$ are independently selected from the group consisting of acetyl, trityl, tert-butoxycarbonyl (Boc), benzyloxycarbonyl (Cbz), cyclopentyloxycarbonyl and fluorenylmethoxycarbonyl (Fmoc).

In some embodiments, X in the compound of Formula (II) is carbonyl. In some embodiments, $R_6$ in the compound of Formula (II) is hydroxyl. In other embodiments, $R_6$ in the compound of Formula (II) is hydrogen. In some embodiments, X in the compound of Formula (II) is methylene. In some embodiments, $R_6$ in the compound of Formula (II) is amino. In some embodiments, $Pg_1$ in the compound of Formula (II) is benzyloxycarbonyl (Cbz). In some embodiments, $Pg_2$ in the compound of Formula (II) is benzyloxycarbonyl (Cbz).

In some embodiments, the compound of Formula (II) is

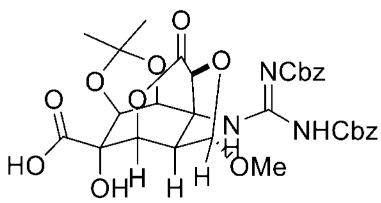

**B1**

.

In some embodiments, the compound of Formula (II) is

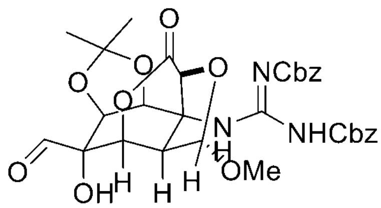

**B2**

.

In some embodiments, the compound of Formula (II) is

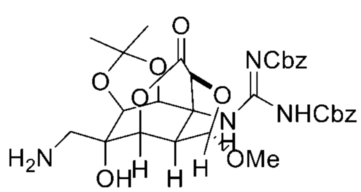

**B3**

.

In some embodiments, the compound of Formula (II) is used as an intermediate for preparing the compound of Formula (I) or a pharmaceutically acceptable derivative thereof. Therefore, provided also is a process for preparing the compound of formula (I) or a pharmaceutically acceptable derivative thereof, comprising using the compound of formula (II); or to use of the compound of formula (II) in the preparation of the compound of formula (I) or a pharmaceutically acceptable derivative thereof.

In some embodiments, the compound of formula (I) or a pharmaceutically acceptable derivative thereof can be synthesized by the following route:

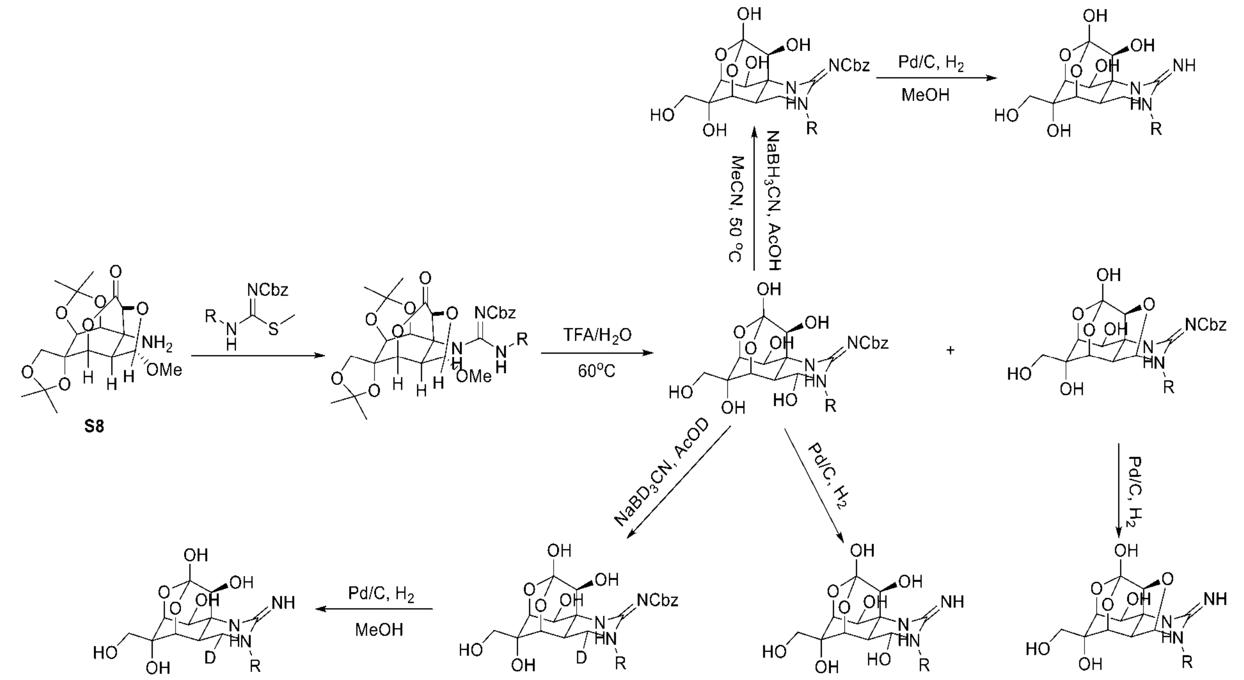


S8

wherein R represents any suitable group at the corresponding position herein.

In some specific embodiments, the synthesis of the compound of formula (I) or a pharmaceutically acceptable derivative thereof comprises the following intermediate synthesis steps:

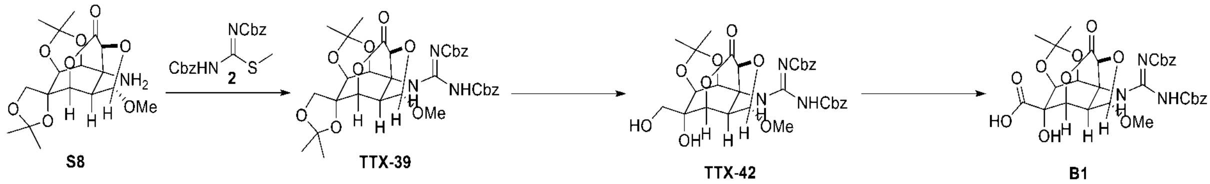

S8 TTX-39 TTX-42 B1

.

In other specific embodiments, the synthesis of the compound of formula (I) or a pharmaceutically acceptable derivative thereof comprises the following intermediate synthesis steps:

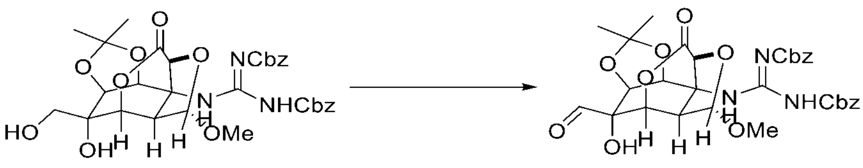

TTX-42 B2

.

In a specific embodiment, the synthesis of the compound of formula (I) or a pharmaceutically acceptable derivative thereof comprises the following intermediate synthesis steps:

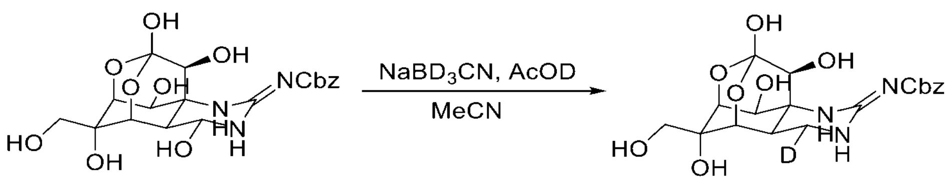


.

In some embodiments, the compound of formula (I) or a pharmaceutically acceptable derivative thereof can be synthesized by the following route:

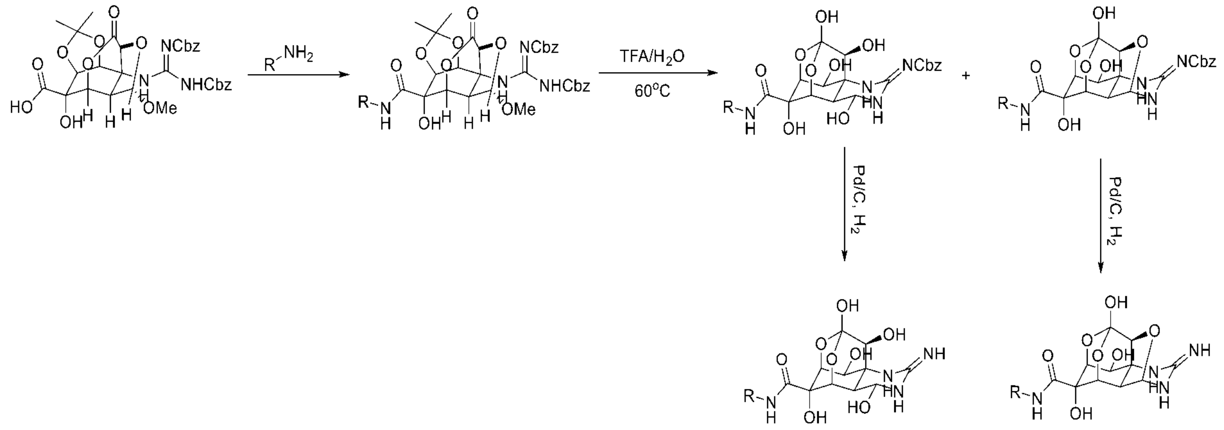


wherein R represents any suitable group at the corresponding position herein.

In other embodiments, the compound of formula (I) or a pharmaceutically acceptable derivative thereof can be synthesized by the following route:

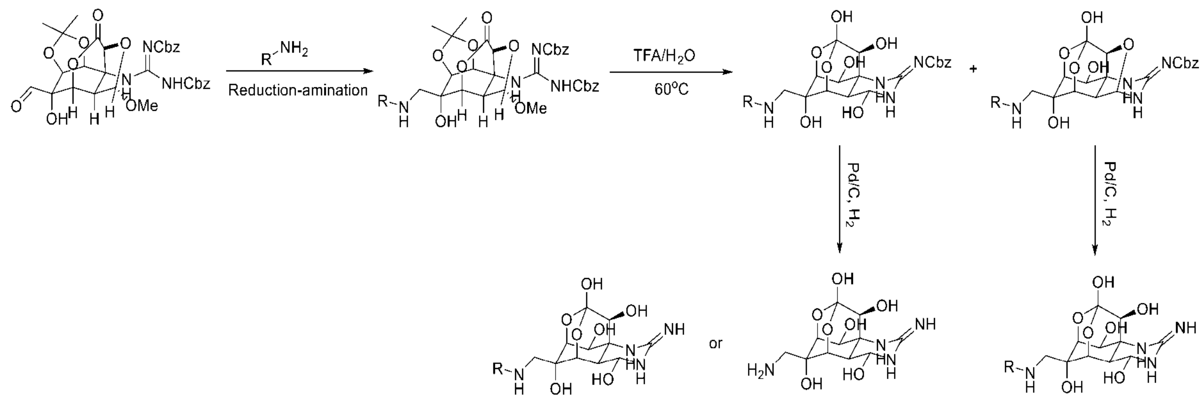


or

,

wherein R represents any suitable group at the corresponding position herein.

In yet other embodiments, the compound of formula (I) or a pharmaceutically acceptable derivative thereof can be synthesized by the following route:

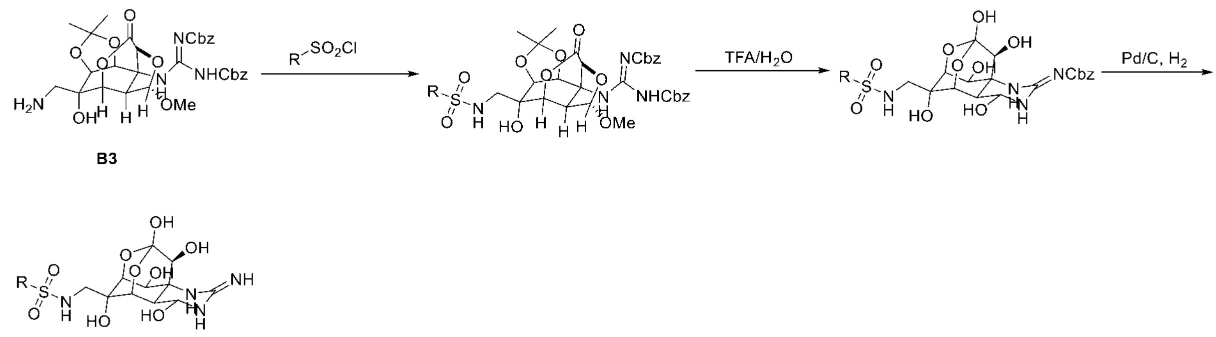


B3

wherein R represents any suitable group at the corresponding position herein.

Exemplary Embodiments

Embodiment 1: In an embodiment, provided is a compound having the structure of formula (I) or a pharmaceutically acceptable derivative thereof:

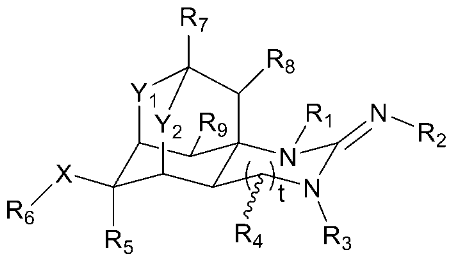

Formula (I)

wherein,

- X is absent or is $-(CH_2)_m-$, wherein $-CH_2-$ is optionally replaced by -O- or carbonyl, or optionally substituted with at least one $R^X$;
- $Y_1$ and $Y_2$ are each independently selected from the group consisting of methylene, O, S and NH, wherein the methylene and NH are optionally substituted with at least one $R^X$;
- $R_1$ and $R_3$ are each independently selected from the group consisting of hydrogen, hydroxyl, amino, cyano, $C_{1-8}$ alkyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl and $C_{1-8}$ alkyl-$C_{5-10}$ heteroaryl, wherein the alkyl, cycloalkyl, heterocyclyl, aryl and heteroaryl are optionally substituted with at least one $R^X$;
- $R_2$ is selected from the group consisting of hydrogen, hydroxyl, amino, $C_{1-8}$ alkyl, $C_{3-8}$ cycloalkyl, $C_{3-8}$ heterocyclyl, $-(C=O)OR_{10}$ and $-O(C=O)R_{11}$, wherein the alkyl, cycloalkyl and heterocyclyl are optionally substituted with at least one $R^X$;
- each $R_4$ is independently selected from the group consisting of hydrogen, deuterium, hydroxyl, halogen, $-OR_{12}$ and $C_{1-8}$ alkyl;
- or $R_3$ and $R_4$ together with the atom(s) to which they are attached form a 5-12 membered heterocycle, wherein the heterocycle is optionally substituted with at least one $R^X$;
- $R_5$ is selected from the group consisting of hydrogen, deuterium, tritium, hydroxyl, halogen, $C_{1-8}$ alkyl, $C_{3-8}$ cycloalkyl and $-OR_{13}$;
- $R_6$ is selected from the group consisting of hydroxy, mercapto, amino, azido, halogen, cyano, $C_{1-12}$ alkyl, $C_{2-8}$ alkenyl, $-O-C_{1-12}$ alkyl, $C_{3-8}$ cycloalkyl, $C_{3-8}$ heterocyclyl, $C_{6-10}$ aryl, $C_{5-10}$ heteroaryl, $-(C=O)-R_{14}$, guanidino, ureido and $-(OCH_2CH_2)_n-OH$, wherein the alkyl, amino, cycloalkyl, heterocyclyl, aryl, heteroaryl, guanidino and ureido are optionally substituted with at least one $R^X$;
- or $R_5$ and $R_6$ together with the atom(s) to which they are attached form a 3-12 membered heterocycle, wherein the heterocycle is optionally substituted with at least one $R^X$;
- $R_7$ is selected from the group consisting of hydrogen, deuterium, tritium, hydroxyl, halogen, $C_{1-4}$ alkyl, $-O-C_{1-4}$ alkyl, $C_{3-8}$ cycloalkyl and $C_{3-8}$ heterocyclyl, wherein the alkyl, cycloalkyl and heterocyclyl are optionally substituted with at least one $R^X$;
- $R_8$ is hydroxyl;
- or $R_4$ and $R_8$ together with the atom(s) to which they are attached form a 5-12 membered heterocycle, wherein the heterocycle is optionally substituted with at least one $R^X$;
- $R_9$ is selected from the group consisting of hydrogen, deuterium, tritium, hydroxyl, $C_{1-8}$ alkyl, $C_{3-8}$ cycloalkyl and $-OR_{15}$;
- $R_{10}$ and $R_{11}$ are each independently selected from the group consisting of $C_{1-8}$ alkyl, $C_{3-8}$ cycloalkyl, $C_{3-8}$ heterocyclyl, $C_{6-10}$ aryl, $C_{1-8}$ alkyl-$C_{6-10}$ aryl, $C_{5-10}$ heteroaryl and $C_{1-8}$ alkyl-$C_{5-10}$ heteroaryl, wherein the alkyl, cycloalkyl, heterocyclyl, aryl and heteroaryl are optionally substituted with at least one $R^X$;
- $R_{12}$, $R_{13}$, $R_{14}$ and $R_{15}$ are each independently selected from the group consisting of $C_{1-8}$ alkyl, $C_{2-8}$ alkenyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl, and $C_{1-8}$ alkyl-$C_{5-10}$ heteroaryl, wherein the alkyl, cycloalkyl, heterocyclyl, aryl, and heteroaryl are optionally substituted with at least one $R^X$;
- each $R^X$ is independently selected from the group consisting of hydrogen, halogen, hydroxyl, carboxyl, amino, cyano, formyl, $C_{1-8}$ alkyl, $C_{2-8}$ alkenyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl, and $C_{1-8}$ alkyl-$C_{5-10}$ heteroaryl, wherein the amino, formyl, alkyl, cycloalkyl, heterocyclyl, aryl, and heteroaryl are optionally substituted with at least one $R^Y$;
- each $R^Y$ is independently selected from the group consisting of hydrogen, halogen, hydroxyl, $C_{1-8}$ alkyl, $-O-C_{1-8}$ alkyl, $C_{3-8}$ cycloalkyl, $-O-C_{3-8}$ cycloalkyl, $C_{3-8}$ heterocyclyl, $-O-C_{3-8}$ heterocyclyl, $C_{1-8}$ alkyl-$C_{3-8}$ heterocyclyl, $C_{6-10}$ aryl, $-O-C_{6-10}$ aryl, $C_{1-8}$ alkyl-$C_{6-10}$ aryl, $C_{5-10}$ heteroaryl, $-O-C_{5-10}$ heteroaryl, and $C_{1-8}$ alkyl-$C_{5-10}$ heteroaryl, wherein the alkyl, cycloalkyl, heterocyclyl, aryl, and heteroaryl are optionally substituted with at least one halogen or hydroxyl;

when X is methylene and $R_1$, $R_2$ and $R_3$ are hydrogen at the same time, $R_4$, $R_5$, $R_6$, $R_7$, $R_8$ and $R_9$ are not hydroxyl at the same time;
m is an integer selected from 0-6;
n is an integer selected from 1-12;
t is an integer selected from 0-4.

Embodiment 2: In an embodiment, in the compound of Embodiment 1 or a pharmaceutically acceptable derivative thereof, X is selected from the group consisting of methylene and carbonyl.
Embodiment 3: In an embodiment, in the compound of Embodiment 1 or 2 or a pharmaceutically acceptable derivative thereof, $R_1$ is hydrogen.
Embodiment 4: In an embodiment, in the compound of any one of Embodiments 1-3 or a pharmaceutically acceptable derivative thereof, $R_2$ is selected from the group consisting of hydrogen and $-(C{=}O)OR_{10}$.
Embodiment 5: In an embodiment, in the compound of Embodiment 4 or a pharmaceutically acceptable derivative thereof, $R_{10}$ is benzyl.
Embodiment 6: In an embodiment, in the compound of any one of Embodiments 1-5 or a pharmaceutically acceptable derivative thereof, $R_3$ is selected from the group consisting of hydrogen, $C_{1-8}$ alkyl, $C_{3-8}$ cycloalkyl, $C_{1-8}$ alkyl-$C_{3-8}$ cycloalkyl and $C_{1-8}$ alkyl-$C_{6-10}$ aryl, wherein the alkyl, cycloalkyl and aryl are optionally substituted with at least one $R^X$.
Embodiment 7: In an embodiment, in the compound of Embodiment 6 or a pharmaceutically acceptable derivative thereof, $R^X$ is selected from the group consisting of hydroxyl and carboxyl.
Embodiment 8: In an embodiment, in the compound of Embodiment 6 or a pharmaceutically acceptable derivative thereof, $R_3$ is selected from the group consisting of hydrogen, methyl, ethyl, isopropyl,

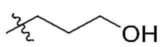, 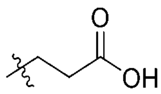, 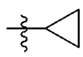, 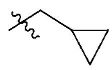, 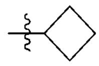,

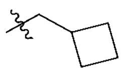 and 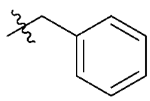.

Embodiment 9: In an embodiment, in the compound of any one of Embodiments 1-8 or a pharmaceutically acceptable derivative thereof, $R_4$ is selected from the group consisting of hydrogen, deuterium and hydroxyl.
Embodiment 10: In an embodiment, in the compound of any one of Embodiments 1-9 or a pharmaceutically acceptable derivative thereof, $R_3$ and $R_4$ together with the atom(s) to which they are attached form a 6-membered heterocycle.
Embodiment 11: In an embodiment, in the compound of Embodiment 10 or a pharmaceutically acceptable derivative thereof, the heterocycle formed by $R_3$ and $R_4$ together with the atom(s) to which they are attached is selected from the group consisting of

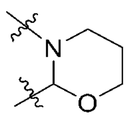

and

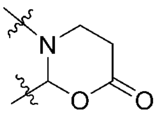.

Embodiment 12: In an embodiment, in the compound of any one of Embodiments 1-11 or a pharmaceutically acceptable derivative thereof, $R_5$ is hydroxyl.
Embodiment 13: In an embodiment, in the compound of any one of Embodiments 1-12 or a pharmaceutically acceptable derivative thereof, $R_6$ is selected from the group consisting of hydroxyl, amino, $-O-C_{1-12}$ alkyl and $C_{3-8}$ heterocyclyl, wherein the amino, alkyl and heterocyclyl are optionally substituted with at least one $R^X$.
Embodiment 14: In an embodiment, in the compound of Embodiment 13 or a pharmaceutically acceptable

derivative thereof, $R^X$ is selected from the group consisting of hydroxyl, $-C_{1-8}$ alkyl, and $C_{1-8}$ alkyl-$C_{6-10}$ aryl.

Embodiment 15: In an embodiment, in the compound of Embodiment 13 or a pharmaceutically acceptable derivative thereof, $R_6$ is selected from the group consisting of hydroxyl, amino, methoxy, methylamino,

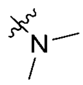 , 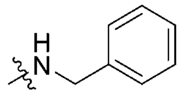 , 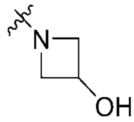 , 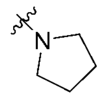

and

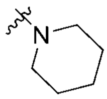 .

Embodiment 16: In an embodiment, in the compound of any one of Embodiments 1-15 or a pharmaceutically acceptable derivative thereof, $R_7$ is hydroxyl.

Embodiment 17: In an embodiment, in the compound of any one of Embodiments 1-16 or a pharmaceutically acceptable derivative thereof, $R_8$ is hydroxyl.

Embodiment 18: In an embodiment, in the compound of any one of Embodiments 1-17 or a pharmaceutically acceptable derivative thereof, $R_4$ and $R_8$ together with the atom(s) to which they are attached form a heterocycle, which has the following structure:

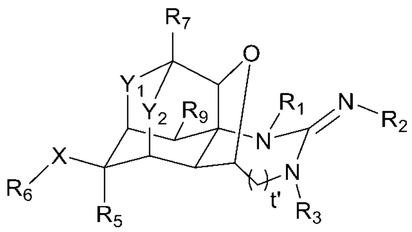 ,

wherein X, $Y_1$, $Y_2$, $R_1$, $R_2$, $R_3$, $R_5$, $R_6$, $R_7$ and $R_9$ are as defined in any one of Embodiments 1-17, and t' is an integer selected from 0-3.

Embodiment 19: In an embodiment, in the compound of any one of Embodiments 1-18 or a pharmaceutically acceptable derivative thereof, $R_4$ and $R_8$ together with the atom(s) to which they are attached form a heterocycle, which has the following structure:

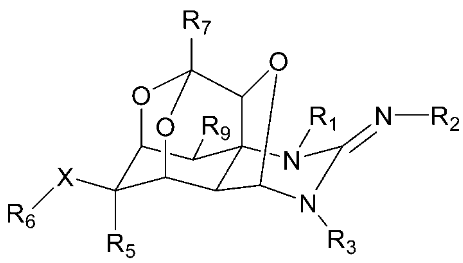 ,

wherein X, $R_1$, $R_2$, $R_3$, $R_5$, $R_6$, $R_7$ and $R_9$ are as defined in any one of Embodiments 1-18.

Embodiment 20: In an embodiment, the compound or a pharmaceutically acceptable derivative thereof has the following structure:

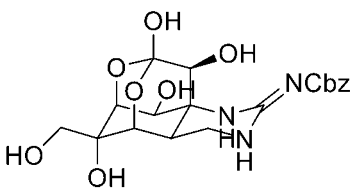 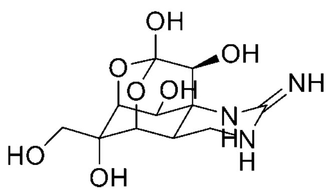 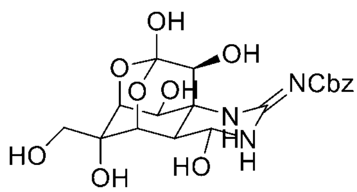

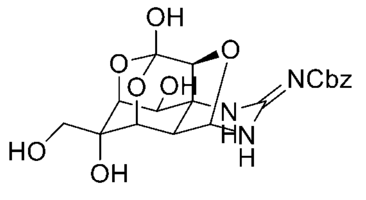
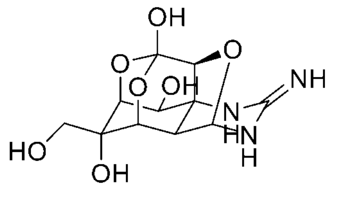
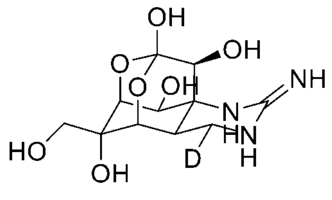

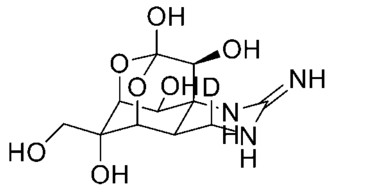
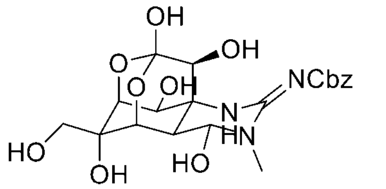
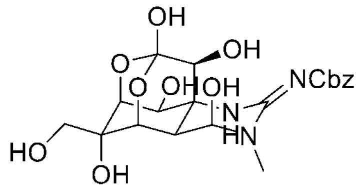

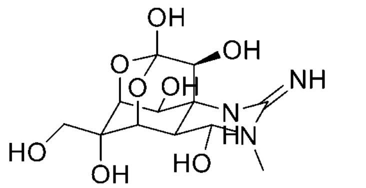
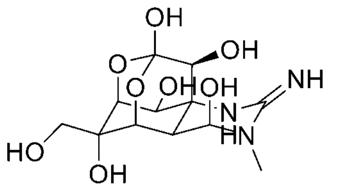
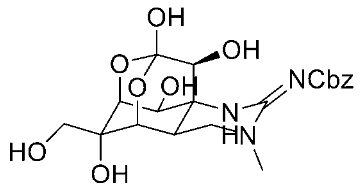

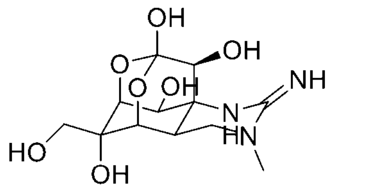
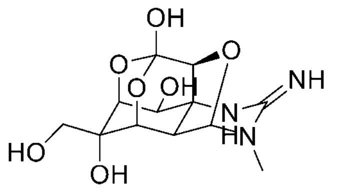
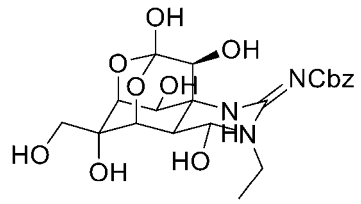

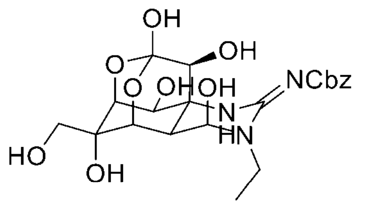
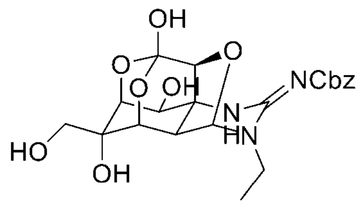
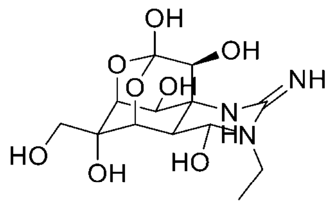

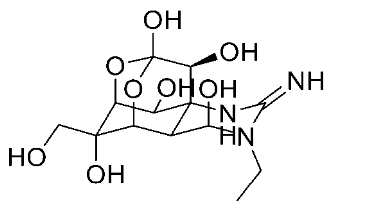
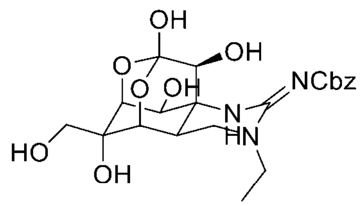
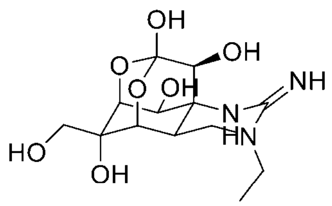

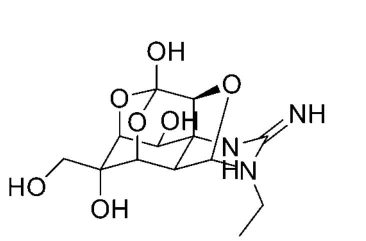
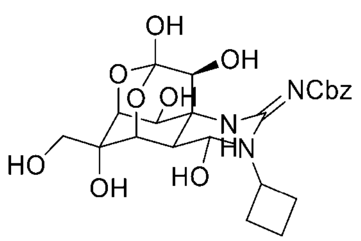
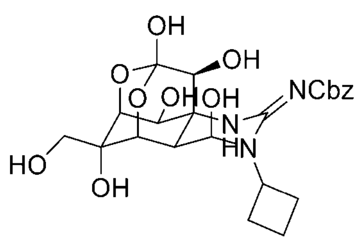

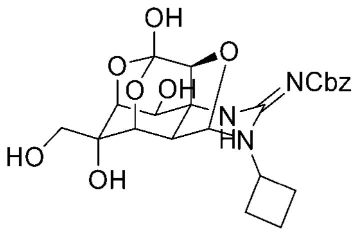
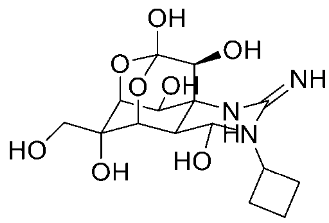
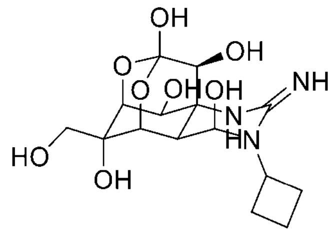

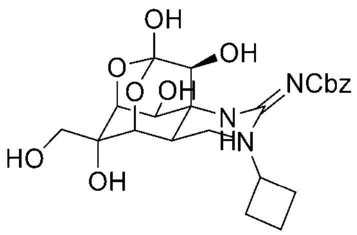
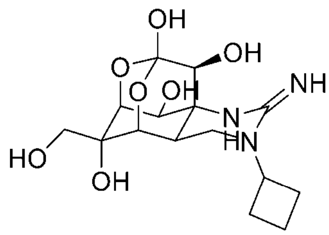
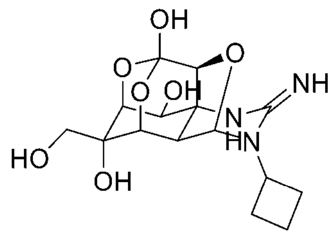

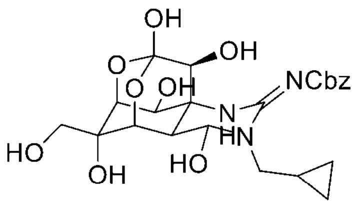
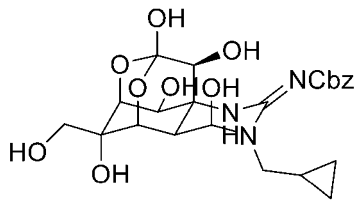
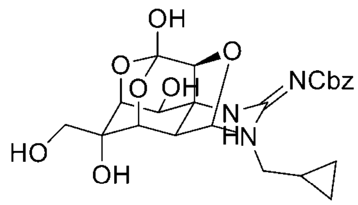

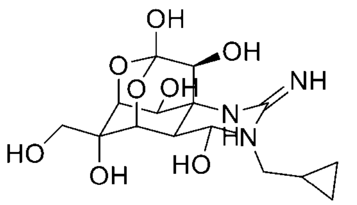
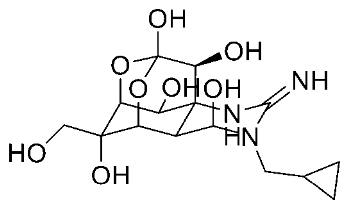
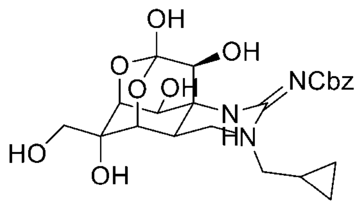

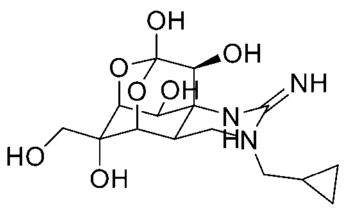
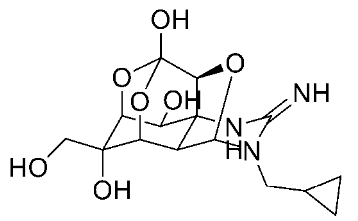
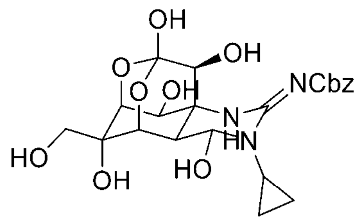

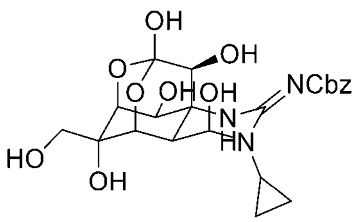
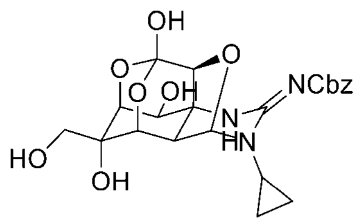
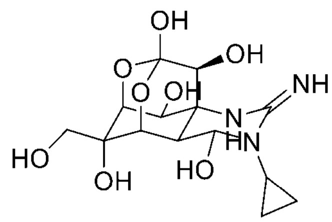

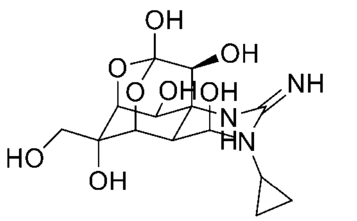
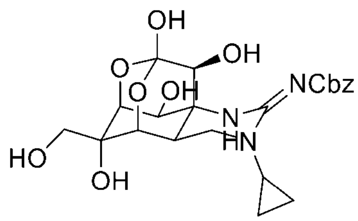
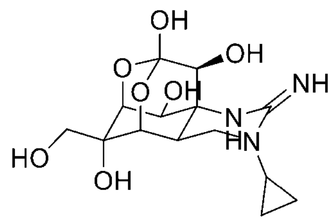

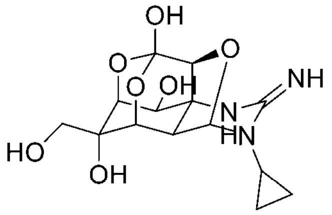
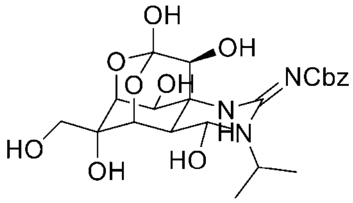
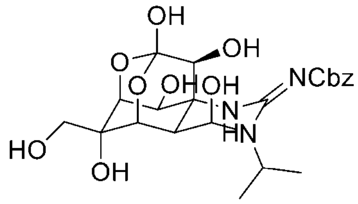
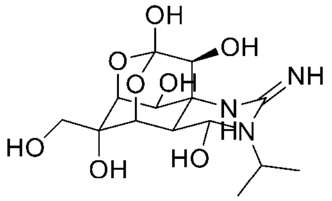
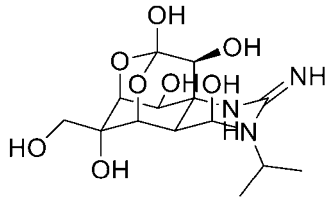
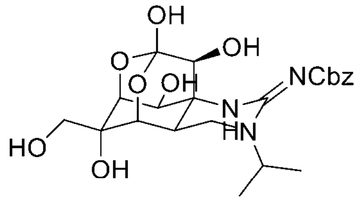
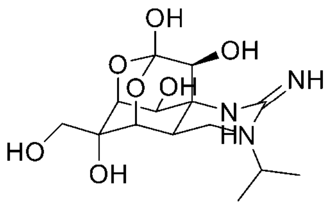
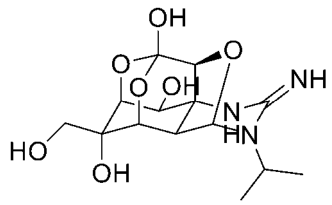
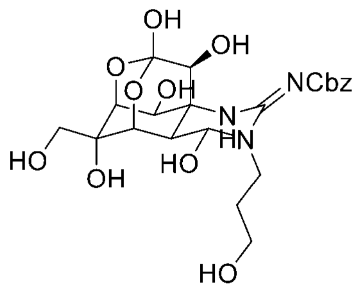
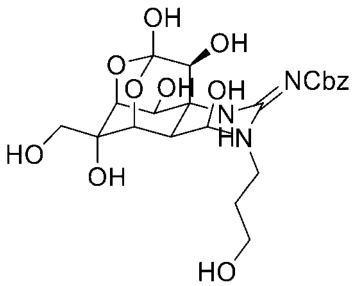
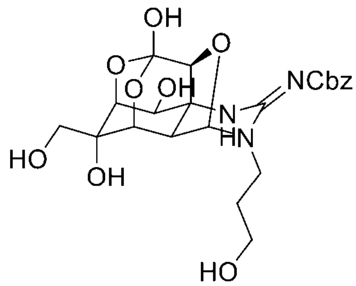
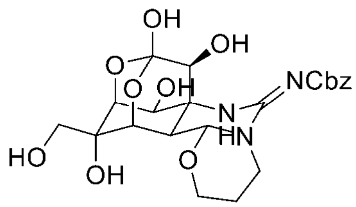
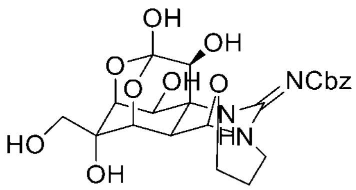
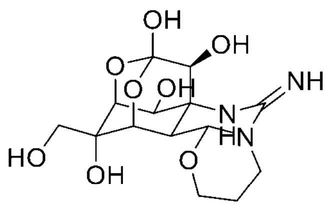
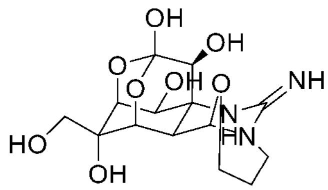
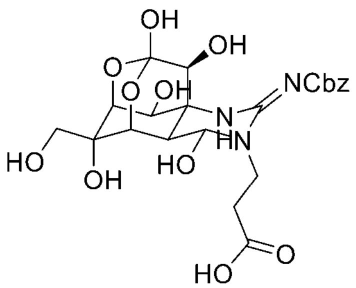
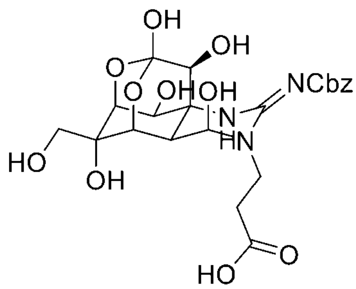
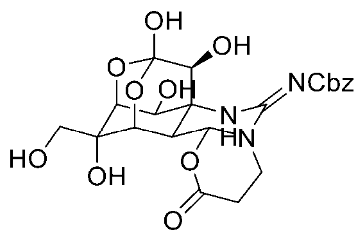

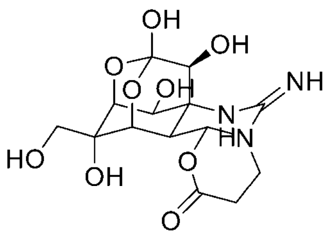
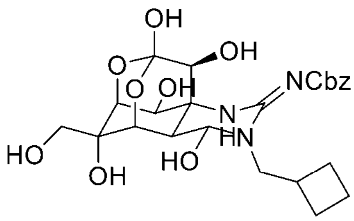
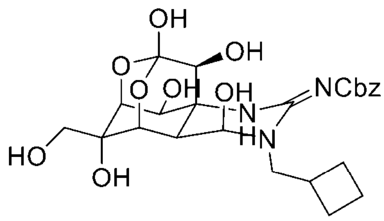

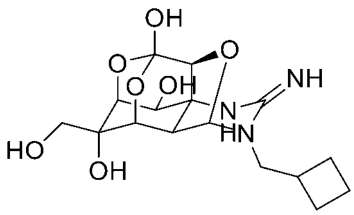
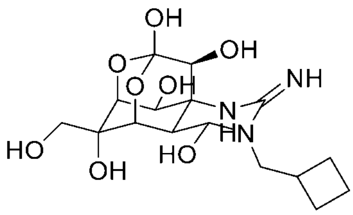
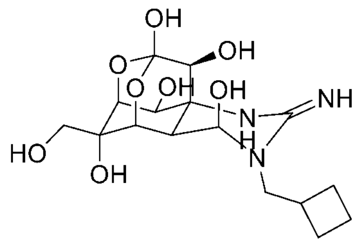

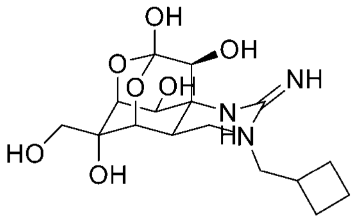
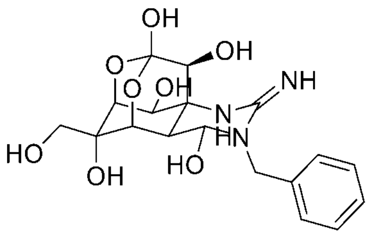
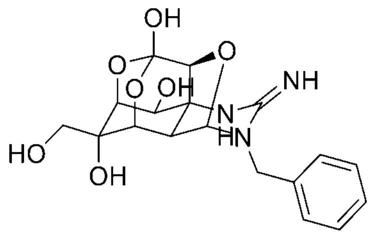

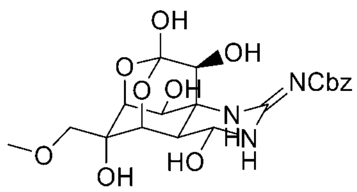
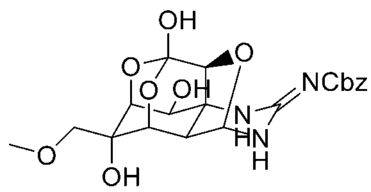
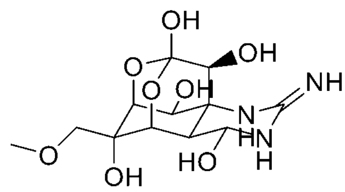

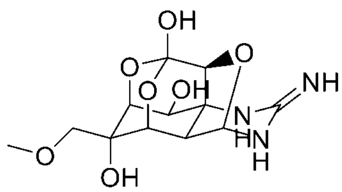
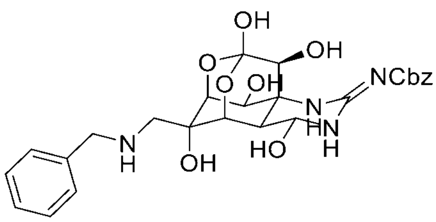
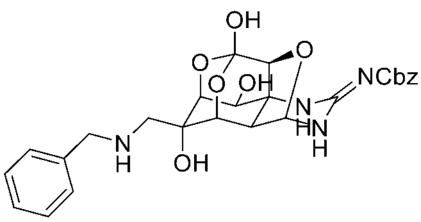

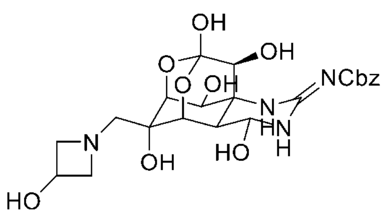
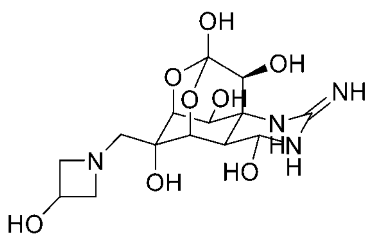
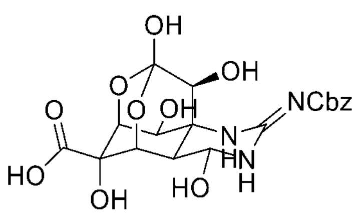

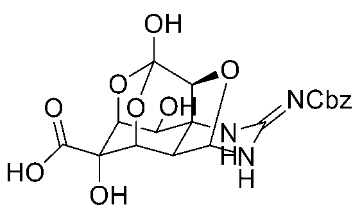
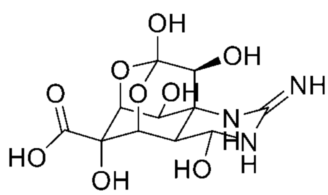
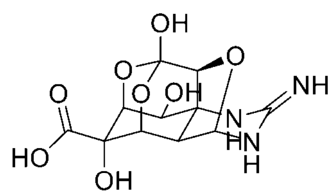

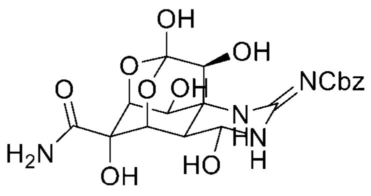
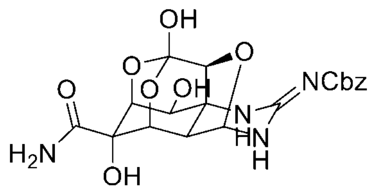
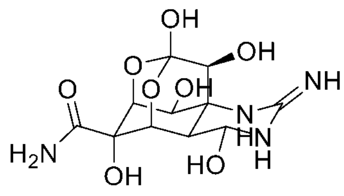
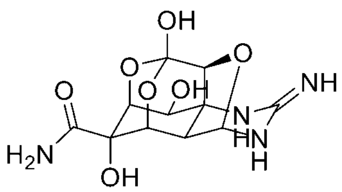
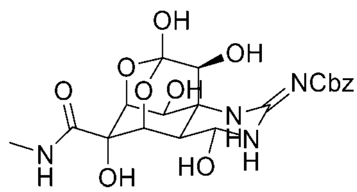
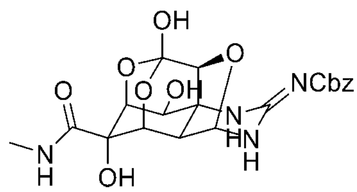
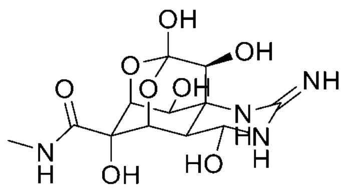
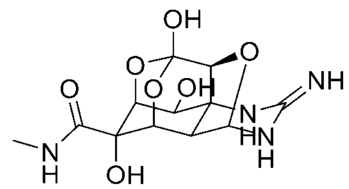
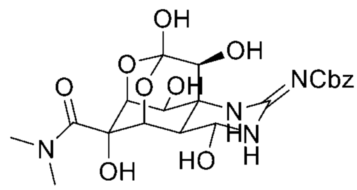
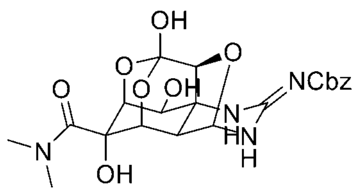
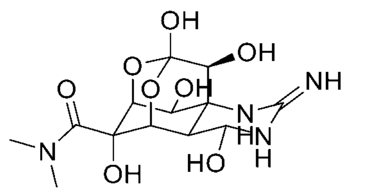
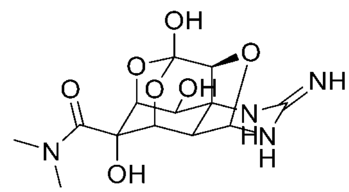
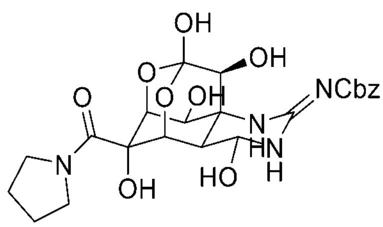
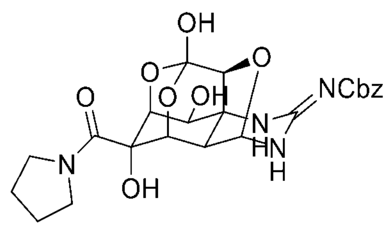
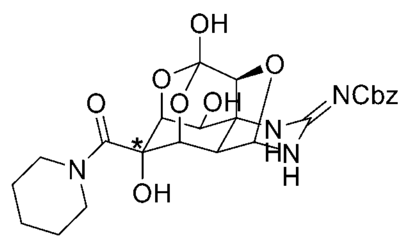
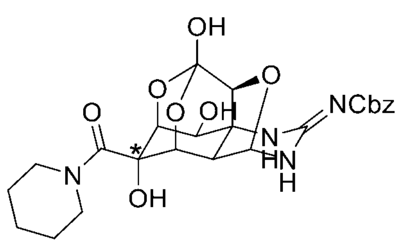
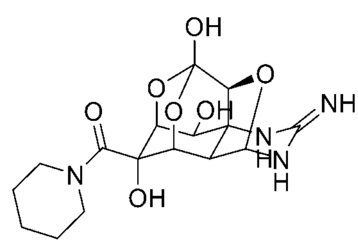
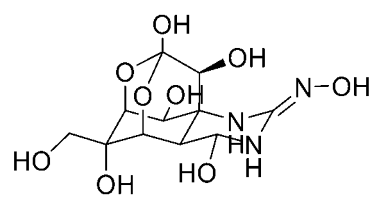
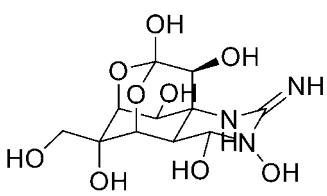
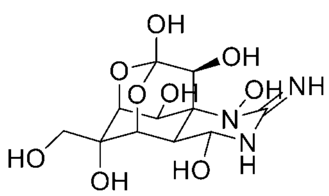
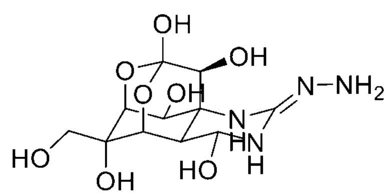

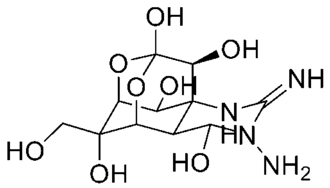
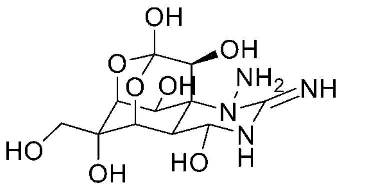
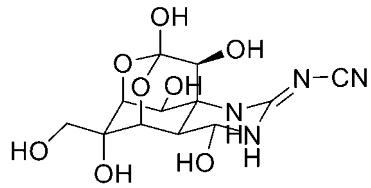
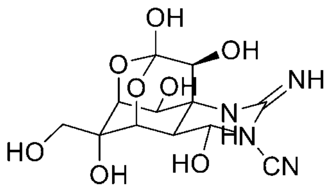
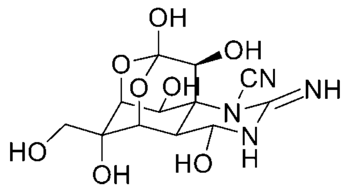
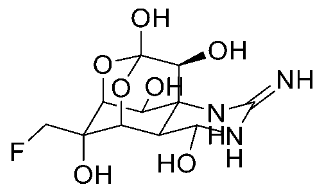
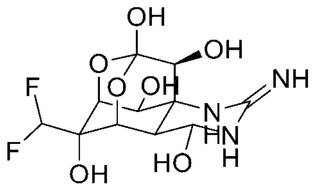
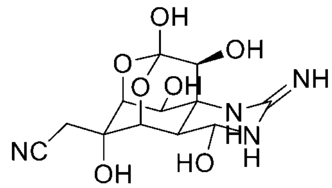
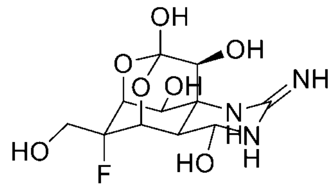
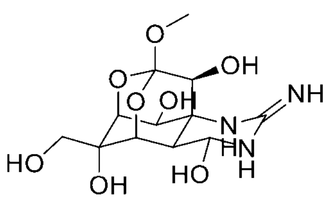
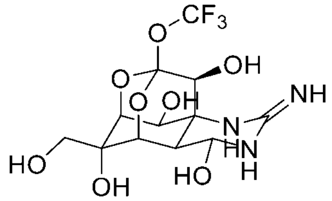
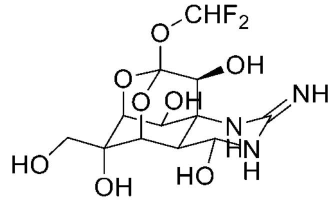
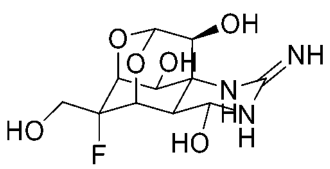
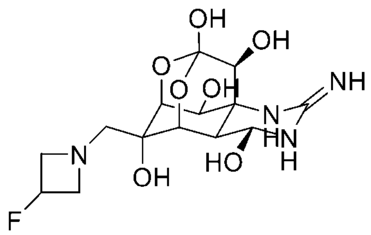
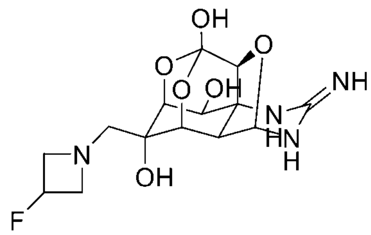
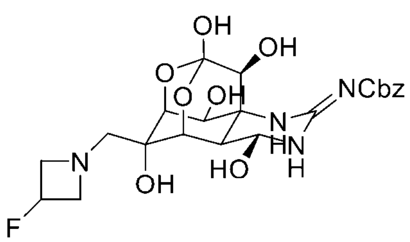
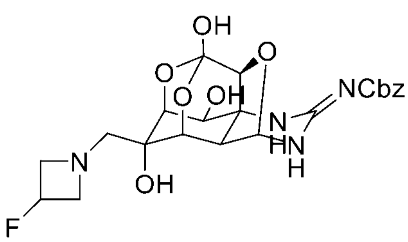
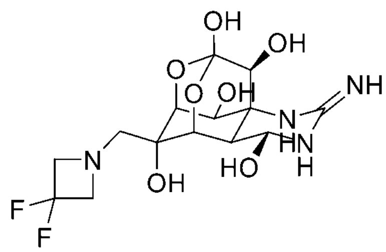
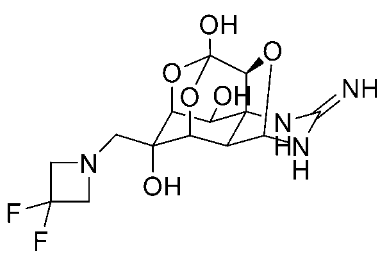
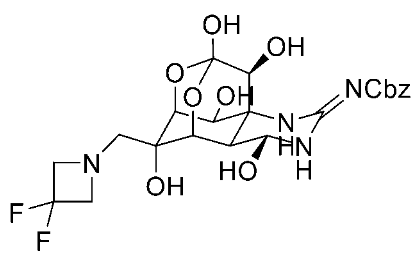

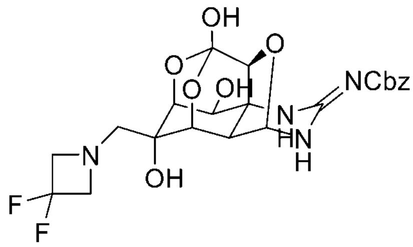
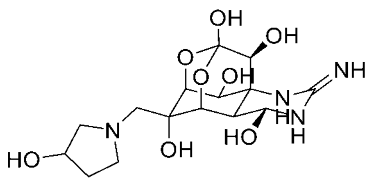
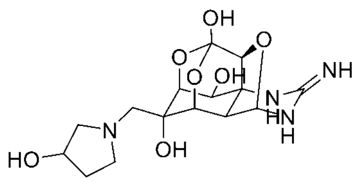

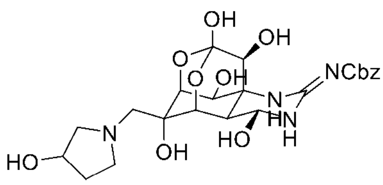
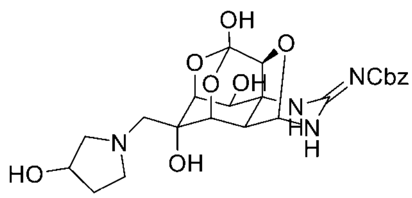
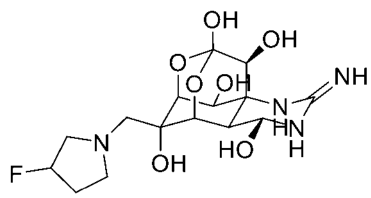

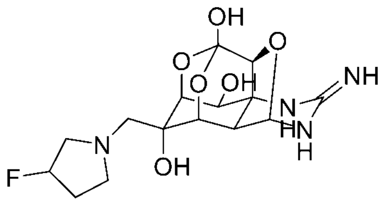
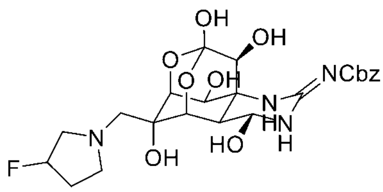
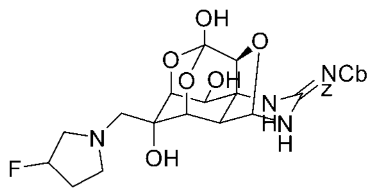

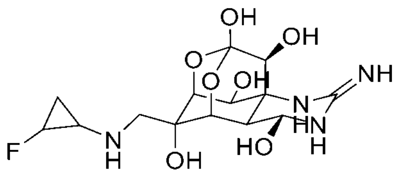
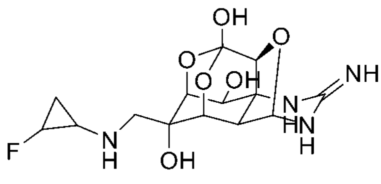
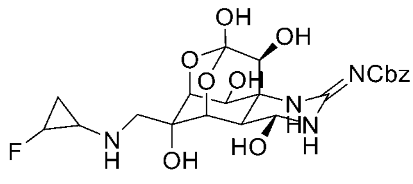

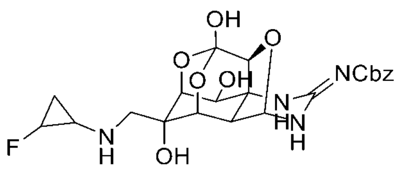
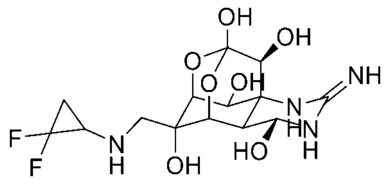
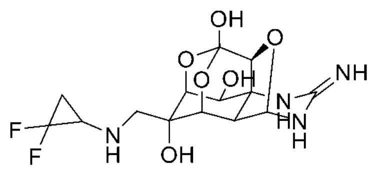

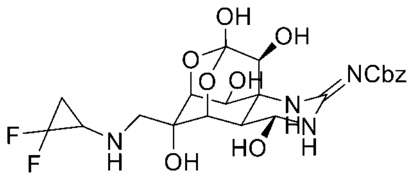
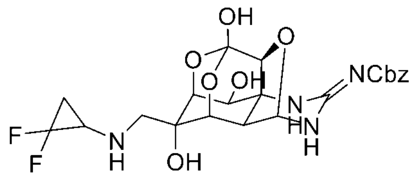
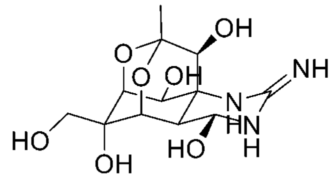

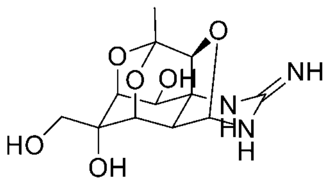
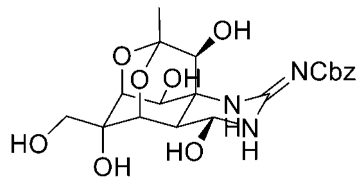
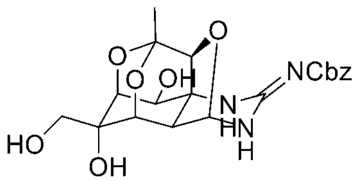

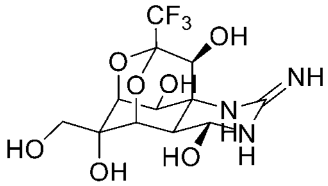
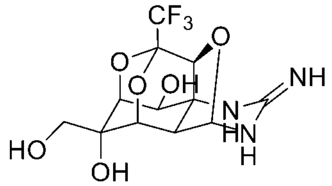
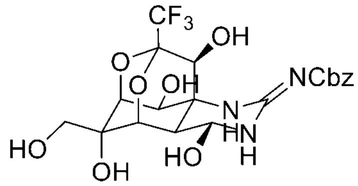

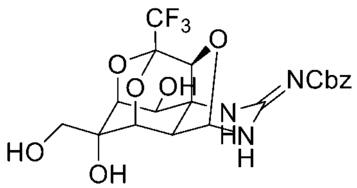
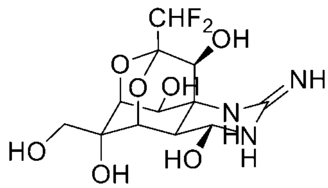
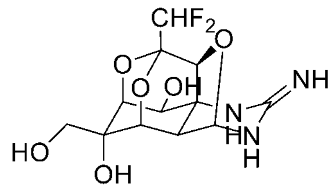

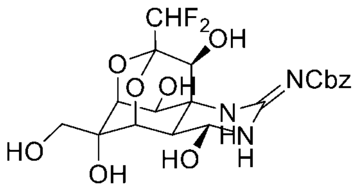
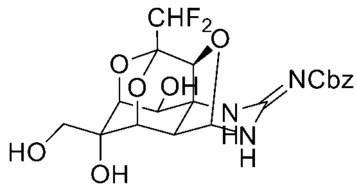
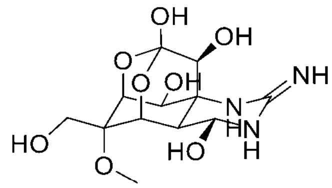

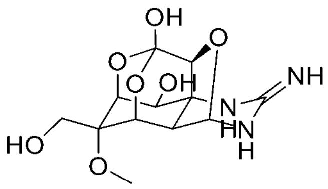
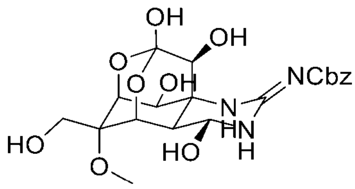
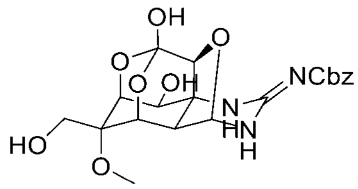

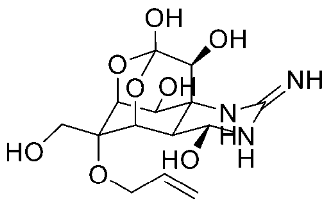
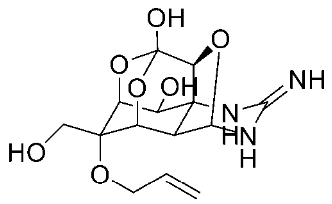
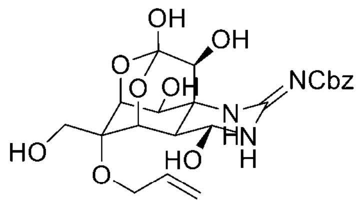

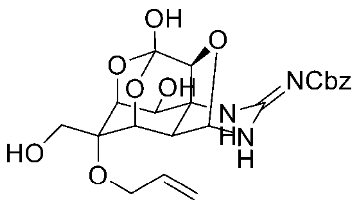
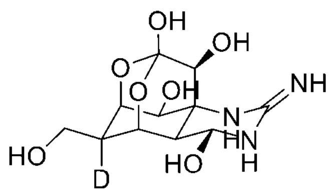
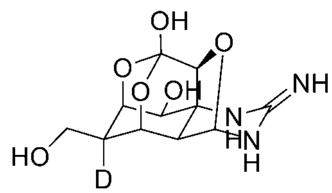

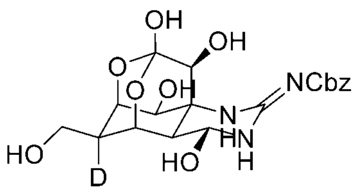
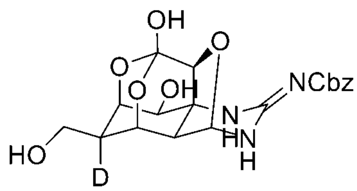
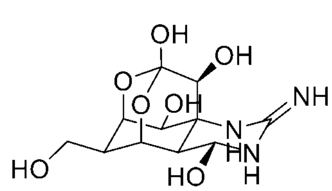

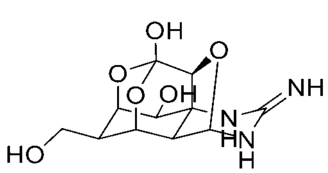
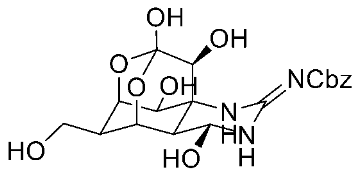
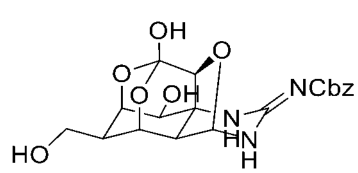

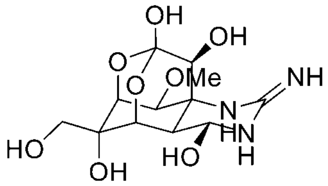
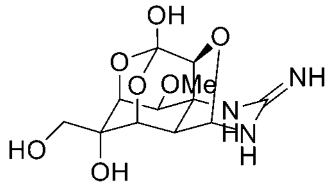
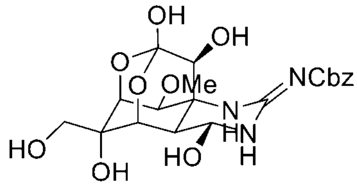
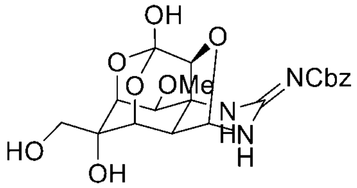
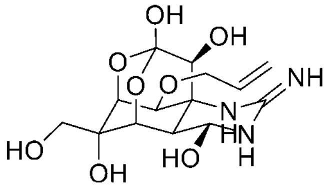
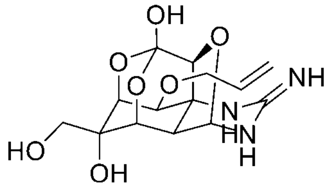
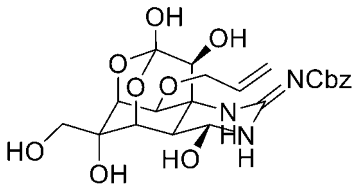
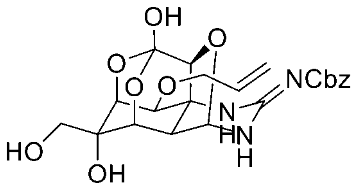
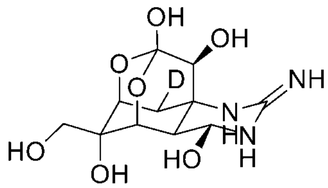
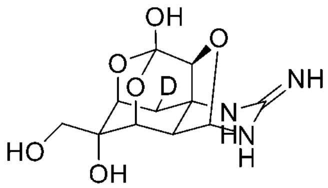
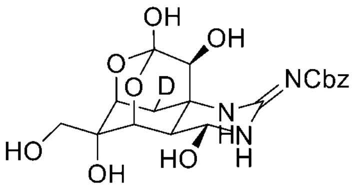
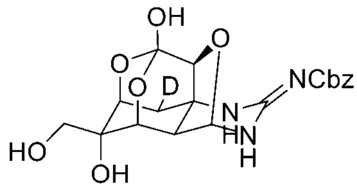
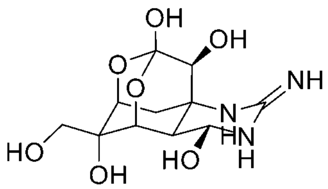
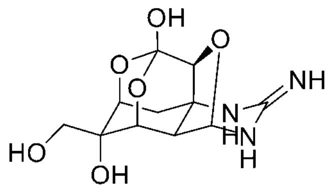
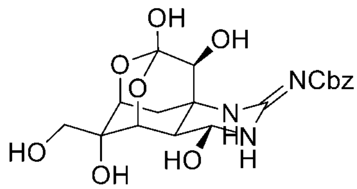
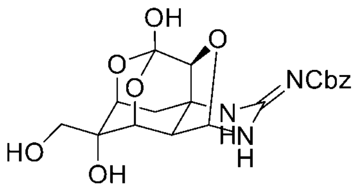
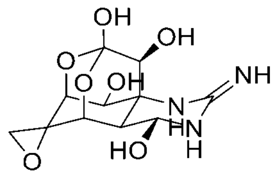
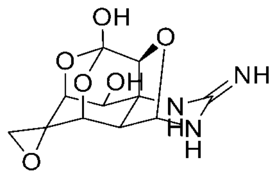
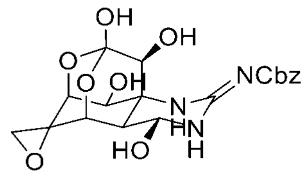
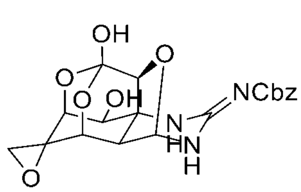
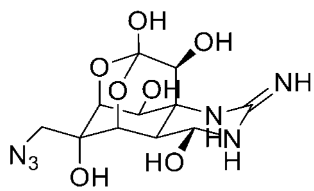
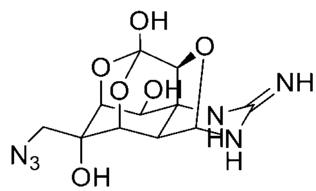
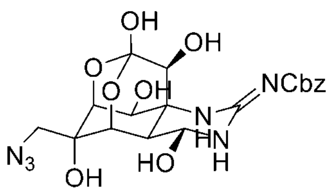
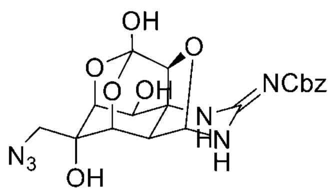
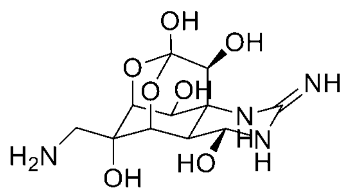

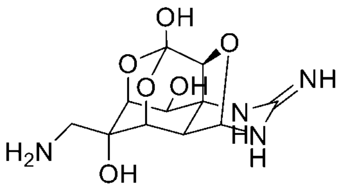
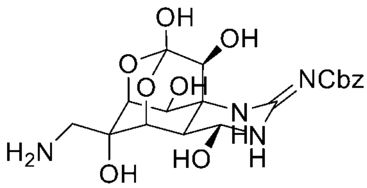
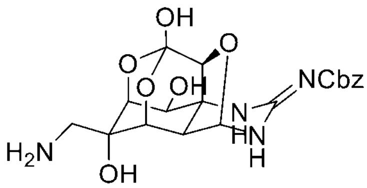

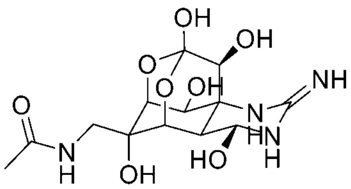
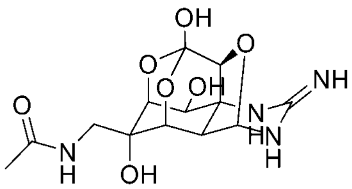
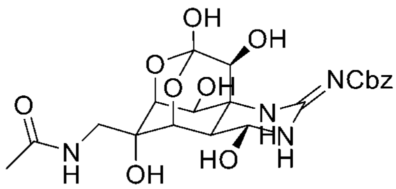

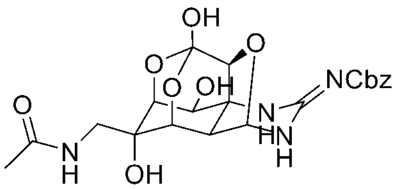
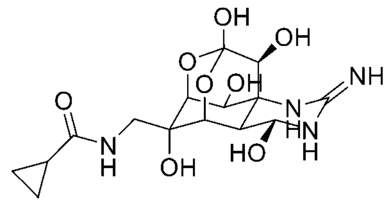
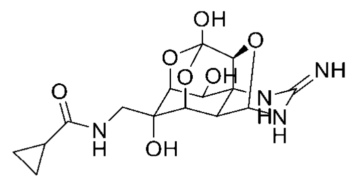

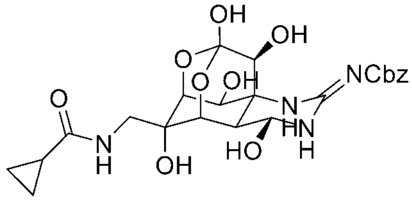
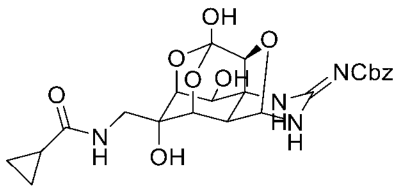

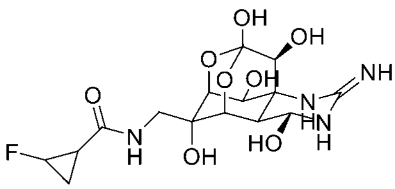
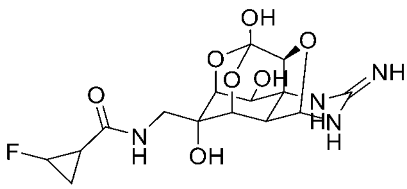
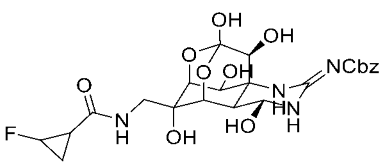

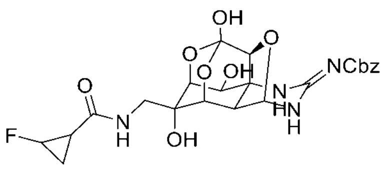
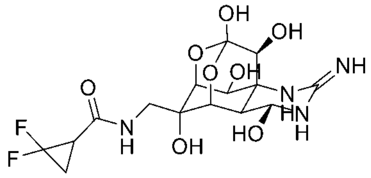
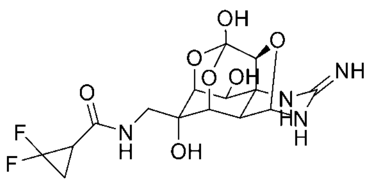

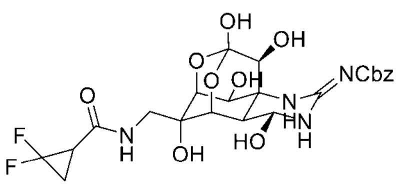
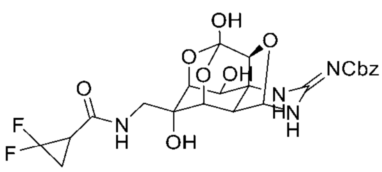
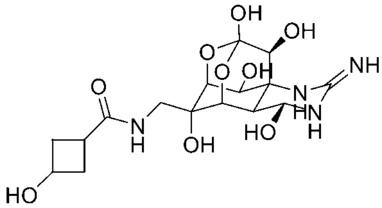

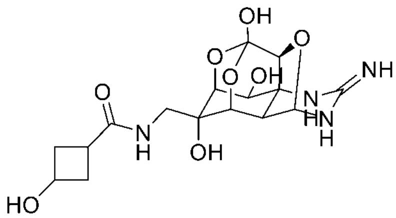

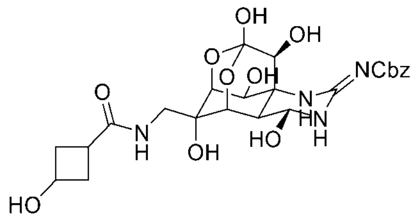

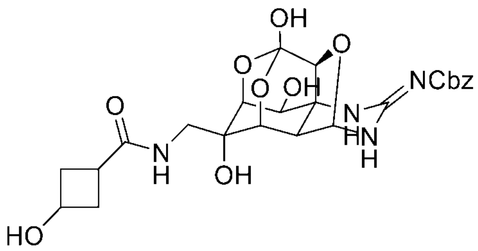

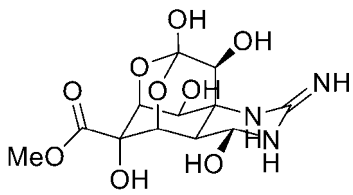

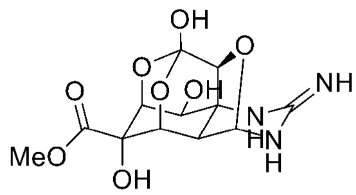

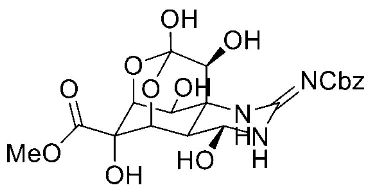

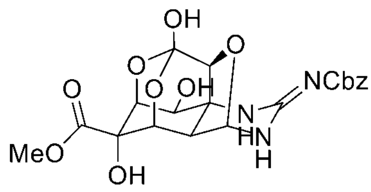

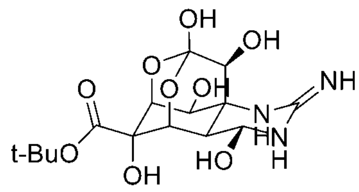

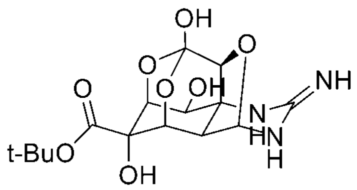

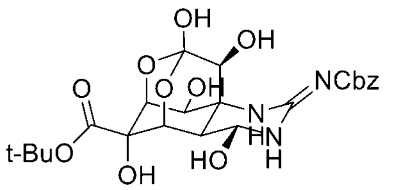

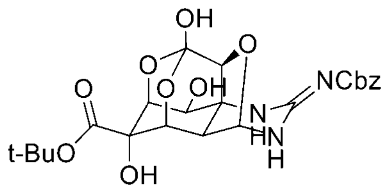

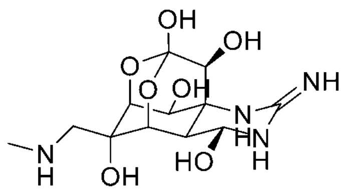

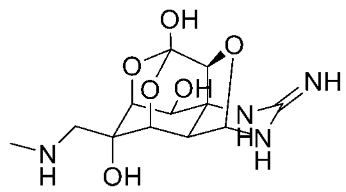

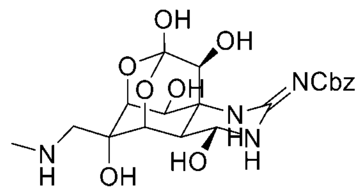

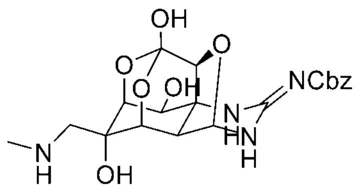

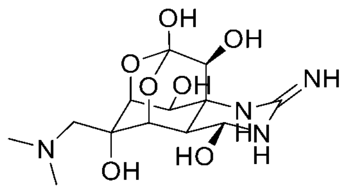

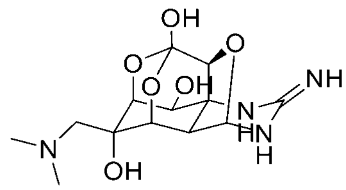

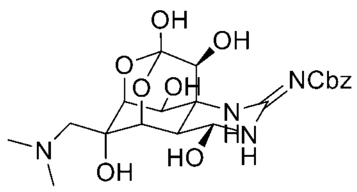

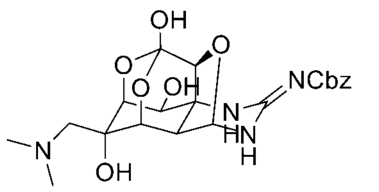

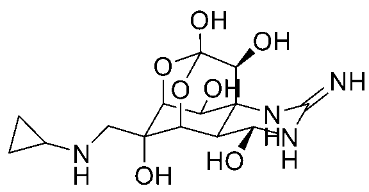

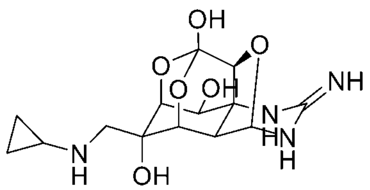
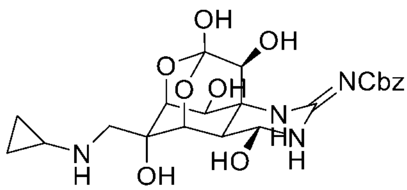
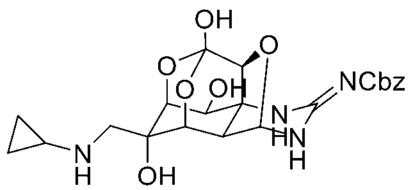

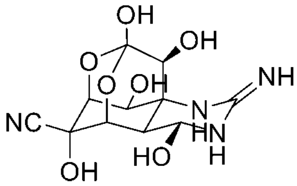
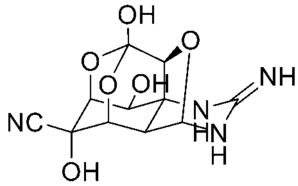
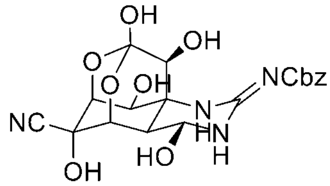

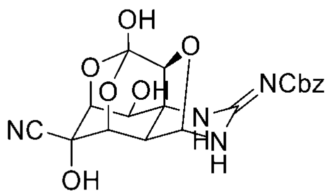
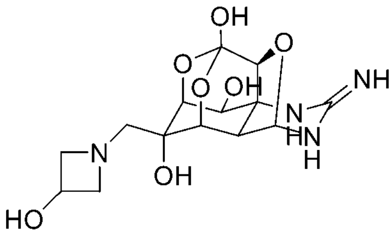
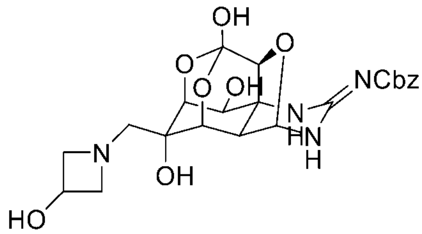

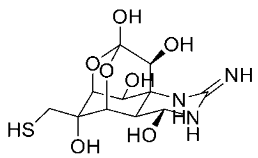
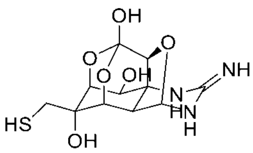
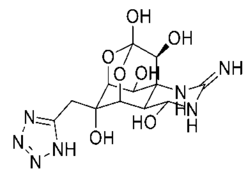
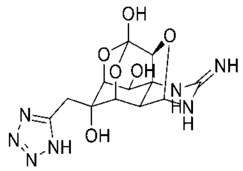

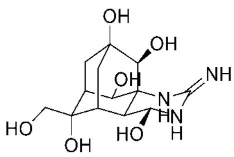
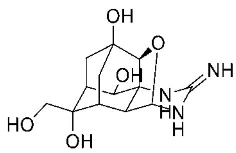
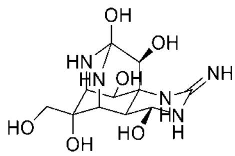
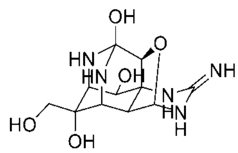

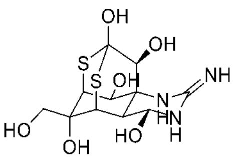
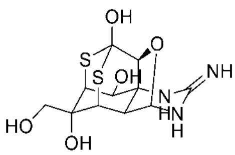
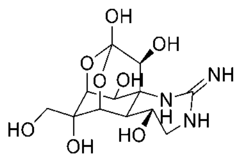
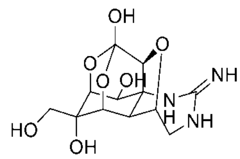

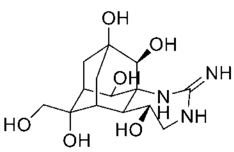
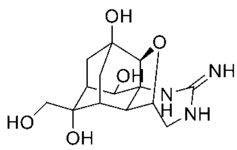
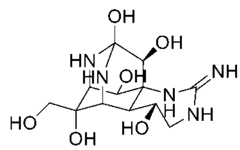
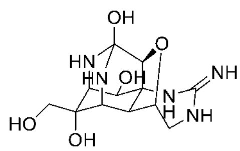

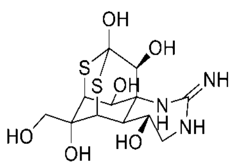
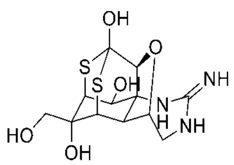
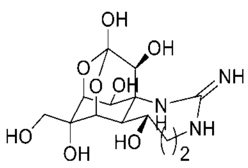
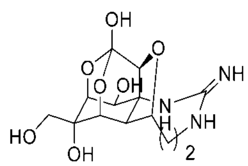

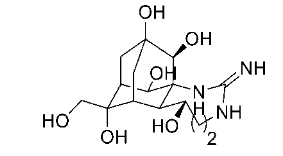
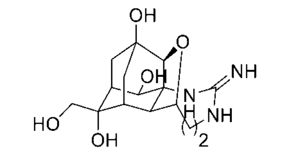
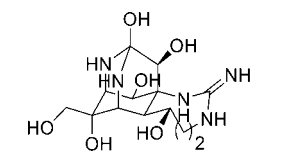
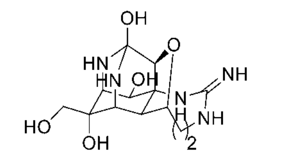

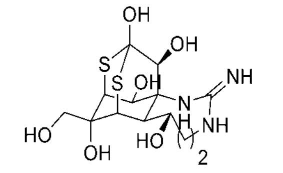
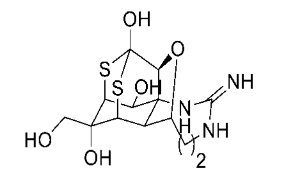
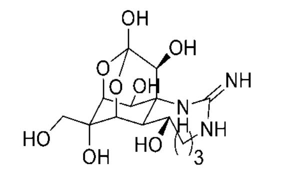
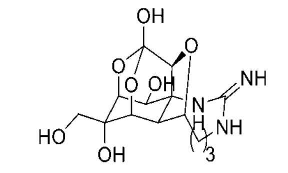

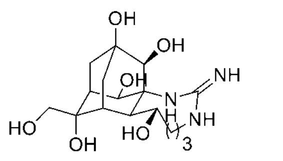
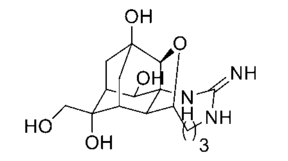
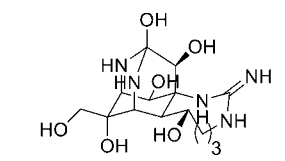
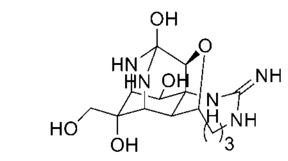

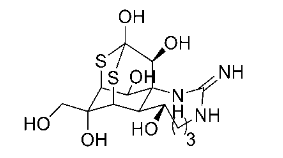
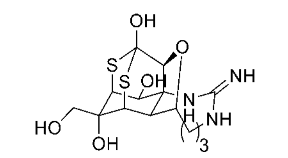
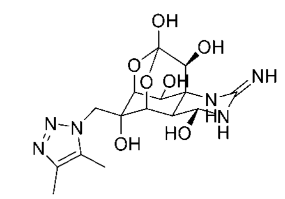
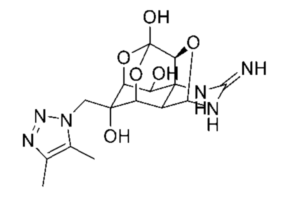

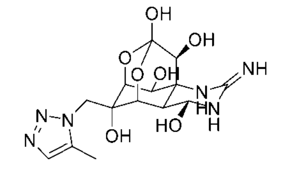
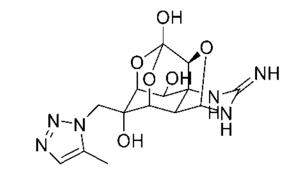
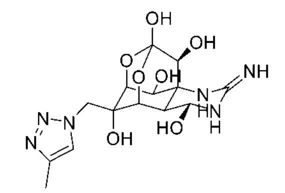
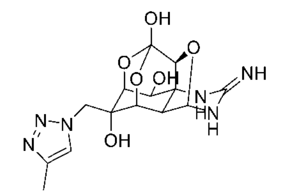

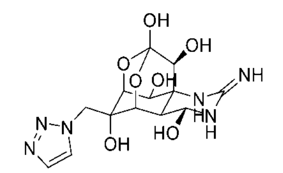
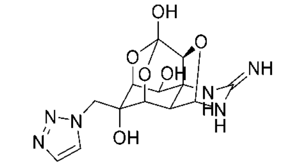
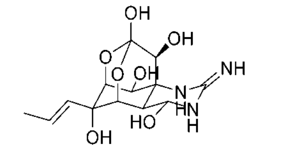
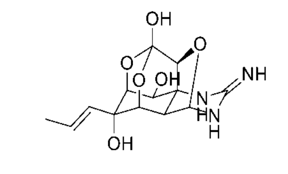

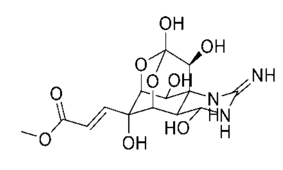
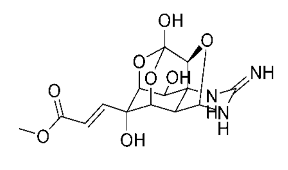
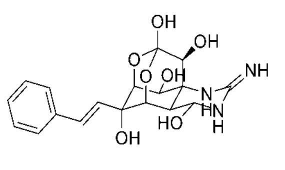
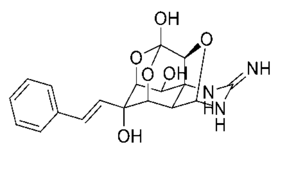

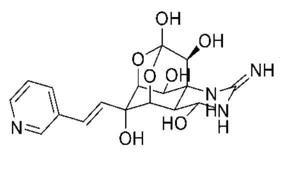
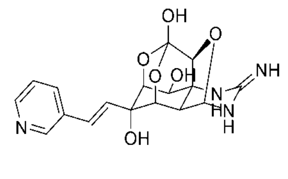
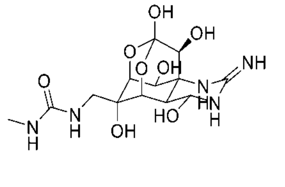
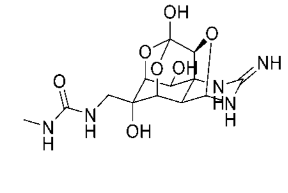

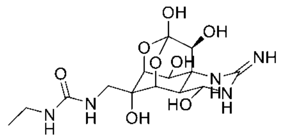 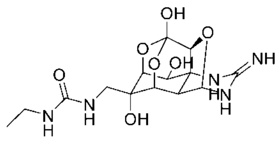 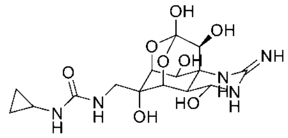 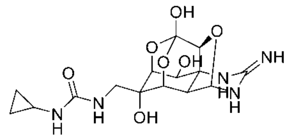

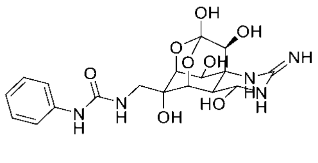 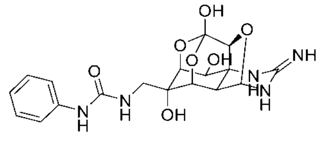 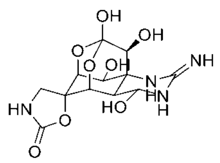 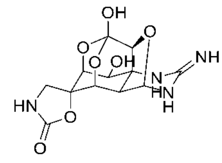

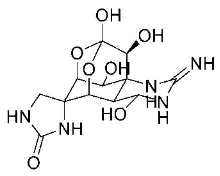 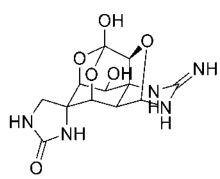 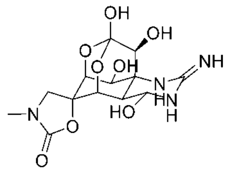 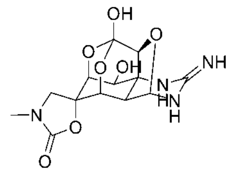

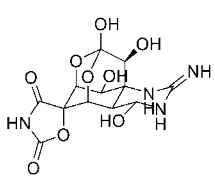 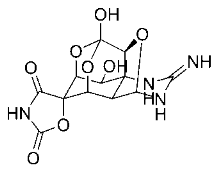 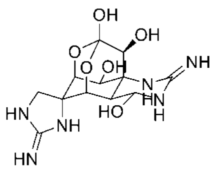 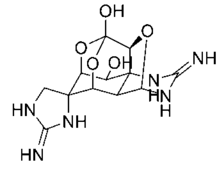

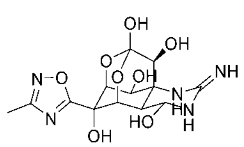 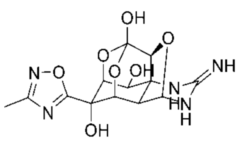 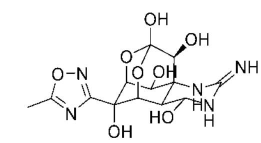 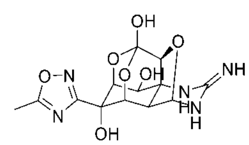

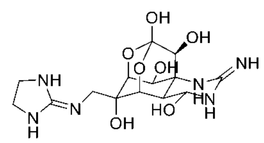 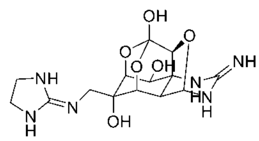 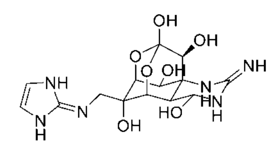 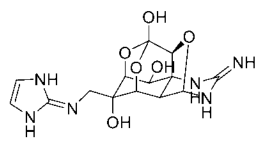

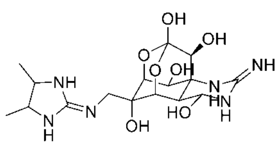 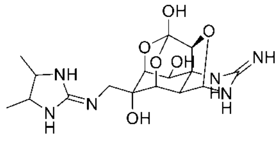 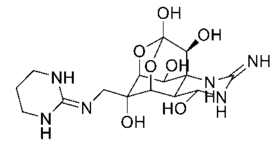 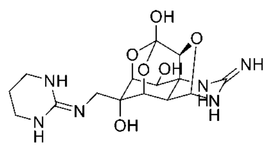

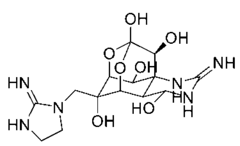 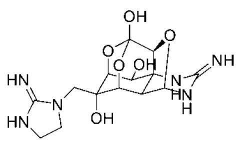 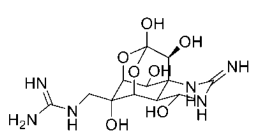 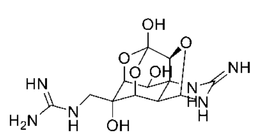

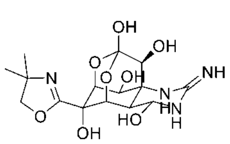 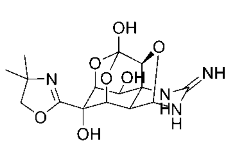 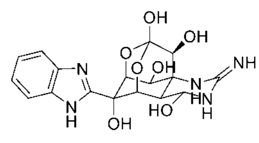 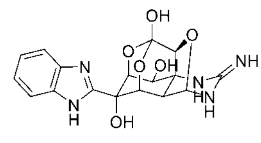

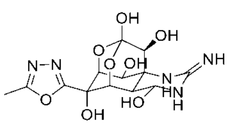
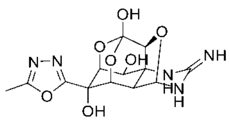
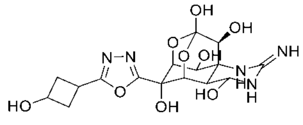
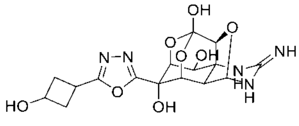

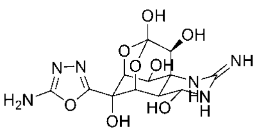
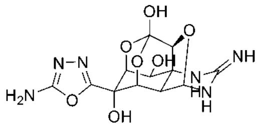
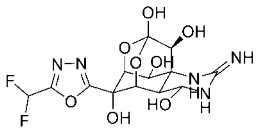
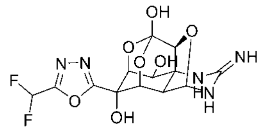

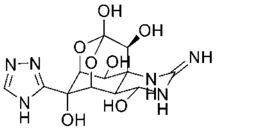
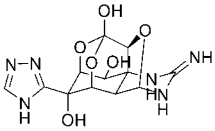
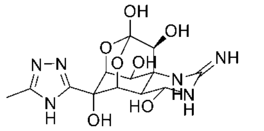
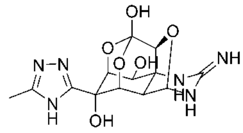

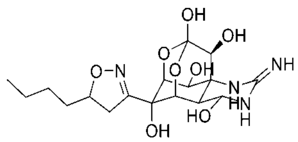
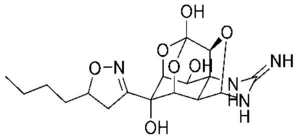
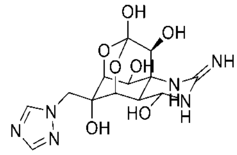
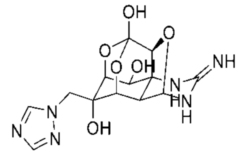

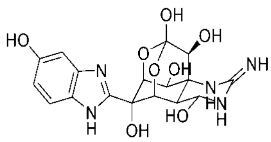
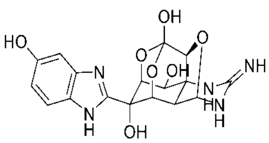
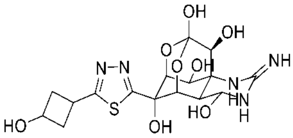
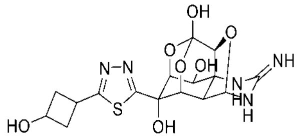

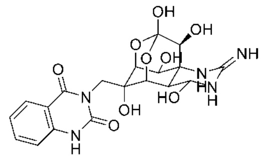
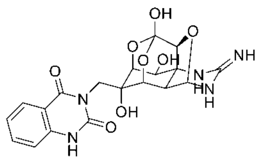
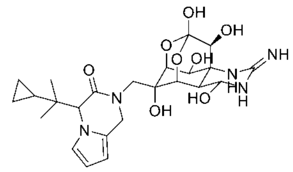
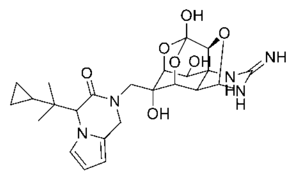

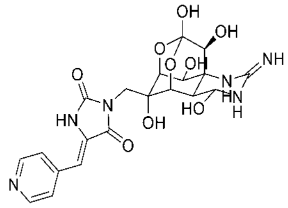
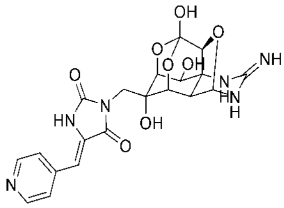
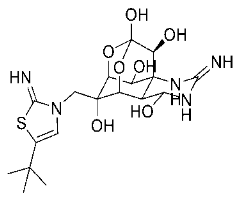
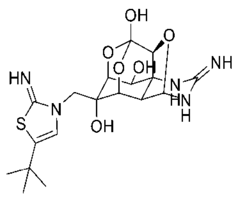

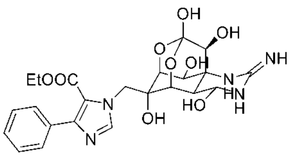
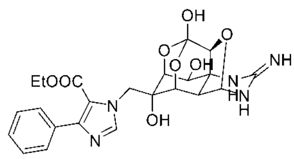
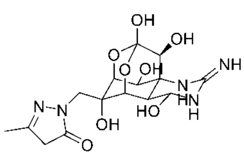
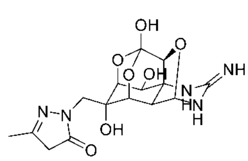

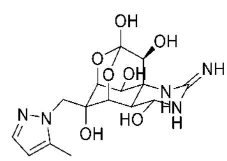
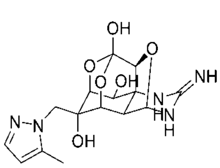
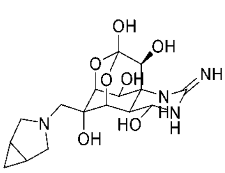
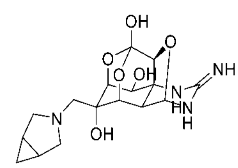

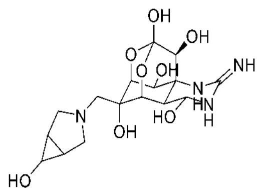
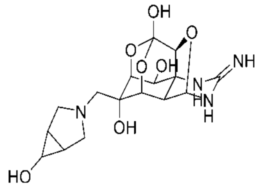
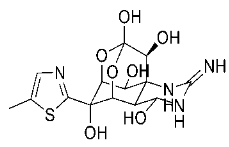
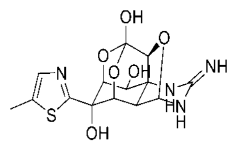

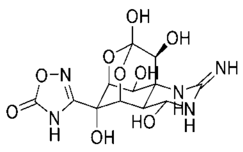
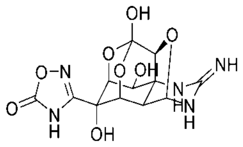
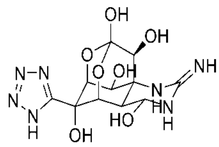
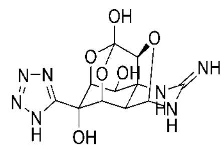

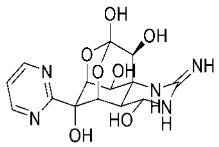
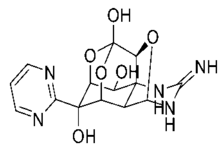
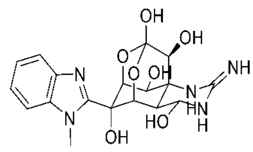
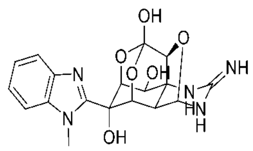
.

Embodiment 21: In an embodiment, provided is a pharmaceutical composition comprising a therapeutically effective amount of the compound of any one of Embodiments 1-20 or a pharmaceutically acceptable derivative thereof and one or more pharmaceutically acceptable carriers.

Embodiment 22: In an embodiment, provided is use of the compound or of any one of Embodiments 1-20 or a pharmaceutically acceptable derivative thereof or the pharmaceutical composition of Embodiment 21 for the manufacture of a medicament for treating a disease or condition associated with a sodium ion channel or for analgesia.

Embodiment 23: In an embodiment, provided is a compound having the structure of Formula (II):

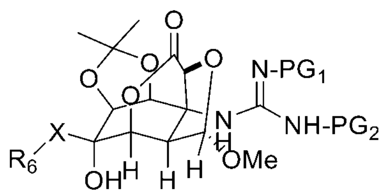
Formula (II)

wherein,

- X, $R_6$ are as defined in any one of Embodiments 1-20,
- $Pg_1$, $Pg_2$ are each independently selected from the group consisting of acetyl, trityl, tert-butoxycarbonyl (Boc), benzyloxycarbonyl (Cbz) and fluorenylmethoxycarbonyl (Fmoc).

Embodiment 24: In an embodiment, the compound of Embodiment 23 has the following structure:

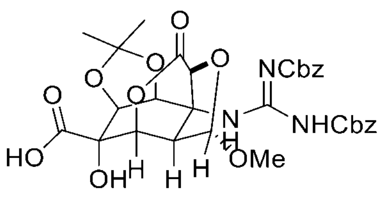
or
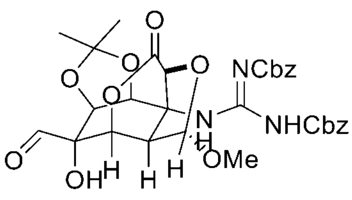
.

Beneficial Effects

The compound of formula (I) or a pharmaceutically acceptable derivative thereof provided by the present disclosure has an excellent blocking effect on the sodium ion channel. Meanwhile, compared with tetrodotoxin, the compound of formula (I) or a pharmaceutically acceptable derivative thereof provided by the present disclosure can be synthesized in a large scale by chemical synthesis, is easy to be obtained, has better analgesic strength and better therapeutic window, and has broader application prospects. The compound of the present disclosure or a pharmaceutically acceptable derivative thereof also has better toxicological properties (for example, lower toxicity) and selectivity to sodium ion channel subtypes, which can avoid the effect on the sodium ion channel hNav1.5 that may cause a cardiac disease, thereby having better drug safety under the same or equivalent therapeutic effects.

## Example

Synthesis Preparation Examples

Unless otherwise specified, the reagents and solvents used in the synthesis are provided by domestic manufacturers and can be used without further purification.
Synthesis of Intermediates:

Intermediate Synthesis Example 1: Synthesis of benzyl (Z)-((methylamino)(methylthio)methylene)carbamate **A1**

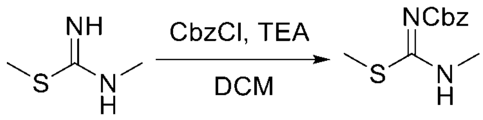


**A1-1** **A1**

**Compound A1-1** (4.00 g, 38.40 mmol), dichloromethane (50 mL) and triethylamine (8.70 g, 85.98 mmol) were added to a reaction flask, which was cooled to 0 °C under nitrogen protection, and benzyl chloroformate (8.60 g, 50.41 mmol) was slowly added dropwise. After the addition was completed, the reaction mixture was stirred at room temperature for 16 h. The reaction mixture was then diluted with water (100 mL) and extracted with dichloromethane (50 mL×3). The organic phases were combined and concentrated to give a residue. The residue was purified by flash column chromatography (ethyl acetate/petroleum ether=10%-15%) to give **Compound A1** (0.80 g, yield: 9%). LCMS (m/z): 239.1[M+1]$^+$.

Intermediate Synthesis Example 2: Synthesis of benzyl *(E)*-((ethylamino)(methylthio)methylene)carbamate **A2**

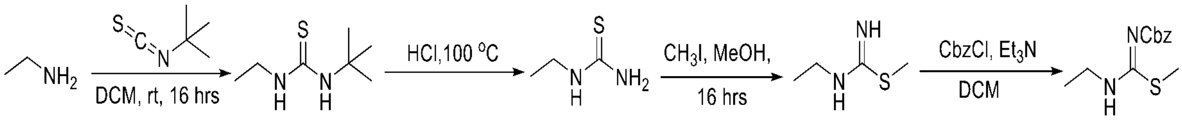


**A2-1** **A2-2** **A2-3** **A2-4** **A2**

Step 1: Synthesis of 1-(tert-butyl)-3-ethylthiourea (A2-2)

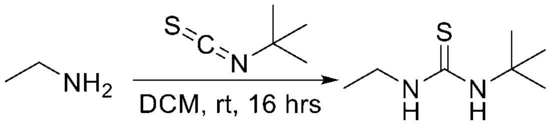


**A2-1** **A2-2**

To a solution of ethylamine (0.78 g, 17.30 mmol) in DCM (40 mL) was added tert-butyl isothiocyanate (2.00 g, 17.36 mmol) at 0 °C. After the addition was completed, the reaction mixture was stirred at room temperature for 3 h, and then the mixture was concentrated to give **Compound A2-2** as white solid (2.50 g, yield: 90%), which was directly used in the subsequent reaction. LCMS (m/z): 161.1[M+1]$^+$.

Step 2: Synthesis of 1-ethylthiourea **(A2-3)**

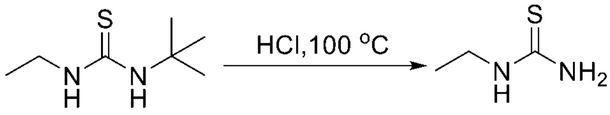


A2-2 A2-3

Concentrated hydrochloric acid (15 mL) and **Compound A2-2** (2.50 g, 15.60 mmol) were added to a reaction flask, wheated to 100 °C, and stirred for 1 h. The reaction mixture was concentrated to give crude **Compound A2-3** as pale yellow oil (2.50 g, yield 100%). LCMS (m/z): 105.0[M+1]$^+$.

Step 3: Synthesis of methyl ethylcarbamimidothioate **(A2-4)**

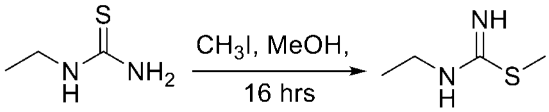


A2-3 A2-4

**Compound A2-3** (2.50 g, 15.60 mmol, crude) and methanol (10 mL) were added to a reaction flask, to which iodomethane (2 mL, 32.13 mmol) was added dropwise at room temperature, and the reaction mixture was stirred at room temperature for 16 h. The reaction mixture was concentrated to give crude **Compound A2-4** as pale yellow oil (2.50 g, yield 100%). LCMS (m/z): 119.1[M+1]$^+$.

Step 4: Synthesis of **Compound A2**

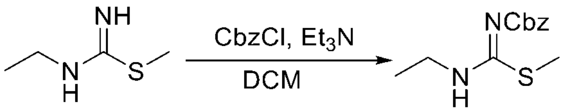


A2-4 A2

The **Crude compound A2-4** (2.50 g, 15.60 mmol), dichloromethane (40 mL) and triethylamine (8.48 g, 83.80 mmol) were added to a reaction flask, which was cooled to 0 °C under nitrogen protection, to which benzyl chloroformate (5.30 g, 31.07 mmol) was added dropwise, and the reaction mixture was stirred at room temperature for 16 h. Then the reaction mixture was diluted with water (100 mL), extracted with dichloromethane (50 mL×3), and the combined organic phases were concentrated to give a residue. The residue was purified by flash column chromatography (ethyl acetate/petroleum ether=10% -100%) to give **Compound A2** as a yellow solid (2.40 g, yield of three-step reaction: 61%). LCMS (m/z): 253.1[M+1]$^+$.

Intermediate Synthesis Example 3: Synthesis of benzyl (*E*)-((cyclopropylamino)(methylthio)methylene)carbamate (A3)

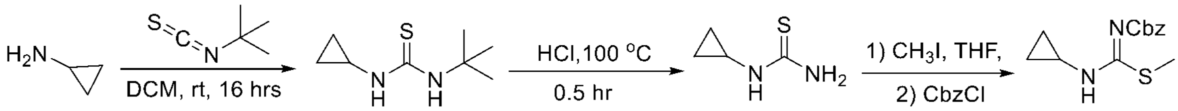


A3-1 A3-2 A3-3 A3

**Compound A3** was synthesized by using cyclopropylamine as a starting material, and the first two steps of synthesis of **Compound A3-3** were conducted according to the synthesis procedures of **Compound A2.** In the third step, **Compound A3** was synthesized by a one-pot method.

Step 3: Synthesis of **Compound A3**

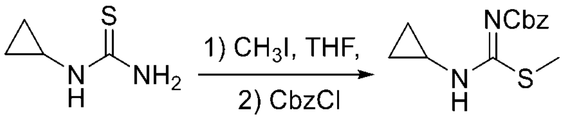


**A3-3** **A3**

**Compound A3-3** (2.50 g, 21.52 mmol) and tetrahydrofuran (30 mL) were added to a reaction flask, to which iodomethane (3.67 g, 25.86 mmol) and sodium bicarbonate (9.05 g, 107.73 mmol) were added under stirring, and the the reaction was carried out overnight at room temperature under nitrogen protection. Then the reaction solution was cooled to 0°C, to which benzyl chloroformate (4.40 g, 25.79 mmol) was added, and the reaction was carried out at room temperature for 3 h. Then water (200 mL) was added, the mixture was extracted with ethyl acetate (50 mL×3), and the organic phases were combined, washed with saturated sodium chloride solution (100 mL), dried over anhydrous sodium sulfate, and concentrated to give a crude product, which was purified by flash column chromatography (ethyl acetate/petroleum ether=25% - 67%) to give **Compound A3** as white solid (3.50, yield: 62%). LCMS (m/z): 265.1[M+1]$^+$.

Intermediate Synthesis Example 4: Synthesis of benzyl (*E*)-((isopropylamino)(methylthio)methylene)carbamate **(A4)**

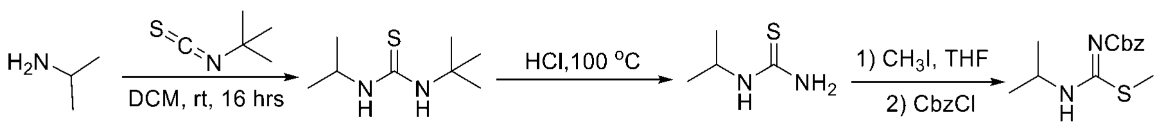


**A4-1** **A4-2** **A4-3** **A4**

**Compound A4** was synthesized by using isopropylamine as a starting material according to the synthesis of **Compound A3.** LCMS (m/z): 267.1[M+1]$^+$.

Intermediate Synthesis Example 5: Synthesis of benzyl (*E*)-(((cyclobutylmethyl)amino)(methylthio)methylene)carbamate **(A5)**

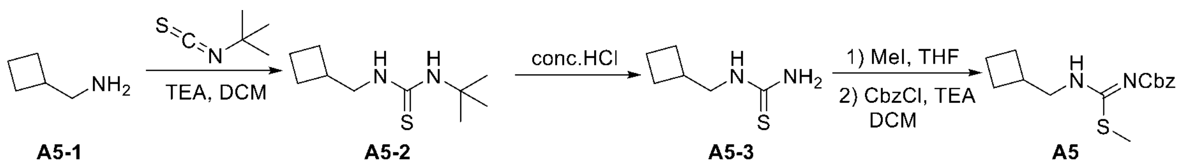


**A5-1** **A5-2** **A5-3** **A5**

**Compound A5** was synthesized by using cyclobutylmethylamine as a starting material according to the synthesis of **Compound A3.** LCMS (m/z): 293.1[M+1]$^+$.

Intermediate Synthesis Example 6: Synthesis of benzyl (*E*)-((cyclobutylamino)(methylthio)methylene)carbamate (A6)

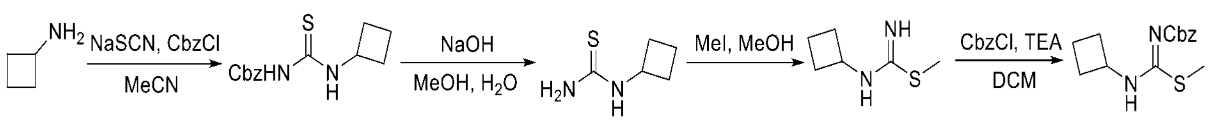


**A6-1** **A6-2** **A6-3** **A6-4** **A6**

Step 1: Synthesis of Compound A6-2

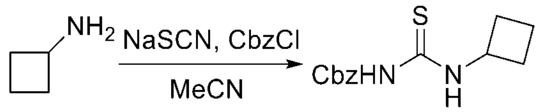


**A6-1** **A6-2**

Sodium thiocyanate (1.25 g, 15.42 mmol) and acetonitrile (10 mL) were added to a reaction flask, which was cooled to 0 °C, and benzyl chloroformate (2.34 g, 13.72 mmol) was added dropwise. After the addition was completed, the temperature was raised to 50 °C, the reaction mixture was stirred for 10 min, and then cyclobutylamine (1.00 g, 14.06 mol) was added. After the addition was completed, the reaction mixture was stirred at 50 °C for 10 h. The reaction solution was concentrated to give a residue. The residue was subjected to flash column chromatography to give **Compound A6-2** (360 mg, yield: 10%) as off-white solid.

$^1$H NMR (400MHz, DMSO) $\delta$ 11.09(s, 1H), 9.89(d, *J*=6.6Hz, 1H), 7.48-7.26(m, 5H), 5.19(s, 2H), 4.67-4.52(m, 1H), 2.43-2.25(m, 2H), 2.06-1.88(m, 2H), 1.81-1.63(m, 2H); LCMS (m/z): 265.1[M+1]$^+$.

Step 2: Synthesis of 1-cyclobutylthiourea **(A6-3)**

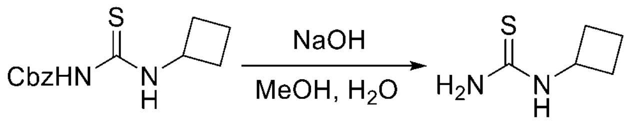


**A6-2** **A6-3**

**Compound A6-2** (360 mg, 1.36 mmol), methanol (3 mL) and water (3 mL) were added into a reaction flask, to which sodium hydroxide (0.55 g, 13.68 mmol) was added under stirring, and the reaction mxiture was stirred at room temperature for 1 h. 1M diluted hydrochloric acid was added to the reaction solution, the pH value was adjusted to about 6, and then the reaction solution was concentrated to give crude **Compound A6-3** (250 mg, yield > 100%). LCMS (m/z): 131.1[M+1]$^+$.

Step 3: Synthesis of methyl cyclobutylcarbamimidothioate (A6-4)

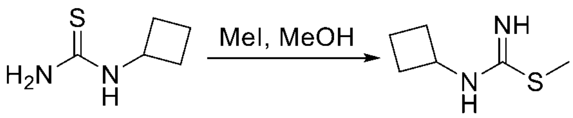


**A6-3** **A6-4**

**Compound A6-3** (1.50 g, 8.75 mmol, crude) and methanol (20 mL) were added to a reaction flask, to which iodomethane (1.86 g, 13.10 mmol) was added dropwise at room temperature, and then the reaction mixture was stirred at room temperature for 16 h. The reaction solution was concentrated to give crude **Compound A6-4** as yellow oily liquid (2.00 g, yield > 100%). LCMS (m/z): 145.1[M+1]$^+$.

Step 4: Synthesis of **Compound A6**

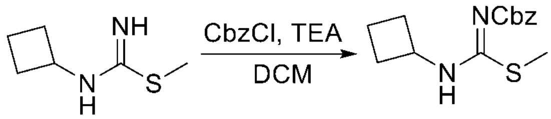


A6-4 A6

The crude product of **Compound A6-4** (2.00 g, 8.75 mmol) and dichloromethane (40 mL) were added to a reaction flask, to which triethylamine (5.60 g, 55.34 mmol) was then added, and the reaction mixture was cooled to 0 °C under nitrogen protection. Benzyl chloroformate (3.50 g, 20.52 mmol) was added dropwise. Then the reaction solution was diluted with water (100 mL) and extracted with dichloromethane (100 mL×3). The organic phases were combined and concentrated to give a residue. The residue was purified by flash column chromatography (ethyl acetate/petroleum ether=10%-20%) to give **Compound A6** as yellow solid (1.20 g, yield: 49%).

$^1$H NMR (400MHz, DMSO) $\delta$ 10.19-9.03(m, 1H), 7.38(d, *J*=3.9Hz, 5H), 5.05(s, 2H), 4.21(t, *J*=56.1Hz, 1H), 2.38(s, 3H), 2.23(d, *J*=23.6Hz, 2H), 2.11-1.96(m, 2H), 1.67(dd, J=20.0, 13.5Hz, 2H); LCMS (m/z): 279.1[M+1]$^+$.

Intermediate Synthesis Example 7: Synthesis of benzyl (E)-(((cyclopropylmethyl)amino)(methylthio)methylene)carbamate **(A7)**

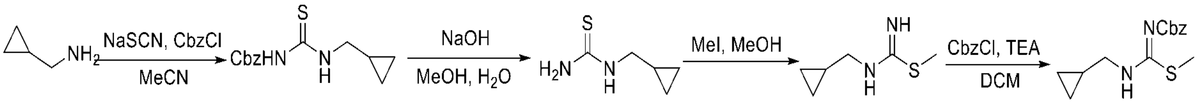


A7-1 A7-2 A7-3 A7-4 A7

**Compound A7** was synthesized according to the synthesis of **Compound A6** using cyclopropylmethylamine as a starting material. LCMS (m/z): 279.1[M+1]$^+$.

Intermediate Synthesis Example 8: Synthesis of tert-butyl *(E)*-((benzylamino)(methylthio)methylene)carbamate (**A8**)

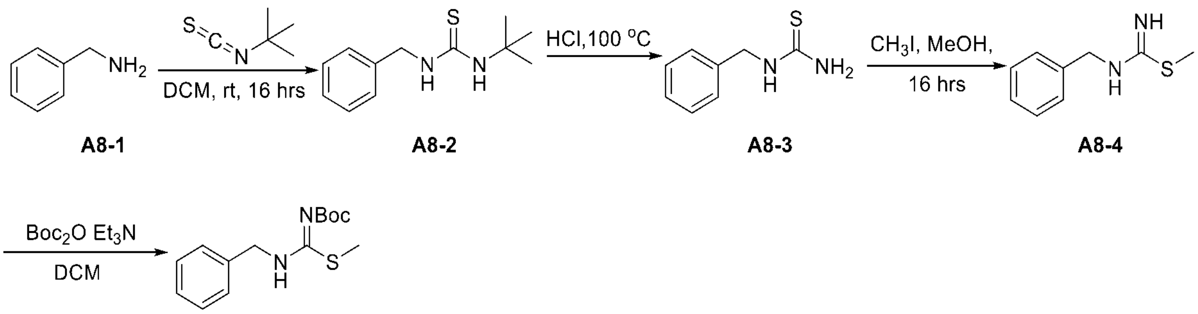


A8-1 A8-2 A8-3 A8-4

A8

**Compound A8** was synthesized with benzylamine as a starting material, and the first three steps of synthesis of **Compound A8-4** were conducted according to the synthesis procedures of **Compound A2.**

Step 4: Synthesis of tert-butyl (*E*)-((benzylamino)(methylthio)methylene)carbamate **(A8)**

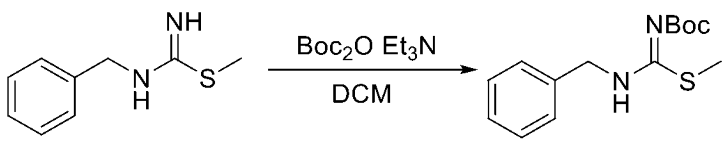


A8-4 A8

Crude **Compound A8-4** (2.10 g, 11.60 mmol) and dichloromethane (40 mL) were added into a reaction flask, to which 4-dimethylaminopyridine (130 mg, 1.06 mmol) and triethylamine (3.50 g, 34.59 mmol) were added under stirring. The temperature was lowered to 0 °C, and di-tert-butyl dicarbonate (3.00 g, 13.75 mmol) was slowly added dropwise. After addition was completed, the reaction was carried out at room temperature for 16 h. The reaction solution was then diluted with water (40 mL) and extracted with dichloromethane (50 mL×3). The organic phases were combined and concentrated to give crude product. The crude product was purified by flash column chromatography (ethyl acetate/petroleum ether=20%) to give **Compound A8** as white solid (1.70 g, yield: 52%). LCMS (m/z): 281.1[M+1]+.

Intermediate Synthesis Example 9: Synthesis of benzyl (*E*)-(10,10-dimethyl-9,9-diphenyl-8-oxo-2-thia-4-aza-9-dodecan-3-ylidene)carbamate **(A9)**

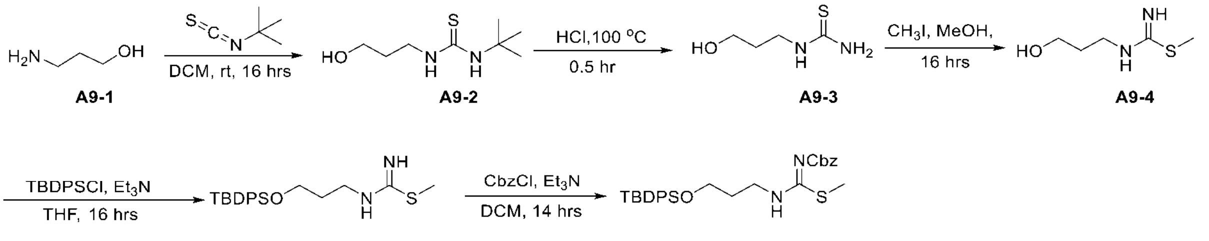


A9-1 A9-2 A9-3 A9-4 A9-5 A9

**Compound A9** was synthesized with 3-aminopropanol as a starting material, and the first three steps of synthesis of **Compound A9-4** were conducted according to the synthesis procedures of **Compound A2.**

Step 4: Synthesis of methyl (3-((tert-butyldiphenylsilyl)oxy)propyl)carbamimidothioate (**A9-5**)

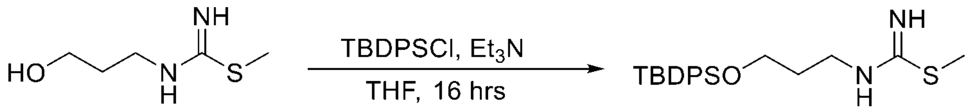


A9-4 A9-5

**Compound A9-4** (1.6 g, 10.79 mmol) and tetrahydrofuran (20 mL) were added to a reaction flask, to which triethylamine (3.27 g, 32.32 mmol) and tert-butyldiphenylchlorosilane (5.92 g, 21.54 mmol) were added under nitrogen protection, and the reaction mixture was stirred at room temperature for 16 h. Then the reaction was quenched with water (80 mL), extracted with ethyl acetate (30 mL×3), the organic phases were combined, dried over anhydrous sodium sulfate, and concentrated to give a crude product. The crude product was purified by flash column chromatography (ethyl acetate/petroleum ether=15%-50%) to give **Compound A9-5** (1.2 g, yield: 29%). LCMS (m/z): 387.2[M+1]+.

Step 5: Synthesis of benzyl (E)-(10,10-dimethyl-9,9-diphenyl-8-oxo-2-thia-4-aza-9-dodecan-3-ylidene)carbamate **(A9)**

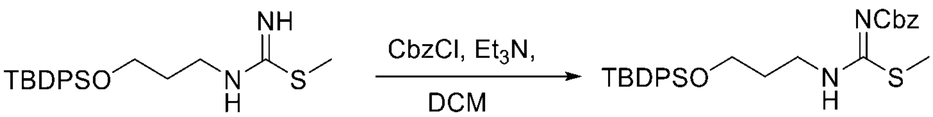


A9-5 A9

**Compound A9-5** (1.20 g, 3.10 mmol) and dichloromethane (20 mL) were added to a reaction flask, then to which triethylamine (0.94 g, 9.29 mmol) was added. The temperature was lowered to 0 °C, and then benzyl chloroformate (0.64 g, 3.76 mmol) was added dropwise. After addition was completed, the mixture was stirred overnight at room temperature. The reaction was quenched with water (20 mL), extracted with dichloromethane (30 mL×3), the organic phases were combined, dried over anhydrous sodium sulfate, and concentrated to give a crude product. The crude product was

subjected to flash column chromatography (ethyl acetate/petroleum ether=15% to 30%) to give **Compound A9** as white solid (1.00 g, yield: 62%). LCMS(m/z): 521.2[M+H]+.

Intermediate Synthesis Example 10: Synthesis of methyl (*E*)-3-(benzyloxy)carbonyl)imino)(methylthio)methyl)amino) propionate **(A10)**

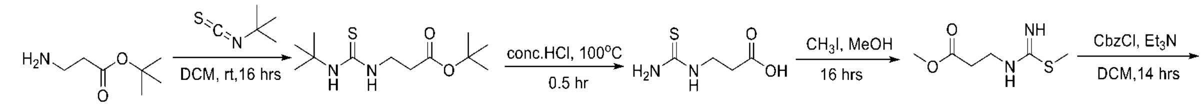


A10-1 A10-2 A10-3 A10-4

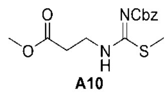

A10

**Compound A10** was synthesized with tert-butyl 3-aminopropionate as a starting material according to the synthesis procedures of **Compound A2.** LCMS(m/z): 311.1[M+1]+.

Intermediate Synthesis Example 11: Synthesis of (6S,8S)-8a-((E)-2,3-bis((benzyloxy)carbonyl)guanidino)-4-hydroxy-6-methoxy-2,2-dimethyl-9-oxooctahydro-5,8-(epoxymethano)[1,3]dioxolo[4,5-e]isobenzofuran-4-carboxylic acid (**B1**)

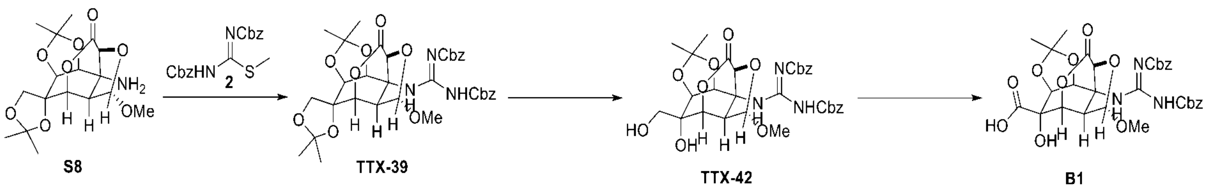

S8 TTX-39 TTX-42 B1

The starting material **S8** and **TTX-42** can be conducted by reference to the method in CN113956266A, which is incorporated herein by reference in its entirety.

Step 3: Synthesis of (6S,8S)-8a-((E)-2,3-bis((benzyloxy)carbonyl)guanidino)-4-hydroxy-6-methoxy-2,2-dimethyl-9-oxooctahydro-5,8-(epoxymethano)[1,3]dioxolo[4,5-e]isobenzofuran-4-carboxylic acid (**B1**)

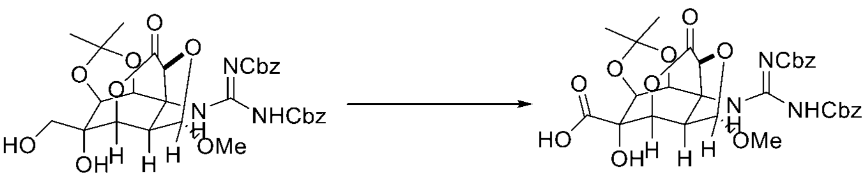

TTX-42 B1

**TTX-42** (160 mg, 0.25 mmol), acetonitrile (2 mL), 2,2,6,6-tetramethylpiperidine oxide (7 mg, 0.04 mmol) and 7% potassium dihydrogen phosphate aqueous solution (2 mL) were added to a reaction flask, which was heated to 40 °C, and 7.5% sodium hypochlorite solution (0.1 mL) and sodium chlorite (45.22 mg, 0.50 mmol) were added in about 10 min. The reaction was continued with stirring overnight at 40 °C. The reaction solution was cooled to room temperature, to which saturated aqueous sodium bicarbonate solution (1 mL) and aqueous sodium sulfite solution (1 mL) were added, extracted with ethyl acetate (5 mL×3), and the organic phases were combined and concentrated to give a crude product. The crude product was purified by flash column chromatography (methanol/dichloromethane=10%) to give **Compound B1** (100 mg, yield: 61%).

$^1$H NMR (400 MHz, $CDCl_3$) $\delta$ 11.49(s, 1H), 8.92(s, 1H), 7.44-7.27(m, 10H), 5.25-5.19(m, 3H), 5.18-5.09(m, 3H),

4.76(s, 1H), 4.65(d, *J*=1.5 Hz, 1H), 4.56(dd, *J*=8.1, 1.1 Hz, 1H), 3.98(t, *J*=1.5 Hz, 1H), 3.28(s, 3H), 2.10(s, 1H), 1.44(s, 3H), 1.27(d, *J*=4.8 Hz, 3H); LCMS (m/z): 656.2[M+1]$^+$.

Intermediate Synthesis Example 12: Synthesis of (6S,8S)-8a-((E)-2,3-bis((benzyloxy)carbonyl)guanidino)-4-hydroxy-6-methoxy-2,2-dimethyl-9-oxooctahydro-5,8-(epoxymethano)[1,3]dioxolo[4,5-e]isobenzofuran-4-carbaldehyde **(B2)**

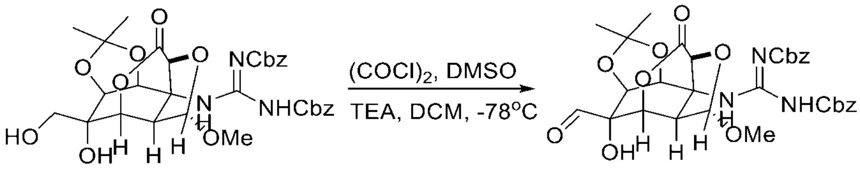


**TTX-42** **B2**

Anhydrous dichloromethane (2 mL) was added to a 10 mL reaction flask, which was purged with nitrogen three times, oxalyl chloride (30 mg, 0.23 mmol) was added, the temperature was lowered to -78 °C, and dimethyl sulfoxide (37 mg, 0.47 mmol) was added dropwise. After the addition was completed, the reaction mixture was stirred for 30 min, and a solution of **TTX-42** (100 mg, 0.16 mmol)/dichloromethane (0.5 mL) was added dropwise. After the addition was completed, the reaction mixture was stirred at -78 °C for 25 min, and triethylamine (79 mg, 0.78 mmol) was added dropwise. After addition was completed, the reaction was carried out at room temperature for 2 h. HPLC was used to monitor the complete reaction of the starting materials. Water (10 mL) was added to the reaction solution, which was extracted with dichloromethane (10 mL×3). The organic phases were combined, washed with saturated sodium chloride solution (10 mL), dried over anhydrous sodium sulfate, and concentrated to give a crude product. The crude product was purified by flash column chromatography (ethyl acetate/petroleum ether=0-60%) to give **Compound B2** as white solid (52 mg, yield: 51%).

$^1$H NMR (400 MHz, $CDCl_3$) $\delta$ 11.85-11.36(m, 1H), 7.46-7.30(m, 6H), 7.26-7.11(m, 4H), 6.24-5.71(m, 1H), 5.48-5.30(m, 1H), 5.27-4.93(m, 5H), 4.86-4.74(m, 1H), 4.71-4.61(m, 1H), 4.58-4.29(m, 1H), 4.21-4.09(m, 1H), 3.36-3.21(m, 3H), 1.48-1.35(m, 3H), 1.34-1.19(m, 3H); LCMS (m/z): 640.2[M+1]$^+$.

Intermediate Synthesis Example 13: Synthesis of (6S,8S)-8a-((E)-2,3-bis((benzyloxy)carbonyl)guanidino)-4-hydroxy-6-methoxy-2,2-dimethyl-9-oxooctahydro-5,8-(epoxymethano)[1,3]dioxolo[4,5-e]isobenzofuran-4-methylamine **(B3)**

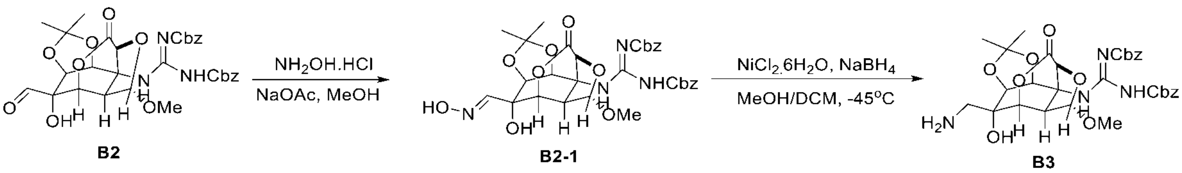


**B2** **B2-1** **B3**

Step 1: Synthesis of (6S,8S)-8a-((E)-2,3-bis((benzyloxy)carbonyl)guanidino)-4-hydroxy-6-methoxy-2,2-dimethyl-9-oxooctahydro-5,8-(epoxymethano)[1,3]dioxolo[4,5-e]isobenzofuran-4-carboxaldehyde oxime (**B2-1**)

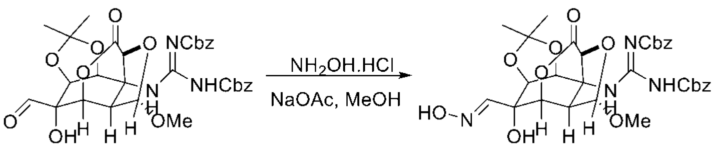


**B2** **B2-1**

**Compound B2** (700 mg, 1.09 mmol), methanol (50 mL) and hydroxylamine hydrochloride (167.40 mg, 2.41 mmol) were added to a reaction flask, and sodium acetate (134.60 mg, 1.64 mmol) was added in an ice bath. After addition was completed, the reaction solution was raised to room temperature and the reaction was carried out for 48 h. HPLC was

used to monitor complete reaction of the raw materials. The reaction solution was concentrated and dried to give a residue. Ethyl acetate (30 mL) was added to the residue, which was filtered, and the filter cake was washed with ethyl acetate. The filtrate was concentrated and dried to give a crude product. The crude product was purified by flash column chromatography (ethyl acetate/petroleum ether=0-50%) to give **Compound B2-1** as white solid (666 mg, yield: 93%).

$^1$H NMR (400 MHz, $CDCl_3$) $\delta$ 11.58(s, 1H), 8.92(s, 1H), 7.90(s, 1H), 7.51(d, J=8.3 Hz, 1H), 7.40-7.29(m, 10H), 5.23-5.18(m, 3H), 5.17-5.11(m, 3H), 4.63(s, 1H), 4.56(s, 1H), 4.43-4.37(m, 1H), 4.11(s, 1H), 4.00(s, 1H), 3.27(s, 3H), 1.41(s, 3H), 1.22(s, 3H); LCMS (m/z): 655.2$[M+1]^+$.

Step 2: Synthesis of **Compound B3**

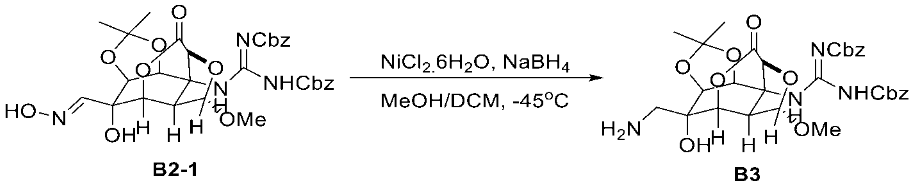


Nickel chloride hexahydrate (752 mg, 3.16 mmol) was dissolved in methanol (32 mL) and dichloromethane (8 mL), **Compound B2-1** (828 mg, 1.27 mmol) was added, the reaction solution was cooled to -45 °C under nitrogen protection, then sodium borohydride (100 mg) was added. Stirring was conducted until the reaction was initiated (the color of the reaction solution became darker with a large amount of bubbles generated), and then the remaining sodium borohydride (187 mg) was added in batches. After addition was completed, the reaction was continued for 30 min. HPLC was used to monitor complete reaction of the raw materials. Acetic acid (2 mL) was added to the reaction solution, the cooling bath was removed, the mixture was stirred until the color of the solution became pale green, then dichloromethane (20 mL) was added, and then saturated sodium bicarbonate solution was slowly added until pH=8. Filtration was conducted and the filter cake was slurried with dichloromethane/methanol (10/1, 30 mL×2) and filtered. The filtrate was combined and separated, the aqueous phase was extracted with dichloromethane/methanol (10/1, 20 mL×2), then all the organic phases were combined, dried over anhydrous sodium sulfate and filtered, and the filtrate was concentrated and dried to give a crude product. The crude product was purified by flash column chromatography (MeOH/DCM=0-10%) to give **Compound B3** as white solid (558 mg, yield: 67.4%).

$^1$H NMR (400 MHz, $CDCl_3$) $\delta$ 11.53(s, 1H), 8.92(s, 1H), 7.43-7.30(m, 10H), 5.24-5.19(m, 2H), 5.16(d, *J*=8.9 Hz, 2H), 5.12-5.08(dd, *J*=6.1, 4.1 Hz, 2H), 4.61(d, *J=1.5* Hz, 1H), 4.42(s, 1H), 4.33(d, *J*=8.3 Hz, 1H), 3.91(s, 1H), 3.27(s, 3H), 3.24(s, 1H), 2.91(d, *J*=13.5 Hz, 1H), 1.38(s, 3H), 1.24(d, J=8.2 Hz, 6H). LCMS (m/z): 641.2$[M+1]^+$.

Synthesis of the Target Compound:

Synthesis of the target compound is carried out according to the following synthetic route 1, 2, 3 or 4:

Synthesis Route 1:

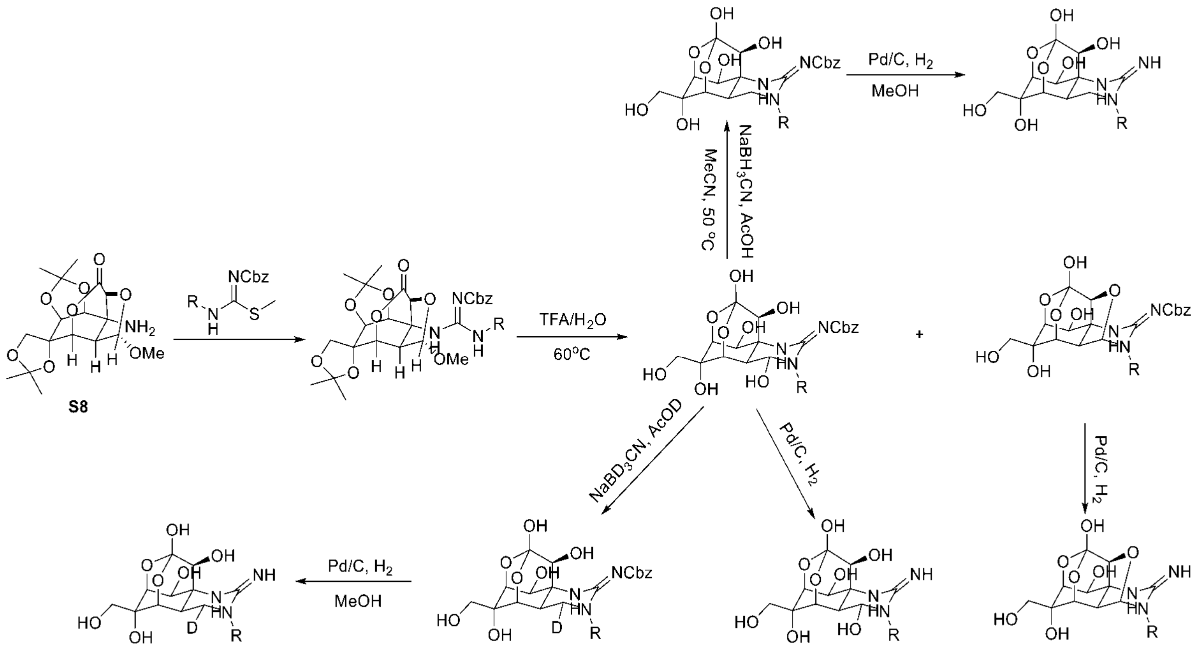


S8

Synthesis Route 2:

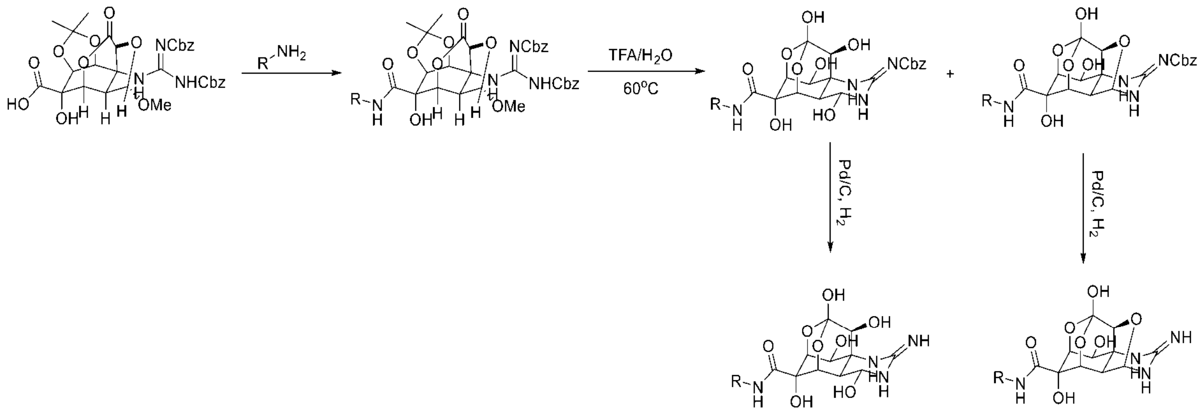


Synthesis Route 3:

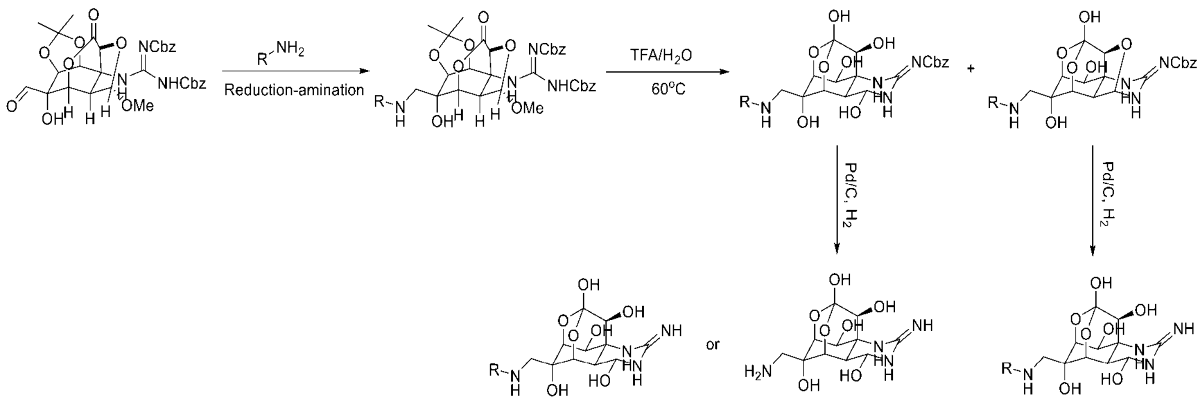


Synthesis Route 4:

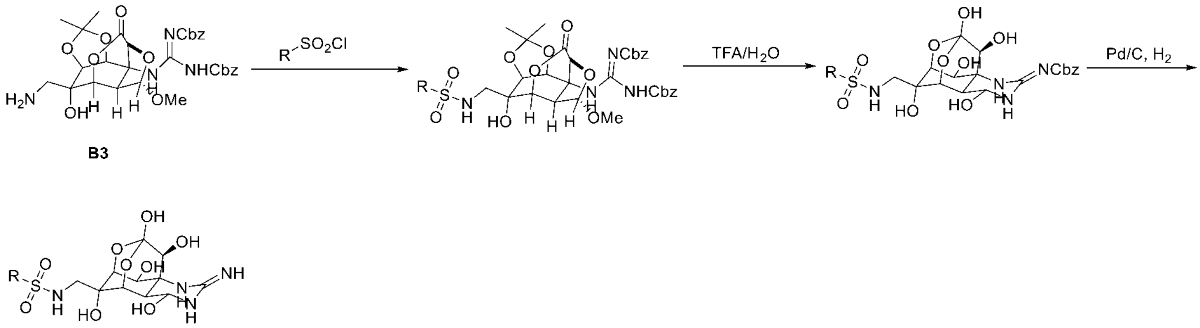

B3

wherein R represents any suitable group at the corresponding position herein.

Example 1: Synthesis of benzyl ((4R,4aR,5R,6S,7S,9S,10S,10aR,11S,E)-4,6,9,10,11-pentahydroxy-6-(hydroxymethyl)octahydro-5,9-epoxy-7,10a-methanooxocino[4,5-d]pyrimidin-2(1H)-ylidene)carbamate **(Compound 1)** and benzyl ((4S,5aS,6S,8R,9S,10S,11S,11aR,12R,E)-6,9,11-trihydroxy-9-(hydroxymethyl)octahydro-4,8,11a-(epimethanetriyl)-6,10-epoxyoxocino[4,3-f][1,3,5]oxadiazepin-2(1H)-ylidene)carbamate **(Compound 2)**

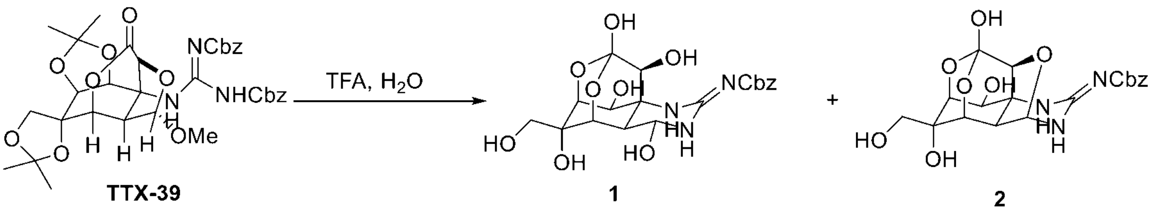

TTX-39 1 + 2

**TTX-39** (850 mg, 1.25 mmol), water (12 mL) and trifluoroacetic acid (12 mL) were added to a reaction flask, which was heated to 60 °C under nitrogen protection, and the reaction was carried out overnight. HPLC was used to monitor complete reaction of the raw materials, and the reaction solution was concentrated to give a residue. The residue was purified by flash column chromatography (methanol/dichloromethane=0-10%) to give **Compound 1** (368 mg, yield: 65%) and **Compound 2** (150 mg, yield: 28%) as white solid, total yield: 93%.

**Compound 1:**

$^1$H NMR (400 MHz, MeOD) $\delta$ 7.45-7.33(m, 5H), 5.69(t, *J*=8.7 Hz, 1H), 5.29(s, 2H), 4.24(d, *J*=7.9 Hz, 1H), 4.18(s, 1H), 4.06-3.95(m, 4H), 2.40(d, *J*=9.4 Hz, 1H); LCMS (m/z): 454.1[M+1]$^+$.

**Compound 2:**

$^1$H NMR (400 MHz, MeOD) $\delta$ 7.42-7.31(m, 5H), 5.40(s, 1H), 5.18(s, 2H), 4.54(d, *J*=2.0 Hz, 1H), 4.49(s, 1H), 4.21(dd, *J*=2.8, 1.4 Hz, 1H), 4.05(s, 1H), 3.97(d, *J*=11.5 Hz, 1H), 3.91(d, *J*=*11.5* Hz, 1H), 2.81(d, *J*=2.8 Hz, 1H); LCMS (m/z): 436.1[M+1]$^+$.

Example 2: Synthesis of (4S,5aS,6S,8R,9S,10S,11S,11aR,12R)-9-(hydroxymethyl)-2-iminooctahydro-4,8,11a-(epimethanetriyl)-6,10-epoxyoxocino[4,3-f][1,3,5]oxadiazepine-6,9,11(5aH)-triol **(Compound 3)**

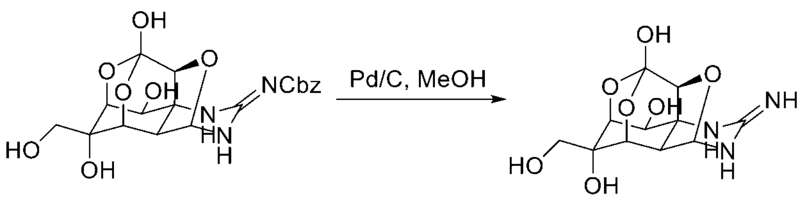

2 3

**Compound 2** (30 mg, 0.07 mmol), methanol (6 mL) and 10% palladium-carbon (5 mg) were added to a reaction flask. The reaction mixture was purged hydrogen three times, and hydrogenated by a hydrogen balloon at 25°C with stirring for 3 h. HPLC was used to monitor complete reaction. The reaction solution was filtered, the filter cake was washed with methanol (200 mL), and the filtrate was concentrated to give **Compound** 3 as white solid (8 mg, yield: 38%).

$^1$H NMR (400 MHz, $D_2O$) $\delta$ 5.36(s, 1H), 4.46(d, *J*=2.1 Hz, 1H), 4.42(s, 1H), 4.19(s, 1H), 4.01(s, 1H), 3.81(d, *J*=7.2 Hz, 1H), 3.76(d, *J*=12.2 Hz, 1H), 2.78(d, *J*=2.9 Hz, 1H); LCMS (m/z): 302.1[M+1]$^+$.

Example 3: Synthesis of (4R,4aR,5R,6S,7S,9S,10S,10aR,11S)-6-(hydroxymethyl)-2-iminooctahydro-5,9-epoxy-7,10a-methanooxocino[4,5-d]pyrimidine-4-d-6,9,10,11(10H)-tetraol **(Compound 4)**

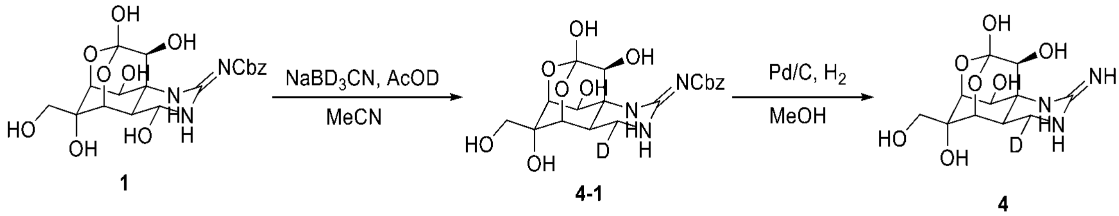


1 4-1 4

Step 1: Synthesis of benzyl ((4R,4aR,5R,6S,7S,9S,10S,10aR,11S,Z)-6,9,10,11-tetrahydroxy-6-(hydroxymethyl)octahydro-5,9-epoxy-7,10a-methanooxocino[4,5-d]pyrimidin-2(1H)-ylidene-4-d)carbamate **(Compound 4-1)**

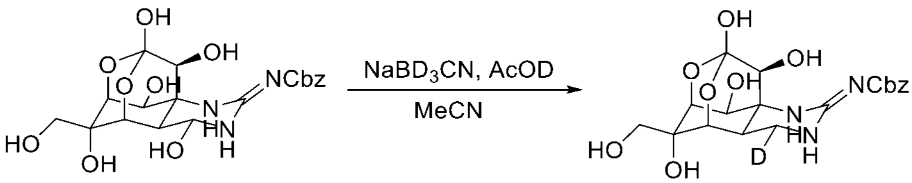


1 4-1

**Compound 1** (100 mg, 0.22 mmol), anhydrous deuterated acetonitrile (3 mL) and deuterated acetic acid (2.5 mL) were added to a reaction flask, to which was added deuterated sodium cyanoborohydride (73 mg, 1.1 mmol) was added under stirring. The reaction was carried out for 8 h under 50 °C. HPLC was used to monitor incomplete reaction of the raw materials. Deuterated sodium cyanoborohydride (73 mg, 1.1 mmol) was added, the reaction was continued for about 48 h, and about 20% of the raw materials remained. The reaction solution was concentrated to give a residue, which was slurried with diethyl ether (15 mL), filtered to remove part of acetic acid, and the filter cake was collected and purified by high performance liquid chromatography (welch xtimate C18×30 mm×150 mm, 10μm; flow rate: 30 mL/min (0.1% $NH_4OAc$, ACN)) to give **Compound 4-1** (39 mg, yield: 40%).

$^1$H NMR (400 MHz, MeOD) δ 7.45-7.30(m, 5H), 5.18(s, 2H), 4.44-3.73(m, 7H), 2.50(d, J=12.4 Hz, 1H); HRMS (ESI +): $C_{19}H_{22}DN_3O_9$ ([M+H]$^+$): calculated: 439.15; measured: 439.21.

Step 2: Synthesis of **Compound 4**

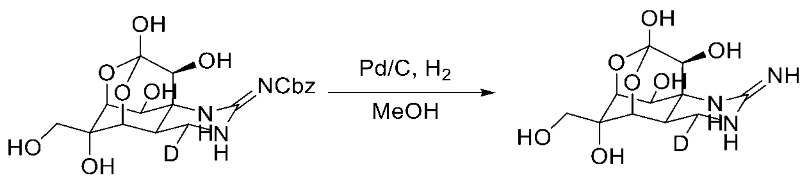


4-1 4

According to the synthesis procedures of Example 2, **Compound 4** as white solid was obtained (26 mg, yield: 88%).

$^{1}H$ NMR (400 MHz, $D_2O$) $\delta$ 4.11(d, $J$=1.3 Hz, 1H), 3.96(s, 1H), 3.93(s, 1H), 3.90-3.87(m, 2H), 3.74(d, $J$=14.9 Hz, 1H), 3.67(d, $J$=8.7 Hz, 1H), 2.49-2.36(m, 1H).

$^{13}C$ NMR (4% $CD_3COOD$/96% $D_2O$, 100 MHz) $\delta$ 154.5(C), 108.6(C), 79.9(CH), 77.0(CH), 74.7(CH), 73.0(CH), 71.9(CH), 70.5(C), 69.3(CH), 62.8(C), 55.4(CH); HRMS (ESI +): calculated $C_{11}H_{16}DN_3O_7$ ($[M+H]^+$): 305.11, measured: 305.21.

Example 4: Synthesis of benzyl ((4aR,5R,6S,7S,9S,10S,10aR,11S,Z)-6,9,10,11-tetrahydroxy-6-(hydroxymethyl)octahydro-5,9-epoxy-7,10a-methanooxocino[4,5-d]pyrimidin-2(1H)-ylidene)carbamate **(Compound 5)**

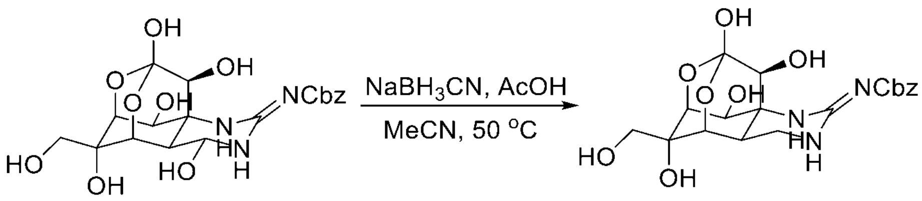


1 5

**Compound 1** (500 mg, 1.10 mmol), acetonitrile (10 mL) and glacial acetic acid (10 mL) were added to a reaction flask, to which sodium cyanoborohydride (0.69 g, 11.00 mmol) was added in batches under stirring, and the reaction was carried out for 16 h under 50 °C. Most of the solvent was concentrated, and the residue was prepared and isolated to give Compound 5 as colorless oil (135 mg, yield: 28%).

$^{1}H$ NMR (400MHz, MeOD) $\delta$ 7.44-7.25(m, 5H), 5.15(s, 2H), 4.29-3.74(m, 7H), 3.29-3.24(m, 1H), 2.48(d, $J$=6.6Hz, 1H); LCMS (m/z): 438.1$[M+1]^+$.

Example 5: Synthesis of (4aR,5R,6S,7S,9S,10S,10aR,11S)-6-(hydroxymethyl)-2-iminooctahydro-5,9-epoxy-7,10a-methanooxocino[4,5-d]pyrimidine-6,9,10,11(10H)-tetraol **(Compound 6)**

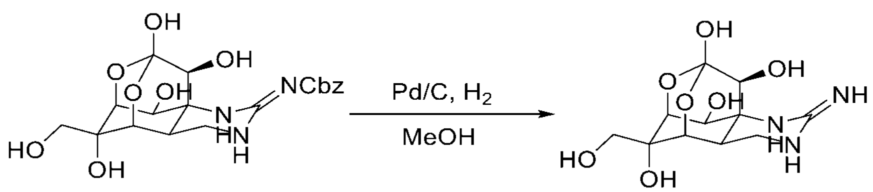


5 6

According to the synthesis method of Example 2, **Compound 6** was obtained as white solid (30 mg, yield: 100%).

$^{1}H$ NMR (400MHz, $D_2O$) $\delta$ 4.17(s, 1H), 4.02(s, 1H), 3.98(s, 1H), 3.93(d, J=4.4Hz, 2H), 3.92(s, 1H), 3.77(t, $J$=11.8Hz, 1H), 3.32-3.28(m, 1H), 2.52-2.46(m, 1H); LCMS (m/z): 304.1$[M+1]^+$.

Example 6: Synthesis of benzyl ((4R,4aR,5R,6S,7S,9S,10S,10aR,11S,E)-4,6,9,10,11-pentahydroxy-6-(hydroxymethyl)-3-methyloctahydro-5,9-epoxy-7,10a-methanooxocino[4,5-d]pyrimidin-2(1H)-ylidene)carbamate **(Compound 7)** and benzyl ((4S,5aS,6S,8R,9S,10S,11S,11aR,12R,E)-6,9,11-trihydroxy-9-(hydroxymethyl)-3-methyloctahydro-4,8,11a-(epimethanetriyl)-6,10-epoxyoxocino[4,3-f][1,3,5]oxadiazepin-2(1H)-ylidene)carbamate **(Compound 8)**

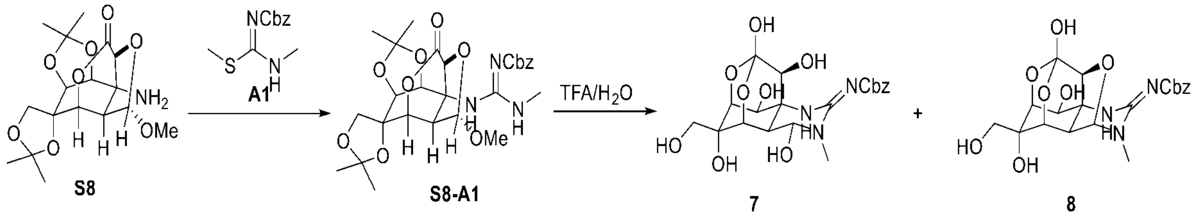


S8 S8-A1 7 8

Step 1: Synthesis of **Compound S8-A1**

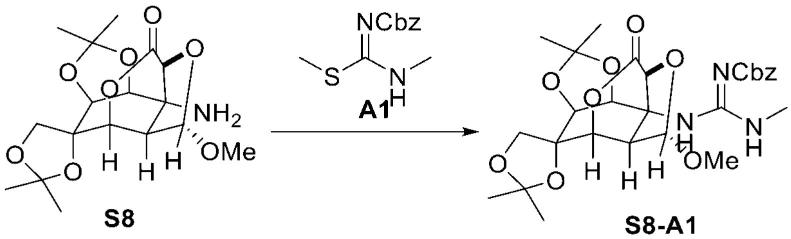


S8 S8-A1

**Compound A1** (0.62 g, 2.60 mmol), anhydrous acetonitrile (40 mL), **S8** (0.80 g, 2.15 mmol) and triethylamine (1.09 g, 10.77 mmol) were added to a reaction flask. Under nitrogen protection, the mixture was cooled to 0 °C, and silver trifluoromethanesulfonate (1.1 g, 4.31 mmol) was added. The reaction solution was stirred at room temperature for 16 h. The reaction solution was diluted with water (40 mL) and extracted with ethyl acetate (50 mL×3). The organic phases were combined and concentrated to give a residue. The residue was purified by flash column chromatography (ethyl acetate/petroleum ether=10%-25%) to give **Compound S8-A1** as off-white solid (0.50 g, yield: 41%). LCMS (m/z): 562.2[M+1]+.

Step 2: Synthesis of **Compound 7** and **Compound 8**

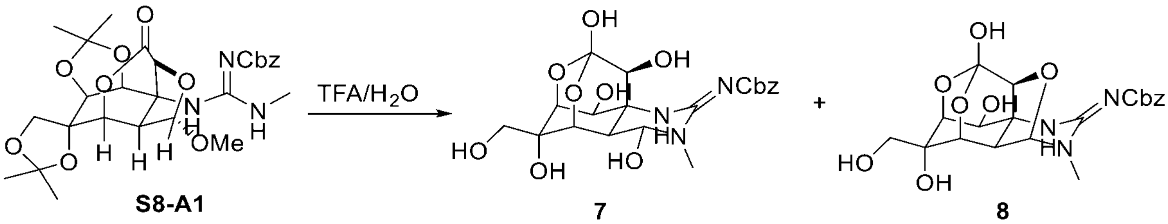


S8-A1 7 8

According to the synthesis procedures of Example 1, **Compound 7** as white solid (170 mg, yield: 36%) and **Compound 8** as white solid (130 mg, yield: 29%) were obtained, total yield: 65%.

**Compound 7:**

$^1$H NMR (400 MHz, MeOD) δ 7.54-7.20(m, 5H), 5.35-5.11(m, 3H), 4.19(t, *J*=7.2 Hz, 1H), 4.14(d, *J*=5.5 Hz, 1H), 4.05-3.98(m, 2H), 3.98-3.91(m, 2H), 3.12-3.04(m, 3H), 2.50-2.37(m, 1H); LCMS (m/z): 468.2[M+1]+.

**Compound 8:**

$^1$H NMR (400 MHz, MeOD) δ 7.48-7.17(m, 5H), 5.30(s, 1H), 5.10(s, 2H), 4.47(d, *J*=1.9 Hz, 1H), 4.38(s, 1H), 4.16(d, *J*=1.3 Hz, 1H), 4.00(s, 1H), 3.96(d, *J*=11.4 Hz, 1H), 3.90(d, *J*=11.4 Hz, 1H), 3.05(s, 3H), 2.73(d, *J*=3.0 Hz, 1H); LCMS (m/z): 450.1[M+1]+.

Example 7: Synthesis of (4R,4aR,5R,6S,7S,9S,10S,10aR,11S)-6-(hydroxymethyl)-2-imino-3-methyloctahydro-5,9-epoxy-7, l0a-methanooxocino[4,5-d]pyrimidine-4,6,9,10,11(10H)-pentaol (**Compound 9**)

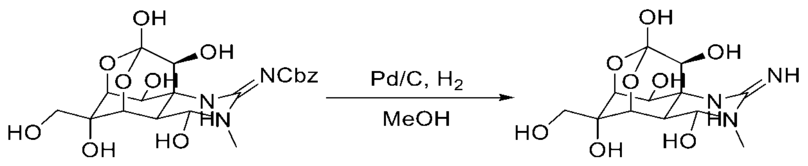


7 9

According to the synthesis procedures of Example 2, **Compound 9** was obtained as white solid (9.0 mg, yield: 90%). $^1H$ NMR (400 MHz, MeOD) $\delta$ 5.55-5.05(m, 1H), 4.11-4.06(m, 1H), 4.06-3.98(m, 1H), 3.97-3.82(m, 4H), 2.91(s, 3H), 2.36-2.12(m, 1H); LCMS (m/z): 334.1[M+1]$^+$.

Example 8: Synthesis of benzyl ((4aR,5R,6S,7S,9S,10S,10aR,11S,E)-6,9,10,11-tetrahydroxy-6-(hydroxymethyl)-3-methyloctahydro-5,9-epoxy-7,10a-methanooxocino[4,5-d]pyrimidin-2(1H)-ylidene)carbamate **(Compound 10)**

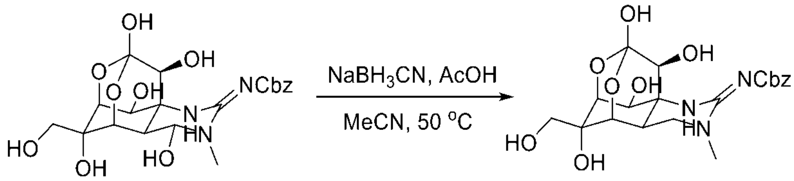


7 10

According to the synthesis procedures of Example 4, **Compound 10** was obtained as white solid (19.5 mg, yield: 12%). $^1H$ NMR(400MHz, MeOD) $\delta$ 7.39-7.31(m, 5H), 5.09(s, 2H), 4.40(s, 1H), 4.22-4.08(m, 2H), 3.97(s, 2H), 3.74(q, *J*=11.6Hz, 1H), 3.55-3.47(m, 1H), 3.11(d, *J*=12.6Hz, 1H), 3.04(s, 3H), 2.51(d, *J*=6.7Hz, 1H); LCMS (m/z): 452.2[M+1]$^+$.

Example 9: Synthesis of (4aR,5R,6S,7S,9S,10S,10aR,11S)-6-(hydroxymethyl)-2-imino-3-methyloctahydro-5,9-epoxy-7,10a-methanooxocino[4,5-d]pyrimidine-6,9,10,11(10H)-tetraol **(Compound 11)**

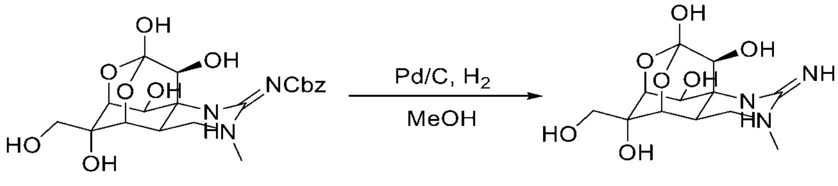


10 11

According to the synthesis procedures of Example 2, **Compound 11** was obtained as white solid (12.0 mg, yield: 88%). $^1H$ NMR (400 MHz, MeOD) $\delta$ 4.12(s, 1H), 4.04(s, 2H), 3.99(d, *J*=7.8 Hz, 2H), 3.86-3.80(m, 1H), 3.78(d, *J*=4.8 Hz, 1H), 3.62-3.50(m, 1H), 3.09(d, *J*=6.5 Hz, 3H), 2.63(s, 1H); LCMS (m/z): 318.1[M+1]$^+$.

Example 10: Synthesis of (4S,5aS,6S,8R,9S,10S,11S,11aR,12R)-9-(hydroxymethyl)-2-imino-3-methyloctahydro-4,8,11a-(epimethanetriyl)-6,10-epoxyoxocino[4,3-f][1,3,5]oxadiazepine-6,9,11(5aH)-triol **(Compound 12)**

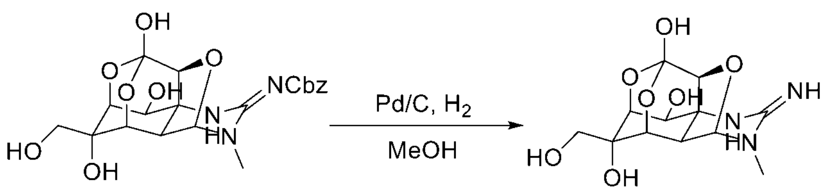


8 12

According to the synthesis procedures of Example 2, **Compound 12** was obtained as white solid (16 mg, yield: 91%). $^1$H NMR (400MHz, MeOD) $\delta$ 5.36(s, 1H), 4.48(d, *J*=1.9Hz, 1H), 4.37(s, 1H), 4.17(s, 1H), 4.01(s, 1H), 3.97(d, J=11.4Hz, 1H), 3.90(d, *J*=11.4Hz, 1H), 3.12(s, 3H), 2.80(d, *J*=2.9Hz, 1H); LCMS (m/z): 316.1[M+1]$^+$.

Example 11: Synthesis of benzyl ((4R,4aR,5R,6S,7S,9S,10S,10aR,11S,E)-3-ethyl-4,6,9,10,11-pentahydroxy-6-(hydroxymethyl)octahydro-5,9-epoxy-7,10a-methanooxocino[4,5-d]pyrimidin-2(1H)-ylidene)carbamate (**Compound 13**) and benzyl ((4S,5aS,6S,8R,9S,10S,11S,11aR,12R,E)-3-ethyl-6,9,11-trihydroxy-9-(hydroxymethyl)octahydro-4,8,11a-(epimethanetriyl)-6,10-epoxyoxocino[4,3-f][1,3,5]oxadiazepin-2(1H)-ylidene)carbamate **(Compound 14)**

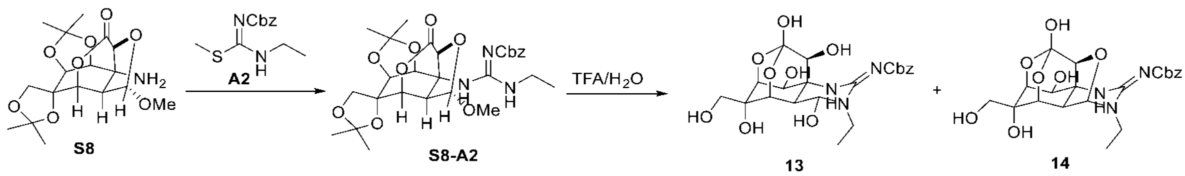


S8 S8-A2 13 14

Step 1: Synthesis of Compound S8-A2

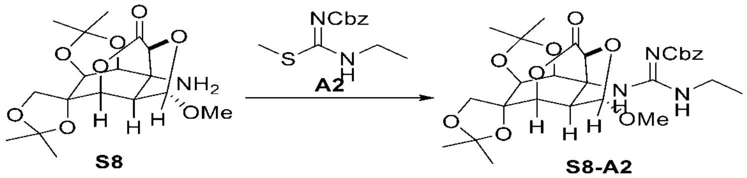


S8 S8-A2

According to the synthesis procedures of **Compound S8-A1** in Example 6, **Compound S8-A2** was obtained as off-white solid (0.75 g, yield: 48%). LCMS (m/z): 576.3[M+1]$^+$.

Step 2: Synthesis of **Compound 13** and **Compound 14**

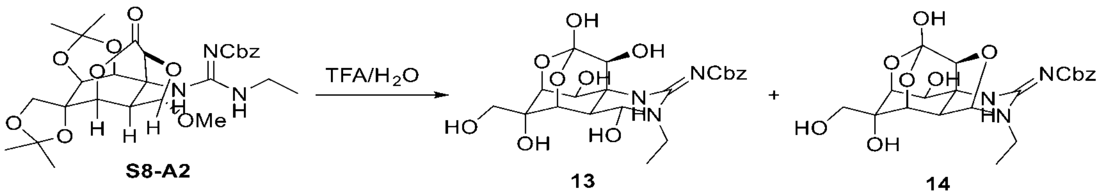


S8-A2 13 14

According to the synthesis procedures of Example 1, **Compound 13** as white solid (360 mg, yield: 58%) and **Compound 14** as white solid (120 mg, 20%) were obtained with a total yield of 78%.

**Compound 13:**

$^1$H NMR (400 MHz, MeOD) $\delta$ 7.42-7.21(m, 5H), 5.35(d, *J*=9.3 Hz, 1H), 5.11(s, 2H), 4.39(s, br, 1H), 4.14(s, 1H), 4.01(s, 2H), 3.94-3.64(m, 3H), 3.53-3.44(m, 1H), 2.34(d, *J*=9.3 Hz, 1H), 1.10(t, *J*=6.7 Hz, 3H); LCMS (m/z): 482.2[M+1]$^+$.

**Compound 14:**

$^1$H NMR (400 MHz, MeOD) $\delta$ 7.46-7.14(m, 5H), 5.37(s, 1H), 5.12(d, *J*=12.7 Hz, 2H), 4.46(d, *J*=1.9 Hz, 1H), 4.38(s, 1H), 4.18(s, 1H), 4.03-3.87(m, 3H), 3.75-3.62(m, 1H), 3.44-3.34(m, 1H), 2.66(d, *J*=2.8 Hz, 1H), 1.15(t, *J*=7.1 Hz, 3H); LCMS (m/z): 464.2[M+1]$^+$.

Example 12: Synthesis of (4R,4aR,5R,6S,7S,9S,10S,10aR,11S)-3-ethyl-6-(hydroxymethyl)-2-iminooctahydro-5,9-epoxy-7,10a-methanooxocino[4,5-d]pyrimidine-4,6,9,10,11(10H)-pentaol **(Compound 15)**

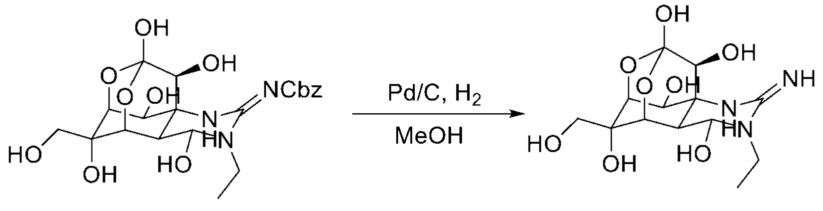


**13** **15**

According to the synthesis procedures of Example 2, **Compound 15** was obtained as white solid (16 mg, yield: 77%). LCMS (m/z): 348.2$[M+1]^+$.

Example 13: Synthesis of benzyl ((4aR,5R,6S,7S,9S,10S,10aR,11S,E)-3-ethyl-6,9,10,11-tetrahydroxy-6-(hydroxymethyl)octahydro-5,9-epoxy-7,10a-methanooxocino[4,5-d]pyrimidin-2(1H)-ylidene)carbamate **(Compound 16)**

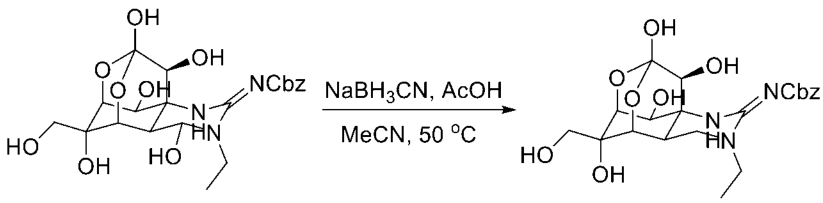


**13** **16**

According to the synthesis procedures of Example 4, **Compound 16** was obtained as white solid (24 mg, yield: 12%). $^1$H NMR (400 MHz, MeOD) $\delta$ 7.41-7.23(m, 5H), 5.10(s, 2H), 4.14(s, br, 3H), 4.05-3.79(m, 4H), 3.77-3.67(m, 1H), 3.43-3.32(m, 1H), 3.20-3.05(m, 1H), 2.47(dd, *J*=12.6, 5.4 Hz, 1H), 1.11(t, *J*=7.1 Hz, 3H); LCMS (m/z): 466.2$[M+1]^+$.

Example 14: Synthesis of (4aR,5R,6S,7S,9S,10S,10aR,11S)-3-ethyl-6-(hydroxymethyl)-2-iminooctahydro-5,9-epoxy-7,10a-methanooxocino[4,5-d]pyrimidine-6,9,10,11(10H)-tetraol **(Compound 17)**

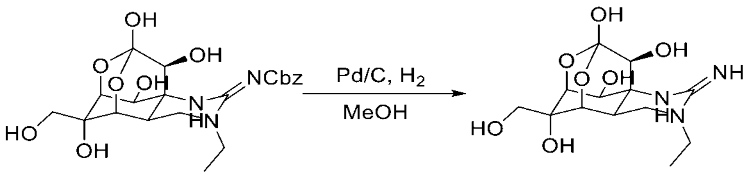


**16** **17**

According to the synthesis procedures of Example 2, **Compound 17** was obtained as white solid (12 mg, yield: 72%). $^1$H NMR (400 MHz, D2O) $\delta$ 4.08(s, 1H), 3.91(s, 4H), 3.84(d, J=8.8 Hz, 2H), 3.47-3.32(m, 1H), 3.30-3.16(m, 2H), 2.47(dd, J=12.6, 6.3 Hz, 1H), 1.10(s, 3H); LCMS (m/z): 332.1$[M+1]^+$.

Example 15: Synthesis of (4S,5aS,6S,8R,958,10S,11S,11aR,12R)-3-ethyl-9-(hydroxymethyl)-2-iminooctahydro-4,8,11a-(epimethanetriyl)-6,10-epoxyoxocino[4,3-f][1,3,5]oxadiazepine-6,9,11(5aH)-triol **(Compound 18)**

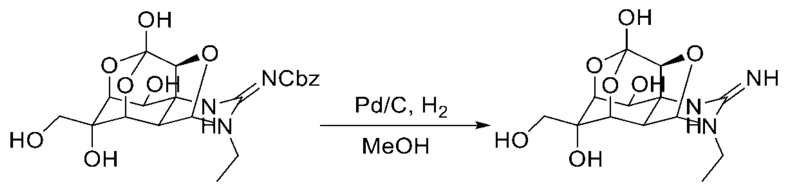


14 18

According to the synthesis procedures of Example 2, **Compound 18** was obtained as white solid (13 mg, yield: 99%).

$^1$H NMR (400 MHz, $D_2O$) $\delta$ 5.44(s, 1H), 4.46(d, $J$=1.9 Hz, 1H), 4.27(s, 1H), 4.13(s, 1H), 3.98-3.93(m, 2H), 3.86(d, $J$=12.1 Hz, 1H), 3.49-3.37(m, 2H), 2.78(d, $J$=2.9 Hz, 1H), 1.23-1.14(m, 3H); LCMS (m/z): 330.1[M+1]$^+$.

Example 16: Synthesis of benzyl ((4R,4aR,5R,6S,7S,9S,10S,10aR,11S,E)-3-cyclobutyl-4,6,9,10,11-pentahydroxy-6-(hydroxymethyl)octahydro-5,9-epoxy-7,10a-methanooxocino[4,5-d]pyrimidin-2(1H)-ylidene)carbamate **(Compound 19)** and benzyl ((4S,5aS,6S,8R,9S,10S,11S,11aR,12R,E)-3-cyclobutyl-6,9,11-trihydroxy-9-(hydroxymethyl)octahydro-4,8,11a-(epimethanetriyl)-6,10-epoxyoxocino[4,3-f][1,3,5]oxadiazepin-2(1H)-ylidene)carbamate **(Compound 20)**

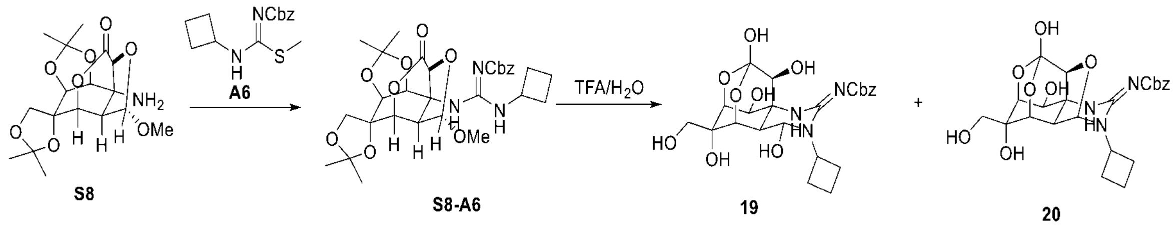


S8 S8-A6 19 20

Step 1: Synthesis of **Compound S8-A6**

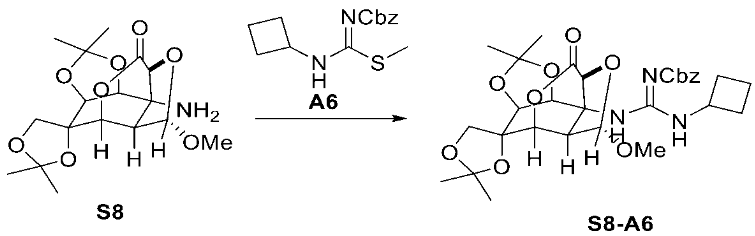


S8 S8-A6

According to the synthesis procedures of **Compound S8-A1** in Example 6, **Compound S8-A6** was obtained as off-white solid (380 mg, yield: 66%). LCMS (m/z): 602.3[M+1]$^+$.

Step 2: Synthesis of **Compound 19** and **Compound 20**

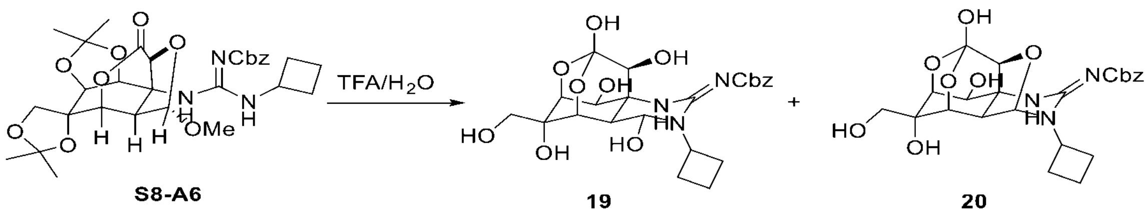


S8-A6 19 20

According to the synthesis procedures of Example 1, **Compound 19** as white solid (160 mg, yield: 50%) and

**Compound 20** as white solid (65 mg, yield: 21%) were obtained with total yield of 71%.

**Compound 19:**

$^1$HNMR (400MHz, MeOD) $\delta$ 7.49-7.32(m, 5H), 5.62(s, 1H), 5.31(s, 2H), 4.66-4.36(m, 1H), 4.31-4.18(m, 2H), 4.09(s, 1H), 4.02(s, 2H), 4.00-3.95(m, 1H), 2.49(d, *J*=9.2Hz, 1H), 2.47-2.31(m, 3H), 2.14-1.94(m, 1H), 1.82-1.61(m, 2H); LCMS (m/z): 508.2[M+1]$^+$.

**Compound 20:**

$^1$H NMR (400MHz, MeOD) $\delta$ 7.43-7.24(m, 5H), 5.75(s, 1H), 5.11(s, 2H), 4.97-4.94(m, 1H), 4.46(d, *J*=2.0Hz, 1H), 4.37(s, 1H), 4.23(s, 1H), 4.01(s, 1H), 3.98(d, J=11.5 Hz, 1H), 3.91(d, *J*=11.5 Hz, 1H), 2.62(d, *J*=2.9Hz, 1H), 2.28-2.07(m, 4H), 1.77-1.57(m, 2H); LCMS (m/z): 490.2[M+1]$^+$.

Example 17: Synthesis of (4R,4aR,5R,6S,7S,9S,10S,10aR,11S)-3-cyclobutyl-6-(hydroxymethyl)-2-iminooctahydro-5,9-epoxy-7,10a-methanooxocino[4,5-d]pyrimidine-4,6,9,10,11(10H)-pentaol (**Compound 21**)

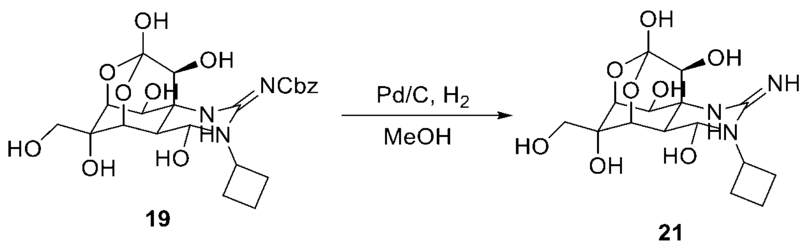


19 21

According to the synthesis procedures of Example 2, **Compound 21** was obtained as white solid (28 mg, yield: 68%). LCMS (m/z): 374.2[M+1]$^+$.

Example 18: Synthesis of benzyl ((4aR,5R,6S,7S,9S,10S,10aR,11S,E)-3-cyclobutyl-6,9,10,11-tetrahydroxy-6-(hydroxymethyl)octahydro-5,9-epoxy-7,10a-methanooxocino[4,5-d]pyrimidin-2(1H)-ylidene)carbamate **(Compound 22)**

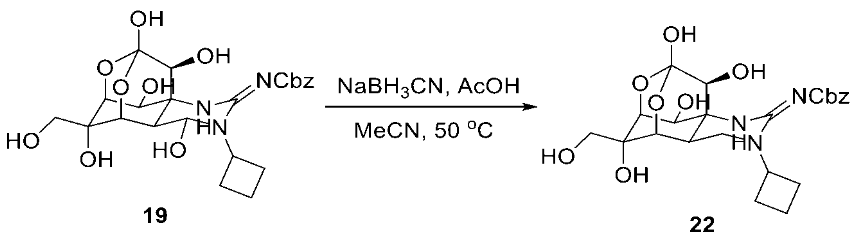


19 22

According to the synthesis procedures of Example 4, **Compound 22** was obtained as white solid (11 mg, yield: 8%).

$^1$H NMR (400MHz, MeOD) $\delta$ 7.44-7.22(m, 5H), 5.22-5.12(m, 1H), 5.10(s, 2H), 4.57(s, 2H), 4.12-3.87(m, 4H), 3.81(s, 1H), 3.47-3.32(m, 1H), 2.48-2.30(m, 1H), 2.28-2.15(m, 2H), 2.12-2.00(m, 2H), 1.72-1.57(m, 2H); LCMS (m/z): 492.2[M+1]$^+$.

Example 19: Synthesis of (4aR,5R,6S,7S,9S,10S,10aR,11S)-3-cyclobutyl-6-(hydroxymethyl)-2-iminooctahydro-5,9-epoxy-7,10a-methanooxocino[4,5-d]pyrimidine-6,9,10,11(10H)-tetraol (**Compound 23**)

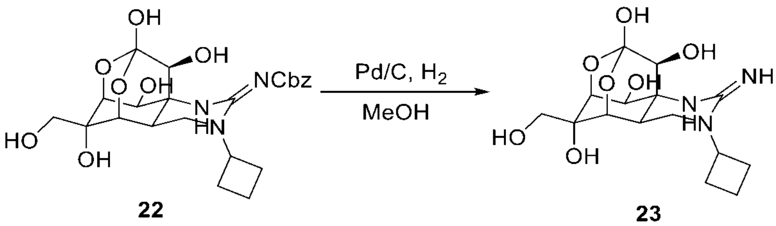


**22** **23**

According to the synthesis procedures of Example 2, **Compound 23** was obtained as white solid (7.0 mg, yield: 89%).

$^1H$ NMR (400MHz, $D_2O$) $\delta$ 4.24-4.13(m, 1H), 4.12(s, 1H), 4.00-3.94(m, 2H), 3.94-3.88(m, 3H), 3.84(t, $J$=12.1Hz, 1H), 3.40(dd, $J$=11.5, 6.3Hz, 1H), 2.44(dd, $J$=12.5, 6.3Hz, 1H), 2.34-2.07(m, 4H), 1.74-1.56(m, 2H); LCMS (m/z): 358.2 $[M+1]^+$.

Example 20: Synthesis of (4S,5aS,6S,8R,9S,10S,11S,11aR,12R)-3-cyclobutyl-9-(hydroxymethyl)-2-iminooctahydro-4,8,11a-(epimethanetriyl)-6,10-epoxyoxocino[4,3-f][1,3,5]oxadiazepine-6,9,11(5aH)-triol **(Compound 24)**

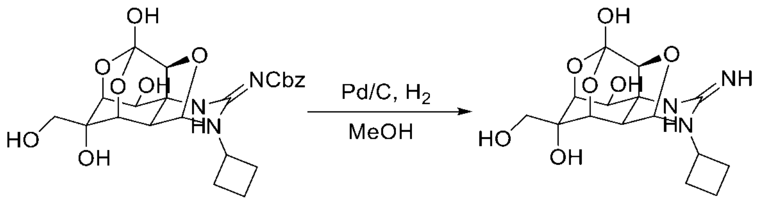


**20** **24**

According to the synthesis procedures of Example 2, **Compound 24** was obtained as white solid(45 mg, yield: 100%).

$^1H$ NMR (400MHz, $D_2O$) $\delta$ 5.94(s, 1H), 4.65(d, $J$=1.8Hz, 1H), 4.57(s, 1H), 4.41(s, 1H), 4.35-4.25(m, 1H), 4.20(s, 1H), 4.07(d, $J$=12.2Hz, 1H), 4.00(s, 1H), 2.99(d, $J$=3.0Hz, 1H), 2.33(d, $J$=10.3 Hz, 4H), 1.89-1.74(m, 2H); LCMS (m/z): 356.1$[M+1]^+$.

Example 21: Synthesis of benzyl ((4R,4aR,5R,6S,75,9S,10S,10aR,11S,E)-3-(cyclopropylmethyl)-4,6,9,10,11-pentahydroxy-6-(hydroxymethyl)octahydro-5,9-epoxy-7,10a-methanooxocino[4,5-d]pyrimidin-2(1H)-ylidene)carbamate (**Compound 25**) and benzyl ((4S,5aS,6S,8R,9S,10S,11S,11aR,12R,E)-3-(cyclopropylmethyl)-6,9,11-trihydroxy-9-(hydroxymethyl)octahydro-4,8,11a-(epimethanetriyl)-6,10-epoxyoxocino[4,3-f][1,3,5]oxadiazepin-2(1H)-ylidene)carbamate **(Compound 26)**

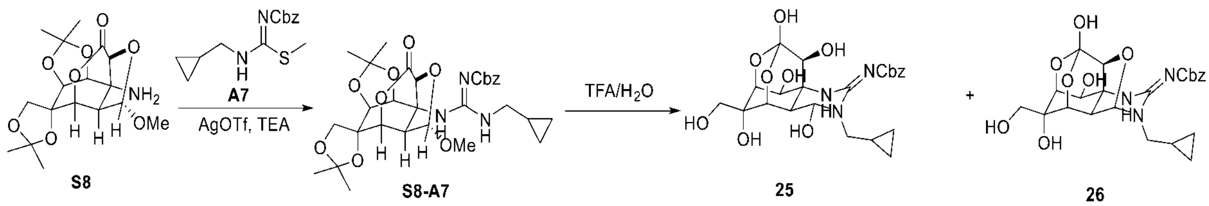


S8 S8-A7 25 26

Step 1: Synthesis of **Compound S8-A7**

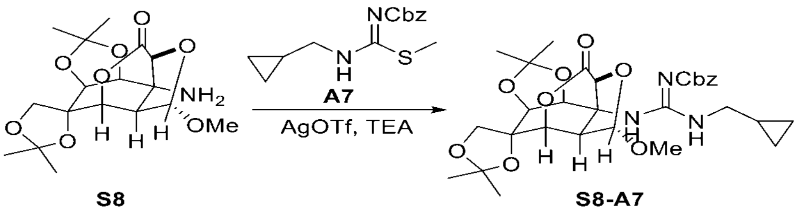


S8 S8-A7

According to the synthesis procedures of **Compound S8-A1** in Example 6, **Compound S8-A7** was obtained as off-white solid (0.80 g, yield: 49%). LCMS (m/z): 602.3[M+1]+.

Step 2: Synthesis of **Compound 25** and **Compound 26**

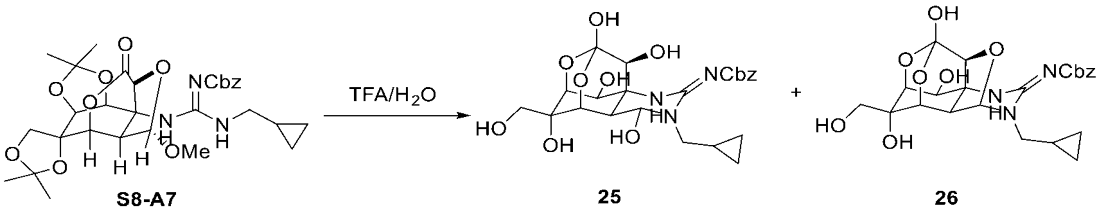


S8-A7 25 26

According to the synthesis procedures of Example 1, **Compound 25 as** white solid (295 mg, yield: 44%) and **Compound 26 as** white solid (280 mg, yield: 43%) were obtained, total yield: 87%.

**Compound 25:**

$^1$H NMR (400MHz, MeOD) $\delta$ 7.49-7.27(m, 5H), 5.71-5.62(m, 1H), 5.26(d, *J*=9.8Hz, 2H), 4.21(d, *J*=1.5Hz, 1H), 4.16(s, 1H), 4.08-3.91(m, 4H), 3.86-3.72(m, 1H), 3.43-3.34(m, 1H), 2.47-2.37(m, 1H), 1.16-1.03(m, 1H), 0.61-0.44(m, 2H), 0.42-0.34(m, 1H), 0.28-0.19(m, 1H); LCMS (m/z): 508.2[M+1]+.

**Compound 26:**

$^1$H NMR (400 MHz, MeOD) $\delta$ 7.47-7.20(m, 5H), 5.45(s, 1H), 5.11(s, 2H), 4.48(d, *J*=2.1 Hz, 1H), 4.39(s, 1H), 4.18(dd, *J*=3.0, 1.6 Hz, 1H), 4.01(t, *J*=1.8 Hz, 1H), 3.97(d, *J*=11.4 Hz, 1H), 3.90(d, *J*=11.5 Hz, 1H), 3.43(dd, *J*=14.4, 7.0 Hz, 1H), 3.34(dd, *J*=12.3, 4.8 Hz, 1H), 2.69(d, *J*=2.6 Hz, 1H), 1.16-0.99(m, 1H), 0.59-0.44(m, 2H), 0.36-0.22(m, 2H); LCMS (m/z): 490.2[M+1]+.

Example 22: Synthesis of (4R,4aR,5R,6S,7S,9S,10S,10aR,11S)-3-(cyclopropylmethyl)-6-(hydroxymethyl)-2-iminooctahydro-5,9-epoxy-7,10a-methanooxocino[4,5-d]pyrimidine-4,6,9,10,11(10H)-pentaol **(Compound 27)**

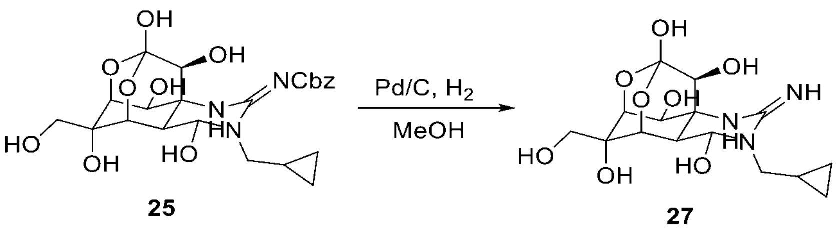


25 27

According to the synthesis procedures of Example 2, **Compound 27** was obtained as white solid (25 mg, yield: 74%). $^1$H NMR (400 MHz, $D_2O$) $\delta$ 5.44(d, *J*=9.6 Hz, 1H), 4.24(s, 1H), 4.13(s, 2H), 3.97(d, *J*=12.1 Hz, 2H), 3.92-3.84(m, 1H), 3.12-2.96(m, 2H), 2.28(d, J=9.5 Hz, 1H), 1.10-0.89(m, 1H), 0.59-0.42(m, 2H), 0.23-0.13(m, 2H); LCMS (m/z): 374.2 [M+1]+.

Example 23: Synthesis of benzyl ((4aR,5R,6S,7S,9S,10S,10aR,11S,E)-3-(cyclopropylmethyl)-6,9,10,11-tetrahydroxy-6-(hydroxymethyl)octahydro-5,9-epoxy-7,10a-methanooxocino[4,5-d]pyrimidin-2(1H)-ylidene)carbamate **(Compound 28)**

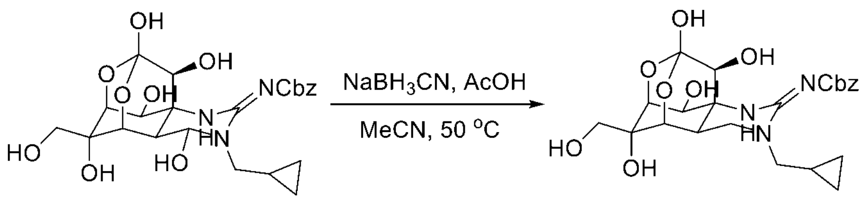


25 28

According to the synthesis procedures of Example 4, **Compound 28** was obtained (14 mg, yield: 12%).

$^1$H NMR (400MHz, MeOD) $\delta$ 7.52-7.20(m, 5H), 5.10(s, 2H), 4.52-4.07(m, 3H), 4.04-3.88(m, 3H), 3.83-3.57(m, 2H), 3.29-3.14(m, 2H), 2.52-2.39(m, 1H), 1.16-0.87(m, 1H), 0.56-0.42(m, 2H), 0.25(d, $J$=4.4Hz, 2H); LCMS (m/z): 492.2 $[M+1]^+$.

Example 24: Synthesis of (4aR,5R,6S,7S,95,10S,10aR,11S)-3-(cyclopropylmethyl)-6-(hydroxymethyl)-2-iminooctahydro-5,9-epoxy-7,10a-methanooxocino[4,5-d]pyrimidine-6,9,10,11(10H)-tetraol **(Compound 29)**

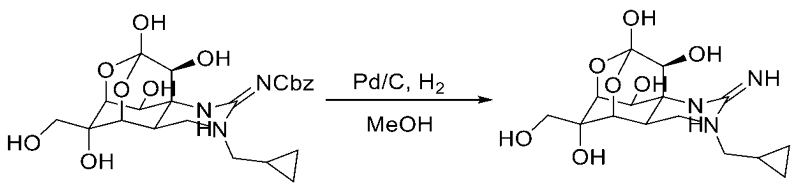


28 29

According to the synthesis procedures of Example 2, **Compound 29** was obtained as white solid (10 mg, yield: 93%).

$^1$H NMR (400 MHz, $D_2O$) $\delta$ 4.12-4.02(m, 2H), 4.00-3.90(m, 3H), 3.86(d, $J$=6.1 Hz, 2H), 3.44-3.30(m, 2H), 3.13-3.00(m, 1H), 2.49(dd, $J$=12.6, 6.2 Hz, 1H), 1.09-0.94(m, 1H), 0.58-0.44(m, 2H), 0.31-0.15(m, 2H); LCMS (m/z): 358.2 $[M+1]^+$.

Example 25: Synthesis of (4S,5aS,6S,8R,9S,10S,11S,11aR,12R)-3-(cyclopropylmethyl)-9-(hydroxymethyl)-2-iminooctahydro-4,8,11a-(epimethanetriyl)-6,10-epoxyoxocino[4,3-f][1,3,5]oxadiazepine-6,9,11(5aH)-triol **(Compound 30)**

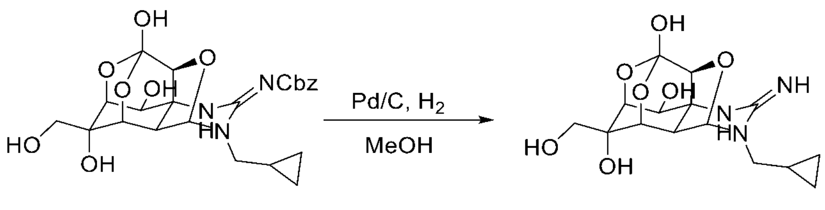


26 30

According to the synthesis procedures of Example 2, **Compound 30** was obtained as white solid (30 mg, yield: 84%).

$^1$H NMR(400MHz, $D_2O$) $\delta$ 5.52(s, 1H), 4.47(d, $J$=2.1Hz, 1H), 4.31(s, 1H), 4.21-4.11(m, 1H), 3.98(t, $J$=1.8Hz, 1H), 3.94(d, J=12.1Hz, 1H), 3.85(d, $J$=12.1Hz, 1H), 3.38-3.30(m,1H), 3.26-3.16(m, 1H), 2.81(d, $J$=2.9Hz, 1H), 1.12-0.95(m, 1H), 0.62-0.48(m, 2H), 0.35-0.18(m, 2H); LCMS (m/z): 356.1$[M+1]^+$.

Example 26: Synthesis of benzyl ((4R,4aR,5R,6S,7S,9S,10S,10aR,11S,E)-3-cyclopropyl-4,6,9,10,11-pentahydroxy-6-(hydroxymethyl)octahydro-5,9-epoxy-7,10a-methanooxocino[4,5-d]pyrimidin-2(1H)-ylidene)carbamate **(Compound 31)** and benzyl ((4S,5aS,6S,8R,9S,108,118,11aR,12R,E)-3-cyclopropyl-6,9,11-trihydroxy-9-(hydroxymethyl) octahydro-4,8,11a-(epimethanetriyl)-6,10-epoxyoxocino[4,3-f][1,3,5]oxadiazepin-2(1H)-ylidene)carbamate (**Compound 32**)

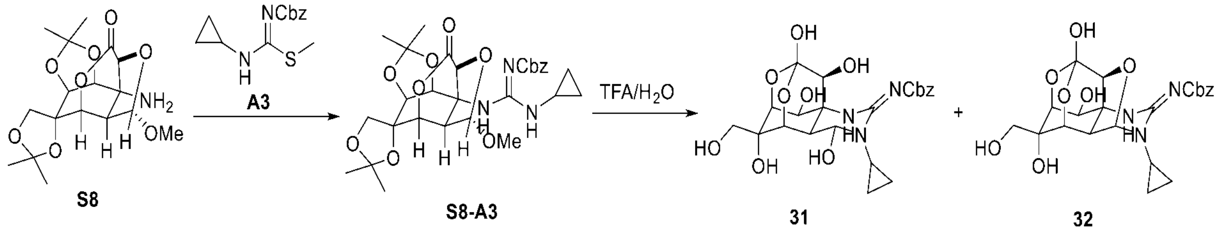


S8 S8-A3 31 + 32

Step 1: Synthesis of **Compound S8-A3**

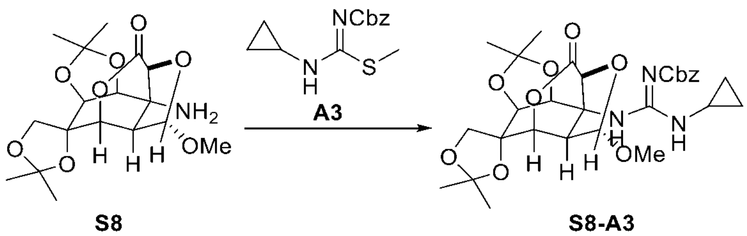


S8 S8-A3

According to the synthesis procedures of **Compound S8-A1** in Example 6, **Compound S8-A3** was obtained as white solid (0.90 g, yield: 57%). LCMS (m/z): 588.3[M+1]$^+$.

Step 2: Synthesis of **Compound 31** and **Compound 32**

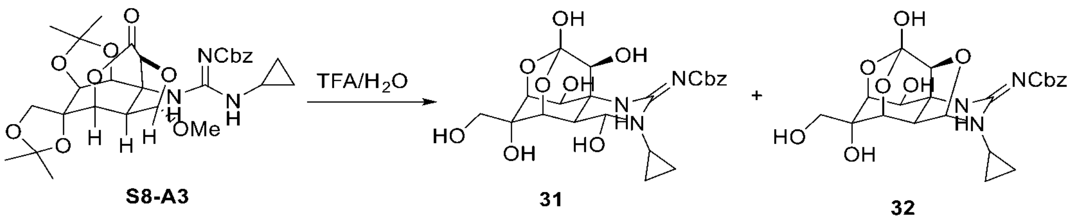


S8-A3 31 + 32

According to the synthesis procedures of Example 1, **Compound 31** (1.35 g, yield: 71%) and **Compound 32** (200 mg, yield: 11%) were obtained as white foam solid, total yield: 88%.

**Compound 31**

$^1$H NMR (400 MHz, MeOD) $\delta$ 7.41-7.27(m, 5H), 5.43-5.30(m, 3H), 4.25(d, *J*=1.4 Hz, 1H), 4.12(s, 1H), 4.06-4.00(m, 2H), 4.00-3.94(m, 2H), 2.89-2.73(m, 1H), 2.52(d, *J*=8Hz, 1H), 1.17-1.08(m, 1H), 1.07-0.95(m, 2H), 0.86-0.72(m, 1H); LCMS (m/z): 494.2[M+1]$^+$.

**Compound 32**

LCMS (m/z): 476.2[M+1]$^+$.

Example 27: Synthesis of (4R,4aR,5R,6S,7S,9S,10S,10aR,11S)-3-cyclopropyl-6-(hydroxymethyl)-2-iminooctahydro-5,9-epoxy-7,10a-methanooxocino[4,5-d]pyrimidine-4,6,9,10,11(10H)-pentaol **(Compound 33)**

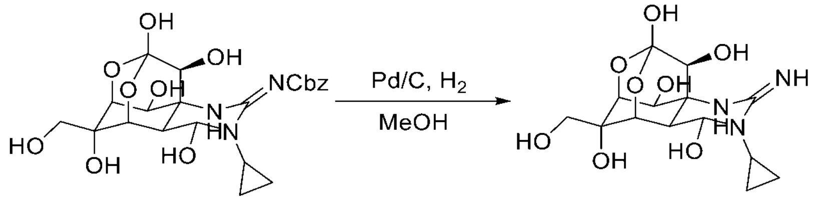


31 33

According to the synthesis procedures of Example 2, **Compound 33** was obtained as white solid (14 mg, yield: 97%).

$^1$H NMR (400 MHz, $D_2O$) $\delta$ 5.52(s, 1H), 4.44(d, *J*=2.1 Hz, 1H), 4.36(s, 1H), 4.19(s, 1H), 4.00(s, 1H), 3.83(s, 1H), 3.52(s, 1H), 2.76(d, *J*=2.7 Hz, 1H), 2.7-2.66(m, 1H), 0.87(d, *J*=6.6 Hz, 2H), 0.79-0.72(m, 2H); LCMS (m/z): 360.1[M+1]$^+$.

Example 28: Synthesis of benzyl ((4aR,5R,6S,7S,9S,10S,10aR,11S,E)-3-cyclopropyl-6,9,10,11-tetrahydroxy-6-(hydroxymethyl)octahydro-5,9-epoxy-7,10a-methanooxocino[4,5-d]pyrimidin-2(1H)-ylidene)carbamate **(Compound 34)**

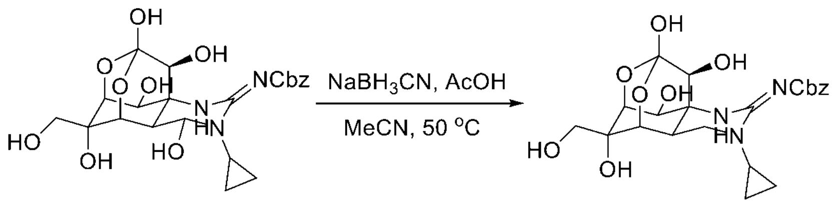


31 34

According to the synthesis procedures of Example 4, **Compound 34** was obtained as white solid (52 mg, yield: 54%).

$^1$H NMR (400 MHz, MeOD) $\delta$ 7.44-7.23(m, 5H), 5.13(s, 2H), 4.59(s, br, 1H), 4.23-3.76(m, 6H), 3.14(s, br, 1H), 2.84-2.73(m, 1H), 2.43(dd, *J*=12.5, 5.5 Hz, 1H), 0.88-0.71(m, 3H), 0.69-0.61(m, 1H); LCMS (m/z): 478.2[M+1]$^+$.

Example 29: Synthesis of (4aR,5R,6S,7S,9S,10S,10aR,11S)-3-cyclopropyl-6-(hydroxymethyl)-2-iminooctahydro-5,9-epoxy-7,10a-methanooxocino[4,5-d]pyrimidine-6,9,10,11(10H)-tetraol (**Compound 35**)

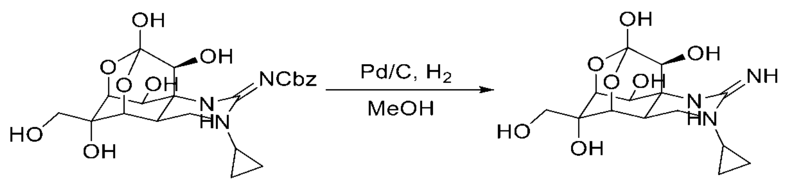


34 35

According to the synthesis procedures of Example 2, **Compound 35** was obtained as white solid (25 mg, yield: 91%).

$^1$H NMR (400 MHz, $D_2O$) $\delta$ 4.15(s, 1H), 4.00(s, 1H), 3.97(s, 1H), 3.94(s, 2H), 3.88(s, 1H), 3.80(d, *J*=11.9 Hz, 1H), 3.37(dd, *J*=11.5, 6.5 Hz, 1H), 2.73(s, 1H), 2.54(dd, *J*=12.3, 6.4 Hz, 1H), 0.89(dd, *J*=14.1, 6.5 Hz, 3H), 0.76(dd, *J*=7.4, 4.1 Hz, 1H); LCMS (m/z): 344.1[M+1]$^+$.

Example 30: Synthesis of (4S,5aS,6S,8R,9S,10S,11S,11aR,12R)-3-cyclopropyl-9-(hydroxymethyl)-2-iminooctahydro-4,8,11a-(epimethanetriyl)-6,10-epoxyoxocino[4,3-f][1,3,5]oxadiazepine-6,9,11(5aH)-triol **(Compound 36)**

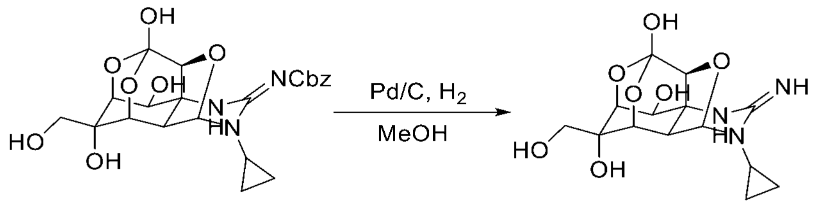


32 36

According to the synthesis procedures of Example 2, **Compound 36** was obtained as white solid (152 mg, yield: 100%).

$^1$H NMR (400 MHz, $D_2O$) $\delta$ 4.15(s, 1H), 4.00(s, 1H), 3.97(s, 1H), 3.94(s, 2H), 3.88(s, 1H), 3.80(d, *J*=11.9 Hz, 1H), 2.73(s, 1H), 2.54(dd, *J*=12.3, 6.4 Hz, 1H), 0.95-0.85(m, 3H), 0.76(dd, *J*=7.4, 4.1 Hz, 1H); LCMS (m/z): 342.1[M+1]$^+$.

Example 31: Synthesis of benzyl ((4R,4aR,5R,6S,7S,9S,10S,10aR,11S,E)-4,6,9,10,11-pentahydroxy-6-(hydroxymethyl)-3-isopropyloctahydro-5,9-epoxy-7,10a-methanooxocino[4,5-d]pyrimidin-2(1H)-ylidene)carbamate **(Compound** 37) and benzyl ((4S,5aS,6S,8R,9S,10S,11S,11aR,12R,E)-6,9,11-trihydroxy-9-(hydroxymethyl)-3-isopropyloctahydro-4,8,11a-(epimethanetriyl)-6,10-epoxyoxocino[4,3-f][1,3,5]oxadiazepin-2(1H)-ylidene)carbamate **(Compound 38)**

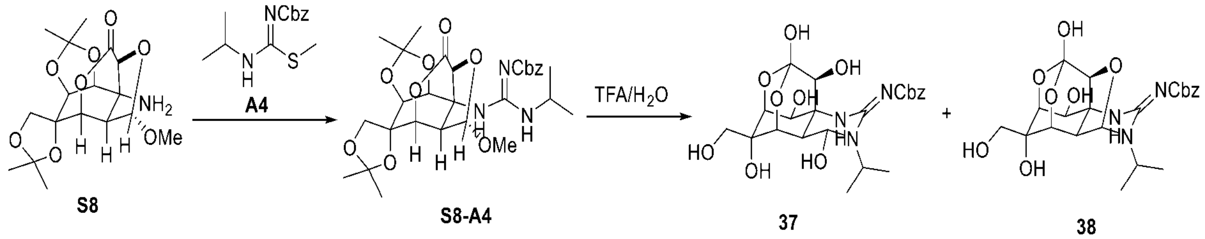


S8 S8-A4 37 + 38

Step 1: Synthesis of **Compound S8-A4**

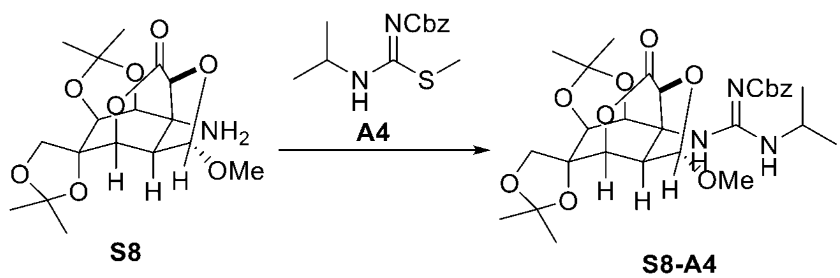


S8 S8-A4

According to the synthesis procedures of **Compound S8-A1** in Example 6, **Compound S8-A4** was obtained as white foam solid (0.91 g, yield: 57%). LCMS (m/z): 590.3[M+1]$^+$.

Step 2: Synthesis of Compound **37** and **Compound 38**

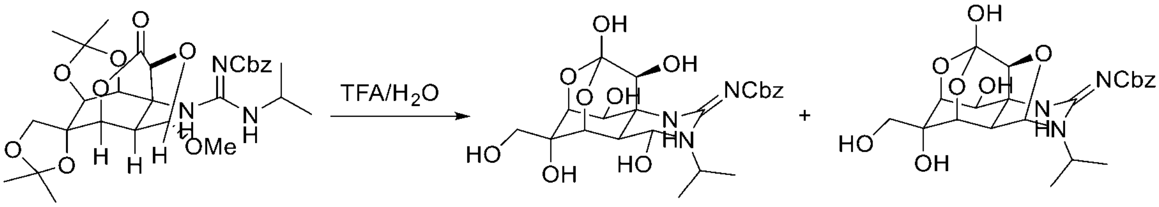


S8-A4 37 38

According to the synthesis procedures of Example 1, **Compound 37** as white foam solid (330 mg, yield: 46%) and **Compound 38** as white foam solid (17 mg, yield: 2%) were obtained, total yield: 48%.

**Compound 37**

$^1$H NMR (400 MHz, MeOD) $\delta$ 7.52-7.28(m, 5H), 5.79(s, 1H), 5.30(s, 2H), 4.59(d, *J*=2.1 Hz, 1H), 4.52(s, 1H), 4.39-4.31(m, 1H), 4.30-4.27(m, 1H), 4.07(t, *J*=1.8 Hz, 1H), 3.98(d, *J*=11.5 Hz, 1H), 3.92(d, *J*=11.5 Hz, 1H), 2.82(d, *J*=2.9 Hz, 1H), 1.35(d, *J*=6.6 Hz, 3H), 1.25(d, *J*=6.7 Hz, 3H); LCMS (m/z): 496.2[M+1]$^+$.

**Compound 38**

LCMS (m/z): 478.2[M+1]$^+$.

Example 32: Synthesis of (4R,4aR,5R,6S,7S,9S,10S,10aR,11S)-6-(hydroxymethyl)-2-imino-3-isopropyloctahydro-5,9-epoxy-7,10a-methanooxocino[4,5-d]pyrimidine-4,6,9,10,11(10H)-pentaol (**Compound 39**)

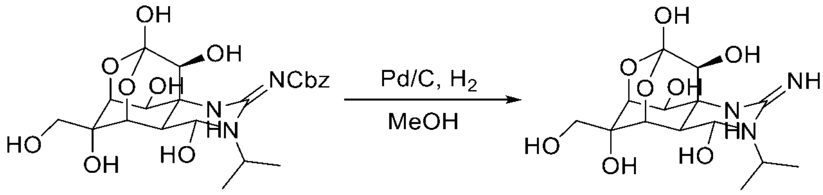


37 39

According to the synthesis procedures of Example 2, **Compound 39** was obtained as white solid (9.8 mg, yield: 90%).

$^1$H NMR (400 MHz, $D_2O$) $\delta$ 5.74(s, 1H), 4.54(d, *J*=1.7 Hz, 1H), 4.47(s, 1H), 4.31(s, 1H), 4.09(s, 1H), 4.03-3.97(m, 1H), 3.96-3.91(m, 1H), 3.85(d, *J*=12.2 Hz, 1H), 2.85(d, J=2.6 Hz, 1H), 1.27(d, J=6.6 Hz, 3H), 1.19(d, J=6.6 Hz, 3H); HRMS (ESI +): calculated: $C_{14}H_{23}O_8N_3$ ([M+H]$^+$): 362.15, measured: 361.35.

Example 33: Synthesis of benzyl ((4aR,5R,6S,7S,9S,10S,10aR,11S,E)-6,9,10,11-tetrahydroxy-6-(hydroxymethyl)-3-isopropyloctahydro-5,9-epoxy-7,10a-methanooxocino[4,5-d]pyrimidin-2(1H)-ylidene)carbamate **(Compound 40)**

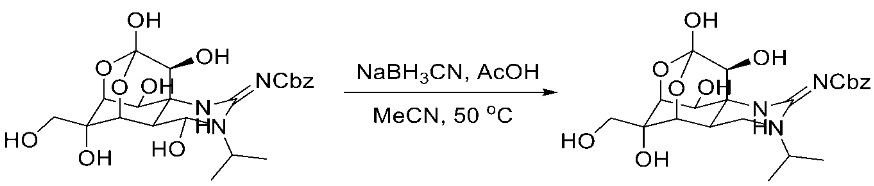


37 40

According to the synthesis procedures of Example 4, **Compound 40** was obtained as white solid (17.8 mg, yield: 19%). LCMS (m/z): 480.2[M+1]$^+$.

Example 34: Synthesis of (4aR,5R,6S,7S,9S,10S,10aR,11S)-6-(hydroxymethyl)-2-imino-3-isopropyloctahydro-5,9-epoxy-7,10a-methanooxocino[4,5-d]pyrimidine-6,9,10,11(10H)-tetraol **(Compound 41)**

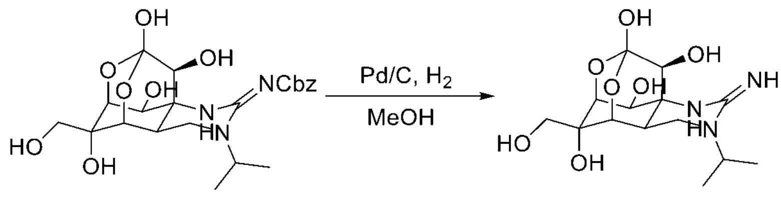


40

41

According to the synthesis procedures of Example 2, **Compound 41** was obtained as white solid (10 mg, yield: 93%).

$^1H$ NMR (400 MHz, $D_2O$) δ 4.47(d, J=35.1 Hz, 1H), 4.21(d, J=61.5 Hz, 1H), 4.04-3.96(m, 2H), 3.95-3.85(d, J=17.8 Hz, 2H), 3.83-3.60(m, 2H), 3.37-3.21(m, 1H), 2.48(dd, J=12.1, 6.0 Hz, 1H), 1.15(t, J=5.8 Hz, 6H); HRMS (ESI +): calculated: $C_{22}H_{29}O_{19}N_3$ ($[M+H]^+$): 346.15, measured: 346.16.

Example 35: Synthesis of (4S,5aS,6S,8R,9S,10S,11S,11aR,12R)-9-(hydroxymethyl)-2-imino-3-isopropyloctahydro-4,8,11a-(epimethanetriyl)-6,10-epoxyoxocino[4,3-f][1,3,5]oxadiazepine-6,9,11(5aH)-triol **(Compound 42)**

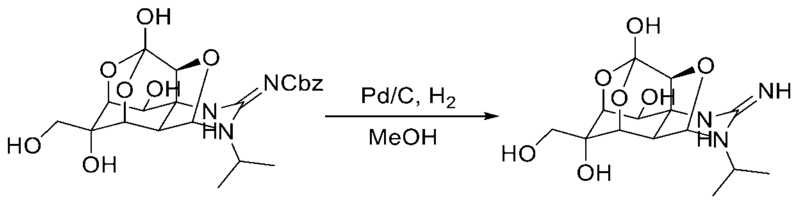


38

42

According to the synthesis procedures of Example 2, **Compound 42** was obtained as white solid (10 mg, yield: 93%).

$^1H$ NMR (400 MHz, $D_2O$) δ 5.74(s, 1H), 4.54(d, *J*=1.8 Hz, 1H), 4.46(s, 1H), 4.30(s, 1H), 4.09(s, 1H), 4.03-3.96(m, 1H), 3.93(d, *J*=12.3 Hz, 1H), 3.85(d, *J*=12.2 Hz, 1H), 2.85(d, *J*=2.6 Hz, 1H), 1.27(d, *J*=6.6 Hz, 3H), 1.18(d, *J*=6.7 Hz, 3H); LCMS (m/z): 344.1$[M+1]^+$.

Example 36: Synthesis of benzyl ((4R,4aR,5R,6S,7S,9S,10S,10aR,11S,E)-4,6,9,10,11-pentahydroxy-6-(hydroxymethyl)-3-(3-hydroxypropyl)octahydro-5,9-epoxy-7,10a-methanooxocino[4,5-d]pyrimidin-2(1H)-ylidene)carbamate **(Compound 43),** benzyl ((4S,5aS,6S,8R,9S,108,11S,11aR,12R,E)-6,9,11-trihydroxy-9-(hydroxymethyl)-3-(3-hydroxypropyl)octahydro-4,8,11a-(epimethanetriyl)-6,10-epoxyoxocino[4,3-f][1,3,5]oxadiazepin-2(1H)-ylidene)carbamate **(Compound 44),** benzyl ((7aR,8S,9S,11S,12S,13R,13aR,13bR,15S,E)-8,9,12,15-tetrahydroxy-12-(hydroxymethyl) octahydro-2H,11H-9,13-epoxy-7a,11-methanooxocino[4',5':4,5]pyrimido[6,1-b][1,3]oxazin-6(7H)-ylidene)carbamate **(Compound 45)** and benzyl ((7aR,8S,9S,11S,12S,13R,13aR,15S,E)-8,9,12,15-tetrahydroxy-12-(hydroxymethyl)octahydro-2H,11H-9,13-epoxy-7a,11-methanooxocino[4',5':4,5]pyrimido[6,1-b][1,3]oxazin-6(7H)-ylidene)carbamate **(Compound 46)**

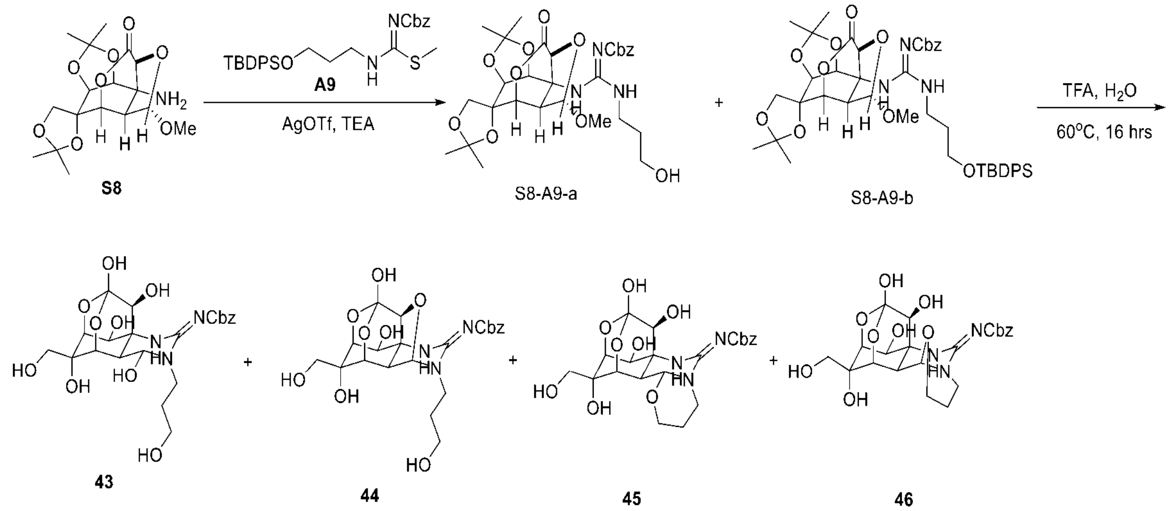


S8 A9 S8-A9-a S8-A9-b

43 44 45 46

Step 1: Synthesis of **Compound S8-A9-a** and **Compound S8-A9-b**

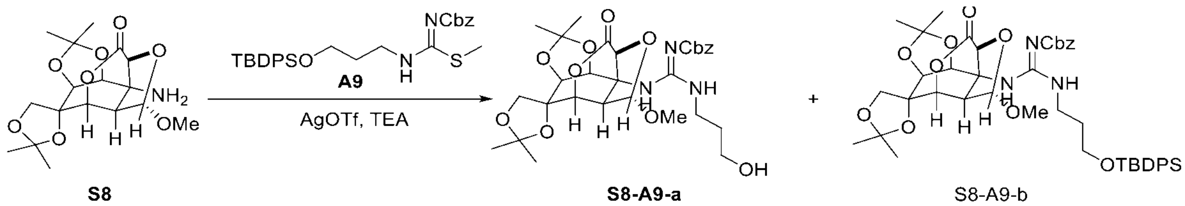


S8 A9 **S8-A9-a** S8-A9-b

According to the synthesis procedures of **Compound S8-A1** in Example 6, a mixture of **Compound S8-A9-a** and Compound S8-A9-b (0.70 g, 43%) was obtained as white solid.

**Compound S8-A9-a:** LCMS (m/z): 606.3[M+H]$^+$.

**Compound S8-A9-b:** LCMS (m/z): 844.4[M+H]$^+$.

Step 2: Synthesis of **Compound 43, Compound 44, Compound 45** and **Compound 46**

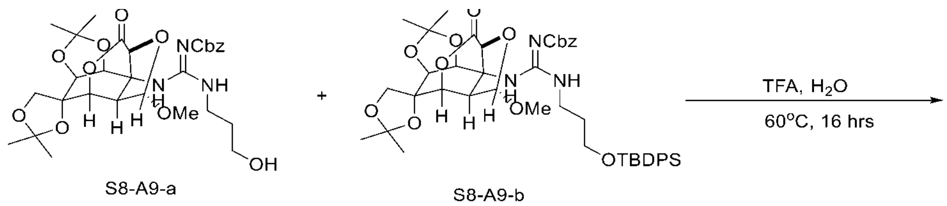


S8-A9-a S8-A9-b

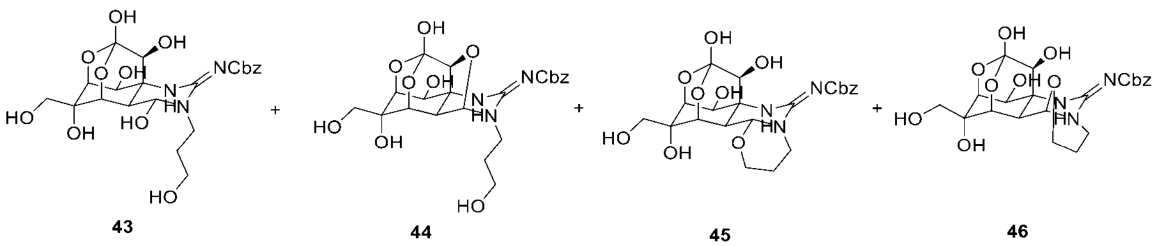

43 44 45 46

According to the synthesis procedures of Example 1, **Compound 43** (30 mg, yield: 7%), **Compound 44** (25 mg, yield: 5%), **Compound 45** (8 mg, yield: 2%) and **Compound 46** (25 mg, yield: 5%) were obtained as white solid.

**Compound 43:**

$^1$H NMR (400 MHz, MeOD) δ 7.38-7.27(m, 5H), 5.42-5.28(d, 1H), 5.08(s, 2H), 4.57(s, br, 1H), 4.15(d, *J*=8.4 Hz, 2H), 4.04-3.91(m, 3H), 3.75-3.69(m, 2H), 3.59-3.53(m, 2H), 2.68(d, *J*=4.8 Hz, 1H), 2.36(d, *J*=9.6 Hz, 1H), 1.84-1.75(m, 2H); LCMS(m/z): 512.2[M+H]$^+$.

**Compound 44:**

$^1$H NMR (400 MHz, MeOD) δ 7.32-7.13(m, 5H), 5.27(s, 1H), 5.00(s, 2H), 4.47(s, 1H), 4.39(d, *J*=2.0 Hz, 1H), 4.31(s, 1H), 4.09(s, 1H), 3.92(s, 1H), 3.84(dd, *J*=27.0, 11.5 Hz, 2H), 3.67-3.58(m, 1H), 3.52-3.44(m, 2H), 3.44-3.33(m, 1H), 2.61(d, *J*=2.8 Hz, 1H), 1.72-1.61(m, 2H); LCMS(m/z): 494.2[M+H]$^+$.

**Compound 45**

$^1$H NMR (400MHz, MeOD) δ 7.43-7.23(m, 5H), 5.09(s, 2H), 5.04(d, *J*=7.2Hz, 1H), 4.56(s, 1H), 4.32-4.08(m, 3H), 4.07-3.92(m, 3H), 3.93-3.66(m, 2H), 3.15-2.96(m, 1H), 2.83(s, 1H), 1.98-1.74(m, 1H), 1.55(d, *J*=13.8Hz, 1H); LCMS(m/z): 494.2[M+H]$^+$.

**Compound 46**

$^1$H NMR (400 MHz, MeOD) δ 7.46-7.19(m, 5H), 5.16(d, *J*=9.5 Hz, 1H), 5.09(s, 2H), 4.77(d, *J*=14.0 Hz, 1H), 4.56(s, 1H), 4.46-4.24(m, 1H), 4.17-3.95(m, 4H), 3.90(d, *J*=9.2 Hz, 1H), 3.70(d, *J*=11.6 Hz, 1H), 2.85(t, *J*=13.1 Hz, 1H), 2.45(d, *J*=9.4 Hz, 1H), 1.86(q, *J*=12.6 Hz, 1H), 1.58(d, *J*=13.3 Hz, 1H); LCMS(m/z):494.2[M+H]$^+$.

Example 37: Synthesis of (7aR,8S,9S,11S,12S,13R,13aR,13bR,15S)-12-(hydroxymethyl)-6-iminooctahydro-2H,11H-9,13-epoxy-7a,11-methanooxocino[4',5':4,5]pyrimido[6,1-b][1,3]oxazine-8,9,12,15(8H)-tetraol **(Compound 47)**

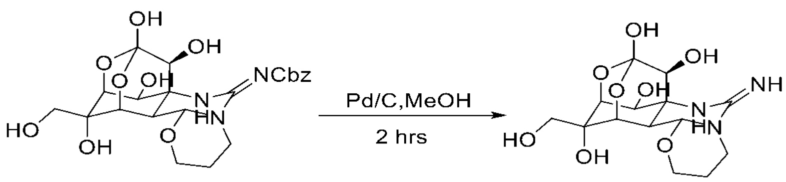


**45** **47**

According to the synthesis procedures of Example 2, **Compound 47** was obtained as white solid (11 mg, yield: 100%).

$^1$H NMR (400MHz, $D_2O$) δ 5.01(d, *J*=20.2Hz, 1H), 4.25(dd, *J*=26.7, 13.3Hz, 1H), 4.09(dd, *J*=5.4, 1.6 Hz, 2H), 4.04-3.91(m, 4H), 3.84(s, 1H), 3.56(s, 1H), 3.46-3.36(m, 1H), 2.76(d, *J*=5.5Hz, 1H), 1.99-1.83(m, 1H), 1.67(d, *J*=14.2 Hz, 1H); LCMS(m/z): 360.1[M+1]$^+$.

Example 38: Synthesis of (7aR,8S,9S,11S,12S,13R,13aR,15S)-12-(hydroxymethyl)-6-iminooctahydro-2H,11H-9,13-epoxy-7a,11-methanooxocino[4',5":4,5]pyrimido[6,1-b][1,3]oxazine-8,9,12,15(8H)-tetraol **(Compound 48)**

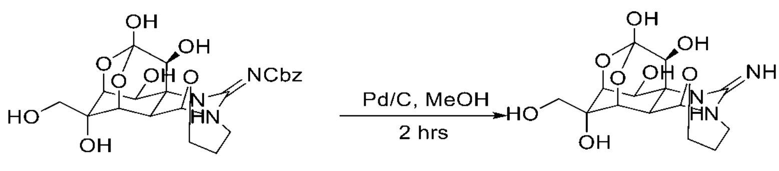


**46** **48**

According to the synthesis procedures of Example 2, **Compound 48** was obtained as white solid (11 mg, yield:

100%).

$^1H$ NMR (400 MHz, $D_2O$) $\delta$ 5.11(d, $J$=9.5Hz, 1H), 4.16(s, 1H), 4.07(dd, $J$=11.4Hz, $J$=4.2Hz, 1H), 4.01-3.89(m, 5H), 3.81(d, $J$=11.9 Hz, 1H), 3.78(s, 1H), 3.30-3.20(m, 1H), 2.40(d, J=9.4Hz, 1H), 2.01-1.81(m, 1H), 1.73(d, $J$=13.8Hz, 1H); LCMS(m/z): 360.1[M+1]$^+$.

Example 39: Synthesis of 3-((4R,4aR,5R,6S,7S,9S,10S,10aR,11S,E)-2-(((benzyloxy)carbonyl)imino)-4,6,9,10,11-pentahydroxy-6-(hydroxymethyl)octahydro-5,9-epoxy-7,10a-methanooxocino[4,5-d]pyrimidin-3(4H)-yl)propanoic acid **(Compound 49)** and benzyl ((7aR,8S,9S,11S,12S,13R,13aR,13bR,15S,E)-8,9,12,15-tetrahydroxy-12-(hydroxymethyl)-2-oxooctahydro-2H,11H-9,13-epoxy-7a,11-methanooxocino[4',5':4,5]pyrimido[6,1-b][1,3]oxazin-6(7H)-ylidene)carbamate **(Compound 50)**

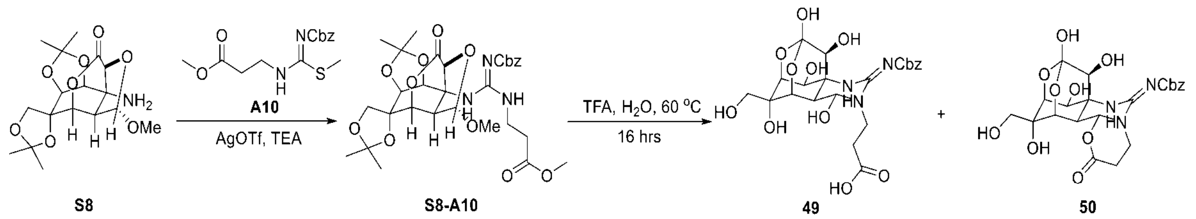


S8 S8-A10 49 50

Step 1: Synthesis of **Compound S8-A10**

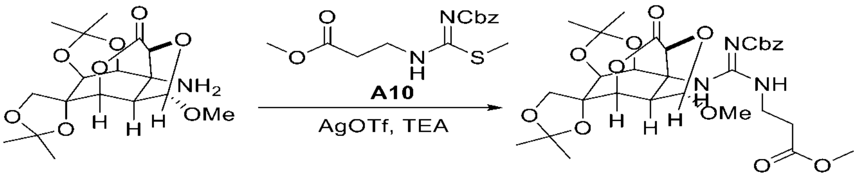


S8 S8-A10

According to the synthesis procedures of **Compound S8-A1** in Example 6, **Compound S8-A10** was obtained as white solid (300 mg, yield: 29%). LCMS(m/z): 634.3[M+1]$^+$.

Step 2: Synthesis of **Compound 49** and **Compound 50**

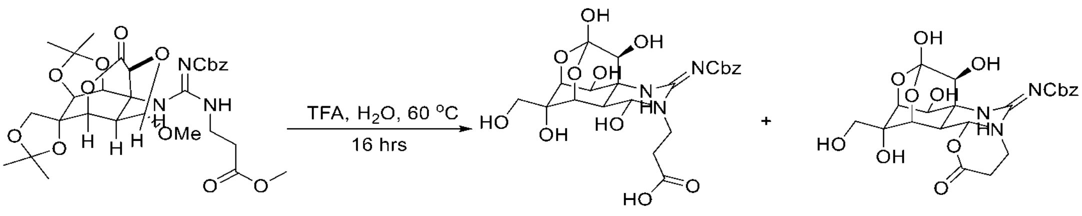


S8-A10 49 50

According to the synthesis procedures of Example 1, **Compound 49** (25.3 mg, yield: 10%) and **Compound 50** (65 mg, yield: 27%) were obtained as white solid.

**Compound 49:**

$^1H$ NMR (400 MHz, MeOD) $\delta$ 7.43-7.25(m, 5H), 5.38(t, $J$=9.1 Hz, 1H), 5.18(t, $J$=9.5 Hz, 2H), 4.40-4.10(m, 2H), 4.06-3.95(m, 2H), 3.95-3.65(m, 4H), 2.76-2.67(m, 1H), 2.65-2.55(m, 1H), 2.41(d, $J$=9.5 Hz, 1H); LCMS(m/z): 526.2 [M+1]$^+$.

**Compound 50:**

$^1H$ NMR (400 MHz, MeOD) $\delta$ 7.43-7.23(m, 5H), 5.47(s, 1H), 5.12(s, 2H), 4.46(d, *J*=2.1 Hz, 1H), 4.37(s, 1H), 4.17(dd, *J*=2.9, 1.5 Hz, 1H), 4.00(t, *J*=1.7 Hz, 1H), 3.95(d, *J*=11.5 Hz, 1H), 3.90(d, *J=11.5* Hz, 1H), 3.85-3.76(m, 1H), 3.71-3.61(m, 1H), 2.68(d, iJ=2.9 Hz, 1H), 2.62(dd, *J*=15.4, 7.7 Hz, 1H), 2.56-2.46(m, 1H); LCMS(m/z): 508.2[M+1]+.

Example 40: Synthesis of (7aR,8S,9S,11S,12S,13R,13aR,13bR,15S)-8,9,12,15-tetrahydroxy-12-(hydroxymethyl)-6-iminodecahydro-2H,11H-9,13-epoxy-7a,11-methanooxocino[4',5':4,5]pyrimido[6,1-b][1,3]oxazin-2-one (**Compound 51**)

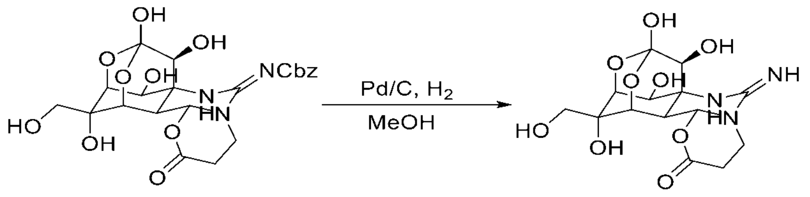


50 51

According to the synthesis procedures of Example 2, **Compound 51** was obtained as white solid (15 mg, yield: 67%).

$^1H$ NMR (400 MHz, $D_2O$) $\delta$ 5.50(s, 1H), 4.52(d, *J*=2.1 Hz, 1H), 4.45(s, 1H), 4.27(s, 1H), 4.08(s, 1H), 3.91(d, *J*=12.2 Hz, 1H), 3.84(d, *J*=12.2 Hz, 1H), 3.71-3.55(m, 2H), 2.87(d, *J*=2.9 Hz, 1H), 2.51-2.42(m, 2H); LCMS(m/z): 374.1 [M+1]+.

Example 41: Synthesis of benzyl ((4R,4aR,5R,6S,7S,9S,10S,10aR,11S,E)-3-(cyclobutylmethyl)-4,6,9,10,11-pentahydroxy-6-(hydroxymethyl)octahydro-5,9-epoxy-7,10a-methanooxocino[4,5-d]pyrimidin-2(1H)-ylidene)carbamate **(Compound 52)** and benzyl ((4S,5aS,6S,8R,9S,10S,11S,11aR,12R,E)-3-(cyclobutylmethyl)-6,9,11-trihydroxy-9-(hydroxymethyl)octahydro-4,8,11a-(epimethanetriyl)-6,10-epoxyoxocino[4,3-f][1,3,5]oxadiazepin-2(1H)-ylidene)carbamate **(Compound 53)**

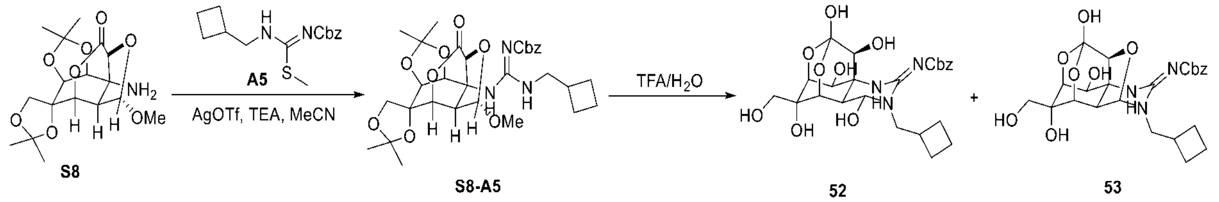


S8 S8-A5 52 53

Step 1: Synthesis of **Compound S8-A5**

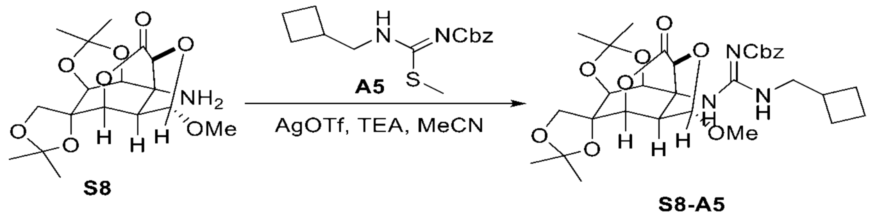


S8 S8-A5

According to the synthesis procedures of **Compound S8-A1** in Example 6, **Compound S8-A5** was obtained as white foam solid (923 mg, yield: 55.7%). LCMS (m/z): 616.3[M+1]+

Step 2: Synthesis of **Compound 52** and **Compound 53**

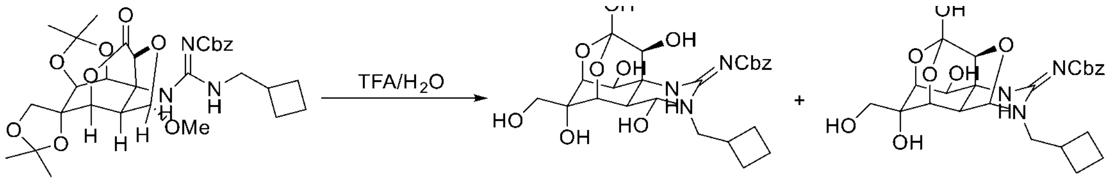


S8-A5 52 + 53

According to the synthesis procedures of Example 1, **Compound 52** as white solid (300 mg, yield: 39%) and **Compound 53 as** white solid (90 mg, yield: 12%) were obtained, total yield: 51%.

**Compound 52**

$^1$H NMR (400 MHz, MeOD) $\delta$ 7.51-7.29(m, 5H), 5.47(dd, *J*=17.2, 9.7 Hz, 1H), 5.28(d, *J*=10.0 Hz, 2H), 4.51-4.31(m, 1H), 4.20(d, *J*=1.5 Hz, 1H), 4.16(s, 1H), 4.06-3.95(m, 3H), 3.91-3.72(m, 1H), 3.66-3.56(m, 1H), 2.74(dd, *J*=14.8, 7.3 Hz, 1H), 2.46-2.36(m, 1H), 2.17-1.98(m, 2H), 1.95-1.69(m, 4H); LCMS (m/z): 522.2[M+1]$^+$.

**Compound 53:**

$^1$H NMR (400 MHz, MeOD) $\delta$ 7.47-7.25(m, 5H), 5.35(s, 1H), 5.14(s, 2H), 4.48(d, *J*=2.1 Hz, 1H), 4.39(s, 1H), 4.18(dd, *J*=3.0, 1.5 Hz, 1H), 4.02(t, *J*=1.8 Hz, 1H), 3.95(dd, *J*=27.9, 11.4 Hz, 2H), 3.60(dd, *J*=14.0, 7.5 Hz, 1H), 3.48(dd, *J*=14.0, 7.3 Hz, 1H), 2.69-2.59(m, 2H), 2.10-1.98(m, 2H), 1.96-1.71(m, 4H); LCMS (m/z): 504.2[M+1]$^+$.

Example 42: Synthesis of (4S,5aS,6S,8R,9S,10S,11S,11aR,12R)-3-(cyclobutylmethyl)-9-(hydroxymethyl)-2-iminooctahydro-4,8,11a-(epimethanetriyl)-6,10-epoxyoxocino[4,3-f][1,3,5]oxadiazepine-6,9,11(5aH)-triol **(Compound 54)**

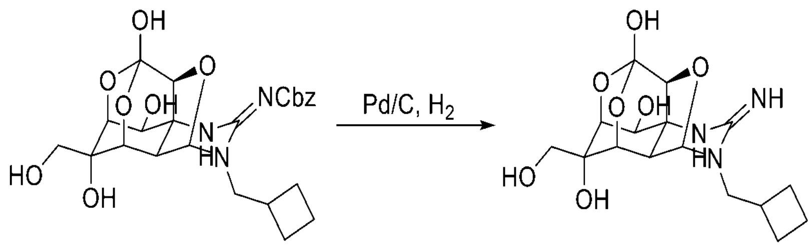


53 54

According to the synthesis procedures of Example 2, **Compound 54** was obtained as off-white solid (9 mg, yield: 55.8%).

$^1$H NMR (400 MHz, $D_2O$) $\delta$ 5.41(s, 1H), 4.44(s, 1H), 4.26(s, 1H), 4.11(s, 1H), 4.01-3.77(m, 3H), 3.41(d, *J*=6.0 Hz, 2H), 2.73(s, 1H), 2.58(s, 1H), 1.99(s, 2H), 1.88-1.55(m, 4H); LCMS (m/z): 370.2[M+1]$^+$.

Example 43: Synthesis of (4R,4aR,5R,6S,7S,9S,10S,10aR,11S)-3-(cyclobutylmethyl)-6-(hydroxymethyl)-2-iminooctahydro-5,9-epoxy-7,10a-methanooxocino[4,5-d]pyrimidine-4,6,9,10,11(10H)-pentaol **(Compound 55)**

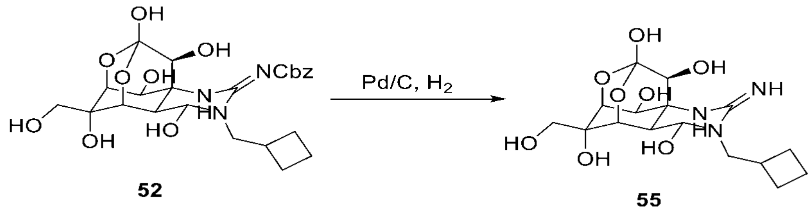


52 55

According to the synthesis procedures of Example 2, **Compound 55** was obtained as off-white solid (35 mg, yield: 47.1%).

$^1H$ NMR (400 MHz, $D_2O$) $\delta$ 5.44(d, $J$=8.6 Hz, 1H), 4.35-3.70(m, 6H), 3.18(d, $J$=5.5 Hz, 2H), 2.49(d, $J$=6.6 Hz, 1H), 2.31(dd, $J$=29.5, 9.2 Hz, 1H), 1.99(s, 2H), 1.86-1.56(m, 4H); LCMS (m/z): 388.2[M+1]+.

Example 44: Synthesis of (4aR,5R,6S,7S,95,10S,10aR,11S)-3-(cyclobutylmethyl)-6-(hydroxymethyl)-2-iminooctahydro-5,9-epoxy-7,10a-methanooxocino[4,5-d]pyrimidine-6,9,10,11(10H)-tetraol **(Compound 56)**

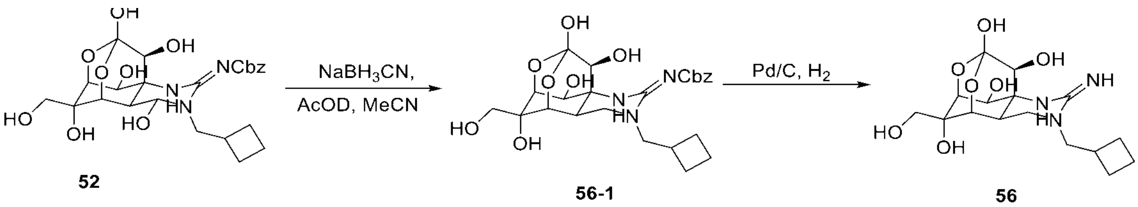


52 56-1 56

Step 1: Synthesis of benzyl ((4aR,5R,6S,7S,9S,10S,10aR,11S,E)-3-(cyclobutylmethyl)-6,9,10,11-tetrahydroxy-6-(hydroxymethyl)octahydro-5,9-epoxy-7,10a-methanooxocino[4,5-d]pyrimidin-2(1H)-ylidene)carbamate **(Compound 56-1)**

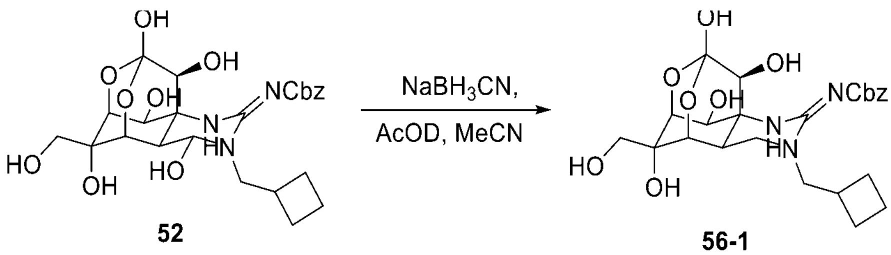


52 56-1

According to the synthesis procedures of Example 4, **Compound 56-1** was obtained as white foam solid (50 mg, yield: 51.1%). LCMS (m/z): 506.2[M+1]+.

Step 2: Synthesis of **Compound 56**

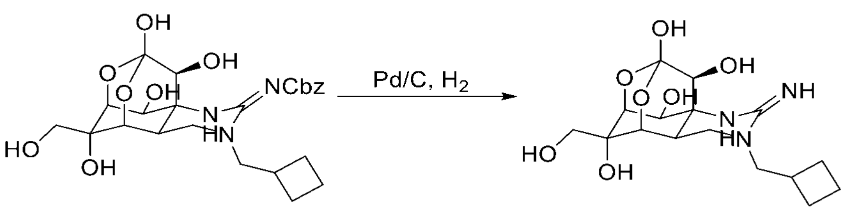


56-1 56

According to the synthesis procedures of Example 2, **Compound 56** was obtained as off-white solid (18 mg, yield: 76.6%).

$^1H$ NMR (400 MHz, $D_2O$) $\delta$ 4.20-3.70(m, 6H), 3.55-3.41(m, 1H), 3.28-3.08(m, 2H), 2.56(d, *J*=66.2 Hz, 3H), 2.09-1.59(m, 6H); LCMS (m/z): 372.2[M+1]$^+$.

Example 45: Synthesis of (4R,4aR,5R,6S,7S,9S,10S,10aR,11S)-3-benzyl-6-(hydroxymethyl)-2-iminooctahydro-5,9-epoxy-7,10a-methanooxocino[4,5-d]pyrimidine-4,6,9,10,11(10H)-pentaol **(Compound 57)** and (4S,5aS,6S,8R,9S,10S,11S,11aR,12R)-3-benzyl-9-(hydroxymethyl)-2-iminooctahydro-4,8,11a-(epimethanetriyl)-6,10-epoxyoxocino[4,3-f][1,3,5]oxadiazepine-6,9,11(5aH)-triol **(Compound 58)**

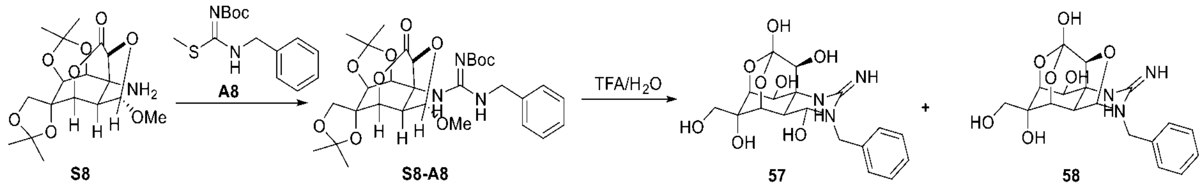

S8 S8-A8 57 58

Step 1: Synthesis of **Compound S8-A8**

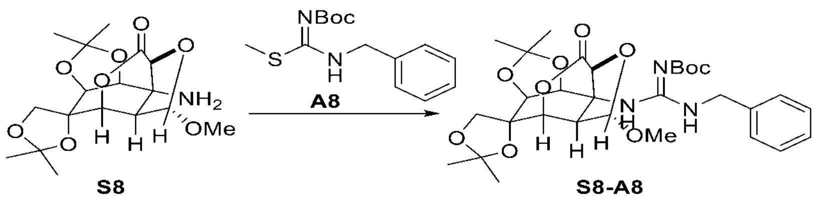

S8 S8-A8

According to the synthesis procedures of **Compound S8-A1** in Example 6, **Compound S8-A8** was obtained as off-white solid (0.84 g, yield: 61%). LCMS (m/z): 604.3[M+1]$^+$.

Step 2: Synthesis of **Compound 57** and **Compound 58**

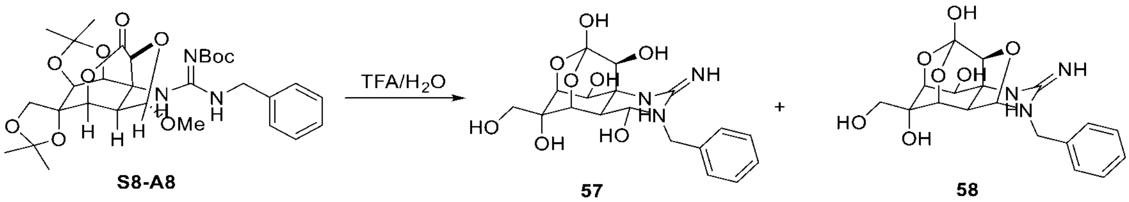

S8-A8 57 58

According to the synthesis procedures of Example 1, **Compound 57** (13 mg, yield: 2%) and **Compound 58** (79 mg, yield: 15%) were obtained as white solid, total yield: 15%.

**Compound 57**

LCMS (m/z): 410.2[M+1]$^+$.

**Compound 58**

$^1H$ NMR (400 MHz, MeOD) $\delta$ 7.48-7.29(m, 5H), 5.39(s, 1H), 4.54(s, 1H), 4.50-4.38(m, 3H), 4.22(s, 1H), 4.06(s, 1H), 3.96(dd, *J*=26.9, 11.5 Hz, 2H), 2.77(d, *J*=2.6 Hz, 1H).

LCMS (m/z): 392.1[M+1]$^+$.

Example 46: Synthesis of benzyl ((4R,4aR,5R,6S,7S,9S,10S,10aR,11S,E)-4,6,9,10,11-pentahydroxy-6-(methoxymethyl)octahydro-5,9-epoxy-7,10a-methanooxocino[4,5-d]pyrimidin-2(1H)-ylidene)carbamate **(Compound 59)** and benzyl ((4S,5aS,6S,8R,9S,10S,11S,11aR,12R,E)-6,9,11-trihydroxy-9-(methoxymethyl)octahydro-4,8,11a-(epimethanetriyl)-6,10-epoxyoxocino[4,3-f][1,3,5]oxadiazepin-2(1H)-ylidene)carbamate **(Compound 60)**

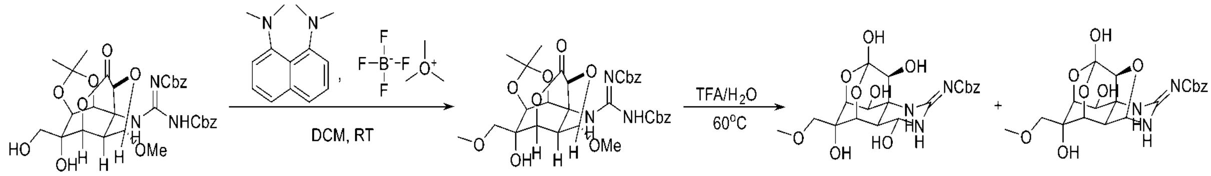


TTX-42 59-1 59 60

Step 1: Synthesis of **Compound 59-1**

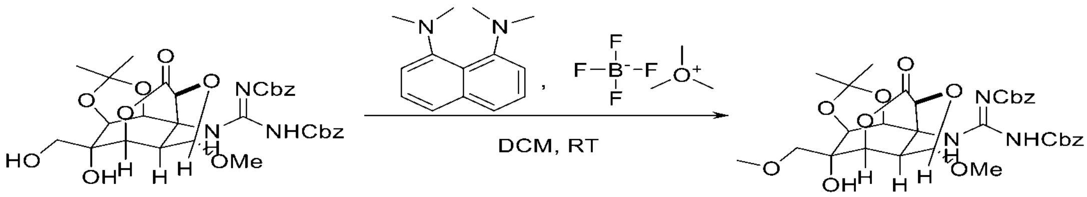


TTX-42 59-1

**TTX-42** (250 mg, 0.39 mmol) and anhydrous dichloromethane (8 mL) were added to a 50 mL single-necked flask, which was cooled to about 0 °C under nitrogen protection. 1,8-bismethylaminonaphthalene (251 mg, 1.17 mmol) was added, and then trimethyloxonium tetrafluoroborate (115 mg, 0.78 mmol) was added. After the addition was completed, the temperature was naturally raised to room temperature, and the reaction mixture was stirred overnight. HPLC was used to detect the complete reaction of most raw materials. Saturated sodium bicarbonate solution (15 mL) was added to the reaction solution, which was extracted with dichloromethane (15 mL×3). The organic phases were combined, washed twice with 0.25 M diluted hydrochloric acid (10 mL×2), once with saturated sodium chloride solution (20 mL), dried over anhydrous sodium sulfate, and filtered, and the filtrate was concentrated to give a crude product. The crude product was purified with prep-TLC (developer: petroleum ether/ethyl acetate=3:1) to give **Compound 59-1** as pale yellow oil (167 mg, yield: 65.3%).

$^1H$ NMR (400 MHz, $CDCl_3$) $\delta$ 11.60(s, 1H), 8.94(s, 1H), 7.43-7.28(m, 10H), 5.28-5.09(m, 5H), 4.71(s, 1H), 4.63(s, 1H), 4.63 4.51-4.32(m, 1H), 4.21-4.01(m, 1H), 3.94(s, 1H), 3.84(d, $J$ = 9.8 Hz, 1H), 3.75(s, 1H), 3.68(d, $J$ = 10.0 Hz, 1H), 3.50(s, 3H), 3.29(s, 2H), 2.07(s, 1H), 1.41(s, 3H), 1.25(m, 3H); LCMS (m/z): 656.2[M+1]$^+$.

Step 2: Synthesis of **Compound 59** and **Compound 60**

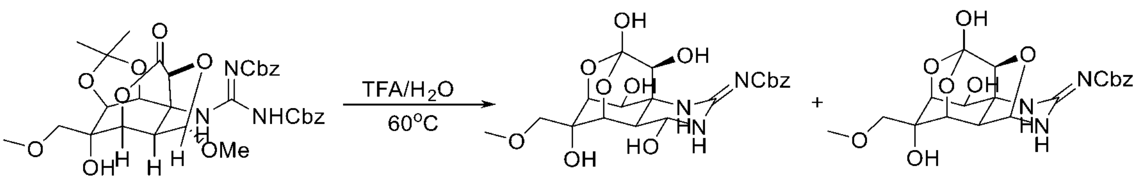


59-1 59 60

According to the synthesis procedures of Example 1, **Compound 59** as white solid (38.4 mg, yield: 33%) and **Compound 60** as white solid (23.7 mg, yield: 21%) were obtained. Total yield: 54%.

**Compound 59**

$^1$H NMR (400 MHz, MeOD) $\delta$ 7.43-7.21(m, 5H), 5.54(d, $J$ = 9.4 Hz, 1H), 5.05(s, 2H), 4.51-3.66(m, 6H), 3.44(s, 3H), 2.22(d, $J$ = 9.4 Hz, 1H); LCMS (m/z): 468.2[M+1]$^+$.

**Compound 60:**

$^1$H NMR (400 MHz, MeOD) $\delta$ 7.40-7.20(m, 5H), 5.27(s, 1H), 5.07(s, 2H), 4.47(d, $J$ = 1.9 Hz, 1H), 4.42(s, 1H), 4.17(s, 1H), 4.01(s, 1H), 3.84(d, $J$ = 9.7 Hz, 1H), 3.75(d, $J$ = 9.7 Hz, 1H), 3.44(s, 3H), 2.68(d, $J$ = 2.8 Hz, 1H); LCMS (m/z): 450.1[M+1]$^+$.

Example 47: Synthesis of (4R,4aR,5R,6S,7S,9S,10S,10aR,11S)-2-imino-6-(methoxymethyl)octahydro-5,9-epoxy-7,10a-methanooxocino[4,5-d]pyrimidine-4,6,9,10,11(10H)-pentaol **(Compound 61)**

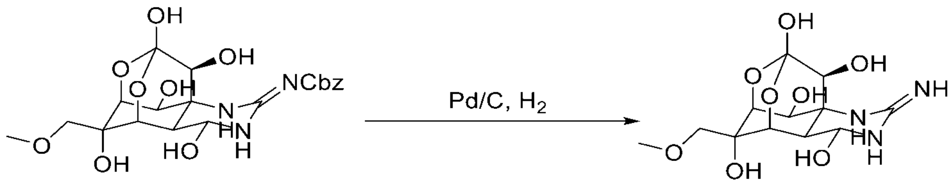


59 61

According to the synthesis procedures of Example 2, after the reaction was completed, the reaction solution was filtered, the filter cake was collected, to which deionized water (30 mL) and 2.5% acetic acid aqueous solution (5 mL) were added, stirred at room temperature for 1 h, and filtered. This operation was repeated once. All filtrates were combined and concentrated to give a crude product. Two drops of methanol were added to the crude product, and all the products were dissolved. Then methyl tert-butyl ether (10 mL) was added, the mixture was stirred for 30 min, allowed to stand, and the supernatant was removed. Then, methyl tert-butyl ether (10 mL) was added again, the mixture was stirred for 30 min, allowed to stand, and the supernatant was removed. The residue was concentrated to give **Compound 61 acetate** as off-white solid (12.5 mg, yield: 98%).

$^1$H NMR (400 MHz, $CD_3OD+D_2O+CD_3COOD$) $\delta$ 5.40(d, $J$ = 9.2 Hz, 1H), 4.32-3.98(m, 2H), 3.88-3.47(m, 4H), 3.32(s, 3H), 2.12(d, $J$ = 9.3 Hz, 1H), 1.86(s, 3H); LCMS (m/z): 334.1[M+1]$^+$.

Example 48: Synthesis of (4S,5aS,6S,8R,9S,10S,11S,11aR,12R)-2-imino-9-(methoxymethyl)octahydro-4,8,11a-(epimethanetriyl)-6,10-epoxyoxocino[4,3-f][1,3,5]oxadiazepine-6,9,11(5aH)-triol **(Compound 62)**

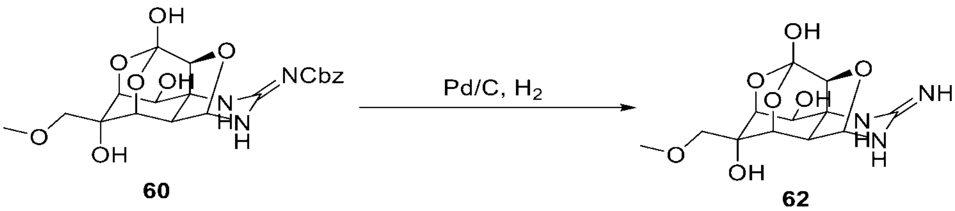


60 62

According to the synthesis procedures of Example 47, **Compound 62 acetate** was obtained as off-white solid (12 mg, yield: 96%).

$^1$H NMR (400 MHz, $D_2O+CD_3COOD$) $\delta$ 5.45(s, 1H), 4.54(d, $J$ = 2.2 Hz, 1H), 4.50(s, 1H), 4.28(dd, $J$= 2.9, 1.6 Hz, 1H), 4.10(t, $J$= 1.8 Hz, 1H), 3.84(d, $J$ = 10.8 Hz, 1H), 3.75(d, $J$ = 10.8 Hz, 1H), 3.39(s, 3H), 2.87(d, $J$ = 2.8 Hz, 1H), 1.95(s, 3H); LCMS (m/z): 316.1[M+1]$^+$.

Example 49: Synthesis of benzyl ((4R,4aR,5R,6S,7S,9S,10S,10aR,11S,E)-6-((benzylamino)methyl)-4,6,9,10,11-pentahydroxyoctahydro-5,9-epoxy-7,10a-methanooxocino[4,5-d]pyrimidin-2(1H)-ylidene)carbamate **(Compound 63)** and benzyl ((4S,5aS,6S,8R,9S,10S,11S,11aR,12R,E)-9-((benzylamino)methyl)-6,9,11-trihydroxyoctahydro-4,8,11a-(epimethanetriyl)-6,10-epoxyoxocino[4,3-f][1,3,5]oxadiazepin-2(1H)-ylidene)carbamate **(Compound 64)**

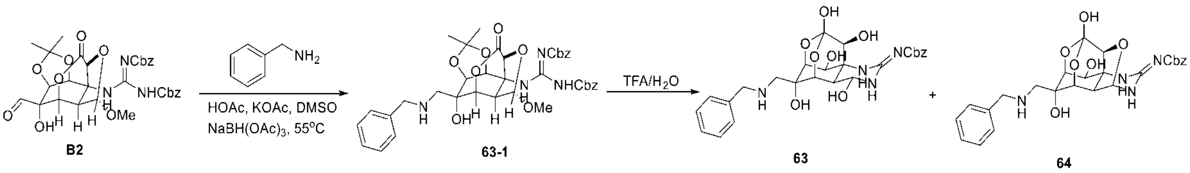


B2 63-1 63 + 64

Step 1: Synthesis of **Compound 63-1**

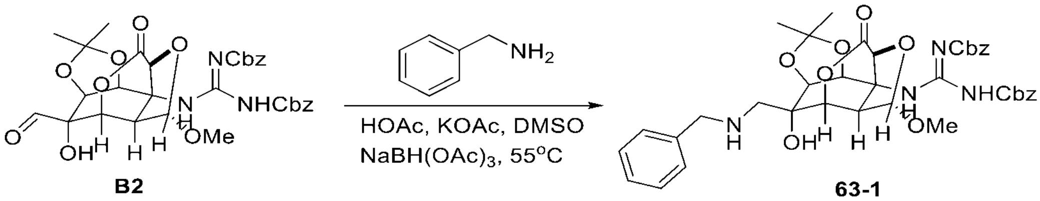


B2 63-1

**B2** (490 mg, 0.77 mmol, crude), dimethyl sulfoxide (8 mL), benzylamine (123 mg, 1.15 mmol), potassium acetate (432 mg, 4.41 mmol) and acetic acid (0.54 g, 9.04 mml) were added to a reaction flask, to which sodium triacetylborohydride (1.83 g, 8.66 mmol) was added under stirring. The reaction mixture was heated to 55 °C under nitrogen protection, and the reaction was carried out for 3 h. HPLC was used to monitor complete reaction of the raw materials. The reaction solution was cooled to room temperature, then water (40 mL) was added to the reaction solution to precipitate a large amount of pale yellow solid, stirring was continued for 30 min. Filtration was performed, and the filter cake was washed with water (10 mL×2), collected, dissolved with ethyl acetate (15 mL), washed with saturated sodium chloride solution (15 mL), dried over anhydrous sodium sulfate, filtered, and concentrated to give a residue (440 mg). The residue was subjected to column chromatography (eluent: petroleum ether/ethyl acetate = 10:1-2:1, then replaced with dichloromethane/methanol = 40:1-10:1) to give a crude product (220 mg), the purity of which was detected by HPLC as about 70%, and further subjected to prep-TLC (developer: dichloromethane/methanol = 25:1) to give **Compound 63-1** as pale yellow foam solid (120 mg, yield: 21.3%).

$^1$H NMR (400 MHz, $CDCl_3$) $\delta$ 11.59(s, 1H), 8.90(s, 1H), 7.43-7.31(m, 15H), 5.26-5.17(m, 4H), 5.17-5.13(m, 2H), 5.11(d, $J$ = 2.1 Hz, 1H), 4.61-4.56(m, 2H), 4.32(d, $J$ = 8.2 Hz, 1H), 3.94-3.85(m, 3H), 3.26(s, 3H), 3.18(d, $J$= 12.6 Hz, 1H), 2.94(d, $J$= 12.6 Hz, 1H), 1.37(s, 3H), 1.22(s, 3H); LCMS (m/z): 731.3[M+1]$^+$.

Step 2: Synthesis of **Compound 63** and **Compound 64**

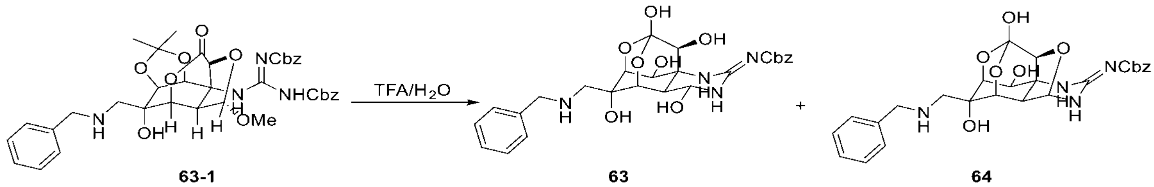


63-1 63 + 64

According to the synthesis procedures of Example 1, **Compound 63** as white solid (7.1 mg, yield: 8.3%) and **Compound 64** as white solid (1.8 mg, yield: 2.2%) were obtained, total yield: 10.5%.

**Compound 63**

$^1$H NMR (400 MHz, MeOD) δ 7.41-7.22(m, 10H), 5.53(d, $J$ = 9.5 Hz, 1H), 5.05(d, $J$ = 3.6 Hz, 2H), 4.24-3.94(m, 4H), 3.93-3.85(m, 2H), 3.22-3.09(m, 2H), 2.20(dd, $J$ = 16.7, 8.8 Hz, 1H); LCMS (m/z): 543.2[M+1]$^+$.

**Compound 64**

$^l$H NMR (400 MHz, MeOD) δ 7.41-7.21(m, 10H), 5.27(s, 1H), 5.07(s, 2H), 4.46(d, $J$= 2.1 Hz, 1H), 4.42(s, 1H), 4.15(dd, $J$ = 3.0, 1.6 Hz, 1H), 4.03(d, $J$ = 1.7 Hz, 1H), 3.86(s, 2H), 3.11(q, $J$ = 12.7 Hz, 2H), 2.69(d, $J$ = 2.9 Hz, 1H); LCMS (m/z): 525.2[M+1]$^+$.

Example 50: Synthesis of benzyl ((4R,4aR,5R,6S,75,9S,10S,10aR,11S,E)-4,6,9,10,11-pentahydroxy-6-((3-hydroxyazetidin-1-yl)methyl)octahydro-5,9-epoxy-7,10a-methanooxocino[4,5-d]pyrimidin-2(1H)-ylidene)carbamate **(Compound 65)** and benzyl ((4S,5aS,6S,8R,9S,10S,11S,11aR,12R,E)-6,9,11-trihydroxy-9-((3-hydroxyazetidin-1-yl)methyl) octahydro-4,8,11a-(epimethanetriyl)-6,10-epoxyoxocino[4,3-f][1,3,5]oxadiazepin-2(1H)-ylidene)carbamate **(Compound 174)**

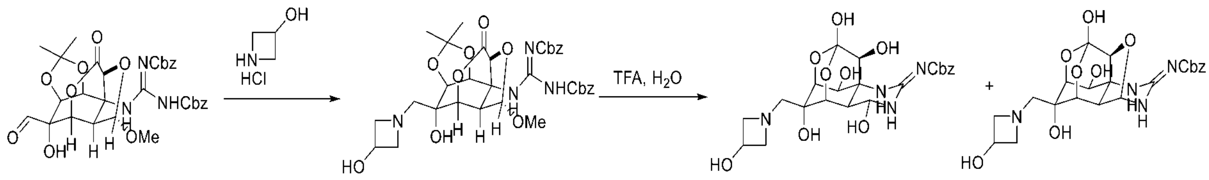


B2 65-1 65 174

Step 1: Synthesis of **Compound 65-1**

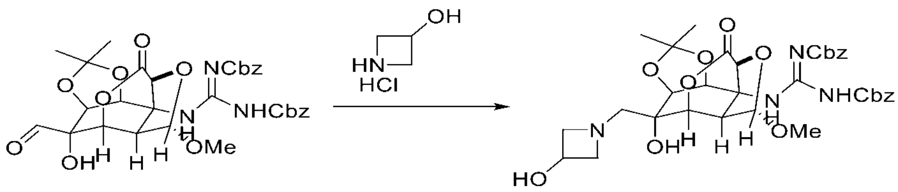


B2 65-1

According to the synthesis procedures of **Compound 63-1** in Example 49, after the reaction was completed, the reaction solution was cooled to room temperature, then added to water (30 mL) to precipitate a white solid, which was filtered. The filter cake was washed with water (10 mL×2), collected, and dried to give **Compound 65-1** as white solid (160 mg, yield: 86%). LCMS (m/z): 697.3[M+1]$^+$.

Step 2: Synthesis of **Compound 65** and **Compound 174**

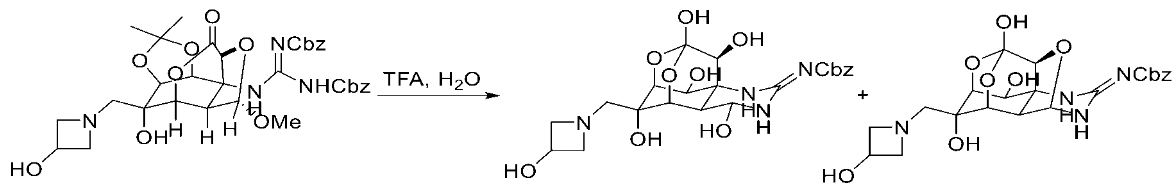


65-1 65 174

According to the synthesis procedures of Example 1, **Compound 65** (58.8 mg, yield: 21.2%) and **Compound 174** (11.9 mg, yield: 4.5%) were obtained as white solid, total yield: 25.7%.

**Compound 65**:$^1$H NMR (400 MHz, MeOD) δ 7.49-7.31(m, 5H), 5.76-5.63(m, 1H), 5.29(s, 2H), 4.68(s, 1H), 4.54(s, 2H), 4.24(s, 1H), 4.16(s, 2H), 4.09(s, br, 1H), 4.03-3.90(m, 3H), 3.85(d, J = 13.2 Hz, 1H), 2.37(d, J = 9.1 Hz, 1H); LCMS (m/z): 509.2[M+1]$^+$.

**Compound 174**:$^1$H NMR (400 MHz, MeOD) δ 7.39-7.19(m, 5H), 5.26(s, 1H), 5.07(s, 2H), 4.45(d, J = 2.1 Hz, 1H), 4.41(s, 1H), 4.36(t, J = 6.2 Hz, 1H), 4.08(dd, J = 2.9, 1.5 Hz, 1H), 3.94(s, 1H), 3.78-3.70(m, 2H), 3.09(d, J = 6.0 Hz, 2H), 3.06(d, J = 4.3 Hz, 1H), 2.97(d, J = 13.3 Hz, 1H), 2.67(d, J = 3.0 Hz, 1H); LCMS (m/z): 491.2[M+1]$^+$.

Example 51: Synthesis of (4R,4aR,5R,6S,7S,9S,10S,10aR,11S)-6-((3-hydroxyazetidin-1-yl)methyl)-2-iminooctahydro-5,9-epoxy-7,10a-methanooxocino[4,5-d]pyrimidine-4,6,9,10,11(10H)-pentaol **(Compound 66)**

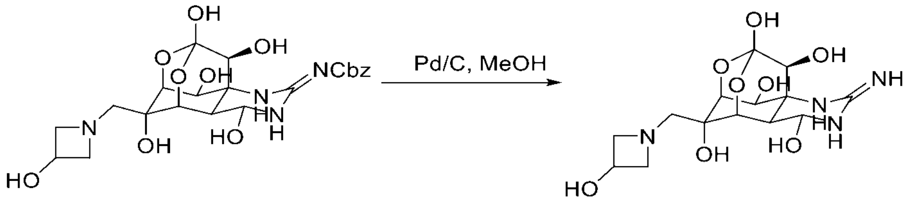


65

66

According to the synthesis procedures of Example 2, **Compound 66** was obtained as white solid (17 mg, yield: 68%).

$^1$H NMR (400 MHz, $D_2O$) $\delta$ 5.40(d, J= 9.5 Hz, 1H), 4.66(s, 1H), 4.55-4.44(m, 2H), 4.23(d, *J= 11.2* Hz, 1H), 4.17-3.90(m, 5H), 3.88-3.67(m, 2H), 2.27(d, *J* = 9.4 Hz, 1H); LCMS (m/z): 375.1[M+1]$^+$.

Example 52: Synthesis of (4R,4aR,5R,6S,7S,9S,10S,10aR,11S,E)-2-(((benzyloxy)carbonyl)imino)-4,6,9,10,11-pentahydroxydecahydro-5,9-epoxy-7,10a-methanooxocino[4,5-d]pyrimidine-6-carboxylic acid **(Compound 67)** and (4S,5aS,6S,8R,9S,10S,11S,11aR,12R,E)-2-(((benzyloxy)carbonyl)imino)-6,9,11-trihydroxydecahydro-4,8,11a-(epimethanetriyl)-6,10-epoxyoxocino[4,3-f][1,3,5]oxadiazepine-9-carboxylic acid **(Compound 68)**

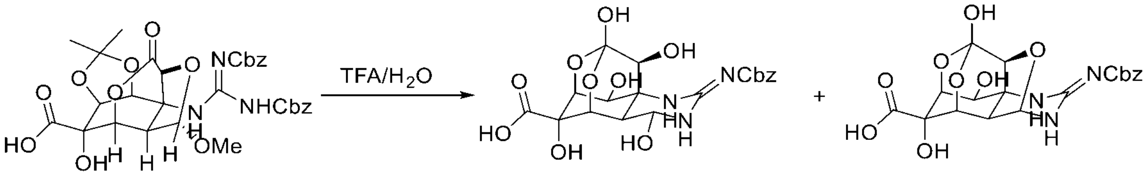


B1

67

68

According to the synthesis procedures of Example **1, Compound 67** as white solid (14.7 mg, yield: 21%) and **Compound 68** (25.0 mg, yield: 37%) as white solid were obtained, total yield: 58%.

**Compound 67**

$^1$H NMR (400MHz, MeOD) $\delta$ 7.50-7.22(m, 5H), 5.60(s, 1H), 5.15(s, 2H), 4.34(m, 4H), 2.25(d, *J* = 9.2Hz, 1H); LCMS (m/z): 468.1[M+1]$^+$.

**Compound 68**

$^1$H NMR (400MHz, MeOD) $\delta$ 7.46-7.20(m, 5H), 5.32(s, 1H), 5.09(s, 2H), 4.59-4.33(m, 4H), 2.72(d, *J* = 3.0Hz, 1H); LCMS (m/z): 450.1[M+1]$^+$.

Example 53: Synthesis of (4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidine-6-carboxylic acid **(Compound 69)**

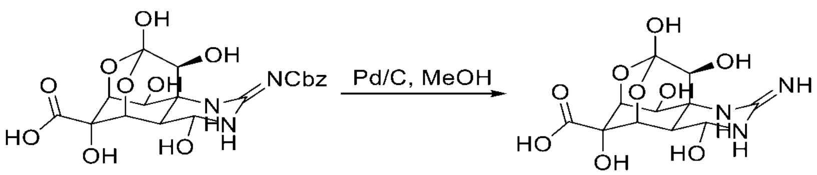


67

69

According to the synthesis procedures of Example 2, **Compound 69** was obtained as white solid (8.1 mg, yield: 77%).

$^1$H NMR (400MHz, $D_2O$) $\delta$ 5.44(d, *J* = 9.5Hz, 1H), 4.44(d, *J* = 66.7Hz, 2H), 4.02(dd, *J* = 61.4, 49.6 Hz, 2H), 2.22(d,

*J* = 9.0Hz, 1H); LCMS (m/z): 334.1[M+1]+.

Example 54: Synthesis of (4S,5aS,6S,8R,9S,10S,11S,11aR,12R)-6,9,11-trihydroxy-2-iminodecahydro-4,8,11a-(epimethanetriyl)-6,10-epoxyoxocino[4,3-f][1,3,5]oxadiazepine-9-carboxylic acid **(Compound 70)**

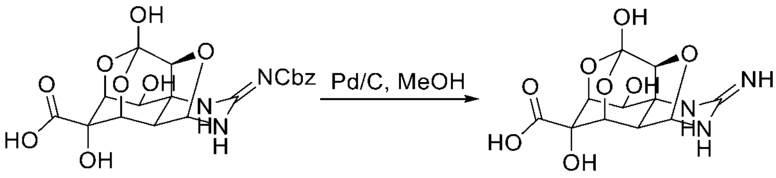


68 70

According to the synthesis procedures of Example 2, **Compound 70** was obtained as white solid (12.0 mg, yield: 69%).

$^1H$ NMR (400MHz, $D_2O$) $\delta$ 5.45(s, 1H), 4.54(s, 1H), 4.52-4.42(m, 3H), 2.84(d, *J* = 2.7Hz, 1H); LCMS (m/z): 316.1 [M+1]+.

Example 55: Synthesis of benzyl ((4R,4aR,5R,6S,7S,9S,10S,10aR,11S,E)-6-carbamoyl-4,6,9,10,11-pentahydroxyoctahydro-5,9-epoxy-7,10a-methanooxocino[4,5-d]pyrimidin-2(1H)-ylidene)carbamate **(Compound 71)** and benzyl ((4S,5aS,6S,8R,9S,10S,11S,11aR,12R,E)-9-carbamoyl-6,9,11-trihydroxyoctahydro-4,8,11a-(epimethanetriyl)-6,10-epoxyoxocino[4,3-f][1,3,5]oxadiazepin-2(1H)-ylidene)carbamate **(Compound 72)**

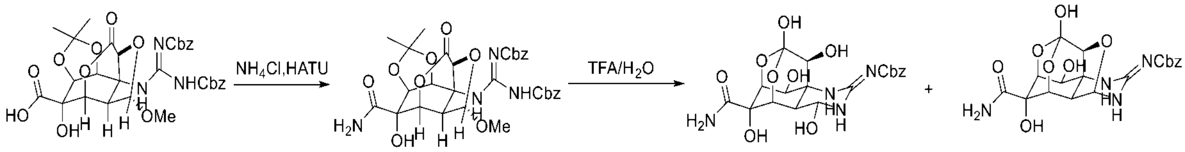


B1 71-1 71 72

Step 1: Synthesis of **Compound 71-1**

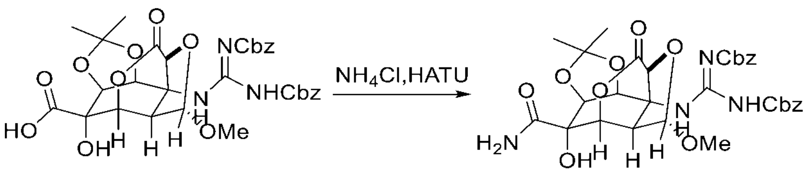


B1 71-1

**B1** (200 mg, 0.31 mmol) and dichloromethane (3 mL) were added into a reaction flask, to which ammonium chloride (20.0 mg, 0.37 mmol), triethylamine (94.0 mg, 0.93 mmol) and 2-(7-azobenzotriazole)- *N,N,N',N'* -tetramethyluronium hexafluorophosphate (143 mg, 0.38 mmol) were added under stirring. The reaction mixture was stirred at room temperature for 16 h after the addition was completed. HPLC was used to monitor complete reaction of the raw materials. Water (20 mL) was added to the reaction solution, which was extracted with dichloromethane (30 mL×3). The organic phases were combined, washed with saturated sodium chloride solution (30 mL), dried over anhydrous sodium sulfate, and concentrated to give a crude product. The crude product was purified by flash column chromatography (ethyl acetate/petroleum ether = 50%) to give **Compound 71-1** as oily liquid (195 mg, yield: 96%). LCMS (m/z): 655.2[M+1]+.

Step 2: Synthesis of **Compound 71** and **Compound 72**

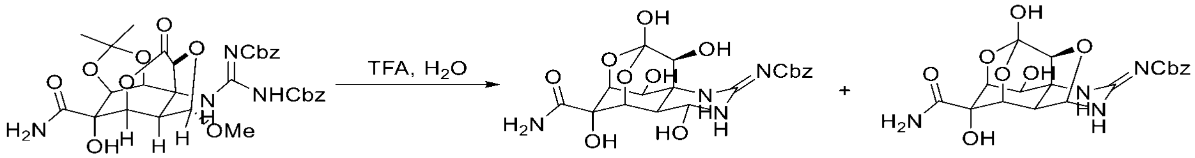


71-1 71 72

According to the synthesis procedures of Example 1, **Compound 71** as white solid (12 mg, yield: 9%) and **Compound 72** as white solid (16 mg, yield: 12%) were obtained, total yield: 21%.

**Compound 71**

$^1H$ NMR (400 MHz, MeOD) $\delta$ 7.43-7.22(m, 5H), 5.57(d, $J$ = 9.5 Hz, 1H), 5.07(s, 2H), 4.34(d, $J$ =28.3 Hz, 1H), 4.16(d, $J$ = 35.0 Hz, 3H), 2.29(d, $J$ = 9.3 Hz, 1H); LCMS (m/z): 467.1[M+1]$^+$.

**Compound 72**

$^1H$ NMR (400 MHz, MeOD) $\delta$ 7.43-7.21(m, 5H), 5.31(s, 1H), 5.07(s, 2H), 4.53-4.45(m, 2H), 4.31(dd, $J$ = 3.0, 1.6 Hz, 1H), 4.17(s, 1H), 2.77(d, $J$ = 3.0 Hz, 1H); LCMS (m/z): 449.1[M+1]$^+$.

Example 56: Synthesis of (4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidine-6-carboxamide **(Compound 73)**

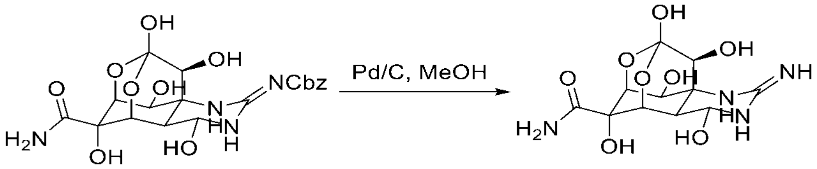


71 73

According to the synthesis procedures of Example 2, **Compound 73** was obtained as white solid (4 mg, yield: 60%).

$^1H$ NMR (400 MHz, $D_2O$) $\delta$ 5.44(d, $J$= 9.4 Hz, 1H), 4.51(s, 1H), 4.32(s, 1H), 4.24(s, 1H), 3.92(s, 1H), 2.30(d, $J$ = 9.4 Hz, 1H); LCMS (m/z): 333.1[M+1]$^+$.

Example 57: Synthesis of (4S,5aS,6S,8R,9S,10S,11S,11aR,12R)-6,9,11-trihydroxy-2-iminodecahydro-4,8,11a-(epimethanetriyl)-6,10-epoxyoxocino[4,3-f][1,3,5]oxadiazepine-9-carboxamide **(Compound 74)**

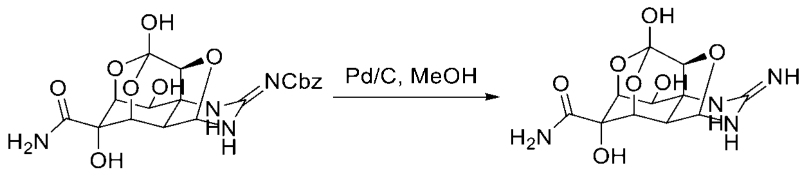


72 74

According to the synthesis procedures of Example 2, **Compound 74** was obtained as white solid (5 mg, yield: 80%).

$^1H$ NMR (400 MHz, $D_2O$) $\delta$ 5.40(s, 1H), 4.50(d, $J$ = 2.2 Hz, 1H), 4.48(s, 1H), 4.45(dd, $J$ = 3.1, 1.8 Hz, 1H), 4.34(t, $J$ = 1.9 Hz, 1H), 2.87(d, $J$ = 3.0 Hz, 1H).

Example 58: Synthesis of benzyl ((4R,4aR,5R,6S,7S,9S,10S,10aR,11S,E)-4,6,9,10,11-pentahydroxy-6-(methylcarbamoyl)octahydro-5,9-epoxy-7,10a-methanooxocino[4,5-d]pyrimidin-2(1H)-ylidene)carbamate **(Compound 75)** and benzyl ((4S,5aS,6S,8R,9S,10S,11S,11aR,12R,E)-6,9,11-trihydroxy-9-(methylcarbamoyl)octahydro-4,8,11a-(epimethanetriyl)-6,10-epoxyoxocino[4,3-f][1,3,5]oxadiazepin-2(1H)-ylidene)carbamate **(Compound 76)**

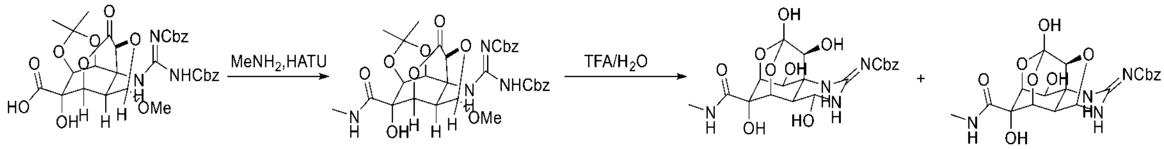


B1 75-1 75 76

Step 1: Synthesis of **Compound 75-1**

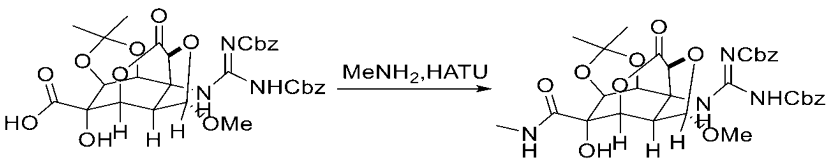


B1 75-1

According to the synthesis procedures of **Compound 71-1** in Example 55, **Compound 75-1** was obtained as oily liquid (200 mg, yield: 96%). LCMS (m/z): 669.2[M+1]$^+$.

Step 2: Synthesis of **Compound 75** and **Compound 76**

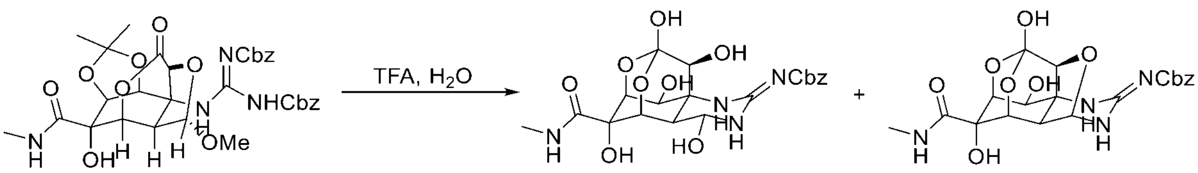


75-1 75 76

According to the synthesis procedures of Example 1, **Compound 75** was obtained as white solid (33 mg, yield: 23%) and **Compound 76** was obtained as white solid (21 mg, yield: 15%), total yield: 38%.

**Compound 75**

$^1$H NMR (400 MHz, MeOD) $\delta$ 7.41-7.27(m, 5H), 5.58(d, $J$ = 9.4 Hz, 1H), 5.09(s, 2H), 4.32(s, 1H), 4.21(s, 1H), 4.18-3.99(m, 2H), 2.87(s, 3H), 2.30(d, $J$ = 9.4 Hz, 1H); LCMS (m/z): 481.2[M+1]$^+$.

**Compound 76**

$^1$H NMR (400 MHz, MeOD) $\delta$ 7.41-7.23(m, 5H), 5.30(s, 1H), 5.07(s, 2H), 4.52-4.46(m, 2H), 4.31(dd, $J$ = 3.0, 1.5 Hz, 1H), 4.17(s, 1H), 2.87(s, 3H), 2.77(d, $J$ = 2.9 Hz, 1H); LCMS (m/z): 463.1[M+1]$^+$.

Example 59: Synthesis of (4R,4aR,5R,6S,7S,9S,10S,10aR,11S)-4,6,9,10,11-pentahydroxy-2-imino-N-methyldecahydro-5,9-epoxy-7,10a-methanooxocino[4,5-d]pyrimidine-6-carboxamide **(Compound 77)**

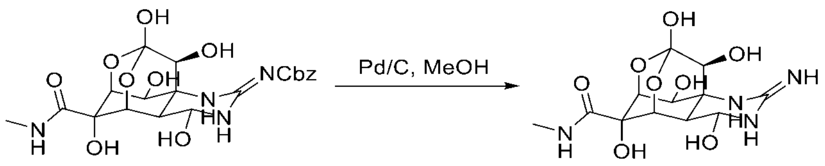


75 77

According to the synthesis procedures of Example 2, **Compound 77** was obtained as white solid (15 mg, yield: 72%).

$^1$H NMR (400 MHz, $D_2O$) $\delta$ 5.62(d, $J$= 9.5 Hz, 1H), 4.63(s, 1H), 4.50(s, 1H), 4.43(s, 1H), 4.16(s, 1H), 2.97(s, 3H), 2.48(d, $J$ = 9.4 Hz, 1H); LCMS (m/z): 347.1[M+1]$^+$.

Example 60: Synthesis of (4S,5aS,6S,8R,9S,10S,11S,11aR,12R)-6,9,11-trihydroxy-2-imino-N-methyldecahydro-4,8,11a-(epimethanetriyl)-6,10-epoxyoxocino[4,3-f][1,3,5]oxadiazepine-9-carboxamide **(Compound 78)**

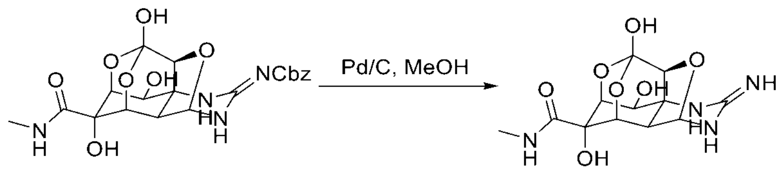


76 78

According to the synthesis procedures of Example 2, **Compound 78** was obtained as white solid (10 mg, yield: 61%).

$^1$H NMR (400 MHz, $D_2O$) $\delta$ 5.59(s, 1H), 4.70(d, $J$ = 2.2 Hz, 1H), 4.64(s, 1H), 4.58(dd, $J$ = 3.0, 1.7 Hz,1H), 4.47(s, 1H), 3.05(d, $J$ = 3.0 Hz, 1H), 2.93(s, 3H); LCMS (m/z): 329.1[M+1]$^+$.

Example 61: Synthesis of benzyl ((4R,4aR,5R,6S,7S,9S,10S,10aR,11S,E)-6-(dimethylcarbamoyl)-4,6,9,10,11-pentahydroxyoctahydro-5,9-epoxy-7,10a-methanooxocino[4,5-d]pyrimidin-2(1H)-ylidene)carbamate **(Compound 79)** and benzyl ((4S,5aS,6S,8R,9S,10S,11S,11aR,12R,E)-9-(dimethylcarbamoyl)-6,9,11-trihydroxyoctahydro-4,8,11a-(epimethanetriyl)-6,10-epoxyoxocino[4,3-f][1,3,5]oxadiazepin-2(1H)-ylidene)carbamate **(Compound 80)**

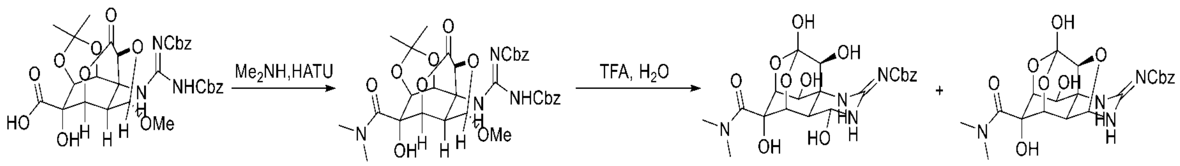


B1 79-1 79 80

Step 1: Synthesis of **Compound 79-1**

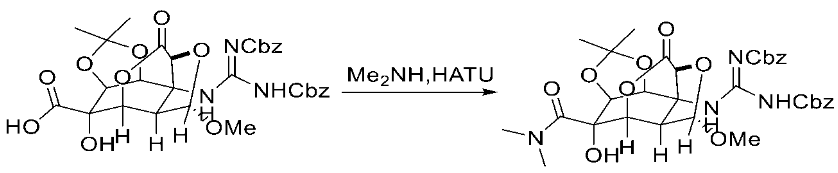


B1 79-1

According to the synthesis procedures **Compound 71-1** in Example 55, **Compound 79-1** was obtained as off-white solid (200 mg, yield: 95%). LCMS (m/z): 683.3[M+1]$^+$.

Step 2: Synthesis of **Compound 79** and **Compound 80**

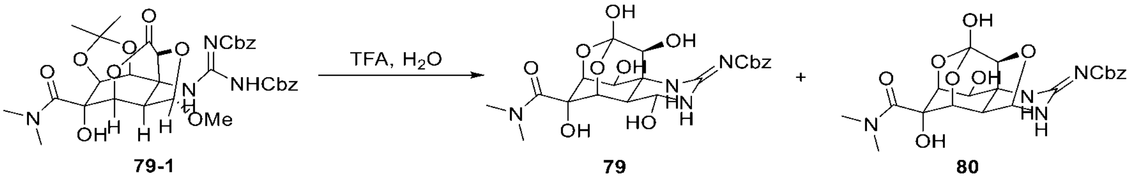


79-1 79 80

According to the synthesis procedures of Example 1, **Compound 79** (24 mg, yield: 17%) and **Compound 80** (16 mg, yield: 12%) were obtained as white solid, total yield: 29%.

**Compound 79**

$^1$H NMR (400 MHz, MeOD) $\delta$ 7.42-7.22(m, 5H), 5.58(d, $J$ = 9.5 Hz, 1H), 5.05(s, 2H), 4.64(d, $J$ = 42.6 Hz, 1H), 4.45(s, 1H), 4.17(dd, $J$ = 103.5, 50.7 Hz, 2H), 3.41(s, 3H), 3.01(s, 3H), 2.26(d, $J$ = 9.5 Hz, 1H); LCMS (m/z): 495.2[M+1]$^+$.

**Compound 80**

$^1$H NMR (400 MHz, MeOD) $\delta$ 7.43-7.23(m, 5H), 5.31(s, 1H), 5.07(s, 2H), 4.77(s, 1H), 4.52(s, 1H), 4.49-4.43(m, 2H), 3.30-2.85(m, 6H), 2.76(d, $J$ = 2.8 Hz, 1H); LCMS (m/z): 477.2[M+1]$^+$.

Example 62: Synthesis of (4R,4aR,5R,6S,7S,9S,10S,10aR,11S)-4,6,9,10,11-pentahydroxy-2-imino-N,N-dimethyldecahydro-5,9-epoxy-7,10a-methanooxocino[4,5-d]pyrimidine-6-carboxamide **(Compound 81)**

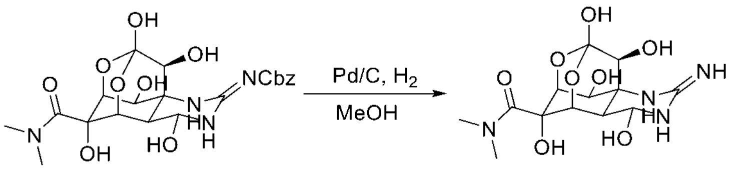


79 81

According to the synthesis procedures of Example 2, **Compound 81** was obtained as white solid (15 mg, yield: 100%).

$^1$H NMR (400 MHz, $D_2O$) $\delta$ 5.45(t, $J$ = 8.1 Hz, 1H), 4.64(s, 1H), 4.50(s, 1H), 4.24(d, $J$ = 8.3 Hz, 1H),3.91(d, $J$ = 7.2 Hz, 1H), 3.33-3.16(m, 3H), 2.92(s, 3H), 2.32(t, $J$ = 13.6 Hz, 1H); LCMS (m/z): 361.1[M+1]$^+$.

Example 63: Synthesis of (4S,5aS,6S,8R,9S,10S,11S,11aR,12R)-6,9,11-trihydroxy-2-imino-N,N-dimethyldecahydro-4,8,11a-(epimethanetriyl)-6,10-epoxyoxocino[4,3-f][1,3,5]oxadiazepine-9-carboxamide **(Compound 82)**

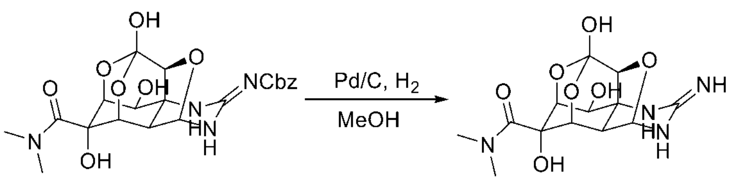


80 82

According to the synthesis procedures of Example 2, **Compound 82** was obtained as white solid (8 mg, yield: 100%).

$^1$H NMR (400 MHz, $D_2O$) $\delta$ 5.45(t, $J$ = 8.1 Hz, 1H), 4.64(s, 1H), 4.50(s, 1H), 4.24(d, $J$= 8.3 Hz, 1H),3.91(d, $J$ = 7.2 Hz, 1H), 3.33-3.16(m, 3H), 2.92(s, 3H), 2.32(t, $J$ = 13.6 Hz, 1H); LCMS (m/z): 343.1[M+1]$^+$.

Example 64: Synthesis of benzyl ((4S,5aS,6S,8R,9S,10S,11S,11aR,12R,E)-6,9,11-trihydroxy-9-(pyrrolidine-1-carbonyl)octahydro-4,8,11a-(epimethanetriyl)-6,10-epoxyoxocino[4,3-f][1,3,5]oxadiazepin-2(1H)-ylidene)carbamate **(Compound 83)** and benzyl ((4R,4aR,5R,6S,7S,9S,10S,10aR,11S,E)-4,6,9,10,11-pentahydroxy-6-(pyrrolidine-1-carbonyl) octahydro-5,9-epoxy-7,10a-methanooxocino[4,5-d]pyrimidin-2(1H)-ylidene)carbamate **(Compound 84)**

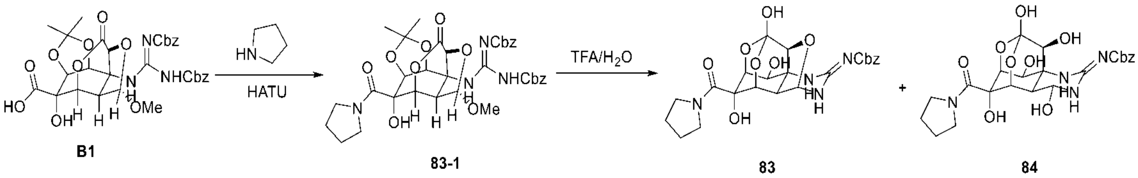


B1 83-1 83 84

Step 1: Synthesis of **Compound 83-1**

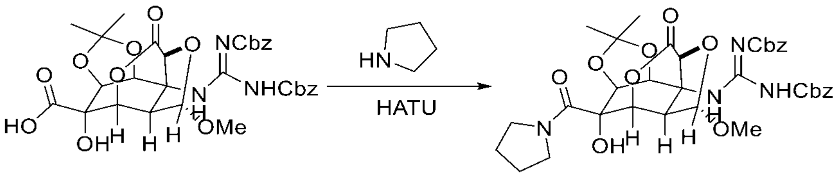


B1 83-1

According to the synthesis procedures of **Compound 71-1** in Example 55, **Compound 83-1** was obtained as pale yellow oil (150 mg, yield: 69%).

Step 2: Synthesis of **Compound 83** and **Compound 84**

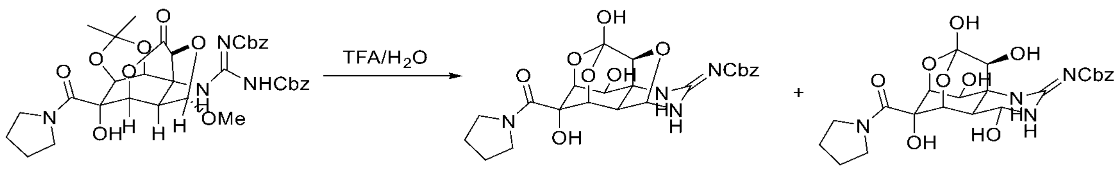


83-1 83 84

According to the synthesis procedures of Example 1, **Compound 83** as white solid (6.8 mg, yield: 6%) and **Compound 84** (15.0 mg, yield: 14%) as white solid were obtained, total yield: 20%.

**Compound 83**

$^1$H NMR (400 MHz, MeOD) $\delta$ 7.39-7.25(m, 5H), 5.31(s, 1H), 5.07(s, 2H), 4.77(s, 1H), 4.56(s, 1H), 4.49-4.41(m, 2H), 4.03-3.90(m, 1H), 3.67(d, $J$ = 10.3 Hz, 1H), 3.51(t, $J$ = 6.5 Hz, 2H), 2.73(d, $J$ = 2.7 Hz, 1H), 1.99-1.81(m, 4H); LCMS (m/z): 503.2[M+1]$^+$.

**Compound 84**

$^1$H NMR (400 MHz, MeOD) $\delta$ 7.39-7.24(m, 5H), 5.58(d, $J$ = 9.3 Hz, 1H), 5.06(s, 2H), 4.76(s, 1H), 4.47(s, 1H), 4.31(d, $J$ = 28.0 Hz, 1H), 4.14(s, 1H), 3.86(s, 1H), 3.79(s, 1H), 3.53(s, 2H), 2.25(s, 1H), 2.01-1.80(m, 4H); LCMS (m/z): 521.2[M+1]$^+$.

Example 65: Synthesis of benzyl ((4S,5aS,6S,8R,95,108,11S,11aR,12R,E)-6,9,11-trihydroxy-9-(piperidine-1-carbonyl) octahydro-4,8,11a-(epimethanetriyl)-6,10-epoxyoxocino[4,3-f][1,3,5]oxadiazepin-2(1H)-ylidene)carbamate **(Compound 85/Compound 86)** and piperidin-1-yl((4S,5aS,6S,8R,9S,10S,11S,11aR,12R)-6,9,11-trihydroxy-2-iminodecahydro-4,8,11a-(epimethanetriyl)-6,10-epoxyoxocino[4,3-f][1,3,5]oxadiazepin-9-yl)methanone **(Compound 87)**

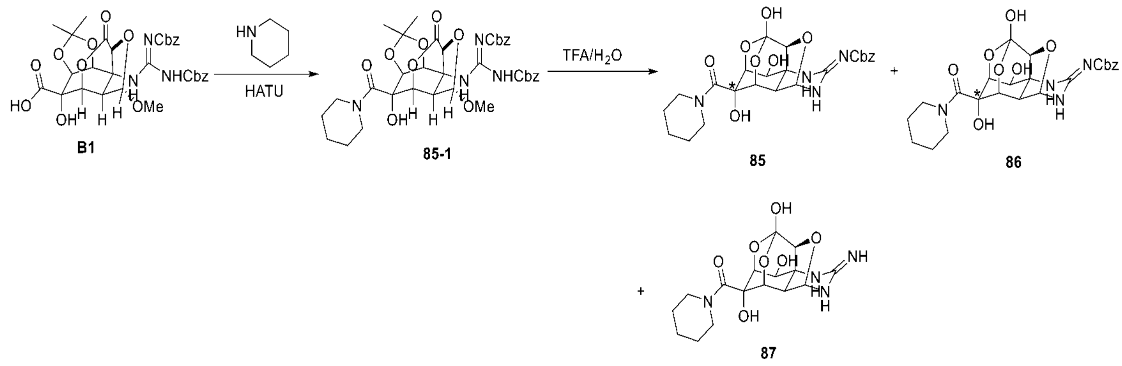

B1 85-1 85 86 87

Step 1: Synthesis of **Compound 85-1**

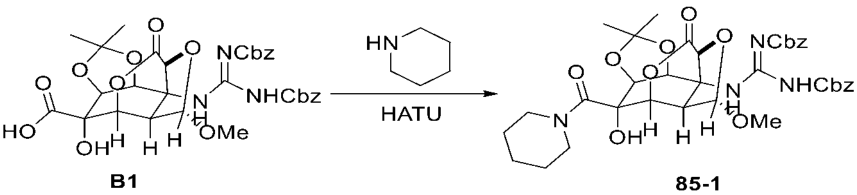


B1 85-1

According to the synthesis procedures of **Compound 71-1** in Example 55, **Compound** 85-1 was obtained as oil (215 mg, yield: 95%). LCMS (m/z): 723.3[M+1]$^+$.

Step 2: Synthesis of **Compound 85/Compound 86** and **Compound 87**

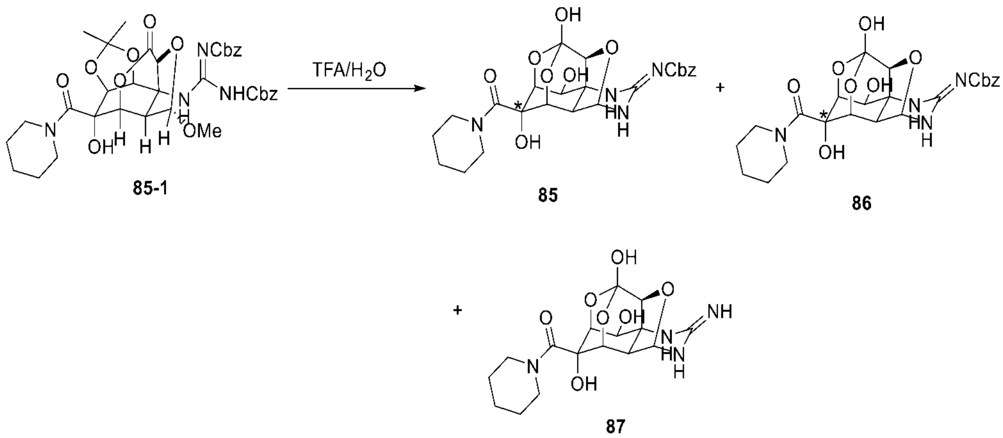

85-1 85 86 87

According to the synthesis procedures of Example 1, two stereoisomers of **Compound 85** as white solid (31 mg, yield: 21%) and **Compound 86** as white solid (16 mg, yield: 10%) with R or S conformation at *chiral carbon in the reaction formula were obtained, and at the same time **Compound 87** as white solid (16 mg, yield: 15%) was obtained, total yield: 46%.

**Compound 85**

$^1H$ NMR (400 MHz, MeOD) $\delta$ 7.41-7.25(m, 5H), 5.32(s, 1H), 5.08(s, 2H), 4.69(s, 1H),4.48(s, 2H), 4.45(s, 1H), 3.84-3.60(m, 4H), 2.77(d, J = 2.7 Hz, 1H), 1.76-1.65(m, 2H), 1.64-1.54(m, 4H); LCMS (m/z): 517.2[M+1]$^+$.

**Compound 86**

$^1H$ NMR (400 MHz, MeOD) $\delta$ 7.41-7.22(m, 5H), 5.40(d, *J* = 3.6 Hz, 1H), 5.06(s, 2H), 4.61(s, 1H),4.48(d, *J* = 1.6 Hz, 1H), 4.28(d, *J* = 3.5 Hz, 1H), 3.96(dd, *J* = 47.3, 13.2 Hz, 2H), 3.39(d, *J* = 15.3 Hz,1H), 3.17(dd, *J* = 21.6, 12.0 Hz, 1H), 2.90-2.82(m, 1H), 1.78-1.44(m, 6H); LCMS (m/z): 517.2[M+1]$^+$.

**Compound 87**

$^1H$ NMR (400 MHz, $D_2O$) $\delta$ 5.43(s, 1H), 4.57(d, *J* = 1.9 Hz, 1H), 4.51(s, 1H), 4.48(s, 1H), 3.80-3.32(m, 5H), 2.92(d, *J* = 2.2 Hz, 1H), 1.55(d, *J* = 35.9 Hz, 6H).LCMS (m/z): 383.2[M+1]$^+$.

Example 66: Synthesis of (4R,4aR,5R,6S,7S,9S,10S,10aR,11S)-6-(aminomethyl)-2-iminooctahydro-5,9-epoxy-7,10a-methanooxocino[4,5-d]pyrimidine-4,6,9,10,11(10H)-pentaol **(Compound 88)**

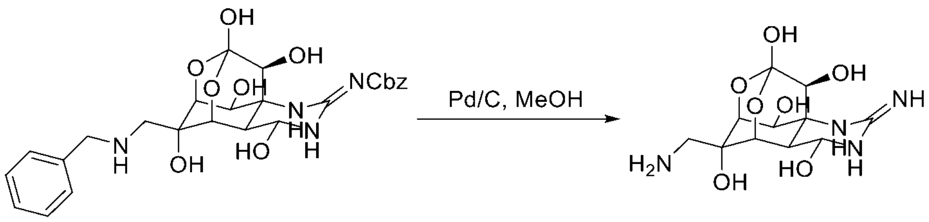


**63** **88**

According to the synthesis procedures of Example 2, **Compound 88** was obtained as white solid (21 mg, yield: 93%).

$^1H$ NMR (400 MHz, $D_2O$) $\delta$ 5.45-5.39(m, 1H), 4.29-4.22(m, 1H), 4.17(s, 1H), 4.07(d, *J* = 12.9 Hz, 1H), 3.93(dd, *J* = 14.4, 9.7 Hz, 1H), 3.58-3.36(m, 2H), 2.27(dd, *J* = 9.4, 4.4 Hz, 1H); LCMS (m/z): 319.1[M+1]$^+$.

Example 67: Synthesis of benzyl ((4R,4aR,5R,6S,75,9S,10S,10aR,11S,E)-4,6,9,10,11-pentahydroxy-6-((methylamino)methyl)octahydro-5,9-epoxy-7,10a-methanooxocino[4,5-d]pyrimidin-2(1H)-ylidene)carbamate **(Compound 89)** and benzyl ((4S,5aS,6S,8R,9S,10S,11S,11aR,12R,E)-6,9,11-trihydroxy-9-((methylamino)methyl)octahydro-4,8,11a-(epimethanetriyl)-6,10-epoxyoxocino[4,3-f][1,3,5]oxadiazepin-2(1H)-ylidene)carbamate **(Compound 90)**

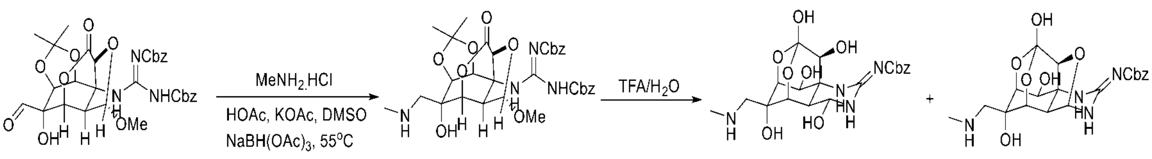


**B2** **89-1** **89** **90**

Step 1: Synthesis of **Compound 89-1**

According to the synthesis procedures of **Compound 63-1** in Example 49, after the reaction was completed, the reaction solution was cooled to room temperature, then added to water (30 mL) to precipitate a white solid, which was filtered. The filter cake was washed with water (10 mL×2), collected, and dried to give **Compound 89-1** as white solid (150 mg, yield: 92%). LCMS (m/z): 655.3[M+1]$^+$.

Step 2: Synthesis of **Compound 89** and **Compound 90**

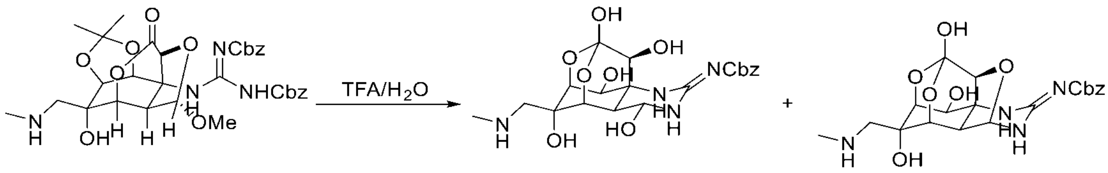


89-1 89 90

According to the synthesis procedures of Example 1, **Compound 89** as white solid (77 mg, yield: 72%) and **Compound 90** as white solid (5.2 mg, yield: 5%) were obtained, total yield: 77%.

**Compound 89**

$^1$H NMR (400 MHz, MeOD) $\delta$ 7.52-7.35(m, 5H), 5.73(d, $J$ = 9.4 Hz, 1H), 5.33(s, 2H), 4.30(dd, $J$ =8.5, 7.1 Hz, 2H), 4.06(s, 2H), 3.66(s, 2H), 2.82(d, $J$ = 3.1 Hz, 3H), 2.50-2.36(m, 1H). LCMS (m/z): 467.2[M+1]$^+$.

**Compound 90**

$^1$H NMR (400 MHz, MeOD) $\delta$ 7.34(d, $J$ = 11.6 Hz, 5H), 5.28(s, 1H), 5.07(s, 2H), 4.47(d, $J$ = 2.0 Hz, 1H), 4.43(s, 1H), 4.15(d, $J$ = 1.3 Hz, 1H), 4.02(s, 1H), 3.15(dd, $J$ = 27.7, 12.7 Hz, 2H), 2.70(d, $J$ = 3.3Hz, 1H), 2.53(s, 3H). LCMS (m/z): 449.2 [M+1]$^+$.

Example 68: Synthesis of (4R,4aR,5R,6S,7S,9S,10S,10aR,11S)-2-imino-6-((methylamino)methyl)octahydro-5,9-epoxy-7,10a-methanooxocino[4,5-d]pyrimidine-4,6,9,10,11(10H)-pentaol **(Compound 91)**

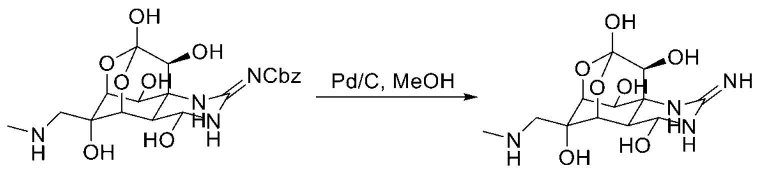


89 91

According to the synthesis procedures of Example 2, **Compound 91** was obtained as white solid (31 mg, yield: 79%).

$^1$H NMR (400 MHz, $D_2O$) $\delta$ 5.46(dd, $J$ = 14.6, 5.7 Hz, 1H), 4.33-4.21(m, 2H), 4.15(s, 1H), 4.09-3.97(m, 1H), 3.66-3.51(m, 2H), 2.78(s, 3H), 2.33(t, $J$ = 9.6 Hz, 1H); LCMS (m/z): 333.1[M+1]$^+$.

Example 69: Synthesis of benzyl ((4R,4aR,5R,6S,75,9S,10S,10aR,11S,E)-6-((dimethylamino)methyl)-4,6,9,10,11-pentahydroxyoctahydro-5,9-epoxy-7,10a-methanooxocino[4,5-d]pyrimidin-2(1H)-ylidene)carbamate **(Compound 92)**

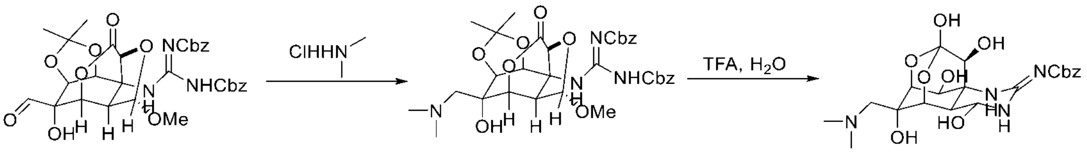


B2 92-1 92

Step 1: Synthesis of **Compound 92-1**

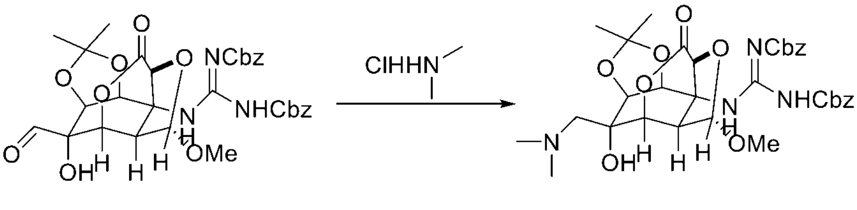


B2

92-1

According to the synthesis procedures of **Compound 63-1** in Example 49, after the reaction was completed, the reaction solution was cooled to room temperature, then added to water (30 mL) to precipitate a white solid, which was filtered. The filter cake was washed with water (10 mL×2), collected, and dried to give **Compound 92-1** as white solid (175 mg, yield: 100%). LCMS (m/z): 669.3[M+1]$^+$.

Step 2: Synthesis of **Compound 92**

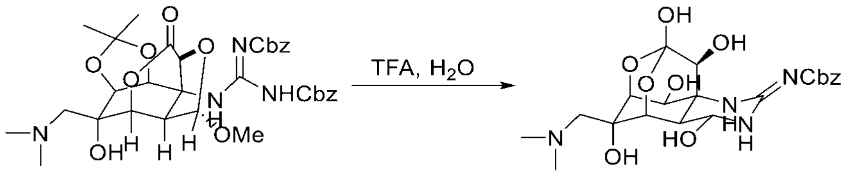


92-1

92

According to the synthesis procedures of Example 1, **Compound 92** was obtained as white solid (33 mg, yield: 27.5%).

$^1$H NMR (400 MHz, MeOD) $\delta$ 7.48-7.32(m, 5H), 5.69(d, $J$ = 9.4 Hz, 1H), 5.27(s, 2H), 4.26(d, $J$ = 18.4 Hz, 2H), 4.06(s, 2H), 3.88-3.62(m, 2H), 3.00(s, 6H), 2.40(d, $J$ = 9.4 Hz, 1H); LCMS (m/z): 481.2[M+1]$^+$.

Example 70: Synthesis of (4R,4aR,5R,6S,7S,9S,10S,10aR,11S)-6-((dimethylamino)methyl)-2-iminooctahydro-5,9-epoxy-7,10a-methanooxocino[4,5-d]pyrimidine-4,6,9,10,11(10H)-pentaol **(Compound 93)**

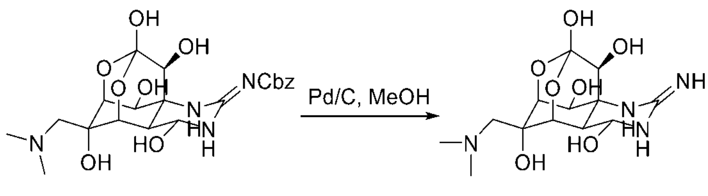


92

93

According to the synthesis procedures of Example 2, **Compound 93** was obtained as white solid (12 mg, yield: 68%).

$^1$H NMR (400 MHz, $D_2O$) $\delta$ 5.63(d, $J$ = 9.2 Hz, 1H), 4.45(s, 2H), 4.30(s, 1H), 4.12(s, 1H), 4.02-3.90(m, 2H), 2.52(d, $J$ = 9.7 Hz, 1H), 2.23-2.14(m, 6H); LCMS (m/z): 347.2[M+1]$^+$.

Example 71: Synthesis of benzyl ((4R,4aR,5R,6S,7S,9S,10S,10aR,11S,E)-6-((cyclopropylamino)methyl)-4,6,9,10,11-pentahydroxyoctahydro-5,9-epoxy-7,10a-methanooxocino[4,5-d]pyrimidin-2(1H)-ylidene)carbamate **(Compound 94)**

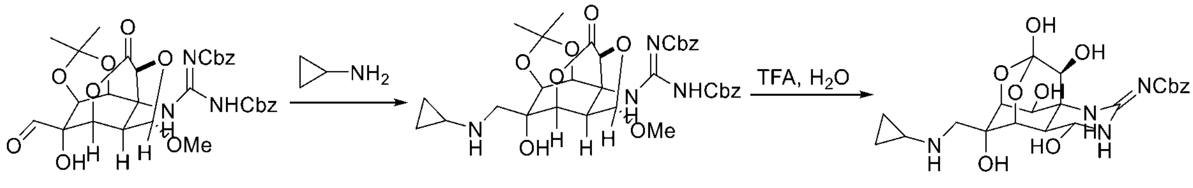


B2 94-1 94

Step 1: Synthesis of **Compound 94-1**

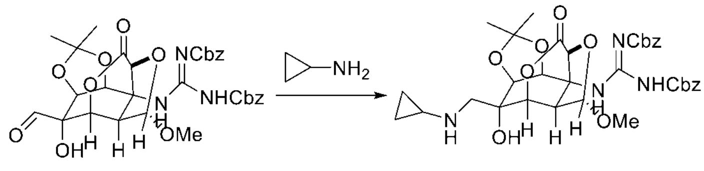

B2 94-1

According to the synthesis procedures of **Compound 63-1** in Example 49, after the reaction was completed, the reaction solution was cooled to room temperature, then added to water (30 mL) to precipitate a white solid, which was filtered. The filter cake was washed with water (10 mL×2), collected, and dried to give **Compound 94-1** as white solid (95 mg, yield: 85%). LCMS (m/z): 681.3$[M+1]^+$.

Step 2: Synthesis of **Compound 94**

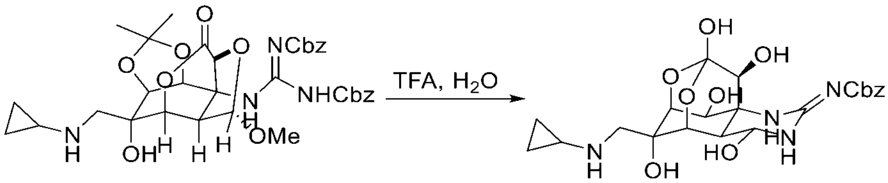


94-1 94

According to the synthesis procedures of Example 1, **Compound 94** was obtained as white solid (7 mg, yield: 10%).

$^1H$ NMR (400 MHz, MeOD) $\delta$ 7.40-7.23(m, 5H), 5.52(d, $J$ = 9.4 Hz, 1H), 5.05(s, 2H), 4.25-3.75(m, 4H), 3.23(s, 2H), 2.33-2.16(m, 2H), 0.53-0.44(m, 2H), 0.42-0.34(m, 2H). LCMS (m/z): 493.2$[M+1]^+$.

Example 72: Synthesis of benzyl ((4R,4aR,5R,6S,7S,9S,10S,10aR,11S,E)-4,6,9,10,11-pentahydroxy-6-(((3-hydroxycyclobutyl)amino)methyl)octahydro-5,9-epoxy-7,10a-methanooxocino[4,5-d]pyrimidin-2(1H)-ylidene)carbamate **(Compound 95)**

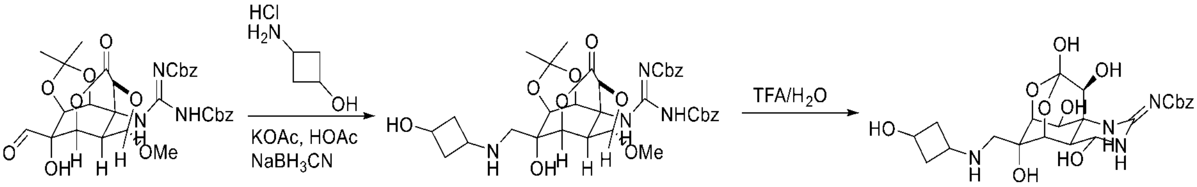


B2 95-1 95

Step 1: Synthesis of **Compound 95-1**

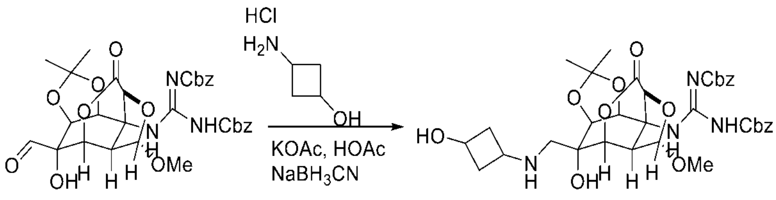


B2 95-1

According to the synthesis procedures of **Compound 63-1** in Example 49, after the reaction was completed, the reaction solution was cooled to room temperature, then added to water (30 mL), extracted with ethyl acetate (15 mL×3), and washed with saturated sodium chloride (20 mL). The organic phase was dried over anhydrous sodium sulfate, filtered, and concentrated to give a residue. The residue was purified by flash column chromatography (dichloromethane/methanol = 0%-8%) to give **Compound 95-1** as white solid(125 mg, yield: 62.5%). LCMS (m/z): 711.3$[M+1]^+$.

Step 2: Synthesis of **Compound 95**

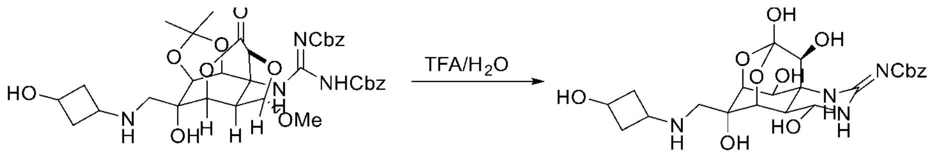


95-1 95

According to the synthesis procedures of Example 1, Compound 95 was obtained as white solid (15 mg, yield: 16.3%).

$^1H$ NMR (400 MHz, MeOD) δ 7.47-7.40(m, 5H), 5.73(d, $J$ = 9.4 Hz, 1H), 5.32(s, 2H), 4.58-4.45(m 1H), 4.29(s, 2H), 4.17-3.94(m, 3H), 3.56(s, 2H), 2.82-2.73(m, 1H), 2.61-2.55(m, 1H), 2.45-2.36(m, 2H), 2.21(m, 1H). LCMS (m/z): 523.2 $[M+1]^+$.

Example 73: Synthesis of (4R,4aR,5R,6S,7S,9S,10S,10aR,11S)-6-(((3-hydroxycyclobutyl)amino)methyl)-2-iminooctahydro-5,9-epoxy-7,10a-methanooxocino[4,5-d]pyrimidine-4,6,9,10,11(10H)-pentaol **(Compound 96)**

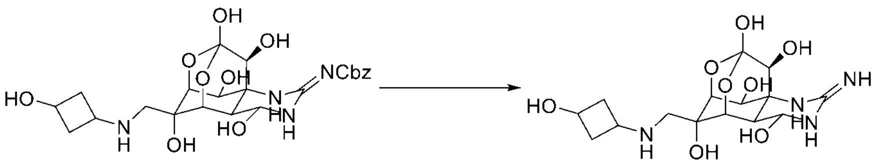

95 96

According to the synthesis procedures of Example 2, **Compound 96** was obtained as white solid (5.2 mg, yield: 67.2%).

$^1H$ NMR (400 MHz, $D_2O$) $\delta$ 5.44(d, $J$ = 9.4 Hz, 1H), 4.32-4.21(m, 2H), 4.17-3.91(m, 3H), 3.57-3.35(m, 3H), 2.80-2.69(m, 1H), 2.60-2.53(m, 1H), 2.42-2.29(m, 2H), 2.11(dd, $J$ = 20.1, 9.1 Hz, 1H). LCMS (m/z): 389.2$[M+1]^+$.

Example 74: Synthesis of (((4R,4aR,5R,6S,7S,9S,10S,10aR,11S,E)-2-(((benzyloxy)carbonyl)imino)-4,6,9,10,11-pentahydroxydecahydro-5,9-epoxy-7,10a-methanooxocino[4,5-d]pyrimidin-6-yl)methyl)alanine **(Compound 97)**

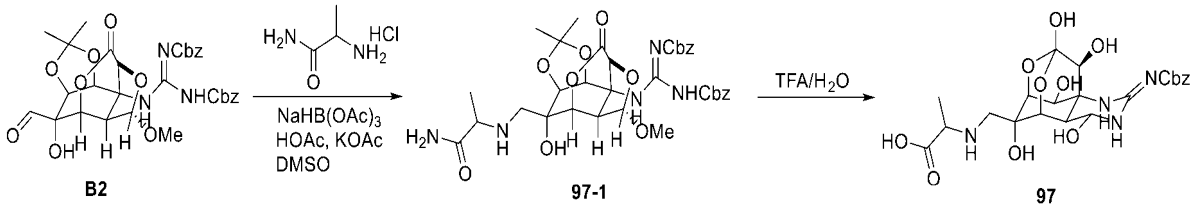


B2 97-1 97

Step 1: Synthesis of **Compound 97-1**

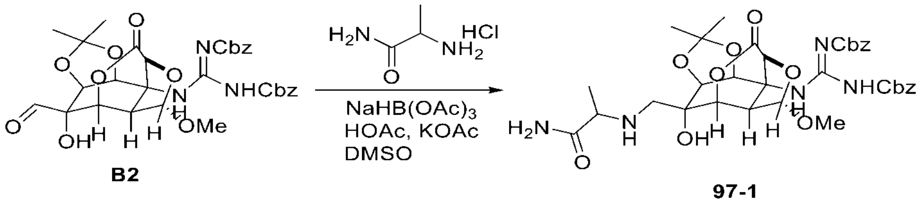


B2 97-1

**B2** (200 mg, 0.31 mmol), 2-aminopropionamide hydrochloride (77.9 mg, 0.63 mmol), KOAc (176.5 mg, 1.80 mmol) and anhydrous DMSO (5.0 mL) were added to a reaction flask, and AcOH (221.2 mg, 3.69 mmol) was added dropwise to the mixed solution. The reaction solution was heated at 60 °C for 1 h. Sodium cyanoborohydride (112 mg, 1.76 mmol) was added to the reaction solution in batches, and the reaction was continued for 1 h under 60 °C. The reaction was monitored by HPLC for completeness. The reaction mixture was cooled to room temperature, ice water (30 mL) was added dropwise to the reaction solution, which was extracted with ethyl acetate (15 mL×2). The organic phases were combined, washed with saturated sodium chloride solution (20 mL), dried over anhydrous sodium sulfate, and concentrated to give a residue. The residue was purified by flash column chromatography (methanol/dichloromethane = 0-8%) to give **Compound 97-1** as white solid (128 mg, yield: 57.5%). LCMS (m/z): 712.3$[M+1]^+$.

Step 2: Synthesis of **Compound 97**

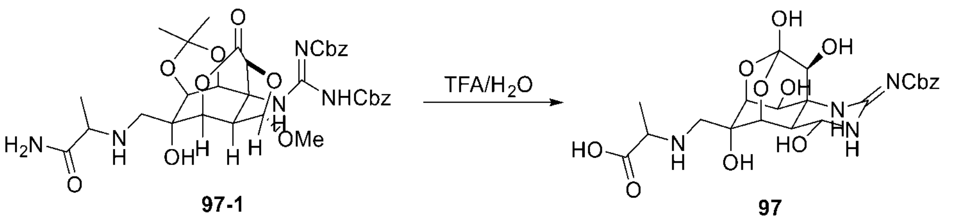


97-1 97

According to the synthesis procedures of Example 1, **Compound 97-1** (123 mg, 0.17 mmol) was added, and the crude product was purified by prep-HPLC (A: 0.1% AcOH, B: MeOH = 0-8%) to give **Compound 97** as white solid (10 mg, yield: 13.6%). LCMS (m/z): 525.2$[M+1]^+$.

Example 75: Synthesis of (((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)alanine (**Compound 98**)

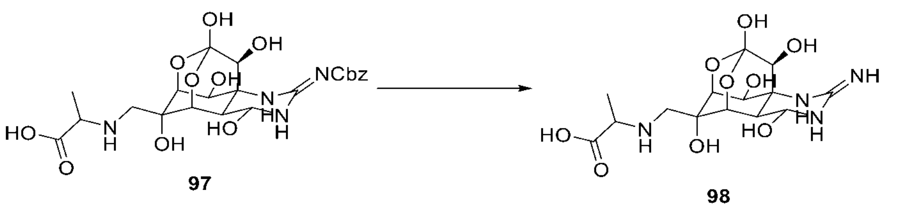

97 98

According to the synthesis procedures of Example 2, **Compound 98** was obtained as white solid (5.0 mg, yield: 67.1%). LCMS (m/z): 391.1[M+1]+.

Example 76: Synthesis of benzyl ((4R,4aR,5R,6S,7S,9S,10S,10aR,11S,E)-6-(azetidin-1-ylmethyl)-4,6,9,10,11-pentahydroxyoctahydro-5,9-epoxy-7,10a-methanooxocino[4,5-d]pyrimidin-2(1H)-ylidene)carbamate (**Compound 99**)

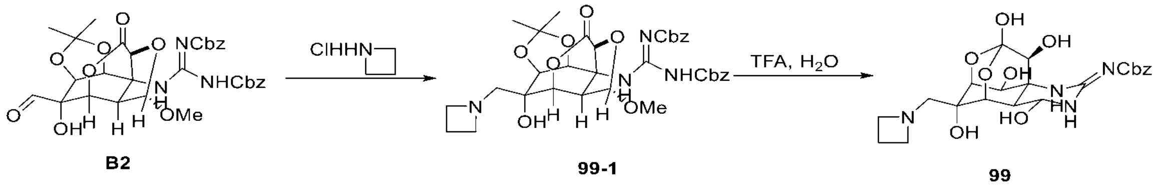


Step 1: Synthesis of **Compound 99-1**

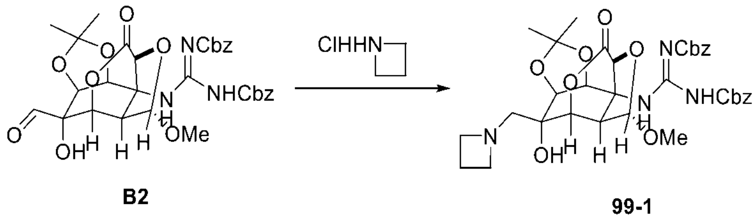


According to the synthesis procedures of **Compound 63-1** in Example 49, after the reaction was completed, the reaction solution was cooled to room temperature, then added to water (30 mL) to precipitate a white solid, which was filtered. The filter cake was washed with water (10 mL×2), collected, and dried to give **Compound 99-1** as white solid (200 mg, yield: 94%). LCMS (m/z): 681.3[M+1]+.

Step 2: Synthesis of **Compound 99**

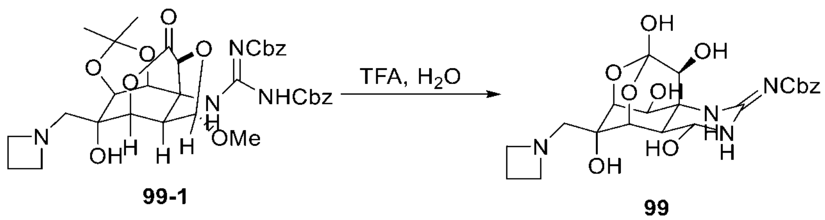


According to the synthesis procedures of Example 1, **Compound 99** was obtained as white solid (33 mg, yield: 23%).

$^1$H NMR (400 MHz, MeOD) $\delta$ 7.53-7.28(m, 5H), 5.71(d, *J* = 8.9 Hz, 1H), 5.32(s, 2H), 4.44-4.23(m, 5H), 4.20-3.57(m, 6H), 3.31-3.25(m, 1H), 2.40(d, *J* = 9.4 Hz, 1H). LCMS (m/z): 493.2[M+1]+.

Example 77: Synthesis of (4R,4aR,5R,6S,7S,9S,10S,10aR,11S)-6-(azetidin-1-ylmethyl)-2-iminooctahydro-5,9-epoxy-7,10a-methanooxocino[4,5-d]pyrimidine-4,6,9,10,11(10H)-pentaol (**Compound 100**)

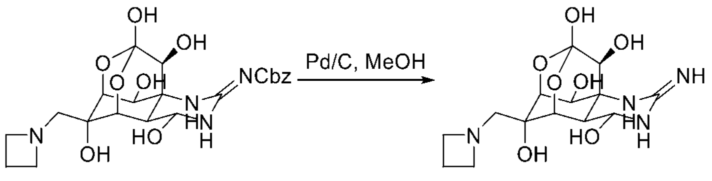


99 100

According to the synthesis procedures of Example 2, **Compound 100** was obtained as white solid (17 mg, yield: 78%).

$^1H$ NMR (400 MHz, $D_2O$) $\delta$ 5.46(d, $J$ = 9.4 Hz, 1H), 4.44-4.23(m, 4H), 4.04-3.76(m, 3H), 3.74-3.42(m, 2H), 2.72-2.57(m, 1H), 2.50-2.35(m, 1H), 2.13-1.97(m, 2H). LCMS (m/z): 359.2[M+1]$^+$.

Example 78: Synthesis of benzyl ((4R,4aR,5R,6S,7S,9S,10S,10aR,11S,E)-6-((3-fluoroazetidin-1-yl) methyl)-4,6,9,10,11-pentahydroxyoctahydro-5,9-epoxy-7,10a-methanooxocino[4,5-d]pyrimidin-2(1H)-ylidene)carbamate (**Compound 101**) and benzyl ((4S,SaS,6S,8R,9S,10S,11S,11aR,12R,E)-9-((3-fluoroazetidin-1-yl) methyl)-6,9,11-trihydroxyoctahydro-4,8,11a-(epimethanetriyl)-6,10-epoxyoxocino[4,3-f][1,3,5]oxadiazepin-2(1H)-ylidene)carbamate (**Compound 102**)

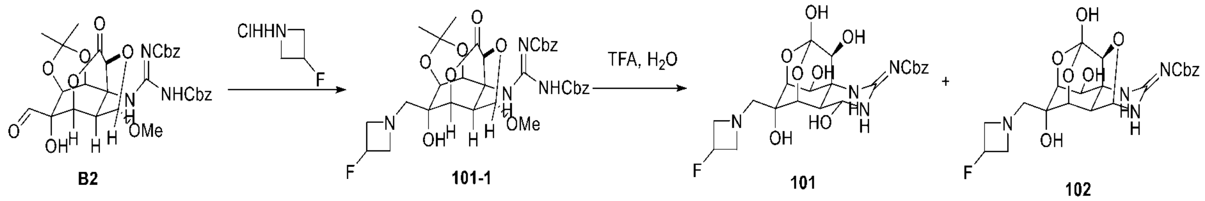


B2 101-1 101 102

Step 1: Synthesis of **Compound 101-1**

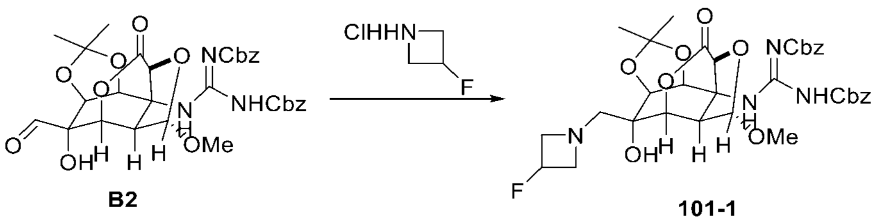


B2 101-1

According to the synthesis procedures of **Compound 97-1** in Example 74, B2 (200 mg, 0.31 mmol) was added to give **Compound 101-1** (210 mg, yield: 96.3%). LCMS (m/z): 699.3[M+1]$^+$.

Step 2: Synthesis of **Compound 101** and **Compound 102**

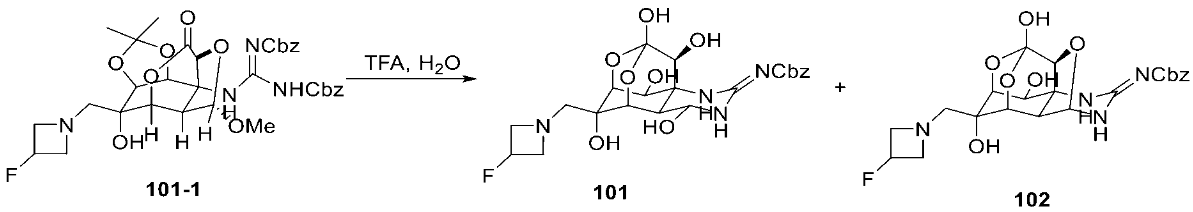


101-1 101 102

According to the synthesis procedures of Example 1, Compound 101-1 (210 mg, 0.30 mmol) was added to to give Compound 101 (25 mg, 16.3%) and Compound 102 (5 mg, 3.4%).

**Compound 101:**

$^1$H NMR (400 MHz, MeOD) δ 7.46-7.26(m, 5H), 5.62(d, *J* = 9.4 Hz, 1H), 5.29-5.15(m, 2.5H), 5.12-5.05(m, 0.5H), 4.30-3.82(m, 6H), 3.59-3.46(m, 2H), 3.29-3.07(m, 2H), 2.31(d, *J* = 9.1 Hz, 1H); LCMS (m/z): 511.2[M+1]$^+$.

**Compound 102:**

$^1$H NMR (400 MHz, MeOD) δ 7.50-7.19(m, 5H), 5.27(s, 1H), 5.22-5.16(m, 0.5H), 5.09-5.01(m, 2.5H), 4.45(d, *J* = 2.0 Hz, 1H), 4.41(s, 1H), 4.10(d, *J* = 1.3 Hz, 1H), 3.95(s, 1H), 3.82-3.69(m, 2H), 3.39(dd, *J* = 9.4, 4.6 Hz, 1H), 3.36-3.32(m, 1H), 3.11(d, *J*= 13.3 Hz, 1H), 3.00(d, *J* = 13.3 Hz, 1H), 2.67(d, *J* = 3.0 Hz, 1H); LCMS (m/z): 493.2[M+1]$^+$.

Example 79: Synthesis of (4R,4aR,5R,6S,7S,9S,10S,10aR,11S)-6-((3-fluoroazetidin-1-yl)methyl)-2-iminooctahydro-5,9-epoxy-7,10a-methanooxocino[4,5-d]pyrimidine-4,6,9,10,11(10H)-pentaol (**Compound 103**)

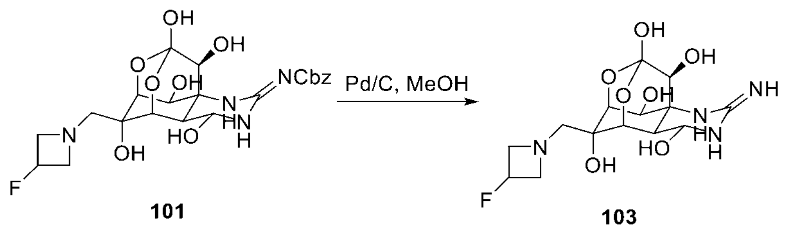


101 103

**According to the synthesis procedures of Example 2, Compound 101 (15 mg, 0.03 mmol) was added to give Compound 103 (10 mg, yield: 90.4%).**

$^1$H NMR (400 MHz, $D_2O$) δ 5.63-5.28(m, 2H), 4.67-4.42(m, 4H), 4.33-4.13(m, 2H), 4.09-3.68(m, 4H), 2.30(d, *J* = 9.5 Hz, 1H); LCMS (m/z): 377.1[M+1]$^+$.

Example 80: Synthesis of benzyl ((4R,4aR,5R,6S,7S,9S,10S,10aR,11S,E)-4,6,9,10,11-pentahydroxy-6-((3-hydroxypyrrolidin-1-yl)methyl)octahydro-5,9-epoxy-7,10a-methanooxocino[4,5-d]pyrimidin-2(1H)-ylidene)carbamate (**Compound 104**) and benzyl ((4S,5aS,6S,8R,9S,10S,11S,11aR,12R,E)-6,9,11-trihydroxy-9-((3-hydroxypyrrolidin-1-yl)methyl)octahydro-4,8,11a-(epimethanetriyl)-6,10-epoxyoxocino[**4**,3-f][1,3,5]oxadiazepin-2(1H)-ylidene)carbamate (**Compound 105**)

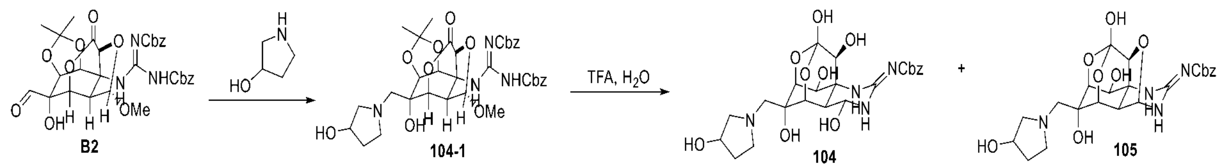


B2 104-1 104 + 105

Step 1: Synthesis of **Compound 104-1**

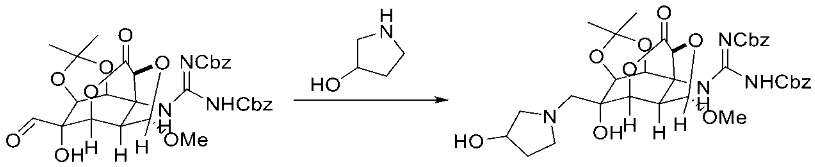

B2 104-1

According to the synthesis procedures of **Compound 63-1** in Example 49, after the reaction was completed, the reaction solution was cooled to room temperature, then added to water (30 mL) to precipitate a white solid, which was

filtered. The filter cake was washed with water (10 mL×2), collected, and dried to give **Compound 104-1** white solid (145 mg, yield: 87%). LCMS (m/z): 711.3[M+1]+.

Step 2: Synthesis of **Compound 104** and **Compound 105**

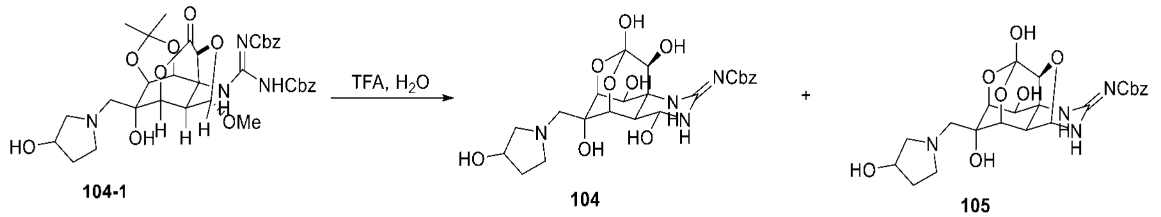


104-1 104 105

According to the synthesis procedures of Example 1, **Compound 104** (33 mg, yield: 31%) and **Compound 105** (3 mg, yield: 3%) were obtained as white solid.

**Compound 104:**

$^1$H NMR (400 MHz, MeOD) δ 7.57-7.23(m, 5H), 5.69(d, *J* = 7.3 Hz, 1H), 5.29(s, 2H), 4.59(s, 1H), 4.29(s, 1H), 4.23(s, 1H), 3.97(d, *J* = 42.4 Hz, 2H), 3.87-3.76(m, 1H), 3.63(t, *J* = 5.0 Hz, 3H), 3.56-3.39(m, 2H), 2.41(d, *J* = 9.2 Hz, 1H), 2.20(d, *J* = 26.9 Hz, 1H), 2.11(s, 1H); LCMS (m/z): 523.2[M+1]+.

**Compound 105:**

LCMS (m/z): 505.2[M+1]+.

Example 81: Synthesis of (4R,4aR,5R,6S,7S,9S,10S,10aR,11S)-6-((3-hydroxypyrrolidin-1-yl)methyl)-2-iminooctahydro-5,9-epoxy-7,10a-methanooxocino[4,5-d]pyrimidine-4,6,9,10,11(10H)-pentaol (**Compound 106**)

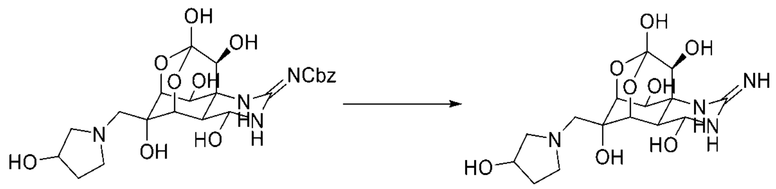

104 106

According to the synthesis procedures of Example 2, **Compound 106** was obtained as white solid (13 mg, yield: 87%).

$^1$H NMR (400 MHz, $D_2O$) δ 5.46(d, *J* = 9.4 Hz, 1H), 4.66(s, 2H), 4.29(d, *J* = 6.6 Hz, 2H), 4.13(s, 1H), 4.05-3.91(m, 2H), 3.89-3.75(m, 2H), 3.63-3.35(m, 2H), 2.35(d, *J* = 9.4 Hz, 2H), 2.16-2.03(m, 1H); LCMS (m/z): 389.2[M+1]+.

Example 82: Synthesis of benzyl ((4R,4aR,5R,6S,7S,9S,10S,10aR,11S,E)-6-((3-fluoropyrrolidin-1-yl)methyl)-4,6,9,10,11-pentahydroxyoctahydro-5,9-epoxy-7,10a-methanooxocino[4,5-d]pyrimidin-2(1H)-ylidene)carbamate (**Compound 107**) and benzyl ((4S,5aS,6S,8R,9S,10S,11S,11aR,12R,E)-9-((3-fluoropyrrolidin-1-yl)methyl)-6,9,11-trihydroxyoctahydro-4,8,11a-(epimethanetriyl)-6,10-epoxyoxocino[4,3-f][1,3,5]oxadiazepin-2(1H)-ylidene)carbamate (**Compound 108**)

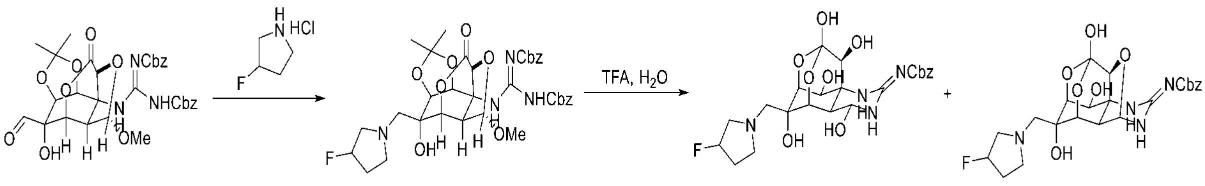


B2 107-1 107 108

Step 1: Synthesis of **Compound 107-1**

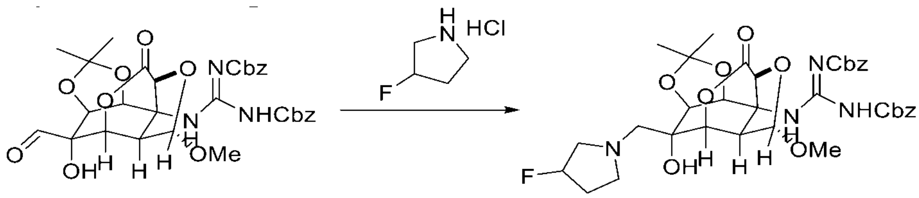


B2 107-1

**Compound B2** (200 mg, 0.31 mmol) was dissolved in dimethyl sulfoxide (3 mL), to which 3-fluoropyrrolidine hydrochloride (59 mg, 0.47 mmol), glacial acetic acid (221.79 mg, 3.69 mmol) and potassium acetate (176.6 mg, 1.8 mmol) were added. The reaction mixture was heated to 60°C under nitrogen protection and stirred for 1 h, and then sodium cyanoborohydride (222.1 mg, 3.537 mmol) was added in batches and the reaction was carried out for 30 min. HPLC was used to monitor complete reaction of the raw materials. The reaction system was cooled to room temperature, then to which ice water (50 mL) was added, extracted with ethyl acetate (25 mL * 3). The organic phases were combined and washed with saturated sodium chloride solution (30 mL), dried over anhydrous sodium sulfate, filtered, and concentrated to give a residue of about 230 mg. The residue was subjected to flash column chromatography (MeOH/DCM = 0-5%) to give **Compound 107-1** (200 mg, yield: 89.9%).

Step 2: Synthesis of **Compound 107** and **Compound 108**

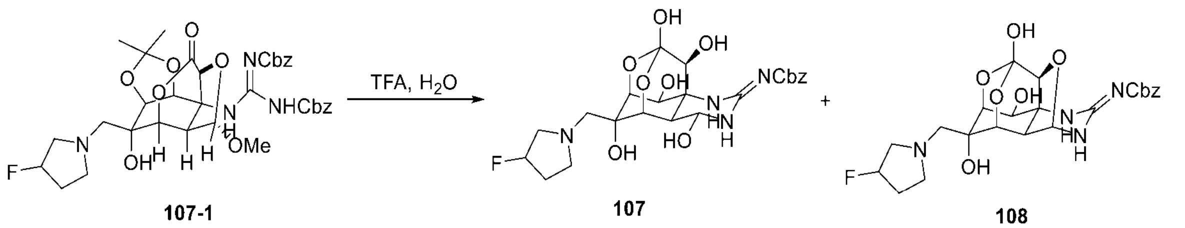


107-1 107 108

**Compound 107-1** (200 mg, 0.28 mmol) was added to a reaction flask, to which water (10 mL) and TFA (10 mL) were added, and the mixture was heated to 60°C under nitrogen protection and stirred for 18 h. The reaction solution was concentrated to dryness, and the residue was subjected to flash column chromatography (MeOH/DCM = 0-5%) to give **Compound 107** (30 mg, yield: 20.4%) and **Compound 108** (6 mg, yield: 4%), total yield: 24.4%.

**Compound 107:**

$^1$H NMR (400 MHz, MeOD) $\delta$ 7.51-7.21(m, 5H), 5.64(d, *J* = 9.3 Hz, 1H), 5.30-5.08(m, 3H), 4.32-3.82(m, 4H), 3.30-2.98(m, 5H), 2.91-2.70(m, 1H), 2.35(d, *J* = 9.4 Hz, 1H), 2.28-2.01(m, 2H); LCMS (m/z): 525.2$[M+1]^+$.

**Compound 108:**

LCMS (m/z): 507.2$[M+1]^+$.

Example 83: Synthesis of (4R,4aR,5R,6S,7S,9S,10S,10aR,11S)-6-((3-fluoropyrrolidin-1-yl)methyl)-2-iminooctahydro-5,9-epoxy-7,10a-methanooxocino[4,5-d]pyrimidine-4,6,9,10,11(10H)-pentaol (**Compound 109**)

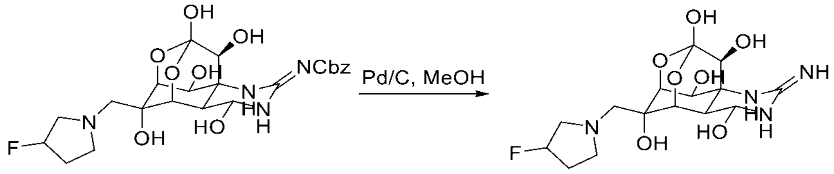


107 109

**Compound 107** (20 mg, 0.04 mmol) was dissolved in methanol (5 mL), to which 10% Pd/C (10 mg) was added. The reaction mixture was purged with hydrogen three times. About 3 h after introduction of hydrogen to reaction solution, HPLC was used to monitor complete reaction of the raw materials. The reaction solution was filtered, and the filter cake was rinsed with 0.05 M AcOH aqueous solution. The filtrate was concentrated to give a crude compound, which was slurried with methyl tert-butyl ether (10 mL), allowed to stand, the solvent was taken out, and the residue was concentrated and dried to give **Compound 109** (10 mg, yield: 67.1%).

**Compound 109:**

$^1H$ NMR (400 MHz, $D_2O$) $\delta$ 5.60-5.38(m, 2H), 4.30(s, 2H), 4.13(s, 1H), 4.08-3.57(m, 7H), 2.51-2.29(m, 3H); LCMS (m/z): 391.4$[M+1]^+$.

Example 84: Synthesis of benzyl ((4R,4aR,5R,6S,7S,9S,10S,10aR,11S,E)-4,6,9,10,11-pentahydroxy-6-(morpholinomethyl)octahydro-5,9-epoxy-7,10a-methanooxocino[4,5-d]pyrimidin-2(1H)-ylidene)carbamate (**Compound 110**) and benzyl ((4S,5aS,6S,8R,9S,10S,11S,11aR,12R,E)-6,9,11-trihydroxy-9-(morpholinomethyl )octahydro-4,8,11a-(epimethanetriyl)-6,10-epoxyoxocino[4,3-f][1,3,5]oxadiazepin-2(1H)-ylidene)carbamate (**Compound 111**)

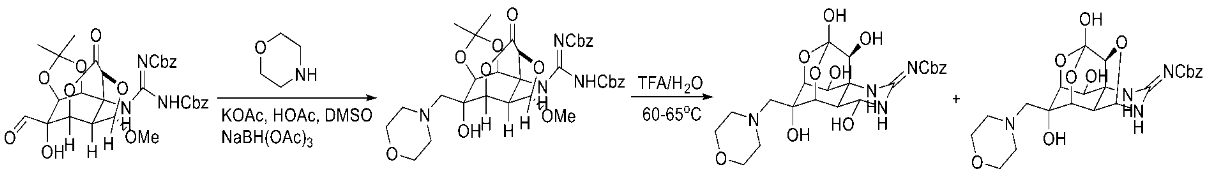


B2 110-1 110 111

Step 1: Synthesis of **Compound 110-1**

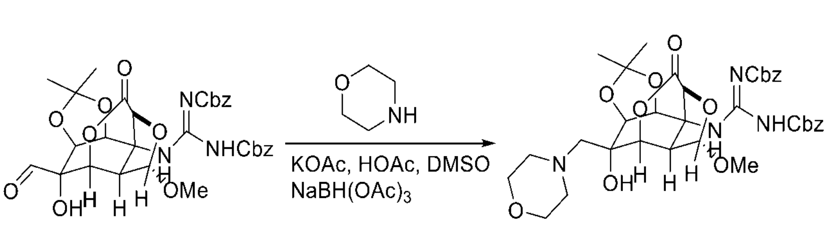


B2 110-1

**Compound B2** (200 mg, 0.31 mmol), morpholine (54.5 mg, 0.63 mmol), potassium acetate (176.6 mg, 1.8 mmol) and dimethyl sulfoxide (8 mL) were added to a reaction flask, to which acetic acid (221.8 mg, 3.69 mmol) was added. The reaction mixture was heated to 60 °C for 2 h, then sodium triacetylborohydride (749.6 mg, 3.54 mmol) was added. The reaction was continued for 1.5 h, and then stirred at room temperature overnight. The reaction solution was diluted with water (40 mL), extracted with ethyl acetate (330 mL). The organic phases were combined, dried over anhydrous sodium

sulfate, and concentrated to give a crude product. The crude product was purified by prep-TLC (developer DCM/MeOH = 30: 1) to give **Compound 110-1** (84 mg, yield 32.9%), LCMS (m/z): 711.3[M+1]$^+$.

Step 2: Synthesis of **Compound 110** and **Compound 111**

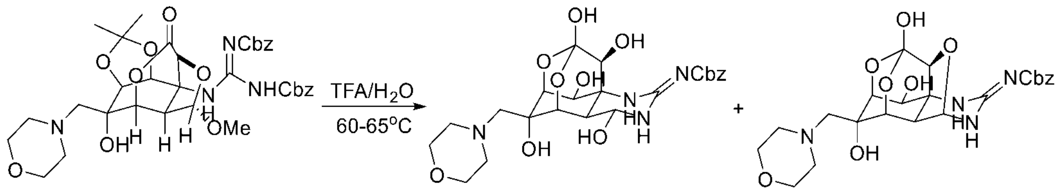


**110-1** **110** **111**

**Compound 110-1** (84 mg, 0.12 mmol) was dissolved in trifluoroacetic acid (2.5 mL) and water (2.5 mL), heated to 65 °C, and the reaction was carried out overnight. HPLC was used to monitor complete reaction of the raw materials. The reaction solution was concentrated and dried, and then purified by reverse phase flash column chromatography (eluent: MeOH/0.1% HOAc aqueous solution = 0-2%) to give **Compound 110** (22 mg, yield: 35.6%) and **Compound 111** (2.5 mg, yield: 4.2%), total yield: 39.8%.

**Compound 110:**

$^1$H NMR (400 MHz, MeOD) $\delta$ 7.46-7.15(m, 5H), 5.56(d, $J$ = 7.4 Hz, 1H), 5.11(s, 2H), 4.33-3.81(m, 4H), 3.69(t, $J$ = 4.5 Hz, 4H), 3.05-2.85(m, 2H), 2.74-2.58(m, 4H), 2.27(d, J= 9.5 Hz, 1H); LCMS (m/z): 523.2[M+1]$^+$.

**Compound 111:**

LCMS (m/z): 505.2[M+1]$^+$.

Example 85: Synthesis of (4R,4aR,5R,6S,7S,9S,10S,10aR,11S)-2-imino-6-(morpholinomethyl)octahydro-5,9-epoxy-7,10a-methanooxocino[4,5-d]pyrimidine-4,6,9,10,11(10H)-pentaol (**Compound 112**)

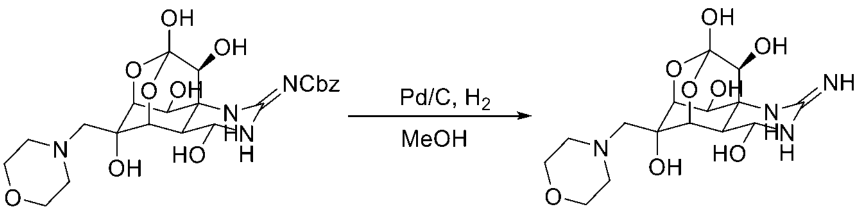


**110** **112**

**Compound 110** (9 mg, 0.02 mmol), MeOH (15 mL) and 10% Pd/C (3 mg) were added to a reaction flask. The reaction mixture was purged with hydrogen three times, and then hydrogenated by a hydrogen balloon under pressure for 3 h. HPLC was used to monitor complete reaction of the raw materials. Then 0.2% acetic acid aqueous solution (5 mL) was added to the reaction solution with stirring for 30 min. The reaction solution was filtered, and the filter cake was slurried with 0.2% acetic acid aqueous solution twice, and filtered. The filtrate was combined, concentrated and dried, then methanol (2 drops) was added. The solid was dissolved, and then methyl tert-butyl ether (10 mL) was added to precipitate a white solid. The mixture was stirred for 15 min, allowed to stand, and the supernatant was removed. The residue was concentrated and dried, and lyophilized to give acetate of **Compound 112** (7.5 mg, yield: 96%).

$^1$H NMR (400 MHz, $D_2O$) $\delta$ 5.44(d, $J$ = 9.4 Hz, 1H), 4.40-4.12(m, 3H), 4.06-3.89(m, 5H), 3.87-3.71(m, 2H), 3.55-3.41(m, 4H), 2.34(d, $J$ = 9.4 Hz, 1H), 2.02(s, 3H); LCMS (m/z): 389.2[M+1]$^+$.

Example 86: Synthesis of (((4R,4aR,5R,6S,7S,9S,10S,10aR,11S,E)-2-(((benzyloxy)carbonyl)imino)-4,6,9,10,11-pentahydroxydecahydro-5,9-epoxy-7,10a-methanooxocino[4,5-d]pyrimidin-6-yl)methyl)glycine (**Compound 113**)

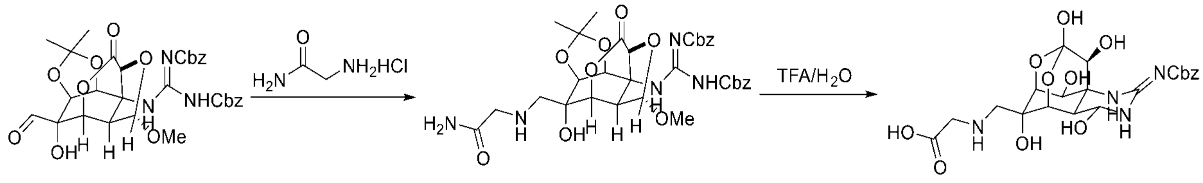


B2 113-1 113

Step 1: Synthesis of **Compound 113-1**

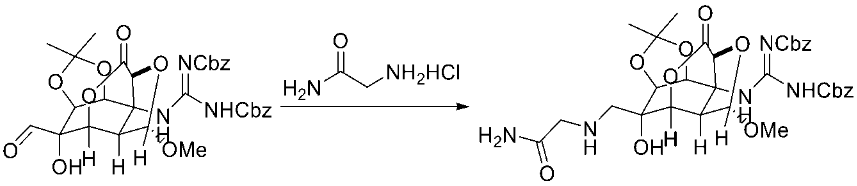

B2 113-1

**Compound B2** (205 mg, 0.32 mmol), glycine hydrochloride (36 mg, 0.48 mmol), AcOH (227 mg, 3.78 mmol) and KOAc (180 mg, 1.84 mmol) were dissolved in DMSO (2.5 mL) and stirred at 60°C for 2 h. Sodium cyanoborohydride (227 mg, 3.62 mmol) was added to the reaction solution in three batches. After the addition was completed, the reaction was continued with stirring at 60°C for 2 h. After the reaction was completed, water was added for quenching, then the reaction mixture was extracted with ethyl acetate (10 mL ×3). The organic phases were combined, washed once with saturated brine. The organic phases were separated, dried over anhydrous sodium sulfate, and filtered. The filtrate was concentrated and dried in vacuo, and purified by normal phase column chromatography (methanol/dichloromethane = 10%) to give **Compound 113-1** (145 mg, yield: 64.8%). LCMS (m/z): 698.3$[M+1]^+$.

Step 2: Synthesis of **Compound 113**

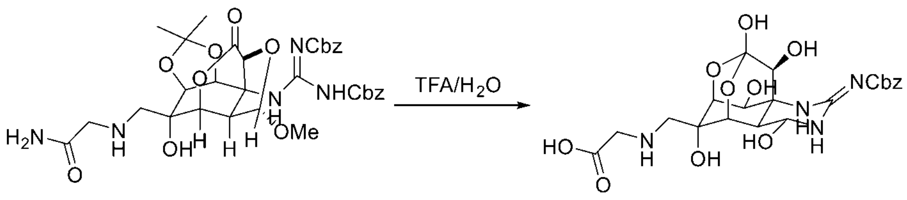


113-1 113

**Compound 113-1** (145 mg, 0.20 mmol) was added to a solvent of water: trifluoroacetic acid = 1:1, and the reaction was carried out with stirring at 65°C for 17 h. After the reaction was completed, the reaction mixture was directly concentrated to give a crude product, which was then purified by reverse phase column chromatography (methanol/0.1% acetic acid aqueous solution = 0-6.5%) to give **Compound 113** (18 mg, yield: 17.0%).

$^1$HNMR (400 MHz, MeOD) $\delta$ 7.38-7.26(m, 5H), 5.54(s, 1H), 5.07(s, 2H), 4.42-3.77(m, 5H), 3.67-3.43(m, 3H), 2.24(s, 1H); LCMS (m/z): 511.2$[M+1]^+$.

Example 87: Synthesis of (((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)glycine (**Compound 114**)

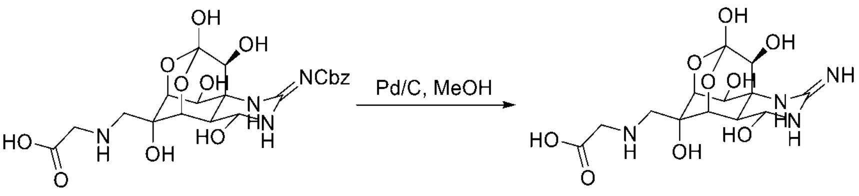


113 114

**Compound 113** (10 mg, 0.02 mmol) and 10% Pd/C (5 mg) were added to MeOH (2 mL). The reaction mixture was purged with hydrogen, and stirred at 25°C for 4 h. After the reaction was completed, 0.5% acetic acid aqueous solution was added and the reaction mixture was stirred for 0.5 h, and then filtered. The filtrate was concentrated and dried to give **Compound 114** (9.03 mg, yield: 122%, purity: 74.00%). LCMS (m/z): 377.1[M+1]+.

Example 88: Synthesis of 1-(((4R,4aR,5R,6S,7S,9S,10S,10aR,11S,E)-2-(((benzyloxy)carbonyl)imino)-4,6,9,10,11-pentahydroxydecahydro-5,9-epoxy-7,10a-methanooxocino[4,5-d]pyrimidin-6-yl)methyl)pyrrolidine-3-carboxylic acid (**Compound 115**)

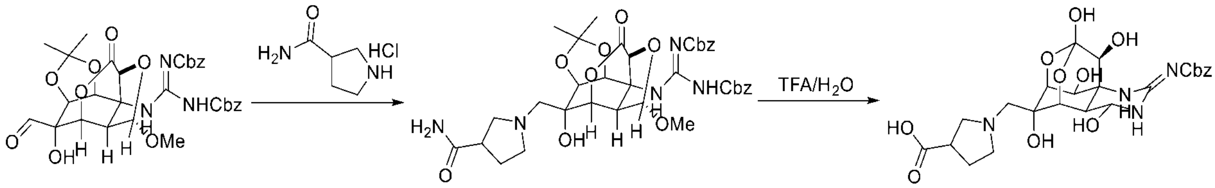


B2 115-1 115

Step 1: Synthesis of **Compound 115-1**

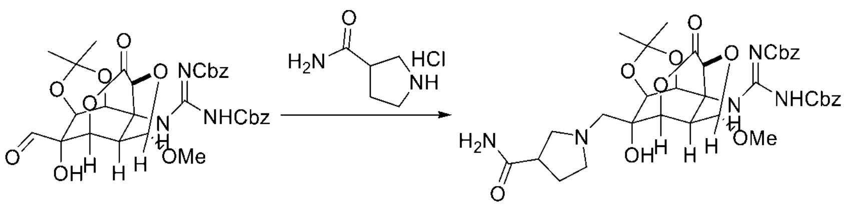

B2 115-1

According to the synthesis procedures of **Compound 63-1** in Example 49, **Compound B2** (190 mg, 0.30 mmol) was added, and after the reaction was completed, the reaction solution was cooled to room temperature, and then added to water (30 mL) to precipitate a white solid, which was filtered. The filter cake was washed with water (10 mL×2), collected and dried to give **Compound 115-1** as white solid (175 mg, yield: 80%). LCMS (m/z): 738.3[M+1]+.

Step 2: Synthesis of **Compound 115**

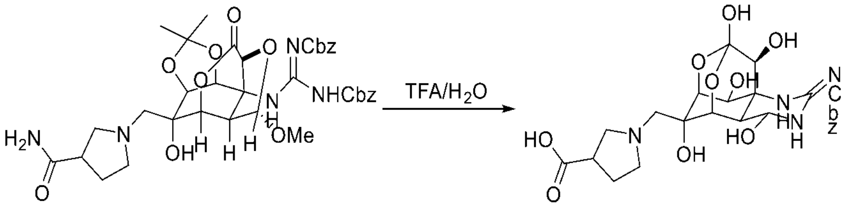


115-1 115

According to the synthesis procedures of Example 1, **Compound 115-1** (175 mg, 0.24 mmol) was added to give **Compound 115** as white solid (29 mg, yield: 22%).

$^1$H NMR (400 MHz, MeOD) $\delta$ 7.50-7.35(m, 5H), 5.69(d, *J* = 9.0 Hz, 1H), 5.27(s, 2H), 4.28(d, *J* = 16.8 Hz, 2H), 4.13(s, 2H), 3.93-3.64(m, 3H), 3.62-3.42(m, 3H), 3.24-3.13(m, 1H), 2.50-2.21(m, 3H); LCMS (m/z): 551.2[M+1]$^+$.

Example 89: Synthesis of 1-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)pyrrolidine-3-carboxylic acid (**Compound 116**)

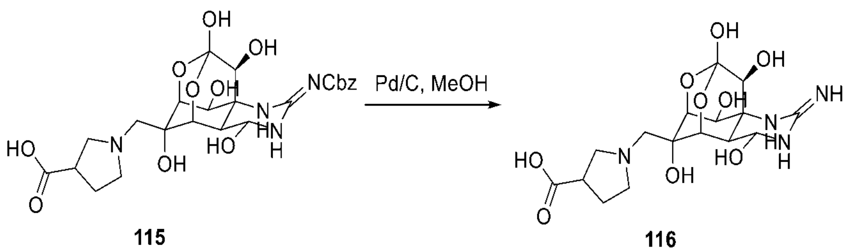


115 116

According to the synthesis procedures of Example 2, **Compound 115** (11 mg, 0.01 mmol) was added to give **Compound 116** as white solid (4.5 mg, yield: 54%). LCMS (m/z): 417.2[M+1]$^+$.

Example 90: Synthesis of benzyl ((4R,4aR,5R,6S,7S,9S,10S,10aR,11S,E)-6-((3-aminopyrrolidin-1-yl) methyl)-4,6,9,10,11-pentahydroxyoctahydro-5,9-epoxy-7,10a-methanooxocino[4,5-d]pyrimidin-2(1H)-ylidene)carbamate (**Compound 117**)

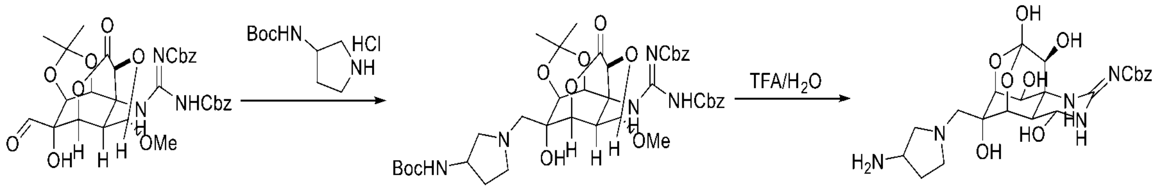


B2 117-1 117

Step 1: Synthesis of **Compound 117-1**

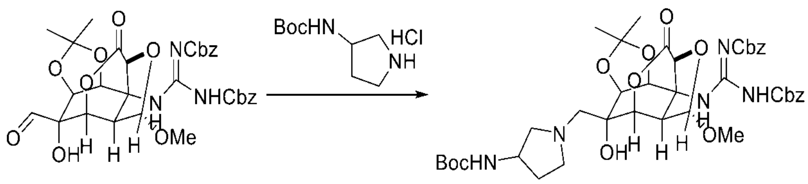

B2 117-1

**Compound B2** (200 mg, 0.31 mmol) was dissolved in dimethyl sulfoxide (3 mL), and Compound 3-(Boc-amino) pyrrolidine hydrochloride (87.4 mg, 0.47 mmol), glacial acetic acid (221.8 mg, 3.69 mmol) and potassium acetate (176.6 mg, 1.8 mmol) were added. The reaction mixture was heated to 60°C under nitrogen protection, stirred for 1 h, and then sodium cyanoborohydride (222.1 mg, 3.54 mmol) was added in batches for reaction for 30 min. HPLC was used to monitor complete reaction of the raw materials. The reaction solution was cooled to room temperature, then to which was added ice water (50 mL), extracted with ethyl acetate (25 mL×3). The organic phases were combined, washed with saturated sodium chloride (30 mL), dried over anhydrous sodium sulfate, filtered, and concentrated to give about 230 mg of residue. The residue was purified by flash column chromatography (MeOH/DCM = 0-5%) to give **Compound 117-1** (240 mg, yield: 94.9%). LCMS (m/z): 810.4[M+1]$^+$.

Step 2: Synthesis of **Compound 117**

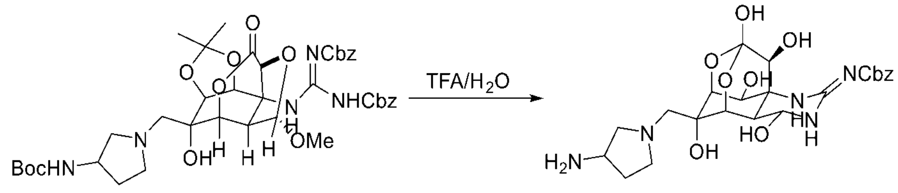


117-1 117

**Compound 117-1** (240 mg, 0.3 mmol) was added to a reaction flask, to which water (15 mL) and TFA (7.5 mL) were added. The reaction mixture was heated to 60°C under nitrogen protection for 18 h. The reaction solution was concentrated and dried, and the resulting residue was purified by flash column chromatography (MeOH/DCM = 0-4%) to give **Compound 117** (30 mg, yield: 19.5%). LCMS (m/z): 522.2$[M+1]^+$.

Example 91: Synthesis of (4R,4aR,5R,6S,7S,9S,10S,10aR,11S)-6-((3-aminopyrrolidin-1-yl)methyl)-2-iminooctahydro-5,9-epoxy-7,10a-methanooxocino[4,5-d]pyrimidine-4,6,9,10,11(10H)-pentaol (**Compound 118**)

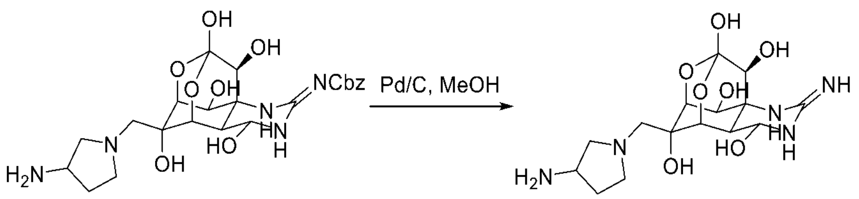


117 118

**Compound 117** (30 mg, 0.06 mmol) was dissolved in methanol (10 mL), to which 10% Pd/C (30 mg) was added, and the reaction solution was purged with hydrogen three times. About 3 h after introduction of hydrogen was introduced into the reaction solution, HPLC was used to monitor complete reaction of the raw materials. The reaction solution was filtered, and the filter cake was rinsed with 0.05 M acetic acid aqueous solution. The filtrate was concentrated to give a crude product. The crude product was slurried with methyl tert-butyl ether (10 mL), allowed to stand, the supernatant was removed, and the residue was concentrated and dried to give **Compound 118** (20 mg, yield: 89.8%).

$^1H$ NMR (400 MHz, $D_2O$) $\delta$ 5.42(d, $J$ = 9.3 Hz, 1H), 4.29-4.16(m, 3H), 4.10(s, 1H), 4.02-3.88(m, 3H), 3.82-3.62(m, 3H), 2.67-2.55(m, 1H), 2.31(d, $J$ = 9.1 Hz, 1H), 2.24-2.15(m, 1H), 1.99(d, $J$ = 9.8 Hz, 1H); LCMS (m/z): 388.2$[M+1]^+$.

Example 92: Synthesis of benzyl ((4R,4aR,5R,6S,7S,9S,10S,10aR,11S,E)-6-(((R)-3-acetamidopyrrolidin-1-yl) methyl)-4,6,9,10,11-pentahydroxyoctahydro-5,9-epoxy-7,10a-methanooxocino[4,5-d]pyrimidin-2(1H)-ylidene)carbamate (**Compound 119**)

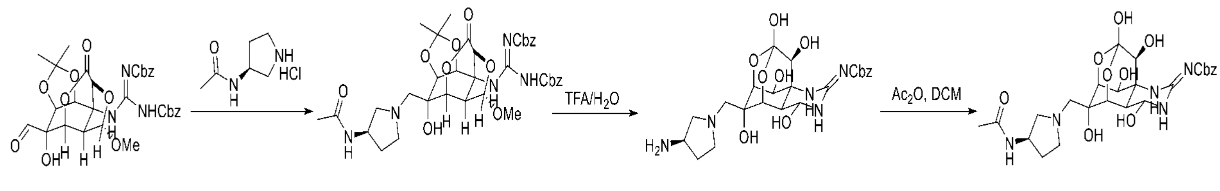


B2 119-1 119-2 119

Step 1: Synthesis of **Compound 119-1**

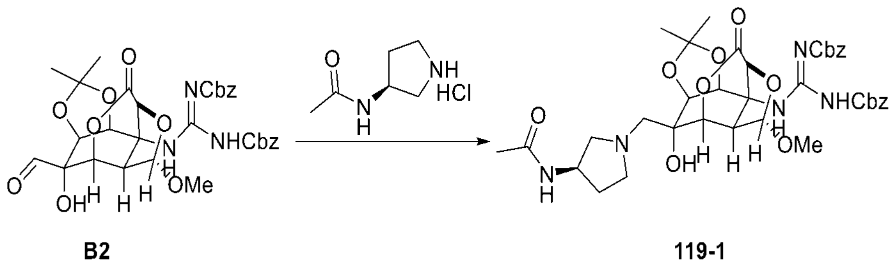


**Compound B2** (200 mg, 0.31 mmol), 3-acetamido-(s)-tetrahydropyrrole (77 mg, 0.47 mmol), AcOH (222 mg, 3.71 mmol) and KOAc (177 mg, 1.80 mmol) were dissolved in DMSO (4 mL) and stirred at 60°C for 2 h. Sodium cyanoborohydride (197 mg, 3.13 mmol) was added to the reaction solution in three batches. After the addition was completed, the reaction mixture was stirred at 60°C for 2 h. After the reaction was completed, water was added for quenching, then the reaction mixture was extracted with ethyl acetate (10 mL×3). The organic phases were combined, washed with saturated brine, dried over anhydrous sodium sulfate, and filtered. The filtrate was concentrated and dried, and the resulting residue was purified by flash column chromatography (methanol/dichloromethane = 0-3.5%) to give **Compound 119-1** (110 mg, yield: 46.8%). LCMS (m/z): 752.3[M+1]$^+$.

Step 2: Synthesis of **Compound 119-2**

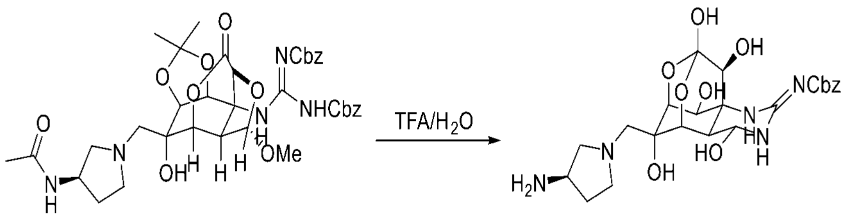


119-1

119-2

**Compound 119-1** (110 mg, 0.14 mmol) was added to a mixed solution of water: trifluoroacetic acid = 1:4 (20 mL), and the reaction mixture was stirred at 60°C for 17 h. After the reaction was completed, the reaction mixture was directly concentrated and dried to give a crude product. The crude product was purified by reverse phase column chromatography (methanol/0.1% acetic acid aqueous solution = 0-4.5%) to give **Compound 119-2** (15 mg, yield: 19.7%). LCMS (m/z): 522.2[M+1]$^+$.

Step 3: Synthesis of **Compound 119**

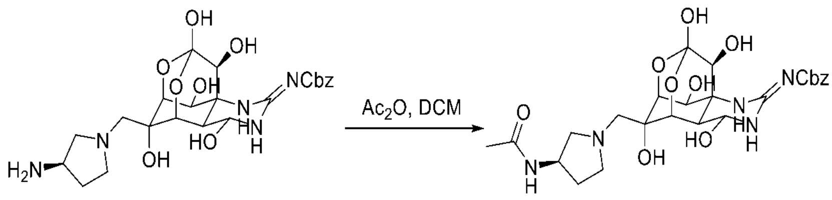


119-2

119

**Compound** 119-2 (15 mg, 0.03 mmol) was dissolved in tetrahydrofuran (2 mL), then acetic anhydride (3.5 mg, 0.03 mmol) was added, and the reaction mixture was stirred at 18 °C for 1 h. After the reaction was completed, the reaction solution was concentrated and dried, and the obtained residue was purified by Prep-HPLC to give **Compound 119** (2.0 mg, yield: 12.3%).

$^1$H NMR (400 MHz, MeOD) δ 7.44-7.17(m, 5H), 5.53(d, *J* = 9.4 Hz, 1H), 5.06(s, 2H), 4.33-4.23(m, 1H), 4.20-4.06(m, 2H), 3.94(s, 2H), 3.17-3.05(m, 2H), 3.04-2.96(m, 1H), 2.91(dd, *J* = 9.8, 6.7 Hz, 1H), 2.73-2.59(m, 2H), 2.30-2.15(m, 2H), 1.92(s, 3H), 1.71-1.58(m, 1H); LCMS (m/z): 564.2[M+1]$^+$.

Example 93: Synthesis of benzyl ((4R,4aR,5R,6S,75,9S,10S,10aR,11S,E)-4,6,9,10,11-pentahydroxy-6-(((2,2,2-trifluoroethyl)amino)methyl)octahydro-5,9-epoxy-7,10a-methanooxocino[4,5-d]pyrimidin-2(1H)-ylidene)carbamate (**Compound 120**)

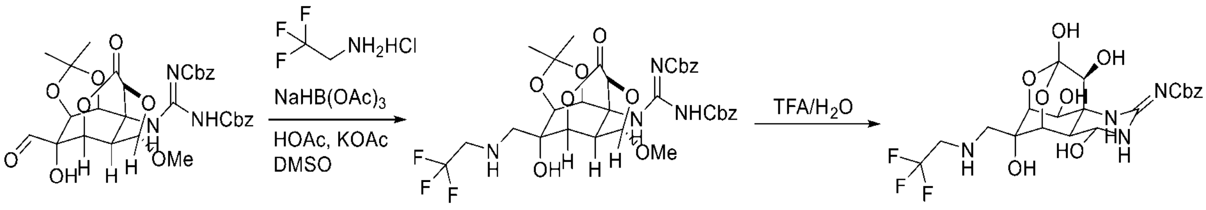


B2 120-1 120

Step 1: Synthesis of **Compound 120-1**

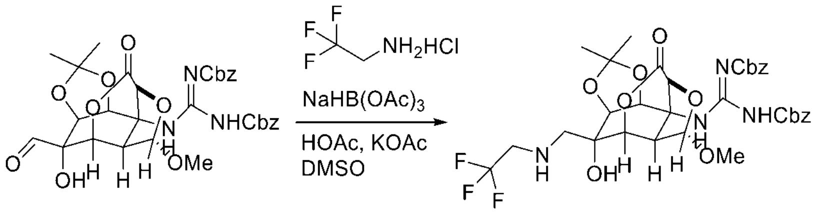


B2 120-1

**Compound B2** (150 mg, 0.23 mmol), trifluoroethylamine hydrochloride (48 mg, 0.35 mmol), AcOH (163 mg, 2.71 mmol) and KOAc (130 mg, 1.32 mmol) were dissolved in DMSO (3 mL) and stirred at 60°C for 2 h. Sodium cyanoborohydride (80 mg, 1.26 mmol) was added to the reaction solution in three batches. After the addition was completed, the reaction mixture was stirred at 60°C for 2 h. After the reaction was completed, the reaction was quenched by adding ice water, then a small amount of sodium chloride solid was added, and a white solid was precipitated from the solution. The reaction system was filtered, and the filter cake was rinsed twice with a small amount of ice water. The filter cake was concentrated and dried to give **Compound 120-1** as white solid (95 mg, yield: 56%). LCMS (m/z): 723.2[M+1]$^+$.

Step 2: Synthesis of **Compound 120**

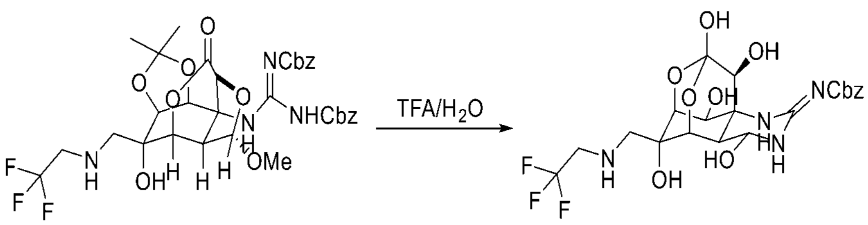


120-1 120

**Compound 120-1** (95 mg, 0.13 mmol) was added to a mixed solution of water: trifluoroacetic acid = 1:2(15 mL), and the reaction mixture was stirred at 60°C for 17 h. After the reaction was completed, the reaction solution was concentrated and dried, and the obtained residue was purified by reverse phase column chromatography (methanol/0.1% acetic acid aqueous solution = 0-7%) to give **Compound 120** (7.5 mg, yield: 10.7%).

$^1$H NMR (400 MHz, MeOD) δ 7.46-7.20(m, 5H), 5.59(d, *J* = 9.0 Hz, 1H), 5.11(s, 2H), 4.62(s, 1H), 4.50-4.35(m, 1H), 4.31-4.12(m, 2H), 3.95(s, 2H), 3.41-3.35(m, 2H), 2.26(d, *J* = 9.4 Hz, 1H); LCMS (m/z): 535.2[M+1]$^+$.

Example 94: Synthesis of (4R,4aR,5R,6S,7S,9S,10S,10aR,11S)-2-imino-6-(((2,2,2-trifluoroethyl)amino)methyl)octahydro-5,9-epoxy-7,10a-methanooxocino[4,5-d]pyrimidine-4,6,9,10,11(10H)-pentaol (**Compound 121**)

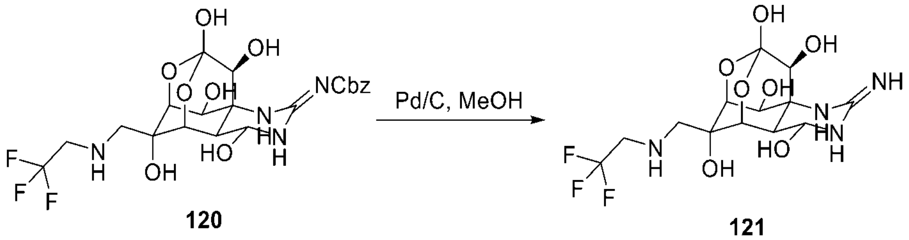


120

121

**Compound 120** (5 mg, 0.01 mmol) and 10% Pd/C (2.5 mg) were added to methanol (5 mL), and the reaction mixture was purged with hydrogen and stirred at 25°C for 4 h under a hydrogen atmosphere. After the reaction was completed, the reaction mixture was filtered, and the filtrate was concentrated and dried. The resulting residue was lyophilized to give **Compound 121** (1.62 mg, yield: 43%).

$^1H$ NMR (400 MHz, $D_2O$) δ 5.44-5.39(m, 1H), 4.49-4.43(m, 2H), 4.23(s, 1H), 4.16-4.01(m, 3H), 3.47-3.39(m, 2H), 2.20(dd, $J$ = 54.7, 9.4 Hz, 1H). LCMS (m/z): 401.1[M+1]$^+$.

Example 95: Synthesis of 3-((((4R,4aR,5R,6S,7S,9S,10S,10aR,11S,E)-2-(((benzyloxy)carbonyl)imino)-4,6,9,10,11-pentahydroxydecahydro-5,9-epoxy-7,10a-methanooxocino[4,5-d]pyrimidin-6-yl)methyl)amino)propanoic acid (**Compound 122**)

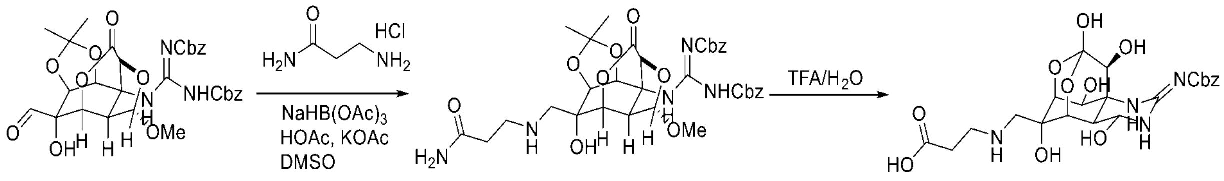


B2

122-1

122

Step 1: Synthesis of **Compound 122-1**

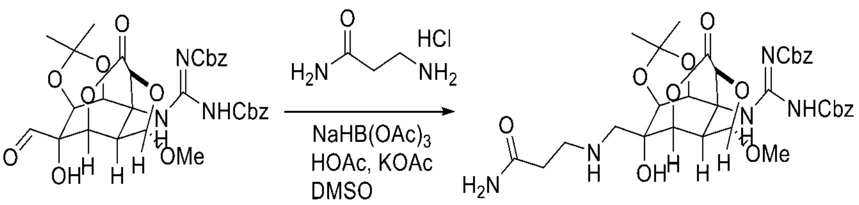


B2

122-1

**Compound B2** (150 mg, 0.24 mmol) was dissolved in DMSO (3 mL), to which 3-aminopropionamide hydrochloride (43.86 mg, 0.35 mmol), acetic acid (166.5 mg, 2.77 mmol) and potassium acetate (132.6 mg, 1.35 mmol) were added, and the reaction mixture was heated to 60°C for 1 h under nitrogen protection. Sodium cyanoborohydride (83.4 mg, 1.33 mmol) was added in batches and the reaction was carried out at 60°C for 30 min. HPLC was used to monitor complete reaction of the raw materials. The reaction solution was cooled to room temperature, to which ice water (20 mL) was added, and stirred, and a large amount of solid was precipitated. Filtration was conducted and the filter cake was collected, concentrated and dried to give crude **Compound 122-1** (100 mg, yield: 60%), which was directly used in the subsequent reaction.

Step 2: Synthesis of **Compound 122**

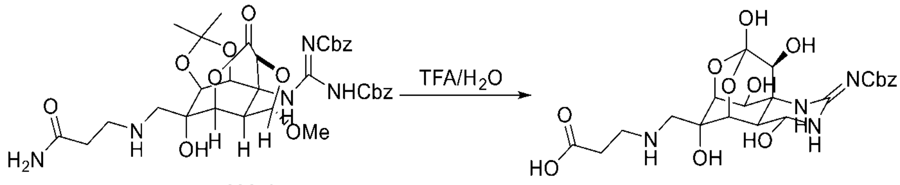


**122-1** **122**

Crude **Compound 122-1** (100 mg, 0.14 mmol) was added to a reaction flask, to which were added water (10 mL) and TFA (5 mL), and the reaction mixture was heated to 60°C under nitrogen protection for 20 h. The reaction solution was concentrated and dried, and the residue was purified by flash column chromatography (MeOH/DCM = 0-3%) to give **Compound 122** (25 mg, yield: 34%).

$^1$H NMR (400 MHz, MeOD) $\delta$ 7.40-7.33(m, 5H), 5.59(d, $J$ = 9.3 Hz, 1H), 5.12(s, 2H), 4.27-4.15(m, 2H), 4.12-3.95(m, 2H), 3.59(s, 2H), 3.24(t, $J$ = 6.0 Hz, 2H), 2.55(t, $J$ = 6.0 Hz, 2H), 2.27(d, $J$ = 9.3 Hz, 1H). LCMS (m/z): 525.2[M+1]$^+$.

Example 96: Synthesis of 3-((((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)amino)propanoic acid (**Compound 123**)

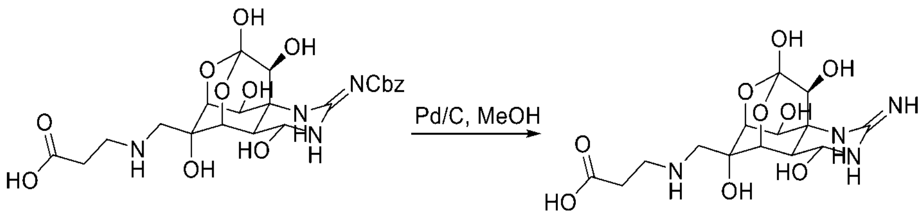


**122** **123**

**Compound 122** (20 mg, 0.04 mmol) was dissolved in methanol (10 mL), to which was added 10% Pd/C (20 mg), the reaction mixture was purged with hydrogen, and the reaction was carried out for 3 h under a hydrogen atmosphere. HPLC was used to monitor complete reaction of the raw materials. The reaction solution was filtered, and the filter cake was rinsed with 0.05 M acetic acid aqueous solution. The filtrate was concentrated to give a crude compound, which was slurried with methyl tert-butyl ether (10 mL), allowed to stand, and the supernatant was taken out. The residue was concentrated and dried to give **Compound 123** (14 mg, yield: 94%).

$^1$H NMR (400 MHz, $D_2O$) $\delta$ 5.43(d, $J$ = 9.2 Hz, 1H), 4.26(s, 2H), 4.09(s, 1H), 3.91(s, 1H), 3.72-3.52(m, 2H), 3.34(t, $J$ = 6.4 Hz, 2H), 2.69(t, $J$ = 6.4 Hz, 2H), 2.31(d, $J$ = 9.4 Hz, 1H); LCMS (m/z): 391.1[M+1]$^+$.

Example 97: Synthesis of benzyl ((4R,4aR,5R,6S,7S,9S,10S,10aR,11S,E)-6-(((2,2-difluoroethyl)amino)methyl)-4,6,9,10,11-pentahydroxyoctahydro-5,9-epoxy-7,10a-methanooxocino[4,5-d]pyrimidin-2(1H)-ylidene)carbamate (**Compound 124**)

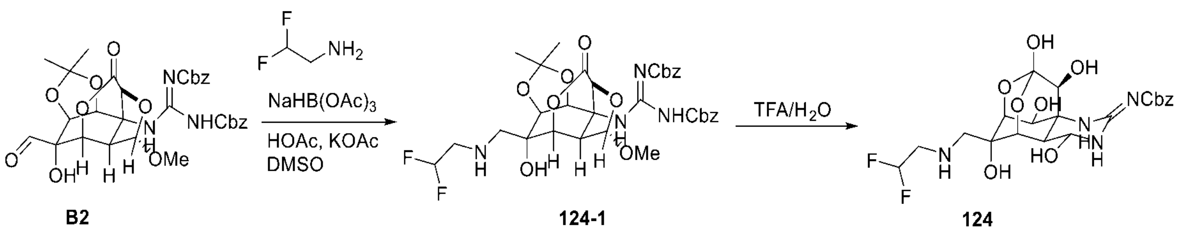


**B2** **124-1** **124**

Step 1: Synthesis of **Compound 124-1**

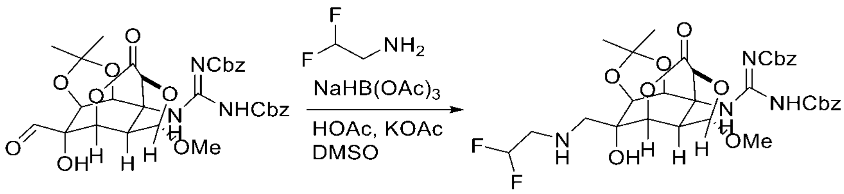


B2 124-1

**Compound B2** (190 mg, 0.30 mmol), 2,2-difluoroethylamine (36.2 mg, 0.45 mmol), acetic acid (196 mg, 3.4 mmol), and potassium acetate (161.7 mg, 1.7 mmol) were dissolved in DMSO (4 mL), which was heated to 60 °C under nitrogen protection and stirred for 1 h. Sodium triacetylborohydride (94.2 mg, 1.5 mmol) was added and the reaction mixture was stirred for another h. After the reaction was completed, the reaction system was cooled to room temperature, water (2 mL) was added to precipitate a white solid. The reaction system was stirred for 5 min, and filtered to give **Compound 124-1** as off-white solid (200 mg, yield: 95.6%). LCMS (m/z): 705.3[M+1]$^+$.

Step 2: Synthesis of **Compound 124**

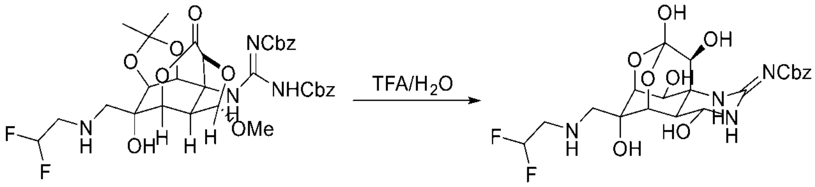


124-1 124

**Compound 124-1** (200 mg, 0.28 mmol) was dissolved in a solution of TFA: $H_2O$ = 2:1(4 mL), and heated to 60 °C under nitrogen protection and stirred for 16 h. After the reaction was completed, the reaction solution was concentrated and dried, and the residue was purified by reverse phase column chromatography (methanol/0.1% acetic acid aqueous solution = 5%-8%) to give **Compound 124** as a white solid (25 mg, yield: 17.1%).

$^1$H NMR (400 MHz, MeOD) δ 7.47-7.29(m, 5H), 6.13-5.77(m, 1H), 5.65(d, *J* = 8.0 Hz, 1H), 5.22(s, 2H), 4.60(s, 1H), 4.19(d, *J* = 17.2 Hz, 2H), 3.97(s, 2H), 3.27(s, 1H), 3.12-3.00(m, 2H), 2.33(d, J= 9.0 Hz, 1H); LCMS (m/z): 517.2[M+1]$^+$.

Example 98: Synthesis of (4R,4aR,5R,6S,7S,9S,10S,10aR,11S)-6-(((2,2-difluoroethyl)amino)methyl)-2-iminooctahydro-5,9-epoxy-7,10a-methanooxocino[4,5-d]pyrimidine-4,6,9,10,11(10H)-pentaol (**Compound 125**)

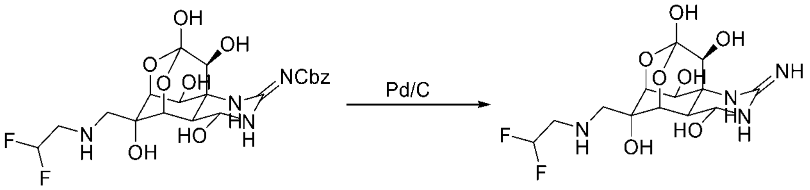


124 125

**Compound 124** (7.0 mg, 0.013 mmol) was dissolved in methanol (2 mL), to which 10% Pd/C (1 mg) was added. The reaction mixture was purged with hydrogen three times and stirred under 25 °C for 8 h. After the reaction was completed, 1% acetic acid aqueous solution was added, the reaction mixture was stirred for 5 min, filtered, then the filter cake was rinsed with 1% acetic acid aqueous solution three times. The filtrate was concentrated to dryness, and then lyophilized to give **Compound 125** as off-white solid (3.2 mg, yield: 61.8%).

$^1$H NMR (400 MHz, $D_2O$) δ 6.31(t, *J* = 53.3 Hz, 1H), 5.42(d, *J* = 9.3 Hz, 1H), 4.27(d, *J* = 10.7 Hz, 2H), 4.10(s, 1H), 3.90(s, 1H), 3.74(s, 2H), 3.71-3.59(m, 2H), 2.30(d, *J* = 9.6 Hz, 1H); LCMS (m/z): 383.1[M+1]$^+$.

Example 99: Synthesis of benzyl ((4R,4aR,5R,6S,7S,9S,10S,10aR,11S,E)-6-(((2-fluoroethyl)amino)methyl)-4,6,9,10,11-pentahydroxyoctahydro-5,9-epoxy-7,10a-methanooxocino[4,5-d]pyrimidin-2(1H)-ylidene)carbamate (**Compound 126**)

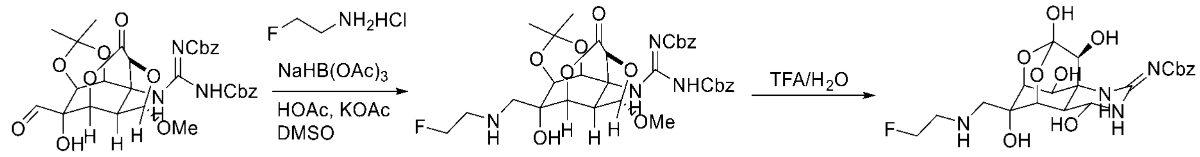


**B2** **126-1** **126**

Step 1: Synthesis of **Compound 126-1**

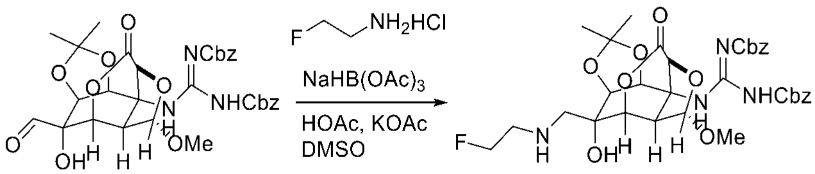


**B2** **126-1**

**Compound B2** (150 mg, 0.23 mmol), 2-fluoroethylamine hydrochloride (35.04 mg, 0.35 mmol), and KOAc (132.4 mg, 1.35 mmol) were dissolved in anhydrous DMSO (1.5 mL), AcOH (166.3 mg, 2.77 mmol) was added dropwise to the mixed solution, and the mixture was heated to 60 °C for 1 h. Sodium cyanoborohydride (73.7 mg, 1.17 mmol) was added to the reaction solution in batches, and the reaction was continued at 60 °C for 1 h. HPLC was used to monitor the reaction until it was completed. Ice water (15 mL) was added dropwise to the reaction solution, white solid was precipitated. The suspension was filtered, the filtrate was extracted with ethyl acetate (10 mL×3). The organic phases were combined, washed with saturated brine (25 mL), dried over anhydrous sodium sulfate, filtered. The filtrate was concentrated under reduced pressure, and combined with the filtered filter cake to give crude **Compound 126-1** (161 mg), which was directly used in the subsequent reaction. LCMS (m/z): 687.5[M+1]$^+$.

Step 2: Synthesis of **Compound 126**

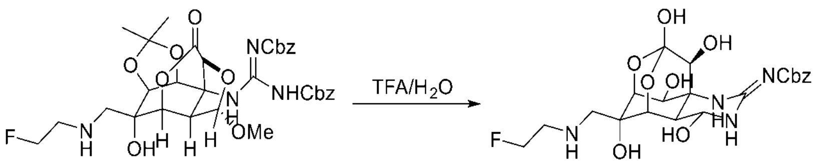


**126-1** **126**

**Compound 126-1** (161 mg, 0.23 mmol) was dissolved in TFA (2 mL) and purified water (4 mL) and the reaction was carried out at 60 °C for 45 h. HPLC was used to monitor complete reaction of the raw materials. The reaction solution was concentrated under reduced pressure to give a crude product. The crude product was purified by reverse phase column chromatography (A: 0.1% AcOH, B: MeOH; 0-0.1%) to give **Compound 126** as white solid (35 mg, two-step yield: 30%).

$^1$H NMR (400 MHz, MeOD) δ 7.45-7.36(m, 5H), 5.71(d, *J* = 9.1 Hz, 1H), 5.30(s, 2H), 4.89-4.86(m, 1H), 4.77-4.73(m, 1H), 4.28(s, 2H), 4.15-3.95(m, 2H), 3.72(s, 2H), 3.54-3.50(m, 1H), 3.50-3.38(m, 1H), 2.41(d, *J* = 9.3 Hz, 1H); LCMS (m/z): 499.2[M+1]$^+$.

Example 100: Synthesis of (4R,4aR,5R,6S,7S,9S,10S,10aR,11S)-6-(((2-fluoroethyl)amino)methyl)-2-iminooctahydro-5,9-epoxy-7,10a-methanooxocino[4,5-d]pyrimidine-4,6,9,10,11(10H)-pentaol (Compound 127)

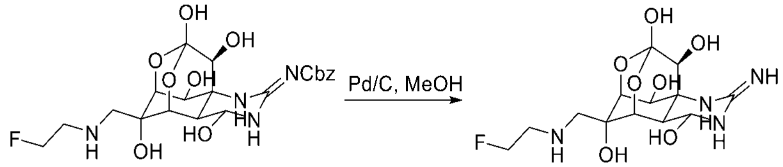


**126** **127**

**Compound 126** (25 mg, 0.05 mmol) was dissolved in MeOH (1.5 mL), to which was Pd/C (2.5 mg), and the reaction was carried out for 3 h at room temperature under a hydrogen atmosphere. HPLC was used to monitor complete reaction of the raw materials. Then 0.5% AcOH/$H_2O$ (6 mL) and MeOH (1.5 mL) were added and the reaction was carried out with stirring for 1 h. Filtration was conducted and the filter cake was rinsed with 0.5% AcOH/$H_2O$ (10 mL) and MeOH (10 mL). The resulting filtrate was concentrated under reduced pressure and lyophilized to give **Compound 127** as off-white solid (11.69 mg, yield: 64.0%).

$^1$H NMR (400 MHz, MeOD) $\delta$ 5.51(d, *J* = 9.4 Hz, 1H), 4.36(d, *J* = 7.6 Hz, 2H), 4.19(s, 1H), 3.99(s, 2H), 3.78(d, *J* = 5.5 Hz, 2H), 3.74-3.67(m, 1H), 3.65-3.60(m, 1H), 3.58-3.53(m, 1H), 2.40(dd, *J* = 9.4, 4.8 Hz, 1H); LCMS (m/z): 365.3 $[M+1]^+$.

Example 101: Synthesis of benzyl ((4R,4aR,5R,6S,7S,9S,10S,10aR,11S,E)-6-(((3,3-difluoropropyl)amino) methyl)-4,6,9,10,11-pentahydroxyoctahydro-5,9-epoxy-7,10a-methanooxocino[4,5-d]pyrimidin-2(1H)-ylidene)carbamate (**Compound 128**)

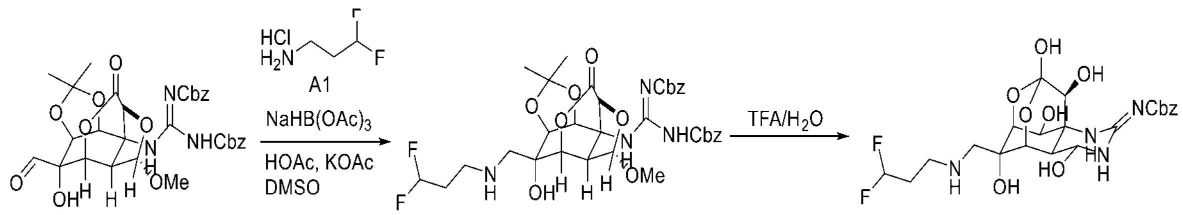


**B2** **128-1** **128**

Step 1: Synthesis of **Compound 128-1**

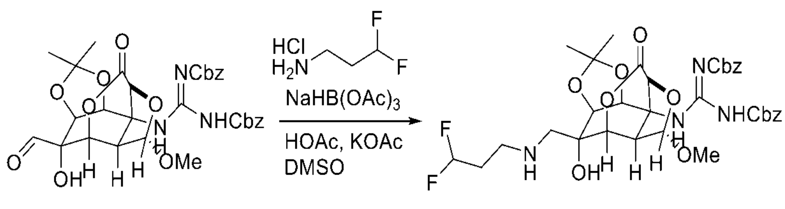


**B2** **128-1**

**Compound B2** (190 mg, 0.30 mmol), 3,3-difluoropropylamine hydrochloride (36.2 mg, 0.45 mmol), acetic acid (196 mg, 3.4 mmol) and potassium acetate (161.7 mg, 1.7 mmol) were dissolved in DMSO (4 mL), and the reaction mixture was heated to 60 °C under nitrogen protection with stirring for 1 h. Sodium triacetylborohydride (94.2 mg, 1.5 mmol) was added and the reaction mixture was stirred for another 1 h. After the reaction was completed, the mixture was cooled to room temperature, water (2 mL) was added to the reaction solution to precipitate a white solid, and the mixture was stirred for 5 min and filtered to give **Compound 128-1** as off-white solid (200 mg, yield: 93.7%). LCMS (m/z): 719.3$[M+1]^+$.

Step 2: Synthesis of **Compound 128**

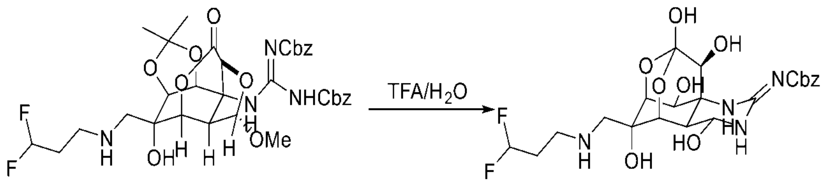


128-1 128

**Compound 128-1** (200 mg, 0.28 mmol) was dissolved in TFA: $H_2O$ = 2:1 aqueous solution (4 mL), and the reaction mixture was heated to 60 °C under nitrogen protection with stirring for 16 h. After the reaction was completed, the reaction solution was purified by reverse phase column chromatography (methanol/0.1% acetic acid aqueous solution = 5%-8%) to give **Compound 128** as white solid (32.5 mg, yield: 22.2%).

$^1$H NMR (400 MHz, MeOD) δ 7.48-7.23(m, 5H), 6.28-5.85(m, 1H), 5.62(d, *J* = 9.4 Hz, 1H), 5.18(s, 2H), 4.22(s, 2H), 4.04(s, 2H), 3.55-3.42(m, 2H), 3.20-3.06(m, 2H), 2.35-2.17(m, 3H); LCMS (m/z): 531.2[M+1]$^+$.

Example 102: Synthesis of (4R,4aR,5R,6S,7S,9S,10S,10aR,11S)-6-(((3,3-difluoropropyl)amino)methyl)-2-iminooctahydro-5,9-epoxy-7,10a-methanooxocino[4,5-d]pyrimidine-4,6,9,10,11(10H)-pentaol (**Compound 129**)

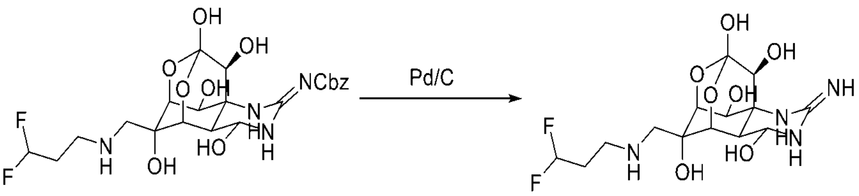


128 129

**Compound 128** (7.0 mg, 0.01 mmol) was dissolved in methanol (2 mL), to which Pd/C (1 mg) was added. The reaction mixture was purged with hydrogen three times, and stirred under 25 °C for 3 h. After the reaction was completed, 1% acetic acid aqueous solution was added, and the reaction mixture was stirred for 5 min, and filtered. The mixture was rinsed three times with 1% acetic acid aqueous solution, the filtrate was concentrated, dried, and lyophilized to give **Compound 129** as off-white solid (3.5 mg, yield: 67.3%).

$^1$H NMR (400 MHz, $D_2O$) δ 6.06(tt, *J* = 55.5, 3.7 Hz, 1H), 5.42(d, *J* = 9.4 Hz, 1H), 4.26(d, *J* = 5.9 Hz, 2H), 4.08(s, 1H), 3.90(s, 1H), 3.69-3.55(m, 2H), 3.39-3.29(m, 2H), 2.45-2.24(m, 3H); LCMS (m/z): 397.2[M+1]$^+$.

Example 103: Synthesis of benzyl ((4R,4aR,5R,6S,7S,9S,10S,10aR,11S,E)-6-(((2,2-difluoroethyl)(methyl)amino) methyl)-4,6,9,10,11-pentahydroxyoctahydro-5,9-epoxy-7,10a-methanooxocino[4,5-d]pyrimidin-2(1H)-ylidene)carbamate (**Compound 130**)

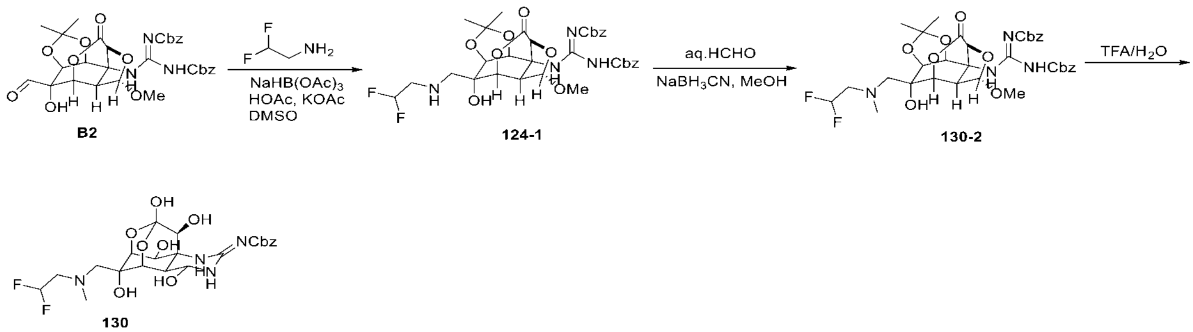


B2 124-1 130-2

130

Step 1: Synthesis of **Compound 124-1**

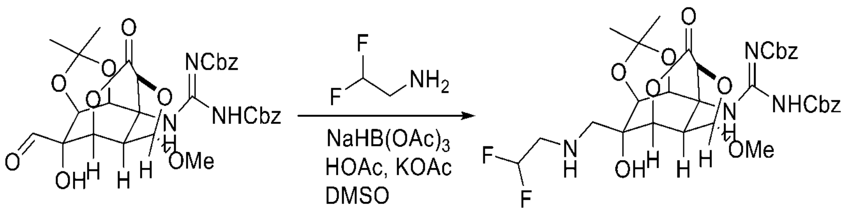


B2 124-1

**Compound B2** (180 mg, 0.28 mmol), 2,2-difluoroethylamine (34.24 mg, 0.42 mmol) and KOAc (158.9 mg, 1.62 mmol) were dissolved in anhydrous DMSO (2 mL), and then AcOH (199.5 mg, 3.32 mmol) was added dropwise to the mixed solution. The reaction solution was heated at 60 °C for 1 h. $NaBH_3CN$ (88.5 mg, 1.41 mmol) was added to the reaction solution in batches, and the reaction was continued at 60 °C for 1 h. HPLC was used to monitor complete reaction of the raw materials. The reaction solution was diluted with water (15 mL), extracted with ethyl acetate (10 mL×3). The organic phases were combined and washed with saturated brine (25 mL), dried over anhydrous sodium sulfate, filtered and concentrated under reduced pressure to give a crude product. The crude product was purified by silica gel column chromatography (EA/PE = 0-35%) to give **Compound 124-1** as white solid (130 mg, yield: 65.6%). LCMS (m/z): 705.3 $[M+1]^+$.

Step 2: Synthesis of **Compound 130-2**

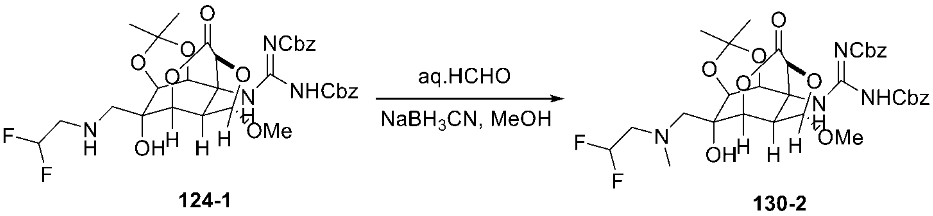


124-1 130-2

**Compound 124-1** (130 mg, 0.18 mmol) and KOAc (104.2 mg, 1.06 mmol) were dissolved in MeOH (2 mL), and AcOH (130.8 mg, 2.18 mmol) and 37% formaldehyde aqueous solution (44.9 mg, 0.55 mmol) were added dropwise to the mixed solution. The reaction was carried out at room temperature for 1 h. $NaBH_3CN$ (58.0 mg, 0.92 mmol) was added to the reaction solution in batches, and the reaction was continued at room temperature for 1 h. HPLC was used to monitor the the reaction until it was completed. The reaction solution was diluted with water (15 mL), extracted with ethyl acetate (10 mL×3). The organic phases were combined and washed with saturated brine (25 mL), dried over anhydrous sodium sulfate, filtered and concentrated under reduced pressure to give a crude product. The crude product was purified by thin-layer chromatography (EA/PE = 1/5) to give **Compound 130-2** as white solid (120 mg, yield: 90.5%). LCMS (m/z): 719.3 $[M+1]^+$.

Step 3: Synthesis of **Compound 130**

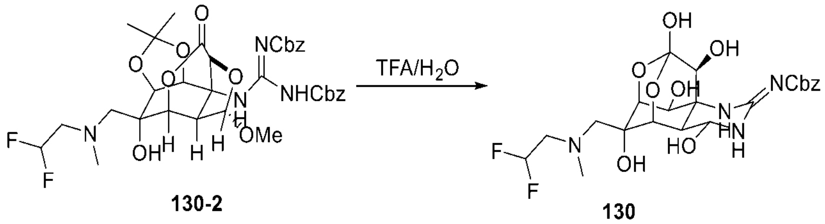


130-2 130

According to the synthesis procedures of Example 1, **Compound 130** was obtained as off-white solid (11.14 mg,

yield: 11.3%).

$^1$H NMR (400 MHz, MeOD) $\delta$ 7.39-7.25(m, 5H), 6.16-5.83(m, 1H), 5.54(d, $J$ = 4.0 Hz, 1H), 5.06(s, 2H), 4.70-4.50(m, 1H), 4.21-4.05(m, 2H), 4.03-3.78(m, 2H), 3.21-3.12(m, 1H), 3.01-2.90(m, 2H), 2.54(s, 3H), 2.27-2.20(m, 1H); LCMS (m/z): 531.4[M+1]$^+$.

Example 104: Synthesis of (4R,4aR,5R,6S,7S,9S,10S,10aR,11S)-6-(((2,2-difluoroethyl)(methyl)amino)methyl)-2-iminooctahydro-5,9-epoxy-7,10a-methanooxocino[4,5-d]pyrimidine-4,6,9,10,11(10H)-pentaol (**Compound 131**)

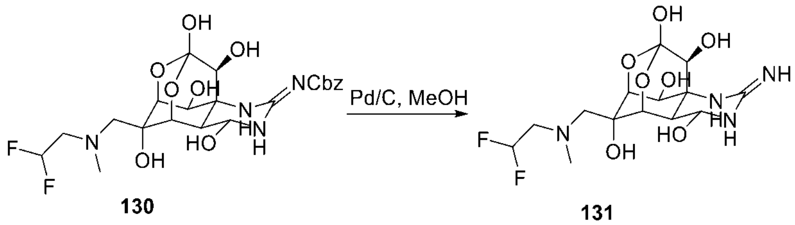


130 131

According to the synthesis procedures of Example 2, **Compound 131** was obtained as off-white solid (1.04 mg, yield: 16.83%).

$^1$H NMR (400 MHz, $D_2O$) $\delta$ 6.59-6.26(m, 1H), 5.52(d, $J$ =6.0 Hz, 1H), 4.42-4.30(m, 2H), 4.24-4.17(m, 1H), 4.08-3.98(m, 1H), 3.95-3.86(m, 2H), 3.85-3.74(m, 2H), 3.12(s, 3H), 2.45-2.37(m, 1H). LCMS (m/z): 397.3[M+1]$^+$.

Example 105: Synthesis of benzyl ((4R,4aR,5R,6S,7S,9S,10S,10aR,11S,E)-4,6,9,10,11-pentahydroxy-6-(((5-oxopyrrolidin-3-yl)amino)methyl)octahydro-5,9-epoxy-7,10a-methanooxocino[4,5-d]pyrimidin-2(1H)-ylidene)carbamate (**Compound 132**)

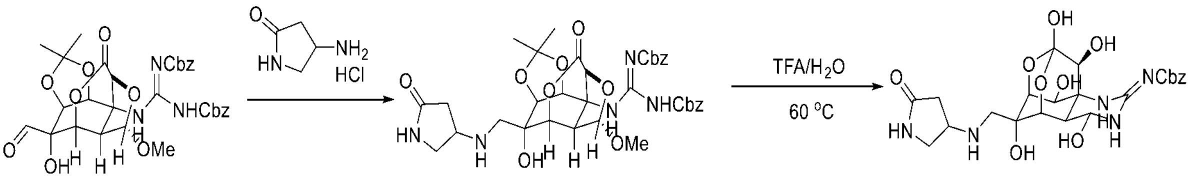


B2 132-1 132

Step 1: Synthesis of **Compound 132-1**

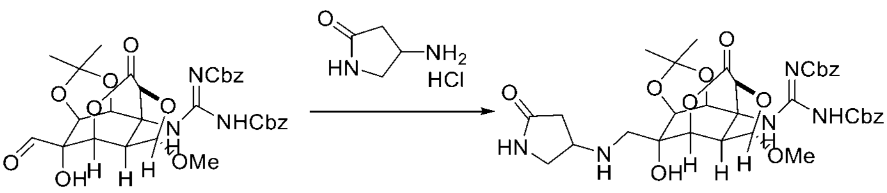


B2 132-1

**Compound B2** (150 mg, 0.24 mmol), compound 4-amino-2-pyrrolidone hydrochloride (64.1 mg, 0.47 mmol), acetic acid (81.1 mg, 1.35 mmol) and potassium acetate (272.1 mg, 2.77 mmol) were dissolved in DMSO (6 mL), and the reaction mixture was heated to 60 °C under nitrogen atmosphere and stirred for 2 h. Sodium triacetylborohydride (562.8 mg, 2.66 mmol) was added, and stirring was continued for 1 h. After the reaction was completed, the mixture was cooled to room temperature, water (30 mL) was added to the reaction solution to precipitate a white solid. The mixture was stirred for another 15 min, and filtered to give **Compound 132-1** as off-white solid (148 mg, yield: 87%). LCMS (m/z): 724.3[M+1]$^+$.

Step 2: Synthesis of **Compound 132**

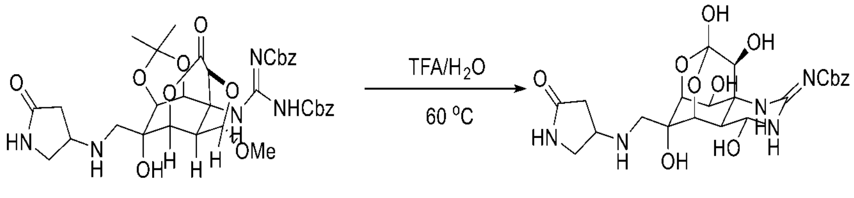


132-1 132

**Compound 132-1** (148 mg, 0.20 mmol) was dissolved in TFA: $H_2O$ = 1:3 aqueous solution (12 mL), which was heated to 60 °C under nitrogen protection and stirred for 17 h. After the reaction was completed, the reaction solution was concentrated and dried, and the resulting residue was purified by reverse phase column chromatography (methanol/0.1% acetic acid aqueous solution = 0-2%) to give **Compound 132** as white solid (21 mg, yield: 19.2%).

$^1H$ NMR (400 MHz, MeOD) $\delta$ 7.49-7.18(m, 5H), 5.61(d, $J$ = 8.5 Hz, 1H), 5.19(s, 2H), 4.60(s, 1H), 4.20(s, 2H), 3.95(s, 2H), 3.75-3.56(m, 2H), 3.25-3.09(m, 2H), 2.63(dd, $J$ = 16.9, 7.5 Hz, 1H), 2.35-2.20(m, 2H); LCMS (m/z): 536.2 $[M+1]^+$.

Example 106: Synthesis of 4-((((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)amino)pyrrolidin-2-one (**Compound 133**)

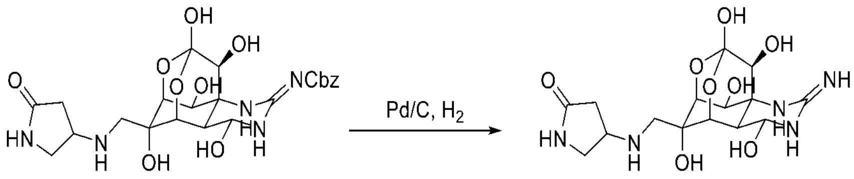


132 133

**Compound 132** (10.0 mg, 0.02 mmol) was dissolved in methanol (2 mL), and 10% Pd/C (1 mg) was added. The reaction mixture was purged with hydrogen three times, and stirred under 25 °C for 3 h. After the reaction was completed, 1% acetic acid aqueous solution was added, and the reaction mixture was stirred for 5 min and filtered. The filter cake was washed with 1% acetic acid aqueous solution three times, and the filtrate was concentrated and dried to give **Compound 133** as off-white solid (4.6 mg, yield: 62.2%).

$^1H$ NMR (400 MHz, $D_2O$) $\delta$ 5.42(d, $J$ = 9.4 Hz, 1H), 4.32-4.21(m, 3H), 4.08(s, 1H), 3.94-3.81(m, 2H), 3.68-3.51(m, 3H), 2.92(dd, J= 18.1, 9.0 Hz, 1H), 2.60(dd, $J$ = 18.1, 4.8 Hz, 1H), 2.29(d, $J$ = 9.5 Hz, 1H); LCMS (m/z): 402.2$[M+1]^+$.

Example 107: Synthesis of benzyl ((4R,4aR,5R,6S,7S,9S,10S,10aR,11S,E)-6-((5,8-dioxa-2-azaspiro[3.4]octan-2-yl)methyl)-4,6,9,10,11-pentahydroxyoctahydro-[5,9]epoxy[7,10a]methanooxocino[4,5-d]pyrimidin-2(1H)-ylidene)carbamate (**Compound 134**)

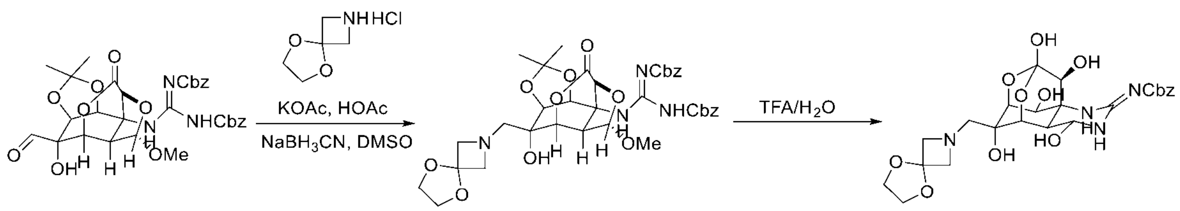


B2 134-1 134

Step 1: Synthesis of **Compound 134-1**

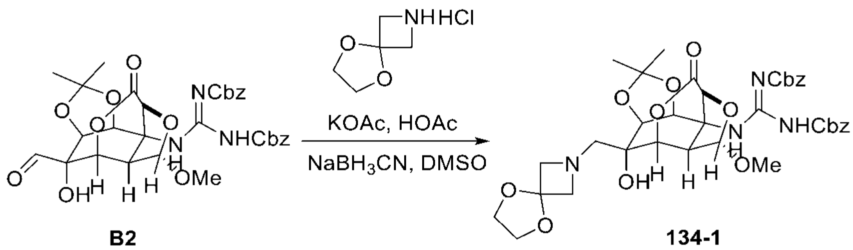


B2

134-1

According to the synthesis procedures of **Compound 63-1** in Example 49, **Compound B2** (200 mg, 0.31 mmol) was added. After the reaction was completed, the reaction solution was cooled to room temperature, then added to water (30 mL) to precipitate a white solid, which was filtered. The filter cake was washed with water (10 mL×2), collected, and dried to give **Compound 134-1** as white solid (220 mg, yield: 96%). LCMS (m/z): 739.3[M+1]$^+$.

Step 2: Synthesis of **Compound 134**

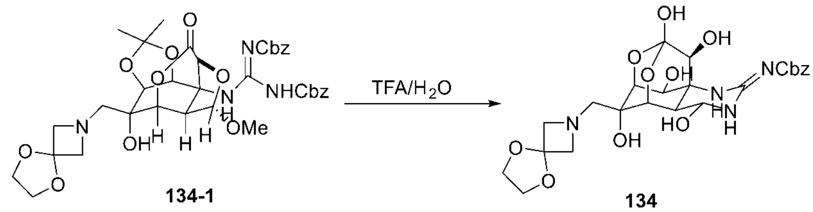


134-1

134

According to the synthesis procedures of Example 1, **Compound 134-1** (210 mg, 0.28 mmol) was added to give **Compound 134** as white solid (30.2 mg, yield: 19%).

$^1$H NMR (400 MHz, MeOD) $\delta$ 7.45-7.27(m, 5H), 5.61(d, *J* = 9.0 Hz, 1H), 5.19(s, 2H), 4.32-4.16(m, 2H), 4.12(s, 1H), 4.04(s, 1H), 4.02(s, 4H), 3.93(s, 4H), 3.61-3.37(m, 2H), 2.32(d, *J* = 9.4 Hz, 1H); LCMS (m/z): 551.2[M+1]$^+$.

Example 108: Synthesis of (4R,4aR,5R,6S,7S,9S,10S,10aR,11S)-6-((5,8-dioxa-2-azaspiro[3.4]octan-2-yl)methyl)-2-iminooctahydro-5,9-epoxy-7,10a-methanooxocino[4,5-d]pyrimidine-4,6,9,10,11(10H)-pentaol (**Compound 135**)

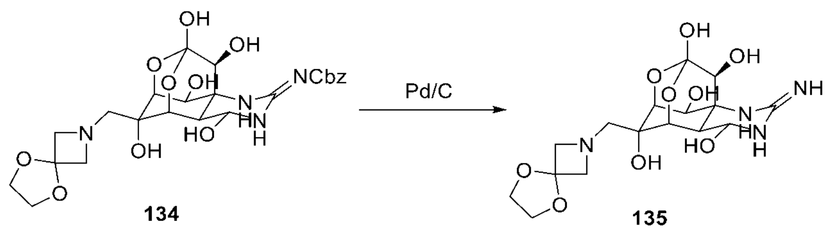


134

135

According to the synthesis procedures of Example 2, **Compound 134** (12 mg, 0.02 mmol) was added to give **Compound 135** as white solid (8 mg, yield: 88%).

$^1$H NMR (400 MHz, $D_2O$) $\delta$ 5.34(d, *J* = 9.4 Hz, 1H), 4.49(s, 4H), 4.16(s, 1H), 4.11(s, 1H), 3.95-3.87(m, 6H), 3.85-3.77(m, 2H), 2.20(d, *J* = 9.5 Hz, 1H); LCMS (m/z): 417.2[M+1]$^+$.

Example 109: Synthesis of benzyl ((4R,4aR,5R,6S,7S,9S,10S,10aR,11S,E)-4,6,9,10,11-pentahydroxy-6-(((2-(methylamino)-2-oxoethyl)amino)methyl)octahydro-5,9-epoxy-7,10a-methanooxocino[4,5-d]pyrimidin-2(1H)-ylidene)carbamate (**Compound 136**)

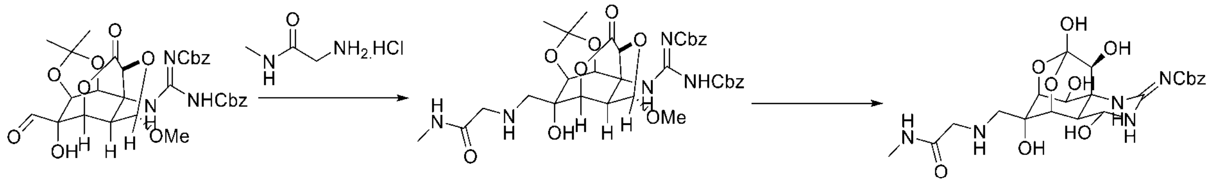

B2 136-1 136

Step 1: Synthesis of **Compound 136-1**

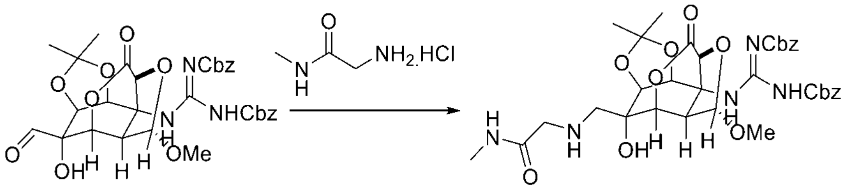

B2 136-1

According to the synthesis procedures of **Compound 63-1** in Example 49, **Compound B2** (190 mg, 0.30 mmol) was added. After the reaction was completed, the reaction solution was cooled to room temperature, then added to water (30 mL) to precipitate a white solid, which was filtered. The filter cake was washed with water (10 mL×2), collected, and dried to give **Compound 136-1** as white solid (210 mg, yield: 99%). LCMS (m/z): 712.3[M+1]$^+$.

Step 2: Synthesis of **Compound 136**

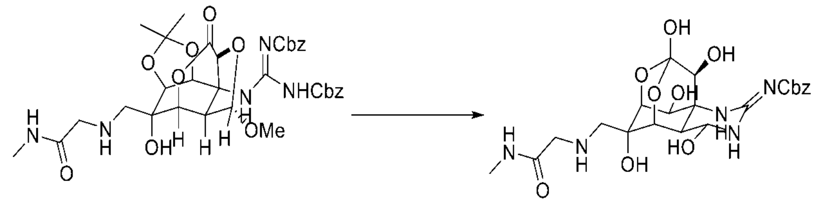

136-1 136

According to the synthesis procedures of Example 1, **Compound 136-1** (210 mg, 0.30 mmol) was added to give **Compound 136** as white solid (32 mg, yield: 21%).

$^1$H NMR (400 MHz, MeOD) δ 7.44-7.30(m, 5H), 5.64(d, $J$ = 8.9 Hz, 1H), 5.22(s, 2H), 4.36-3.84(m, 4H), 3.45(s, 2H), 3.25(s, br, 2H), 2.79(s, 3H), 2.35(d, $J$ = 9.4 Hz, 1H); LCMS (m/z): 524.2[M+1]$^+$.

Example 110: Synthesis of N-methyl-2-((((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)amino)acetamide (**Compound 137**)

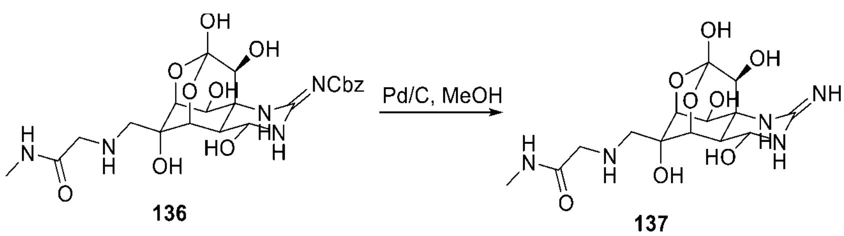


136 137

According to the synthesis procedures of Example 2, **Compound 136** (30 mg, 0.06 mmol) was added to give

**Compound 137** as white solid (19 mg, yield: 85%).

$^1H$ NMR (400 MHz, $D_2O$) δ 5.57(d, $J$ = 9.4 Hz, 1H), 4.42(d, $J$ = 13.6 Hz, 2H), 4.26(s, 1H), 4.06(s, 3H), 3.82-3.70(m, 2H), 2.87(s, 3H), 2.45(d, $J$ = 9.4 Hz, 1H). LCMS (m/z): 390.4[M+1]$^+$.

Example 111: Synthesis of benzyl ((4R,4aR,5R,6S,7S,95,10S,10aR,11S,E)-4,6,9,10,11-pentahydroxy-6-(((6-oxopiperidin-3-yl)amino)methyl)octahydro-5,9-epoxy-7,10a-methanooxocino[4,5-d]pyrimidin-2(1H)-ylidene)carbamate (**Compound 138**)

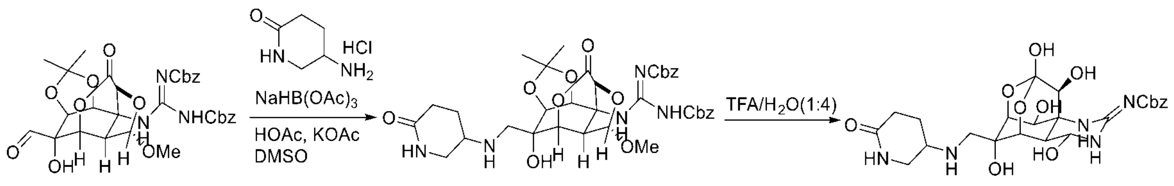


**B2** **138-1** **138**

Step 1: Synthesis of **Compound 138-1**

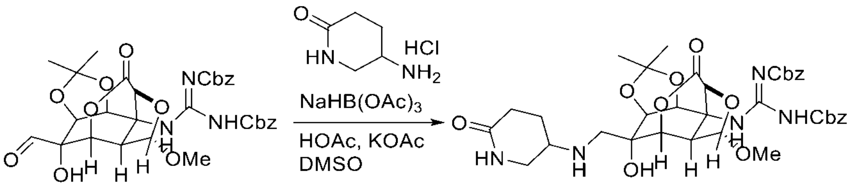


**B2** **138-1**

According to the synthesis procedures of **Compound 63-1** in Example 49, **Compound B2** (180 mg, 0.28 mmol) was added. After the reaction was completed, the reaction solution was cooled to room temperature, then added to water (30 mL) to precipitate a white solid, which was filtered. The filter cake was washed with water (10 mL×2), collected, and dried to give **Compound 138-1** as white solid (190 mg, yield: 92%). LCMS (m/z): 738.3[M+1]$^+$.

Step 2: Synthesis of **Compound 138**

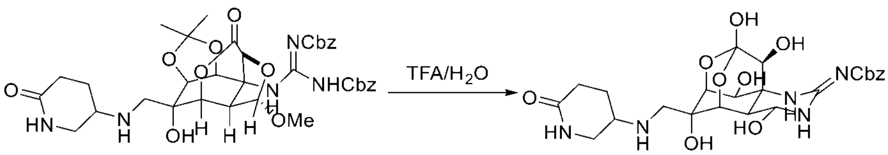


**138-1** **138**

According to the synthesis procedures of Example 1, **Compound 138-1** (190 mg, 0.26 mmol) was added to give **Compound 138** as white solid (33 mg, yield: 23%).

$^1H$ NMR (400 MHz, MeOD) δ 7.41-7.29(m, 5H), 5.57(d, $J$ = 9.5 Hz, 1H), 5.17-5.08(m, 2H), 4.22(d, $J$ = 5.9 Hz, 1H), 4.16-4.01(m, 3H), 4.00-3.80(m, 2H), 3.79-3.63(m, 1H), 3.39-3.32(m, 1H), 3.23-3.11(m, 1H), 2.66-2.21(m, 4H), 2.06-1.98(m, 1H). LCMS (m/z): 550.2[M+1]$^+$.

Example 112: Synthesis of 5-((((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)amino)piperidin-2-one (**Compound 139**)

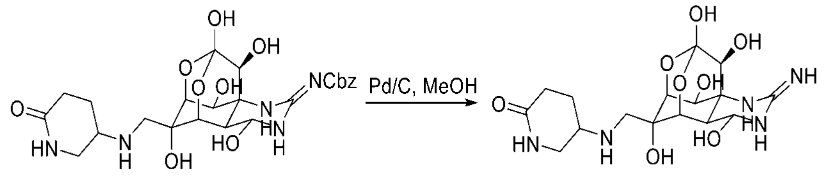


138 139

According to the synthesis procedures of Example 2, **Compound 138** (30 mg) was added to give **Compound 139** as white solid (22 mg, yield: 97%).

$^1H$ NMR (400 MHz, $D_2O$) $\delta$ 5.34(d, $J$ = 9.4 Hz, 1H), 4.18-4.00(m, 4H), 3.92-3.78(m, 2H), 3.49(dd, $J$ = 14.3, 7.3 Hz, 1H), 3.26(d, $J$ = 13.3 Hz, 1H), 3.15-3.07(m, 1H), 2.49-2.37(m, 2H), 2.33-2.24(m, 1H), 2.19(t, $J$ = 8.9 Hz, 1H), 1.92-1.89(m, 1H); LCMS (m/z): 416.4[M+1]+.

Example 113: Synthesis of benzyl ((4R,4aR,5R,6S,7S,9S,10S,10aR,11S,E)-6-((3,3-difluoroazetidin-1-yl)methyl)-4,6,9,10,11-pentahydroxyoctahydro-5,9-epoxy-7,10a-methanooxocino[4,5-d]pyrimidin-2(1H)-ylidene)carbamate (**Compound 140**) and benzyl ((4S,5aS,6S,8R,9S,10S,11S,11aR,12R,E)-9-((3,3-difluoroazetidin-1-yl)methyl)-6,9,11-trihydroxyoctahydro-4,8,11a-(epimethanetriyl)-6,10-epoxyoxocino[4,3-f][1,3,5]oxadiazepin-2(1H)-ylidene)carbamate (**Compound 141**)

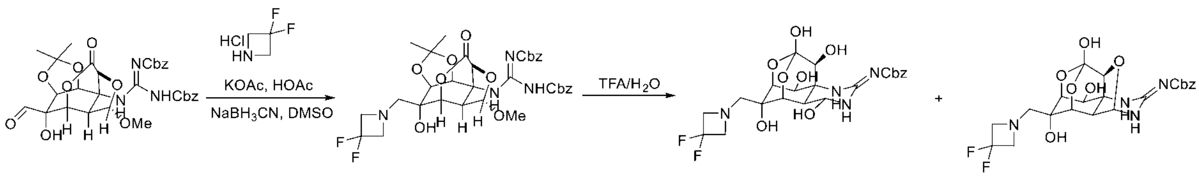


B2 140-1 140 141

Step 1: Synthesis of **Compound 140-1**

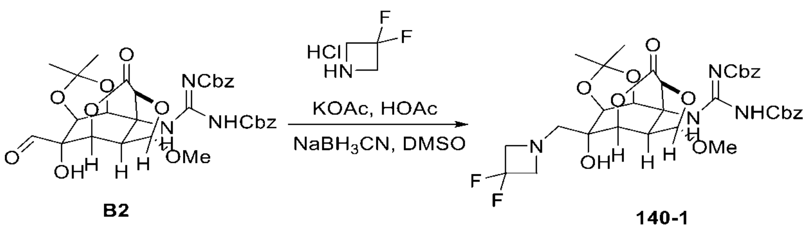


B2 140-1

**Compound B2** (250 mg, 0.39 mmol) was dissolved in DMSO (5 mL), to which 3,3-difluoroazetidine hydrochloride (75.9 mg, 0.59 mmol), glacial acetic acid (276.35 mg, 4.6 mmol) and potassium acetate (220 mg, 2.24 mmol) were added, and the reaction mixture was heated to 60°C and stirred for 1 h under nitrogen protection. Sodium cyanoborohydride (138 mg, 2.2 mmol) was added to the reaction solution in batches and the reaction was carried out for 30 min. HPLC was used to monitor complete reaction of the raw materials. The reaction solution was cooled to room temperature, to which was added ice water (50 mL), extracted with ethyl acetate (25 mL×3). The organic phases were combined, washed with saturated sodium chloride solution (30 mL), dried over anhydrous sodium sulfate, filtered, and the filtrate was concentrated to give about 230 mg of residue. The residue was purified by flash column chromatography (MeOH/DCM = 0-5%) to give **Compound 140-1** (100 mg, yield: 35.8%). LCMS (m/z): 717.3[M+1]+.

Step 2: Synthesis of **Compound 140** and **Compound 141**

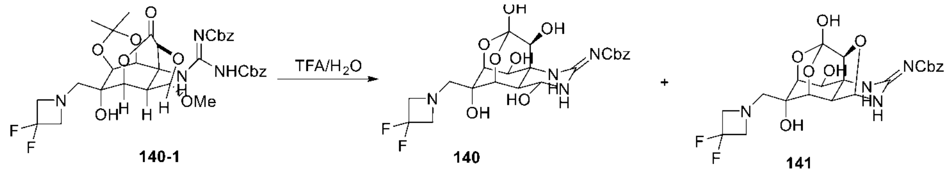


140-1 140 141

**Compound 140-1** (100 mg, 0.14 mmol) was dissolved in water (7.5 mL) and TFA (7.5 mL), heated to 60°C and the reaction was carried out for 20 h under nitrogen protection. The reaction solution was concentrated and dried, and the residue was purified by flash column chromatography (MeOH/DCM = 0-4%) to give **Compound 140** (20 mg, yield: 27.2%) and **Compound 141** (20 mg, yield: 27.3%).

**Compound 140:**

$^1H$ NMR (400 MHz, MeOD) $\delta$ 7.48-7.29(m, 5H), 5.61(d, *J* = 9.1 Hz, 1H), 5.16(s, 2H), 4.65-4.42(m, 1H), 4.16(d, *J* = 19.5 Hz, 2H), 3.94(s, 2H), 3.77(t, *J* = 12.3 Hz, 4H), 3.17(d, *J* = 12.0 Hz, 1H), 2.28(d, *J* = 9.3 Hz, 1H). LCMS (m/z): 529.2 $[M+1]^+$.

**Compound 141:**

$^1H$ NMR (400 MHz, MeOD) $\delta$ 7.49-7.34(m, 5H), 5.49(s, 1H), 5.28(s, 2H), 4.56(d, *J* = 2.1 Hz, 1H), 4.55(s, 1H), 4.27-4.21(m, 1H), 4.10-4.03(m, 1H), 3.77(t, *J* = 12.2 Hz, 4H), 3.23(d, *J* = 13.3 Hz, 1H), 3.08(d, *J* = 13.4 Hz, 1H), 2.88(d, *J* = 3.0 Hz, 1H). LCMS (m/z): 511.2$[M+1]^+$.

Example 114: Synthesis of (4R,4aR,5R,6S,7S,9S,10S,10aR,11S)-6-((3,3-difluoroazetidin-1-yl)methyl)-2-iminooctahydro-5,9-epoxy-7,10a-methanooxocino[4,5-d]pyrimidine-4,6,9,10,11(10H)-pentaol (**Compound 142**)

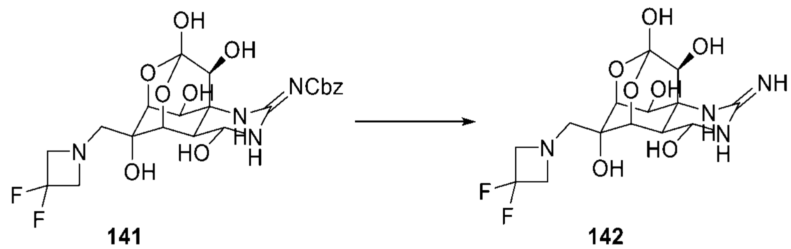

141 142

**Compound 141** (20 mg, 0.04 mmol) was dissolved in methanol (10 mL), to which 10% Pd/C (15 mg, 0.01 mmol) was added. The reaction mixture was purged with hydrogen three times, and hydrogen was introduced into the reaction solution for about 3 h. HPLC was used to monitor complete reaction of the raw materials. The reaction solution was filtered, and the filter cake was rinsed with 0.05 M acetic acid aqueous solution. The filtrate was concentrated to give a crude compound, which was then slurried with methyl tert-butyl ether (10 mL), allowed to stand, and the supernatant was removed. The residue was concentrated and dried to give **Compound 142** (13 mg, yield: 87.2%).

$^1H$ NMR (400 MHz, $D_2O$) $\delta$ 5.47-5.37(m, 1H), 5.09-5.01(m, 1H), 4.66-4.54(m, 1H), 4.28-4.16(m, 2H), 4.01(s, 1H), 3.97-3.76(m, 4H), 2.27(d, *J* = 9.5 Hz, 1H), 1.97(dt, *J* = 4.4, 2.2 Hz, 1H); LCMS (m/z): 395.1$[M+1]^+$.

Example 115: Synthesis of benzyl ((4R,4aR,5R,6S,7S,9S,10S,10aR,11S,E)-6-(((3,3-difluorocyclobutyl)amino) methyl)-4,6,9,10,11-pentahydroxyoctahydro-5,9-epoxy-7,10a-methanooxocino[4,5-d]pyrimidin-2(1H)-ylidene)carbamate (**Compound 143**)

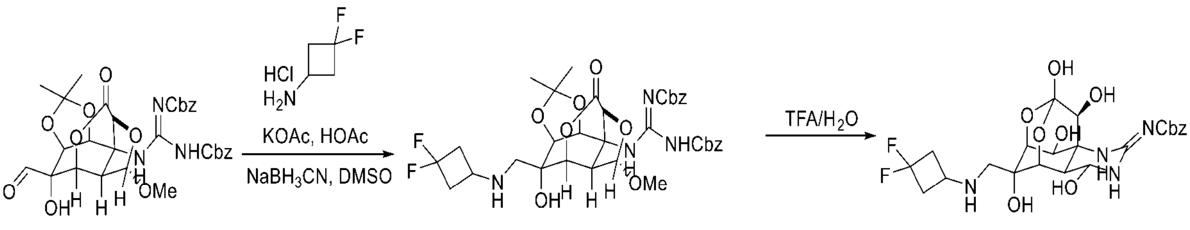


B2 143-1 143

Step 1: Synthesis of **Compound 143-1**

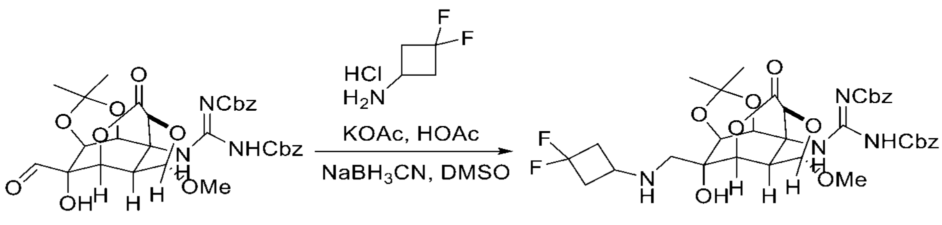


B2 143-1

**Compound B2** (250 mg, 0.39 mmol) was dissolved in dimethyl sulfoxide (5 mL), to which 3,3-difluorocyclobutan-1-amine hydrochloride (75.9 mg, 0.529 mmol), acetic acid (276.35 mg, 4.6 mmol) and potassium acetate (220 mg, 2.24 mmol) were added. The reaction mixture was heated to 60°C under nitrogen protection with stirring for 1 h, then sodium cyanoborohydride (138 mg, 2.2 mmol) was added to the reaction solution in batches, and the reaction was carried out at 60°C for 30 min. HPLC was used to monitor until the feedstock reaction was complete. The reaction solution was cooled to room temperature, to which was added ice water (50 mL), and extracted with ethyl acetate (25 mL×3). The organic phases were combined, washed with saturated brine (30mL), and dried over anhydrous sodium sulfate. The filtrate was concentrated and dried to give a residue (220 mg). The residue was subjected to flash column chromatography (MeOH/DCM = 0-5%) to give **Compound 143-1** (150 mg, yield: 52.6%). LCMS (m/z): 731.3[M+1]$^+$.

Step 2: Synthesis of **Compound 143**

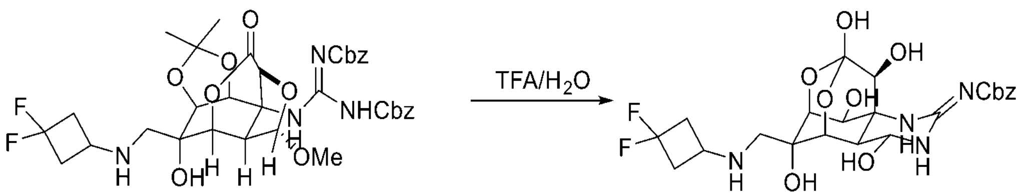


143-1 143

**Compound 143** (150 mg, 0.2 mmol) was dissolved in water (7.5 mL) and TFA (7.5 mL), heated to 60°C under nitrogen protection, and the reaction was carried out for 20 h. HPLC was used to monitor complete reaction of the raw materials. The reaction solution was concentrated and dried, and the residue was subjected to flash column chromatography (MeOH/DCM = 0-4%) to give **Compound 143** (20 mg, yield: 18%).

$^1$H NMR (400 MHz, MeOD) δ 7.43-7.27(m, 5H), 5.59(d, *J* = 9.1 Hz, 1H), 5.15(s, 2H), 4.30-3.81(m, 4H), 3.36-3.32(m, 1H), 3.12(d, *J* = 6.8 Hz, 2H), 2.88-2.74(m, 2H), 2.51-2.34(m, 2H), 2.28(d, *J* = 9.5 Hz, 1H); LCMS (m/z): 543.2[M+1]$^+$.

Example 116: Synthesis of (4R,4aR,5R,6S,7S,9S,10S,10aR,11S)-6-(((3,3-difluorocyclobutyl)amino)methyl)-2-iminooctahydro-5,9-epoxy-7,10a-methanooxocino[4,5-d]pyrimidine-4,6,9,10,11(10H)-pentaol (**Compound 144**)

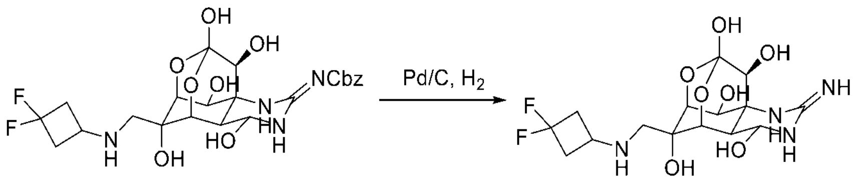


143 144

**Compound 143** (15 mg, 0.03 mmol) was dissolved in methanol (10 mL), to which 10% Pd/C (7.5 mg) was added. The reaction mixture was purged with hydrogen, and hydrogen was introduced into the reaction solution for about 3 h. HPLC was used to monitor complete reaction of the raw materials. The reaction solution was filtered, and the filter cake was rinsed with 0.05 M AcOH aqueous solution. The filtrate was concentrated to give a crude compound, which was slurried with methyl tert-butyl ether (10 mL), allowed to stand, and the supernatant was removed. The residue was concentrated and dried to give **Compound 144** (8 mg, yield: 70.8%).

$^1$H NMR (400 MHz, $D_2O$) $\delta$ 5.35(d, $J$ = 9.4 Hz, 1H), 4.18(s, 2H), 4.00(s, 1H), 3.87-3.69(m, 2H), 3.57-3.39(m, 2H), 3.06-2.94(m, 2H), 2.91-2.78(m, 2H), 2.22(d, $J$ = 9.4 Hz, 1H). LCMS (m/z): 409.2[M+1]$^+$.

Example 117: Synthesis of benzyl ((4R,4aR,5R,6S,7S,9S,10S,10aR,11S,E)-4,6,9,10,11-pentahydroxy-6-(piperazin-1-ylmethyl)octahydro-5,9-epoxy-7,10a-methanooxocino[4,5-d]pyrimidin-2(1H)-ylidene)carbamate (**Compound 145**)

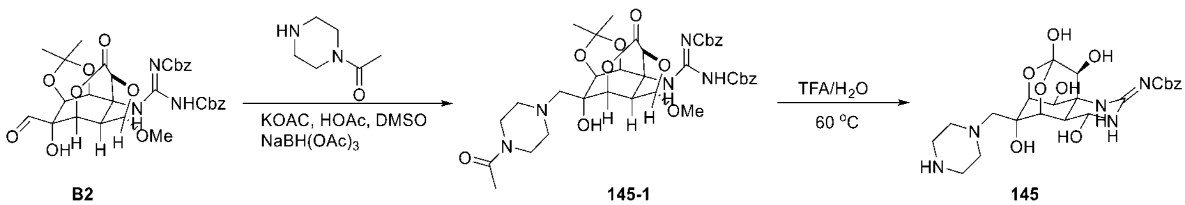


B2 145-1 145

Step 1: Synthesis of **Compound 145-1**

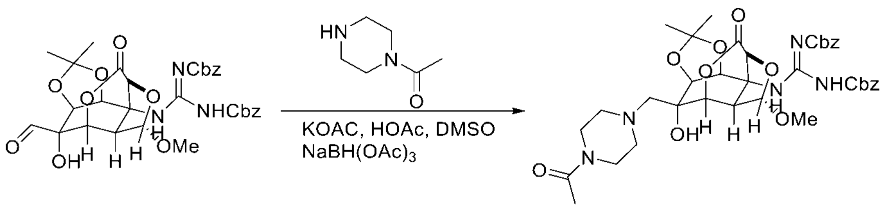


B2 145-1

**Compound B2** (160 mg, 0.25 mmol), compound 1-(piperazin-1-yl)ethanone (36.2 mg, 0.45 mmol), acetic acid (196 mg, 3.4 mmol), and potassium acetate (161.7 mg, 1.7 mmol) were dissolved in DMSO (4 mL), and heated to 60 °C under nitrogen protection and stirred for 1 h. Sodium triacetylborohydride (94.2 mg, 1.5 mmol) was added, and the reaction mixture was continued to be stirred for 1 h. After the reaction was completed, the mixture was cooled to room temperature, water (2 mL) was added to precipitate a white solid. The reaction mixture was stirred for 5 min, and filtered to give **Compound 145-1** as off-white solid (150 mg, yield: 80.2%). LCMS (m/z): 752.3[M+1]$^+$.

Step 2: Synthesis of **Compound 145**

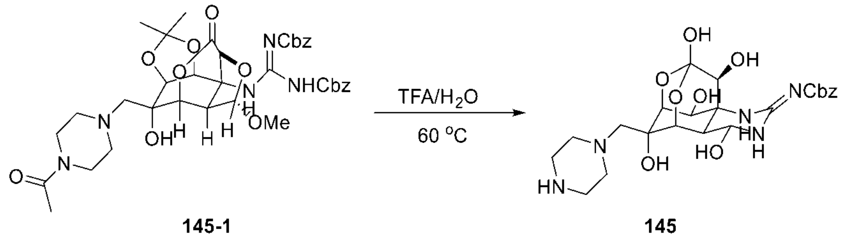


145-1 145

**Compound 145-1** (150 mg, 0.20 mmol) was dissolved in TFA: $H_2O$ = 2:1 aqueous solution (4 mL), and heated to 60 °C under nitrogen protection and stirred for 16 h. After the reaction was completed, the reaction solution was purified by reverse phase column chromatography (methanol/0.1% acetic acid aqueous solution = 5%-8%) to give **Compound 145** as white solid (18.6 mg, yield: 17.9%).

$^1H$ NMR (400 MHz, MeOD) $\delta$ 7.42-7.22(m, 5H), 5.54(s, 1H), 5.06(s, 2H), 4.56(s, 2H), 4.14(s, 2H), 3.91(s, 1H), 3.23-3.12(m, 4H), 3.04(s, 2H), 2.88(s, 4H); LCMS (m/z): 522.2[M+1]$^+$.

Example 118: Synthesis of benzyl ((4R,4aR,5R,6S,7S,9S,10S,10aR,11S,E)-6-(((1,3-dihydroxypropan-2-yl)amino)methyl)-4,6,9,10,11-pentahydroxyoctahydro-5,9-epoxy-7,10a-methanooxocino[4,5-d]pyrimidin-2(1H)-ylidene)carbamate (**Compound 146**)

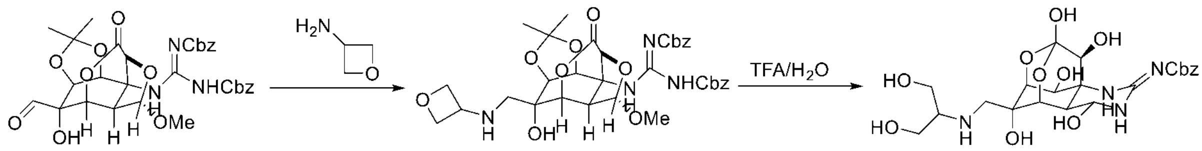


B2 146-1 146

Step 1: Synthesis of **Compound 146-1**

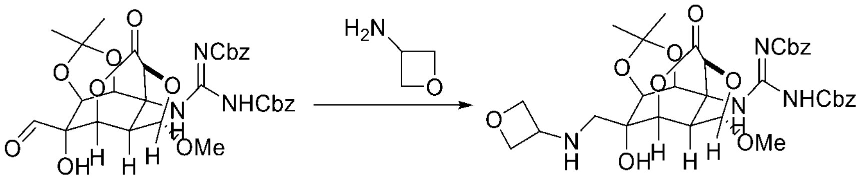

B2 146-1

**Compound B2** (150 mg, 0.23 mmol), 3-oxacyclobutylamine (25.73 mg, 0.35 mmol), and KOAc (132.4 mg, 1.35 mmol) were dissolved in anhydrous DMSO (2.5 mL), and then AcOH (166.3 mg, 2.77 mmol) was added dropwise to the mixed solution. The reaction solution was heated to 60 °C for 1 h. $NaBH_3CN$ (73.7 mg, 1.17 mmol) was added to the reaction solution in batches, and the reaction was continued at 60 °C for 1 h. HPLC was used to monitor the the reaction until it was completed. The reaction solution was diluted with water (25 mL), extracted with ethyl acetate (12 mL×3), the combined organic phases were washed with saturated brine (30 mL), dried over anhydrous sodium sulfate, filtered, and concentrated under reduced pressure to give a crude product. The crude product was purified by silica gel column chromatography (MeOH/DCM = 0-5%) to give **Compound 146-1** as white foam solid (149 mg, yield: 91.2%). LCMS (m/z): 697.3[M+1]$^+$.

Step 2: Synthesis of **Compound 146**

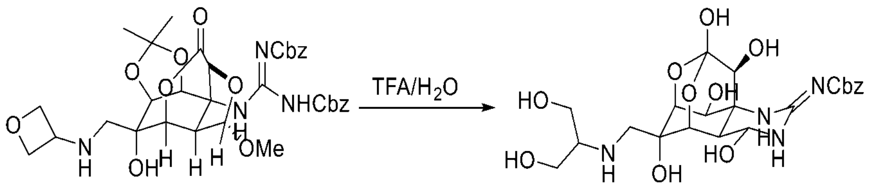


146-1 146

**Compound 146-1** (149 mg, 0.21 mmol) was dissolved in TFA (2 mL) and purified water (8 mL), and heated to 60 °C and stirred for 15 h. LCMS was used to monitor the reaction until it was completed. The reaction solution was concentrated under reduced pressure to give a crude product. The crude product was purified by Prep-HPLC (A: 0.1% AcOH, B: MeOH; 0-0.5%) to give **Compound 146** as white solid (22.85 mg, yield 20.3%).

$^1H$ NMR (400 MHz, MeOD) δ 7.43-7.35(m, 5H), 5.64(d, *J* = 9.3 Hz, 1H), 5.21(s, 2H), 4.26(d, *J* = 9.0 Hz, 2H), 4.15-3.96(m, 2H), 3.84(dd, *J* = 11.6, 4.6 Hz, 2H), 3.75(dd, *J* = 11.8, 6.3 Hz, 2H), 3.63(s, 2H), 3.27-3.22(m, 1H), 2.34(d, *J* = 9.4 Hz, 1H); LCMS (m/z): 527.2$[M+1]^+$.

Example 119: Synthesis of (4R,4aR,5R,6S,7S,9S,10S,10aR,11S)-6-(((1,3-dihydroxypropan-2-yl)amino)methyl)-2-iminooctahydro-5,9-epoxy-7,10a-methanooxocino[4,5-d]pyrimidine-4,6,9,10,11(10H)-pentaol (**Compound 147**)

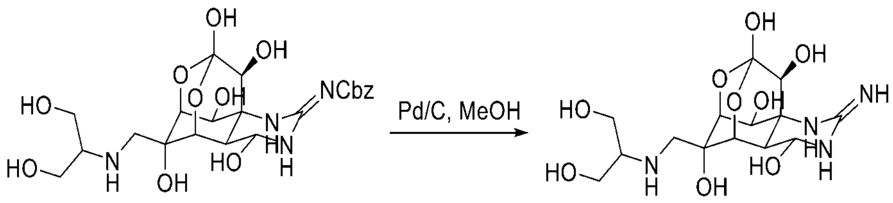


146 147

**Compound** 146 (17 mg, 29.01 μmol) was dissolved in MeOH (8 mL), to which was added 10% Pd/C (5 mg), and the reaction was carried out at room temperature for 2 h under a hydrogen atmosphere. HPLC was used to monitor the the reaction until it was completed. $H_2O$ (8 mL) was added to the reaction solution, which was stirred for 10 min. After filtration, the filter cake was rinsed with MeOH/$H_2O$ = 1:1 (20 mL). The filtrate was concentrated under reduced pressure and lyophilized to give **Compound 147** as white solid (7.39 mg, yield: 58.3%).

$^1H$ NMR (400 MHz, $D_2O$) δ 5.39(dd, *J* = 9.5, 3.1 Hz, 1H), 4.24(d, *J* = 1.8 Hz, 1H), 4.18(s, 1H), 4.13(s, 1H), 3.99(s, 1H), 3.76-3.67(m, 2H), 3.66-3.57(m, 2H), 3.43-3.28(m, 2H), 3.09-2.98(m, 1H), 2.27(d, *J* = 9.5 Hz, 1H). LCMS (m/z): 393.2 $[M+1]^+$.

Example 120: Synthesis of benzyl ((4R,4aR,5R,6S,7S,9S,10S,10aR,11S,E)-6-(aminomethyl)-4,6,9,10,11-pentahydroxyoctahydro-5,9-epoxy-7,10a-methanooxocino[4,5-d]pyrimidin-2(1H)-ylidene)carbamate (**Compound 148**)

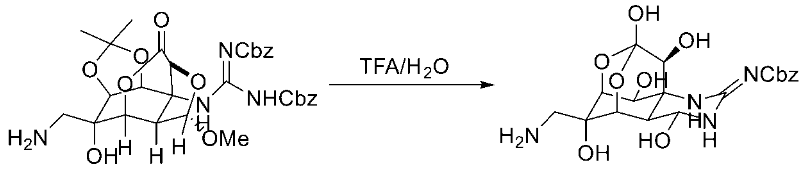


B3 148

**Compound B3** (120 mg, 0.19 mmol) was dissolved in TFA (2 mL) and $H_2O$ (2 mL), and heated to 60 °C for 48 h. HPLC was used to monitor complete reaction of the raw materials. The reaction solution was concentrated and dried, and the residue was purified by reverse phase column chromatography (MeOH/0.1% HOAc aqueous solution = 0-1.5%) to give **Compound 148** (25 mg, yield: 28.3%).

$^1H$ NMR (400 MHz, MeOD) δ 7.53-7.30(m, 5H), 5.73(d, *J* = 9.3 Hz, 1H), 5.32(s, 2H), 4.28(d, *J* = 10.8 Hz, 2H),

4.04(d, $J$ = 6.7 Hz, 2H), 3.62-3.52(m, 2H), 2.43(d, $J$ = 9.3 Hz, 1H); LCMS (m/z): 453.2[M+1]$^+$.

Example 121: Synthesis of benzyl ((4R,4aR,5R,6S,7S,9S,10S,10aR,11S,E)-6-(((3-fluorocyclobutyl)amino) methyl)-4,6,9,10,11-pentahydroxyoctahydro-5,9-epoxy-7,10a-methanooxocino[4,5-d]pyrimidin-2(1H)-ylidene)carbamate (**Compound 149**)

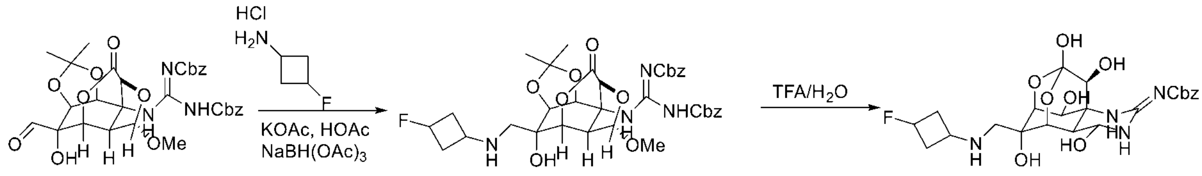


B2 149-1 149

Step 1: Synthesis of **Compound 149-1**

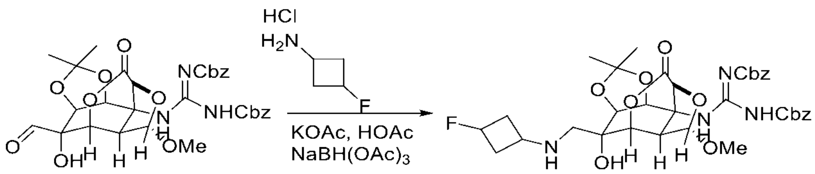


B2 149-1

**Compound B2** (200 mg, 0.31 mmol) was dissolved in dimethyl sulfoxide (4 mL), to which 3-fluorocyclobutan-1-amine hydrochloride (43.86 mg, 0.35 mmol), acetic acid (221 mg, 3.69 mmol) and potassium acetate (176.3 mg, 1.8 mmol) were added, and the reaction mixture was heated to 60°C for 1 h under nitrogen protection. Sodium cyanoborohydride (110.8 mg, 1.77 mmol) was added to the reaction solution in batches, and the reaction was carried out at 60°C for 30 min. HPLC was used to monitor complete reaction of the raw materials. The reaction solution was cooled to room temperature, to which ice water (20 mL) was added, and stirred to precipitate a large amount of solid. The filter cake was collected, concentrated and dried to give crude **Compound 149-1** (180 mg, yield: 80%), which was directly used in the subsequent step. LCMS (m/z): 713.3[M+1]$^+$.

Step 2: Synthesis of **Compound 149**

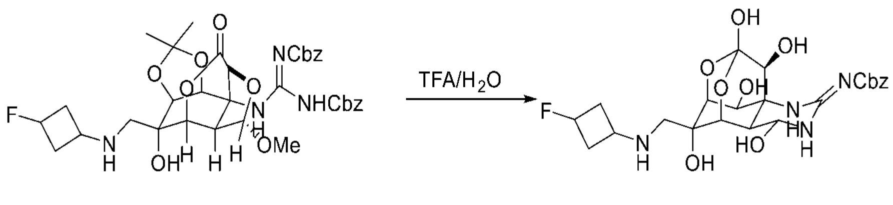


149-1 149

**Compound 149-1** (180 mg, 0.25 mmol) was dissolved in water (10 mL) and TFA (5 mL), and heated to 60°C under nitrogen protection for 20 h. HPLC was used to monitor complete reaction of the raw materials. The reaction solution was concentrated and dried, and the residue was purified by flash column chromatography (MeOH/DCM = 0-5%) to give **Compound 149** (30 mg, yield: 22.6%).

$^1$H NMR (400 MHz, MeOD) $\delta$ 7.49-7.26(m, 5H), 5.66(d, J = 9.3 Hz, 1H), 5.22(s, 2H), 4.25(s, 2H), 4.17-3.85(m, 3H), 3.52-3.37(m, 2H), 3.00-2.82(m, 2H), 2.73-2.50(m, 2H), 2.46-2.29(d, J = 9.4 Hz, 2H); LCMS (m/z): 525.2[M+1]+.

Example 122: Synthesis of (4R,4aR,5R,6S,7S,9S,10S,10aR,11S)-6-(((3-fluorocyclobutyl)amino)methyl)-2-iminooctahydro-5,9-epoxy-7,10a-methanooxocino[4,5-d]pyrimidine-4,6,9,10,11(10H)-pentaol (**Compound 150**)

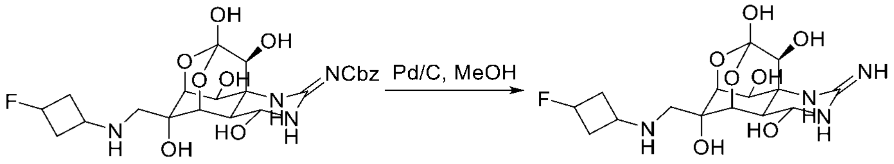


**149** **150**

**Compound 149** (20 mg, 0.04 mmol) was dissolved in methanol (10 mL), to which 10% Pd/C (20 mg) was added. The reaction mixture was purged with hydrogen, and hydrogen was introduced into the reaction solution for 3 h. HPLC was used to monitor complete reaction of the raw materials. The reaction solution was filtered, and the filter cake was rinsed with 0.05 M AcOH aqueous solution. The filtrate was concentrated to give a crude compound, which was slurried with methyl tert-butyl ether (10 mL), allowed to stand, and the supernatant was removed. The residue was concentrated and dried to give acetate of **Compound 150** (15 mg, yield: 100%).

$^1$H NMR (400 MHz, $D_2O$) δ 5.42(d, *J* = 9.4 Hz, 1H), 4.25(s, 2H), 4.17-4.00(m, 2H), 3.90(s, 1H), 3.61-3.38(m, 3H), 2.92-2.82(m, 1H), 2.71-2.57(m, 2H), 2.52-2.36(m, 1H), 2.29(d, *J* = 9.4 Hz, 1H); LCMS (m/z):391.2[M+1]$^+$.

Example 123: Synthesis of methyl (4R,4aR,5R,6S,7S,9S,10S,10aR,11S,E)-2-(((benzyloxy)carbonyl)imino)-4,6,9,10,11-pentahydroxydecahydro-5,9-epoxy-7,10a-methanooxocino[4,5-d]pyrimidine-6-carboxylate (**Compound 151**)

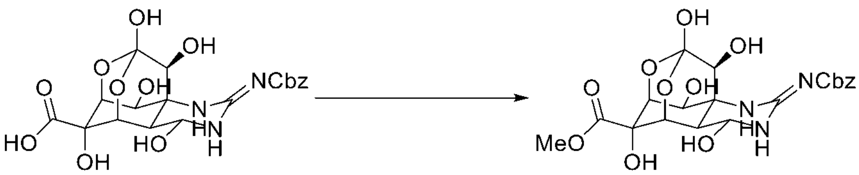

**67** **151**

**Compound 67** (44 mg, 0.09 mmol), 18-Crown-6 (25 mg, 0.09 mmol), methanol (150 mg, 4.7 mmol), HATU (53 mg, 0.14 mmol) and triethylamine (29 mg, 0.28 mmol) were dissolved in dichloromethane (2 mL) and stirred at room temperature for 17 h. After the reaction was completed, reaction mixture was directly concentrated to give a crude product, which was then purified by reverse phase column (methanol/0.1% acetic acid aqueous solution = 0-4%) to give **Compound 151** (4.2 mg, yield: 9.3%).

$^1$H NMR (400 MHz, MeOD) *δ* 7.44-7.19(m, 5H), 5.32(s, 1H), 5.07(s, 2H), 4.65(dd, *J* = 3.0, 1.7 Hz, 1H), 4.53(t, *J* = 1.9 Hz, 1H), 4.47-4.40(m, 2H), 3.81(s, 3H), 2.68(d, *J* = 2.8 Hz, 1H); LCMS (m/z): 482.1[M+1]$^+$.

Example 124: Synthesis of benzyl ((4R,4aR,5R,6S,7S,9S,10S,10aR,11S,E)-6-(3-fluoroazetidine-1-carbonyl)-4,6,9,10,11-pentahydroxyoctahydro-5,9-epoxy-7,10a-methanooxocino[4,5-d]pyrimidin-2(1H)-ylidene)carbamate (**Compound 152**)

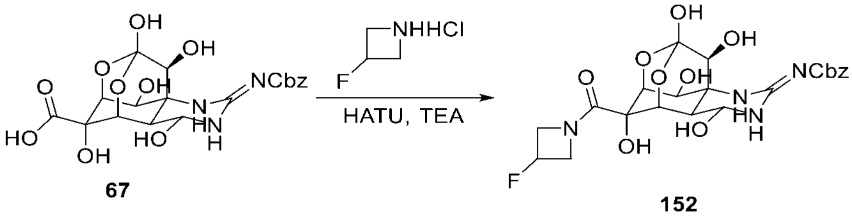


**67** **152**

**Compound 67** (32 mg, 0.07 mmol), 3-fluoroazetidine hydrochloride (7.6 mg, 0.07 mmol) and EDCI (20 mg, 0.11 mmol) were dissolved in pyridine (2 mL), and the reaction mixture was stirred for 1 h under nitrogen protection at 25 °C. After the reaction was completed, the reaction solution was purified by reverse phase column chromatography (methanol/5% acetic acid aqueous solution = 0%-10%) to give **Compound 152** as off-white solid (11.3 mg, yield: 31.4%).

$^1$H NMR (400 MHz, MeOD) $\delta$ 7.45-7.17(m, 5H), 5.57(d, *J* = 9.4 Hz, 1H), 5.48-5.26(m, 1H), 5.07(s, 2H), 4.57(s, 4H), 4.47-4.30(m, 2H), 4.27-3.83(m, 2H), 2.18(d, *J* = 7.3 Hz, 1H); LCMS (m/z): 525.2[M+1]$^+$.

Example 125: Synthesis of benzyl ((4S,5aS,6S,8R,9S,10S,11S,11aR,12R,E)-9-(3-fluoroazetidine-1-carbonyl)-6,9,11-trihydroxyoctahydro-4,8,11a-(epimethanetriyl)-6,10-epoxyoxocino[4,3-f][1,3,5]oxadiazepin-2(1H)-ylidene)carbamate (**Compound 153**)

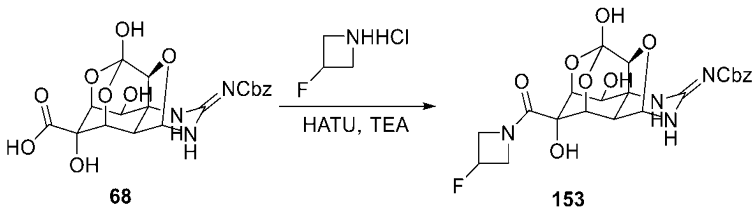


68 153

**Compound** 68 (25 mg, 0.06 mmol), 3-fluoroazetidine hydrochloride (7.6 mg, 0.07 mmol) and EDCI (20 mg, 0.11 mmol) were dissolved in pyridine (2 mL), and the reaction mixture was stirred for 1 h under nitrogen protection. After the reaction was completed, the reaction solution was purified by reverse phase column chromatography (methanol/5% acetic acid aqueous solution = 0%-10%) to give **Compound 153** as off-white solid (10.5 mg, yield: 37.3%).

$^1$H NMR (400 MHz, MeOD) $\delta$ 7.44-7.22(m, 5H), 5.46-5.39(m, 0.5H), 5.32(s, 1H), 5.31-5.26(m, 0.5H), 5.07(s, 2H), 4.58(s, 4H), 4.45(d, *J* = 3.1 Hz, 1H), 4.43(d, *J* = 2.0 Hz, 1H), 4.39-4.27(m, 1H), 4.22-3.97(m, 1H), 2.70(d, *J* = 2.9 Hz, 1H); LCMS (m/z): 507.1[M+1]$^+$.

Example 126: Synthesis of (3-fluoroazetidin-1-yl)((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methanone (**Compound 154**)

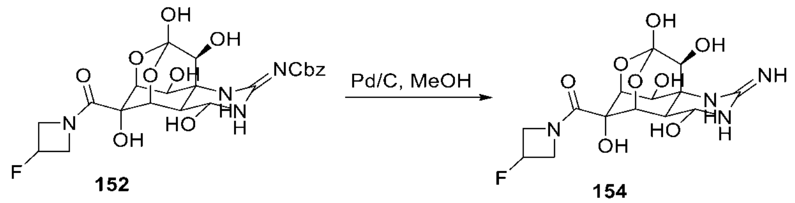


152 154

**Compound 152** (7.0 mg, 0.01 mmol) was dissolved in methanol (2 mL), to which Pd/C (1 mg) was added. The reaction mixture was purged with hydrogen three times, and the reaction mixture was stirred under 25 °C for 3 h. After the reaction was completed, 1% acetic acid aqueous solution was added, and the reaction mixture was stirred for 5 min, filtered, washed three times with 1% acetic acid aqueous solution. The filtrate was concentrated, dried and lyophilized to give **Compound 154** as off-white solid (3.4 mg, yield: 65.3%).

$^1$H NMR (400 MHz, $D_2O$) $\delta$ 5.48-5.24(m, 2H), 4.65-4.45(m, 3H), 4.42-4.25(m, 2H), 4.22-4.07(m, 2H), 4.03-3.86(m, 1H), 2.25(dd, J= 24.1, 9.8 Hz, 1H); LCMS (m/z): 391.1[M+1]$^+$.

Example 127: Synthesis of benzyl ((4R,4aR,5R,6S,7S,9S,10S,10aR,11S,E)-6-((3-fluorocyclobutyl)carbamoyl)-4,6,9,10,11-pentahydroxyoctahydro-5,9-epoxy-7,10a-methanooxocino[4,5-d]pyrimidin-2(1H)-ylidene)carbamate (**Compound 155**)

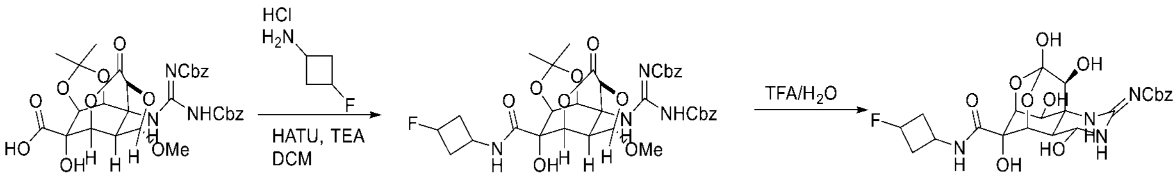


B1 155-1 155

Step 1: Synthesis of **Compound 155-1**

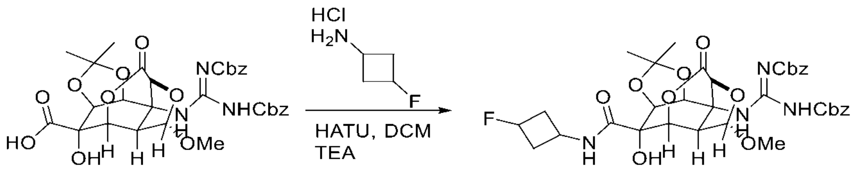


B1 155-1

**Compound B1** (150 mg, 0.23 mmol) was dissolved in DCM (10 mL), then to which 3-fluorocyclobutan-1-amine hydrochloride (43 mg, 0.34 mmol) and HATU (130 mg, 0.34 mmol) were added. TEA (69.4 mg, 0.69 mmol) was added dropwise, and the reaction was carried out overnight at room temperature under nitrogen protection. The reaction solution was poured into water (50 mL) and extracted with DCM (30 mL×3). The organic phases were combined, washed with saturated brine (50 mL), dried over anhydrous sodium sulfate, and concentrated to give a residue. The residue was purified by flash column chromatography (EA/PE = 0-40%) to give **Compound 155-1** (100 mg, yield: 60%). LCMS (m/z): 727.3 $[M+1]^+$.

Step 2: Synthesis of **Compound 155**

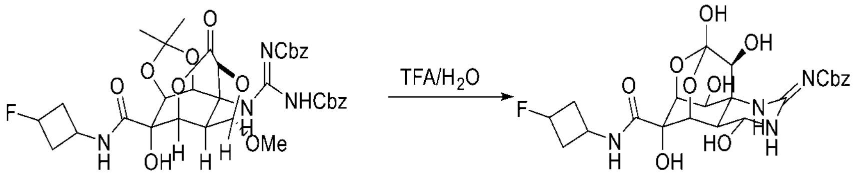


155-1 155

**Compound 155-1** (120 mg, 0.17 mmol) was dissolved in water (10 mL) and TFA (5 mL), and heated to 60°C under nitrogen protection for 20 h. The reaction solution was concentrated and dried, and the residue was purified by reverse phase column chromatography ($MeOH/H_2O$ = 0-15%) to give **Compound 155** (20 mg, yield: 22.5%).

$^1H$ NMR (400 MHz, MeOD) $\delta$ 7.57-7.12(m, 5H), 5.58(d, $J$ = 9.3 Hz, 1H), 5.09(s, 2H), 4.8-4.68(m, 1H), 4.64-4.47(m, 1H), 4.38(s, 1H), 4.30-4.14(m, 2H), 4.11-3.86(m, 1H), 2.87-2.75(m, 1H), 2.65-2.40(m, 2H), 2.38-2.19(m, 2H); LCMS (m/z): 539.2$[M+1]^+$.

Example 128: Synthesis of (4R,4aR,5R,6S,7S,9S,10S,10aR,11S)-N-(3-fluorocyclobutyl)-4,6,9,10,11-pentahydroxy-2-iminodecahydro-5,9-epoxy-7,10a-methanooxocino[4,5-d]pyrimidine-6-carboxamide (**Compound 156**)

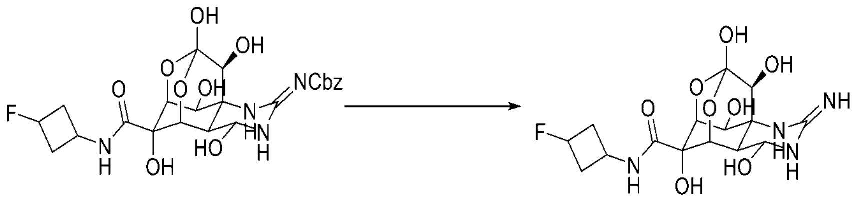

155 156

**Compound 155** (20 mg, 0.04 mmol) was dissolved in methanol (10 mL), to which 10% Pd/C (20 mg) was added. The reaction mixture was purged with hydrogen, and hydrogen was introduced into the reaction solution for 3 h. HPLC was used to monitor complete reaction of the raw materials. The reaction solution was filtered, and the filter cake was rinsed with 0.05 M AcOH aqueous solution. The filtrate was concentrated to give a crude compound, which was slurried with methyl tert-butyl ether (10 mL), allowed to stand, and the supernatant was removed. The residue was concentrated and dried to give acetate of **Compound 156** (15 mg, yield: 100%).

$^1$H NMR (400 MHz, $D_2O$) $\delta$ 5.42(d, $J$ = 9.5 Hz, 1H), 4.52(s, 1H), 4.41(s, 1H), 4.33(s, 1H), 4.21(s, 1H), 3.90(s, 1H), 3.87-3.73(m, 1H), 2.85-2.71(m, 1H), 2.65-2.49(m, 1H), 2.4-2.33(m, 1H), 2.31-2.12(m, 2H), 1.99(s, 3H); LCMS (m/z): 405.1[M+1]$^+$.

Example 129: Synthesis of benzyl ((4R,4aR,5R,6S,7S,9S,10S,10aR,11S,E)-4,6,9,10,11-pentahydroxy-6-((3-hydroxycyclobutyl)carbamoyl)octahydro-5,9-epoxy-7,10a-methanooxocino[4,5-d]pyrimidin-2(1H)-ylidene)carbamate (**Compound 157**)

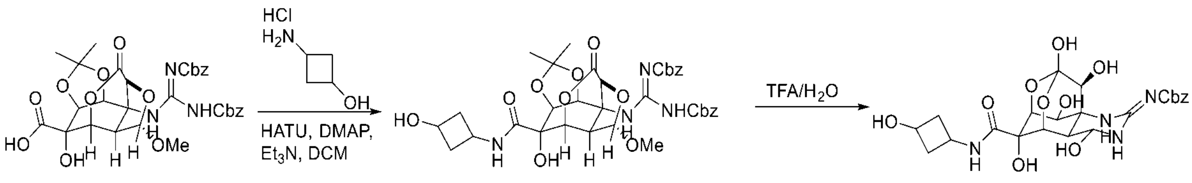


B1 157-1 157

Step 1: Synthesis of **Compound 157-1**

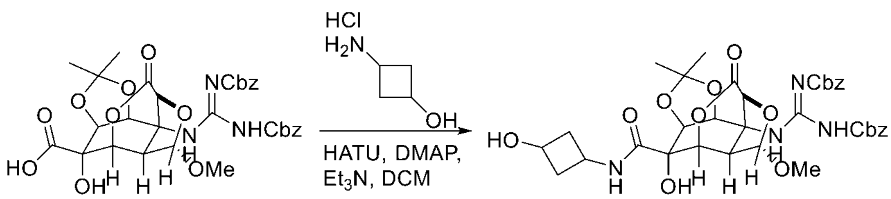


B1 157-1

**Compound B1** (200 mg, 0.30 mmol) and 3-hydroxycyclobutylamine hydrochloride (68 mg, 0.56 mmol) were dissolved in anhydrous DCM (10 mL), triethylamine (92.1 mg, 0.91 mmol), DMAP (3.7 mg, 0.03 mmol) and HATU (173.3 mg, 0.46 mmol) were added to the mixed solution, and the reaction was carried out at room temperature for 3 h.

LC-MS was used to monitor the reaction until it was completed. The organic phase was diluted with dichloromethane (40 mL), washed with saturated sodium chloride (20 mL×2), dried over anhydrous sodium sulfate, and concentrated to give a residue. The residue was purified by flash column chromatography (methanol/dichloromethane = 0-8%) to give **Compound 157-1** as white solid (120 mg, yield: 54.3%). LCMS (m/z): 725.3[M+1]$^+$.

Step 2: Synthesis of **Compound 157**

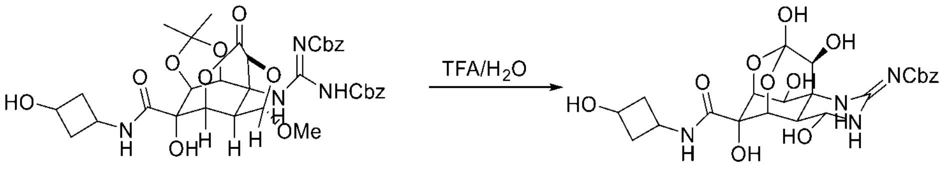


157-1 157

**Compound 157-1** (120 mg, 0.17 mmol) was dissolved in TFA (5 mL) and purified water (10 mL), and heated to 60 °C for 18 h. MS was used to monitor the reaction until it was completed. The reaction solution was concentrated under reduced pressure to give a crude product. The crude product was purified by prep-HPLC (A: 0.1% AcOH, B: MeOH; 0-8%) to give **Compound 157** as white solid (13 mg, yield: 25.9%).

$^1H$ NMR (400 MHz, MeOD) $\delta$ 7.45-7.37(m, 5H), 5.71(d, $J$ = 9.4 Hz, 1H), 5.30(s, 2H), 4.48-4.38(m, 3H), 4.24(d, $J$ = 8.4 Hz, 2H), 4.02(s, 1H), 2.48-2.24(m, 5H); LCMS (m/z): 537.2[M+1]$^+$.

Example 130: Synthesis of (4R,4aR,5R,6S,7S,9S,10S,10aR,11S)-4,6,9,10,11-pentahydroxy-N-(3-hydroxycyclobutyl)-2-iminodecahydro-5,9-epoxy-7,10a-methanooxocino[4,5-d]pyrimidine-6-carboxamide (**Compound 158**)

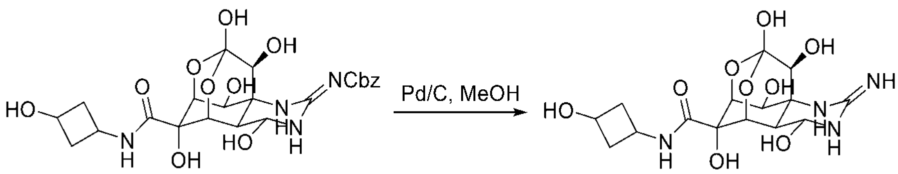


157 158

**Compound** 157 (17 mg, 0.03 mmol) and 10% palladium on carbon (2 mg) were added to methanol (10 mL). The reaction mixture was purged hydrogen three times, and the reaction was carried out at room temperature for 2 h. HPLC was used to monitor the reaction until it was completed. The reaction solution was concentrated under reduced pressure and lyophilized to give **Compound 158** as white solid (10 mg, yield: 78.4%).

$^1H$ NMR (400 MHz, $D_2O$) $\delta$ 5.45(d, $J$ = 9.4 Hz, 1H), 4.55(s, 1H), 4.48-4.40(m, 1H), 4.39-4.28(m, 2H), 4.24(s, 1H), 3.93(s, 1H), 2.46-2.11(m, 5H); LCMS (m/z): 403.4[M+1]$^+$.

Example 131: Synthesis of benzyl ((4R,4aR,5R,6S,7S,9S,10S,10aR,11S,E)-6-((2,2-difluoroethyl)carbamoyl)-4,6,9,10,11-pentahydroxyoctahydro-5,9-epoxy-7,10a-methanooxocino[4,5-d]pyrimidin-2(1H)-ylidene)carbamate (**Compound 159**)

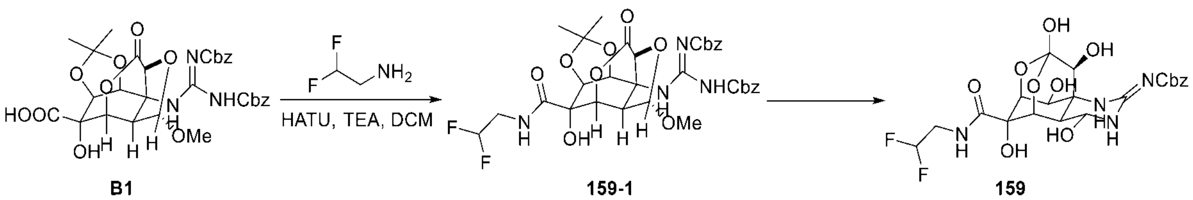


B1 159-1 159

Step 1: Synthesis of **Compound 159-1**

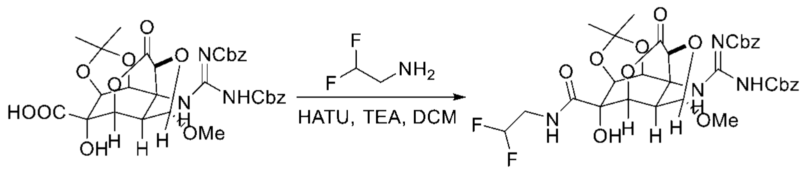


B1 159-1

**Compound B1** (150 mg, 0.23 mmol), 2,2-difluoroethylamine (28 mg, 0.345 mmol), HATU (132 mg, 0.345 mmol) and triethylamine (70 mg, 0.69 mmol) were dissolved in dichloromethane (3 mL) and stirred at 25°C for 17 h. After the reaction was completed, water was added and the reaction mixture was extracted with dichloromethane (10 mL×3). The organic phases were combined, washed with saturated brine once, separated, the organic phase was dried over anhydrous sodium sulfate, and filtered. The filtrate was concentrated and dried, and then purified by normal phase column chromatography (ethyl acetate/petroleum ether = 0-32%) to give **Compound 159-1** (161 mg, yield: 98%). LCMS (m/z): 719.2[M+1]$^+$.

Step 2: Synthesis of **Compound 159**

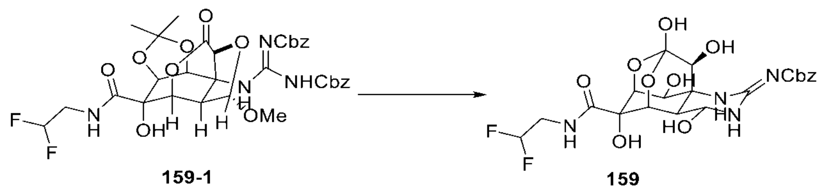

159-1 159

**Compound 159-1** (161 mg, 0.22 mmol) was added to water: trifluoroacetic acid = 1:1 (16 mL), and the reaction mixture was stirred at 65°C for 17 h. After the reaction was completed, the reaction mixture was directly concentrated to give a crude product, which was directly concentrated and then purified by reverse phase column (methanol/0.1% acetic acid aqueous solution = 0-7%) to give **Compound 159** (14 mg, yield: 11.8%).

$^1$H NMR (400 MHz, MeOD) $\delta$ 7.46-7.25(m, 5H), 5.99(tt, J= 56.2, 4.1 Hz, 1H), 5.62(d, *J* = 9.4 Hz, 1H), 5.14(s, 2H), 4.43(s, 1H), 4.24(s, 2H), 4.11-3.97(m, 1H), 3.71(td, *J* = 14.7, 4.1 Hz, 2H), 2.33(d, *J* = 9.5 Hz, 1H); LCMS (m/z): 531.2[M+1]$^+$.

Example 132: Synthesis of (4R,4aR,5R,6S,7S,9S,10S,10aR,11S)-N-(2,2-difluoroethyl)-4,6,9,10,11-pentahydroxy-2-iminodecahydro-5,9-epoxy-7,10a-methanooxocino[4,5-d]pyrimidine-6-carboxamide (**Compound 160**)

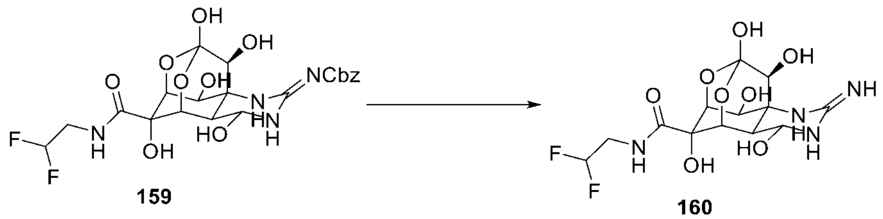

159 160

**Compound 159** (6 mg, 0.01 mmol) and 10% Pd/C (3 mg) were added to methanol (5 mL). The reaction mixture was purged with hydrogen and stirred at 25°C for 4 h under a hydrogen atmosphere. After the reaction was completed, 1% acetic acid aqueous solution was added to the reaction mixture which was stirred for 0.5 h, then filtered. The filtrate was collected, and concentrated under reduced pressure to give **Compound 160** (2.1 mg, yield: 46.9%).

$^1$H NMR (400 MHz, $D_2O$) $\delta$ 6.15-5.80(m, 1H), 5.45(d, *J* = 9.4 Hz, 1H), 4.56(s, 1H), 4.36(s, 1H), 4.25(s, 1H), 4.05-3.85(m, 1H), 3.77-3.62(m, 2H), 2.31(d, *J* = 9.4 Hz, 1H); LCMS (m/z): 397.1[M+1]$^+$.

Example 133: Synthesis of benzyl ((4R,4aR,5R,6S,7S,9S,10S,10aR,11S,E)-6-(cyclopropanecarboxamidomethyl)-4,6,9,10,11-pentahydroxyoctahydro-5,9-epoxy-7,10a-methanooxocino[4,5-d]pyrimidin-2(1H)-ylidene)carbamate (**Compound 161**)

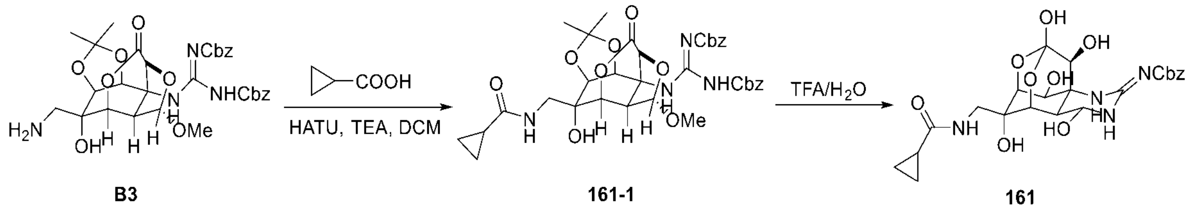


B3 161-1 161

Step 1: Synthesis of **Compound 161-1**

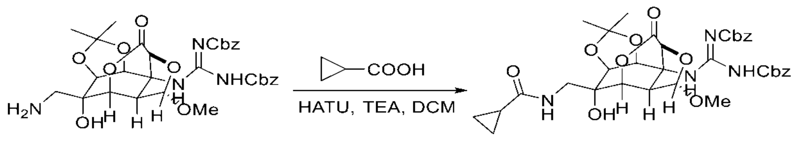


B3 161-1

In a 25 mL single-necked flask, **Compound B3** (140 mg, 0.22 mmol) and cyclopropionic acid (22.6 mg, 0.26 mmol) were dissolved in anhydrous dichloromethane (5 mL). The reaction solution was cooled to 0 °C, to which HATU (100 mg, 0.26 mmol) and triethylamine (55.5 mg, 0.55 mmol) were added. The reaction mixture was stirred for 30 min under nitrogen protection, and then monitored by HPLC until most of the raw materials were consumed. Water (10 mL) was added to the reaction solution, which was extracted with dichloromethane (15 mL×3). The organic phases were combined, dried over anhydrous sodium sulfate, filtered, and the filtrate was concentrated and dried to give a crude product. The crude product was purified by normal phase column (dichloromethane/methanol = 20:1) to give **Compound 161-1** as white solid (150 mg, yield: 97.3%). LCMS (m/z): 709.3$[M+1]^+$.

Step 2: Synthesis of **Compound 161**

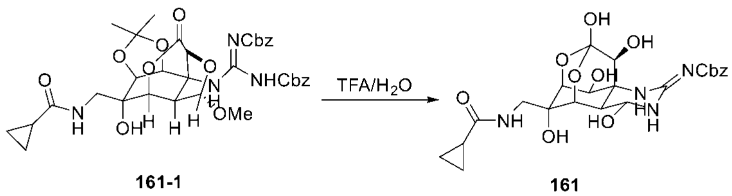


161-1 161

In a 25 mL reaction flask, **Compound 161-1** (140 mg, 0.20 mmol) was dissolved in trifluoroacetic acid: water = 2:1(5 mL) and heated to 60 °C overnight. HPLC was used to monitor complete reaction of the raw materials. The reaction solution was concentrated and dried to give a crude product. The crude product was purified by prep-HPLC to give **Compound 161** (24.5 mg, yield: 23.5%).

$^1H$ NMR (400 MHz, MeOD) δ 7.49-7.31(m, 5H), 5.62(d, *J* = 9.4 Hz, 1H), 5.13(s, 2H), 4.35-3.84(m, 6H), 2.28(d, J= 9.7 Hz, 1H), 1.79-1.72(m, 1H), 0.99-0.83(m, 4H); LCMS (m/z): 521.2$[M+1]^+$.

Example 134: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)cyclopropanecarboxamide (**Compound 162**)

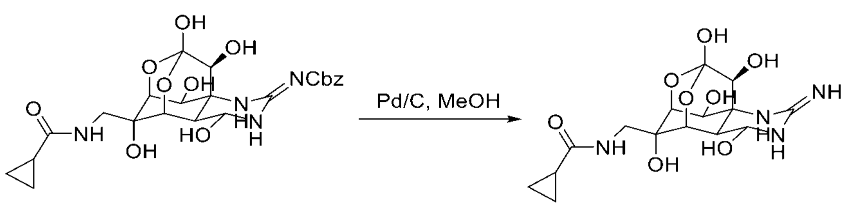


161 162

In a 25 mL single-necked flask, **Compound 161** (10.0 mg, 0.02 mmol) was dissolved in methanol (2 mL), and 10% palladium on carbon (2 mg) was added. The reaction mixture was purged with hydrogen three times, and the hydrogenation reaction was carried out by using a hydrogen balloon under pressure for 3 h. HPLC was used to monitor complete reaction of the raw materials. The reaction solution was filtered, and the filter cake was collected, to which deionized water (10 mL) and 2.5% acetic acid aqueous solution (5 mL) were added, stirred at room temperature for 1 h, and filtered. This operation is repeated once. All filtrates were combined, concentrated and dried, and washed twice with methanol to give a crude product. Two drops of methanol were added to the crude product, all the products were dissolved, then methyl tert-butyl ether (2 mL) was added. The reaction mixture was stirred for 5 min, allowed to stand, and the supernatant was removed. Then methyl tert-butyl ether (2 mL) was added again, the reaction mixture was stirred for 5 min, allowed to stand, and the supernatant was removed. The residue was concentrated and dried to give **Compound 162** as white solid (5.2 mg, yield: 69.4%).

$^1$H NMR (400 MHz, $D_2O$) $\delta$ 5.47(d, $J$ = 9.4 Hz, 1H), 4.43(s, 1H), 4.25(d, $J$ = 9.0 Hz, 1H), 4.17(s, 1H), 4.00(s, 1H), 3.95(d, $J$ = 7.6 Hz, 1H), 3.84(s, 1H), 2.31(d, $J$ = 9.4 Hz, 1H), 1.71-1.63(m, 1H), 0.85(d, $J$ = 6.2 Hz, 4H); LCMS (m/z): 387.1 $[M+1]^+$.

Example 135: Synthesis of benzyl ((4R,4aR,5R,6S,7S,9S,10S,10aR,11S,E)-6-(acetamidomethyl)-4,6,9,10,11-pentahydroxyoctahydro-5,9-epoxy-7,10a-methanooxocino[4,5-d]pyrimidin-2(1H)-ylidene)carbamate (**Compound 163**)

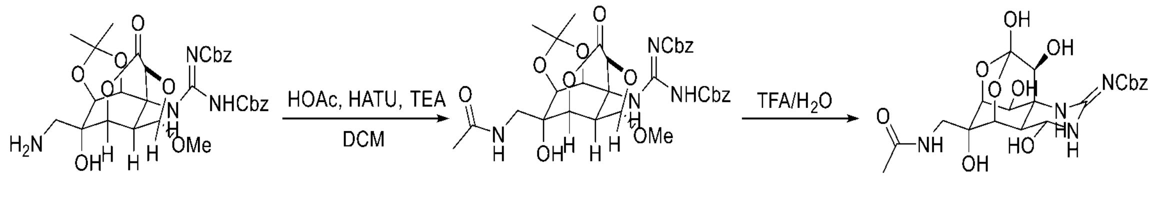


B3 163-1 163

Step 1: Synthesis of **Compound 163-1**

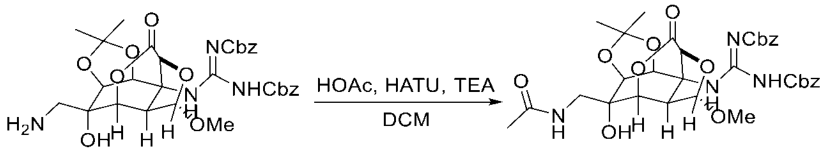


B3 163-1

In a 25 mL single-necked flask, **Compound B3** (150 mg, 0.23 mmol) and acetic acid (16.8 mg, 0.28 mmol) were dissolved in anhydrous dichloromethane (5 mL). The reaction mixture was cooled to 0°C. HATU (106 mg, 0.28 mmol) and triethylamine (59 mg, 0.59 mmol) were added to the reaction solution, and the reaction mixture was stirred for 30 min under nitrogen protection. HPLC was used to detect the complete reaction of most raw materials. Water (10 mL) was added to the reaction solution, which was extracted with dichloromethane (15 mL×3). The organic phases were combined, dried over anhydrous sodium sulfate, filtered, and the filtrate was concentrated and dried to give a crude product. The crude product was purified by normal phase column chromatography (dichloromethane/methanol = 20:1) to give **Compound 163-1** as white solid (135 mg, yield: 85.1%). LCMS (m/z): 683.3$[M+1]^+$.

Step 2: Synthesis of **Compound 163**

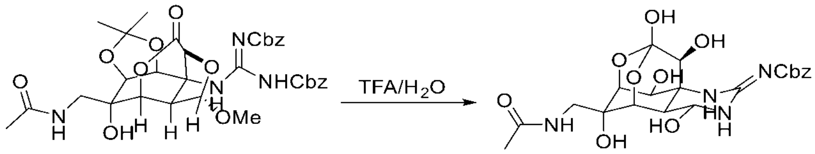


**163-1** **163**

In a 25 mL reaction flask, **Compound 163-1** (135 mg, 0.20 mmol) was dissolved in trifluoroacetic acid: water = 2:1(5 mL), heated to 60 °C, and the reaction was carried out overnight. HPLC was used to monitor complete reaction of the raw materials. The reaction solution was concentrated and dried to give a crude product. The crude product was purified by prep-HPLC to give **Compound 163** (24.5 mg, yield: 23.5%).

$^1H$ NMR (400 MHz, MeOD) $\delta$ 7.41-7.26(m, 5H), 5.56(d, $J$ = 9.3 Hz, 1H), 5.08(s, 2H), 4.30-3.75(m, 6H), 2.22(d, $J$ = 9.3 Hz, 1H), 1.96(s, 3H); LCMS (m/z): 495.2[M+1]$^+$.

Example 136: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)acetamide (**Compound 164**)

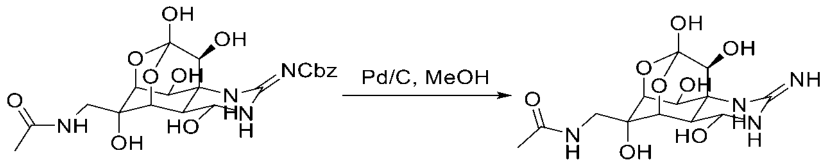


**163** **164**

In a 25 mL single-necked flask, **Compound 163** (7.1 mg, 0.01 mmol) was dissolved in methanol (2 mL). 10% palladium on carbon (1 mg) was added, and the reaction mixture was purged with hydrogen three times. The hydrogenation reaction was carried out by using a hydrogen balloon under pressure for 3 h. HPLC was used to monitor complete reaction of the raw materials. The reaction solution was filtered, and the filter cake was collected, to which deionized water (10 mL) and 2.5% acetic acid aqueous solution (5 mL) were added, stirred at room temperature for 1 h, and filtered. The above procedures were repeated once. All filtrates were combined, concentrated and dried, and washed twice with methanol to give a crude product. Two drops of methanol were added to the crude product, all the products were dissolved, then methyl tert-butyl ether (2 mL) was added. The reaction mixture was stirred for 5 min, allowed to stand, and the supernatant was removed. Then methyl tert-butyl ether (2 mL) was added again, the reaction mixture was stirred for 5 min, allowed to stand, and the supernatant was removed. The residue was concentrated and dried to give **Compound 164** as white solid (4.1 mg, yield: 82.7%).

$^1H$ NMR (400 MHz, $D_2O$) $\delta$ 5.52(dd, $J$ = 8.8, 3.6 Hz, 1H), 4.47(s, 1H), 4.22(s, 1H), 4.06-3.96(m, 2H), 3.92-3.83(m, 2H), 2.36(d, $J$ = 9.5 Hz, 1H), 2.08(s, 3H); LCMS (m/z):361.1[M+1]$^+$.

Example 137: Synthesis of N-((4R,4aR,5R,6S,7S,9S,10S,10aR,11S,Z)-4,6,9,10,11-pentahydroxy-6-(hydroxymethyl) octahydro-5,9-epoxy-7,10a-methanooxocino[4,5-d]pyrimidin-2(1H)-ylidene)acetamide (**Compound 165**) and N-((4S,5aS,6S,8R,9S,10S,11S,11aR,12R,Z)-6,9,11-trihydroxy-9-(hydroxymethyl)octahydro-4,8,11a-(epimethanetriyl)-6,10-epoxyoxocino[4,3-f][1,3,5]oxadiazepin-2(1H)-ylidene)acetamide (**Compound 166**)

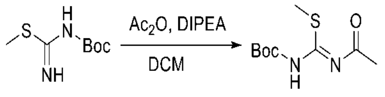


165-1 165-2

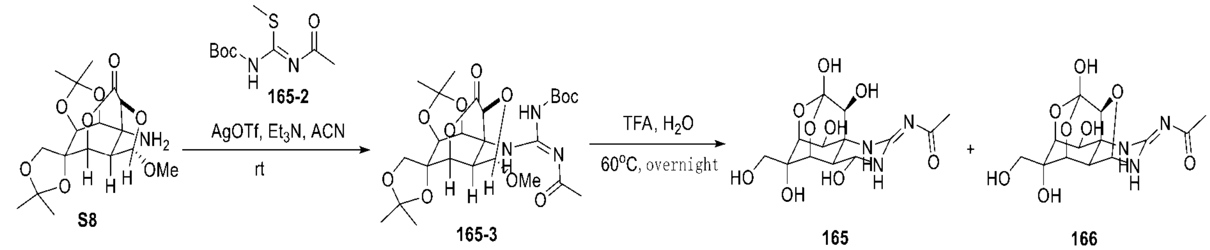


S8 165-3 165 166

Step 1: Synthesis of **Compound 165-2**

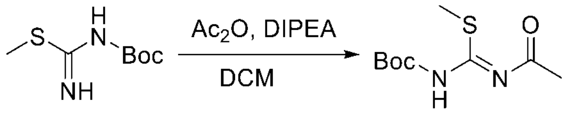


165-1 165-2

**Compound 165-1** (1 g, 5.26 mmol) was dissolved in DCM (3 mL), to which was added diisopropylethylamine (0.7 g, 5.43 mmol). Under nitrogen protection, the reaction mixture was cooled to 0 °C, to which was added acetic anhydride (0.54 g, 5.32 mmol) dropwise, and the reaction was carried out at room temperature for 3 h. The reaction solution was poured into water (100 mL) and extracted with DCM (30 mL×3). The organic phases were combined, washed with saturated brine (50 mL), dried over anhydrous sodium sulfate, and concentrated to give a crude product. The crude product was purified by flash column chromatography (EA/PE = 0-30%) to give **Compound 165-2** as colorless oily liquid (1.1 g, yield: 90.2%).

HRMS (ESI$^+$): $C_9H_{16}N_2O_3S$([M+H]$^+$): calculated: 233.09, measured: 232.30

Step 2: Synthesis of **Compound 165-3**

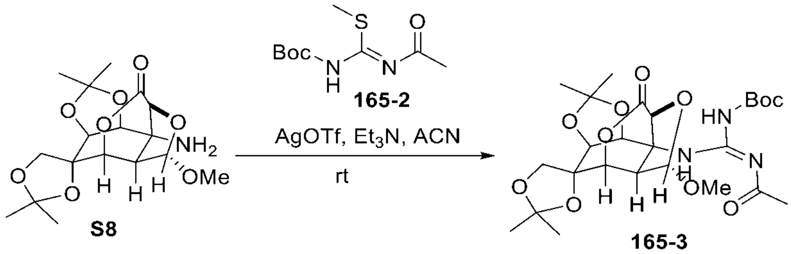


S8 165-3

**Compound S8** (200 mg, 0.54 mmol) was dissolved in acetonitrile (10 mL), to which **Compound 165-2** (150 mg, 0.65 mmol) and triethylamine (272 mg, 2.7 mmol) were added. The reaction mixture was cooled to 0 °C under nitrogen protection, and silver trifluoromethanesulfonate (277.5 mg, 1.08 mmol) was added. After the addition was completed, the temperature was slowly raised to room temperature and the reaction was carried out overnight. The reaction solution was filtered and the filtrate was concentrated to give a residue. The residue was purified by flash column chromatography (EA/PE = 0-30%) to give **Compound 165-3** as white foam solid (225 mg, yield: 75.2%).

HRMS (ESI +): $C_{25}H_{37}N_3O_{11}$ ([M+H]$^+$): calculated: 556.24, measured: 556.58.

Step 3: Synthesis of **Compound 165** and **Compound 166**

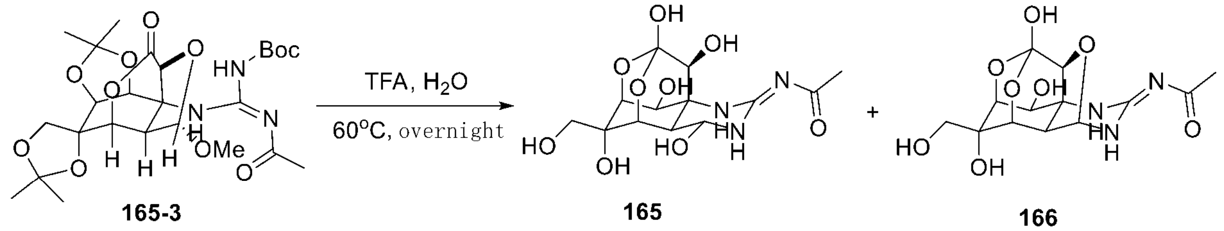


165-3 165 166

**Compound 165-3** (220 mg, 0.40 mmol) was dissolved in water (10 mL) and TFA (10 mL), heated to 60°C under nitrogen protection, and the reaction was carried out for 18 h. HPLC was used to monitor complete reaction of the raw materials. The reaction solution was concentrated and dried, and the residue was purified by Prep-HPLC to give **Compound 165** formate (22 mg, yield: 15%) and **Compound 166** formate (12 mg, yield: 8.2%), total yield: 23.2%.

**Compound 165:**

$^1H$ NMR (400 MHz, MeOD) $\delta$ 8.49(s, 1H), 5.66(d, $J$ = 8.7 Hz, 1H), 4.28-4.12(m, 2H), 4.06-3.92(m, 4H), 2.34(d, $J$ = 9.6 Hz, 1H), 2.16(s, 3H); LCMS (m/z): 362.1[M+1]$^+$.

**Compound 166:**

$^1H$ NMR (400 MHz, MeOD) $\delta$ 8.42(s, 1H), 5.39(s, 1H), 4.52(d, $J$ = 1.9 Hz, 1H), 4.50(s, 1H), 4.21(s, 1H), 4.04(s, 1H), 3.97(d, $J$ = 11.4 Hz, 1H), 3.91(d, $J$ = 11.4 Hz, 1H), 2.79(d, $J$ = 2.8 Hz, 1H), 2.12(s, 3H); LCMS (m/z): 344.1[M+1]$^+$.

Example 138: Synthesis of cyclopentyl ((4R,4aR,5R,6S,7S,9S,10S,10aR,11S,Z)-4,6,9,10,11-pentahydroxy-6-(hydroxymethyl)octahydro-5,9-epoxy-7,10a-methanooxocino[4,5-d]pyrimidin-2(1H)-ylidene)carbamate (**Compound 167**)

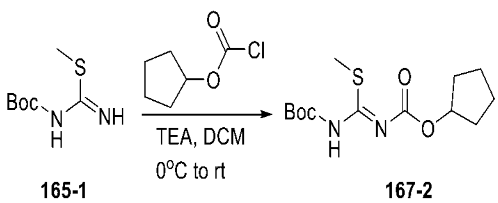


165-1 167-2

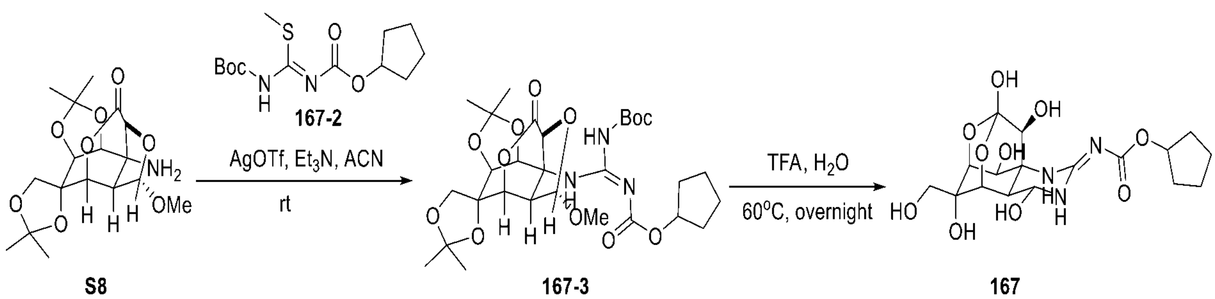


S8 167-3 167

Step 1: Synthesis of **Compound 167-2**

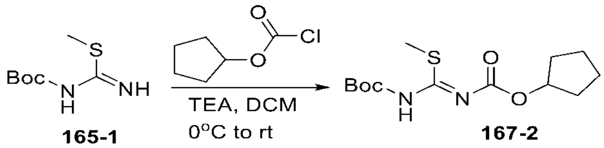


165-1 167-2

**Compound 165-1** (0.25 g, 1.68 mmol) and DIPEA (0.24 g, 1.85 mmol) were dissolved in anhydrous dichloromethane (2.5 mL). The reaction mixture was cooled to 0 °C under nitrogen protection, and cyclopentyl chloroformate (0.26 g, 1.35 mmol) was added. The reaction solution was subjected to reaction at 0 °C for 30 min, and then stirred at room temperature for 2.5 h. The reaction solution was diluted with dichloromethane (50 mL), washed with saturated sodium chloride (20 mL×3). The organic phase was dried over anhydrous sodium sulfate, filtered, and concentrated to give a residue. The residue was purified by flash column chromatography (ethyl acetate/petroleum ether = 0-20%) to give **Compound 167-2** as white solid (0.30 g, yield: 56.7%). LCMS (m/z): 303.1[M+1]+.

Step 2: Synthesis of **Compound 167-3**

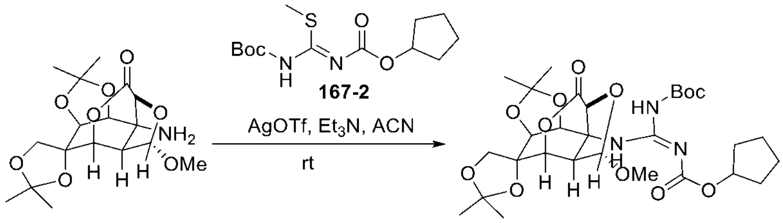


S8 167-3

**Compound S8** (0.20 g, 0.54 mmol), **Compound 167-2** (0.18 g, 0.59 mmol), triethylamine (0.24 g, 1.85 mmol) and silver trifluoroacetate (0.28 g, 1.08 mmol) were dissolved in anhydrous acetonitrile (2.0 mL), and the reaction mixture was stirred at room temperature for 16 h under nitrogen protection. The reaction solution was quenched with saturated sodium chloride (1mL), diluted with ethyl acetate (30 mL), and washed with saturated sodium chloride (2×10 mL). The organic phase was dried over anhydrous sodium sulfate, filtered, and concentrated to give a residue. The residue was purified by flash column chromatography (ethyl acetate/petroleum ether = 0%-20%) to give **Compound 167-3** as white solid (0.35 g, yield: > 100%). LCMS (m/z): 626.3[M+1]+.

Step 3: Synthesis of **Compound 167**

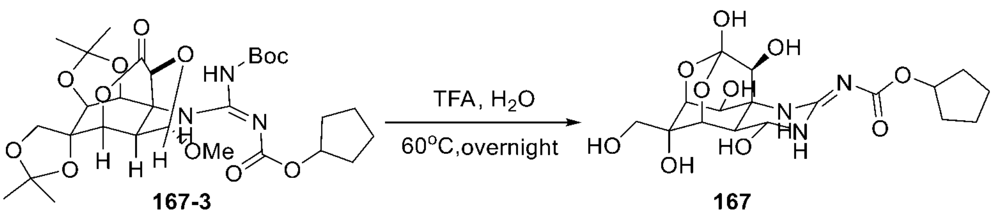


167-3 167

**Compound 167-3** (0.15 g, 0.25 mmol) was dissolved in a mixed solution of trifluoroacetic acid (0.5 mL) and purified water (0.5 mL), and the reaction mixture was heated to 60 °C under nitrogen protection and stirred for 18 h. The reaction solution was concentrated to give a residue. The residue was purified by reverse phase preparation to give **Compound 167** as white solid (31 mg, yield: 30%).

$^1H$ NMR (400 MHz, MeOD) $\delta$ 5.66(d, $J$ = 9.4 Hz, 1H), 5.23-5.17(m, 1H), 4.22(d, $J$ = 20.6 Hz, 2H), 4.01(d, $J$ = 22.7 Hz, 4H), 2.36(d, $J$ = 9.4 Hz, 1H), 1.96-1.89(m, 2H), 1.83-1.76(m, 4H), 1.71-1.63(m, 2H); LCMS (m/z): 432.2[M+1]+.

Example 139: Synthesis of benzyl ((4R,4aR,5R,6S,7S,9S,10S,10aR,11S,E)-6-cyano-4,6,9,10,11-pentahydroxyoctahydro-5,9-epoxy-7,10a-methanooxocino[4,5-d]pyrimidin-2(1H)-ylidene)carbamate (**Compound 168**) and benzyl ((4S,5aS,6S,8R,9S,10S,11S,11aR,12R,E)-9-cyano-6,9,11-trihydroxyoctahydro-4,8,11a-(epimethanetriyl)-6,10-epoxyoxocino[4,3-f][1,3,5]oxadiazepin-2(1H)-ylidene)carbamate (**Compound 169**)

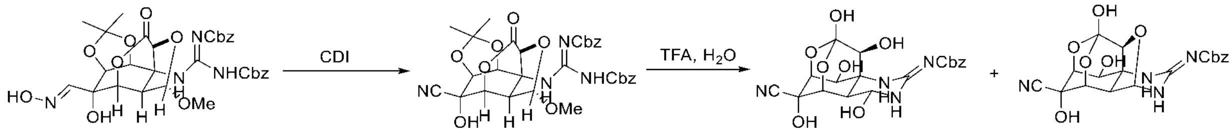


**B2-1** **168-2** **168** **169**

Step 1: Synthesis of **Compound 168-2**

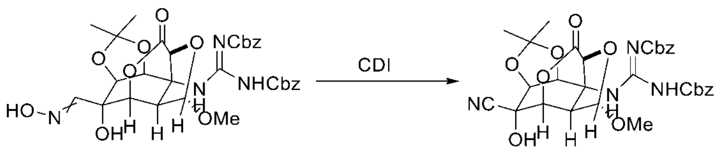


**B2-1** **168-2**

**Compound B2-1** (210 mg, 0.32 mmol) was dissolved in acetonitrile (4 mL) and tetrahydrofuran (2 mL), to which N,N'-carbonyldiimidazole (CDI, 182.2 mg, 1.12 mmol) was added. The reaction mixture was stirred overnight at room temperature under nitrogen protection. The reaction solution was poured into ice water (50 mL), extracted with ethyl acetate (30 mL×3). The organic phases were combined, washed with saturated sodium chloride solution (50 mL), dried over anhydrous sodium sulfate, and concentrated to give crude **Compound 168-**2 (220 mg, yield: 108%), which was directly used in the subsequent reaction. LCMS (m/z): 637.2[M+1]+.

Step 2: Synthesis of **Compound 168** and **Compound 169**

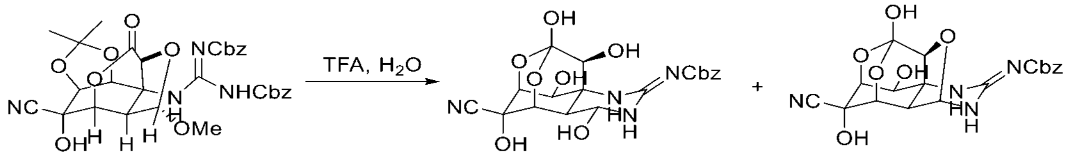


**168-2** **168** **169**

**Compound 168-2** (220 mg, 0.35 mmol) was dissolved in (10 mL) and TFA (10 mL) and heated to 60°C under nitrogen atmosphere and stirred overnight. HPLC was used to monitor complete reaction of the raw materials. The reaction solution was concentrated and dried to give a residue. The residue was purified by flash column chromatography (MeOH/DCM = 0-5%) to give Compound 168 (79 mg, yield: 51%) and **Compound 169** (25 mg, yield: 16.12%).

**Compound 168**:

$^1$H NMR (400 MHz, MeOD) $\delta$ 7.41-7.25(m, 5H), 5.58(d, *J* = 9.3 Hz, 1H), 5.10(s, 2H), 4.64-4.34(m, 1H), 4.21(s, 1H), 4.11-3.82(m, 2H), 2.16(d, *J* = 8.0 Hz, 1H); LCMS (m/z): 449.1[M+1]+.

**Compound** 169:

$^1$H NMR (400 MHz, MeOD) $\delta$ 7.49-7.11(m, 5H), 5.36(s, 1H), 5.09(s, 2H), 4.52-4.46(m, 2H), 4.41(d, *J* = 2.1 Hz, 1H), 4.34(s, 1H), 2.71(d, J= 2.9 Hz, 1H); LCMS (m/z): 431.1[M+1]+.

Example 140: Synthesis of benzyl ((4R,4aR,5R,6S,7S,9S,10S,10aR,11S,E)-4,6,9,10,11-pentahydroxy-6-(((5-methyl-1,2,4-oxadiazol-3-yl)amino)methyl)octahydro-5,9-epoxy-7,10a-methanooxocino[4,5-d]pyrimidin-2(1H)-ylidene) carbamate (**Compound 170**), benzyl ((4R,4aR,5R,6S,7S,9S,10S,10aR,11S,E)-4,6,9,10,11-pentahydroxy-6-(ureidomethyl)octahydro-5,9-epoxy-7,10a-methanooxocino[4,5-d]pyrimidin-2(1H)-ylidene)carbamate (**Compound 171**) and 1-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)urea (**Compound 172**)

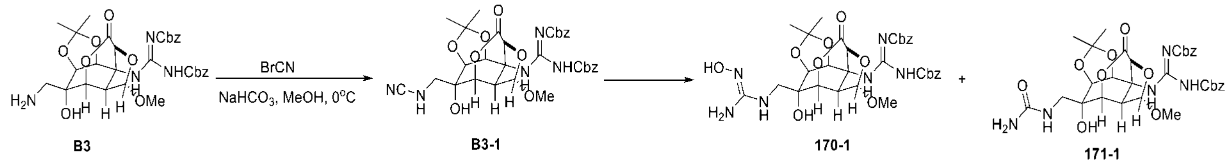


B3 B3-1 170-1 + 171-1

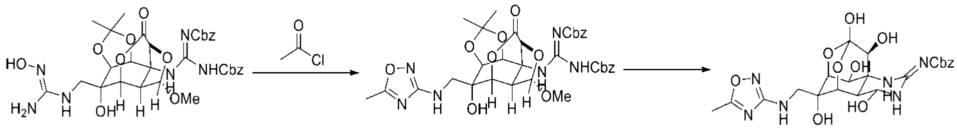

170-1 170-2 170

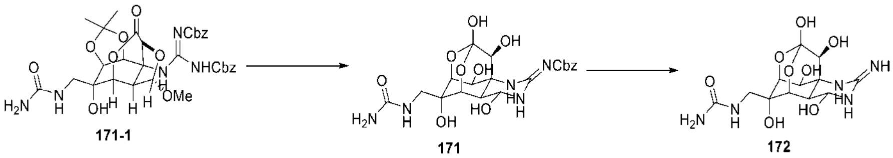

171-1 171 172

Step 1: Synthesis of **Compound B3-1**

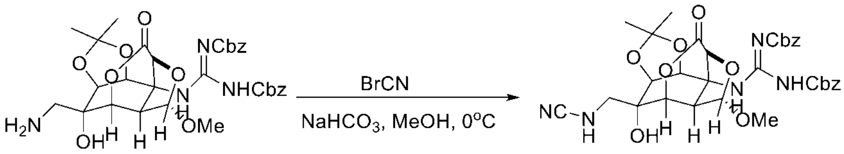


B3 B3-1

**Compound B3** (100 mg, 0.16 mmol) and $NaHCO_3$ (26 mg, 0.31 mmol) were dissolved in methanol (6 mL). The reaction mixture was cooled to 0 °C in an ice bath, and BrCN (18 mg, 0.17 mmol) was added for reaction. After the reaction was completed, water was added to the reaction solution, which was extracted with dichloromethane. The organic phase was concentrated, and purified by column chromatography to give a **Compound B3-1** as white solid (70 mg, yield: 67.4%).

Step 2: Synthesis of **Compound 170-1** and **Compound 171-1**

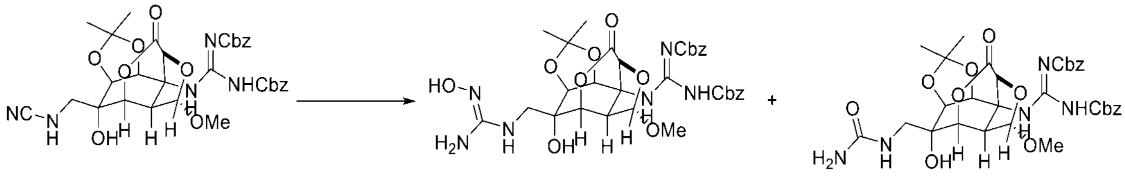

B3-1 170-1 171-1

**Compound B3-1** (240 mg, 0.36 mmol) and potassium carbonate (149 mg, 1.08 mmol) were added to ethanol (12 mL), then hydroxylamine hydrochloride (30.1 mg, 0.43 mmol) was added, and the reaction mixture was stirred at room temperature. After the reaction was completed, water (40 mL) was added to the system, which was extracted with dichloromethane (3×20 mL). The organic phases were combined, dried over anhydrous sodium sulfate, and concentrated to give a crude product. The crude product was subjected to flash column chromatography to give **Compound 170-1** as white solid (105 mg, yield: 41.7%) and **Compound 171-1** as colorless oil (50 mg, yield: 20.3%).

Step 3: Synthesis of **Compound 170-2**

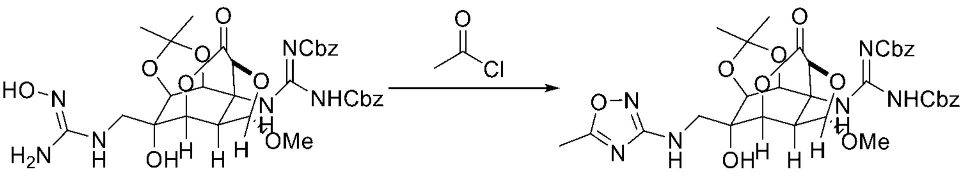

170-1 170-2

**Compound** 170-1 (95 mg, 0.14 mmol) was dissolved in DCM (5 mL), the temperature was controlled at 0 °C in an ice bath. Acetyl chloride (13.5 mg, 0.171 mmol) was added to the reaction solution, and the reaction mixture was stirred at room temperature for 1 h. Then 1,2 dichloroethane (15 mL) was added, and the reaction mixture was heated to 80 °C, and stirred for 2 h. After the raw materials were completely consumed, the system was concentrated and dried to give a crude product. The crude product was purified by column chromatography (ethyl acetate: petroleum ether = 0-50%) to give **Compound** 170-2 (70 mg, yield: 71.2%).

Step 4: Synthesis of Compound 170

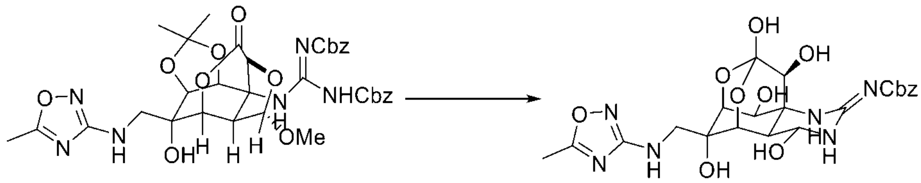

170-2 170

**Compound 170-2** (70 mg, 0.10 mmol) was dissolved in a mixed solution of water (3.2 mL) and TFA (0.8 mL), and the reaction mixture was stirred at 60 °C for 16 h. Then the reaction solution was concentrated and dried to give a crude product. The crude product was purified by wet sample loading reverse phase column chromatography and lyophilized to give **Compound 170** as white solid (16 mg, yield: 30.9%), LCMS (m/z): 535.2[M+1]$^+$.

Step 5: Synthesis of **Compound 171**

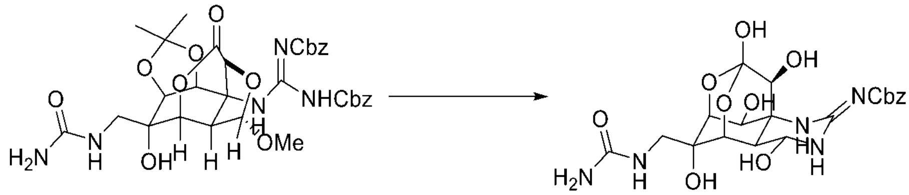

171-1 171

**Compound 171-1** (49 mg, 0.07 mmol) was dissolved in a mixed solution of water (4 mL) and TFA (1 mL), and the reaction mixture was stirred at 60 °C for 16 h. Then the reaction system was concentrated and dried to give a crude product,

which was purified by wet sample loading reverse phase column chromatography and lyophilized to give **Compound 171** as white solid (14 mg, yield: 39.4%).

$^1$H NMR (400MHz, MeOD) $\delta$ 7.39(dt, J = 19.2, 6.5Hz, 5H), 5.66(d,J = 9.4Hz, 1H), 5.24(d,J = 12.5 Hz, 2H), 4.19(d,J = 15.5Hz, 1H), 4.12(d,J = 11.9Hz, 1H), 3.98-3.85(m, 2H), 3.74(s, 2H), 2.35(d, J= 9.4Hz, 1H); LCMS (m/z):496.2[M+1]$^+$.

Step 6: Synthesis of **Compound 172**

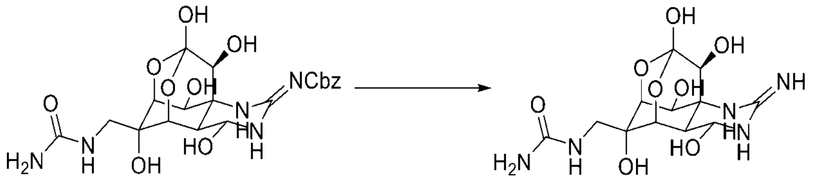

**171** **172**

**Compound 171** (11 mg, 0.02 mmol) was dissolved in methanol (3 mL), to which 10% palladium on carbon (4 mg) was added. The reaction mixture was purged with hydrogen, and the reaction was carried out by using a hydrogen balloon under pressure with stirring for 3 h. After the reaction was completed, the mixture was filtered, and the filtrate was concentrated to give **Compound 172** as white solid (7 mg, yield: 87.3%).

$^1$H NMR (400 MHz, $D_2O$) $\delta$ 5.40(d, *J* = 9.5 Hz, 1H), 4.27-4.05(m, 2H), 3.90(d, *J* = 26.2 Hz, 2H), 3.65(s, 2H), 2.24(d, *J* = 9.4 Hz, 1H); LCMS (m/z):362.1[M+1]$^+$.

Example 141: Synthesis of benzyl ((4R,4aR,5R,6S,7S,9S,10S,10aR,11S,E)-4,6,9,10,11-pentahydroxy-6-(((E)-2-hydroxyguanidino)methyl)octahydro-5,9-epoxy-7,10a-methanooxocino[4,5-d]pyrimidin-2(1H)-ylidene)carbamate (**Compound 173**)

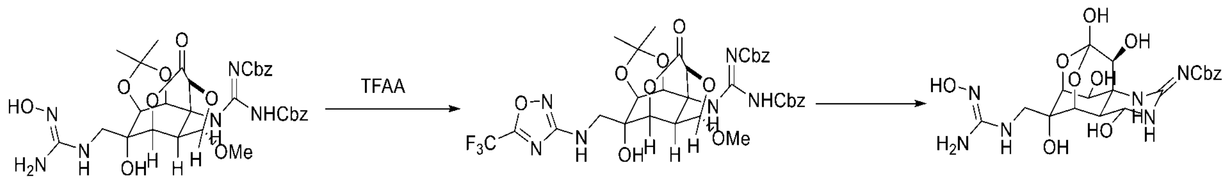


**170-1** **173-1** **173**

Step 1: Synthesis of **Compound 173-1**

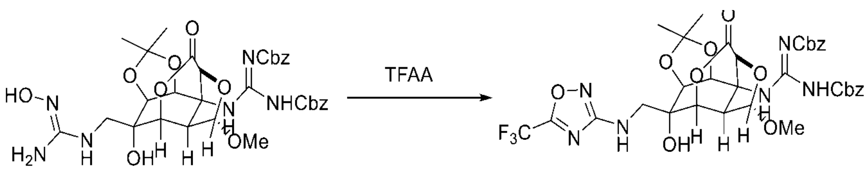


**170-1** **173-1**

**Compound 170-1** (95 mg, 0.14 mmol) was dissolved in dichloromethane (5 mL), the temperature was controlled at 0 °C in an ice bath, and trifluoroacetic anhydride (36 mg, 0.17 mmol) was added to the reaction solution. The reaction mixture was stirred at room temperature for 1 h. Then 1,2 dichloroethane (15 mL) was added, and the reaction mixture was heated to 80 °C and stirred for 2 h. The system was concentrated after complete reaction of the raw materials to give a crude product, which was purified by column chromatography (ethyl acetate: petroleum ether = 0-35%), and concentrated to give crude **Compound 173-1** (50 mg, yield: 47.3%). LCMS (m/z):777.2[M+1]$^+$.

Step 2: Synthesis of **Compound 173**

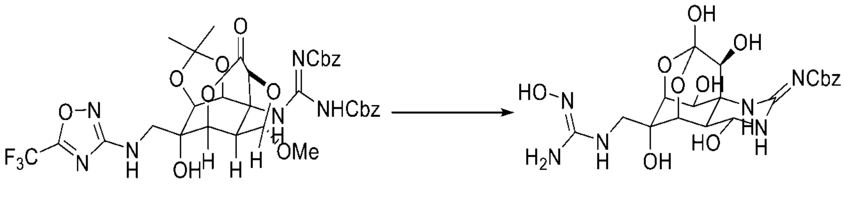

**173-1** **173**

**Compound 173-1** (50 mg, 0.06 mmol) was dissolved in a mixed solution of water (2.4 mL) and TFA (0.6 mL), and the reaction mixture was stirred at 60 °C for 16 h. Then the reaction system was rotary dried to give a crude product, which was purified by wet sample loading reverse phase column chromatography and lyophilized to give **Compound 173** as white solid (7 mg, yield: 21.3%).

$^1$H NMR (400 MHz, MeOD) $\delta$ 7.38-7.25(m, 5H), 5.52(dd, $J$ = 17.6, 8.1 Hz, 1H), 5.06(s, 2H), 4.17(s, 1H), 4.10(d, $J$ = 9.8 Hz, 2H), 3.96(s, 1H), 3.76(d, J = 14.8 Hz, 2H), 2.22(d, J= 9.4 Hz, 1H); LCMS (m/z): 511.2[M+1]$^+$.

Example 142: (4S,5aS,6S,8R,9S,10S,11S,11aR,12R)-9-((3-hydroxyazetidin-1-yl)methyl)-2-iminooctahydro-4,8,11a-(epimethanetriyl)-6,10-epoxyoxocino[4,3-f][1,3,5]oxadiazepine-6,9,11(5aH)-triol (**Compound 175**)

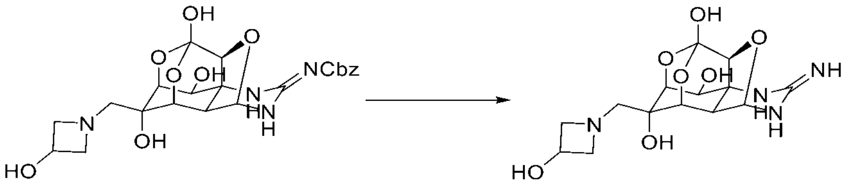

**174** **175**

According to the synthesis procedures of Example 2, **Compound 175** acetate was obtained as white solid (4.8 mg, yield: 66%).

$^1$H NMR (400 MHz, $D_2O$) $\delta$ 5.49(s, 1H), 4.60-4.52(m, 4H), 4.33-4.28(m, 1H), 4.20-4.08(s, 4H), 3.88(d, $J$ = 14.1 Hz, 1H), 3.76(d, $J$ = 14.1 Hz, 1H), 2.95(d, $J$ = 2.8 Hz, 1H), 1.91(s, 3H); LCMS (m/z): 357.1[M+1]$^+$.

Example 143: Synthesis of benzyl ((4R,4aR,5R,6S,7S,9S,10S,10aR,11S,E)-6-(benzylcarbamoyl)-4,6,9,10,11-pentahydroxyoctahydro-5,9-epoxy-7,10a-methanooxocino[4,5-d]pyrimidin-2(1H)-ylidene)carbamate (**Compound 176**) and benzyl ((4S,5aS,6S,8R,9S,10S,11S,11aR,12R,E)-9-(benzylcarbamoyl)-6,9,11-trihydroxyoctahydro-4,8,11a-(epimethanetriyl)-6,10-epoxyoxocino[4,3-f][1,3,5]oxadiazepin-2(1H)-ylidene)carbamate (**Compound 177**)

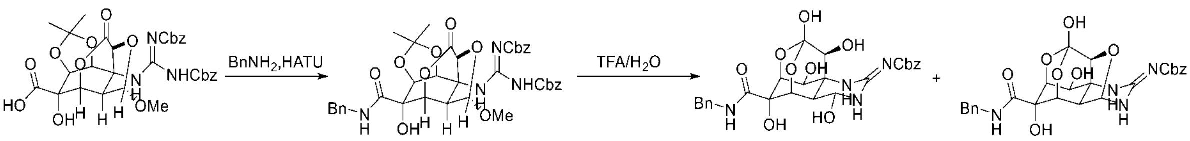


**B1** **176-1** **176** **177**

Step 1: Synthesis of **Compound 176-1**

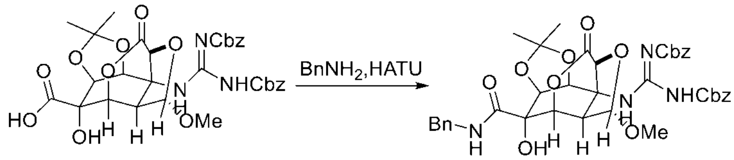


B1 176-1

**Compound B1** (50 mg, 0.08 mmol) and benzylamine (9.5 mg, 0.09 mmol) were dissolved in dichloromethane (4 mL), then to which HATU (36 mg, 0.09 mmol) and triethylamine (24 mg, 0.24 mmol) were added, and the reaction mixture was stirred at room temperature for 16 h. After the raw materials were completely consumed, water (10 mL) was added to the reaction solution, which was extracted with dichloromethane (10 mL×3). The organic phases were combined, dried over anhydrous sodium sulfate, filtered, and the filtrate was concentrated and dried to give a crude product. The crude product was purified by column chromatography (ethyl acetate/petroleum ether = 0-40%) to give **Compound 176-1** as white solid (60 mg, yield > 100%). LCMS (m/z): 745.3[M+1]$^+$.

Step 2: Synthesis of **Compound 176** and **Compound 177**

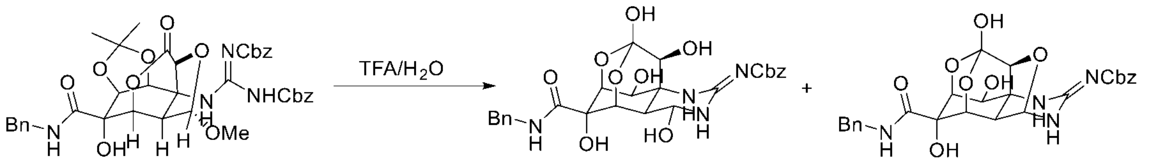


176-1 176 177

**Compound** 176-1 (60 mg, 0.08 mmol) was dissolved in a mixed solution of water (1 mL) and trifluoroacetic acid (2 mL), the reaction system was heated to 60 °C and stirred for 16 h. The reaction solution was concentrated and dried to give a crude product. The crude product was purified by high performance liquid phase to give **Compound 176** as white solid (12 mg, yield: 28.3%) and **Compound 177** as white solid (6 mg, yield: 14.1%). Total yield: 42.4%.

**Compound 176**: $^1$H NMR (400 MHz, MeOD) δ 7.39-7.21(m, 10H), 5.56(d, J = 9.4 Hz, 1H), 5.05(s, 2H), 4.54(d, J = 3.1 Hz, 2H), 4.37(s, 1H), 4.21(s, 2H), 4.08(d, J = 14.2 Hz, 1H), 2.29(d, J = 9.4 Hz, 1H); LCMS (m/z): 557.2[M+1]$^+$.

**Compound 177**: $^1$H NMR (400 MHz, MeOD) δ 7.43-7.20(m, 10H), 5.30(s, 1H), 5.07(s, 2H), 4.54(s, 2H), 4.50(d, J = 2.0 Hz, 1H), 4.47(s, 1H), 4.34(s, 1H), 4.21(s, 1H), 2.77(d, J = 2.8 Hz, 1H); LCMS (m/z): 539.2[M+1]$^+$.

Example 144: benzyl ((4R,4aR,5R,6S,7S,9S,10S,10aR,11S,E)-6-(((4,5-dihydro-1H-imidazol-2-yl)amino)methyl)-4,6,9,10,11-pentahydroxyoctahydro-5,9-epoxy-7,10a-methanooxocino[4,5-d]pyrimidin-2(1H)-ylidene)carbamate (**Compound 178**)

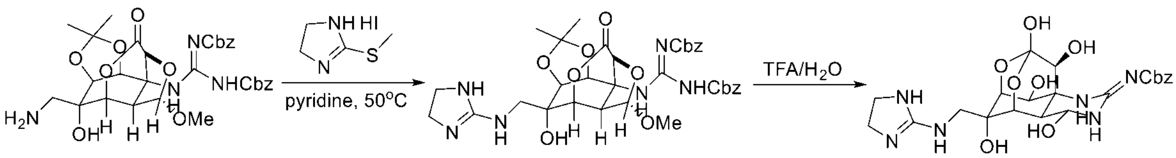


B3 178-1 178

Step 1: Synthesis of **Compound 178-1**

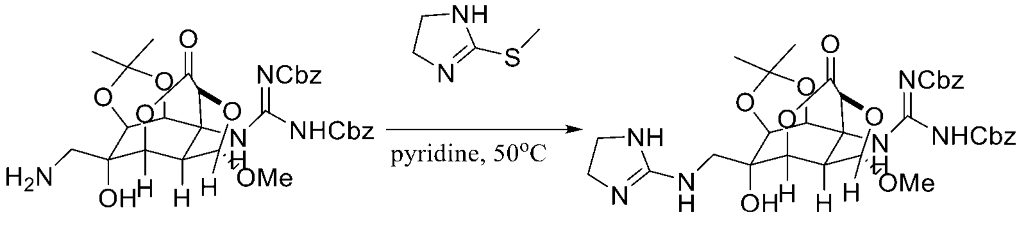


B3 178-1

**Compound B3** (80 mg, 0.12 mmol) and compound 2-(methylthio)-4,5-dihydro-1H-imidazole (36.5 mg, 0.15 mmol) were dissolved in pyridine (2 mL), and the reaction mixture was heated to 50 °C under nitrogen protection and stirred for 16 h. After the reaction was completed, the reaction solution was concentrated and dried to give a residue. The residue was purified by reverse phase column chromatography (methanol/5% acetic acid aqueous solution = 5%-20%) to give **Compound 178-1** as off-white solid (60 mg, yield: 67.8%). LCMS (m/z): 709.3[M+1]+.

Step 2: Synthesis of **Compound 178**

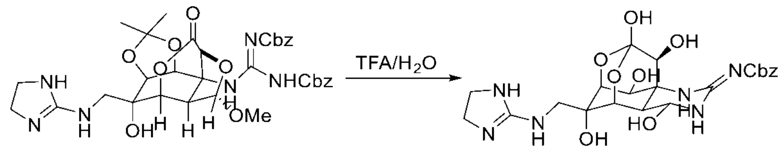


178-1 178

**Compound 178-1** (60 mg, 0.08 mmol) was dissolved in a mixed solution of TFA: $H_2O$ = 2:1 (4 mL). Under nitrogen protection, the temperature was raised to 60 °C and the reaction mixture was stirred for 16 h. After the reaction was completed, the reaction solution was concentrated and dried to give a residue. The residue was purified by reverse phase column chromatography (methanol: 0.1% acetic acid aqueous solution = 5%-8%) to give **Compound 178** as white solid (7.5 mg, yield: 17%).

$^1H$ NMR (400 MHz, MeOD) δ 7.47-7.22(m, 5H), 5.55(d, *J* = 8.7 Hz, 1H), 5.07(s, 2H), 4.17(s, 1H), 4.09(s, 1H), 4.04-3.82(m, 2H), 3.81-3.65(m, 6H), 2.23(d, J= 9.6 Hz, 1H); LCMS (m/z): 521.2[M+1]+.

Example 145: (4R,4aR,5R,6S,7S,9S,10S,10aR,11S)-6-(((4,5-dihydro-1H-imidazol-2-yl)amino)methyl)-2-iminooctahydro-5,9-epoxy-7,10a-methanooxocino[4,5-d]pyrimidine-4,6,9,10,11(10H)-pentaol (**Compound 179**)

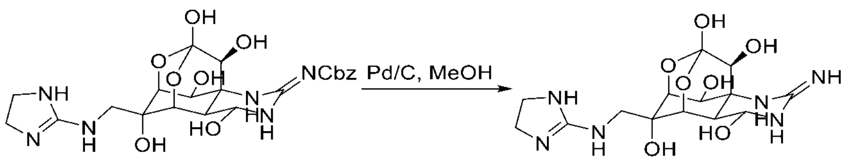


178 179

**Compound 178** (7.5 mg, 0.01 mmol) was dissolved in methanol (2 mL), and 10% Pd/C (1 mg) was added. The reaction mixture was purged with hydrogen three times, and stirred under 25 °C for 8 h. After the reaction was completed, 1% acetic acid aqueous solution was added. The reaction mixture was stirred for 5 min, filtered, and then rinsed 2-3 times with 1% acetic acid aqueous solution. The solution was lyophilized to give **Compound 179** as off-white solid (3.2 mg, yield: 57.3%).

$^1H$ NMR (400 MHz, $D_2O$) *δ* 5.41(d, *J* = 9.3 Hz, 1H), 4.27-4.19(m, 1H), 4.15(s, 1H), 3.97(s, 1H), 3.91(d, *J* = 8.5 Hz, 1H), 3.68-3.61(m, 6H), 2.27(d, *J* = 9.4 Hz, 1H); LCMS (m/z): 387.2[M+1]+.

Example 146: benzyl ((4R,4aR,5R,6S,7S,9S,10S,10aR,11S,E)-6-(guanidinomethyl)-4,6,9,10,11-pentahydroxyoctahydro-5,9-epoxy-7,10a-methanooxocino[4,5-d]pyrimidin-2(1H)-ylidene)carbamate (**Compound 180**)

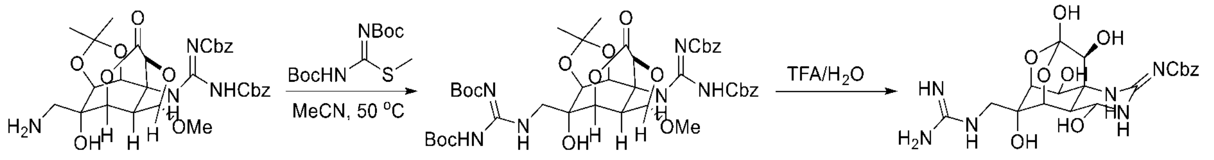


B3 180-1 180

Step 1: Synthesis of **Compound 180-1**

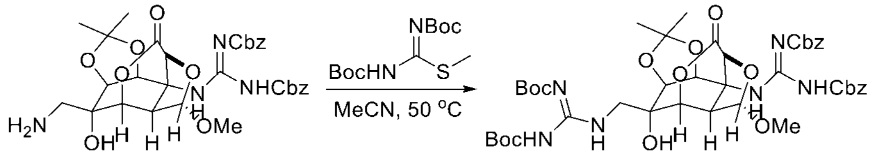


B3 180-1

**Compound B3** (238 mg, 0.37 mmol) was dissolved in acetonitrile (15 mL), to which N,N'-bis-BOC-S-methylisothiourea (130 mg, 0.447 mmol) was added. The reaction mixture was heated to 50 °C under nitrogen protection and stirred for 16 h. HPLC was used to monitor complete reaction of the raw materials. The reaction solution was concentrated and dried to give a residue (400 mg). The residue was purified by flash column chromatography (EA/PE = 0-30%) to give **Compound 180-1** (180 mg, yield: 54.8%). LCMS (m/z): 883.4[M+1]$^+$.

Step 2: Synthesis of **Compound 180**

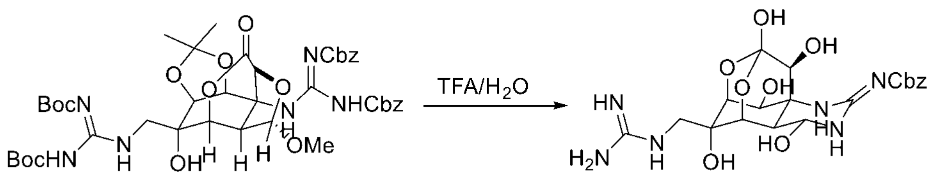


180-1 180

**Compound 180-1** (180 mg, 0.2 mmol) was dissolved in a mixed solution of water (10 mL) and TFA (5 mL). The reaction mixture was heated to 60 °C under nitrogen protection, and stirred for 20 h. HPLC was used to monitor complete reaction of the raw materials. The reaction solution was concentrated and dried to give a residue. The residue was purified by flash column chromatography (MeOH/DCM = 0-4%) to give **Compound 180** (37.5 mg, yield: 37.2%).

$^1$H NMR (400 MHz, MeOD) $\delta$ 7.47-7.25(m, 5H), 5.64(d, $J$ = 8.8 Hz, 1H), 5.19(s, 2H), 4.24(s, 1H), 4.14(s, 1H), 4.00(s, 1H), 3.92(s, 1H), 3.81(s, 2H), 2.32(d, $J$ = 9.5 Hz, 1H); LCMS (m/z): 495.2[M+1]$^+$.

Example 147: 1-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)guanidine (**Compound 181**)

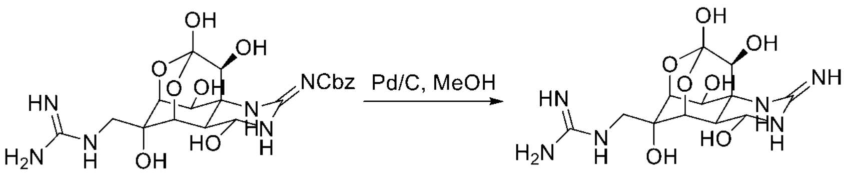


180 181

**Compound 180** (30 mg, 0.06 mmol) was dissolved in methanol (10 mL), to which 10% Pd/C (15 mg) was added. The reaction mixture was purged with hydrogen, and hydrogen was introduced into the reaction solution for 3 h. HPLC was used to monitor complete reaction of the raw materials. The reaction solution was filtered and the filter cake was rinsed with 0.05 M HOAc aqueous solution. The filtrate was concentrated to dryness to give a crude compound. The crude product was slurried with methyl tert-butyl ether (10 mL), allowed to stand, the supernatant was removed, and the residue was concentrated and dried to give **Compound 181** (20 mg, yield: 91.5%).

$^1H$ NMR (400 MHz, $D_2O$) δ 5.42(d, *J* = 9.3 Hz, 1H), 4.23(s, 1H), 4.17(s, 1H), 3.98(s, 1H), 3.90(s, 1H), 3.79(d, *J* = 15.1 Hz, 1H), 3.70(d, *J* = 15.1 Hz, 1H), 2.28(d, *J* = 9.2 Hz, 1H). LCMS (m/z): 361.1[M+1]$^+$.

Example 148: benzyl ((4R,4aR,5R,6S,7S,9S,10S,10aR,11S,E)-4,6,9,10,11-pentahydroxy-6-(methylsulfonamidomethyl)octahydro-5,9-epoxy-7,10a-methanooxocino[4,5-d]pyrimidin-2(1H)-ylidene)carbamate (**Compound 182**)

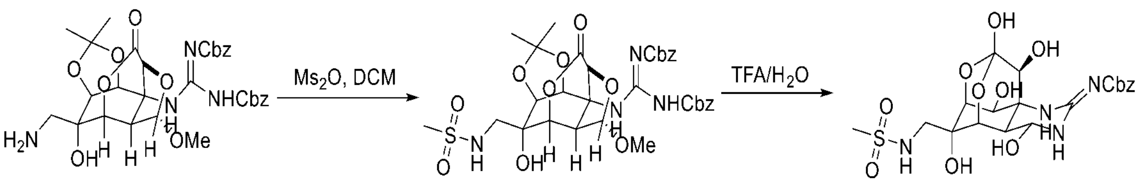


B3 182-1 182

Step 1: Synthesis of **Compound 182-1**

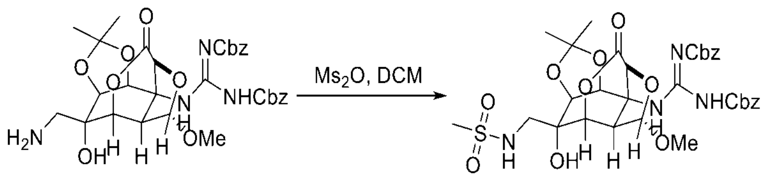


B3 182-1

**Compound B3** (140 mg, 0.22 mmol), methylsulfonic anhydride (46 mg, 0.26 mmol) and triethylamine (67 mg, 0.66 mmol) were dissolved in DCM (5 mL) and the reaction mixture was stirred at room temperature for 1 h. After the reaction was completed, the mixture was directly concentrated to dryness to give crude **Compound 182-1** (157 mg, yield: 100%). LCMS (m/z): 719.2[M+1]$^+$.

Step 2: Synthesis of **Compound 182**

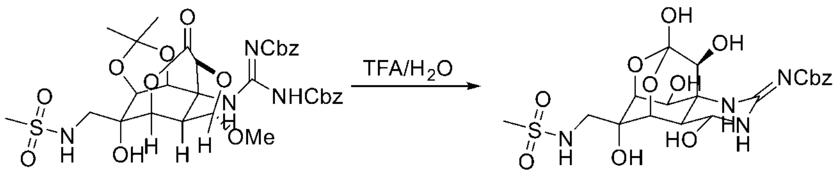


182-1 182

**Compound 182-1** (157 mg, 0.22 mmol) was dissolved in a mixed solution of water: trifluoroacetic acid = 1:1 (6 mL), and reaction mixture waheated to 65°C and stirred for 17 h. After the reaction was completed, the mixture was directly concentrated to dryness to give a crude product. The crude product was purified by reverse phase column chromatography (methanol/0.1% acetic acid aqueous solution = 0-7%) to give **Compound 182** (14 mg, yield: 12%).

$^1$H NMR (400 MHz, MeOD) $\delta$ 7.41-7.27(m, 5H), 5.59(d, *J* = 8.8 Hz, 1H), 5.09(s, 2H), 4.56(s, 1H), 4.19(s, 1H), 3.96(s, 1H), 3.80-3.52(m, 3H), 3.05(s, 3H), 2.26(d, *J* = 9.1 Hz, 1H); LCMS (m/z): 531.1[M+1]$^+$.

Example 149: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)methanesulfonamide (**Compound 183**)

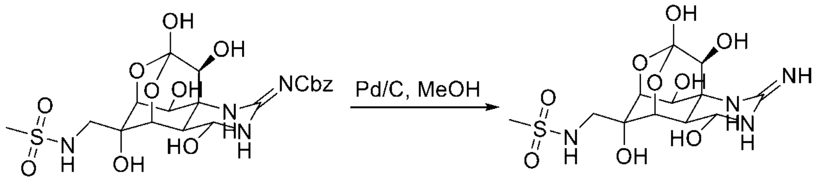


182 183

**Compound 182** (6 mg, 0.01 mmol) was dissolved in methanol (2 mL), to which 10% Pd/C (3 mg) was added. The reaction mixture was purged with hydrogen, and stirred at 28 °C for 4 h. After the reaction was completed, 0.5% acetic acid aqueous solution was added and the reaction mixture was stirred for 0.5 h. After filtration, the filtrate was concentrated to dryness and lyophilized to give **Compound 183** (3 mg, yield: 67%). LCMS (m/z): 397.1[M+1]$^+$.

Example 150: Synthesis of benzyl ((4R,4aR,5R,6S,7S,9S,10S,10aR,11S,E)-4,6,9,10,11-pentahydroxy-6-((N-methylmethylsulfonamido)methyl)octahydro-5,9-epoxy-7,10a-methanooxocino[4,5-d]pyrimidin-2(1H)-ylidene)carbamate (**Compound 184**)

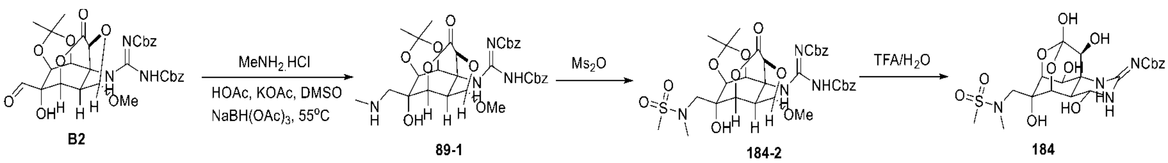


B2 89-1 184-2 184

Step 1: Synthesis of **Compound 89-1**

**Compound B2** (120 mg, 0.19 mmol), methylamine hydrochloride (19 mg, 0.28 mmol), potassium acetate (106 mg, 1.08 mmol) and acetic acid (133 mg, 2.22 mmol) were dissolved in dimethyl sulfoxide (6 mL). The reaction mixture was heated to 60 °C under nitrogen protection for 30 min, then sodium triacetylborohydride (450 mg, 2.12 mmol) was added in batches, and the reaction was carried out for 3 h. HPLC was used to monitor complete reaction of the raw materials. After the reaction was completed, the reaction solution was cooled to room temperature, then added to water (30 mL). After the reaction was completed, the reaction solution was cooled to room temperature, then added to water (30 mL) to precipitate a white solid, which filtered. The filter cake was washed with water (10 mL×2), collected, and dried to give **Compound 89-1** as white solid (140 mg, yield: > 100%). LCMS (m/z): 655.3[M+1]$^+$.

Step 2: Synthesis of **Compound 184-2**

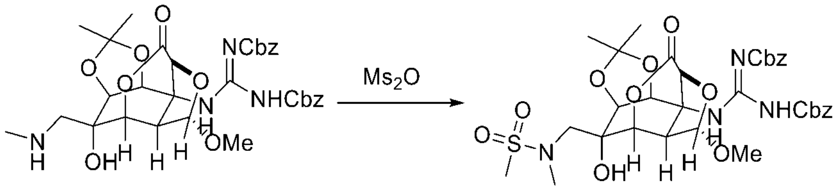


89-1 184-2

**Compound 89-1** (170 mg, 0.26 mmol) was dissolved in dichloromethane (5 mL), to which triethylamine (105 mg, 1.04 mmol) was added. Methanesulfonic anhydride (68 mg, 0.39 mmol) was added under ice bath temperature control, and the reaction mixture was stirred at room temperature for 1 h. Water was added to the reactin system, which was extracted with dichloromethane (10 mL×2). The organic phase was washed with saturated brine, dried over anhydrous sodium sulfate, concentrated and dried to give a crude product. The crude product was purified by column chromatography (ethyl acetate: petroleum ether = 0-54%) to give **Compound 184-2** (150 mg, yield: 79%). LCMS (m/z):733.2$[M+1]^+$.

Step 3: Synthesis of **Compound 184**

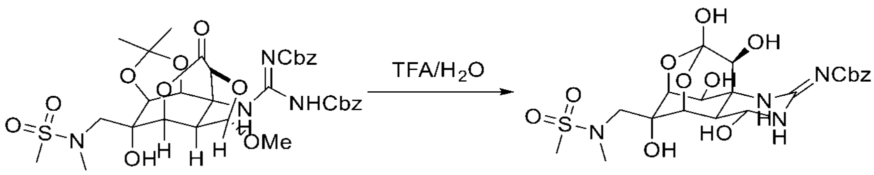


184-2 184

**Compound 184-2** (150 mg, 0.20 mmol) was dissolved in a mixed solution of water (8 mL) and TFA (2 mL), and the mixture was heated to 60 °C and stirred for 16 h. The reaction system was concentrated and dried to give a crude product. The crude product was purified by wet sample loading reverse phase column chromatography and lyophilized to give **Compound 184** as white solid (25 mg, yield: 22%).

$^1H$ NMR (400MHz, MeOD) $\delta$ 7.49-7.36(m, 5H), 5.65(d, J = 9.4Hz, 1H), 5.16(s, 2H), 4.45-3.82(m, 6H), 3.35(s, 3H), 2.37-2.29(m, 1H), 2.03(s, 3H); LCMS (m/z): 545.2$[M+1]^+$.

Example 151: Synthesis of N-methyl-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)methanesulfonamide (**Compound 185**)

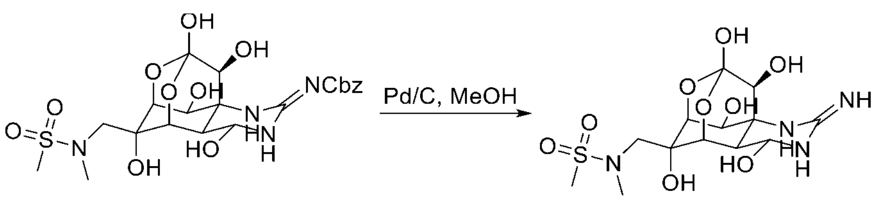


184 185

**Compound 184** (20 mg, 0.04 mmol) was dissolved in methanol (3 mL), to which 10% palladium on carbon (3 mg) was added. The reaction mixture was purged with hydrogen three times, and stirred for 3 h. After the reaction was completed, the mixture was filtered, and the filtrate was concentrated and dried to give **Compound 185** as white solid (15 mg, yield: 99%).

$^1H$ NMR (400 MHz, $D_2O$) $\delta$ 5.40(dd, $J$ = 9.3, 6.0 Hz, 1H), 4.16(d, $J$ = 5.2 Hz, 2H), 4.08(s, 1H), 3.90(dd, $J$ = 14.3, 5.5 Hz, 3H), 3.12(s, 3H), 2.75-2.68(m, 1H), 2.09(d, $J$ = 4.9 Hz, 3H); LCMS (m/z): 411.1$[M+1]^+$.

Example 152: Synthesis of benzyl ((4R,4aR,5R,6S,7S,9S,10S,10aR,11S,E)-4,6,9,10,11-pentahydroxy-6-(((trifluoromethyl)sulfonamido)methyl)octahydro-5,9-epoxy-7,10a-methanooxocino[4,5-d]pyrimidin-2(1H)-ylidene)carbamate (**Compound 186**)

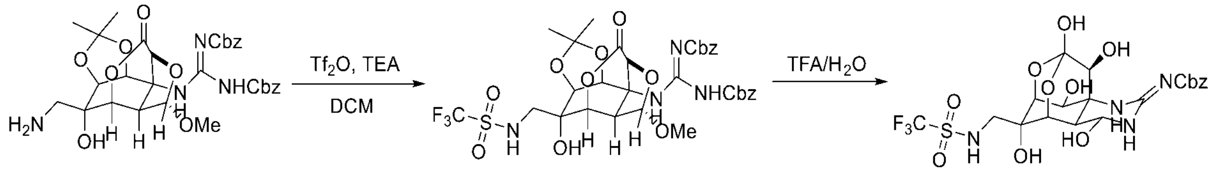


B3 186-1 186

Step 1: Synthesis of **Compound 186-1**

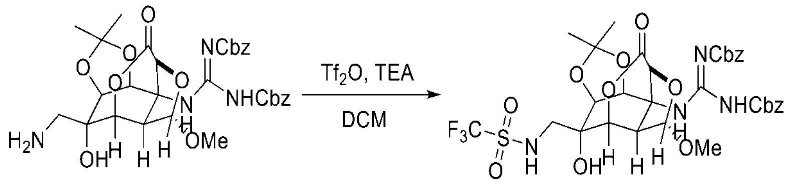


B3 186-1

**Compound B3** (150 mg, 0.23 mmol) was dissolved in dichloromethane (5 mL), to which triethylamine (70 mg, 0.69 mmol) and trifluoromethanesulfonic anhydride (99 mg, 0.35 mmol) were added, and the reaction mixture was stirred at 17°C for 1.5 h. After the reaction was completed, water was added to the reaction solution, which was extracted with dichloromethane (10 mL×3), the organic phases were combined, washed with saturated brine, and separated, and the organic phase was dried over anhydrous sodium sulfate. Filtration was conducted and the filtrate was concentrated and dried to give a residue. The residue was purified by flash column chromatography (methanol/dichloromethane = 0-2%) to give **Compound 186-1** (145 mg, yield: 80.2%). LCMS (m/z): 773.2[M+1]$^+$.

Step 2: Synthesis of **Compound 186**

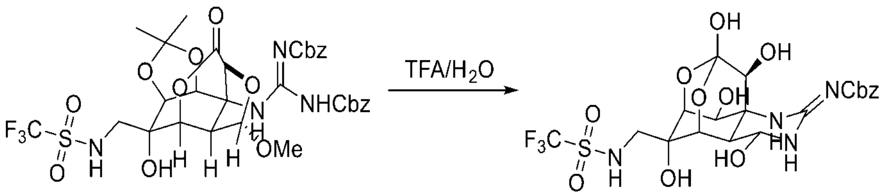


186-1 186

**Compound 186-1** (145 mg, 0.19 mmol) was dissolved in a mixed solution of water: trifluoroacetic acid = 1:2 (10 mL), and the reaction mixture was heated to 60 °C and stirred for 20 h. After the reaction was completed, the reaction solution was directly concentrated and dried to give a residue. The residue was purified by reverse phase column chromatography (methanol/0.1% acetic acid aqueous solution = 0-7%) to give **Compound 186** (17.1 mg, yield: 15.6%).

$^1$H NMR (400 MHz, MeOD) $\delta$ 7.40-7.29(m, 5H), 5.59(d, *J* = 10.0 Hz, 1H), 5.09(s, 2H), 4.18(s, 2H), 3.94(s, 2H), 3.84(d, *J* = 14.1 Hz, 2H), 2.26(d, *J* = 9.2 Hz, 1H); LCMS (m/z): 585.1[M+1]$^+$.

Example 153: Synthesis of 1,1,1-trifluoro-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)methanesulfonamide (**Compound** 187)

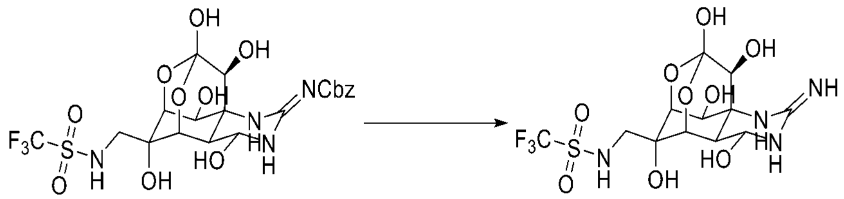

186 187

**Compound 186** (8 mg, 0.014 mmol) was dissolved in methanol (5 mL), to which 10% Pd/C (4 mg) was added. The reaction mixture was purged with hydrogen and stirred under hydrogen protection and 25 °C for 4 h. After the reaction was completed, the reaction mixture was filtered. The filtrate was concentrated and dried and then lyophilized to give **Compound 187** (4.0 mg, yield: 64.9%).

$^1$H NMR (400 MHz, $D_2O$) $\delta$ 5.42(d, $J$ = 9.5 Hz, 1H), 4.24(s, 1H), 4.18(s, 1H), 4.02(s, 1H), 3.89(s, 1H), 3.78-3.65(m, 2H), 2.29(d, $J$ = 9.4 Hz, 1H); LCMS (m/z): 451.1[M+1]$^+$.

Example 154: Synthesis of benzyl ((4R,4aR,5R,6S,7S,9S,10S,10aR,11S,E)-4,6,9,10,11-pentahydroxy-6-(propylsulfonamidomethyl)octahydro-5,9-epoxy-7,10a-methanooxocino[4,5-d]pyrimidin-2(1H)-ylidene)carbamate (**Compound 188**)

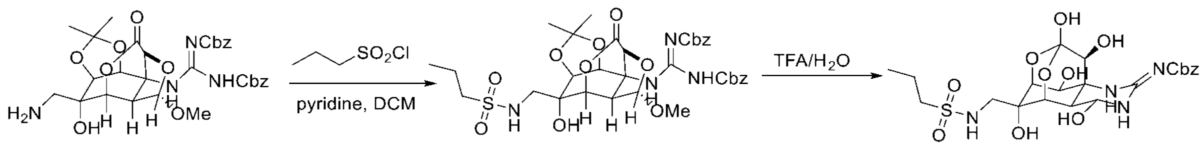


B3 188-1 188

Step 1: Synthesis of **Compound 188-1**

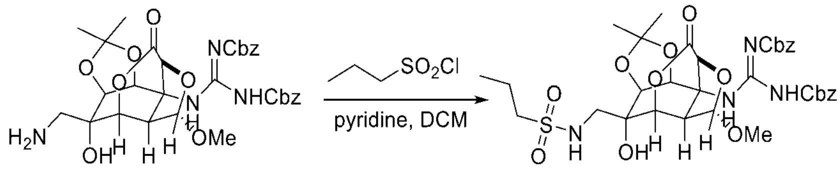


B3 188-1

**Compound B3** (130 mg, 0.20 mmol), propane-1-sulfonyl chloride (42.61 mg, 0.30 mmol) and pyridine (38.9 mg, 0.50 mmol) were dissolved in DCM (5 mL). Under nitrogen protection, the reaction mixture was stirred at 25 °C for 3 h. After the reaction was completed, water (5 mL) was added to the reaction solution, which was extracted with DCM (5 mL×3), and the organic phases were combined and concentrated to give a residue. The residue was purified by normal phase column chromatography (ethyl acetate/petroleum ether = 5%-30%) to give **Compound 188-1** as off-white solid (140 mg, yield: 92.6%). LCMS (m/z): 747.3[M+1]$^+$.

Step 2: Synthesis of **Compound 188**

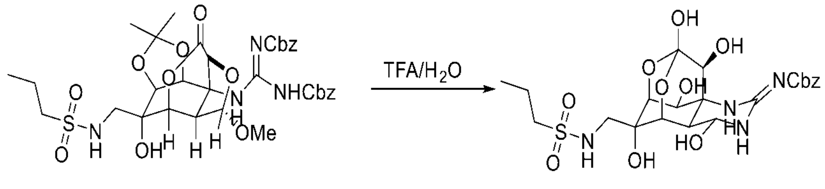


188-1 188

**Compound 188-1** (140 mg, 0.18 mmol) was dissolved in a mixed solution of trifluoroacetic acid: water = 2:1(4 mL). Under nitrogen protection, the reaction mixture was heated to 60 °C and stirred for 16 h. After the reaction was completed, the reaction solution was concentrated and dried to give a residue. The residue was purified by reverse phase column chromatography (methanol/0.1% acetic acid aqueous solution = 5-8%) to give **Compound 188** as white solid (24.3 mg, yield: 23.1%).

$^1$H NMR (400 MHz, MeOD) δ 7.44-7.15(m, 5H), 5.57(d, *J* = 9.3 Hz, 1H), 5.09(s, 2H), 4.58(d, *J* = 6.7 Hz, 1H), 4.16(s, 2H), 3.93(s, 1H), 3.75-3.59(m, 2H), 3.17-3.07(m, 2H), 2.24(d, *J* = 9.5 Hz, 1H), 1.88-1.76(m, 2H), 1.07(t, *J* = 7.5 Hz, 3H); LCMS (m/z): 559.2[M+1]$^+$.

Example 155: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)propane-1-sulfonamide (**Compound 189**)

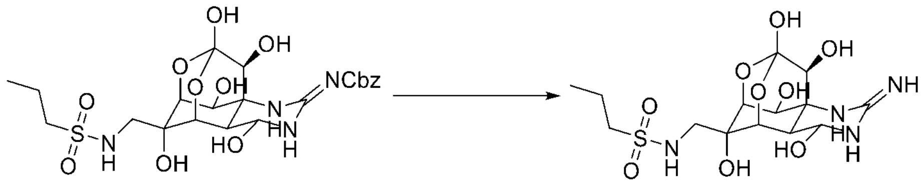

188 189

**Compound 188** (10 mg, 0.02 mmol) was dissolved in methanol (2 mL), to which 10% Pd/C (1 mg) was added. The reaction was purged with hydrogen three times, and stirred under 25 °C for 8 h. After the reaction was completed, 1% acetic acid aqueous solution was added. The reaction mixture was stirred for 5 min, filtered to remove Pd/C, and then the filter cake was washed three times with 1% acetic acid aqueous solution. The filtrate was concentrated and dried, and then lyophilized to give **Compound 189** as off-white solid (4.6 mg, yield: 60.6%). LCMS (m/z): 425.1[M+1]$^+$.

Example 156: Synthesis of benzyl ((4R,4aR,5R,6S,7S,95,10S,10aR,11S,E)-6-(cyclohexanesulfonamidomethyl)-4,6,9,10,11-pentahydroxyoctahydro-5,9-epoxy-7,10a-methanooxocino[4,5-d]pyrimidin-2(1H)-ylidene)carbamate (**Compound 190**)

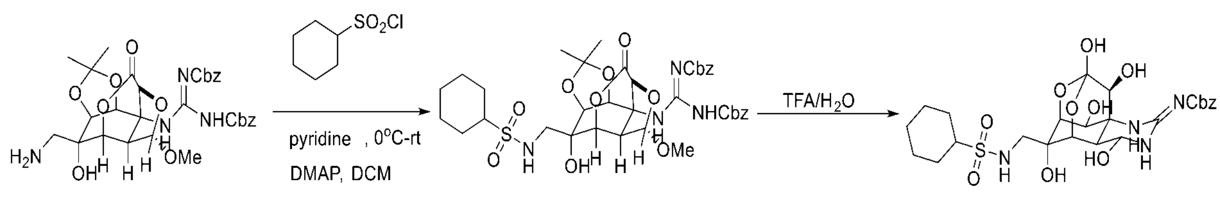


B3 190-1 190

Step 1: Synthesis of **Compound 190-1**

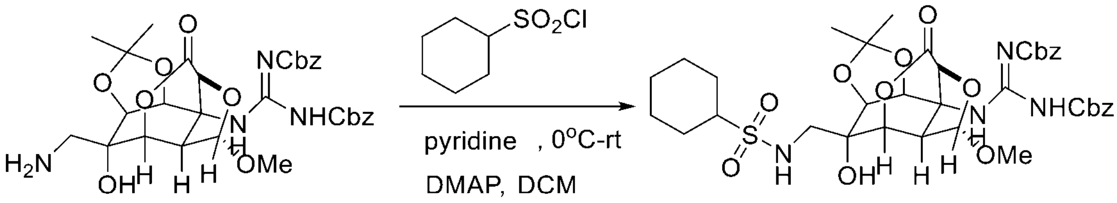


B3 190-1

**Compound B3** (140 mg, 0.22 mmol) was dissolved in DCM (8 mL), to which pyridine (69.2 mg, 0.33 mmol) and 4-dimethylaminopyridine (DMAP) (13.6 mg, 0.11 mmol) were added. Cyclohexane sulfonyl chloride (60 mg, 0.33 mmol) was added under the condition of ice bath temperature control, and the reaction mixture was stirred at room temperature for 4 h. HPLC was used to monitor complete reaction of the raw materials. Water was added to the reaction solution, which was extracted with DCM, washed with saturated brine, dried over anhydrous sodium sulfate, and filtered. The filtrate was concentrated and dried to give a residue. The residue was purified by flash column chromatography (EA/PE = 0-35%) to give **Compound 190-1** (126 mg, yield: 73%). LCMS (m/z):787.3[M+1]$^+$.

Step 2: Synthesis of **Compound 190**

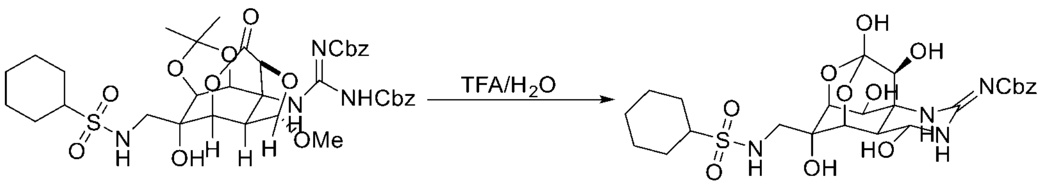


190-1 190

**Compound 190-1** (125 mg, 0.16 mmol) was dissolved in water (4 mL), dioxane (1.5 mL) and TFA (1 mL), and the reaction mixture was heated to 60 °C for 16 h. The reaction solution was concentrated to dryness, purified by wet sample loading reverse phase column chromatography, and lyophilized to give **Compound 190** as white solid (13 mg, yield: 14%).

$^1$HNMR (400MHz, MeOD) $\delta$ 7.43-7.26(m, 5H), 5.58(d, *J* = 9.4Hz, 1H), 5.09(s, 2H), 4.18(s, 2H), 3.95(s, 2H), 3.78-3.60(m, 2H), 3.17-3.06(m, 1H), 2.30-2.14(m, 3H), 1.95-1.87(m, 2H), 1.74(d, *J* = 12.3Hz, 1H), 1.59-1.47(m, 2H), 1.42-1.27(m, 3H). LCMS (m/z): 599.2[M+1]$^+$.

Example 157: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)cyclohexanesulfonamide (**Compound 191**)

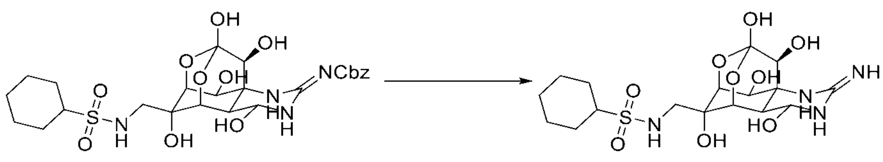

190 191

**Compound 190** (11 mg, 0.02 mmol) was dissolved in methanol (3 mL), to which was added 10% palladium on carbon (3 mg). The reaction mixture was purged with hydrogen three times, and stirred for 3 h. After the reaction was completed, the mixture was filtered, and the filtrate was concentrated to give **Compound 191** as as white solid (8 mg, yield: 94%).

$^1$HNMR (400MHz, MeOD) $\delta$ 5.51(d, *J*= 9.3Hz, 1H), 4.15(d, *J*= 10.3Hz, 2H), 3.96(s, 2H), 3.74-3.51(m, 2H), 3.13-3.00(m, 1H), 2.23(d, *J* = 9.4Hz, 1H), 2.15(d, *J* = 10.9Hz, 2H), 1.86(s, 2H), 1.71(d, *J* = 13.2Hz, 1H), 1.55-1.42(m, 2H), 1.40-1.16(m, 3H); LCMS (m/z):465.2[M+1]$^+$.

Example 158: Synthesis of benzyl ((4R,4aR,5R,6S,7S,9S,10S,10aR,11S,E)-6-(cyclopropanesulfonamidomethyl)-4,6,9,10,11-pentahydroxyoctahydro-5,9-epoxy-7,10a-methanooxocino[4,5-d]pyrimidin-2(1H)-ylidene)carbamate (**Compound 192**)

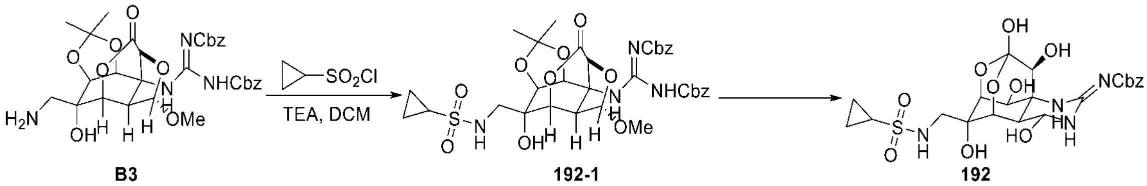


B3 192-1 192

Step 1: Synthesis of **Compound 192-1**

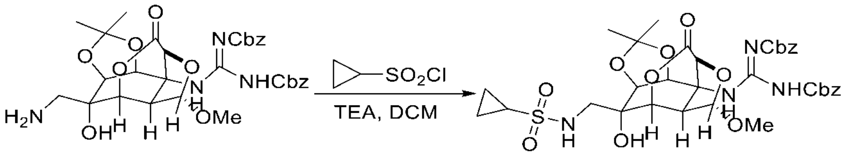


B3 192-1

**Compound B3** (160 mg, 0.25 mmol) was dissolved in DCM (5 mL), to which triethylamine (TEA) (76 mg, 0.75 mmol) was added, and cyclopropylsulfonyl chloride (53 mg, 0.375 mmol) was added under temperature control in an ice bath, and the reaction mixture was stirred at room temperature for 16 h. HPLC was used to monitor complete reaction of the raw materials. Water was added to the reaction solution, which was extracted with ethyl acetate (EA), washed with saturated brine, dried over anhydrous sodium sulfate, and the organic phase was concentrated to give crude **Compound 192-1** (210 mg, yield: 100%). LCMS (m/z):745.2[M+1]$^+$.

Step 2: Synthesis of **Compound 192**

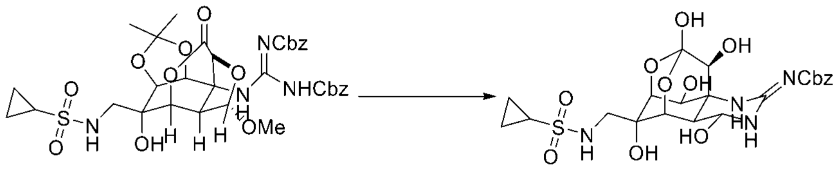

192-1 192

**Compound 192-1** (210 mg, 0.25 mmol) was dissolved in a mixed solution of water (6 mL) and TFA (1.5 mL), and the reaction mixture was stirred at 60 °C for 16 h. The reaction system was concentrated and dried to give a residue. The residue was purified by reverse phase column chromatography and then lyophilized to give **Compound 192** as white solid (32 mg, yield: 23%).

$^1$HNMR (400MHz, MeOD) $\delta$ 7.40-7.25(m, 5H), 5.55(d, *J* = 9.1Hz, 1H), 5.05(s, 2H), 4.18(s, 2H), 4.01(s, 2H), 3.78-3.58(m, 2H), 2.72-2.61(m, 1H), 2.23(d, *J* = 9.4Hz, 1H), 1.13-1.06(m, 2H), 1.05-0.99(m, 2H); LCMS (m/z):557.4 [M+1]$^+$.

Example 159: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)cyclopropanesulfonamide (**Compound 193**)

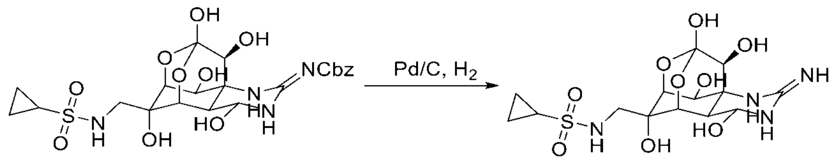


192 193

**Compound 192** (20 mg, 0.04 mmol) was dissolved in methanol (3 mL), to whichw as added 10% palladium on carbon (3 mg). The reaction mixture was purged with hydrogen three times, stirred for 3 h. HPLC was used to monitor complete reaction of the raw materials. The filtrate was concentrated to give **Compound 193** as white solid (14 mg, yield: 92%).

$^1H$ NMR (400 MHz, $D_2O$) $\delta$ 5.42(d, $J$ = 9.4 Hz, 1H), 4.24-4.17(m, 2H), 4.02(s, 1H), 3.89(d, $J$ = 7.3 Hz, 1H), 3.75-3.57(m, 2H), 2.70-2.59(m, 1H), 2.27(d, $J$ = 9.4 Hz, 1H), 1.09-1.02(m, 4H); LCMS (m/z):423.1$[M+1]^+$.

Example 160: Synthesis of benzyl ((4R,4aR,5R,6S,7S,9S,10S,10aR,11S,E)-4,6,9,10,11-pentahydroxy-6-(((1-methylethyl)sulfonamido)methyl)octahydro-5,9-epoxy-7,10a-methanooxocino[4,5-d]pyrimidin-2(1H)-ylidene)carbamate (**Compound 194**)

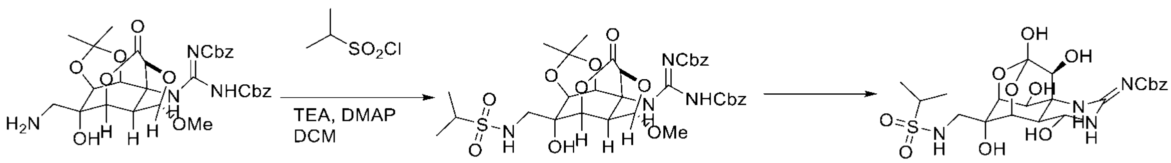


B3 194-1 194

Step 1: Synthesis of **Compound 194-1**

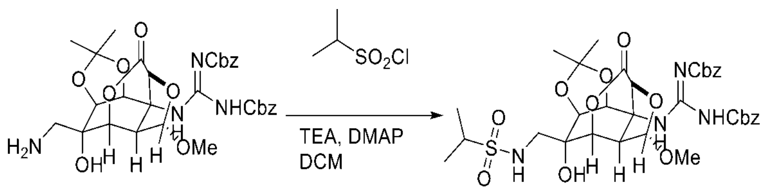


B3 194-1

**Compound B3** (180 mg, 0.28 mmol), 2-propanesulfonyl chloride (53.25 mg, 0.37 mmol), DMAP (3.7 mg, 0.031 mmol) and triethylamine (78.9 mg, 0.78 mmol) were dissolved in DCM (5 mL), and the reaction mixture was stirred for 16 h under nitrogen protection at 5 °C. After the reaction was completed, water (5 mL) was added to the reaction solution, which was extracted with DCM (5 mL×3), and the organic phases were combined and concentrated to give a residue. The residue was purified by normal phase column chromatography (ethyl acetate/petroleum ether = 5%-30%) to give **Compound 194-1** as off-white solid (100 mg, yield: 47.6%). LCMS (m/z): 747.3$[M+1]^+$.

Step 2: Synthesis of **Compound 194**

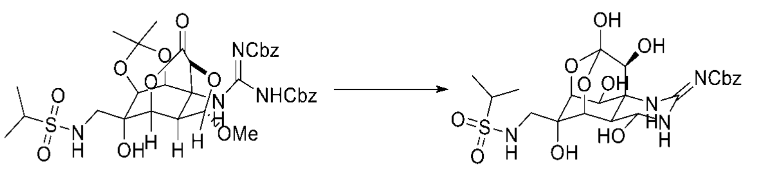

194-1 194

**Compound 194-1** (100 mg, 0.13 mmol) was dissolved in a mixed solution of TFA: $H_2O$ = 2:1 (4 mL), and the reaction mixture was heated to 60 °C under nitrogen protection and stirred for 16 h. After the reaction was completed, the reaction solution was concentrated to dryness to give a residue. The residue was purified by reverse phase column chromatography (methanol/0.1% acetic acid aqueous solution = 5-8%) to give **Compound 194** as white solid (10 mg, yield: 13.3%).

$^1H$ NMR (400 MHz, MeOD) $\delta$ 7.41-7.23(m, 5H), 5.56(d, $J$ = 9.4 Hz, 1H), 5.06(s, 2H), 4.58(s, 1H), 4.16(d, $J$ = 5.9 Hz, 2H), 3.92(d, $J$ = 8.4 Hz, 2H), 3.70(d, $J$ = 10.9 Hz, 1H), 3.39-3.33(m, 1H), 2.26-2.20(m, 1H), 1.35(t, $J$ = 8.5 Hz, 6H); LCMS (m/z): 559.2[M+1]$^+$.

Example 161: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)propane-2-sulfonamide (**Compound 195**)

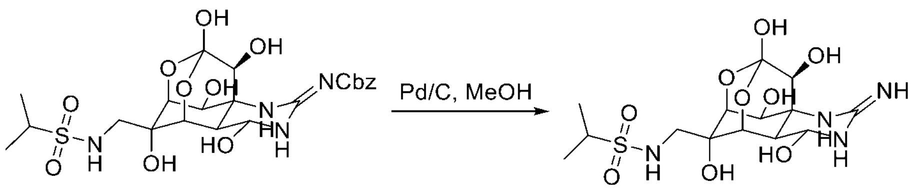


**194** **195**

**Compound 194** (10 mg, 0.02 mmol) was dissolved in methanol (2 mL), to which 10% Pd/C (1 mg) was added. The reaction mixture was purged with three times, and stirred at 25 °C for 8 h. After the reaction was completed, 1% acetic acid aqueous solution was added to the reaction mixture, which was stirred for 5 min, filtered, washed three times with 1% acetic acid aqueous solution. The filtrate was concentrated to dryness and lyophilized to give **Compound 195** as off-white solid (4.6 mg, yield: 60.6%).

$^1H$ NMR (400 MHz, $D_2O$) $\delta$ 5.40(d, $J$ = 9.4 Hz, 1H), 4.24-4.13(m, 2H), 3.98(d, $J$ = 15.8 Hz, 1H), 3.88(d, $J$ = 7.2 Hz, 1H), 3.69-3.54(m, 2H), 3.41-3.32(m, 1H), 2.26(d, $J$ = 9.4 Hz, 1H), 1.27(d, $J$ = 6.8 Hz, 6H); LCMS (m/z): 425.1[M+1]$^+$.

Example 162: Synthesis of benzyl ((4R,4aR,5R,6S,7S,9S,10S,10aR,11S,E)-4,6,9,10,11-pentahydroxy-6-((3-(methylsulfonyl)guanidino)methyl)octahydro-5,9-epoxy-7,10a-methanooxocino[4,5-d]pyrimidin-2(1H)-ylidene)carbamate (**Compound 196**)

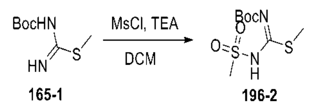


165-1 196-2

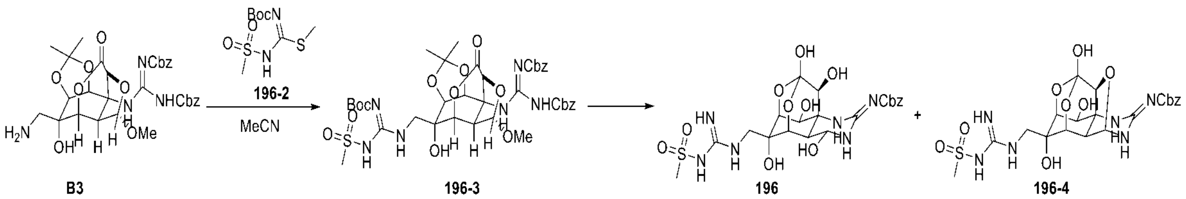


B3 196-3 196 196-4

Step 1: Synthesis of **Compound 196-2**

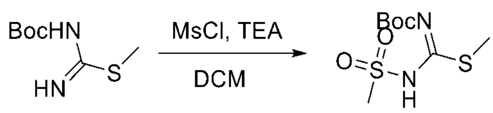


**165-1** **196-2**

**Compound 165-1** (500 mg, 2.63 mmol) was dissolved in dichloromethane (10 mL), to which diisopropylethylamine (339 mg, 2.63 mmol) was added. The reaction mixture was cooled to 0 °C under nitrogen protection, to which methylsulfonyl chloride (300 mg, 2.63 mmol) was added dropwise, and the reaction was carried out overnight at room temperature. LC-MS was used to monitor complete reaction of the raw materials. The reaction solution was poured into water (50 mL) and extracted with dichloromethane (30 mL×3). The organic phases were combined, washed with saturated brine (50 mL), dried over anhydrous sodium sulfate, and concentrated to give a residue, which was subjected to flash column chromatography (EA/PE = 0-20%) to give **Compound 196-2** (669 mg, yield: 94.9%). LCMS (m/z): 269.1 $[M+1]^+$.

Step 2: Synthesis of **Compound 196-3**

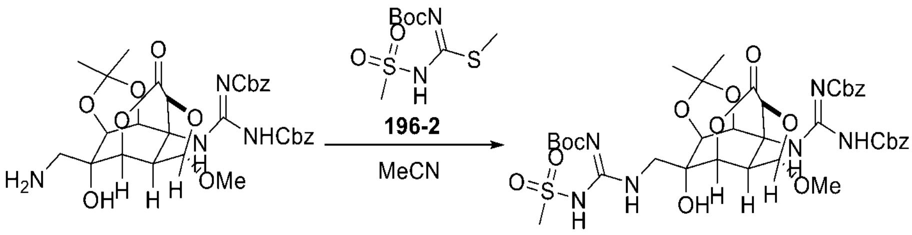


B3

196-3

**Compound B3** (200 mg, 0.31 mmol) was dissolved in acetonitrile (15 mL), to which **Compound 196-2** (125.6 mg, 0.468 mmol) was added, and the reaction mixture was heated to 60 °C under nitrogen protection overnight. HPLC was used to monitor complete reaction of the raw materials. The reaction solution was concentrated to give a residue (310 mg). The residue was subjected to flash column chromatography (EA/PE = 0-30%) to give **Compound 196-3** (160 mg, yield: 59.5%). LCMS (m/z): 861.3$[M+1]^+$.

Step 3: Synthesis of **Compound 196**

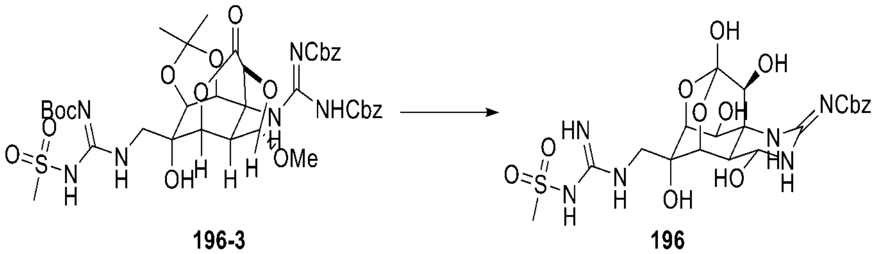

196-3 196

**Compound 196-3** (170 mg, 0.20 mmol) was dissolved in a mixed solution of water (10 mL) and TFA (5 mL), and heated to 60 °C under nitrogen protection for 20 h. HPLC was used to monitor complete reaction of the raw materials. The reaction solution was concentrated to dryness to give a residue. The residue was purified by reverse phase column chromatography (MeOH/$H_2O$ = 0-10%) to give acetate of **Compound 196** (30 mg, yield: 26.5%).

$^1$H NMR (400 MHz, MeOD) $\delta$ 7.48-7.22(m, 5H), 5.58(d, *J* = 9.4 Hz, 1H), 5.12(s, 2H), 4.35-3.71(m, 6H), 2.92(s, 3H), 2.26(d, *J* = 8.9 Hz, 1H), 1.97(s, 3H); LCMS (m/z): 573.2$[M+1]^+$.

Example 163: Synthesis of N-(N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)carbamimidoyl)methanesulfonamide (**Compound 197**)

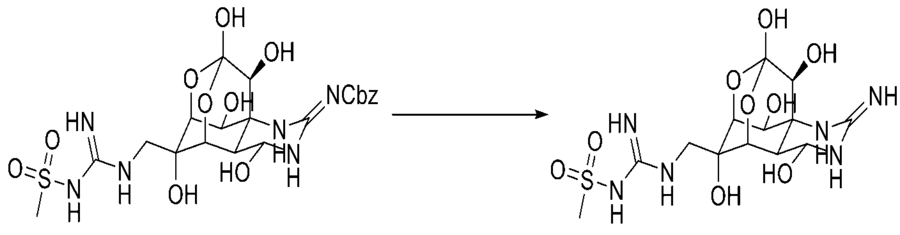

196 197

**Compound 196** (20 mg, 0.04 mmol) was dissolved in methanol (10 mL), to which 10% Pd/C (20 mg) was added. The reaction mixture was purged with hydrogen, and hydrogen was introduced into the reaction solution for 3 h. HPLC was used to monitor complete reaction of the raw materials. The reaction solution was filtered, and the filter cake was rinsed with 0.05 M AcOH aqueous solution. The filtrate was concentrated to give a crude product, which was slurried with methyl tert-butyl ether (10 mL), allowed to stand, and the supernatant was removed to give a residue. The residue was concentrated and dried to give acetate of **Compound 197** (13 mg, yield: 85%).

$^1H$ NMR (400 MHz, MeOD) $\delta$ 5.51(d, $J$ = 9.4 Hz, 1H), 4.29(d, $J$ = 5.9 Hz, 1H), 4.23(s, 1H), 4.04(s, 1H), 3.99(d, $J$ = 7.7 Hz, 1H), 3.95-3.81(m, 2H), 3.06(s, 3H), 2.35(d, $J$ = 9.3 Hz, 1H), 2.09(s, 3H); LCMS (m/z): 439.1[M+1]$^+$.

Example 164: Synthesis of benzyl ((4aR,5R,6S,7S,9S,10S,10aR,11S,E)-4,6,9,10,11-pentahydroxy-6-(phenylsulfonamidomethyl)octahydro-5,9-epoxy-7,10a-methanooxocino[4,5-d]pyrimidin-2(1H)-ylidene)carbamate (**Compound 198**)

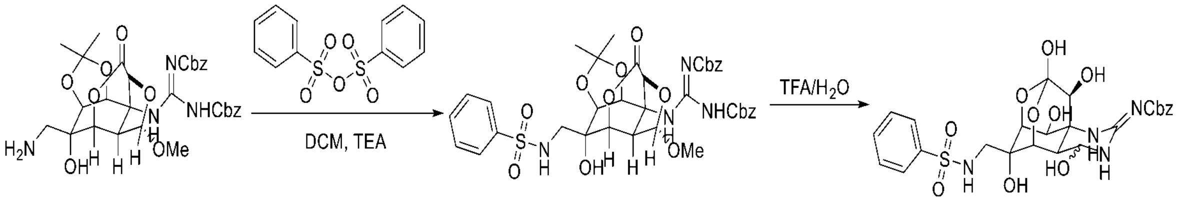


B3 198-1 198

Step 1: Synthesis of **Compound 198-1**

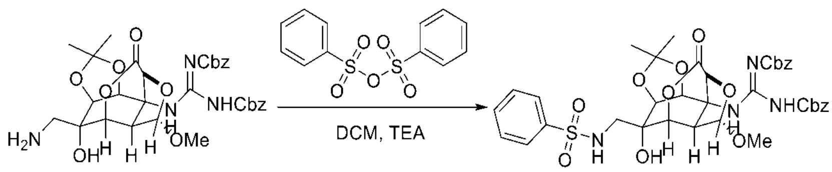


B3 198-1

**Compound B3** (500 mg, 0.78 mmol) was dissolved in DCM (10 mL), to which triethylamine (58.78 mg, 0.583 mmol) was added, and benzenesulfonic anhydride (279.7 mg, 0.94 mmol) was added under nitrogen protection, and the reaction was carried out at room temperature for 1 h. The reaction solution was poured into water (50 mL) and extracted with DCM (30 mL×3). The organic phases were combined, washed with saturated brine (50 mL), dried over anhydrous sodium sulfate, filtered, and the filtrate was concentrated and dried to give a residue. The residue was purified by flash column chromatography (EA/PE = 0-30%) to give **Compound 198-1** as white solid (510 mg, yield: 83.7%). LCMS (m/z): 781.2[M+1]$^+$.

Step 2: Synthesis of **Compound 198**

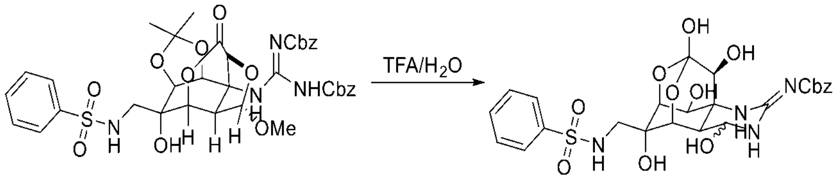


198-1 198

**Compound 198-1** (500 mg, 0.64 mmol) was dissolved in a mixed solution of water (20 mL) and TFA (20 mL), and the reaction mixture was heated to 60 °C under nitrogen protection overnight. HPLC was used to monitor complete reaction of the raw materials. The reaction solution was concentrated to dryness to give a residue. The residue was purified by flash column chromatography (MeOH/DCM = 0-10%) to give **Compound 198** as white solid (200 mg, yield: 52.7%).
$^1$H NMR (400 MHz, DMSO) $\delta$ 7.89-7.82(m, 2H), 7.69-7.59(m, 3H), 7.37-7.28(m, 5H), 5.68-5.45(m, 1H), 5.36-5.20(m, 2H), 4.98(s, 2H), 4.23-4.12(m, 1H), 3.99-3.93(m, 1H), 3.82-3.60(m, 2H), 2.02-1.93(m, 1H); LCMS (m/z): 593.2[M+1]$^+$.

Example 165: Synthesis of N-(((4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 199**)

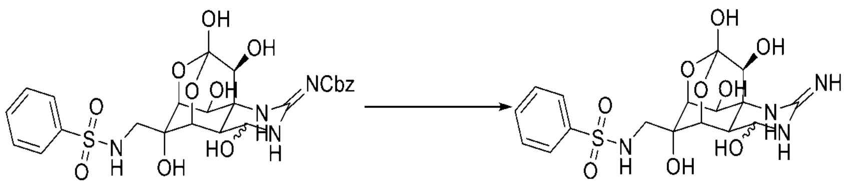

198 199

**Compound 198** (200 mg, 0.338 mmol) was dissolved in methanol (50 mL), to which 10% Pd/C (50 mg) was added. The reaction mixture was purged with hydrogen, and hydrogen was introduced into the reaction solution for 3 h. HPLC was used to monitor complete reaction of the raw materials. The reaction solution was filtered, and the filter cake was rinsed with 0.05 M AcOH aqueous solution. The filtrate was concentrated to give a crude product. The crude product was slurried with methyl tert-butyl ether (10 mL), allowed to stand, and the supernatant was removed to give a residue. The residue was concentrated and dried to give **Compound 199** acetate as white solid (220 mg, yield: > 100%).
$^1$H NMR (400 MHz, $D_2O$) $\delta$ 7.85(d, $J$ = 7.4 Hz, 2H), 7.66(t, $J$ = 7.4 Hz, 1H), 7.58(t, $J$ = 7.6 Hz, 2H), 5.39(d, $J$ = 9.5 Hz, 1H), 4.20(s, 1H), 4.12(s, 1H), 3.95(s, 1H), 3.87(d, $J$ = 8.0 Hz, 1H), 3.49-3.37(m, 2H), 2.25(d, $J$ = 9.4 Hz, 1H); LCMS (m/z): 459.1[M+1]$^+$.

Example 166: Synthesis of N-(((4S,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 199-A**) and N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 199-B**)

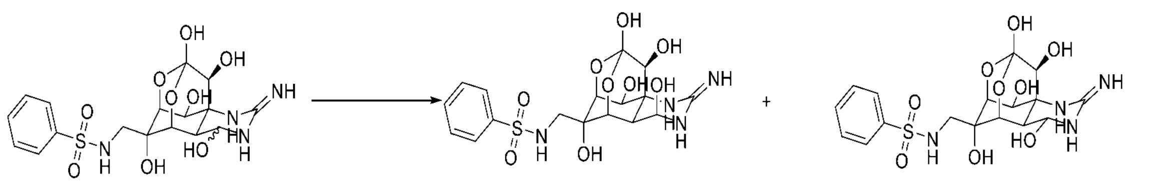

199 199-A 199-B

**Compound 199** was further separated and purified to give **Compound 199-A** and **Compound 199-B.**
**Compound 199** acetate (100.94 mg, HPLC showed that **Compound 199-A** accounted for 12.83%, and **Compound 199-B** accounted for 70.15%) was separated by high performance liquid chromatography (mobile phase A: 0.5% acetic acid aqueous solution; mobile phase B: ACN; wavelength: 214 nm; chromatographic column: Waters

Atlantis T3, 19×150 mm, 5 um, flow rate: 15 mL/min) to give **Compound 199-B** (34.61 mg, recovery rate: 48.9%, HPLC purity: 98.70%, retention time: 24.92 min) and **Compound 199-A** (2.26 mg, recovery rate: 17.5%, HPLC purity: 96.05%, retention time: 25.19 min).

**Compound 199-A:** LCMS (m/z): 459.1[M+1]$^+$.

**Compound 199-B:** $^1$H NMR (400 MHz, MeOD) δ 7.99-7.85(m, 2H), 7.74-7.54(m, 3H), 5.52(d, J = 9.3 Hz, 1H), 4.16(d, J = 20.9 Hz, 2H), 3.99(s, 2H), 3.44(s, 2H), 2.24(d, J = 9.4 Hz, 1H), 1.90(s, 3H); LCMS (m/z): 459.1[M+1]$^+$.

Example 167: cyclopentyl ((4S,4aR,5R,6S,7S,9S,10S,10aR,11S,Z)-4,6,9,10,11-pentahydroxy-6-(phenylsulfonamidomethyl)octahydro-5,9-epoxy-7,10a-methanooxocino[4,5-d]pyrimidin-2(1H)-ylidene)carbamate (**Compound 200-A**), cyclopentyl ((4R,4aR,5R,6S,7S,9S,10S,10aR,11S,Z)-4,6,9,10,11-pentahydroxy-6-(phenylsulfonamidomethyl)octahydro-5,9-epoxy-7,10a-methanooxocino[4,5-d]pyrimidin-2(1H)-ylidene)carbamate (**Compound 200-B**) and cyclopentyl ((4S,5aS,6S,8R,9S,10S,11S,11aR,12R,Z)-6,9,11-trihydroxy-9-(phenylsulfonamidomethyl)octahydro-4,8,11a-(epimethanetriyl)-6,10-epoxyoxocino[4,3-f][1,3,5]oxadiazepin-2(1H)-ylidene)carbamate (**Compound 201**)

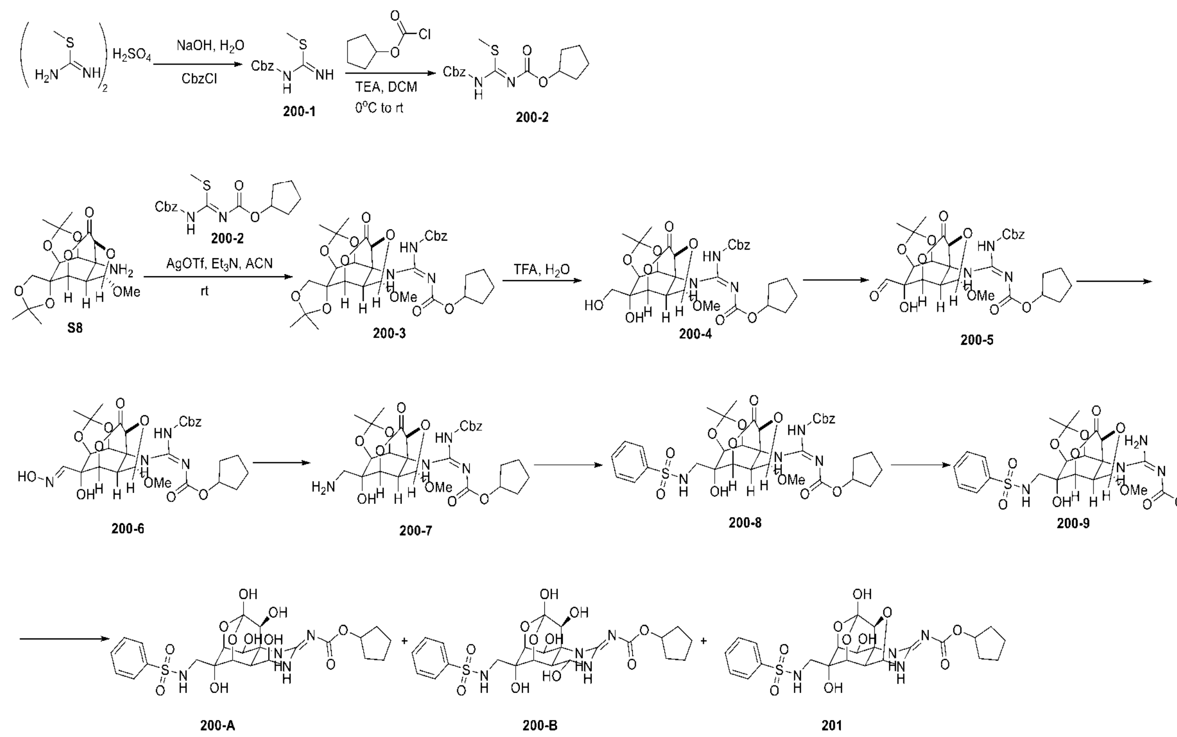


Step 1: Synthesis of **Compound 200-1**

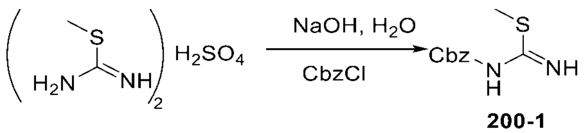


**200-1**

2-methyl-2-mercaptourea sulfate (2 g, 7.18 mmol) was dissolved in $H_2O$ (20 mL), the mixture was cooled to 0 °C, to which benzyl chloroformate (CbzCl) (2.45 g, 14.37 mmol) and NaOH (1.15 g, 28.74 mmol)/$H_2O$ (8 mL) were added dropwise, and a white solid was precipitated during the addition. The reaction was continued for 2 h after addition. TLC (PE/EA = 5:1) was used for monitoring and there were two product points generated. Dichloromethane (50 mL) was added to the reaction solution, which was stirred and separated. The aqueous phase was extracted with dichloromethane (30 mL×2). The organic phases were combined, washed with saturated brine (50 mL), dried over anhydrous sodium sulfate, filtered, and concentrated to give a crude product. The crude product was purified by flash column chromatography (EA/PE = 0-35%) to give **Compound 200-1** as white solid (1.6 g, yield: 49.7%). LCMS (m/z): 225.1[M+1]$^+$.

$^1$H NMR (400 MHz, $CDCl_3$) $\delta$ 7.41(d, $J$ = 7.0 Hz, 2H), 7.38-7.28(m, 3H), 5.16(s, 2H), 2.47(s, 3H); LCMS (m/z): 225.1[M+1]$^+$.

Step 2: Synthesis of **Compound 200-2**

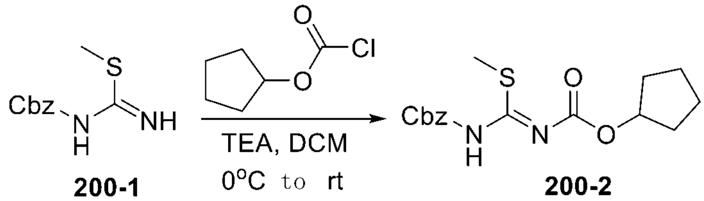


200-1 200-2

**Compound 200-1** (600 mg, 2.68 mmol) was dissolved in anhydrous dichloromethane (10 mL), to which triethylamine (1.35 g, 13.38 mmol) was added under ice bath temperature control, then cyclopentyl chloroformate (795 mg, 5.35 mmol) was added dropwise. After the addition was completed, the mixture was stirred overnight at room temperature. The reaction solution was concentrated and dried to give a residue. The residue was purified by flash column chromatography (EA/PE = 0-15%) to give **Compound 200-2** as oil (374 mg, yield: 41.6%).
$^1$H NMR (400 MHz, $CDCl_3$) $\delta$ 11.81(d, $J$ = 99.8 Hz, 1H), 7.46-7.30(m, 5H), 5.25-5.05(m, 3H), 2.41(s, 3H), 1.97-1.84(m, 2H), 1.82-1.67(m, 4H), 1.65-1.56(m, 2H); LCMS (m/z): 337.1[M+1]$^+$.

Step 3: Synthesis of **Compound 200-3**

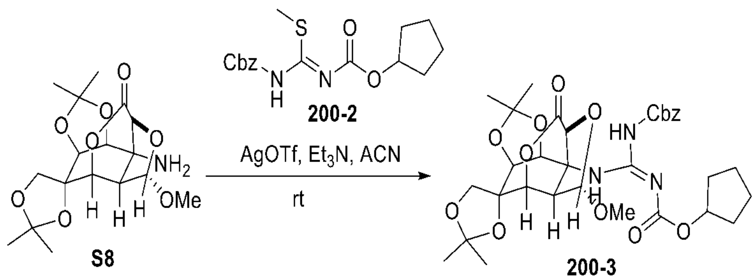


S8 200-2 200-3

**Compound S8** can be synthesized by reference to the method of CN113956266A, which is incorporated herein by reference in its entirety.
**Compound S8** (371 mg, 1 mmol) was dissolved in acetonitrile (10 mL), to which **Compound 200-2** (336 mg, 1 mmol) and triethylamine (505 mg, 5 mmol) were added. The reaction mixture was cooled to 0 °C under nitrogen protection, and then silver trifluoromethanesulfonate (514 mg, 2 mmol) was added. After the addition was completed, the reaction solution slowly returned to room temperature and the reaction was carried out overnight. The reaction solution was filtered through Celite, and the filtrate was concentrated to give a colloidal residue. The residue was purified by flash column chromatography (EA/PE = 0-30%) to give **Compound 200-3** as white foam solid (330 mg, yield: 50%). LCMS (m/z): 660.3 [M+1]$^+$.

Step 4: Synthesis of **Compound 200-4**

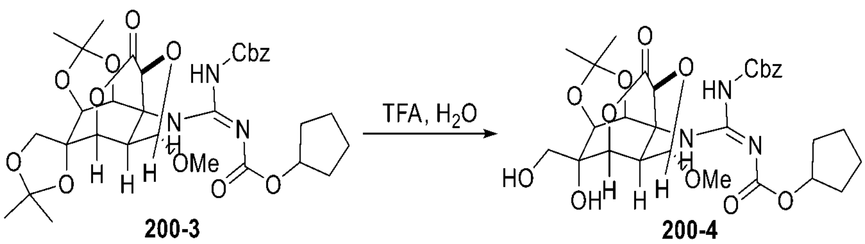


200-3 200-4

**Compound 200-3** (330 mg, 0.5 mmol) was dissolved in a mixed solution of water (15 mL) and TFA (15 mL), and the reaction was carried out overnight under nitrogen protection and about 10 °C. The reaction solution was poured into water (100 mL), extracted with EA (30 mL×3). The organic phases were combined, washed with saturated brine (50 mL), dried over anhydrous sodium sulfate, filtered, and the filtrate was concentrated to give a residue. The residue was purified by flash column chromatography (MeOH/DCM = 0-10%) to give **Compound 200-4** as white foam solid (280 mg, yield: 80%). LCMS (m/z): 620.2[M+1]+.

Step 5: Synthesis of **Compound 200-5**

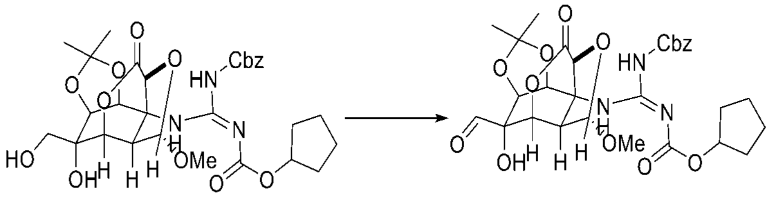

200-4 200-5

Under nitrogen protection and -78 °C, oxalyl chloride (86.3 mg, 0.68 mmol) was dissolved in DCM (8 mL), then to which dimethyl sulfoxide (106.2 mg, 1.36 mmol) was slowly added dropwise. After the addition was completed, the reaction was kept at -78 °C for 30 min. Then a solution of **Compound 200-4** (280 mg, 0.45 mmol)/DCM (2 mL) was added dropwise. After the addition was completed, TEA (228.7 mg, 2.26 mmol) was added dropwise, and the reaction mixture slowly returned to room temperature for 1 h. HPLC was used to monitor complete reaction of the raw materials. The reaction solution was poured into water (50 mL), extracted with DCM (30 mL×3). The organic phases were combined, washed with saturated brine (50 mL), dried over anhydrous sodium sulfate, filtered, and the filtrate was concentrated to give a residue. The residue was purified by flash column chromatography (EA/PE = 0-35%) to give **Compound 200-5** as a white foam solid (160 mg, yield: 80%). LCMS (m/z): 618.2[M+1]+.

Step 6: Synthesis of **Compound 200-6**

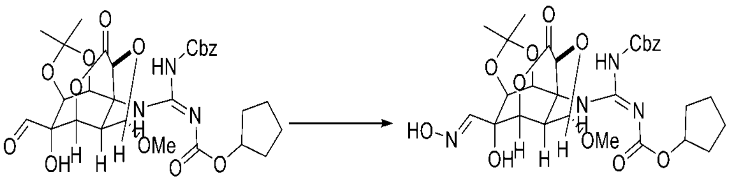

200-5 200-6

**Compound 200-5** (160 mg, 0.26 mmol) was dissolved in methanol (10 mL), to which sodium acetate (31.9 mg, 0.39 mmol) and hydroxylamine hydrochloride (39.6 mg, 0.57 mmol) were added, and the reaction was carried out overnight under nitrogen protection and 35 °C. The reaction solution was concentrated and dried, water (30 mL) was added to the residue, which was stirred for about 30 min, and a large amount of white solid was precipitated. The filter cake was collected, dried in the air to give crude **Compound 200-6** (160 mg, yield: 97.6%), which was directly used in the subsequent reaction. LCMS (m/z): 633.2[M+1]+.

Step 7: Synthesis of **Compound 200-7**

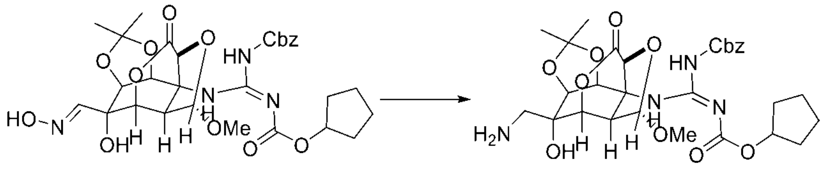

200-6 200-7

**Compound 200-6** (150 mg, 0.24 mmol) was dissolved in a mixed solution of methanol (5.9 mL) and DCM (1.5 mL), and nickel chloride hexahydrate (140 mg, 0.59 mmol) was added. Under nitrogen protection and at -40 °C, $NaBH_4$ (53.8 mg, 1.42 mmol) was added in batches. After addition was completed, the temperature was kept at -40 °C for 30 min. HPLC was used to monitor complete reaction of the raw materials. Saturated sodium bicarbonate solution (30 mL) was added to the reaction solution, which was stirred until it was pale green. DCM (30 mL) was added, the reaction solution was filtered through Celite, and the filter cake was washed with DCM (10 mL×2). The filtrate was separated, and the aqueous phase was extracted with dichloromethane/methanol (10:1, 30 mL×2). The organic phases were combined, dried over anhydrous sodium sulfate, filtered, and the filtrate was concentrated and dried to give a residue. The residue was purified by flash column chromatography (MeOH/DCM = 0-5%) to give **Compound 200-7** as white foam solid (125 mg, yield: 85.3%). LCMS (m/z): 619.3[M+1]$^+$.

Step 8: Synthesis of **Compound 200-8**

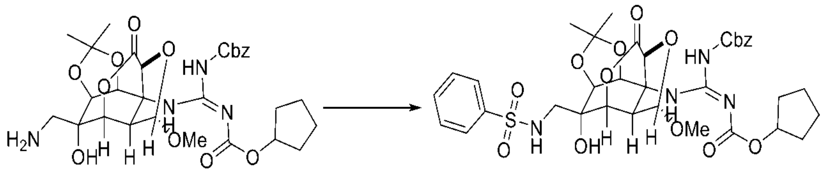

200-7 200-8

**Compound 200-7** (120 mg, 0.194 mmol) was dissolved in DCM (5 mL), to which triethylamine (58.78 mg, 0.583 mmol) and benzenesulfonic anhydride (69.5 mg, 0.233 mmol) were added, and the reaction was carried out at room temperature for 1 h under nitrogen protection. The reaction solution was poured into water (50 mL), and extracted with DCM (30 mL×3). The organic phases were combined, washed with saturated brine (50 mL), dried over anhydrous sodium sulfate, filtered, and the filtrate was concentrated and dried to give a residue. The residue was purified by flash column chromatography (EA/PE = 0-30%) to give **Compound 200-8** as white foam solid (108 mg, yield: 73.5%). LCMS (m/z): 759.3[M+1]$^+$.

Step 9: Synthesis of **Compound 200-9**

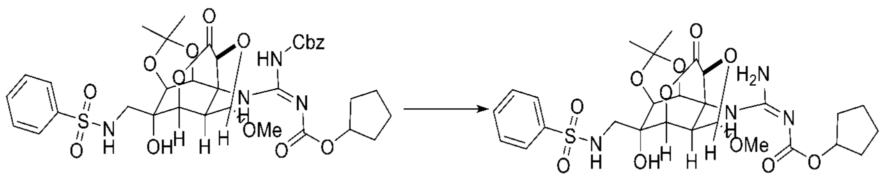

200-8 200-9

**Compound 200-8** (100 mg, 0.132 mmol) was dissolved in methanol (10 mL), to which 10% Pd/C (50 mg) was added. The reaction mixture was purged with hydrogen, and hydrogen was introduced into the reaction solution at room temperature for 3 h. HPLC was used to monitor complete reaction of the raw materials. The reaction solution was filtered, the filter cake was rinsed with methanol, and the filtrate was concentrated and dried to give crude **Compound 200-9** (83 mg, yield: 100%), which was directly used in the subsequent reaction. LCMS (m/z): 625.2[M+1]$^+$.

Step 10: Synthesis of **Compound 200-A, Compound 200-B,** and **Compound 201**

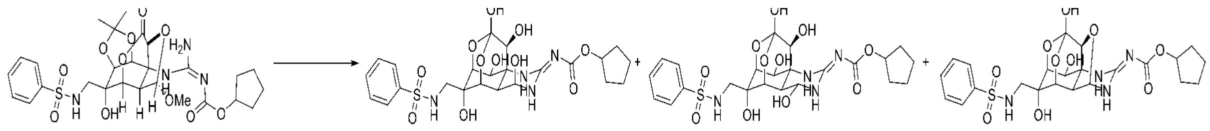

200-9 200-A 200-B 201

**Compound 200-9** (82 mg, 0.131 mmol) was dissolved in a mixed solution of water (3 mL) and TFA (3 mL), and the reaction mixture was heated to 60 °C under nitrogen protection overnight. HPLC was used to monitor complete reaction of the raw materials. The reaction solution was concentrated and dried to give a residue. The residue was purified by flash column chromatography (MeOH/DCM = 0-10%) to give **Compound 200-A** (2.7 mg, yield: 3.6%), **Compound 200-B** (25 mg, yield: 33.3%) and **Compound 201** (12 mg, yield: 16.5%) as white foam solid, total yield: 53.4%.

**Compound 200-A:**

LCMS (m/z): 571.2[M+1]$^+$.

**Compound 200-B**

$^1$H NMR (400 MHz, MeOD) $\delta$ 7.91(d, $J$ = 7.1 Hz, 2H), 7.68-7.54(m, 3H), 5.55(d, $J$ = 9.3 Hz, 1H), 5.09-5.00(m, 1H), 4.12(s, 2H), 3.90(s, 2H), 3.45(s, 2H), 2.29-2.21(m, 1H), 1.94-1.80(m, 2H), 1.79-1.67(m, 4H), 1.65-1.56(m, 2H); LCMS (m/z): 571.2[M+1]$^+$.

**Compound 201:**

$^1$H NMR (400 MHz, MeOD) $\delta$ 7.95-7.86(m, 2H), 7.67-7.54(m, 3H), 5.26(s, 1H), 5.04-4.97(m, 1H), 4.45(d, $J$ = 1.9 Hz, 1H), 4.40(s, 1H), 4.13(d, $J$ = 2.7 Hz, 1H), 4.00(s, 1H), 3.38(s, 2H), 2.68(d, $J$ = 2.8 Hz, 1H), 1.91-1.79(m, 2H), 1.78-1.65(m, 4H), 1.64-1.55(m, 2H); LCMS (m/z): 553.2[M+1]$^+$.

Example 168: Synthesis of benzyl ((4R,4aR,5R,6S,7S,9S,10S,10aR,11S,E)-4,6,9,10,11-pentahydroxy-6-(((4-methoxyphenyl)sulfonamido)methyl)octahydro-5,9-epoxy-7,10a-methanooxocino[4,5-d]pyrimidin-2(1H)-ylidene)carbamate (**Compound 202**)

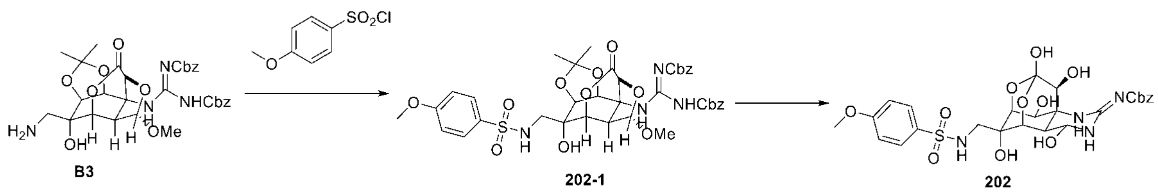

B3 202-1 202

Step 1: Synthesis of **Compound 202-1**

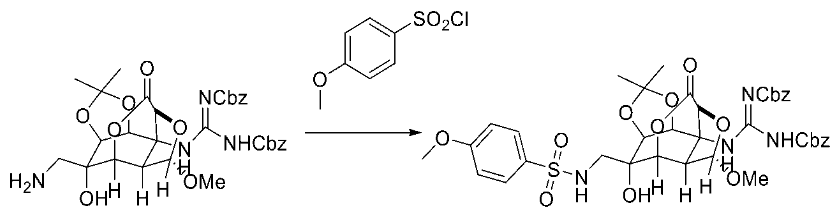

B3 202-1

**Compound B3** (110 mg, 0.17 mmol), 4-methoxybenzenesulfonyl chloride (43.3 mg, 0.21 mmol) and pyridine (27.5 mg, 0.34 mmol) were dissolved in DCM (5 mL), and the reaction mixture was stirred at 25 °C for 3 h under nitrogen protection. After the reaction was completed, water (5 mL) was added to the reaction solution, which was extracted with DCM (5 mL×3), and the organic phases were combined, concentrated and dried to give a residue. The residue was purified by normal phase column chromatography (ethyl acetate/petroleum ether = 5%-40%) to give **Compound 202-1** as off-white solid (120 mg, yield: 86.3%). LCMS (m/z): 811.2[M+1]$^+$.

Step 2: Synthesis of **Compound 202**

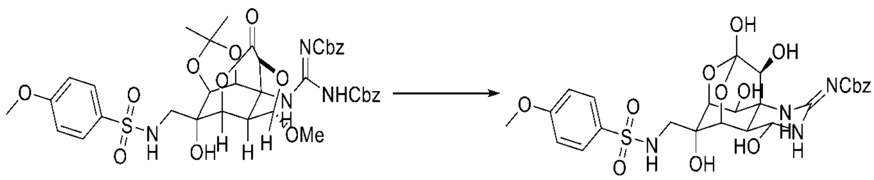

202-1 202

**Compound 202-1** (120 mg, 0.15 mmol) was dissolved in a mixed solution of TFA: $H_2O$ = 2:1(4 mL), and the reaction mixture was heated to 60 °C under nitrogen protection and stirred for 16 h. After the reaction was completed, the reaction solution was concentrated and dried to give a residue. The residue was purified by normal phase column chromatography (ethyl acetate/petroleum ether = 5%-40%) to give **Compound 202** as white solid (24.3 mg, yield: 26.4%).

$^1$H NMR (400 MHz, MeOD) δ7.86(d, *J* = 8.9 Hz, 2H), 7.43-7.26(m, 5H), 7.11(d, *J* = 8.9 Hz, 2H), 5.57(d, *J* = 9.3 Hz, 1H), 5.10(d, *J* = 4.6 Hz, 2H), 4.15(s, 2H), 3.98-3.92(s, 2H), 3.91(s, 3H), 3.48(s, 2H), 2.25(d, *J* = 9.4 Hz, 1H); LCMS (m/z): 623.2[M+1]$^+$.

Example 169: Synthesis of 4-methoxy-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 203**)

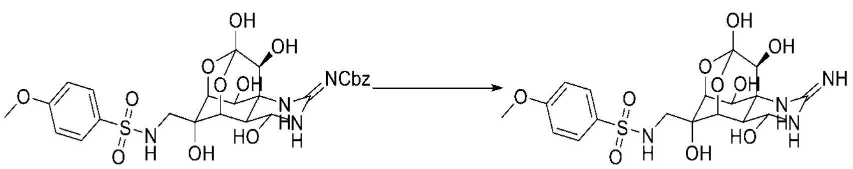

202 203

**Compound 202** (10 mg, 0.016 mmol) was dissolved in methanol (2 mL), to which 10% Pd/C (1 mg) was added. The reaction mixture was purged with hydrogen three times, and stirred under 25 °C for 8 h. After the reaction was completed, the reaction mixture was filtered, and the filtrate was concentrated to dryness and lyophilized to give **Compound 203** as off-white solid (3.5 mg, yield: 44.6%).

$^1$H NMR (400 MHz, MeOD) δ 7.90-7.81(m, 2H), 7.13(d, *J* = 8.9 Hz, 2H), 5.53(d, *J* = 9.3 Hz, 1H), 4.18(d, *J* = 8.9 Hz, 2H), 3.95(s, 1H), 3.92(s, 1H), 3.90(s, 3H), 3.45(s, 2H), 2.25(d, *J* = 9.7 Hz, 1H); LCMS (m/z): 489.1[M+1]$^+$.

Example 170: Synthesis of benzyl ((4R,4aR,5R,6S,7S,9S,10S,10aR,11S,E)-4,6,9,10,11-pentahydroxy-6-(((4-methylphenyl)sulfonamido)methyl)octahydro-5,9-epoxy-7,10a-methanooxocino[4,5-d]pyrimidin-2(1H)-ylidene)carbamate (**Compound 204**)

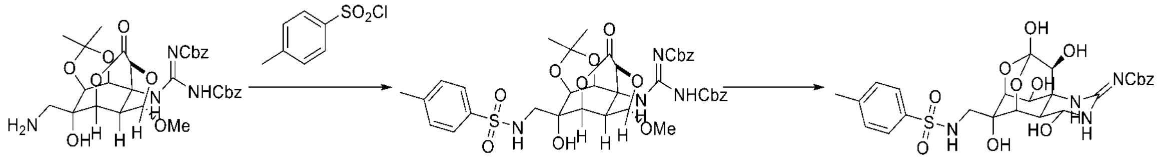

B3 204-1 204

Step 1: Synthesis of **Compound 204-1**

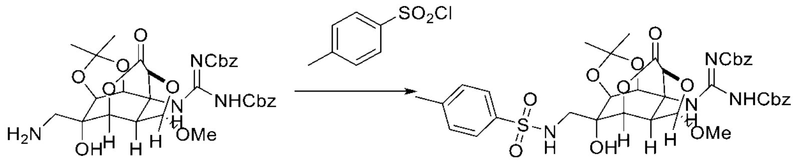

B3 204-1

**Compound B3** (110.0 mg, 0.17 mmol), 4-methylbenzenesulfonyl chloride (39.2 mg, 0.20 mmol) and pyridine (26.9 mg, 0.36 mmol) were dissolved in DCM (5 mL), and the reaction mixture was stirred at 25 °C for 3 h under nitrogen protection. After the reaction was completed, water (5 mL) was added to the reaction solution, which was extracted with DCM (5 mL×3), and the organic phases were combined and concentrated to give a residue. The residue was purified by normal phase column chromatography (ethyl acetate/petroleum ether = 5%-40%) to give **Compound 204-1** as off-white solid (120 mg, yield: 88.2%). LCMS (m/z): 795.3$[M+1]^+$.

Step 2: Synthesis of **Compound 204**

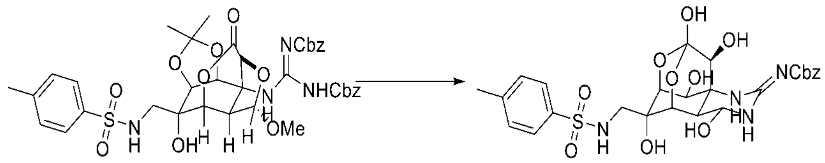

204-1 204

**Compound 204-1** (110 mg, 0.14 mmol) was dissolved in a mixed solution of TFA: $H_2O$ = 2:1 (4 mL), and the reaction mixture was heated to 60 °C under nitrogen protection and stirred for 16 h. After the reaction was completed, the reaction solution was concentrated and dried to give a residue. The residue was purified by normal phase column chromatography (ethyl acetate/petroleum ether = 5%-40%) to give **Compound 204** as white solid (24.3 mg, yield: 28.9%).

$^1$H NMR (400 MHz, MeOD) $\delta$ 7.81(d, $J$ = 8.3 Hz, 2H), 7.46-7.25(m, 7H), 5.56(d, $J$ = 9.3 Hz, 1H), 5.09(s, 2H), 4.15(s, 2H), 3.91(s, 2H), 3.48(d, $J$ = 19.3 Hz, 2H), 2.46(s, 3H), 2.24(d, $J$ = 9.3 Hz, 1H); LCMS (m/z): 607.2$[M+1]^+$.

Example 171: Synthesis of 4-methyl-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 205**)

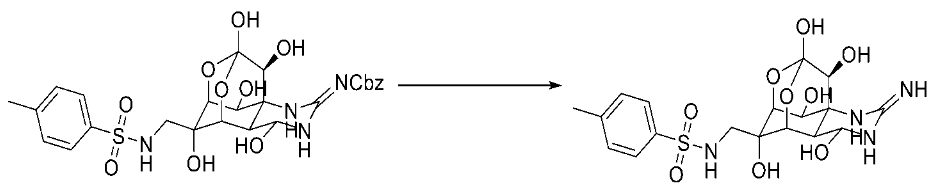

204 205

**Compound 204** (10 mg, 0.02 mmol) was dissolved in methanol (2 mL), to which 10% Pd/C (1 mg) was added. The reaction mixture was purged with hydrogen three times, and stirred under 25 °C for 8 h. After the reaction was completed, filtration was conducted. The filtrate was concentrated and dried, and lyophilized to give **Compound 205** as off-white solid (6.1 mg, yield: 78.2%).

$^1$H NMR (400 MHz, $D_2O$) $\delta$ 7.70(d, $J$ = 8.3 Hz, 2H), 7.38(d, $J$ = 8.1 Hz, 2H), 5.38(d, $J$ = 9.3 Hz, 1H), 4.18(s, 1H), 4.11(s, 1H), 3.94(s, 1H), 3.84(s, 1H), 3.45-3.30(m, 2H), 2.34(s, 3H), 2.24(d, $J$ = 9.5 Hz, 1H); LCMS (m/z):473.1[M+1]$^+$.

Example 172: Synthesis of benzyl ((4R,4aR,5R,6S,7S,9S,10S,10aR,11S,E)-4,6,9,10,11-pentahydroxy-6-(((3-methoxyphenyl)sulfonamido)methyl)octahydro-5,9-epoxy-7,10a-methanooxocino[4,5-d]pyrimidin-2(1H)-ylidene)carbamate (**Compound 206**)

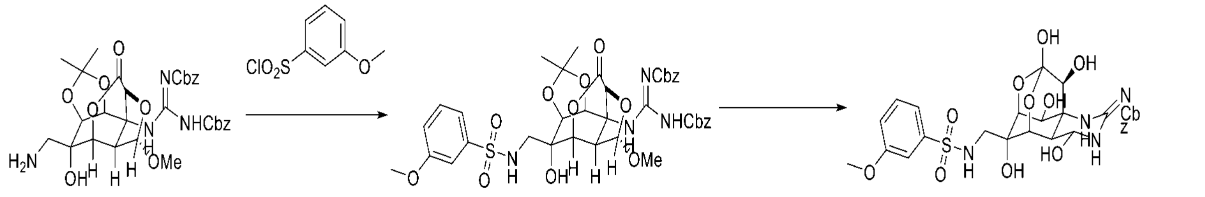

B3 206-1 206

Step 1: Synthesis of **Compound 206-1**

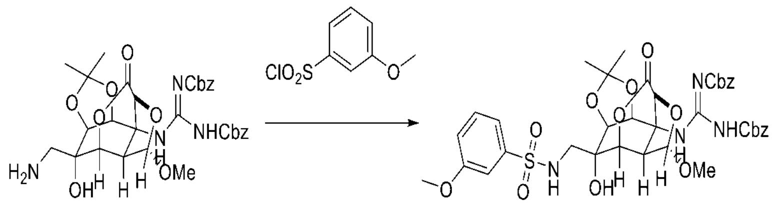

B3 206-1

**Compound B3** (120 mg, 0.19 mmol), m-methoxybenzenesulfonyl chloride (47 mg, 0.23 mmol) and pyridine (74 mg, 0.94 mmol) were dissolved in dichloromethane (5 mL), and the reaction mixture was stirred at 17 °C for 2 h. After the reaction was completed, water was added to the reaction solution, which was extracted with dichloromethane (10 mL×3). The organic phases were combined, washed with saturated brine once, separated. The organic phase was dried over anhydrous sodium sulfate, filtered, and the filtrate was concentrated and dried to give a residue. The residue was purified by normal phase column chromatography (petroleum ether: ethyl acetate = 0-55%) to give **Compound 206-1** (130 mg, yield: 85.6%). LCMS (m/z):811.2[M+1]$^+$.

Step 2: Synthesis of **Compound 206**

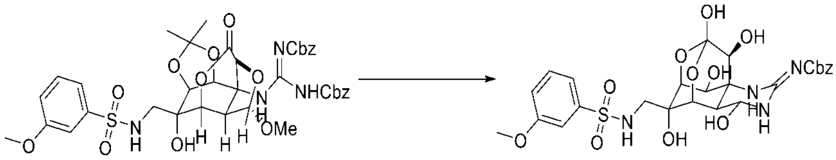

206-1 206

**Compound 206-1** (130 mg, 0.16 mmol) was dissolved in a mixed solution of water: trifluoroacetic acid = 1:1 (10 mL), and the reaction mixture was stirred at 60 °C for 20 h. After the reaction was completed, the reaction mixture was was directly concentrated to give a crude product. The crude product was purified by normal phase column (methanol/dichloromethane = 0-8.7%) to give **Compound 206** (20.1 mg, yield: 20.1%).

$^1$H NMR (400 MHz, MeOD) $\delta$ 7.54-7.47(m, 2H), 7.47-7.44(m, 1H), 7.42-7.27(m, 5H), 7.24-7.17(m, 1H), 5.57(d, $J$ = 9.3 Hz, 1H), 5.10(s, 2H), 4.11(s, 2H), 3.98-3.80(m, 5H), 3.48(s, 2H), 2.25(d, $J$ = 9.3 Hz, 1H); LCMS (m/z):623.2[M+1]$^+$.

Example 173: Synthesis of 3-methoxy-N-(((4R,4aR,5R,6S,7S,95,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 207**)

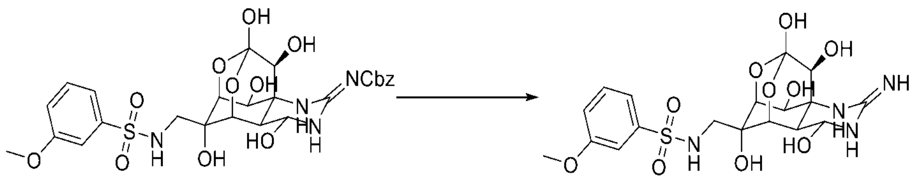

206 207

**Compound 206** (16.5 mg, 0.03 mmol) was dissolved in methanol (5 mL), to which 10% Pd/C (8 mg) was added. The reaction mixture was purged with hydrogen, and stirred at 25 °C for 4 h. After the reaction was completed, the reaction mixture was filtered, the filtrate was concentrated to dryness, and then freeze-dried to give **Compound 207** (10.1 mg, yield: 78.0%).

$^1$H NMR (400 MHz, MeOD) $\delta$ 7.53-7.45(m, 2H), 7.43(d, $J$ = 2.1 Hz, 1H), 7.23-7.14(m, 1H), 5.52(d, $J$ = 9.4 Hz, 1H), 4.15(d, $J$ = 4.6 Hz, 2H), 3.91(s, 2H), 3.88(s, 3H), 3.47(s, 2H), 2.23(d, $J$ = 9.5 Hz, 1H); LCMS (m/z):489.1[M+1]$^+$.

Example 174: Synthesis of benzyl ((4R,4aR,5R,6S,7S,9S,10S,10aR,11S,E)-4,6,9,10,11-pentahydroxy-6-(((3-methylphenyl)sulfonamido)methyl)octahydro-5,9-epoxy-7,10a-methanooxocino[4,5-d]pyrimidin-2(1H)-ylidene)carbamate (**Compound 208**)

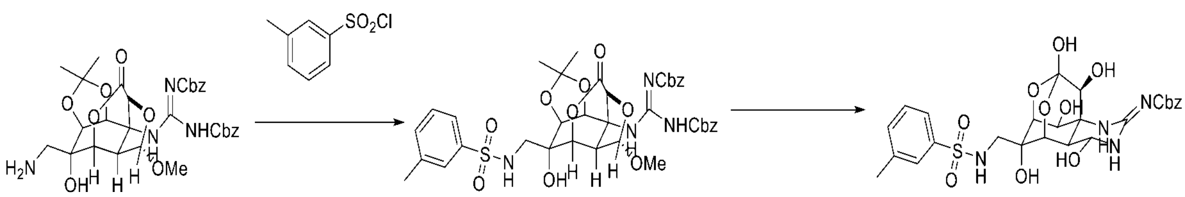

B3 208-1 208

Step 1: Synthesis of **Compound 208-1**

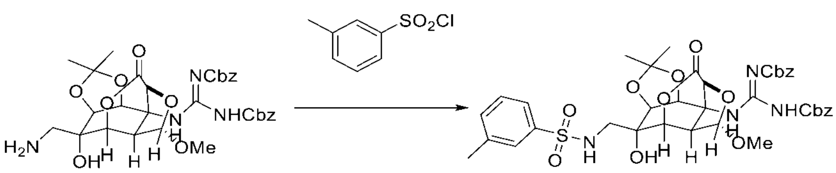

B3 208-1

**Compound B3** (120 mg, 0.19 mmol), m-methylbenzenesulfonyl chloride (43 mg, 0.23 mmol) and pyridine (74 mg, 0.94 mmol) were dissolved in dichloromethane (5 mL), and the reaction mixture was stirred at 17 °C for 2 h. After the reaction was completed, water was added to the reaction solution, which was extracted with dichloromethane (10 mL×3). The organic phases were combined, washed with saturated brine, and separated. The organic phase was dried over anhydrous sodium sulfate, filtered, and the filtrate was concentrated and dried to give a residue. The residue was purified by normal phase column (ethyl acetate/petroleum ether = 0-33%) to give **Compound 208-1** (130 mg, yield: 87.3%). LCMS (m/z):795.3[M+1]$^+$.

Step 1: Synthesis of **Compound 208**

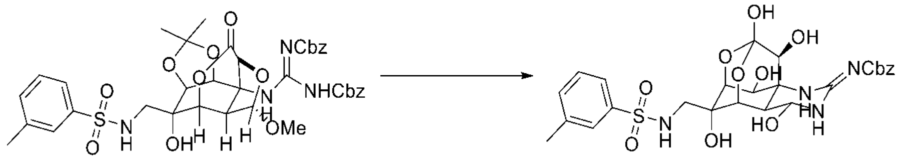

208-1 208

**Compound 208-1** (130 mg, 0.16 mmol) was dissolved in a mixed solution of water: trifluoroacetic acid = 1:1 (10 mL), and the reaction mixture was stirred at 60 °C for 20 h. After the reaction was completed, the reaction mixture was directly concentrated to give a crude product, which was purified by a normal phase column (methanol/dichloromethane = 0-7.5%) to give **Compound 208** (16.1 mg, yield: 16.2%).

$^1H$ NMR (400 MHz, MeOD) $\delta$ 7.78-7.67(m, 2H), 7.48(d, $J$ = 4.9 Hz, 2H), 7.42-7.26(m, 5H), 5.56(d, $J$ = 9.4 Hz, 1H), 5.09(s, 2H), 4.14(s, 2H), 3.91(s, 2H), 3.47(s, 2H), 2.47(s, 3H), 2.24(d, $J$ = 9.6Hz, 1H); LCMS (m/z):607.2[M+1]$^+$.

Example 175: Synthesis of 3-methyl-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 209**)

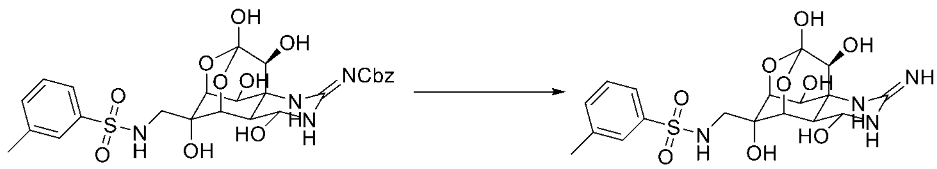

208 209

**Compound 208** (12.5 mg, 0.02 mmol) was dissolved in methanol (5 mL), to which 10% Pd/C (6 mg) was added. The reaction mixture was purged with hydrogen, and stirred at 25 °C for 4 h. After the reaction was completed, the reaction mixture was filtered. The filtrate was concentrated to dryness, and then lyophilized to give **Compound 209** (7.5 mg, yield: 77%).

$^1H$ NMR (400 MHz, MeOD) $\delta$ 7.78-7.66(m, 2H), 7.47(d, $J$ = 4.8 Hz, 2H), 5.52(d, $J$ = 9.4 Hz, 1H), 4.15(s, 2H), 3.91(s, 2H), 3.49-3.36(m, 2H), 2.45(s, 3H), 2.23(d, $J$ = 9.2 Hz, 1H); LCMS (m/z): 473.1[M+1]$^+$.

Example 176: Synthesis of benzyl ((4R,4aR,5R,6S,7S,9S,10S,10aR,11S,E)-4,6,9,10,11-pentahydroxy-6-(((4-(trifluoromethyl)phenyl)sulfonamido)methyl)octahydro-5,9-epoxy-7,10a-methanooxocino[4,5-d]pyrimidin-2(1H)-ylidene)carbamate (**Compound 210**)

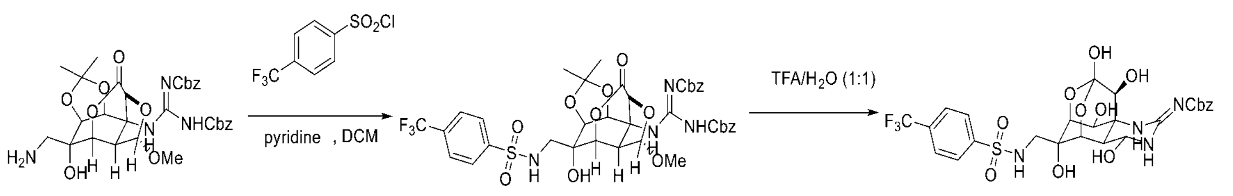


B3 210-1 210

Step 1: Synthesis of **Compound 210-1**

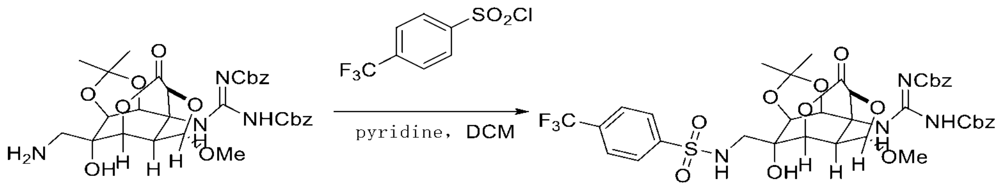


B3 210-1

**Compound B3** (130 mg, 0.2 mmol) was dissolved in DCM (5 mL), to which pyridine (47.5 mg, 0.6 mmol) was added, the reaction mixture was cooled to 0 °C under nitrogen protection, to which 4-trifluoromethylbenzenesulfonyl chloride (74.5 mg, 0.3 mmol) was added dropwise, and the reaction was carried out at room temperature for 30 min after the addition was completed. HPLC was used to monitor complete reaction of the raw materials. The reaction solution was poured into water (50 mL), and extracted with DCM (30 mL×3). The organic phases were combined, washed with saturated brine (50 mL), dried over anhydrous sodium sulfate, filtered, and the filtrate was concentrated and dried to give a residue. The residue was purified by flash column chromatography (EA/PE = 0-30%) to give **Compound 210-1** as white foam solid (134 mg, yield: 77.8%). LCMS (m/z): 849.2[M+1]+.

Step 2: Synthesis of **Compound 210**

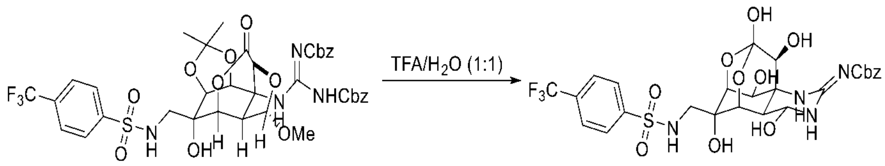


210-1 210

**Compound 210-1** (140 mg, 0.16 mmol) was dissolved in a mixed solution of water (10 mL) and TFA (10 mL), heated to 60 °C under nitrogen protection, and the reaction was carried out overnight. HPLC was used to monitor complete reaction of the raw materials. The reaction solution was concentrated and dried to give a residue. The residue was purified by flash column chromatography (MeOH/DCM = 0-10%) to give **Compound 210** as white solid (25 mg, yield: 23%).

$^1H$ NMR (400 MHz, MeOD) $\delta$ 8.10(d, *J* = 8.2 Hz, 2H), 7.90(d, *J* = 8.3 Hz, 2H), 7.41-7.21(m, 5H), 5.51(d, *J* = 9.4 Hz, 1H), 5.05(s, 2H), 4.19-4.07(m, 2H), 4.04-3.86(m, 1H), 3.56-3.39(m, 2H), 2.20(d, *J* = 9.6 Hz, 2H); LCMS (m/z): 661.1[M+1]+.

Example 177: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)-4-(trifluoromethyl)benzenesulfonamide (**Compound 211**)

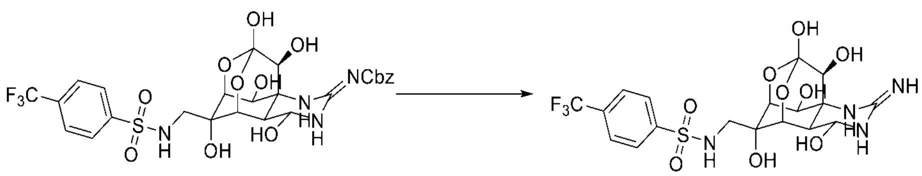

210 211

**Compound 210** (25 mg, 0.038 mmol) was dissolved in methanol (10 mL), to which 10% Pd/C (20 mg) was added. The reaction mixture was purged with hydrogen, and hydrogen was introduced into the reaction solution for 3 h. HPLC was used to monitor complete reaction of the raw materials. The reaction solution was filtered, and the filter cake was rinsed with 0.05 M AcOH aqueous solution. The filtrate was concentrated to give a crude product. The crude product was slurried with methyl tert-butyl ether (10 mL), allowed to stand, and the supernatant was removed to give a residue. The residue was concentrated to give **Compound 211** (16 mg, yield: 80%).

$^1H$ NMR (400 MHz, $D_2O$) $\delta$ 7.97(d, *J* = 8.3 Hz, 2H), 7.85(d, *J* = 8.4 Hz, 2H), 5.35(d, *J* = 9.4 Hz, 1H), 4.15(s, 1H), 4.08(s, 1H), 3.89(d, *J* = 8.8 Hz, 1H), 3.83(d, *J* = 7.5 Hz, 1H), 3.51-3.32(m, 2H), 2.20(d, *J* = 9.3 Hz, 1H); LCMS (m/z): 527.1[M+1]+.

Example 178: Synthesis of benzyl ((4R,4aR,5R,6S,7S,9S,10S,10aR,11S,E)-6-(((4-fluorophenyl)sulfonamido)methyl)-4,6,9,10,11-pentahydroxyoctahydro-5,9-epoxy-7,10a-methanooxocino[4,5-d]pyrimidin-2(1H)-ylidene)carbamate (**Compound 212**)

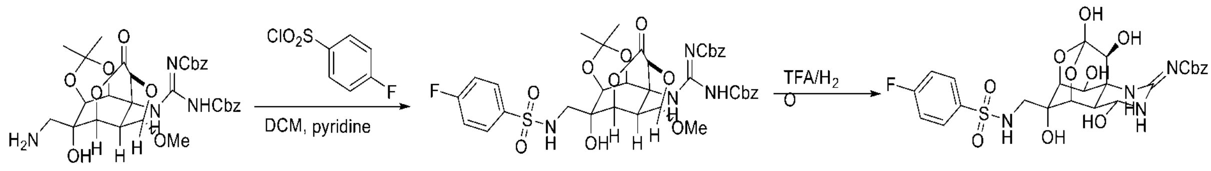


**B3** **212-1** **212**

Step 1: Synthesis of **Compound 212-1**

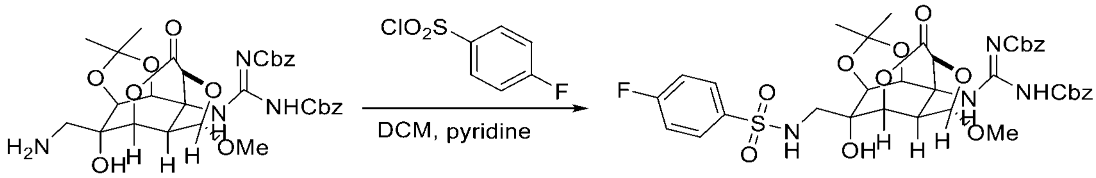


**B3** **212-1**

**Compound B3** (130 mg, 0.20 mmol) was dissolved in DCM (5 mL), to which pyridine (64 mg, 0.81 mmol) was added. 4-fluorobenzenesulfonyl chloride (59 mg, 0.31 mmol) was added under ice bath temperature control, and the reaction mixture was stirred at room temperature for 4 h. HPLC was used to monitor complete reaction of the raw materials. Water was added to the reaction mixture, which was extracted with DCM. The organic phase was washed with saturated brine, dried over anhydrous sodium sulfate, and purified by wet sample loading column chromatography (ethyl acetate: petroleum ether = 0-32%) to give **Compound 212-1** (135 mg, yield: 83.3%). LCMS (m/z):799.2[M+1]+.

Step 2: Synthesis of **Compound 212**

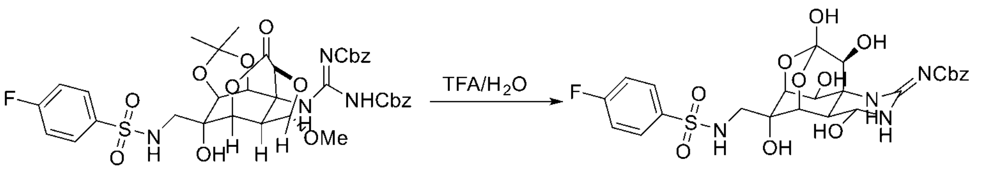


**212-1** **212**

**Compound 212-1** (135 mg, 0.17 mmol) was dissolved in a mixed solution of water (6 mL) and TFA (1.5 mL), and the reaction mixture was stirred at 60 °C for 16 h. The reaction system was concentrated and dried, purified by wet sample loading reverse phase column chromatography, and lyophilized to give **Compound 212** as white solid (17 mg, yield: 16.4%).

$^1$H NMR (400 MHz, MeOD) $\delta$ 8.01-7.93(m, 2H), 7.39-7.26(m, 7H), 5.54(d, $J$ = 9.4 Hz, 1H), 5.07(s, 2H), 4.11(s, 2H), 3.89(s, 2H), 3.58-3.36(m, 2H), 2.22(d, $J$ = 9.3 Hz, 1H). LCMS (m/z): 611.1[M+1]+.

Example 179: Synthesis of 4-fluoro-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 213**)

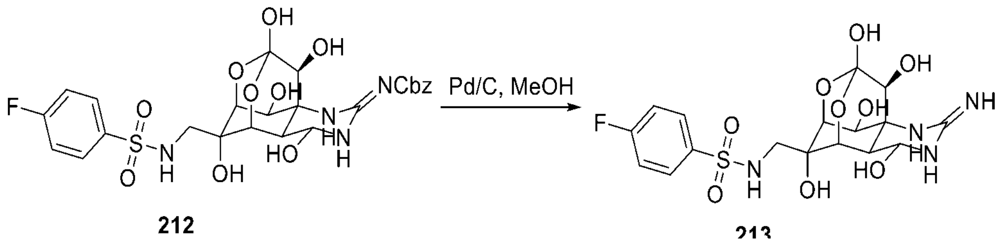


212 213

**Compound 212** (7 mg, 0.01 mmol) was dissolved in methanol (2 mL), to which $Pd(OH)_2/C$ (2 mg) was added. The reaction mixture was purged with hydrogen three times and stirred for 3 h. After the reaction was completed, the reaction mixture was filtered, and the filtrate was concentrated to give **Compound 213** as white solid (5.7 mg, yield: 100%).

$^1H$ NMR (400MHz, $D_2O$) $\delta$ 7.90-7.81(m, 2H), 7.32-7.23(m, 2H), 5.38(d, $J$ = 9.5Hz, 1H), 4.13(s, 1H), 3.99(s, 1H), 3.87(s, 1H), 3.83-3.72(m, 1H), 3.44-3.37(m, 2H), 2.15(d, $J$ =9.6Hz, 1H); LCMS (m/z): 477.1[M+1]$^+$.

Example 180: Synthesis of benzyl ((4R,4aR,5R,6S,7S,9S,10S,10aR,11S,E)-6-(((2-fluorophenyl)sulfonamido) methyl)-4,6,9,10,11-pentahydroxyoctahydro-5,9-epoxy-7,10a-methanooxocino[4,5-d]pyrimidin-2(1H)-ylidene)carbamate (**Compound 214**)

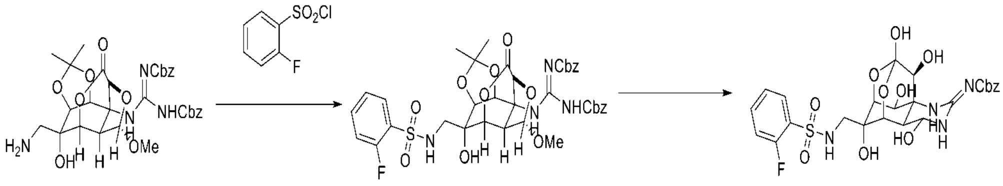

B3 214-1 214

Step 1: Synthesis of **Compound 214-1**

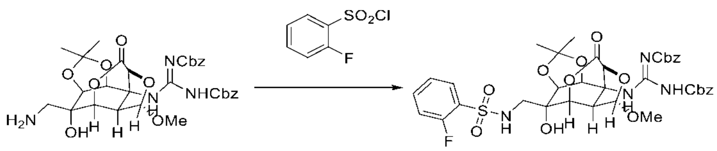

B3 214-1

**Compound B3** (110 mg, 0.17 mmol) was dissolved in DCM (5 mL), to which pyridine (54.4 mg, 0.69 mmol) was added. 2-fluorobenzenesulfonyl chloride (40 mg, 0.21 mmol) was added under ice bath temperature control, and the reaction mixture was stirred at room temperature for 3 h. After the reaction was completed, water was added to the reaction solution, which was extracted with DCM. The organic phase was washed with saturated brine, dried over anhydrous sodium sulfate, purified by wet sample loading column chromatography (ethyl acetate: petroleum ether = 0-35%), and concentrated to give **Compound 214-1** (140 mg, yield: 100%). LCMS (m/z): 799.2[M+1]$^+$.

Step 2: Synthesis of **Compound 214**

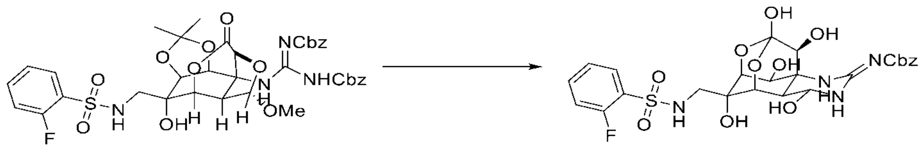

214-1 214

**Compound 214-1** (140 mg, 0.17 mmol) was dissolved in a mixed solution of water (5 mL) and TFA (3 mL), and the

reaction mixture was stirred at 60 °C for 16 h. The reaction system was concentrated to dryness, purified by wet sample loading column chromatography (methanol: dichloromethane = 0-8%), and lyophilized to give **Compound 214** as white solid (32 mg, yield: 30%).

$^1$H NMR (400MHz, MeOD) $\delta$ 7.95-7.87(m, 1H), 7.71-7.62(m, 1H), 7.48-7.21(m, 7H), *5.55(d, J* = 9.4Hz, 1H), 5.08(s, 2H), 4.75-3.55(m, 4H), 3.63-3.44(m, 2H), 2.23(d, *J* =9.5Hz, 1H); LCMS (m/z):611.1[M+1]$^+$.

Example 181: Synthesis of 2-fluoro-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 215**)

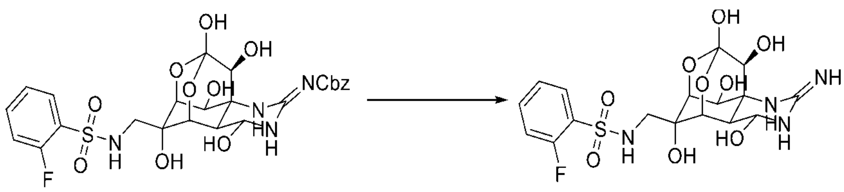

**214** **215**

**Compound 214** (24 mg, 0.04 mmol) was dissolved in methanol (2 mL), to which $Pd(OH)_2/C$ (2 mg) was added. The reaction mixture was purged with hydrogen three times, and stirred for 3 h. After the reaction was completed, the reaction mixture was filtered, and the filtrate was concentrated to give **Compound 215** as white solid (14 mg, yield: 75%).

$^1$HNMR (400MHz, $D_2O$) $\delta$ 7.82(t, *J* = 7.7Hz, 1H), 7.65(dd, *J* = 13.4, 7.6Hz, 1H), 7.31(dd, *J* = 16.3, 8.5Hz, 2H), 5.37(d, *J* = 9.4Hz, 1H), 4.18(s, 1H), 4.11(s, 1H), 3.92(d, *J* = 10.2Hz, 1H), 3.85(d, *J* = 8.6Hz, 1H), 3.56-3.43(m, 2H), 2.22(d, *J* = 9.4Hz, 1H); LCMS (m/z):477.1[M+1]$^+$.

Example 182: Synthesis of benzyl ((4R,4aR,5R,6S,7S,9S,10S,10aR,11S,E)-6-(((3-fluorophenyl)sulfonamido)methyl)-4,6,9,10,11-pentahydroxyoctahydro-5,9-epoxy-7,10a-methanooxocino[4,5-d]pyrimidin-2(1H)-ylidene)carbamate (**Compound 216**)

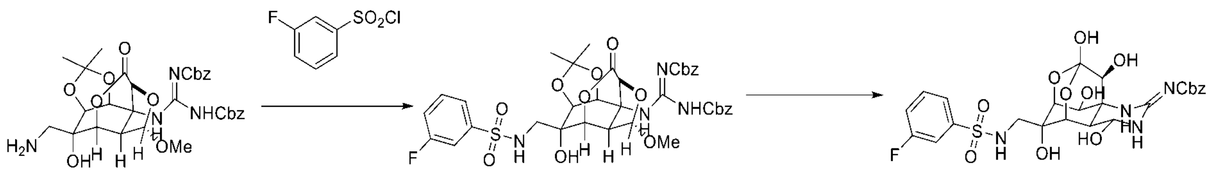

**B3** **216-1** **216**

Step 1: Synthesis of **Compound 216-1**

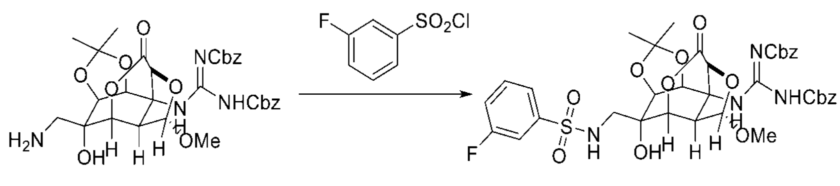

**B3** **216-1**

**Compound B3** (110 mg, 0.17 mmol) was dissolved in DCM (5 mL), to which pyridine (54.4 mg, 0.69 mmol) was added. 3-fluorobenzenesulfonyl chloride (40 mg, 0.21 mmol) was added under ice bath temperature control, and the reaction mixture was stirred at room temperature for 3 h. After the reaction was completed, water was added to the reaction solution, which was extracted with DCM. The organic phase was washed with saturated brine, dried over anhydrous sodium sulfate, purified by wet sample loading column chromatography (ethyl acetate: petroleum ether = 0-35%), and concentrated to give **Compound 216-1** (140 mg, yield: 100%). LCMS (m/z): 799.2[M+1]$^+$.

Step 2: Synthesis of **Compound 216**

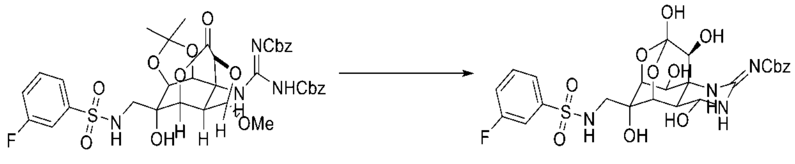

216-1 216

**Compound 216-1** (140 mg, 0.17 mmol) was dissolved in a mixed solution of water (5 mL) and TFA (3 mL), and the reaction mixture was stirred at 60 °C for 16 h. The reaction solution was concentrated and dried, purified by wet sample loading column chromatography (methanol: dichloromethane = 0-8.2%), and lyophilized to give **Compound 216** as white solid (30 mg, yield: 28%).

$^1$H NMR (400MHz, MeOD) $\delta$ 7.74(d, $J$ = 8.2Hz, 1H), 7.68-7.58(m, 2H), 7.46-7.23(m, 6H), 5.53(d, $J$ = 9.3Hz, 1H), 5.06(s, 2H), 4.12(s, 2H), 3.88(s, 2H), 3.62-3.42(m, 2H), 2.21(d, 1H); LCMS (m/z): 611.2[M+1]$^+$.

Example 183: Synthesis of 3-fluoro-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 217**)

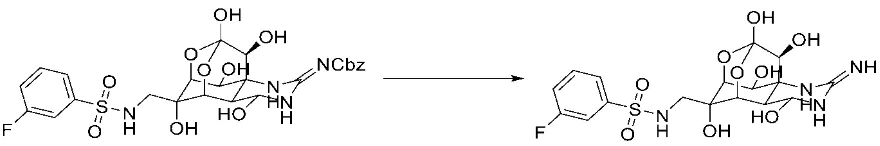

216 217

**Compound 216** (25 mg, 0.04 mmol) was dissolved in methanol (2 mL), to which Pd(OH) $_2$/C (2 mg) was added. The reaction mixture was purged with hydrogen three times, and stirred for 3 h. After the reaction was completed, the reaction mixture was filtered, and the filtrate was concentrated to give **Compound 217** as white solid (15 mg, yield: 77%).

$^1$HNMR (400MHz, $D_2O$) $\delta$ 7.69-7.54(m, 3H), 7.38(t, $J$ = 8.2Hz, 1H), 5.38(d, $J$ = 9.4Hz, 1H), 4.19(s, 1H), 4.10(s, 1H), 3.94(s, 1H), 3.86(d, $J$ = 8.1Hz, 1H), 3.51-3.34(m, 2H), 2.23(d, $J$ = 9.3Hz, 1H); LCMS (m/z):477.1[M+1]$^+$.

Example 184: Synthesis of benzyl ((4R,4aR,5R,6S,7S,9S,10S,10aR,11S,E)-6-(((4-(tert-butyl)phenyl)sulfonamido)methyl)-4,6,9,10,11-pentahydroxyoctahydro-5,9-epoxy-7,10a-methanooxocino[4,5-d]pyrimidin-2(1H)-ylidene)carbamate (**Compound 218**)

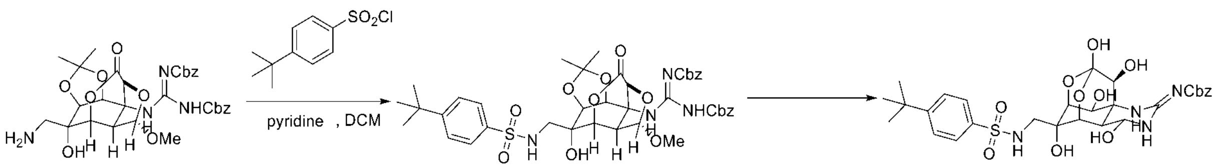


B3 218-1 218

Step 1: Synthesis of **Compound 218-1**

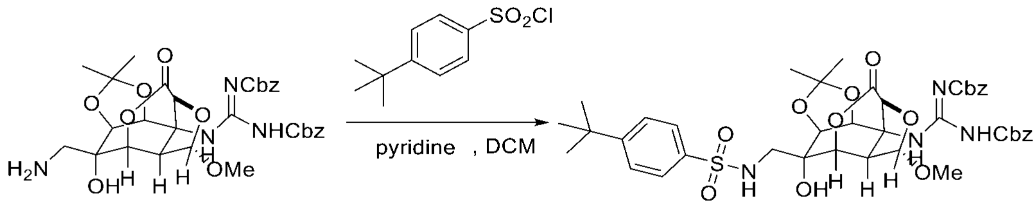


B3 218-1

**Compound B3** (130 mg, 0.20 mmol), p-tert-butylbenzenesulfonyl chloride (57 mg, 0.24 mmol) and pyridine (64 mg, 0.81 mmol) were dissolved in dichloromethane (5 mL), and the reaction mixture was stirred at 17 °C for 1 h. After the reaction was completed, water was added to the reaction solution, which was extracted with dichloromethane (10 mL×3). The organic phases were combined, washed with saturated brine once, separated. The organic phase was dried over anhydrous sodium sulfate, filtered, and the filtrate was concentrated and dried to give a residue. The residue was purified by normal phase column (ethyl acetate/petroleum ether = 0-25.4%) to give **Compound 218-1** (127 mg, yield: 74.8%). LCMS (m/z): 837.3[M+1]$^+$.

Step 2: Synthesis of **Compound 218**

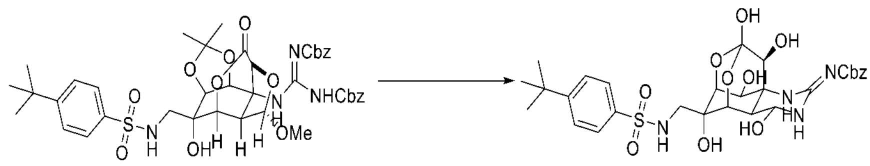

218-1 218

**Compound 218-1** (127 mg, 0.15 mmol) was dissolved in a mixed solution of water: trifluoroacetic acid = 1:1, and the reaction mixture was stirred at 60 °C for 20 h. After the reaction was completed, the reaction mixture was directly concentrated to give a crude product. The crude product was purified by normal phase column chromatography (methanol/dichloromethane = 0-5.3%) to give **Compound 218** (24 mg, yield: 24.4%).

$^1$H NMR (400 MHz, MeOD) $\delta$ 7.85(d, $J$ = 8.5 Hz, 2H), 7.65(d, $J$ = 8.6 Hz, 2H), 7.43-7.18(m, 5H), 5.56(d, $J$ = 9.4 Hz, 1H), 5.08(s, 2H), 4.15(s, 2H), 3.92(s, 2H), 3.56-3.38(m, 2H), 2.24(d, $J$ = 9.5 Hz, 1H), 1.39(s, 9H); LCMS (m/z):649.2[M+1]$^+$.

Example 185: Synthesis of 4-(tert-butyl)-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 219**)

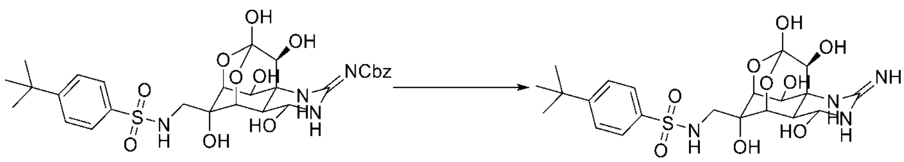

218 219

**Compound 218** (20 mg, 0.03 mmol) was dissolved in methanol (10 mL), to which 10% Pd/C (10 mg) was added. The reaction mixture was purged with hydrogen, and stirred at 25 °C for 4 h. After the reaction was completed, the reaction mixture was filtered, and the filtrate was concentrated to dryness to give **Compound 219** (8.1 mg, yield: 51.1%).

$^1$H NMR (400 MHz, MeOD) $\delta$ 7.83(d, $J$ = 8.5 Hz, 2H), 7.64(d, $J$ = 8.5 Hz, 2H), 5.53(d, $J$ = 9.4 Hz, 1H), 4.16(s, 2H), 3.96-3.86(m, 2H), 3.45(s, 2H), 2.27-2.21(m, 1H), 1.37(s, 9H); LCMS (m/z): 515.2[M+1]$^+$.

Example 186: Synthesis of benzyl ((4R,4aR,5R,6S,7S,9S,10S,10aR,11S,E)-6-(((3,4-difluorophenyl)sulfonamido)methyl)-4,6,9,10,11-pentahydroxyoctahydro-5,9-epoxy-7,10a-methanooxocino[4,5-d]pyrimidin-2(1H)-ylidene)carbamate (**Compound 220**) and 3,4-difluoro-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 221**)

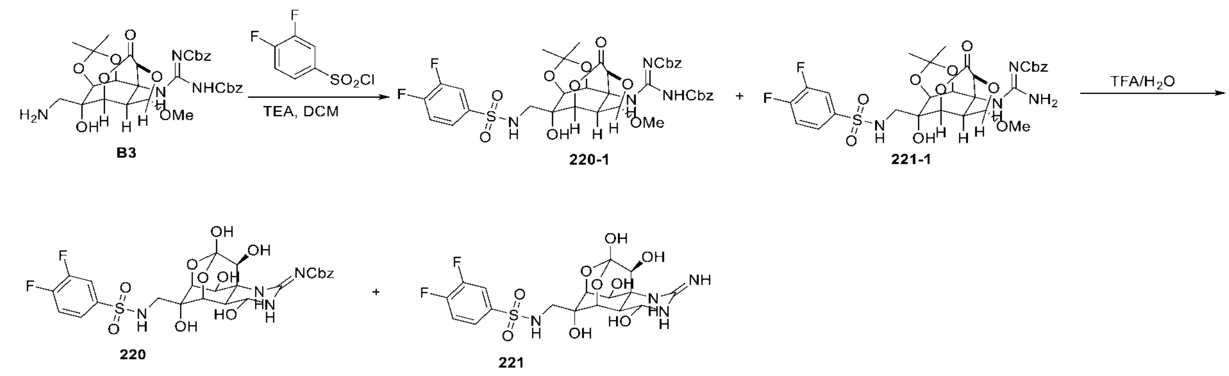

B3 220-1 221-1 220 221

Step 1: Synthesis of **Compound 220-1** and **Compound 221-1**

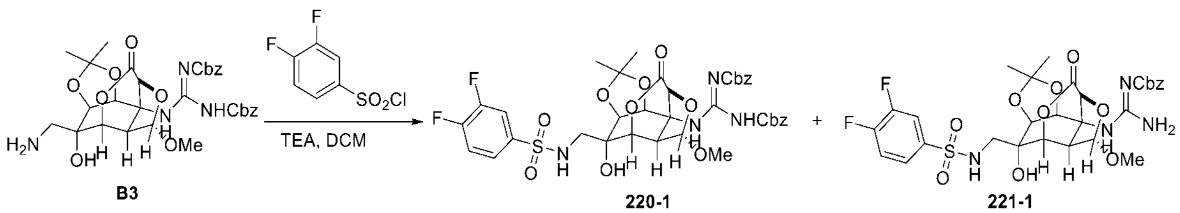

B3 220-1 221-1

**Compound B3** (200 mg, 0.31 mmol) was dissolved in dry dichloromethane (10 mL), to which triethylamine (63 mg, 0.62 mmol) was added. The reaction mixture was cooled to 0 °C in an ice bath, to which compound 3,4-difluorobenzenesulfonyl chloride (68 mg, 0.56 mmol) was added, and the reaction was carried out for 30 min under 0 °C, and the reaction mixture was stirred at room temperature for 5 h. LC-MS was used to monitor completion of the reaction. Saturated sodium chloride (3 mL) was added dropwise to the reaction solution, which was diluted with water (30 mL), extracted with ethyl acetate (15 mL×3). The organic phase was washed with saturated sodium chloride (20 mL), and the combined organic phases were dried over anhydrous sodium sulfate and concentrated to give a residue. The residue was purified by flash column chromatography (methanol/dichloromethane = 0-8%) to give **Compound 220-1** (76 mg, yield: 29.8%) and **Compound 221-1** (68 mg, yield: 31.9%) as white solid.

**Compound 220-1:** LCMS (m/z): 817.2[M+1]$^+$; **Compound 221-1:** LCMS (m/z): 683.2[M+1]$^+$.

Step 2: Synthesis of **Compound 220** and **Compound 221**

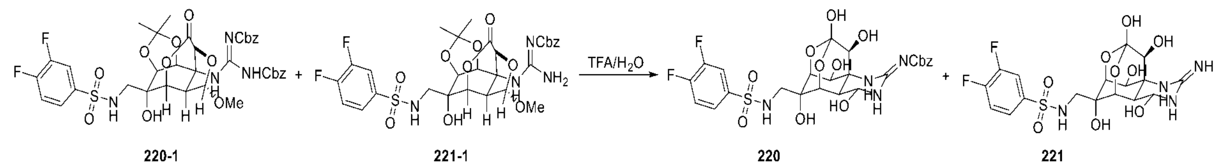

220-1 221-1 220 221

A mixture of **Compound 220-1** and **Compound 221-1** (140 mg, 0.17 mmol) was dissolved in a mixed solution of TFA (5 mL) and water (15 mL), and the reaction was carried out for 16 h under 60 °C. MS was used to monitor the reaction until it was completed. The reaction solution was concentrated to give a crude product. The crude product was purified by prep-HPLC ((A: 0.1% AcOH): (B: MeOH) = 0-8%) to give **Compound 220** (7.3 mg, yield: 5.7%) and **Compound 221** (21 mg, yield: 20.7%) as white solid.

**Compound 220:**

$^1H$ NMR (400 MHz, MeOD) $\delta$ 7.85(ddd, $J$ = 9.7, 7.4, 2.2 Hz, 1H), 7.79-7.72(m, 1H), 7.49(dd, $J$ = 18.1, 8.4 Hz, 1H), 7.42-7.26(m, 5H), 5.57(d, $J$ = 9.1 Hz, 1H), 5.13(s, 2H), 4.13(d, $J$ = 17.5 Hz, 2H), 3.89(d, $J$ = 11.5 Hz, 2H), 3.56-3.45(m, 2H), 2.25(d, $J$ = 9.5 Hz, 1H); LCMS (m/z): 629.1[M+1]$^+$.

**Compound 221:**

LCMS (m/z): 495.1[M+1]$^+$.

Example 187: Synthesis of benzyl ((4R,4aR,5R,6S,7S,9S,10S,10aR,11S,E)-4,6,9,10,11-pentahydroxy-6-((pyridine-3-sulfonamido)methyl)octahydro-5,9-epoxy-7,10a-methanooxocino[4,5-d]pyrimidin-2(1H)-ylidene)carbamate (**Compound 222**)

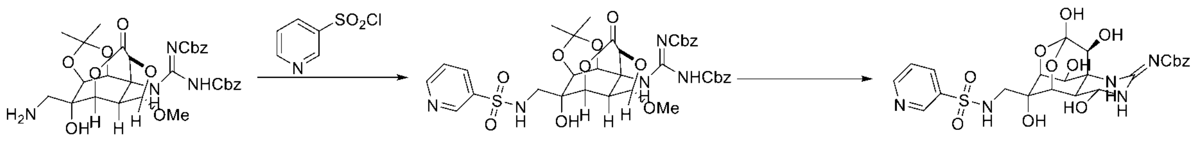

B3 222-1 222

Step 1: Synthesis of **Compound 222-1**

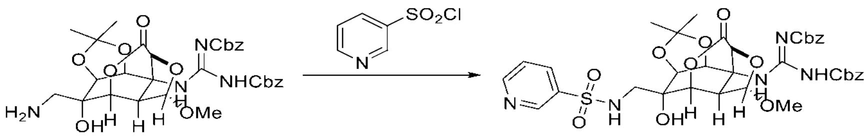

B3 222-1

**Compound B3** (130 mg, 0.20 mmol), pyridine-3-sulfonyl chloride (39.2 mg, 0.21 mmol) and pyridine (26.9 mg, 0.36 mmol) were dissolved in DCM (5 mL), and the reaction mixture was stirred for 3 h under nitrogen protection at 25 °C.. After the reaction was completed, water (5 mL) was added to the reaction solution, which was extracted with DCM (5 mL×3), and the organic phases were combined and concentrated to give a residue. The residue was purified by normal phase column chromatography (ethyl acetate/petroleum ether = 5%-40%) to give **Compound 222-1** as off-white solid (128 mg, yield: 80.7%). LCMS (m/z): 782.2[M+1]$^+$.

Step 2: Synthesis of **Compound 222**

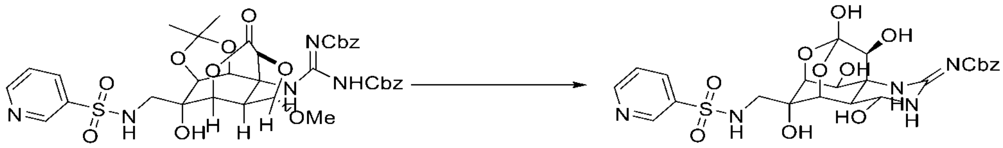

222-1 222

**Compound 222-1** (120 mg, 0.15 mmol) was dissolved in a mixed solution of TFA: $H_2O$ = 2:1 (4 mL). Under nitrogen protection, the reaction mixture was heated to 60 °C and stirred for 16 h. After the reaction was completed, the reaction solution was concentrated and dried to give a residue. The residue was purified by normal phase column chromatography (ethyl acetate/petroleum ether = 5%-40%) to give **Compound 222** as white solid (15.3 mg, yield: 16.7%).

$^1H$ NMR (400 MHz, MeOD) $\delta$ 9.04(d, $J$ = 1.8 Hz, 1H), 8.82-8.72(m, 1H), 8.35-8.27(m, 1H), 7.63(dd, $J$ = 8.1, 4.9 Hz, 1H), 7.46-7.32(m, 5H), 5.66(d, $J$ = 9.4 Hz, 1H), 5.30(s, 2H), 4.20(dd, $J$ = 6.3, 1.7 Hz, 1H), 4.14(d, $J$ = 17.6 Hz, 1H), 3.96(d, $J$ = 10.7 Hz, 1H), 3.91(d, $J$ = 1.6 Hz, 1H), 3.56(d, $J$ = 8.1 Hz, 2H), 2.36(d, $J$ = 9.4 Hz, 1H); LCMS (m/z): 594.1[M+1]$^+$.

Example 188: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)pyridine-3-sulfonamide (**Compound 223**)

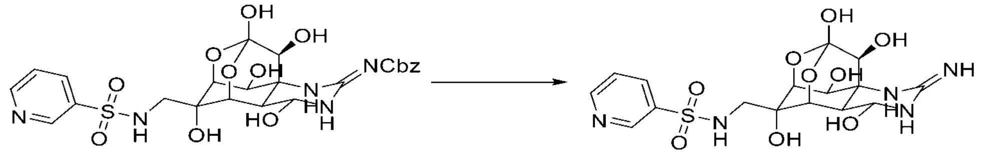

222 223

**Compound 222** (15 mg, 0.03 mmol) was dissolved in methanol (2 mL), to which 10% Pd/C (1 mg) was added. The reaction mixture was purged with hydrogen three times, and stirred under 25 °C for 8 h. After the reaction was completed, the reaction mixture was filtered. The filtrate was concentrated to dryness and then lyophilized to give **Compound 223** as off-white solid (8.6 mg, yield: 74.2%).

$^1H$ NMR (400 MHz, $D_2O$) $\delta$ 8.97(s, 1H), 8.73(d, $J$ = 4.7 Hz, 1H), 8.31(d, $J$ = 8.2 Hz, 1H), 7.66(dd, $J$ = 8.0, 5.0 Hz, 1H), 5.36(d, $J$ = 9.4 Hz, 1H), 4.25-4.17(m, 2H), 4.10-3.96(m, 2H), 3.91-3.81(m, 2H), 2.21(d, $J$ = 9.5 Hz, 1H); LCMS (m/z): 460.1[M+1]$^+$.

Example 189: Synthesis of benzyl ((4R,4aR,5R,6S,7S,9S,10S,10aR,11S,E)-4,6,9,10,11-pentahydroxy-6-(((5-(trifluoromethyl)pyridine)-2-sulfonamido)methyl)octahydro-5,9-epoxy-7,10a-methanooxocino[4,5-d]pyrimidin-2(1H)-ylidene) carbamate (**Compound 224**)

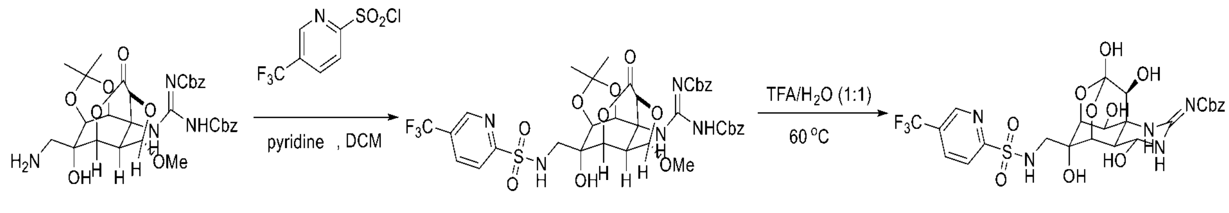


B3 224-1 224

Step 1: Synthesis of **Compound 224-1**

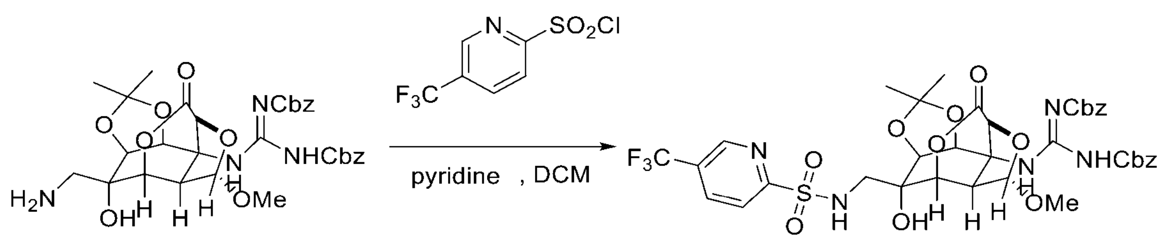


B3 224-1

**Compound B3** (130 mg, 0.20 mmol) was dissolved in DCM (5 mL), to which pyridine (47.46 mg, 0.6 mmol) was added, the reaction was cooled to 0 °C under nitrogen protection, to which 4-trifluoromethylbenzenesulfonyl chloride (74.53 mg, 0.3 mmol) was added dropwise, and then the reaction was carried out overnight at room temperature. HPLC was used to detect the complete reaction of most raw materials. The reaction solution was poured into water (50 mL), and extracted with DCM (30 mL×3). The organic phases were combined, washed with saturated brine (50 mL), and dried over anhydrous sodium sulfate. Filtration was conducted and the filtrate was concentrated and dried to give a residue. The residue was purified by flash column chromatography (EA/PE = 0-30%) to give **Compound 224-1** as a white foam solid (82 mg, yield: 47.5%). LCMS (m/z): 850.2[M+1]$^+$.

Step 2: Synthesis of **Compound 224**

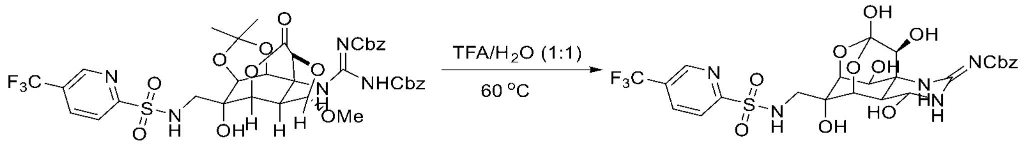


**224-1** **224**

**Compound 224-1** (80 mg, 0.09 mmol) was dissolved in a mixed solution of water (10 mL) and TFA (10 mL), and the reaction mixture was heated to 60 °C under nitrogen protection overnight. HPLC was used to monitor complete reaction of the raw materials. The reaction solution was concentrated and dried, and the residue was purified by flash column chromatography (MeOH/DCM = 0-10%) to give **Compound 224** as white solid (10 mg, yield: 16%).

$^1$H NMR (400 MHz, $D_2O$) $\delta$ 9.03(s, 1H), 8.39(d, $J$ = 8.3 Hz, 1H), 8.20(d, $J$ = 8.2 Hz, 1H), 7.45-7.17(m, 5H), 5.52(d, $J$ = 9.4 Hz, 1H), 5.05(s, 2H), 4.11(s, 2H), 3.88(s, 2H), 3.76(s, 2H), 2.18(d, $J$ = 9.2 Hz, 1H); LCMS (m/z): 662.1[M+1]$^+$.

Example 190: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)-5-(trifluoromethyl)pyridine-2-sulfonamide (**Compound 225**)

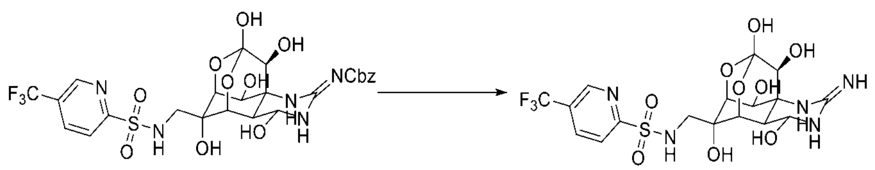

**224** **225**

**Compound 224** (10 mg, 0.02 mmol) was dissolved in methanol (10 mL), to which 10% Pd/C (20 mg) was added. The reaction mixture was purged with hydrogen, and hydrogen was introduced into the reaction solution for 3 h. HPLC was used to monitor complete reaction of the raw materials. The reaction solution was filtered, and the filter cake was rinsed with 0.05 M AcOH aqueous solution. The filtrate was concentrated to give a crude product, which was slurried with methyl tert-butyl ether (10 mL), allowed to stand, and the supernatant was removed to give a residue. The residue was concentrated and dried to give **Compound 225** as white solid (8 mg, yield: 99.6%).

$^1$H NMR (400 MHz, MeOD) $\delta$ 9.04(s, 1H), 8.42(d, $J$ = 8.0 Hz, 1H), 8.21(d, $J$ = 8.2 Hz, 1H), 5.51(d, $J$ = 9.4 Hz, 1H), 4.14(s, 2H), 3.91(s, 2H), 3.76(s, 2H), 2.20(d, $J$ = 9.4 Hz, 1H); LCMS (m/z): 528.1[M+1]$^+$.

Example 191: Synthesis of benzyl ((4R,4aR,5R,6S,7S,9S,10S,10aR,11S,E)-6-(([1,1'-biphenyl]-4-sulfonamido)methyl)-4,6,9,10,11-pentahydroxyoctahydro-5,9-epoxy-7,10a-methanooxocino[4,5-d]pyrimidin-2(1H)-ylidene)carbamate (**Compound 226**)

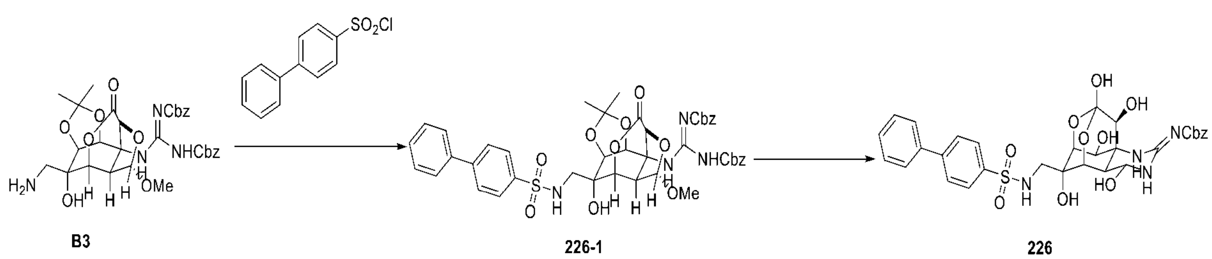

B3 **226-1** **226**

Step 1: Synthesis of **Compound 226-1**

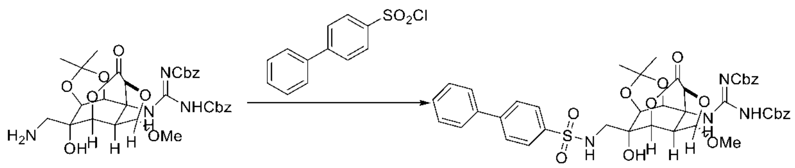

B3

226-1

**Compound B3** (130 mg, 0.20 mmol), [1,1'-biphenyl]-4-sulfonyl chloride (82.3 mg, 0.30 mmol) and pyridine (38.9 mg, 0.50 mmol) were dissolved in DCM (5 mL), and the reaction mixture was stirred under nitrogen protection at 25 °C for 3 h. After the reaction was completed, water (5 mL) was added to the reaction solution, which was extracted with DCM (5 mL×3), and the organic phases were combined and concentrated to give a residue. The residue was purified by normal phase column chromatography (ethyl acetate/petroleum ether = 5%-40%) to give **Compound 226-1** as off-white solid (145 mg, yield: 83.4%). LCMS (m/z): 857.3[M+1]$^+$.

Step 2: Synthesis of **Compound 226**

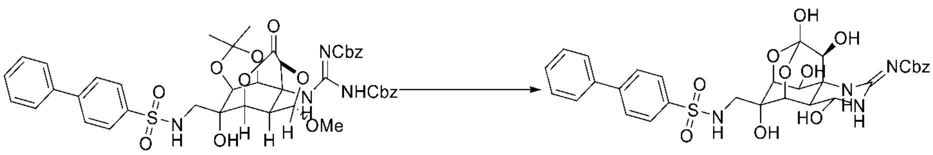

226-1

226

**Compound 226-1** (140 mg, 0.16 mmol) was dissolved in a mixed solution of TFA: $H_2O$ = 2:1 (4 mL), and heated to 60 °C under nitrogen protection and stirred for 16 h. After the reaction was completed, the reaction solution was purified by reverse phase column chromatography (methanol/0.1% acetic acid aqueous solution = 5%-8%) to give **Compound 226** as white solid (8.0 mg, yield: 7.3%).

$^1$H NMR (400 MHz, MeOD) $\delta$8.00(d, $J$ = 8.5 Hz, 2H), 7.86(d, $J$ = 8.4 Hz, 2H), 7.71(d, $J$ = 7.2 Hz, 2H), 7.51(t, $J$ = 7.5 Hz, 2H), 7.47-7.26(m, 6H), 5.56(d, $J$ = 9.4 Hz, 1H), 5.08(s, 2H), 4.16(s, 2H), 3.92(s, 2H), 3.56-3.47(m, 2H), 2.24(d, $J$ = 9.7 Hz, 1H); LCMS (m/z): 669.2[M+1]$^+$.

Example 192: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)-[1,1'-biphenyl]-4-sulfonamide (**Compound 227**)

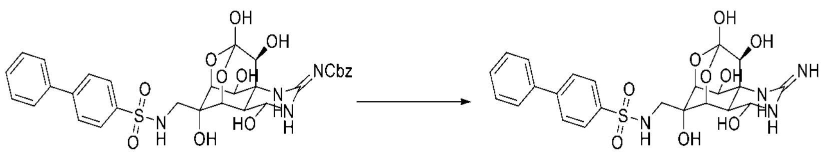

226

227

**Compound 226** (6.0 mg, 0.01 mmol) was dissolved in methanol (2 mL), to which 10% Pd/C (1 mg) was added. The reaction mixture was purged with hydrogen three times, and stirred under 25 °C for 8 h. After the reaction was completed, the reaction mixture was filtered. The filtrate was concentrated to dryness, and lyophilized to give **Compound 227** as off-white solid (3.0 mg, yield: 62.5%).

$^1$H NMR (400 MHz, DMSO) $\delta$ 8.01-7.85(m, 4H), 7.76(d, $J$ = 7.3 Hz, 2H), 7.52(t, $J$ = 7.5 Hz, 2H), 7.44(t, $J$ = 7.3 Hz, 1H), 5.31(dd, $J$ = 12.7, 7.9 Hz, 1H), 4.15-3.76(m, 2H), 3.66(s, 2H), 3.19-2.97(m, 2H), 2.08-1.88(m, 1H); LCMS (m/z): 535.1 [M+1]$^+$.

Example 193: Synthesis of benzyl ((4R,4aR,5R,6S,7S,9S,10S,10aR,11S,E)-6-(((4'-fluoro-[1,1'-biphenyl])-4-sulfonamido)methyl)-4,6,9,10,11-pentahydroxyoctahydro-5,9-epoxy-7,10a-methanooxocino[4,5-d]pyrimidin-2(1H)-ylidene)carbamate (**Compound 228**)

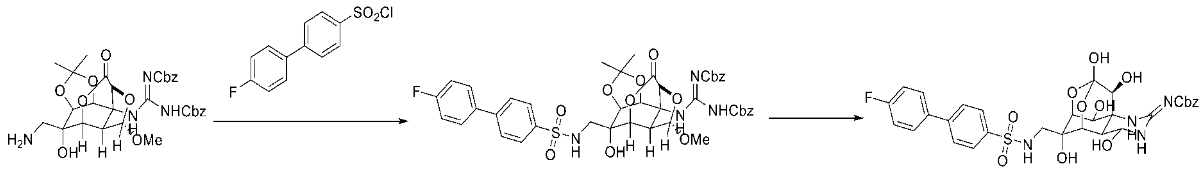

B3 228-1 228

Step 1: Synthesis of **Compound 228-1**

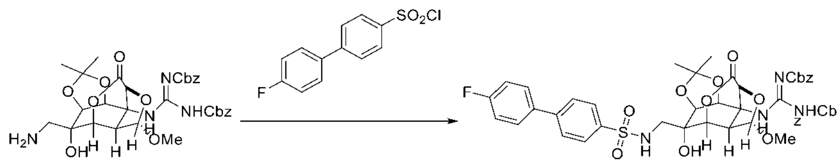

B3 228-1

**Compound B3** (130 mg, 0.20 mmol), 4'-fluoro-[1,1'-biphenyl]-4-sulfonyl chloride (82.3 mg, 0.30 mmol) and pyridine (38.9 mg, 0.50 mmol) were dissolved in DCM (5 mL), and the reaction mixture was stirred for 3 h under nitrogen protection at 25 °C.. After the reaction was completed, water (5 mL) was added to the reaction solution, which was extracted with DCM (5 mL×3), and the organic phases were combined and concentrated to give a residue. The residue was purified by normal phase column chromatography (ethyl acetate/petroleum ether = 5%-40%) to give **Compound 228-1** as off-white solid (150 mg, yield: 84.6%). LCMS (m/z): 875.3[M+1]+.

Step 2: Synthesis of **Compound 228**

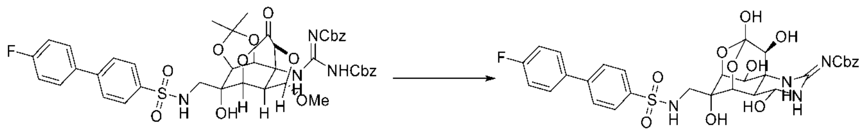

228-1 228

**Compound 228-1** (140 mg, 0.16 mmol) was dissolved in a mixed solution of TFA: $H_2O$ = 2:1 (4 mL), and the reaction mixture was heated to 60 °C under nitrogen protection and stirred for 16 h. After the reaction was completed, the reaction solution was concentrated to dryness to give a residue. The residue was purified by reverse phase column chromatography (methanol/0.1% acetic acid aqueous solution = 5%-8%) to give **Compound 228** as white solid (24.3 mg, yield: 22.1%).

$^1$H NMR (400 MHz, MeOD) $\delta$ 8.00(d, $J$ = 8.5 Hz, 2H), 7.84(d, $J$ = 8.4 Hz, 2H), 7.77-7.70(m, 2H), 7.43-7.20(m, 7H), 5.56(d, $J$ = 9.5 Hz, 1H), 5.08(s, 2H), 4.16(s, 2H), 3.92(s, 2H), 3.50(s, 2H), 2.24(d, $J$ = 9.5 Hz, 1H); LCMS (m/z): 687.2[M+1]+.

Example 194: Synthesis of 4'-fluoro-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)-[1,1'-biphenyl]-4-sulfonamide (**Compound 229**)

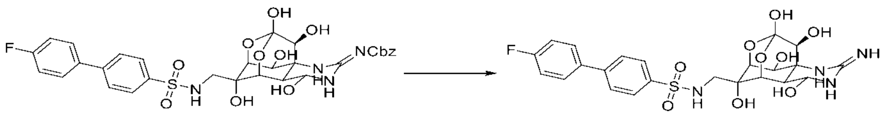

228 229

**Compound 228** (10 mg, 0.01 mmol) was dissolved in methanol (2 mL), to which 10% Pd/C (1 mg) was added. The reaction mixture was purged with hydrogen three times, and stirred under 25 °C for 8 h. After the reaction was completed, the reaction mixture was filtered. The filtrate was concentrated to dryness, and then lyophilized to give **Compound 229** as off-white solid (3.5 mg, yield: 43.7%).

$^1H$ NMR (400 MHz, MeOD) $\delta$ 7.99(t, $J$ = 9.2 Hz, 2H), 7.85(d, $J$ = 8.5 Hz, 2H), 7.78-7.70(m, 2H), 7.31-7.22(m, 2H), 5.54(d, $J$ = 9.4 Hz, 1H), 4.16(s, 2H), 4.03(s, 2H), 3.50(d, $J$ = 3.1 Hz, 2H), 2.26(d, $J$ = 9.7 Hz, 1H); LCMS (m/z): 553.1[M+1]+.

Example 195: Synthesis of benzyl ((4R,4aR,5R,6S,7S,9S,10S,10aR,11S,E)-4,6,9,10,11-pentahydroxy-6-((naphthalene-2-sulfonamido)methyl)octahydro-5,9-epoxy-7,10a-methanooxocino[4,5-d]pyrimidin-2(1H)-ylidene)carbamate (**Compound 230**)

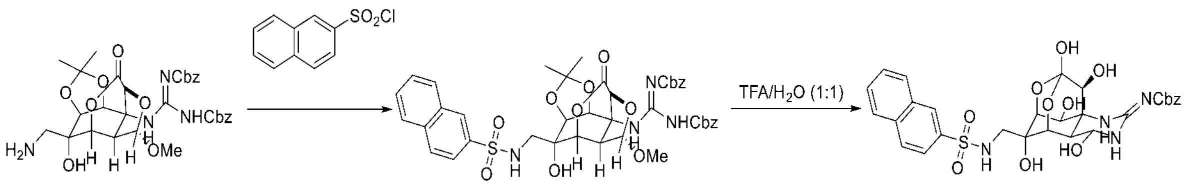


B3 230-1 230

Step 1: Synthesis of **Compound 230-1**

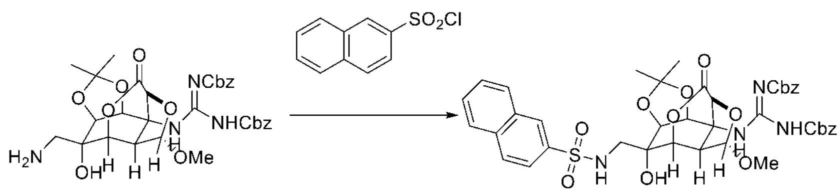

B3 230-1

**Compound B3** (130 mg, 0.20 mmol), 2-naphthalenesulfonyl chloride (69 mg, 0.30 mmol) and pyridine (64 mg, 0.81 mmol) were dissolved in dichloromethane (5 mL), and the reaction mixture was stirred at 18 °C for 1 h. After the reaction was completed, water was added to the reaction solution, which was extracted with dichloromethane (10 mL×3). The organic phases were combined, washed once with saturated brine, separated. The organic phased was dried over anhydrous sodium sulfate, and filtered.. The filtrate was concentrated and dried to give a residue. The residue was purified by normal phase column chromatography (ethyl acetate/petroleum ether = 0-30%) to give **Compound 230-1** (128 mg, yield: 75.9%). LCMS (m/z):831.3[M+1]+.

Step 2: Synthesis of **Compound 230**

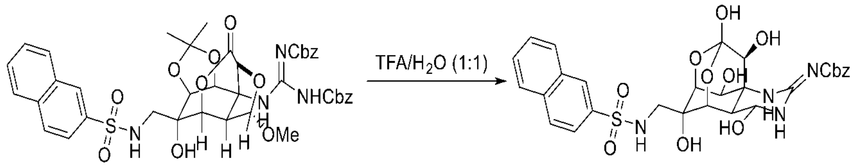


230-1

230

**Compound 230-1** (128 mg, 0.15 mmol) was dissolved in a mixed solution of water: trifluoroacetic acid = 1:1 (10 mL), and the reaction mixture was stirred at 60 °C for 20 h. After the reaction was completed, the reaction mixture was directly concentrated to give a crude product, which was subjected to normal phase column chromatography (methanol/dichloromethane = 0-5.5%) to give **Compound 230** (13 mg, yield: 13.1%).

$^1$H NMR (400 MHz, MeOD) $\delta$ 8.50(s, 1H), 8.12-8.06(m, 2H), 8.01(d, $J$ = 7.7 Hz, 1H), 7.93(dd, $J$ = 8.7, 1.8 Hz, 1H), 7.73-7.64(m, 2H), 7.41-7.26(m, 5H), 5.55(d, $J$ = 9.3 Hz, 1H), 5.07(s, 2H), 4.16(s, 2H), 3.91(d, $J$ = 9.0 Hz, 2H), 3.50(s, 2H), 2.24(d, $J$ = 9.5 Hz, 1H); LCMS (m/z):643.2[M+1]$^+$.

Example 196: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)naphthalene-2-sulfonamide (**Compound 231**)

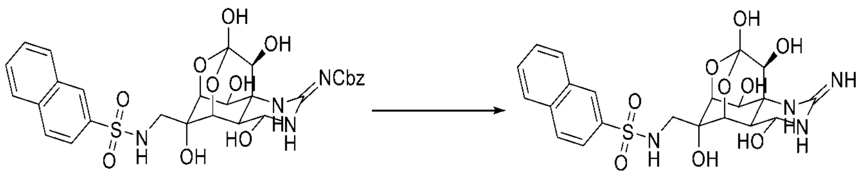

230

231

**Compound 230** (9 mg, 0.01 mmol) was dissolved in methanol (5 mL), to which 10% Pd/C (4.5 mg) was added. The reaction mixture was purged with hydrogen, and stirred at 25 °C for 4 h. After the reaction was completed, the reaction mixture was filtered. The filtrate was concentrated and dried to give **Compound 231** (2 mg, yield: 28.2%). LCMS (m/z): 509.1[M+1]$^+$.

Example 197: Synthesis of benzyl ((4R,4aR,5R,6S,7S,9S,10S,10aR,11S,E)-6-((1H-indole-3-sulfonamido)methyl)-4,6,9,10,11-pentahydroxyoctahydro-5,9-epoxy-7,10a-methanooxocino[4,5-d]pyrimidin-2(1H)-ylidene)carbamate (**Compound 232**)

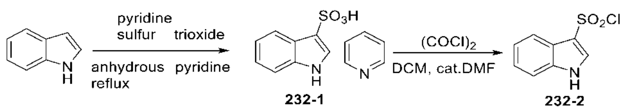


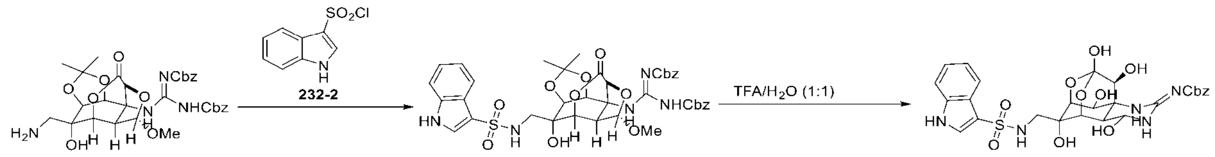


B3

232-3

232

Step 1: Synthesis of **Compound 232-1**

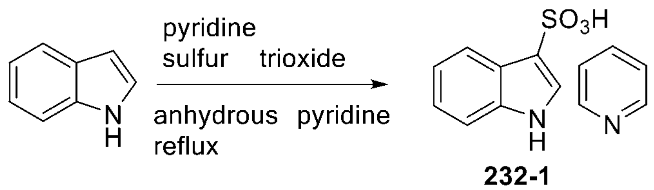


**232-1**

1H-indole (400 mg, 3.42 mmol) and pyridine sulfur trioxide (815.2 mg, 5.12 mmol) were dissolved in anhydrous pyridine (5 mL), and the reaction mixture was heated to reflux under nitrogen protection for 5 h. HPLC was used to monitor complete reaction of the raw materials. The reaction solution was cooled to room temperature, to which was added water (15 mL), and extracted with methyl tert-butyl ether (30 mL×2). The aqueous phase was concentrated and dried to give a residue. The residue was washed twice with toluene plus water to give crude **Compound 232-1** (1.1 g, yield > 100%). LCMS (m/z):198.0[M+1]$^+$.

Step 2: Synthesis of **Compound 232-2**

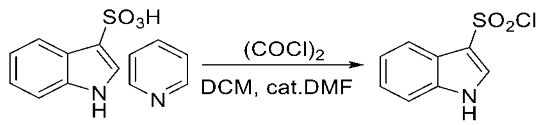


**232-1** **232-2**

**Compound 232-1** (1.1 g, 3.42 mmol, crude) was dissolved in a mixed solution of anhydrous dichloromethane (10 mL) and DMF (0.05 mL), and oxalyl chloride (1.01 g, 7.96 mmol) was added dropwise at room temperature. A large amount of bubbles were generated, the solid was gradually dissolved, and stirring was continued for 2 h. The reaction solution was concentrated and dried, to which was added ethyl acetate (50 mL), then ice water (30 mL), stirred, separated, and the aqueous phase was extracted with ethyl acetate (30 mL×2). The organic phases were combined, washed with saturated brine (40 mL), dried over anhydrous sodium sulfate, filtered, and concentrated to give crude **Compound 232-2** as light pink solid (613 mg, yield: 83.3%).

Step 3: Synthesis of **Compound 232-3**

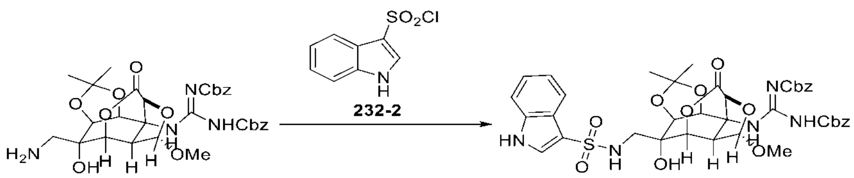


**B3** **232-3**

**Compound B3** (130 mg, 0.20 mmol) was dissolved in anhydrous dichloromethane (8 mL), cooled in an ice bath under nitrogen protection, to which pyridine (48.2 mg, 0.61 mmol) was added, and then crude **Compound 232-2** (65.6 mg, 0.30 mmol). After the addition was completed, the reaction mixture was stirred at room temperature for 1.5 h. HPLC was used to monitor the the reaction until it was completed. Water (10 mL) was added to the reaction solution, which was extracted with dichloromethane (10 mL×3). The organic phases were combined, washed with saturated brine (15 mL), dried over anhydrous sodium sulfate, filtered, and concentrated to give a crude product. The crude product was purified by Prep-TLC (EA/PE = 1:1) to give **Compound 232-3** as white solid (125 mg, yield: 75.1%). LCMS (m/z):820.2[M+1]$^+$.

Step 4: Synthesis of **Compound 232**

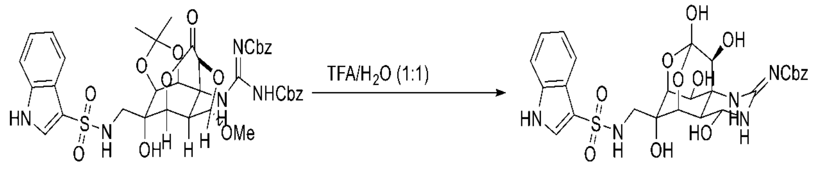


232-3 232

**Compound 232-3** (120 mg, 0.146 mmol) was dissolved in a mixed solution of $H_2O$ (5 mL) and TFA (5 mL), and the reaction was carried out at 60 °C for 24 h. HPLC was used to monitor complete reaction of the raw materials. The reaction solution was concentrated to dryness to give a residue. The residue was subjected to flash column chromatography (MeOH/DCM = 0-5%) to give **Compound 232** (23 mg, yield: 24.9%).

$^1$H NMR (400 MHz, MeOD) $\delta$ 7.92(d, $J$ = 7.7 Hz, 1H), 7.87(s, 1H), 7.49(d, $J$ = 8.0 Hz, 1H), 7.39-7.20(m, 7H), 5.53(d, $J$ = 9.4 Hz, 1H), 5.06(s, 2H), 4.13(s, 2H), 3.89(s, 2H), 3.53-3.36(m, 2H), 2.22(d, $J$ = 9.6 Hz, 1H); LCMS (m/z): 632.2 $[M+1]^+$.

Example 197: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)-1H-indole-3-sulfonamide (**Compound 233**)

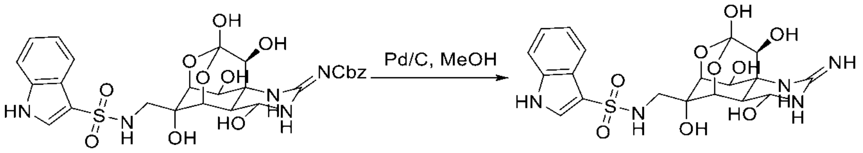


232 233

**Compound 232** (12 mg, 0.02 mmol) was dissolved in methanol (5mL), to which 10% Pd/C (6 mg) was added. The reaction mixture was purged with hydrogen, and stirred at 25 °C for 4 h. After the reaction was completed, the reaction mixture was filtered, and the filtrate was concentrated and dried to give **Compound 233** (6 mg, yield: 63.5%).

$^1$H NMR (400 MHz, DMSO) $\delta$ 7.90(dd, $J$ = 8.6, 2.2 Hz, 2H), 7.51(d, $J$ = 8.0 Hz, 1H), 7.25(t, $J$ = 7.1 Hz, 2H), 7.19(t, $J$ = 7.1 Hz, 1H), 5.95(s, 1H), 5.27(d, $J$ = 9.3 Hz, 1H), 4.77(s, 1H), 4.46(s, 1H), 3.97(d, $J$ = 13.3 Hz, 2H), 3.89-3.78(m, 3H), 2.01(d, $J$ = 9.2 Hz, 1H); LCMS (m/z): 498.1$[M+1]^+$.

Example 198: Synthesis of benzyl ((4R,4aR,5R,6S,7S,9S,10S,10aR,11S,E)-6-((1H-imidazole-2-sulfonamido)methyl)-4,6,9,10,11-pentahydroxyoctahydro-5,9-epoxy-7,10a-methanooxocino[4,5-d]pyrimidin-2(1H)-ylidene)carbamate (**Compound 234**)

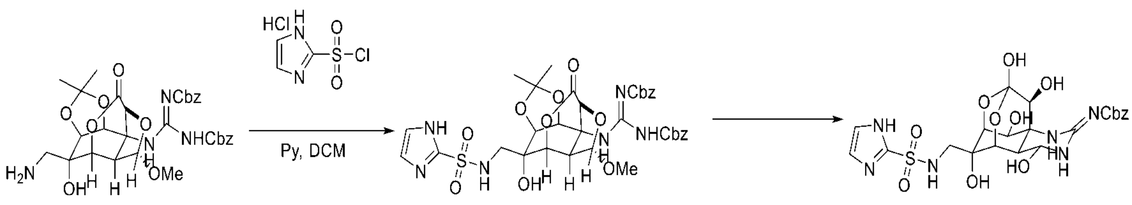


B3 234-1 234

Step 1: Synthesis of **Compound 234-1**

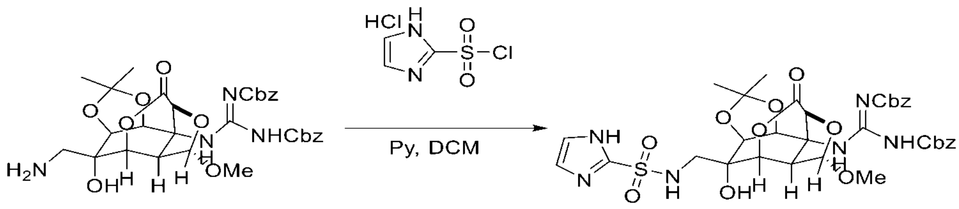


B3 234-1

**Compound B3** (110 mg, 0.17 mmol) was dissolved in a mixed solution of pyridine (68 mg, 0.86 mmol) and dichloromethane (5 mL), to which 2-sulfonyl chloride imidazole hydrochloride (42 mg, 0.21 mmol) was added in an ice bath, and then the reaction mixture was stirred under 17 °C for 2 h. After the reaction was completed, water (10 mL) was added to the reaction system, which was extracted with dichloromethane (10 mL×3). The organic phases were combined, washed with saturated brine (20 mL). The organic phase was dried over anhydrous sodium sulfate, filtered, and the filtrate was concentrated and dried to give a crude product. The crude product was purified by normal phase column chromatography (ethyl acetate/petroleum ether = 0-30%) to give **Compound 234-1** (100 mg, yield: 75.6%). LCMS (m/z):771.2[M+1]$^+$.

Step 2: Synthesis of **Compound 234**

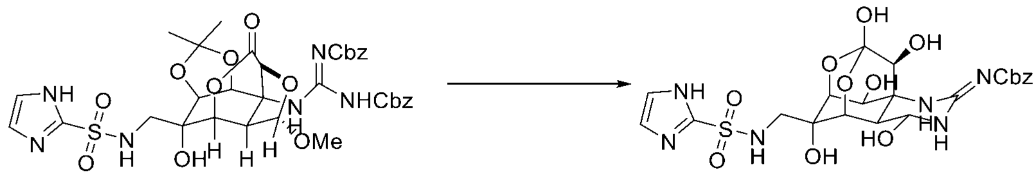

234-1 234

Compound **234-1** (100 mg, 0.13 mmol) was dissolved in a mixed solution of water (7 mL) and trifluoroacetic acid (7 mL), and the reaction mixture was stirred at 60 °C for 24 h. After the reaction was completed, the reaction solution was concentrated and dried to give a crude product. The crude product was purified by normal phase column chromatography (methanol/dichloromethane = 0-9.3%) and then purified by high performance liquid preparation chromatography to give **Compound 234** as white solid (12.1 mg, yield: 16%).

$^1$H NMR (400 MHz, MeOD) $\delta$ 7.42-7.27(m, 5H), 7.24(s, 2H), 5.56(d, $J$ = 9.3 Hz, 1H), 5.09(s, 2H), 4.14(s, 2H), 3.91(s, 2H), 3.74(s, 2H), 2.26(d, $J$ = 9.4 Hz, 1H); LCMS (m/z):583.1[M+1]$^+$.

Example 199: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)-1H-imidazole-2-sulfonamide (**Compound 235**)

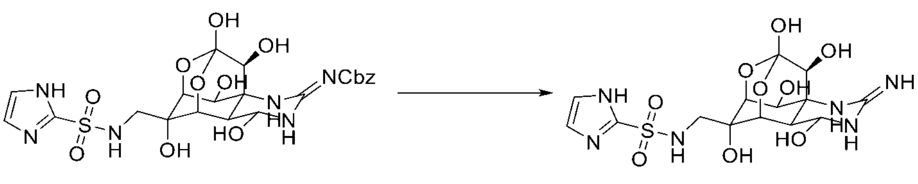

234 235

**Compound 234** (9.0 mg, 0.02 mmol) was dissolved in methanol (10mL), to which 10% Pd/C (5 mg) was added. The reaction mixture was purged with hydrogen, and stirred under hydrogen protection at 25 °C for 4 h. After the reaction was completed, the reaction mixture was filtered. The filtrate was concentrated and dried, and then lyophilized to give **Compound 235** as white solid (5.1 mg, yield: 73.6%).

$^1$H NMR (400 MHz, $D_2O$) $\delta$ 7.22(s, 2H), 5.36(d, $J$ = 9.4 Hz, 1H), 4.17(s, 1H), 4.08(s, 1H), 3.91(s, 1H), 3.83(s, 1H), 3.64-3.49(m, 2H), 2.22(d, $J$ = 9.4 Hz, 1H); LCMS (m/z):449.1[M+1]$^+$.

Example 200: Synthesis of benzyl ((4R,4aR,5R,6S,7S,9S,10S,10aR,11S,E)-6-(((((3S,5S,7S)-adamantan-1-yl)methyl) sulfonamido)methyl)-4,6,9,10,11-pentahydroxyoctahydro-5,9-epoxy-7,10a-methanooxocino[4,5-d]pyrimidin-2(1H)-ylidene)carbamate (**Compound 236**)

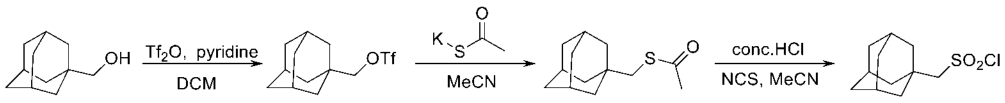


**236-1** **236-2** **236-3** **236-4**

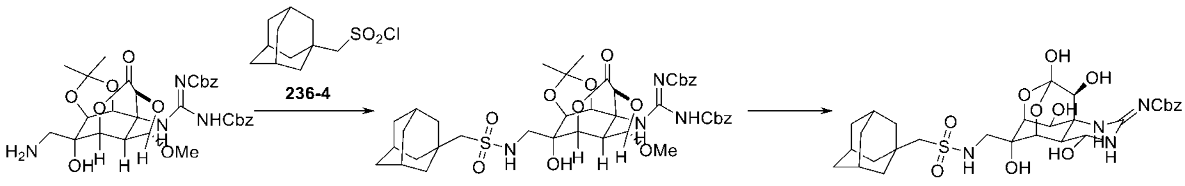

**236-4**

**B3** **236-5** **236**

Step 1: Synthesis of **Compound 236-2**

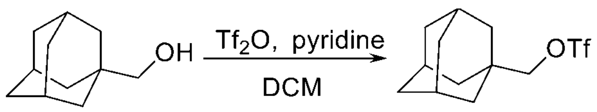


**236-1** **236-2**

**Compound 236-1** (1.0 g, 6.02 mmol) was dissolved in a mixed solution of pyridine (1.2 g, 15.05 mmol) and dichloromethane (10 mL), to which trifluoromethanesulfonic anhydride (2.5 g, 9.03 mmol) was added under nitrogen protection and ice bath, and then the reaction was continued to be stirred at room temperature for 1 h. After the reaction was completed, water (20 mL) was added to the reaction system, which was extracted with dichloromethane (20 mL×3). The organic phases were combined, dried over anhydrous sodium sulfate, filtered and concentrated to give a crude product. The crude product was purified by normal phase column chromatography (ethyl acetate/petroleum ether = 5%-30%) to give **Compound 236-2** as white solid (1.7 g, yield: 94.8%). LCMS (m/z): 299.1[M+1]$^+$.

Step 2: Synthesis of **Compound 236-3**

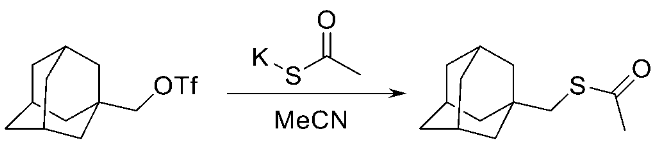


**236-2** **236-3**

**Compound 236-2** (1.0 g, 3.4 mmol) was dissolved in acetonitrile (10 mL), to which potassium thioacetate (455 mg, 4.0 mmol) was added in batches under nitrogen protection and ice bath, then the temperature was raised to room temperature, and the reaction was continued to be stirred for 2 h. After the reaction was completed, water (20 mL) was added to the reaction solution, which was extracted with ethyl acetate (20 mL×3). The organic phases were combined, washed with saturated brine (30 mL), dried over anhydrous sodium sulfate, filtered, and concentrated to give a crude product. The crude product was purified by column chromatography (ethyl acetate/petroleum ether = 0-10%) to give **Compound 236-3** as white solid (600 mg, yield: 79.8%).

$^1$H NMR (400 MHz, $CDCl_3$) $\delta$ 2.73(s, 2H), 2.35(s, 3H), 1.96(s, 3H), 1.69(d, $J$ = 12.2 Hz, 3H), 1.64-1.57(m, 3H),

1.50(d, $J$ = 2.4 Hz, 6H). LCMS (m/z): 225.1[M+1]$^+$.

Step 3: Synthesis of **Compound 236-4**

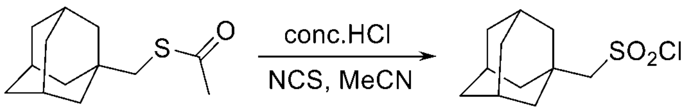


**236-3** **236-4**

N-chlorosuccinimide (NCS, 1.18 g, 8.8 mmol) was dissolved in acetonitrile (5 mL), to which 2.0 M dilute hydrochloric acid (1 mL) was added, and the reaction mixture was stirred at room temperature for 10 min. Then **Compound 236-3** (500 mg, 2.23 mmol) was added, and the reaction mixture was stirred at room temperature for 2 h. After the reaction was completed, water (20 mL) was added to the reaction solution to precipitate a white solid, which was filtered. The filter cake was dissolved in dichloromethane, dried over anhydrous sodium sulfate, and concentrated to give **Compound 236-4** as white solid (350 mg, yield: 63.1%).

$^1$H NMR (400 MHz, $CDCl_3$) $\delta$ 2.93(s, 2H), 2.05(s, 3H), 1.84(d, $J$ = 2.6 Hz, 6H), 1.78-1.63(m, 6H); LCMS (m/z): 249.1, 251.1[M+1]$^+$.

Step 4: Synthesis of **Compound 236-5**

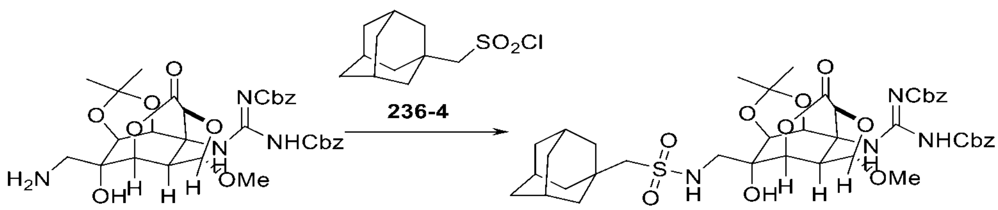


**B3** **236-5**

**Compound B3** (100 mg, 0.16 mmol) was dissolved in a mixed solution of pyridine (35.2 mg, 0.45 mmol) and dichloromethane (5 mL), and **Compound 236-4** (42.9 mg, 0.17 mmol) was added in an ice bath. Under nitrogen protection, the reaction mixture was stirred at room temperature for 3 h. After the reaction was completed, water (5 mL) was added to the reaction solution, which was extracted with dichloromethane (5 mL×3). The organic phases were combined, dried over anhydrous sodium sulfate, filtered, and the filtrate was concentrated and dried to give a crude product. The crude product was purified by normal phase column chromatography (ethyl acetate/petroleum ether = 5%-30%) to give **Compound 236-5** as off-white solid (122 mg, yield: 91.7%). LCMS (m/z): 853.3[M+1]$^+$.

Step 5: Synthesis of **Compound 236**

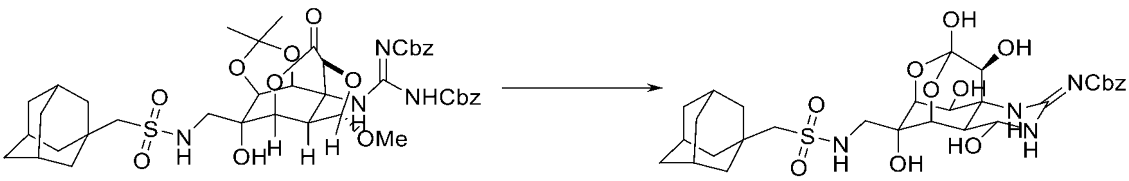

**236-5** **236**

**Compound 236-5** (122 mg, 0.14 mmol) was dissolved in a mixed solution of water (2 mL) and trifluoroacetic acid (4 mL), and the reaction mixture was heated to 60 °C under nitrogen protection and stirred for 16 h. After the reaction was completed, the reaction solution was concentrated and dried to give a crude product. The crude product was purified by normal phase column chromatography (methanol/dichloromethane = 5%-8%) to give **Compound 236** as white solid (24.3

mg, yield: 25.6%).

$^1H$ NMR (400 MHz, MeOD) $\delta$ 7.40-7.24(m, 5H), 5.55(d, $J$ = 9.3 Hz, 1H), 5.06(s, 2H), 4.16(s, 2H), 4.00-3.84(m, 2H), 3.76-3.56(m, 2H), 2.98(s, 2H), 2.22(d, $J$ = 9.4 Hz, 1H), 1.98(s, 3H), 1.85(d, $J$ = 2.2 Hz, 6H), 1.74(q, $J$ = 12.4 Hz, 6H). LCMS (m/z): 665.2$[M+1]^+$.

Example 201: Synthesis of 1-((3S,5S,7S)-adamantan-1-yl)-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)methanesulfonamide (**Compound 237**)

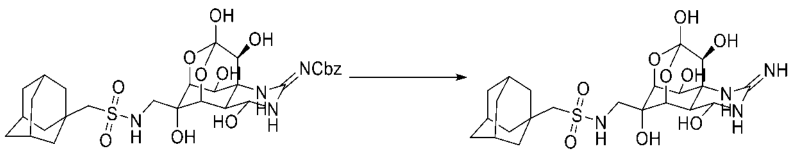

236 237

**Compound 236** (10 mg, 0.02 mmol), methanol (2 mL) and 10% Pd/C (1 mg) were added to a reaction flask. The reaction mixture was purged with hydrogen three times and stirred at room temperature for 8 h. After the reaction was completed, Pd/C was removed by filtration. The filter cake was rinsed with methanol three times, the filtrate was concentrated to dryness, and then lyophilized to give **Compound 237** as off-white solid (4.6 mg, yield: 57.6%).

$^1H$ NMR (400 MHz, MeOD) $\delta$ 5.48-5.42(m, 1H), 4.12-4.06(m, 2H), 3.91-3.85(m, 1H), 3.85-3.80(m, 1H), 3.58(d, $J$ = 3.8 Hz, 1H), 3.45-3.37(m, 1H), 2.94-2.86(m, 2H), 2.19-2.08(m, 1H), 1.89(s, 3H), 1.75(s, 6H), 1.64(q, $J$ = 12.1 Hz, 6H); LCMS (m/z): 531.2$[M+1]^+$.

Example 202: Synthesis of benzyl ((4R,4aR,5R,6S,7S,9S,10S,10aR,11S,E)-4,6,9,10,11-pentahydroxy-6-(((2-methylphenyl)sulfonamido)methyl)octahydro-5,9-epoxy-7,10a-methanooxocino[4,5-d]pyrimidin-2(1H)-ylidene)carbamate (**Compound 238**)

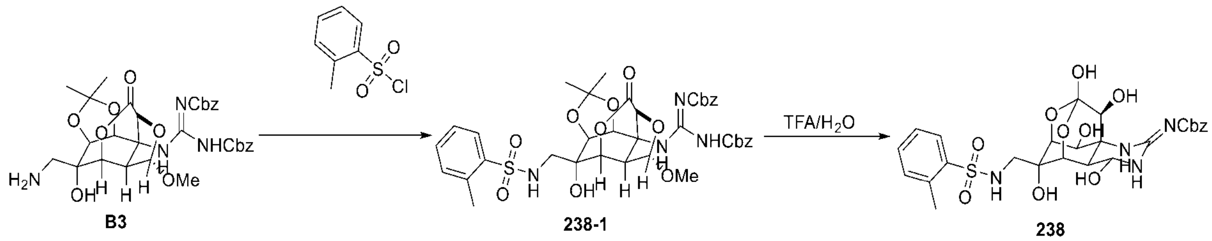


B3 238-1 238

Step 1: Synthesis of **Compound 238-1**

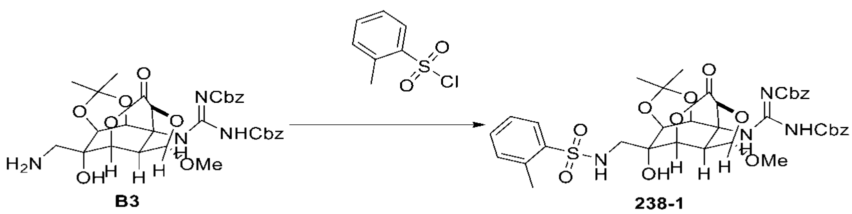

B3 238-1

**Compound B3** (100 mg, 0.16 mmol) was dissolved in a mixed solution of dichloromethane (5 mL) and pyridine (35.2 mg, 0.45 mmol), to which o-tosyl chloride (59.1 mg, 0.31 mmol) was added, and the reaction mixture was stirred at room temperature for 3 h under nitrogen protection. HPLC was used to monitor complete reaction of the raw materials. Water (5 mL) was added to the reaction solution, which was extracted with dichloromethane (5 mL×3). The organic phases were combined, dried over anhydrous sodium sulfate, filtered, and concentrated to dryness to give a crude product. The

crude product was purified by normal phase column chromatography (ethyl acetate/petroleum ether = 5%-30%) to give **Compound 238-1** as off-white solid (123 mg, yield: 99.2%). LCMS (m/z): 795.3[M+1]+.

Step 2: Synthesis of **Compound 238**

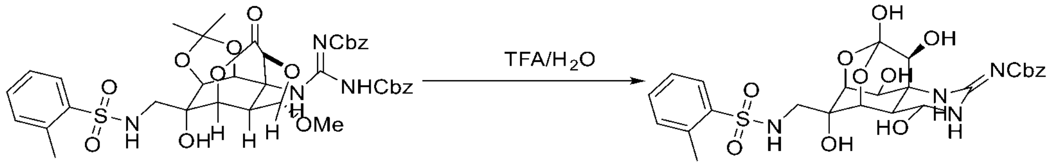


238-1 238

**Compound 238-1** (120 mg, 0.15 mmol) was dissolved in a mixed solution of water (4 mL) and trifluoroacetic acid (8 mL), and the reaction mixture was heated to 60 °C under nitrogen protection and stirred for 16 h. After the reaction was completed, the reaction solution was concentrated to dryness to give a crude product. The crude product was purified by normal phase column chromatography (methanol/dichloromethane = 5%-8%) to give **Compound 238** as white solid (52.3 mg, yield: 57.1%). LCMS (m/z): 607.2[M+1]+.

Example 203: Synthesis of 2-methyl-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 239**)

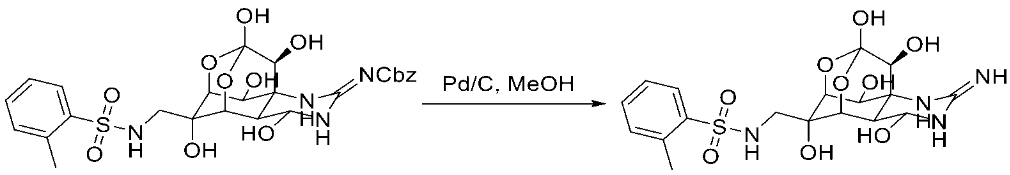


238 239

**Compound 238** (10 mg, 0.02 mmol) was dissolved in methanol (2 mL), to which 10% Pd/C (1 mg) was added. The reaction mixture was purged with hydrogen three times, and stirred under 25 °C for 8 h. After the reaction was completed, Pd/C was removed by filtration. The filter cake was washed with methanol, the filtrate was concentrated and dried, and then lyophilized to give **Compound 239** as white solid (5.7 mg, yield: 73.2%).

$^1H$ NMR (400 MHz, MeOD) $\delta$ 7.79(d, $J$ = 8.3 Hz, 1H), 7.59-7.47(m, 1H), 7.46-7.38(m, 2H), 5.53(dd, $J$ = 9.4, 3.0 Hz, 1H), 4.17(d, $J$ = 8.0 Hz, 2H), 3.93(d, $J$ = 9.0 Hz, 2H), 3.52-3.43(m, 2H), 2.67(s, 3H), 2.25(d, $J$ = 9.5 Hz, 1H); LCMS (m/z): 473.1[M+1]+.

Example 204: Synthesis of benzyl ((4R,4aR,5R,6S,7S,9S,10S,10aR,11S,E)-4,6,9,10,11-pentahydroxy-6-(((2-methoxyphenyl)sulfonamido)methyl)octahydro-5,9-epoxy-7,10a-methanooxocino[4,5-d]pyrimidin-2(1H)-ylidene)carbamate (**Compound 240**)

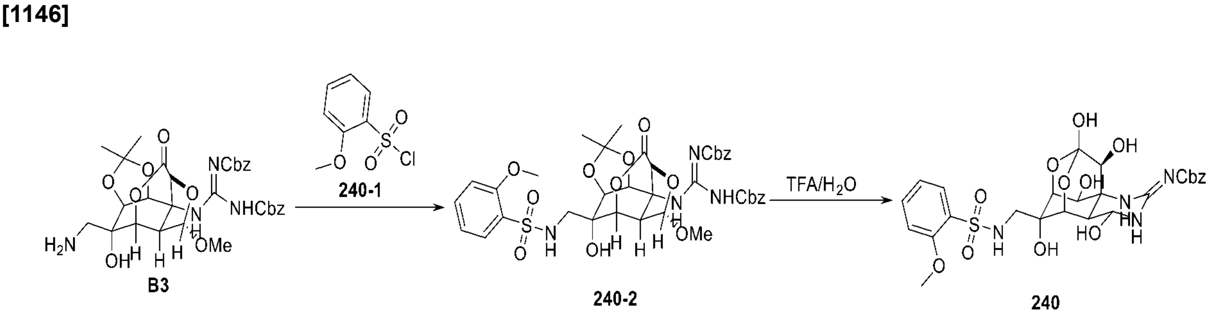


B3 240-2 240

Step 1: Synthesis of **Compound 240-2**

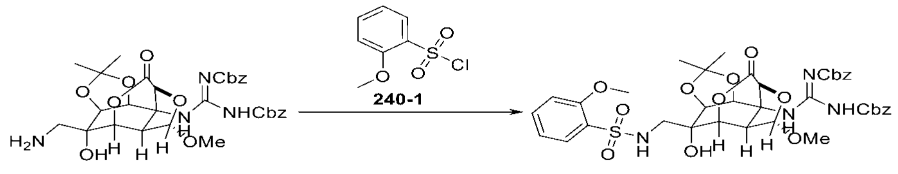


B3 240-2

**Compound B3** (100 mg, 0.16 mmol) was dissolved in a mixed solution of pyridine (35.2 mg, 0.45 mmol) and dichloromethane (5 mL), to which **Compound 240-1** (59.1 mg, 0.29 mmol) was added under stirring, and the reaction mixture was stirred at room temperature for 3 h under nitrogen protection. After the reaction was completed, water (5 mL) was added to the reaction solution, which was extracted with dichloromethane (5 mL×3). The organic phases were combined, dried over anhydrous sodium sulfate, and concentrated to give a crude product. The crude product was purified by normal phase column chromatography (ethyl acetate/petroleum ether = 5%-30%) to give **Compound 240-2** as off-white solid (123 mg, yield: 97.2%). LCMS (m/z): 811.2$[M+1]^+$.

Step 2: Synthesis of **Compound 240**

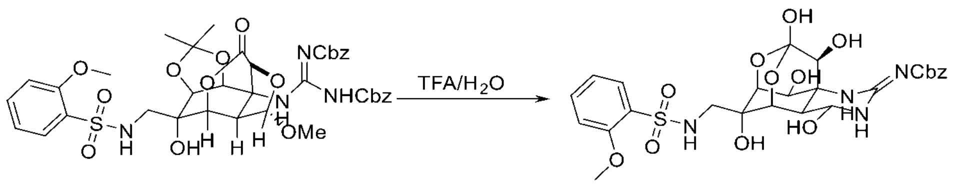


240-2 240

**Compound 231** (120 mg, 0.15 mmol) was dissolved in a mixed solution of water (8 mL) and trifluoroacetic acid (4 mL), and the reaction mixture was heated to 60 °C under nitrogen protection and stirred for 16 h. After the reaction was completed, the reaction solution was concentrated and dried to give a crude product. The crude product was purified by normal phase column chromatography (methanol/dichloromethane = 5%-8%) to give **Compound 240** as white solid (52.3 mg, yield: 56.8%).

$^1$H NMR (400 MHz, MeOD) $\delta$ 7.86(dd, $J$ = 7.8, 1.6 Hz, 1H), 7.67-7.60(m, 1H), 7.46-7.36(m, 5H), 7.23(d, $J$ = 8.4 Hz, 1H), 7.11(t, $J$ = 7.6 Hz, 1H), 5.67(d, $J$ = 9.4 Hz, 1H), 5.30(s, 2H), 4.23(d, $J$ = 1.7 Hz, 1H), 4.16(s, 1H), 3.99(s, 3H), 3.95(d, $J$ = 1.9 Hz, 2H), 3.43(d, $J$ = 4.6 Hz, 2H), 2.42-2.33(m, 1H); LCMS (m/z): 623.2$[M+1]^+$.

Example 205: Synthesis of 2-methoxy-N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)benzenesulfonamide (**Compound 241**)

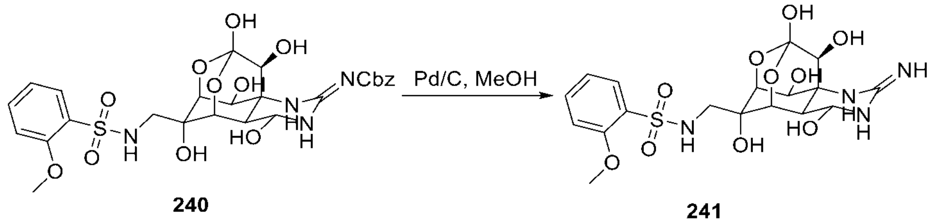


240 241

**Compound 240** (10 mg, 0.02 mmol) was dissolved in methanol (2 mL), to which 10% Pd/C (1 mg) was added. The reaction mixture was purged with hydrogen three times, and stirred at room temperature for 8 h. After the reaction was completed, palladium on carbon was removed by filtration. The filter cake was rinsed with methanol, the filtrate was concentrated and dried, and then lyophilized to give **Compound 241** as white solid (5.7 mg, yield: 72.6%).

$^1$H NMR (400 MHz, MeOD) $\delta$ 7.86(dd, $J$ = 7.8, 1.6 Hz, 1H), 7.69-7.63(m, 1H), 7.25(d, $J$ = 8.3 Hz, 1H), 7.13(t, $J$ = 7.6 Hz, 1H), 5.52(d, $J$ = 9.3 Hz, 1H), 4.17-4.13(m, 2H), 4.00(s, 3H), 3.92(d, $J$ = 8.0 Hz, 2H), 3.43(d, $J$ = 1.5 Hz, 2H), 2.21(d, $J$ = 4.8 Hz, 1H); LCMS (m/z):489.1[M+1]$^+$.

Example 206: Synthesis of benzyl ((4R,4aR,5R,6S,7S,9S,10S,10aR,11S,E)-4,6,9,10,11-pentahydroxy-6-(((3-(trifluoromethyl)phenyl)sulfonamido)methyl)octahydro-5,9-epoxy-7,10a-methanooxocino[4,5-d]pyrimidin-2(1H)-ylidene)carbamate (**Compound 242**)

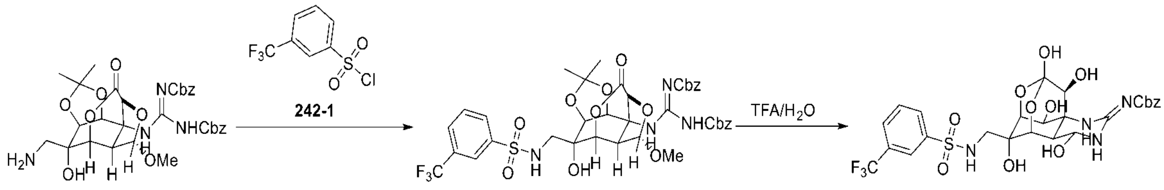


B3 242-2 242

Step 1: Synthesis of **Compound 242-2**

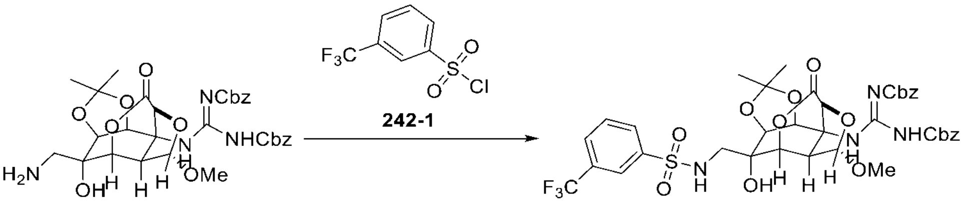


B3 242-2

**Compound B3** (100 mg, 0.16 mmol) was dissolved in a mixed solution of pyridine (50 mg, 0.63 mmol) and dichloromethane (5 mL), to which **Compound 242-1** (45.8 mg, 0.19 mmol) was added with stirring, and the reaction mixture was stirred at room temperature for 2 h. After the reaction was completed, water (10 mL) was added to the reaction solution, which was extracted with dichloromethane (10 mL×3). The organic phases were combined, washed with saturated brine (10 mL). The organic phase was dried over anhydrous sodium sulfate, filtered, and the filtrate was concentrated and dried to give a crude product. The crude product was purified by normal phase column chromatography (ethyl acetate/petroleum ether = 0-35%) to give **Compound 242-2** as white solid (120 mg, yield: 90.6%). LCMS (m/z):849.2[M+1]$^+$.

Step 2: Synthesis of **Compound 242**

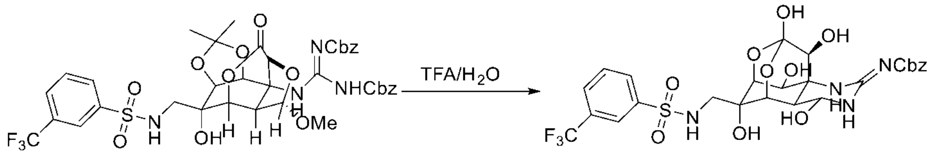


242-2 242

**Compound 242-2** (120 mg, 0.14 mmol) was dissolved in a mixed solution of water (10 mL) and trifluoroacetic acid (10 mL), and the reaction mixture was heated to 60 °C under nitrogen protection and stirred for 24 h. After the reaction was completed, the reaction mixture was directly concentrated and dried to give a crude product. The crude product was purified by normal phase column chromatography (methanol/dichloromethane = 0-7%) to give **Compound 242** (22 mg, yield: 23.6%).

$^1$H NMR (400 MHz, MeOD) $\delta$ 8.23-8.13(m, 2H), 7.95(d, $J$ = 7.8 Hz, 1H), 7.80(t, $J$ = 7.8 Hz, 1H), 7.42-7.26(m, 5H),

5.57(d, *J* = 9.2 Hz, 1H), 5.12(s, 2H), 4.13(d, *J* = 12.9 Hz, 2H), 3.90(s, 2H), 3.53-3.45(m, 2H), 2.25(d, *J* = 9.5 Hz, 1H); LCMS (m/z):661.1[M+1]+.

Example 207: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)-3-(trifluoromethyl)benzenesulfonamide (**Compound 243**)

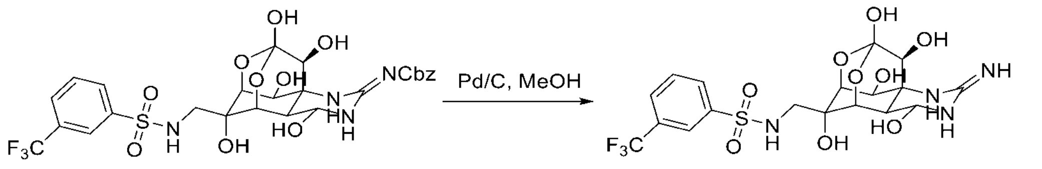


242 243

**Compound 242** (15.0 mg, 0.02 mmol) was dissolved in methanol (5 mL), to which 10% palladium on carbon (7 mg) was added. The reaction mixture was purged with hydrogen, and stirred for 3 h with introduction of hydrogen. HPLC was used to monitor complete reaction of the raw materials. The reaction mixture was filtered, and the filter cake was rinsed with methanol. The filtrate was concentrated and dried to give **Compound 243** as white solid (10 mg, yield: 83.7%).

$^1$H NMR (400 MHz, MeOD) $\delta$ 8.26-8.17(m, 2H), 7.99(d, *J* = 8.0 Hz, 1H), 7.84(t, *J* = 7.8 Hz, 1H), 5.55(d, *J* = 9.4 Hz, 1H), 4.16(d, *J*= 5.5 Hz, 2H), 3.94(s, 2H), 3.57-3.48(m, 2H), 2.25(d, *J*= *9.8* Hz, 1H); LCMS (m/z):527.1[M+1]+.

Example 208: Synthesis of benzyl ((4R,4aR,5R,6S,7S,9S,10S,10aR,11S,E)-4,6,9,10,11-pentahydroxy-6-(((2-(trifluoromethyl)phenyl)sulfonamido)methyl)octahydro-5,9-epoxy-7,10a-methanooxocino[4,5-d]pyrimidin-2(1H)-ylidene)carbamate (**Compound 244**)

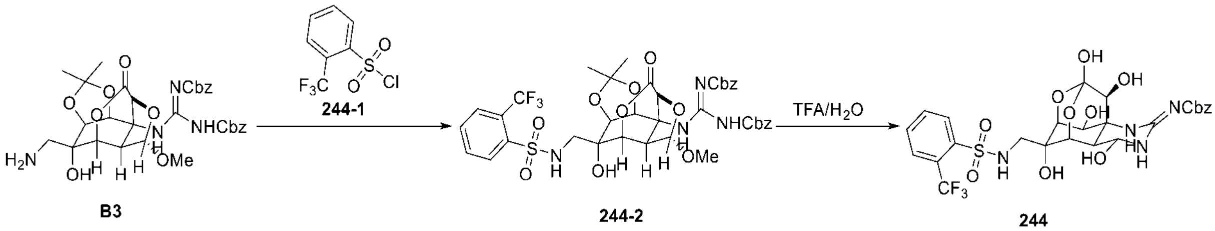


B3 244-2 244

Step 1: Synthesis of **Compound 244-2**

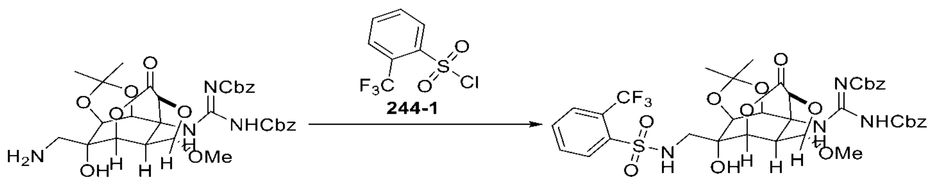


B3 244-2

**Compound B3** (100 mg, 0.16 mmol) was dissolved in a mixed solution of pyridine (50 mg, 0.624 mmol) and dichloromethane (5 mL), to which **Compound 244-1** (45.8 mg, 0.187 mmol) was added with stirring, and the reaction mixture was stirred at room temperature for 2 h. After the reaction was completed, water (10 mL) was added to the reaction solution, which was extracted with dichloromethane (10 mL×3). The organic phases were combined, washed with saturated brine (20 mL). The organic phase was dried over anhydrous sodium sulfate, filtered, and the filtrate was concentrated and dried to give a crude product. The crude product was purified by normal phase column chromatography (ethyl acetate/petroleum ether = 0-30%) to give **Compound 244-2** (100 mg, yield: 75.5%). LCMS (m/z):849.2[M+1]+.

Step 2: Synthesis of **Compound 244**

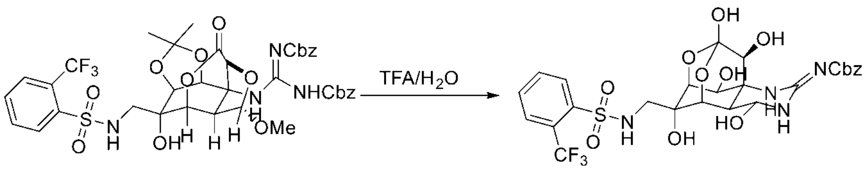


244-2 244

**Compound 244-2** (100 mg, 0.12 mmol) was dissolved in a mixed solution of water (14 mL) and trifluoroacetic acid (7 mL), and the reaction mixture was heated to 60 °C under nitrogen protection and stirred for 24 h. After the reaction was completed, the reaction solution was concentrated and dried to give a crude product. The crude product was purified by normal phase column chromatography (methanol/dichloromethane = 0-7.0%) to give **Compound 244** as white solid (10 mg, yield: 12.9%).

$^1H$ NMR (400 MHz, MeOD) $\delta$ 8.24-8.18(m, 1H), 8.01-7.92(m, 1H), 7.86-7.76(m, 2H), 7.44-7.28(m, 5H), 5.61(d, *J* = 9.3 Hz, 1H), 5.19(s, 2H), 4.18(s, 1H), 4.14(s, 1H), 3.92(s, 2H), 3.60(s, 2H), 2.30(d, *J* = 9.4 Hz, 1H); LCMS (m/z):661.1 $[M+1]^+$.

Example 209: Synthesis of N-(((4R,4aR,5R,6S,7S,9S,10S,10aR,11S)-4,6,9,10,11-pentahydroxy-2-iminodecahydro-5,9-epoxy-7,10a-methanooxocino[4,5-d]pyrimidin-6-yl)methyl)-2-(trifluoromethyl)benzenesulfonamide (**Compound 245**)

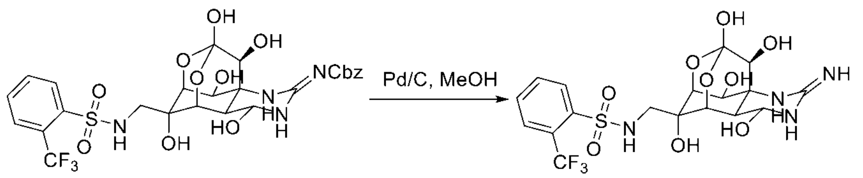


244 245

**Compound 244** (8 mg, 0.022 mmol) was dissolved in methanol (5 mL), to which 10% Pd/C (7 mg) was added. The reaction mixture was purged with hydrogen, and stirred for 3 h with introduction of hydrogen. HPLC was used to monitor complete reaction of the raw materials. The reaction mixture was filtered, the filter cake was rinsed with methanol, and the filtrate was concentrated and dried to give **Compound 245** as white solid (5.5 mg, yield: 86.2%).

$^1H$ NMR (400 MHz, MeOD) $\delta$ 8.16-8.06(m, 1H), 7.91-7.83(m, 1H), 7.79-7.66(m, 2H), 5.42(d, *J* = 9.3 Hz, 1H), 4.06-4.01(m, 2H), 3.81(d, *J* = 1.8 Hz, 2H), 3.50(d, *J* = 3.2 Hz, 2H), 2.13(d, *J* = 9.5 Hz, 1H); LCMS (m/z):527.1$[M+1]^+$.

## Biological Testing

### Biological Example 1: Effect of the compound by total cell manual voltage clamp on hNav sodium channel

HEK293 cells stably expressing hNav (from the cell bank of Chinese Academy of Sciences) were cultured in a cell culture dish with a diameter of 35 mm, and cultured in an incubator at 37°C, 5% $CO_2$, with a medium formula of 90% DMEM (Invitrogen), 10% fetal bovine serum (Gibco) and 300 g/mL hygromycin B (Invitrogen). On the day of the experiment, the cell culture medium was removed. After the cells were washed once with extracellular fluid, 0.25% Trypsin-EDTA (Invitrogen) solution was added, and digestion was carried out at room temperature for 3-5 min. The digestive fluid was removed, and the cells were resuspended with extracellular fluid and transferred to an experimental dish for electrophysiological recording for later use.

The compound was dissolved with double distilled water ($ddH_2O$) or DMSO to prepare a stock solution. On the day of testing, the stock solution of the compound was serially diluted 3 times with the corresponding stock solution preparation solvent, that is, 10μL of the stock solution of the compound was added to 20μL of the solvent, and the intermediate concentrations of the compound serially diluted 3 times was obtained sequentially. Then 10μL of the compound in intermediate concentration was added to 4990μL of the extracellular fluid and diluted 500 times to give the final concentration to be tested. The inhibition rate of hNav1.7 at a concentration of 30μM was determined, and then some

molecules were selected to determine $IC_{50}$ for hNav1.7 and $IC_{50}$ for different sodium ion channel subtypes. The solvent content in the final test concentration did not exceed 0.2%, and the solvent at this concentration had no effect on the hNav sodium channel.

The hNav sodium channel current of HEK293 cells stably expressing the hNav sodium channel was recorded at room temperature using a whole-cell voltage clamp technique. The glass microelectrode was formed by drawing a glass electrode blank (BF150-86-10, Sutter) with a drawing instrument, the tip resistance after the liquid in the electrode was injected was about 2-5 MΩ, and the glass microelectrode was inserted into the amplifier probe to be connected to the patch clamp amplifier. The clamping voltage and data recording were controlled and recorded by pClamp software through a computer with a sampling frequency of 20 kHz and a filtering frequency of 2 kHz. After the whole cell records were obtained, the cells were clamped at -100 mV, a 20 ms depolarization voltage was applied to -20 mV induced sodium current (T1), then a -40 mV deactivation voltage was applied for 5s, repolarized to -120 mV, and a 20 ms depolarization voltage was applied to -20 mV induced sodium current (T2) again after 50 ms. This voltage stimulation was administered every 10s, and the administration process was initiated after the hNav1.7 sodium current was determined as stable (1 min). Compounds were administered continuously from low test concentrations, with each test concentration administered for 1 min to steady state or up to 3 min. The compounds were tested for at least 3 cells at each concentration (n≥3).

The data were analyzed and processed with pClamp, GraphPad Prism 8 and Excel software. The inhibition degree of different compound concentrations on hNav sodium current was calculated by the following formula:

$$\text{Inhibition\%} = [1-(I/I_o)]\times 100\%$$

wherein, Inhibition% represents inhibition percentage of the compound on hNav sodium current, and I and $I_o$ represent the amplitude of the hNav sodium current after dosing and before dosing, respectively.

Compound $IC_{50}$ (compound concentration inhibiting maximum current by 50%) was calculated using GraphPad Prism 8 software by fitting the following equation:

$$Y=Bottom +(Top-Bottom)/(1+10^{\wedge}((LogIC_{50}-X)*HillSlope))$$

wherein X is Log value of tested concentration of the compound, Y is inhibition percentage at the corresponding concentration, and Bottom and Top are the minimum and maximum inhibition percentages, respectively. HillSlope is slope of the dose-effect curve.

The test results are shown in Table 1, Table 2 and Table 3 below.

**Table 1. Inhibition rate results of compounds on hNav1.7 sodium channel by total cell manual voltage clamp**

| Compound No. | hNav1.7 30μM | Compound No. | hNav1.7 30μM | Compound No. | hNav1.7 30μM | Compound No. | hNav1.7 30μM |
|---|---|---|---|---|---|---|---|
| TTX-N | A | 76 | E | 134 | C | 193 | A |
| TTX-S | A | 77 | B | 135 | A | 194 | c |
| 1 | C | 78 | E | 136 | D | 195 | A |
| 2 | E | 79 | E | 137 | A | 197 | A |
| 3 | A | 80 | E | 138 | D | 198 | A |
| 4 | A | 81 | C | 139 | A | 199 | A |
| 5 | E | 82 | E | 140 | D | 201 | E |
| 6 | A | 83 | E | 141 | E | 202 | B |
| 9 | D | 84 | E | 142 | A | 203 | A |
| 11 | E | 85 | E | 143 | D | 204 | A |
| 14 | E | 86 | E | 144 | A | 205 | A |
| 15 | E | 87 | E | 145 | A | 206 | B |
| 16 | E | 88 | A | 146 | B | 207 | A |
| 17 | E | 89 | B | 147 | A | 208 | B |
| 18 | E | 90 | E | 148 | A | 209 | A |
| 19 | E | 91 | A | 149 | A | 210 | B |
| 23 | E | 92 | C | 150 | A | 211 | A |
| 24 | E | 93 | A | 151 | B | 212 | A |
| 26 | E | 94 | B | 152 | E | 213 | A |
| 29 | E | 95 | A | 153 | E | 214 | A |
| 30 | E | 96 | E | 154 | B | 215 | A |
| 31 | B | 96 | A | 155 | E | 216 | A |
| 32 | E | 97 | A | 156 | A | 217 | A |
| 33 | E | 98 | A | 157 | E | 218 | B |
| 34 | E | 99 | A | 158 | A | 219 | A |
| 35 | E | 100 | A | 159 | E | 220 | B |

(continued)

| Compound No. | hNav1.7 30μM | Compound No. | hNav1.7 30μM | Compound No. | hNav1.7 30μM | Compound No. | hNav1.7 30μM |
|---|---|---|---|---|---|---|---|
| 36 | A | 101 | B | 160 | A | 221 | A |
| 37 | E | 102 | E | 161 | D | 222 | A |
| 39 | E | 103 | A | 162 | A | 223 | A |
| 40 | E | 104 | B | 163 | C | 224 | C |
| 41 | E | 105 | C | 164 | A | 225 | A |
| 42 | E | 106 | A | 165 | A | 226 | A |
| 43 | E | 107 | B | 166 | A | 227 | A |
| 47 | E | 108 | E | 167 | B | 228 | A |
| 48 | E | 109 | A | 168 | E | 229 | A |
| 49 | E | 110 | B | 169 | E | 230 | A |
| 50 | E | 111 | D | 170 | C | 231 | A |
| 51 | E | 112 | A | 171 | B | 232 | c |
| 52 | E | 113 | E | 172 | A | 233 | A |
| 54 | E | 114 | A | 173 | B | 234 | c |
| 56 | E | 115 | C | 174 | E | 235 | A |
| 58 | E | 116 | A | 175 | A | 236 | B |
| 59 | E | 117 | B | 176 | E | 237 | A |
| 60 | E | 118 | A | 177 | E | 238 | B |
| 61 | A | 119 | E | 178 | C | 239 | A |
| 62 | E | 120 | E | 179 | A | 240 | B |
| 63 | B | 121 | A | 180 | D | 241 | A |
| 64 | D | 122 | B | 181 | A | 242 | B |
| 65 | C | 123 | A | 182 | A | 243 | A |
| 66 | A | 124 | D | 183 | A | 244 | B |
| 67 | E | 125 | A | 184 | D | 245 | A |
| 68 | E | 126 | A | 185 | A | 199-A | A |

(continued)

| Compound No. | hNav1.7 30μM | Compound No. | hNav1.7 30μM | Compound No. | hNav1.7 30μM | Compound No. | hNav1.7 30μM |
|---|---|---|---|---|---|---|---|
| 69 | E | 127 | A | 186 | A | 199-B | A |
| 70 | E | 128 | A | 187 | A | 200-A | A |
| 71 | E | 129 | A | 188 | C | 200-B | D |
| 72 | E | 130 | D | 189 | A | | |
| 73 | A | 131 | A | 190 | C | | |
| 74 | C | 132 | E | 191 | A | | |
| 75 | E | 133 | A | 192 | A | | |

Note: TTX-N: TTX extracted from nature, purchased from Kangte (Taizhou) Bioengineering Co., Ltd.; TTX-S: TTX synthesized by the present disclosure (synthesized by reference to the processes of CN113956266A); Inhibition rate: A: greater than 80%; B: greater than 60% to less than or equal to 80%; C: greater than 40% to less than or equal to 60%; D: greater than 20% to less than or equal to 40%; E: less than or equal to 20%.

The experimental results shown in Table 1 show that the compounds of the present disclosure have inhibitory activity on the hNav1.7 sodium channel, wherein some compounds have inhibitory activity on the hNav1.7 sodium channel of even greater than 80% at 30μM.

According to the experimental results in Table 1, some of the compounds were further tested for $IC_{50}$ for hNav1.7, and the test results are shown in Table 2 below.

**Table 2. $IC_{50}$ results of compounds on hNav1.7 sodium channel by total cell manual voltage clamp**

| Compound No. | **hNav1.7 IC50 (nM)** | Compound No. | **hNav1.7 IC50 (nM)** | Compound No. | **hNav1.7 IC50 (nM)** | Compound No. | **hNav1.7 IC50(nM)** |
|---|---|---|---|---|---|---|---|
| TTX-N | +++++ | 109 | +++ | 156 | ++ | 205 | ++++ |
| TTX-S | +++++ | 110 | ++ | 158 | +++ | 206 | + |
| 3 | ++ | 112 | +++ | 160 | ++ | 207 | ++++ |
| 4 | +++ | 114 | +++ | 162 | +++ | 208 | + |
| 6 | +++ | 116 | +++ | 165 | +++ | 209 | +++++ |
| 31 | ++ | 117 | ++ | 166 | ++ | 210 | ++ |
| 36 | ++ | 118 | +++ | 172 | +++ | 211 | ++++ |
| 61 | ++ | 121 | ++ | 173 | ++ | 212 | +++ |
| 63 | ++ | 122 | + | 175 | ++ | 213 | +++++ |
| 65 | ++ | 123 | +++ | 179 | ++++ | 214 | ++ |
| 66 | ++++ | 125 | +++ | 181 | ++++ | 215 | ++++ |
| 73 | ++ | 126 | ++ | 182 | ++ | 216 | ++ |
| 74 | + | 127 | ++++ | 182 | +++ | 217 | ++++ |
| 77 | + | 128 | ++ | 183 | ++++ | 219 | ++++ |
| 81 | + | 129 | +++++ | 185 | +++ | 220 | ++ |
| 88 | +++++ | 131 | +++ | 186 | +++ | 221 | +++ |
| 89 | ++ | 133 | ++++ | 187 | +++++ | 222 | +++ |
| 91 | ++++ | 134 | + | 189 | ++++ | 223 | +++++ |
| 93 | +++ | 135 | +++ | 191 | +++++ | 225 | ++++ |
| 94 | ++ | 137 | +++ | 192 | ++ | 226 | +++ |
| 95 | +++ | 139 | +++ | 193 | ++++ | 227 | +++ |
| 96 | +++ | 142 | ++++ | 195 | ++++ | 228 | +++ |
| 97 | +++ | 144 | ++++ | 197 | ++++ | 229 | +++ |
| 98 | ++++ | 145 | ++ | 198 | ++ | 230 | ++ |
| 99 | ++ | 146 | ++ | 199 | ++++ | 231 | ++++ |
| 100 | ++++ | 147 | +++ | 199-A | +++ | 233 | ++++ |
| 101 | ++ | 148 | ++ | 199-B | +++++ | 235 | ++++ |
| 103 | ++++ | 149 | +++ | 200-A | +++ | 239 | ++++ |
| 104 | ++ | 150 | ++++ | 202 | ++ | 243 | ++++ |

(continued)

| Compound No. | **hNav1.7 IC50 (nM)** | Compound No. | **hNav1.7 IC50 (nM)** | Compound No. | **hNav1.7 IC50 (nM)** | Compound No. | **hNav1.7 IC50(nM)** |
|---|---|---|---|---|---|---|---|
| 106 | +++ | 151 | + | 203 | +++ | | |
| 107 | + | 154 | ++ | 204 | +++ | | |
| Note: TTX-N: TTX extracted from nature, purchased from Kangte (Taizhou) Bioengineering Co., Ltd.; TTX-S: TTX synthesized by the present disclosure (synthesized by reference to the processes of CN113956266A); $IC_{50}$ value: +++++: less than or equal to 20 nM; ++++: greater than 20 nM and less than or equal to 100 nM; +++: greater than 100 nM and less than or equal to 1μM; ++ : greater than 1μM and less than or equal to 10μM; +: greater than 10μM. | | | | | | | |

The experimental results shown in Table 2 show that the compounds of the present disclosure have significant inhibitory activity on the hNav1.7 sodium channel, wherein the inhibitory $IC_{50}$ values of multiple compounds, such as Compound 88, Compound 129, Compound 187, Compound 191, Compound 199-B, Compound 209, Compound 213 and Compound 223 and the like, on the hNav1.7 sodium channel are even less than 20 nM, which are comparable to TTX or even stronger.

In addition to hNav1.7, the inventors also selected some compounds to test the inhibitory $IC_{50}$ activity on different subtypes of the sodium ion channels to evaluate the selectivity of the compounds, and the test results are shown in Table 3 below.

**Table 3. $IC_{50}$ results of compounds on different subtypes of sodium channels by total cell manual voltage clamp**

| **Compound No.** | **hNav1.1 $IC_{50}$(nM)** | **hNav1.2 $IC_{50}$(nM)** | **hNav1.3 $IC_{50}$(nM)** | **hNav1.4 $IC_{50}$(nM)** | **hNav1.5 $IC_{50}$(nM)** | **hNav1.6 $IC_{50}$(nM)** | **hNav1.8 $IC_{50}$(nM)** |
|---|---|---|---|---|---|---|---|
| TTX-N | +++++ | +++++ | +++++ | +++++ | ++ | +++++ | + |
| TTX-S | +++++ | +++++ | +++++ | +++++ | ++ | +++++ | + |
| 3 | +++ | +++ | +++ | +++ | + | +++ | + |
| 73 | ++ | ++ | ++ | ++ | + | + | + |
| 88 | +++++ | ++++ | +++++ | +++++ | ++ | +++++ | + |
| 129 | +++++ | +++++ | +++++ | +++++ | ++ | +++++ | + |
| 154 | ++++ | ++++ | +++++ | ++++ | ++ | +++++ | + |
| 181 | ++++ | +++ | +++ | +++ | + | ++++ | + |
| 183 | +++++ | ++++ | +++++ | ++++ | ++ | +++++ | + |
| 191 | +++++ | +++++ | +++++ | ++++ | ++ | +++++ | + |
| 199-B | +++++ | ++++ | +++++ | ++++ | ++ | +++++ | + |
| 209 | +++++ | +++++ | +++++ | ++++ | ++ | +++++ | + |
| 221 | +++ | +++ | ++++ | +++ | + | ++++ | + |
| Note: TTX-N: TTX extracted from nature, purchased from Kangte (Taizhou) Bioengineering Co., Ltd.; TTX-S: TTX synthesized by the present disclosure (synthesized by reference to the processes of CN113956266A); $IC_{50}$ value: +++++: less than or equal to 20 nM; ++++: greater than 20 nM and less than or equal to 100 nM; +++: greater than 100 nM and less than or equal to 1μM; ++: greater than 1μM and less than or equal to 10μM; +: greater than 10μM. | | | | | | | |

The experimental results shown in Table 2 and Table 3 show that the compounds of the present disclosure have strong inhibitory activity on TTX-sensitive sodium ion channel subtypes (hNav1.1, hNav1.2, hNav1.3, hNav1.4, hNav1.6 and hNav1.7), while weak inhibitory activity on TTX-insensitive sodium ion channels (hNav1.5 and hNav1.8), showing that the selectivities of the compounds of the present disclosure are consistent with that of TTX. hNav1.5 is an important target that may cause a risk of cardiotoxicity. The inhibitory activity of the compound of the present disclosure on the target is over 100 times lower than that of the TTX-sensitive sodium ion channel subtypes, such that the compound of the present

disclosure has a lower risk of cardiotoxicity.

**Biological Example 2: Study on Toxicity in Single Administration of Mice**

SPF grade ICR mice (half male and half female) of 6 weeks were adaptively fed for 5-7 days and randomly grouped, with 3 male and 3 female in each group. The compound was dissolved in deionized water to prepare the desired concentration for administration. The administration was carried out by the "up and down" method, and the maximum tolerated dose (MTD) of each test substance was explored. The administration volume was 5 mL/kg, the administration route was subcutaneous administration on the neck and back, the administration frequency and cycle were single administration, and 4-hour cage edge observation was carried out after the administration. The detailed clinical responses of the animals were recorded. The survival animals were observed continuously for 7 days, one observation in morning and one in the afternoon each day. The death, general clinical condition, weight and food intake were recorded. The results are shown in Table 4 below.

**Table 4. Test Results of Single-Dose Toxicity Study on ICR Mice**

| Compound No. | Acute Toxicity Parameters | Compound No. | Acute Toxicity Parameters | Compound No. | Acute Toxicity Parameters | Compound No. | Acute Toxicity Parameters |
|---|---|---|---|---|---|---|---|
| TTX-N | MTD: 4μg/kg | 114 | $LD_{100}$: about 0.5mpk MTD: 0.1mpk | 165 | $LD_{50}$: about 0.2mpk MTD: 0.1mpk | 208 | $LD_{100}$: 0.4mpk MTD: 0.2mpk |
| TTX-S | $LD_{50}$: about 16μg/kg MTD: about 12μg/kg | 116 | $LD_{100}$: about 1mpk MTD: 0.3mpk | 166 | $LD_{50}$: about 2mpk MTD: 1mpk | 209 | $LD_{100}$: 0.03mpk MTD: 0.02mpk |
| 4 | MTD: about 0.1mpk $LD_{50}$: about 0.5mpk | 118 | $LD_{100}$: about 0.5mpk MTD: 0.1mpk | 167 | MTD>1mg/kg | 210 | $LD_{50}$: 0.3mpk MTD: 0.24mpk |
| 6 | MTD: about 0.1mpk $LD_{50}$: about 0.5mpk | 121 | $LD_{100}$: 0.05mpk MTD: 0.03mpk | 172 | $LD_{100}$: 0.4mpk MTD: 0.2mpk | 211 | $LD_{100}$: 0.03mpk MTD: 0.015mpk |
| 33 | $LD_{100}$: about 30mpk MTD: 15mpk | 122 | MTD: 0.5mpk | 179 | $LD_{100}$: about 0.08mpk MTD: 0.02mpk | 212 | $LD_{50}$: about 0.3mpk MTD: 0.2mpk |
| 61 | $LD_{100}$: about 0.3mpk MTD: 0.1mpk | 123 | $LD_{100}$: about 0.1mpk MTD: 0.03mpk | 181 | $LD_{100}$: about 0.1mpk MTD: 0.06mpk | 213 | $LD_{10}$: 0.05mpk MTD: 0.03mpk |
| 63 | $LD_{100}$: about 2.5mpk MTD: 1mpk | 125 | $LD_{50}$: about 0.3mpk MTD: 0.2mpk | 182 | $LD_{100}$: about 1mpk MTD: 0.5mpk | 214 | $LD_{100}$: 1mpk MTD: about 0.4mpk |
| 65 | $LD_{100}$: about 30mpk MTD: 10mpk | 129 | $LD_{100}$: about 0.1mpk MTD: 0.03mpk | 183 | $LD_{100}$: about 0.02mpk MTD: 0.01mpk | 215 | $LD_{100}$: 0.03mpk MTD: 0.015mpk |
| 66 | $LD_{100}$: about 0.1mpk MTD: 0.03mpk | 131 | $LD_{10}$: about 0.1mpk MTD: 0.08mpk | 185 | $LD_{50}$: about 0.3mpk MTD: 0.2mpk | 216 | $LD_{100}$: 1mpk MTD: about 0.4mpk |

(continued)

| Compound No. | Acute Toxicity Parameters | Compound No. | Acute Toxicity Parameters | Compound No. | Acute Toxicity Parameters | Compound No. | Acute Toxicity Parameters |
|---|---|---|---|---|---|---|---|
| 73 | $LD_{50}$: about 2mpk MTD: 0.8mpk | 135 | $LD_{50}$: about 0.1mpk MTD: 0.05mpk | 186 | $LD_{50}$: about 0.4mpk MTD: 0.3mpk | 217 | $LD_{100}$: 0.03mpk MTD: 0.01mpk |
| 77 | $LD_{100}$: about 2mpk MTD: 0.8mpk | 137 | $LD_{50}$: about 0.1mpk MTD: 0.075mpk | 189 | MTD: 0.02mpk | 219 | $LD_{50}$: 0.1mpk MTD: 0.05mpk |
| 81 | $LD_{100}$: about 0.5mpk MTD: 0.25mpk | 139 | $LD_{100}$: about 0.5mpk MTD: 0.1mpk | 191 | $LD_{100}$: 0.1mpk MTD: 0.05mpk | 220 | $LD_{50}$: about 0.3mpk MTD: 0.2mpk |
| 88 | $LD_{100}$: about 1mpk MTD: 0.05mpk | 144 | $LD_{50}$: about 0.04mpk MTD: 0.03mpk | 192 | $LD_{100}$: about 0.5mpk MTD: 0.1mpk | 221 | $LD_{100}$: about 0.5mpk MTD: 0.3mpk |
| 89 | $LD_{100}$: about 5mpk MTD: 2mpk | 145 | MTD>1mg/ kg | 193 | $LD_{100}$: about 0.05mpk MTD: 0.02mpk | 223 | $LD_{100}$: 0.2mpk MTD: 0.024mpk |
| 91 | $LD_{100}$: about 0.1mpk MTD: 0.02mpk | 147 | $LD_{50}$: 0.03mpk MTD: 0.02mpk | 197 | $LD_{100}$: about 0.5mpk MTD: 0.05mpk | 225 | $LD_{50}$: 0.02mpk MTD: 0.015mpk |
| 94 | $LD_{100}$: about 10mpk MTD: 1mpk | 148 | $LD_{50}$: about 5mpk MTD: 3mpk | 198 | $LD_{50}$: about 0.4mpk MTD: 0.3mpk | 227 | MTD: 0.8mpk |
| 95 | MTD>2m pk | 149 | $LD_{50}$: about 0.5mpk MTD: 0.3mpk | 199-B | $LD_{100}$: 0.02mpk MTD: 0.014mpk | 229 | MTD>0.4m pk |
| 96 | LD10: about 0.02mpk | 150 | $LD_{100}$: about 0.05mpk MTD: 0.02mpk | 202 | $LD_{50}$: 1mpk MTD: 0.4mpk | 230 | MTD: 0.8mpk |
| 98 | $LD_{100}$: about 0.5mpk MTD: 0.1mpk | 154 | $LD_{50}$: about 0.03mpk MTD: 0.02mpk | 203 | $LD_{100}$: 0.05mpk MTD: 0.015mpk | 231 | $LD_{10}$: 0.8mpk MTD: 0.7mpk |
| 99 | $LD_{100}$: about 10mpk MTD: 2mpk | 156 | MTD>0.5m pk | 204 | MTD>0.8m pk | 233 | $LD_{50}$: 0.02mpk MTD: 0.01mpk |

(continued)

| Compound No. | Acute Toxicity Parameters | Compound No. | Acute Toxicity Parameters | Compound No. | Acute Toxicity Parameters | Compound No. | Acute Toxicity Parameters |
|---|---|---|---|---|---|---|---|
| 100 | $LD_{50}$: about 0.05mpk MTD: 0.02mpk | 158 | MTD: 0.5mpk | 205 | $LD_{100}$: 0.03mpk MTD: 0.01mpk | | |
| 103 | $LD_{100}$: about 0.1mpk MTD: 0.015mpk | 160 | $LD_{100}$: about 1mpk MTD: 0.2mpk | 206 | $LD_{50}$: 0.03mpk MTD: 0.02mpk | | |
| 112 | $LD_{100}$: about 0.5mpk MTD: 0.1mpk | 164 | $LD_{50}$: about 0.1mpk MTD: 0.06mpk | 207 | $LD_{100}$: 0.03mpk MTD: 0.02mpk | | |
| Note: mpk: mg/kg; TTX-N: TTX extracted from nature, purchased from Kangte (Taizhou) Bioengineering Co., Ltd.; TTX-S: TTX synthesized by the present disclosure (synthesized by reference to the processes of CN113956266A). | | | | | | | |

Main clinical observations of dead animals: depression, reduced activity, shortness of breath, immobility in prone, and death after convulsion;
$LD_{100}$: absolute lethal dose; $LD_{50}$: median lethal dose; $LD_{10}$: mild lethal dose; MTD: maximum tolerated dose.

The experimental results in Table 4 show that the compounds of the present disclosure show different toxicity conditions in animals according to their activity. The toxicity performance of the molecules with strong cell-level activity of the compounds of the present disclosure in animals is substantially consistent with TTX.

In this example, the maximum tolerated dose (MTD) of the compound of the present disclosure was determined, providing a dosage design basis for subsequent biological testing of the maximum effective dose of the compound of the present disclosure, and a basis for calculating therapeutic window size of the compound of the present disclosure.

**Biological Example 3: Study of analgesic effect of compounds on acetic acid writhing model in ICR Mice**

Male SPF grade ICR mice were randomly grouped 3-5 days after adaptive feeding, and divided into a model control group, a TTX (3μg/kg) group, and a test group, with 10 mice in each group. The dose of the test group was the MTD dose inferred from the single administration toxicity study of the mice in Biological Example 2. The control group was administered with normal saline, the TTX group and the test group used normal saline as vehicle. The administration volume was 5 mL/kg, the administration route was subcutaneous administration on the neck and back, and the administration frequency and cycle were single administration. After 30 min of pre-administration of the positive control and the test sample, the mice were intraperitoneally injected with 0.8% (v/v) acetic acid solution with an injection volume of 10 mL/kg. The number of writhing in the mice within 0-15 min and 15-30 min was observed, and the pain inhibition rate was calculated.

Inhibition rate = (number of writhing in control group - number of writhing in administration group)/number of writhing in control group * 100%

Mice having behavioral responses like abdominal retraction, trunk and hind limb extension, and elevation of the hip, was considered as a complete writhing response. The results are shown in Table 5.

**Table 5. Test results of analgesic effect of the acetic acid writhing pain model in ICR mice**

| Compound No. | Pain Inhibition Rate (%) | P Value (vs control group) |
|---|---|---|
| TTX-N | 65.56** | 0.002 |
| TTX-S | 65.86*** | 0.000 |
| 4 | 47.37* | 0.034 |

(continued)

| Compound No. | Pain Inhibition Rate (%) | P Value (vs control group) |
|---|---|---|
| 6 | 20 | 0.439 |
| 33 | 52.65* | 0.041 |
| 61 | 47.35* | 0.077 |
| 63 | 32.66* | 0.386 |
| 65 | 69.42** | *0.005* |
| 66 | 72.73** | 0.002 |
| 73 | 65.01** | 0.007 |
| 77 | 85.07*** | 0.000 |
| 81 | 65.46** | 0.007 |
| 88 | 78.01** | 0.002 |
| 89 | 43.43 * | 0.180 |
| 91 | 31.27* | 0.312 |
| 94 | 38.21* | 0.183 |
| 95 | 57.01*** | 0.000 |
| 96 | 37.08* | 0.215 |
| 98 | 41.37* | 0.054 |
| 99 | 60.47* | 0.121 |
| 100 | 32.09* | 0.622 |
| 103 | 82.05*** | 0.000 |
| 112 | 22.54 | 0.172 |
| 114 | 67.98*** | 0.000 |
| 116 | 66.06** | 0.005 |
| 118 | 73.48** | 0.004 |
| 121 | 54.63*** | 0.000 |
| 122 | 47.64* | 0.017 |
| 123 | 70.19*** | 0.000 |
| 125 | 64.60*** | 0.000 |
| 129 | 57.26** | 0.008 |
| 131 | 74.30*** | 0.000 |
| 135 | 46.89* | 0.045 |
| 137 | 54.62** | 0.005 |
| 139 | 68.34*** | 0.000 |
| 144 | 94.02** | 0.005 |
| 145 | 35.55* | 0.070 |
| 147 | 65.28*** | 0.000 |
| 148 | 23.1 | 0.103 |
| 149 | 77.78*** | 0.000 |
| 150 | 63.71*** | 0.000 |
| 156 | 85.19*** | 0.000 |

(continued)

| Compound No. | Pain Inhibition Rate (%) | P Value (vs control group) |
|---|---|---|
| 158 | 24.54 | 0.588 |
| 160 | 60.09* | 0.017 |
| 164 | 38.76* | 0.162 |
| 165 | 34.65* | 0.082 |
| 166 | 34.93* | 0.130 |
| 167 | 64.45** | 0.006 |
| 172 | 57.87*** | 0.001 |
| 179 | 62.33*** | 0.000 |
| 181 | 84.00*** | 0.000 |
| 182 | 82.54*** | 0.000 |
| 183 | 65.35*** | 0.000 |
| 185 | 74.24*** | 0.000 |
| 186 | 43.63** | 0.004 |
| 189 | 63.32*** | 0.000 |
| 191 | 67.01*** | 0.000 |
| 192 | 25.68 | 0.161 |
| 193 | 90.33*** | 0.000 |
| 197 | 73.36*** | 0.000 |
| 198 | 45.93* | 0.011 |
| 199 | 91.20*** | 0.000 |
| 199-B | 88.48*** | 0.000 |
| 202 | 55.50*** | 0.000 |
| 203 | 86.24*** | 0.000 |
| 204 | 36.46* | 0.010 |
| 205 | 33.51 * | 0.018 |
| 206 | 95.87*** | 0.000 |
| 207 | 30.83* | 0.028 |
| 208 | 41.29** | 0.004 |
| 209 | 95.75*** | 0.000 |
| 210 | 87.64*** | 0.000 |
| 211 | 54.82*** | 0.001 |
| 212 | 61.46*** | 0.000 |
| 213 | 54.31*** | 0.001 |
| 214 | 63.53*** | 0.000 |
| 215 | 29.5 | 0.063 |
| 216 | 75.69*** | 0.000 |
| 219 | 62.70*** | 0.000 |
| 220 | 79.91*** | 0.000 |
| 221 | 96.26*** | 0.000 |

(continued)

| Compound No. | Pain Inhibition Rate (%) | P Value (vs control group) |
|---|---|---|
| 223 | 81.59*** | 0.000 |
| 225 | 36.89** | 0.003 |
| 227 | 54.31*** | 0.001 |
| 229 | 71.31*** | 0.000 |
| 230 | 52.55*** | 0.000 |
| 231 | 35.04** | 0.004 |
| 233 | 76.57*** | 0.000 |
| Note: TTX-N: TTX extracted from nature, purchased from Kangte (Taizhou) Bioengineering Co., Ltd.; TTX-S: TTX synthesized by the present disclosure (synthesized by reference to the processes of CN113956266A); Compared with the control group, *: P<0.05, **: P<0.01, ***: P<0.001. | | |

The experimental results shown in Table 5 show that the compounds of the present disclosure have excellent analgesic effects in the acetic acid writhing pain mouse model, wherein the pain inhibition rates of Compound 77, Compound 103, Compound 144, Compound 156, Compound 181, Compound 182, Compound 193, Compound 199, Compound 199-B, Compound 203, Compound 206, Compound 209, Compound 210, Compound 221 and Compound 223 even reach more than 80%, which are significantly superior over TTX.

Some compounds were further selected to detect the analgesic effect of each compound in the acetic acid writhing pain mouse model at different doses, and the test results are shown in Table 6 below and FIG. 1.

**Table 6. Test results of analgesic effect of the acetic acid writhing pain model in ICR mice**

| Compound No. | Dose (mg/kg) | number of writhing | Pain Inhibition Rate (%) | P Value (vs Vehicle) | Magnification of the therapeutic window size # |
|---|---|---|---|---|---|
| TTX-S | Vehicle | 49.5±17.1 | - | - | 2 fold |
| | 0.012 | 16.9±12.4*** | 65.86 | 0.000 | |
| | 0.006 | 31.3±15.2** | 36.77 | 0.009 | |
| | 0.003 | 48.3±14.2 | 2.42 | 0.857 | |
| 73 | Vehicle | 43±14 | - | - | 4 fold |
| | 0.8 | 9±10*** | 79.91 | 0.000 | |
| | 0.6 | 11±9*** | 74.30 | 0.000 | |
| | 0.2 | 31±17* | 28.04 | 0.02 | |
| 77 | Vehicle | 44±10.11 | - | - | 8 fold |
| | 0.8 | 9.4±8.22*** | 78.64 | 0.000 | |
| | 0.1 | 29.2±11.58** | 33.64 | 0.002 | |
| 199 | Vehicle | 38±8 | - | - | >4 fold |
| | Positive | 19±9** | 49.07 | 0.001 | |
| | 0.02 | 3±4*** | 91.20 | 0.000 | |
| | 0.01 | 16±9*** | 58.40 | 0.000 | |
| | 0.005 | 21±17** | 43.47 | 0.002 | |
| 199-B | Vehicle | 53.8±10.05 | - | - | >7 fold |
| | 0.014 | 6.2±8.77*** | 88.48 | 0.000 | |
| | 0.006 | 17.2±12.52*** | 68.03 | 0.000 | |
| | 0.002 | 20.3±15.64*** | 62.27 | 0.000 | |

(continued)

| Compound No. | Dose (mg/kg) | number of writhing | Pain Inhibition Rate (%) | P Value (vs Vehicle) | Magnification of the therapeutic window size # |
|---|---|---|---|---|---|
| 210 | Vehicle | 43±15 | - | - | >4 fold |
| | 0.24 | 9±11*** | 78.70 | 0.000 | |
| | 0.1 | 15±10*** | 66.44 | 0.000 | |
| Note: TTX-S: TTX synthesized by the present disclosure (synthesized by reference to the processes of CN113956266A);<br>Compared with the control group, *: P<0.05, **: P<0.01, ***: P<0.001;<br>#: Magnification of the therapeutic window size is defined as: MTD/effective dose. | | | | | |

The experimental results in Table 6 and FIG. 1 show that in the acetic acid writhing pain efficacy mouse model test, TTX-S was subjected to a dose-effect relationship test with its MTD dose as high dose and 2-fold gradient, and the results show that TTX has a pain inhibition rate of only 65.86% (p<0.001) at its MTD dose of 0.012 mg/kg, a pain inhibition rate of 36.77% (p<0.01) at 0.006 mg/kg, showing a significant analgesic effect, while the pain inhibition rate is only 2.42% (p>0.05) when the dose is reduced to 0.003 mg/kg, which shows no significant analgesic effect, indicating that TTX has a low pain relief intensity in the acetic acid writhing pain efficacy mouse model, and the therapeutic window (i.e., having a therapeutic effect in a certain dose range, animals will die when the dose is increased, and the analgesic effect will be lost when the dose is reduced) has a magnification of only 2 folds, and the concentration of the dose range with analgesic effect is about 0.006 mg/kg- 0.012 mg/kg. However, the pain inhibition rates of Compound 73, Compound 77, Compound 199, Compound 199-B and Compound 210 of the present disclosure at their MTD doses can almost reach 80% or more, and the magnification of the therapeutic window size can reach 4-8 folds. In particular, the maximum pain inhibition rates of Compound 77 and Compound 199-B are respectively as high as 78.64% (p<0.001) and 88.48 (p < 0.001), and the magnification of the therapeutic window size can reach 8 folds. It is shown that the compounds of the present disclosure are significantly stronger than TTX in pain relief intensity, and the therapeutic windows are also significantly larger than TTX.

**Biological Example 4: Study on analgesic effect of compounds in rat formalin inflammatory pain model**

SPF grade SD rats (half male and half female) weighing 180-220 g. 3-5 days after adaptive feeding, the animals were randomly divided into a model control group, a TTX group, and a test group, with 10 mice in each group. The dose of the test group was the MTD dose inferred from the toxicity study in rats. The control group was administered with normal saline, the TTX group and the test group used normal saline as vehicle. The administration volume was 5 mL/kg, the administration route was subcutaneous administration on the neck and back, and the administration frequency and cycle were single administration.

After the positive control and the test substance were pre-administered for 30 min, the right sole was injected with 5% formalin solution through a micro syringe to observe the number of pain responses in rats. Rats have behavioral responses like paw lifting, paw licking, and paw flinching, was considered as a pain response. The spontaneous pain response was observed every 5 min, and the number of pain responses in rats within 1 min was counted for 60 min. The pain inhibition rate was calculated.

Inhibition rate = (number of pain responses in control group - number of pain responses in administration group)/number of pain responses in control group * 100%. The results are shown in Table 7.

**Table 7. Test results of study of analgesic effect in formalin inflammatory pain in SD rat model**

| Compound No. | Pain Inhibition Rate ( %) | P Value (vs Vehicle) |
|---|---|---|
| TTX-N | 64.20*** | 0.000 |
| TTX-S | 54.32*** | 0.000 |
| 65 | 51.55*** | 0.000 |
| 73 | 58.62*** | 0.000 |
| 77 | 97.28*** | 0.000 |
| 103 | 34.05* | 0.010 |
| 109 | 37.21** | 0.005 |

(continued)

| Compound No. | Pain Inhibition Rate ( %) | P Value (vs Vehicle) |
|---|---|---|
| 116 | 69.32*** | 0.000 |
| 121 | 53.66** | 0.000 |
| 123 | 47.74*** | 0.000 |
| 125 | 41.15* | 0.034 |
| 131 | 66.13*** | 0.000 |
| 133 | 60.56*** | 0.000 |
| 139 | 57.06** | 0.003 |
| 144 | 68.68*** | 0.000 |
| 147 | 43.52** | 0.002 |
| 149 | 69.87*** | 0.000 |
| 156 | 41.87*** | 0.000 |
| 160 | 58.88*** | 0.000 |
| 167 | 65.71*** | 0.000 |
| 179 | 52.52*** | 0.000 |
| 181 | 42.44** | 0.001 |
| 182 | 42.00** | 0.001 |
| 183 | 51.20** | 0.008 |
| 185 | 63.15*** | 0.000 |
| 189 | 82.22*** | 0.000 |
| 191 | 71.18*** | 0.000 |
| 193 | 42.26** | 0.001 |
| 197 | 51.74*** | 0.000 |
| 199 | 80.37*** | 0.000 |
| 199-B | 78.68*** | 0.000 |
| 204 | 27.07** | 0.010 |
| 208 | 43.30*** | 0.000 |
| 209 | 38.71*** | 0.000 |
| 210 | 75.29*** | 0.000 |
| 212 | 37.56*** | 0.000 |
| 219 | 97.19*** | 0.000 |
| 220 | 77.66*** | 0.000 |
| 221 | 79.44*** | 0.000 |
| 223 | 86.19*** | 0.000 |
| 229 | 56.89*** | 0.000 |
| 230 | 29.89** | 0.002 |
| 233 | 67.68** | 0.002 |
| Note: TTX-N: TTX extracted from nature, purchased from Kangte (Taizhou) Bioengineering Co., Ltd.; TTX-S: TTX synthesized by the present disclosure (synthesized by reference to the processes of CN113956266A); Compared with the control group, *: P<0.05, **: P<0.01, ***: P<0.001. | | |

The experimental results shown in Table 7 show that the compounds of the present disclosure have excellent analgesic effects in the formalin inflammatory pain rat model, wherein the pain inhibition rates of Compound 77, Compound 189, Compound 199, Compound 199-B, Compound 210, Compound 219, Compound 220, Compound 221, Compound 223 and the like reach 75% or more, which are significantly superior over TTX.

Some compounds were further selected to detect the analgesic effect of each compound in the formalin inflammatory pain rat model at different doses, and the results are shown in Table 8 below and FIG. 2.

**Table 8. Test results of study of analgesic effect in formalin inflammatory pain in SD rat model**

| Compound No. | Dose (mg/kg) | Area under the pain response curve (Times*min) | Pain Inhibition Rate (%) | P Value (vs Vehicle) | Magnification of the therapeutic window size # |
|---|---|---|---|---|---|
| TTX-S | Vehicle | 764.5±156.6 | - | - | 2 fold |
| | 0.012 | 349.3±195.9*** | 54.32 | 0 | |
| | 0.006 | 525.8±207.4* | 31.23 | 0.02 | |
| | 0.003 | 771.4±291.2 | -0.9 | 0.944 | |
| 77 | Vehicle | 919.1±208.5 | - | - | 3 fold |
| | 0.8 | 25.0±36.7*** | 97.28 | 0 | |
| | 0.6 | 346.8±182.8*** | 62.27 | 0 | |
| | 0.3 | 462.8±179.6*** | 49.65 | 0 | |
| 189 | Vehicle | 1152.7±230.9 | - | - | 1 fold |
| | 0.02 | 205±126.73*** | 82.22 | 0 | |
| | 0.01 | 574.5±192.43*** | 50.16 | 0 | |
| | 0.005 | 825.05±261.25** | 28.42 | 0.001 | |
| 199 | Vehicle | 850.55±199.05 | - | - | 1 fold |
| | 0.018 | 256.5±161.84*** | 69.84 | 0 | |
| | 0.012 | 555.5±219.51* | 34.69 | 0.01 | |
| | 0.006 | 532.25±260.66** | 37.42 | 0.005 | |
| 199-B | Vehicle | 938.6±258.3 | - | - | 7 fold |
| | 0.014 | 141.25±87.85*** | 85 | 0 | |
| | 0.003 | 568.25±228.92* | 39.5 | 0 | |
| | 0.002 | 619±113.28** | 34.1 | 0 | |
| 206 | Vehicle | 961.85±278.71 | - | - | 5 fold |
| | 0.02 | 434.25±217.43*** | 54.9 | 0 | |
| | 0.01 | 494.5±139.03*** | 48.6 | 0 | |
| | 0.004 | 709.75±361.5*** | 26.2 | 0.038 | |
| 209 | Vehicle | 1080.45±321.65 | - | - | 5 fold |
| | 0.02 | 142.25±84.54*** | 86.8 | 0 | |
| | 0.01 | 375.25±154.17*** | 65.3 | 0 | |
| | 0.004 | 539±227.78*** | 50.1 | 0 | |

(continued)

| Compound No. | Dose | Area under the pain response curve | Pain Inhibition Rate | P Value | Magnification of the therapeutic window size # |
|---|---|---|---|---|---|
| | (mg/kg) | (Times*min) | (%) | (vs Vehicle) | |
| 223 | Vehicle | 743.8±222.7 | - | - | 6 fold |
| | 0.024 | 103.3±49.1*** | 86.19 | 0 | |
| | 0.0048 | 553.6±199.9* | 25.95 | 0.026 | |
| | 0.004 | 471.8±205.9** | 36.89 | 0.002 | |
| Note: TTX-S: TTX synthesized by the present disclosure (synthesized by reference to the processes of CN113956266A);<br>Compared with the control group, *: P<0.05, **: P<0.01, ***: P<0.001;<br>#: The magnification of the therapeutic window size is defined as: MTD/effective dose. | | | | | |

The experimental results in Table 8 and FIG. 2 show that in the test of the formalin inflammatory pain rat model, TTX-S was subjected to a dose-effect relationship test with its MTD dose as high dose and 2-fold gradient, and the results show that TTX has a pain inhibition rate of only 54.32% ($p<0.001$) at its MTD dose of 0.012 mg/kg, a pain inhibition rate of 31.23% ($p<0.05$) at 0.006 mg/kg, showing a significant analgesic effect, while the pain inhibition rate is -0.9% when the dose is reduced to 0.003 mg/kg, which shows no analgesic effect, indicating that TTX has a low pain relief intensity in the formalin inflammatory pain rate model, and the therapeutic window (i.e., having a therapeutic effect in a certain dose range, animals will die when the dose is increased, and the analgesic effect will be lost when the dose is reduced) has a magnification of only 2 folds, and the dose range with analgesic effect is 0.006 mg/kg-0.012 mg/kg. However, the pain inhibition rates of Compound 77, Compound 189, Compound 199, Compound 199-B, Compound 209 and Compound 223 in the present disclosure at their MTD doses can almost reach 80% or more, and the magnification of the therapeutic window size can reach 4-7 folds. Particularly, for Compound 199-B, the maximum pain inhibition rate is 85.0% ($p<0.001$), and the magnification of the therapeutic window size reaches 7 folds. It is shown that the compounds of the present disclosure are significantly stronger than TTX in pain relief intensity, and the therapeutic windows are also significantly larger than TTX.

The above description only provides specific examples of the present disclosure without limiting the scope of the present disclosure. Any equivalent transformation made by using the present disclosure, or direct or indirect application in other relevant technical fields, is likewise included in the protection scope of the present disclosure.

## Claims

**1.** A compound having a structure of formula (I) or a pharmaceutically acceptable derivative thereof:

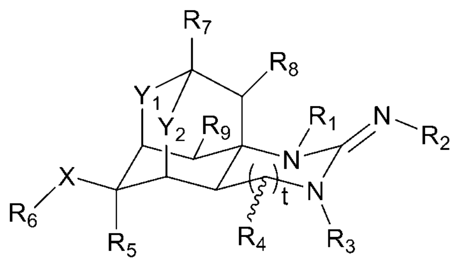

Formula (I)

wherein,

X is absent or selected from the group consisting of $-(CH_2)_m-$ and

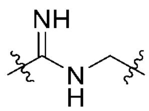

,

wherein - $CH_2$- is optionally replaced by -O- or carbonyl or optionally substituted with at least one $R^X$;

$Y_1$ and $Y_2$ are each independently selected from the group consisting of methylene, O, S and NH, wherein methylene and NH are optionally substituted with at least one $R^X$;

$R_1$ and $R_3$ are each independently selected from the group consisting of hydrogen, hydroxyl, amino, cyano, $C_{1-8}$ alkyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl and $C_{1-8}$ alkyl-$C_{5-10}$ heteroaryl, wherein the alkyl, cycloalkyl, heterocyclyl, aryl and heteroaryl are optionally substituted with at least one $R^X$;

$R_2$ is selected from the group consisting of hydrogen, hydroxyl, amino, formyl, acetyl, $C_{1-8}$ alkyl, $C_{3-8}$ cycloalkyl, $C_{3-8}$ heterocyclyl, -(C=O)$OR_{10}$ and -O(C=O)$R_{11}$, wherein the formyl, acetyl, alkyl, cycloalkyl and heterocyclyl are optionally substituted with at least one $R^X$;

each $R_4$ is independently selected from the group consisting of hydrogen, deuterium, hydroxyl, halogen, -$OR_{12}$ and $C_{1-8}$ alkyl;

or $R_3$ and $R_4$ together with the atom(s) to which they are attached form a 5-12 membered heterocycle, wherein the heterocycle is optionally substituted with at least one $R^X$;

$R_5$ is selected from the group consisting of hydrogen, deuterium, tritium, hydroxyl, halogen, $C_{1-8}$ alkyl, $C_{3-8}$ cycloalkyl and -$OR_{13}$;

$R_6$ is selected from the group consisting of hydroxyl, mercapto, amino, azido, halogen, cyano, $C_{1-12}$ alkyl, $C_{2-8}$ alkenyl, -O-$C_{1-12}$ alkyl, $C_{3-8}$ cycloalkyl, $C_{3-8}$ heterocyclyl, $C_{6-10}$ aryl, $C_{5-10}$heteroaryl, -(C=O)-$R_{14}$, guanidino, ureido, -$(OCH_2CH_2)_n$-OH,

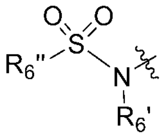 , 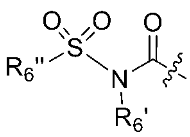 ,

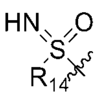 and 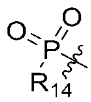 ,

wherein the alkyl, amino, cycloalkyl, heterocyclyl, aryl, heteroaryl, guanidino and ureido are optionally substituted with at least one $R^X$;

or $R_5$ and $R_6$ together with the atom(s) to which they are attached form a 3-12 membered heterocycle, wherein the heterocycle is optionally substituted with at least one $R^X$;

$R_7$ is selected from the group consisting of hydrogen, deuterium, tritium, hydroxyl, halogen, $C_{1-4}$ alkyl, -O-$C_{1-4}$ alkyl, $C_{3-8}$ cycloalkyl and $C_{3-8}$ heterocyclyl, wherein the alkyl, cycloalkyl and heterocyclyl are optionally substituted with at least one $R^X$;

$R_8$ is selected from the group consisting of hydrogen, deuterium, tritium, hydroxyl, halogen, $C_{1-8}$ alkyl, $C_{3-8}$ cycloalkyl and -$OR_{15}$;

or $R_4$ and $R_8$ together with the atom(s) to which they are attached form a 5-12 membered heterocycle, wherein the heterocycle is optionally substituted with at least one $R^X$;

$R_9$ is selected from the group consisting of hydrogen, deuterium, tritium, hydroxyl, $C_{1-8}$ alkyl, $C_{3-8}$ cycloalkyl and -$OR_{16}$;

$R_{10}$ and $R_{11}$ are each independently selected from the group consisting of $C_{1-8}$ alkyl, $C_{3-8}$ cycloalkyl, $C_{3-8}$ heterocyclyl, $C_{6-20}$ aryl, $C_{1-8}$ alkyl-$C_{6-20}$ aryl, $C_{5-20}$ heteroaryl and $C_{1-8}$ alkyl-$C_{5-20}$ heteroaryl, wherein the alkyl, cycloalkyl, heterocyclyl, aryl and heteroaryl are optionally substituted with at least one $R^X$;

each of $R_{12}$, $R_{13}$, $R_{14}$, $R_{15}$ and $R_{16}$ is independently selected from the group consisting of $C_{1-8}$ alkyl, $C_{2-8}$ alkenyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl, and $C_{1-8}$ alkyl-$C_{5-10}$ heteroaryl, wherein the alkyl, cycloalkyl, heterocyclyl, aryl, and heteroaryl are optionally substituted with at least one $R^X$;

$R_{6'}$ is selected from the group consisting of hydrogen, hydroxyl, amino, cyano, $C_{1-8}$ alkyl, $C_{3-8}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl and $C_{1-8}$ alkyl-$C_{5-10}$ heteroaryl, wherein the alkyl, cycloalkyl, heterocyclyl, aryl and heteroaryl are optionally substituted with at least one $R^X$;

$R_{6''}$ is selected from the group consisting of hydroxyl, mercapto, amino, halogen, $C_{1-12}$ alkyl, $C_{2-8}$ alkenyl, -O-$C_{1-12}$ alkyl, $C_{3-8}$ cycloalkyl, $C_{3-8}$ heterocyclyl, $C_{6-10}$ aryl and $C_{5-10}$ heteroaryl, wherein the alkyl, amino, cycloalkyl, heterocyclyl, aryl and heteroaryl are optionally substituted with at least one $R^X$;

each $R^X$ is independently selected from the group consisting of hydrogen, halogen, hydroxyl, carboxyl, amino, cyano, formyl, $C_{1-8}$ alkyl, -O-$C_{1-8}$ alkyl, $C_{2-8}$ alkenyl, $C_{3-20}$ cycloalkyl, $C_{1-8}$ alkyl-$C_{3-8}$ cycloalkyl, $C_{3-8}$ heterocyclyl, $C_{1-8}$ alkyl-$C_{3-8}$ heterocyclyl, $C_{6-10}$ aryl, $C_{1-8}$ alkyl-$C_{6-10}$ aryl, $C_{5-10}$ heteroaryl, $C_{1-8}$ alkyl-$C_{5-10}$ heteroaryl,

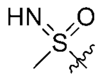 , 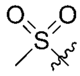 and 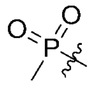 ,

wherein the amino, formyl, alkyl, cycloalkyl, heterocyclyl, aryl and heteroaryl are optionally substituted with at least one $R^Y$;
each $R^Y$ is independently selected from the group consisting of hydrogen, halogen, hydroxyl, amino, carboxyl, formyl, acetyl, $C_{1-8}$ alkyl, -O-$C_{1-8}$ alkyl, $C_{3-8}$ cycloalkyl, -O-$C_{3-8}$ cycloalkyl, $C_{3-8}$ heterocyclyl, -O-$C_{3-8}$ heterocyclyl, $C_{1-8}$ alkyl-$C_{3-8}$ heterocyclyl, $C_{6-10}$ aryl, -O-$C_{6-10}$ aryl, $C_{1-8}$ alkyl-$C_{6-10}$ aryl, $C_{5-10}$ heteroaryl, -O-$C_{5-10}$ heteroaryl and $C_{1-8}$ alkyl-$C_{5-10}$ heteroaryl, wherein the formyl, acetyl, alkyl, cycloalkyl, heterocyclyl, aryl, and heteroaryl are optionally substituted with at least one halogen, hydroxyl, or $R^{Y1}$;
each $R^{Y1}$ is independently selected from the group consisting of amino, $C_{1-8}$ alkyl, $C_{3-8}$ cycloalkyl, $C_{3-8}$ heterocyclyl, $C_{6-1O}$ aryl, and $C_{5-10}$ heteroaryl, wherein the alkyl, cycloalkyl, heterocyclyl, aryl, and heteroaryl are optionally substituted with at least one $R^{Y2}$;
each $R^{Y2}$ is independently selected from the group consisting of amino, $C_{1-8}$ alkyl, $C_{3-8}$ cycloalkyl, $C_{3-8}$ heterocyclyl, $C_{6-10}$ aryl, and $C_{5-10}$ heteroaryl;
when X is methylene and $R_1$, $R_2$ and $R_3$ are hydrogen at the same time, $R_4$, $R_5$, $R_6$, $R_7$, $R_8$ and $R_9$ are not hydroxyl at the same time;
m is an integer selected from 0-6;
n is an integer selected from 1-12;
t is an integer selected from 0-4.

**2.** The compound according to claim 1 or a pharmaceutically acceptable derivative thereof, wherein X is selected from the group consisting of methylene, carbonyl and

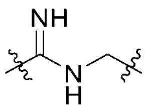 .

**3.** The compound according to claim 1 or 2 or a pharmaceutically acceptable derivative thereof, wherein $R_1$ is hydrogen.

**4.** The compound according to any one of claims 1-3 or a pharmaceutically acceptable derivative thereof, wherein $R_2$ is selected from the group consisting of hydrogen, acetyl and - (C=O)O$R_{10}$.

**5.** The compound according to claim 4 or a pharmaceutically acceptable derivative thereof, wherein $R_{10}$ is selected from the group consisting of benzyl and cyclopentyl.

**6.** The compound according to any one of claims 1-5 or a pharmaceutically acceptable derivative thereof, wherein $R_3$ is selected from the group consisting of hydrogen, $C_{1-8}$ alkyl, $C_{3-8}$ cycloalkyl, $C_{1-8}$ alkyl-$C_{3-8}$ cycloalkyl and $C_{1-8}$ alkyl-$C_{6-10}$ aryl, wherein the alkyl, cycloalkyl and aryl are optionally substituted with at least one $R^X$.

**7.** The compound according to claim 6 or a pharmaceutically acceptable derivative thereof, wherein $R^X$ is selected from the group consisting of hydroxyl and carboxyl.

**8.** The compound according to claim 6 or a pharmaceutically acceptable derivative thereof, wherein $R_3$ is selected from the group consisting of hydrogen, methyl, ethyl, isopropyl,

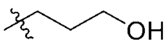 , 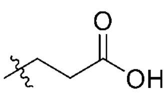 , 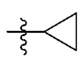 , 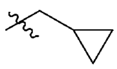 , 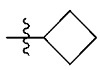 , 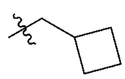 and 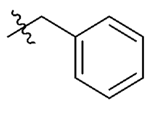 ;

preferably hydrogen and

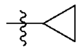.

**9.** The compound according to any one of claims 1-8 or a pharmaceutically acceptable derivative thereof, wherein $R_4$ is selected from the group consisting of hydrogen, deuterium and hydroxyl.

**10.** The compound according to any one of claims 1-9 or a pharmaceutically acceptable derivative thereof, wherein $R_3$ and $R_4$ together with the atom(s) to which they are attached form a 6-membered heterocycle.

**11.** The compound according to claim 10 or a pharmaceutically acceptable derivative thereof, wherein $R_3$ and $R_4$ together with the atom(s) to which they are attached form a heterocycle selected from the group consisting of

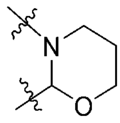 and 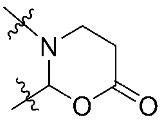.

**12.** The compound according to any one of claims 1-11 or a pharmaceutically acceptable derivative thereof, wherein $R_5$ is hydroxyl.

**13.** The compound according to any one of claims 1-12 or a pharmaceutically acceptable derivative thereof, wherein $R_6$ is selected from the group consisting of hydroxyl, amino, cyano, -O-$C_{1-12}$ alkyl, guanidino, ureido, $C_{3-8}$ heterocyclyl and

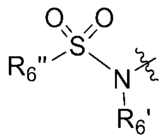,

wherein the amino, alkyl, guanidino and heterocyclyl are optionally substituted with at least one $R^X$.

**14.** The compound according to claim 13 or a pharmaceutically acceptable derivative thereof, wherein $R^X$ is selected from the group consisting of halogen, hydroxyl, amino, formyl, $C_{1-8}$ alkyl, $C_{3-20}$ cycloalkyl, $C_{1-8}$ alkyl-$C_{6-10}$ aryl, $C_{3-8}$ heterocyclyl and

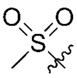,

wherein the amino, formyl, alkyl, cycloalkyl and heterocyclyl are optionally substituted with at least one $R^Y$.

**15.** The compound according to claim 14 or a pharmaceutically acceptable derivative thereof, wherein $R^Y$ is selected from the group consisting of halogen, hydroxyl, carboxyl, formyl, acetyl and $C_{1-8}$ alkyl, wherein the formyl is optionally substituted with hydroxyl or $R^{Y1}$; $R^{Y1}$ is selected from the group consisting of amino, the amino is optionally substituted with $R^{Y2}$; $R^{Y2}$ is selected from the group consisting of $C_{1-8}$ alkyl.

**16.** The compound according to claim 13 or a pharmaceutically acceptable derivative thereof, wherein $R_{6'}$ is selected from the group consisting of hydrogen and methyl.

**17.** The compound according to claim 13 or pharmaceutically acceptable derivative thereof, wherein $R_{6''}$ is selected from the group consisting of $C_{1-12}$ alkyl, $C_{3-8}$ cycloalkyl, $C_{6-10}$ aryl and $C_{5-10}$ heteroaryl, wherein the alkyl, cycloalkyl, aryl and heteroaryl are optionally substituted with at least one $R^X$.

**18.** The compound according to claim 17 or a pharmaceutically acceptable derivative thereof, wherein $R^X$ is selected from the group consisting of halogen, $C_{1-8}$ alkyl, -O-$C_{1-8}$ alkyl, $C_{3-20}$ cycloalkyl and $C_{6-10}$ aryl, wherein the alkyl, cycloalkyl and aryl are optionally substituted with at least one $R^Y$.

**19.** The compound according to claim 18 or a pharmaceutically acceptable derivative thereof, wherein $R^Y$ is halogen, the halogen is fluorine.

**20.** The compound according to claim 17 or a pharmaceutically acceptable derivative thereof, wherein $R_{6''}$ is selected from the group consisting of methyl, trifluoromethyl, propyl, isopropyl,

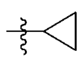, 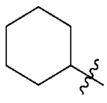, 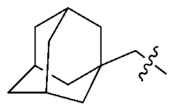, 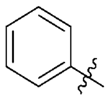, 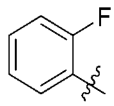, 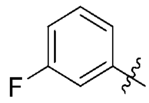, 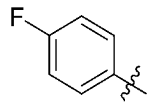,

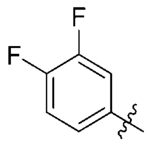, 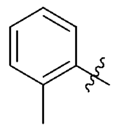, 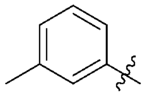, 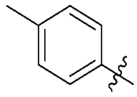, 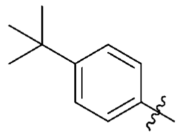, 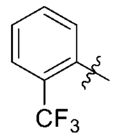, 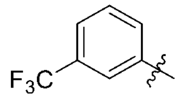,

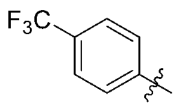, 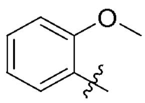, 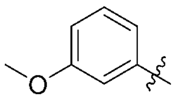, 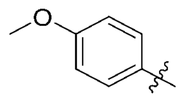, 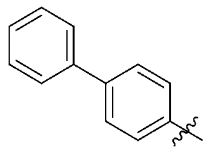,

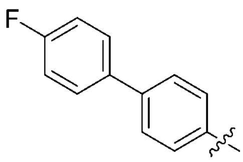, 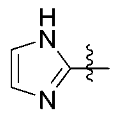, 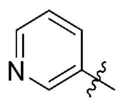, 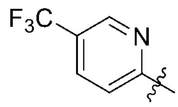, 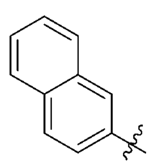 and 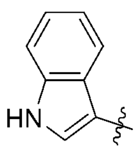.

**21.** The compound according to any one of claims 13-20 or a pharmaceutically acceptable derivative thereof, wherein $R_6$ is selected from the group consisting of hydroxyl, halogen, cyano, amino, azido, methoxy, methylamino, tert-butyl, propenyl, guanidino, ureido,

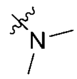,

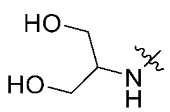, 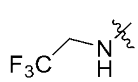, 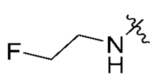, 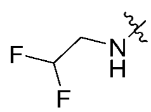, 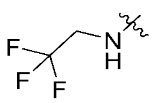, 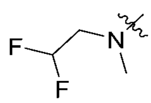,

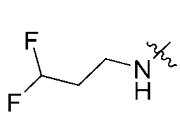, 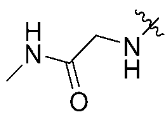, 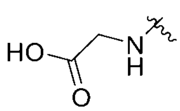, 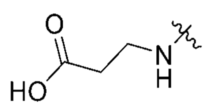, 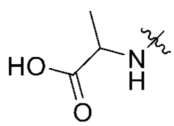, 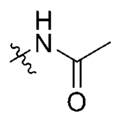,

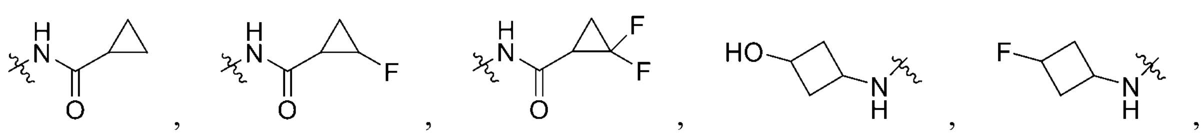

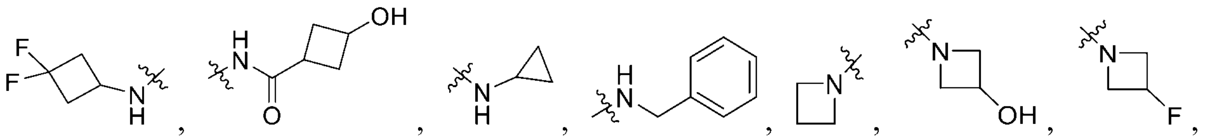
, , , , , , ,

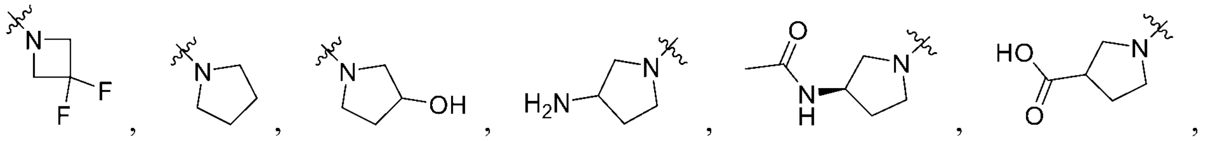
, , , , , ,

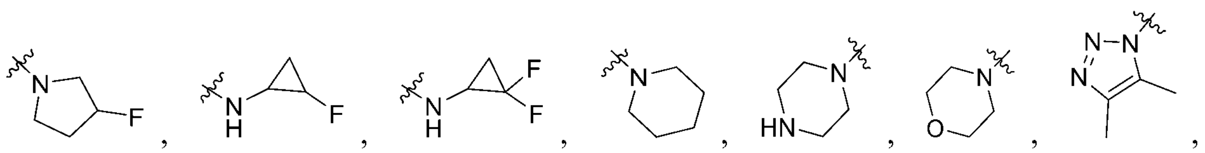
, , , , , , ,

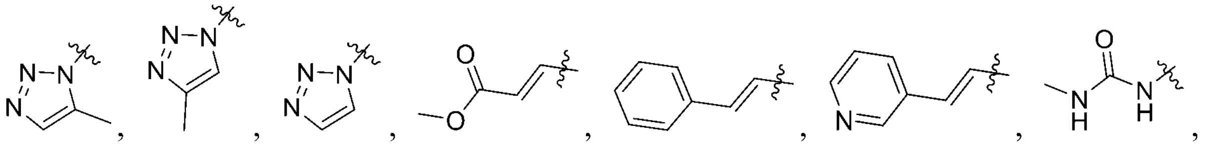
, , , , , , ,

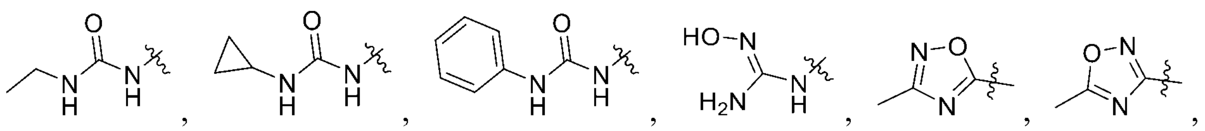
, , , , , ,

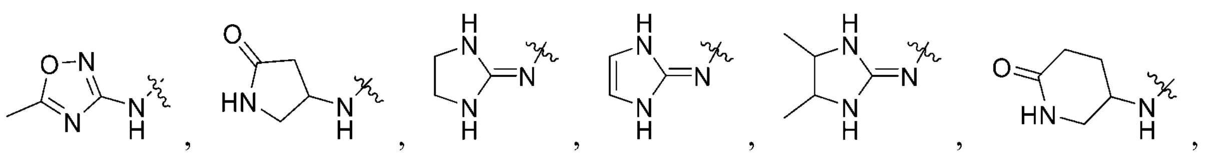
, , , , , ,

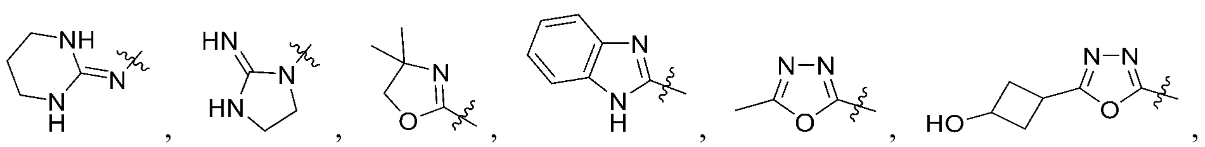
, , , , , ,

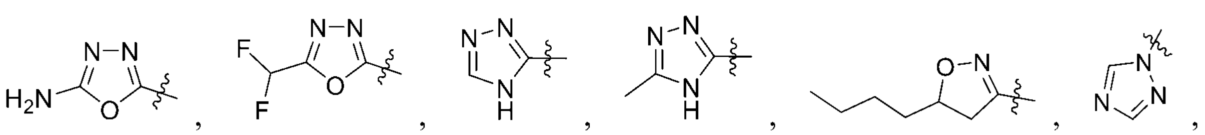
, , , , , ,

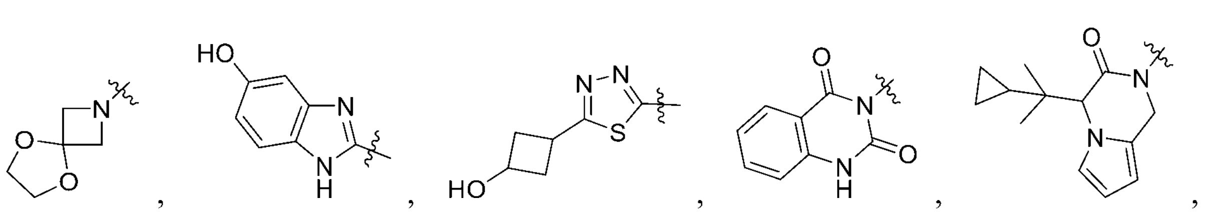
, , , , ,

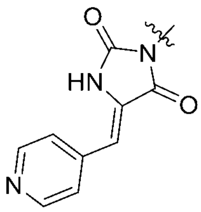, 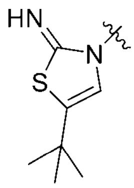, 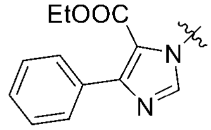, 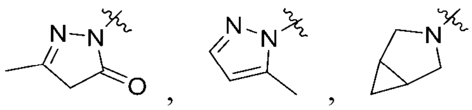, , ,

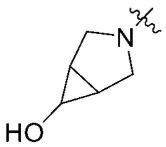, 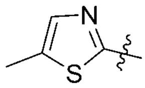, 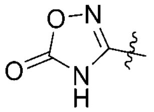, 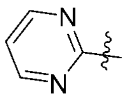, 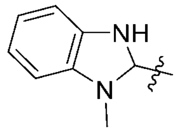, 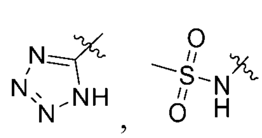, ,

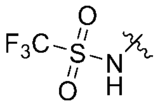, 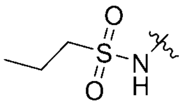, 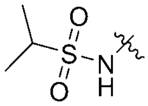, 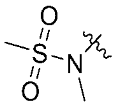, 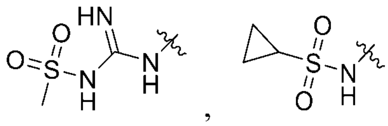, ,

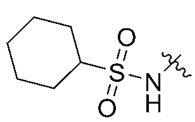, 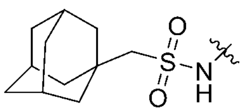, 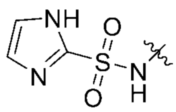, 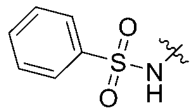, 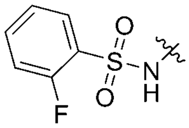,

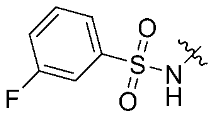, 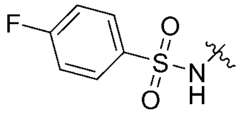, 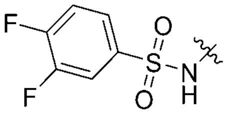, 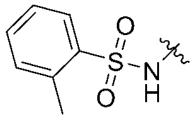, 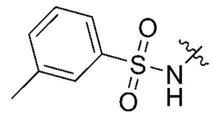,

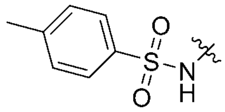, 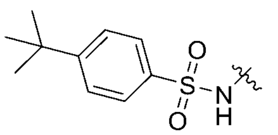, 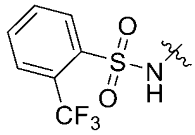, 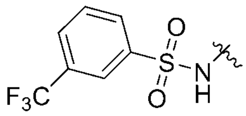,

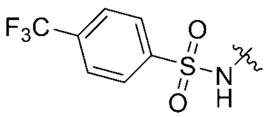, 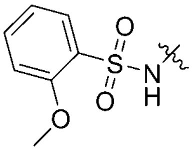, 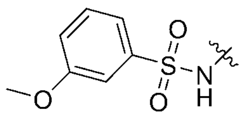, 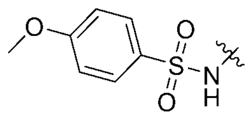,

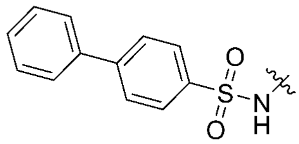, 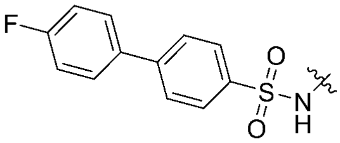, 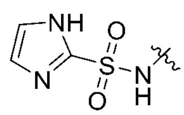, 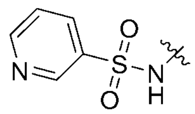,

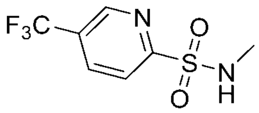, 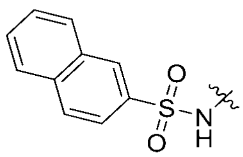 and 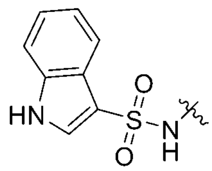;

preferably hydroxyl, amino, methoxy, methylamino, guanidino, ureido,

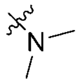,

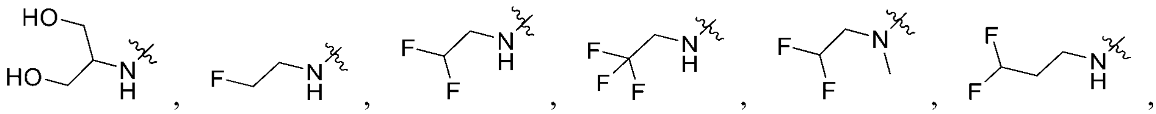
, , , , , ,

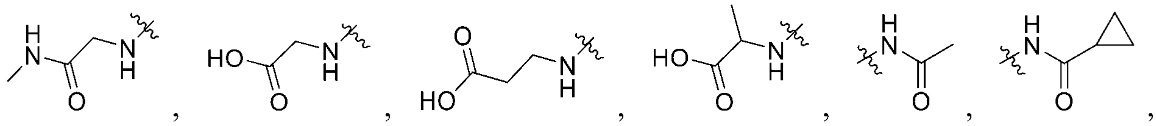
, , , , , ,

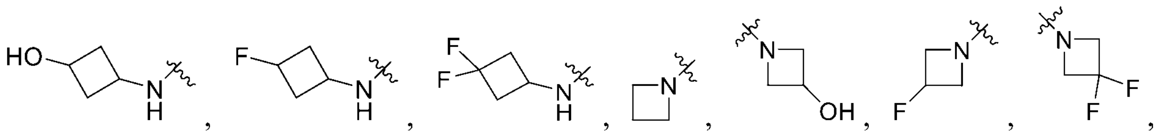
, , , , , , ,

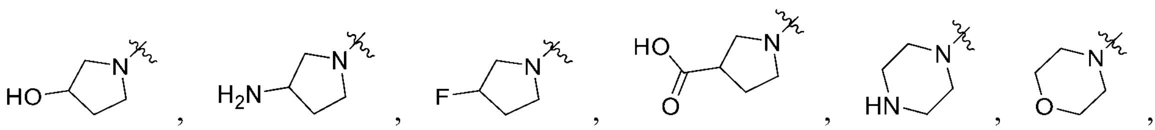
, , , , , ,

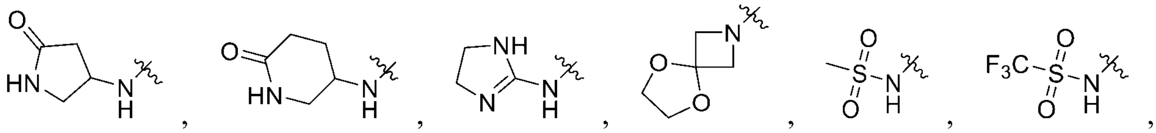
, , , , , ,

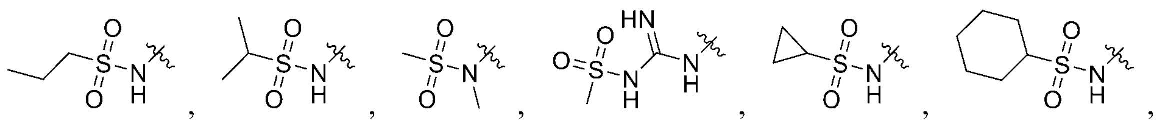
, , , , , ,

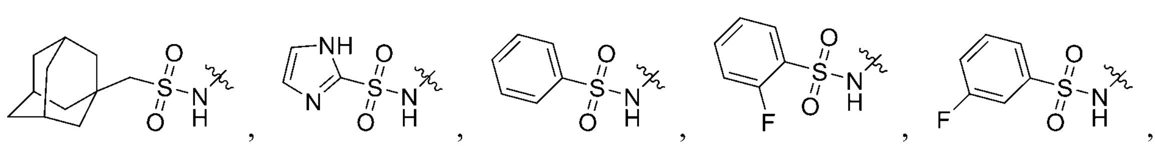
, , , , ,

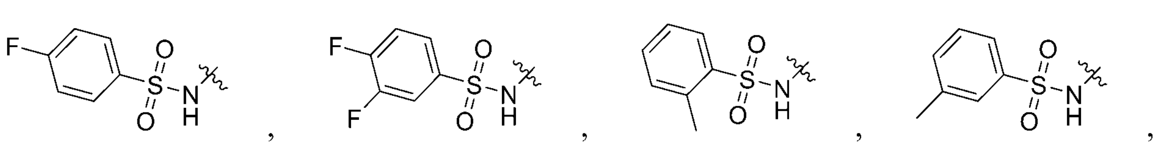
, , , ,

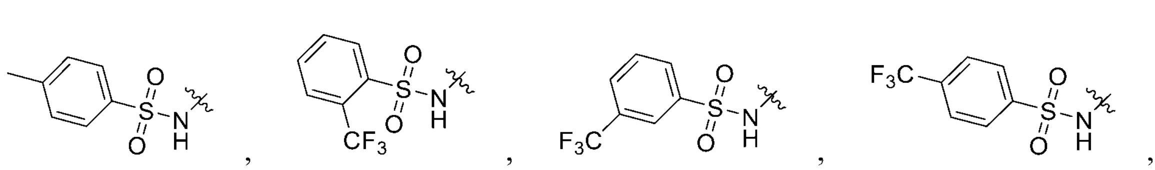
, , , ,

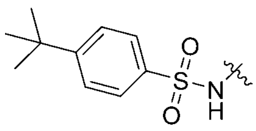, 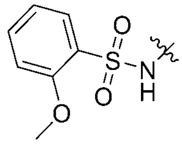, 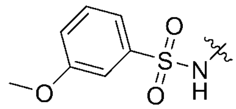, 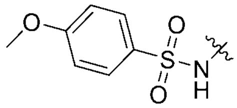,

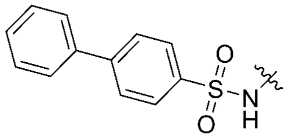, 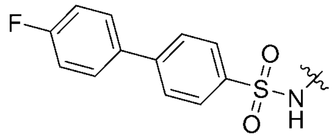, 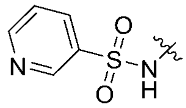, 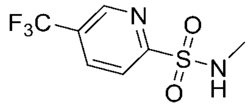,

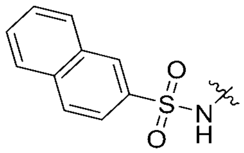 and 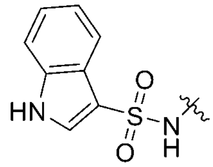;

more preferably hydroxyl, amino, methylamino, guanidino, ureido,

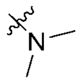, 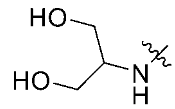,

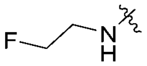, 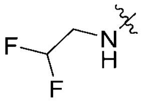, 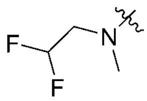, 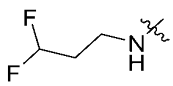, 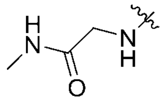, 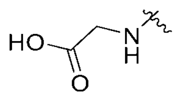,

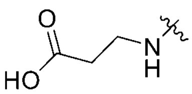, 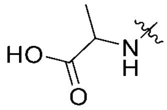, 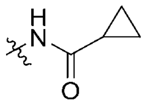, 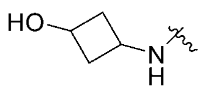, 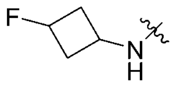,

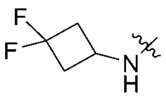, 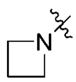, 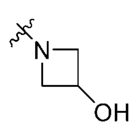, 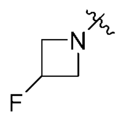, 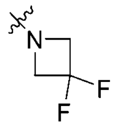, 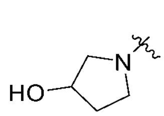, 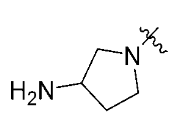,

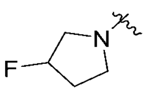, 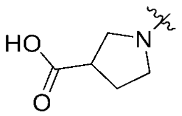, 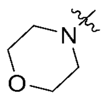, 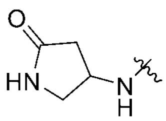, 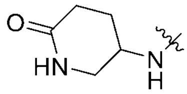, 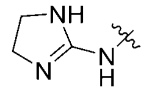,

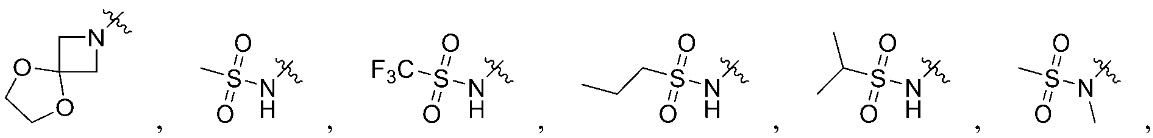

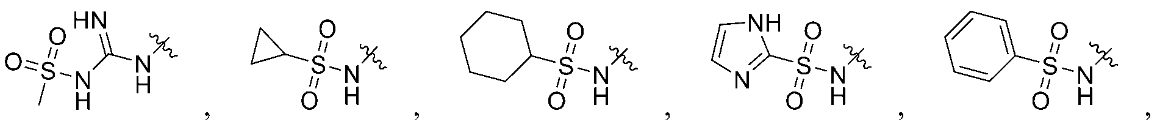

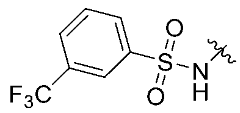, 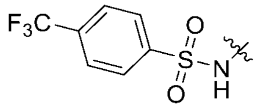, 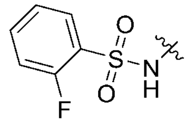, 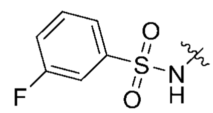,

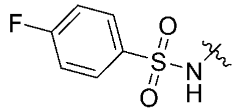, 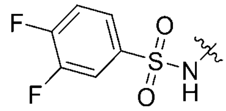, 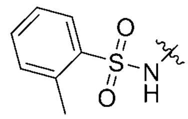, 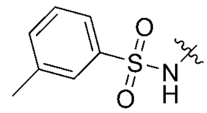,

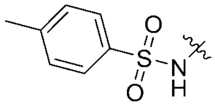, 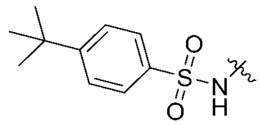, 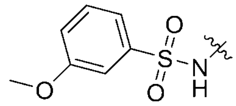, 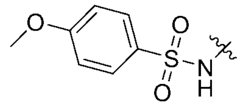,

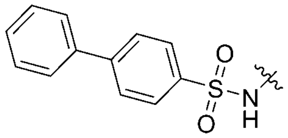, 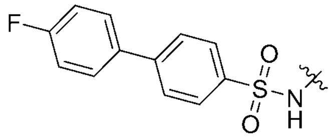, 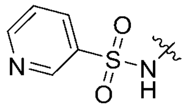, 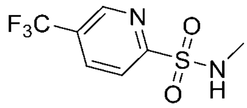,

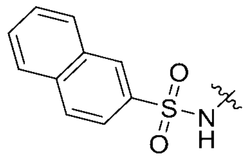 and 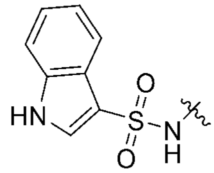.

**22.** The compound according to any one of claims 1-21 or a pharmaceutically acceptable derivative thereof, wherein $R_7$ is hydroxyl.

**23.** The compound according to any one of claims 1-22 or a pharmaceutically acceptable derivative thereof, wherein $R_8$ is hydroxyl.

**24.** The compound according to any one of claims 1-23 or a pharmaceutically acceptable derivative thereof, wherein $R_4$ and $R_8$ together with the atom(s) to which they are attached form a heterocycle, the compound having the following structure:

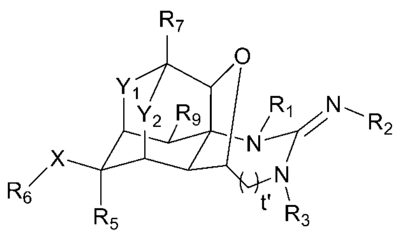,

wherein X, $Y_1$, $Y_2$, $R_1$, $R_2$, $R_3$, $R_5$, $R_6$, $R_7$ and $R_9$ are as defined in any one of claims 1-23, and t' is an integer selected from 0-3.

**25.** The compound according to any one of claims 1-24 or a pharmaceutically acceptable derivative thereof, wherein $R_4$ and $R_8$ together with the atom(s) to which they are attached form a heterocycle, the compound having the following structure:

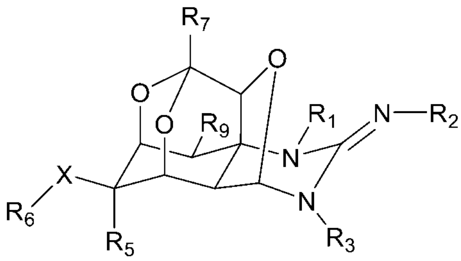

,

wherein X, $R_1$, $R_2$, $R_3$, $R_5$, $R_6$, $R_7$, and $R_9$ are as defined in any one of claims 1-24.

**26.** The compound according to any one of claims 1-25 or a pharmaceutically acceptable derivative thereof, wherein $R_9$ is hydroxyl.

**27.** A compound having the following structure or a pharmaceutically acceptable derivative thereof:

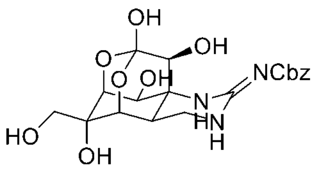
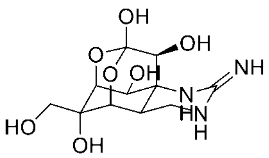
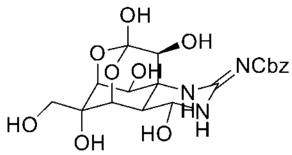
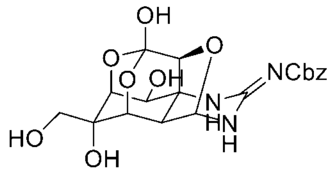

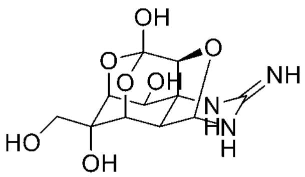
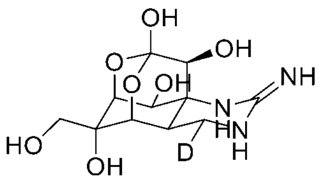
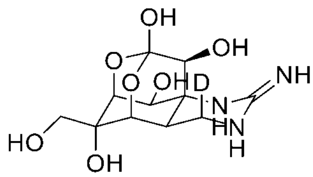

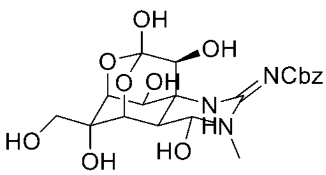
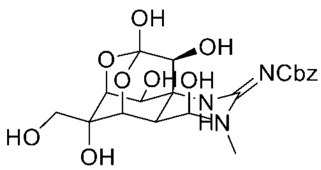
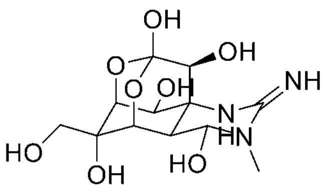

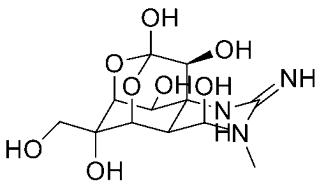
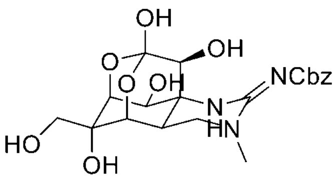
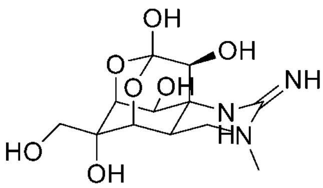

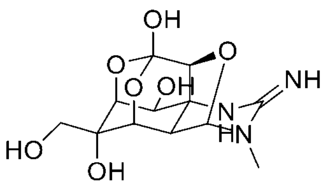
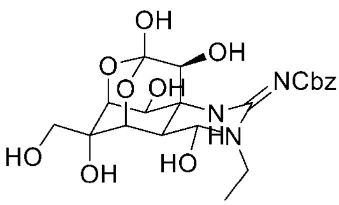
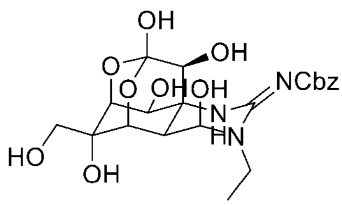

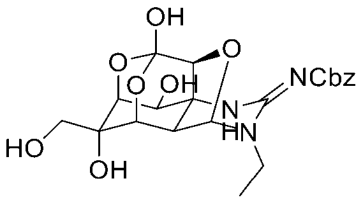
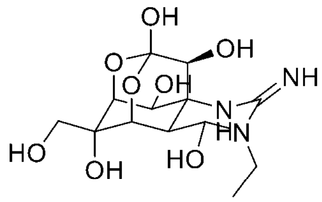
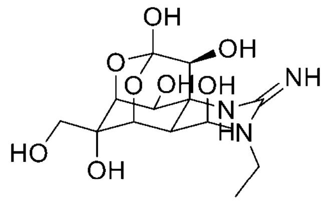
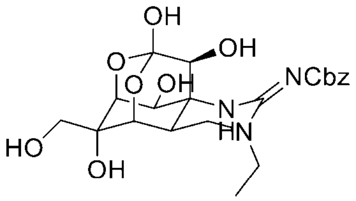
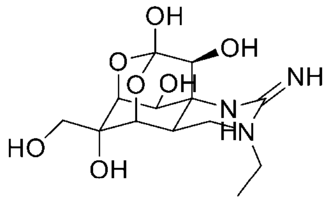
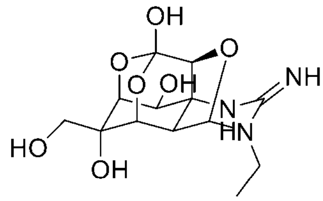
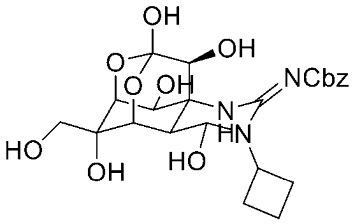
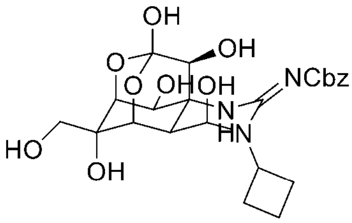
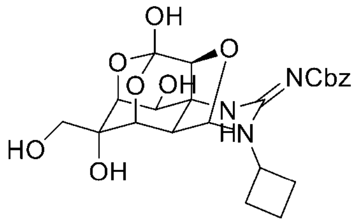
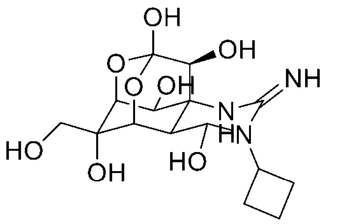
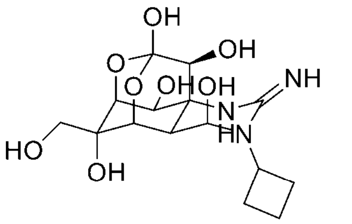
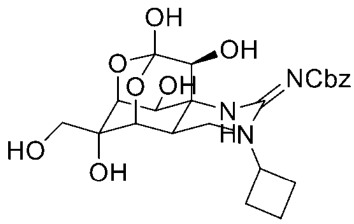
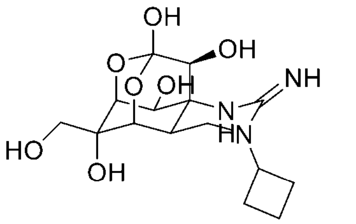
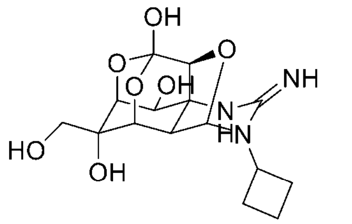
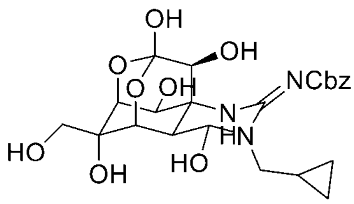
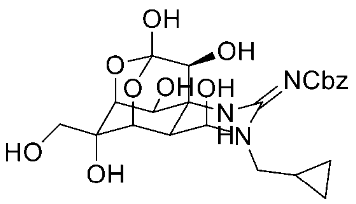
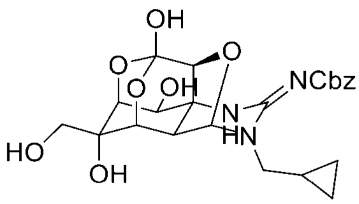
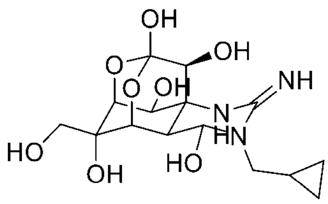
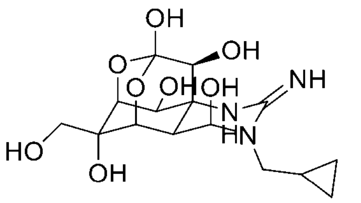
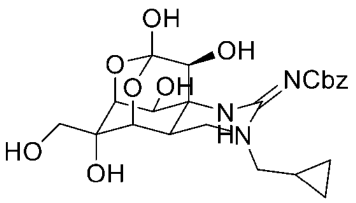
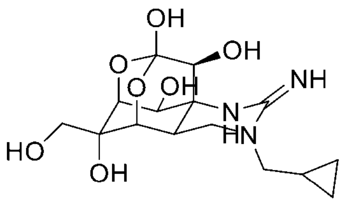

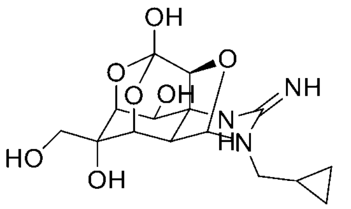
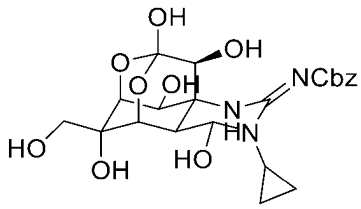
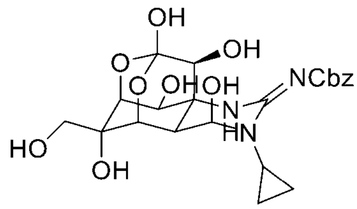

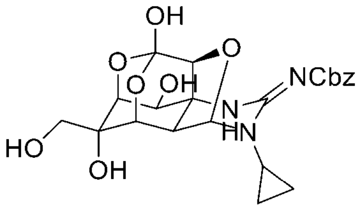
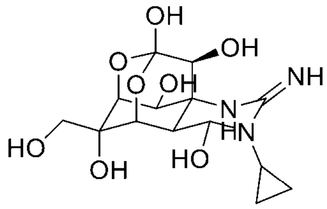
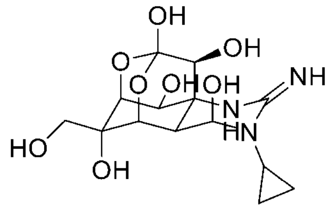

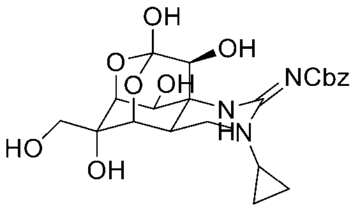
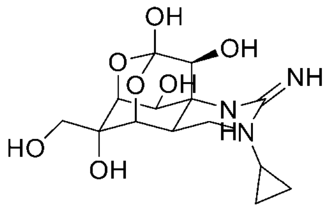
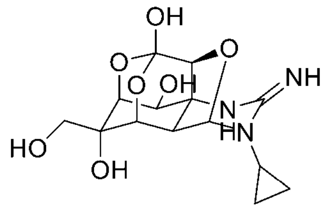

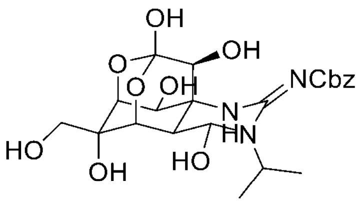
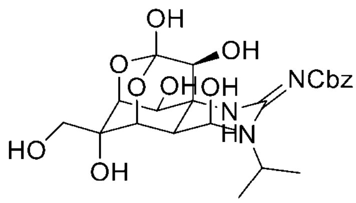
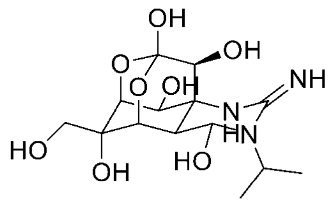

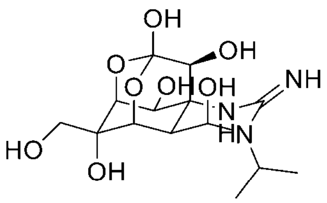
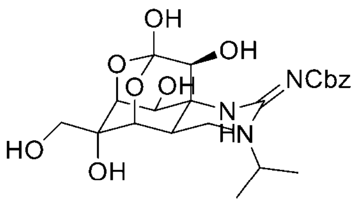
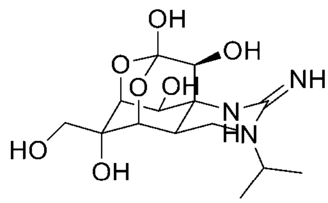

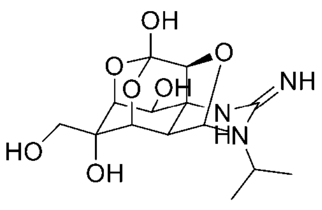
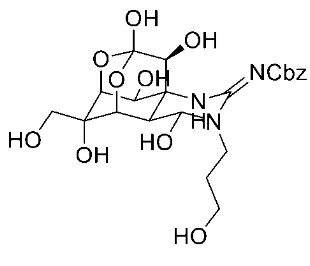
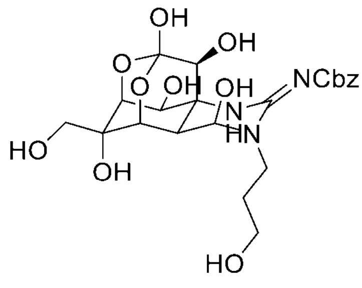

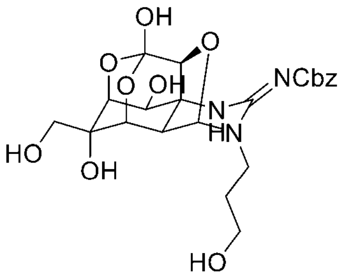
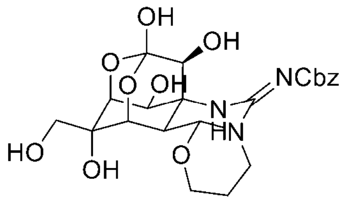
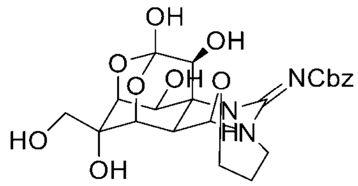

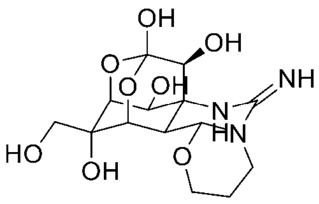
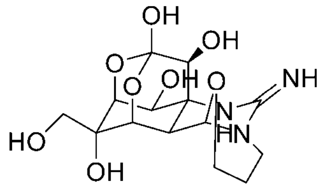
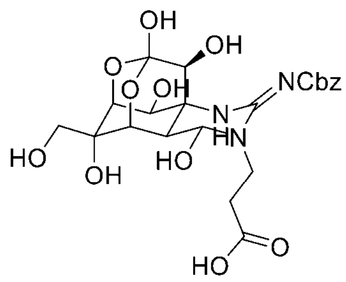

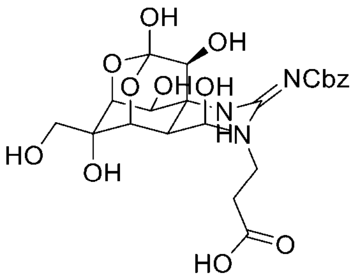
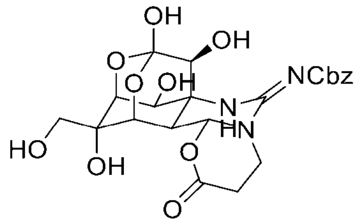
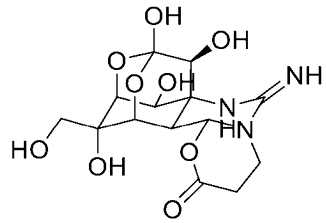

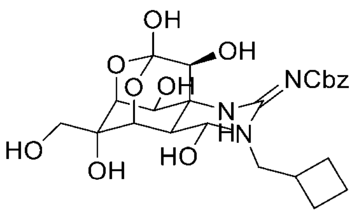
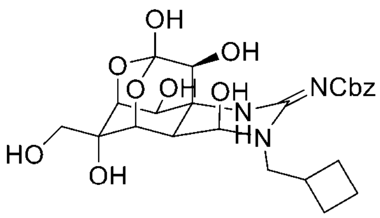
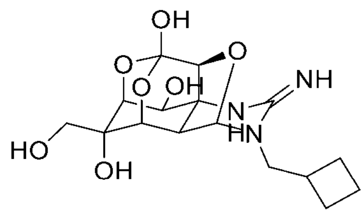

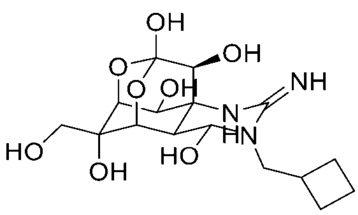
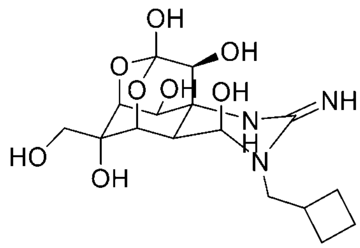
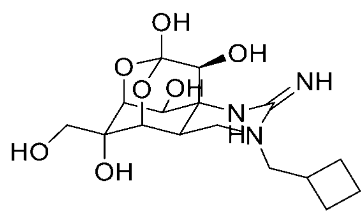

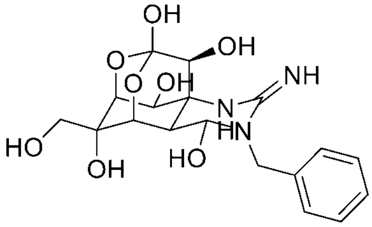
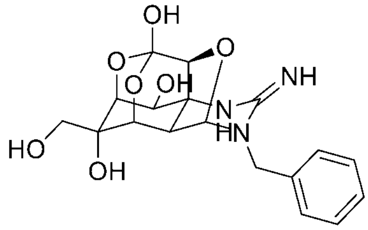
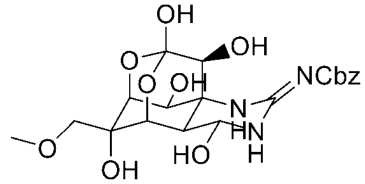

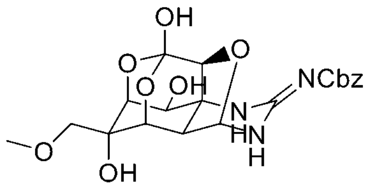
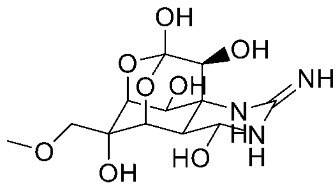
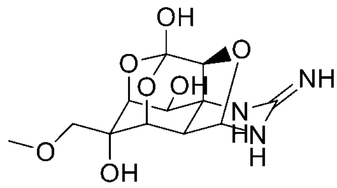

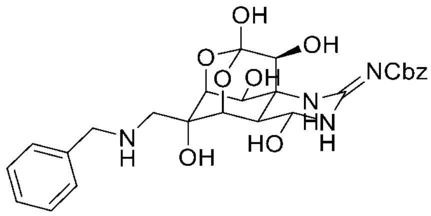
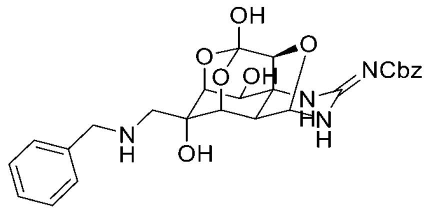

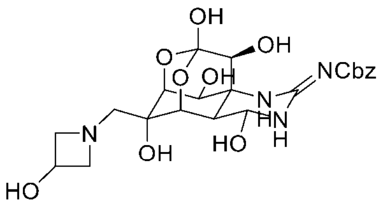
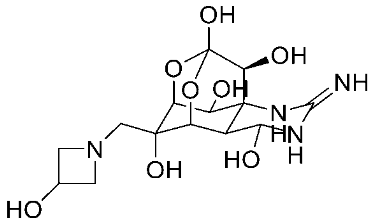
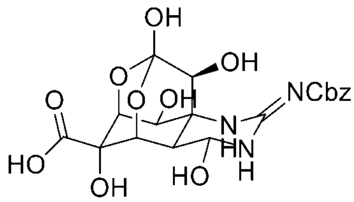

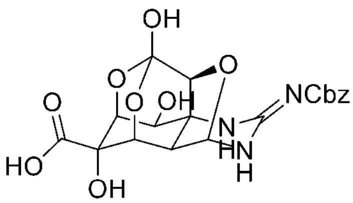
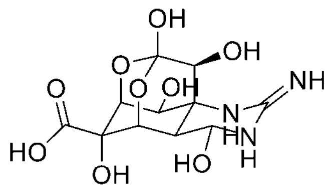
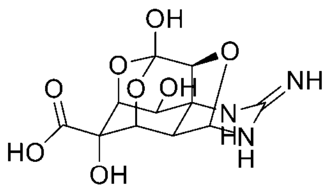

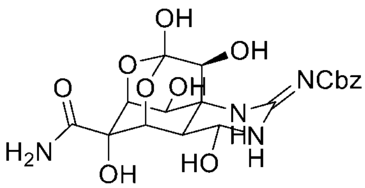
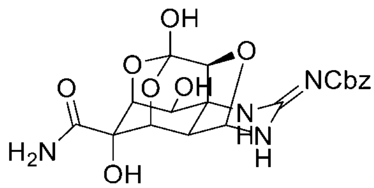
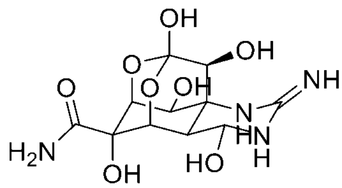

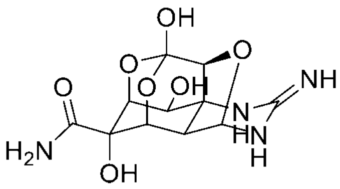
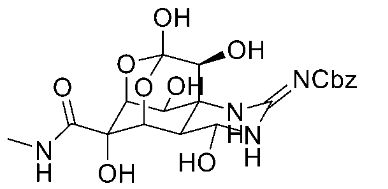
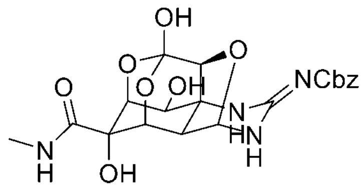

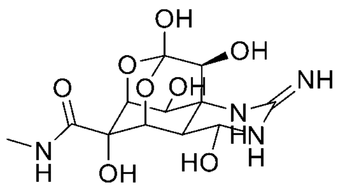
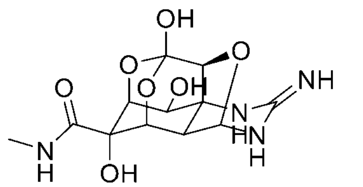
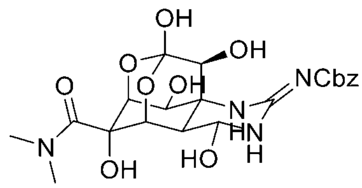

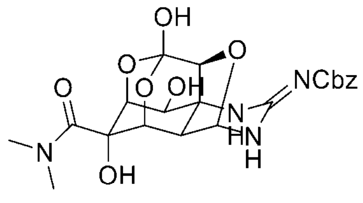 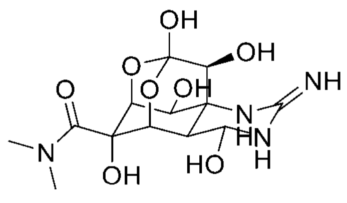 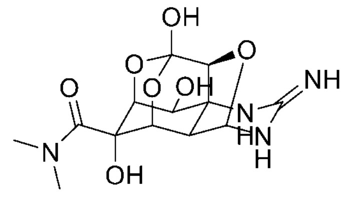

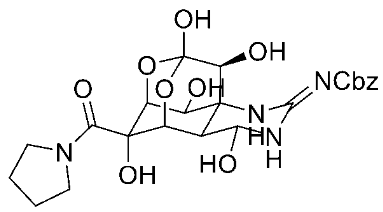 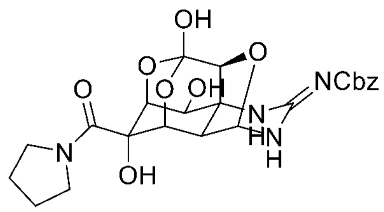 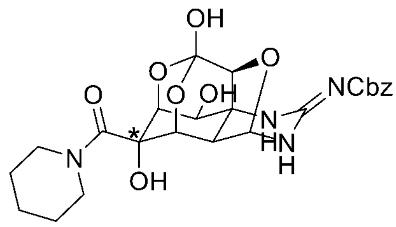

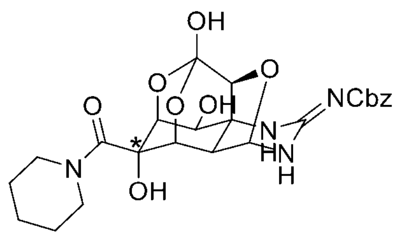 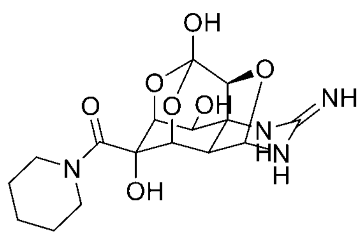 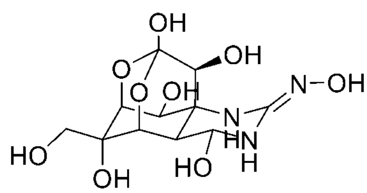

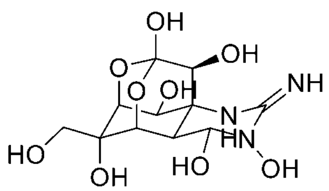 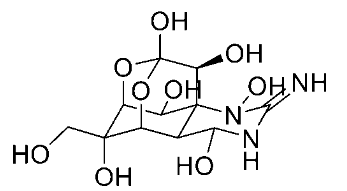 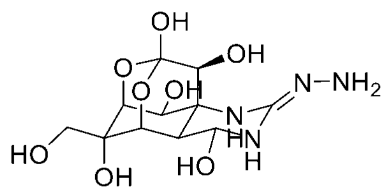

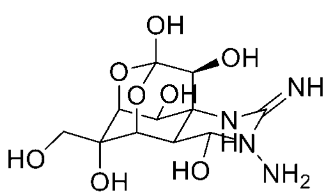 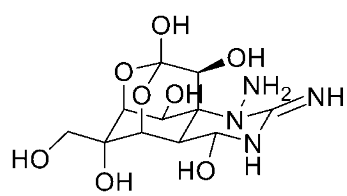 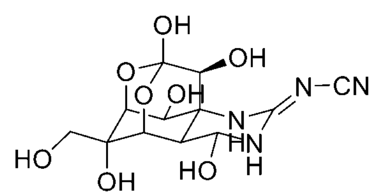

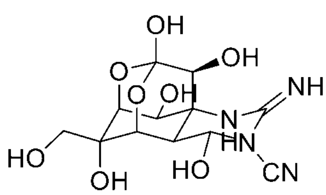 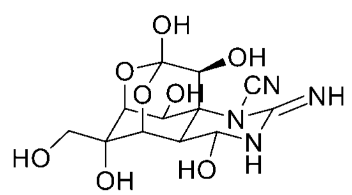 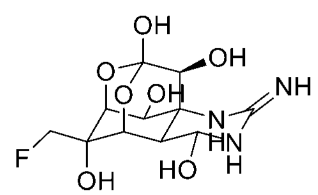

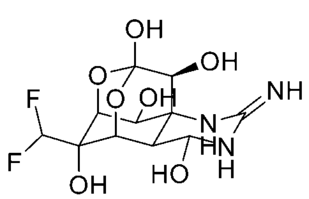 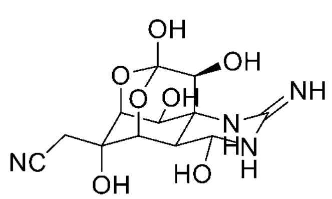 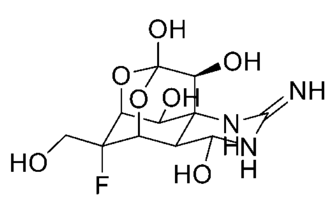

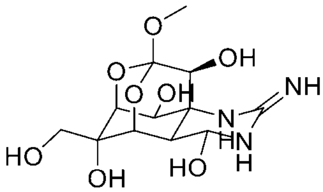
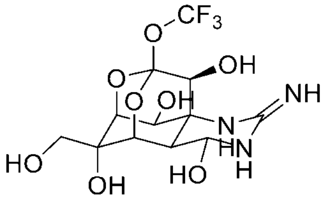
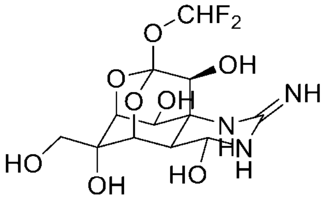

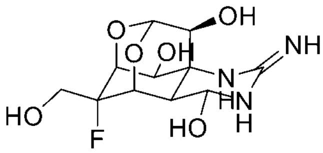
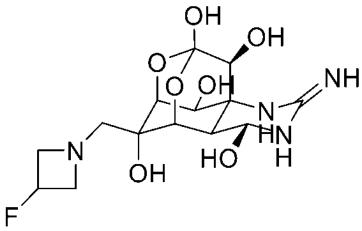
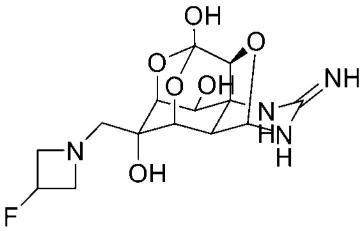

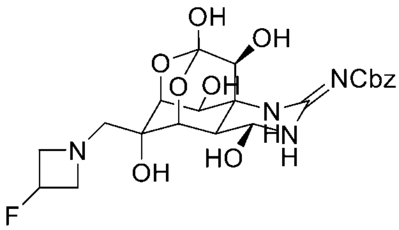
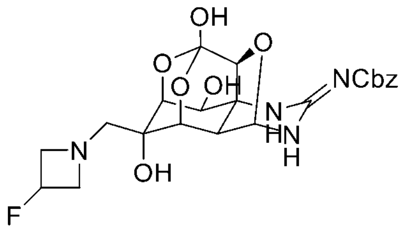
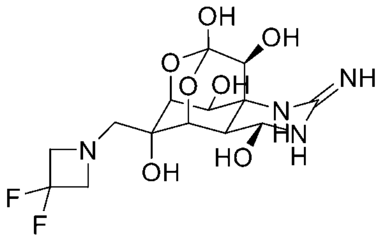

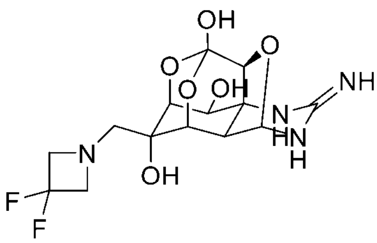
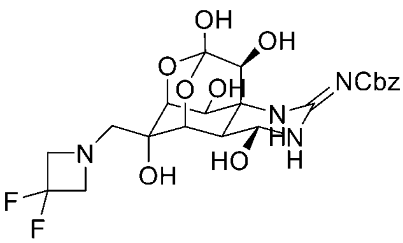
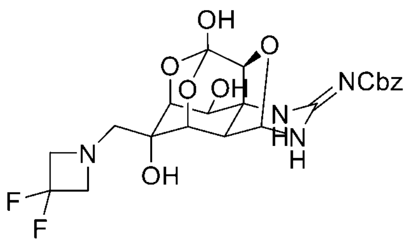

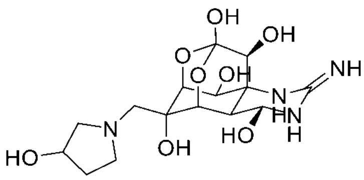
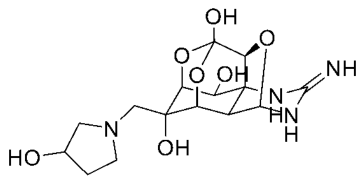
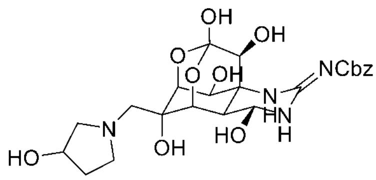

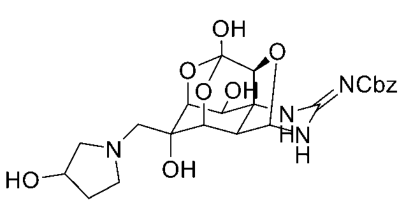
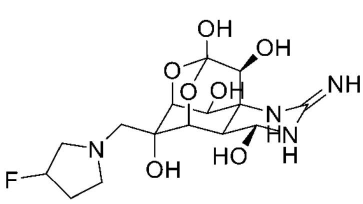
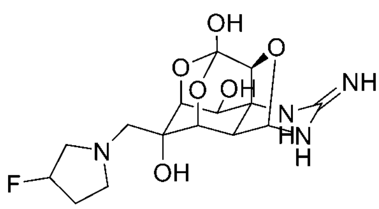

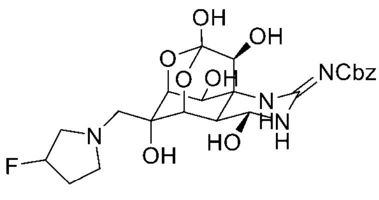
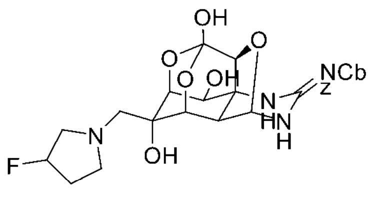
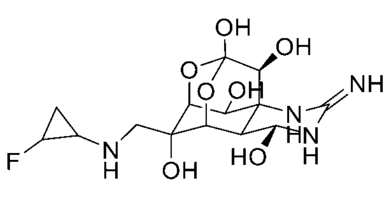

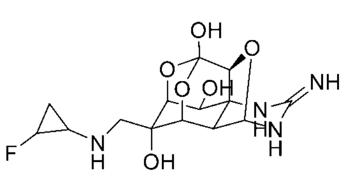
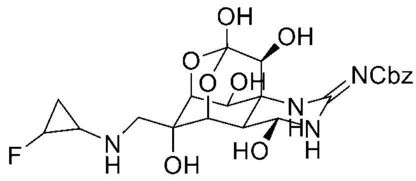
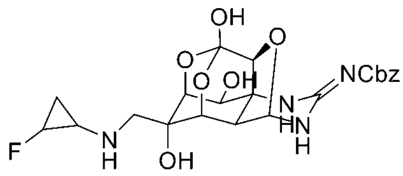

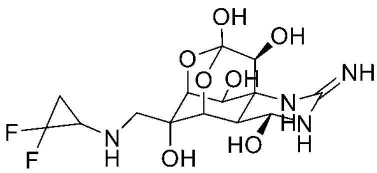
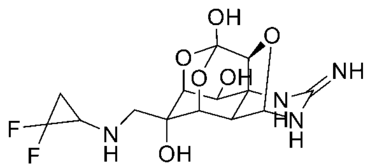
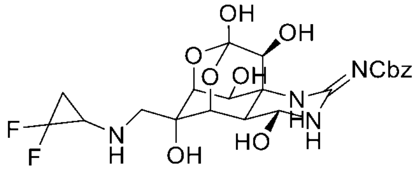

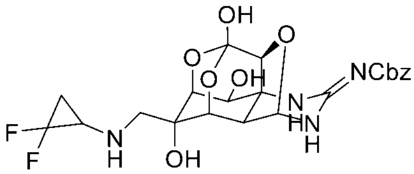
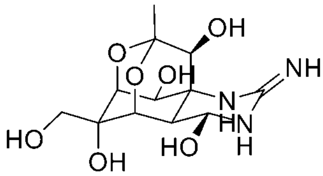
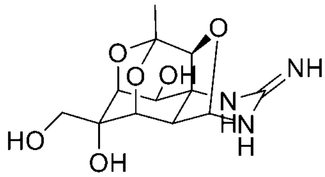

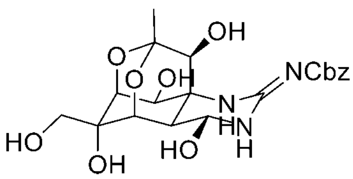
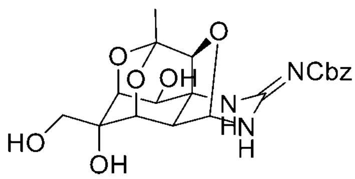
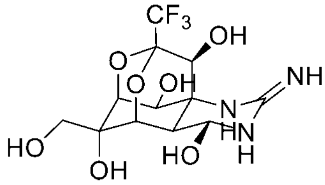

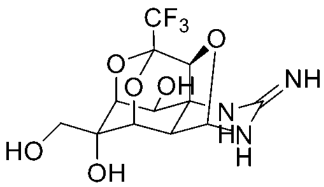
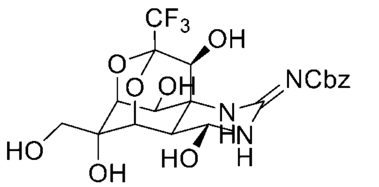
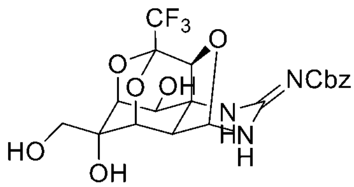

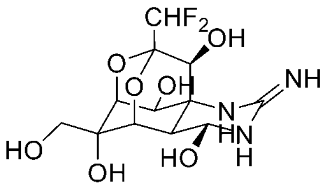
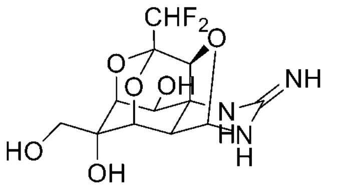
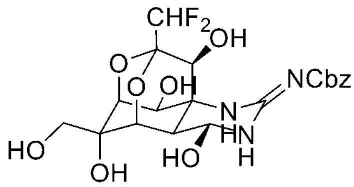

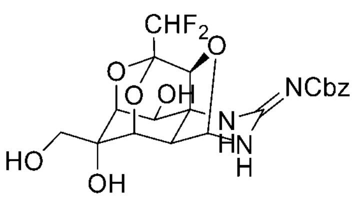
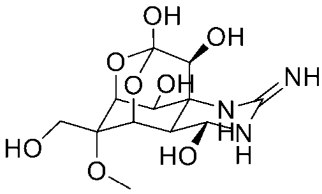
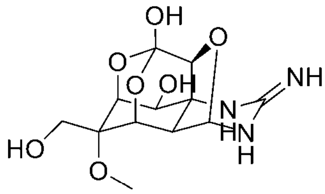

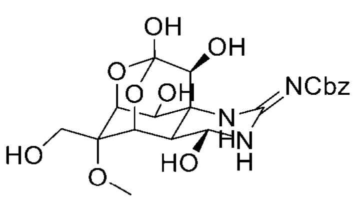
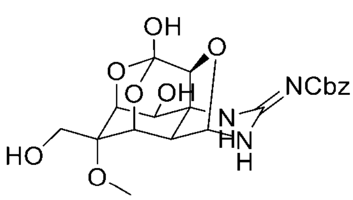
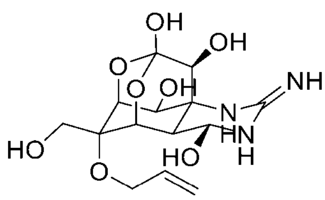

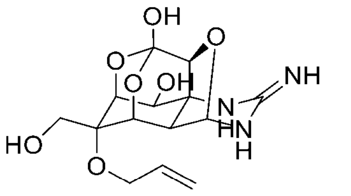
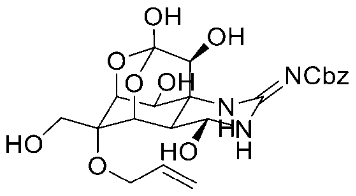
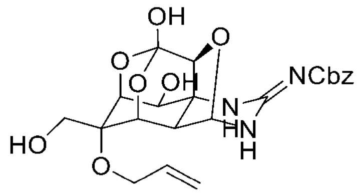

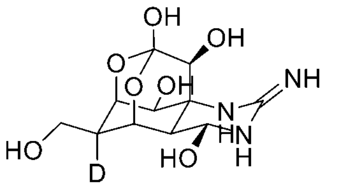
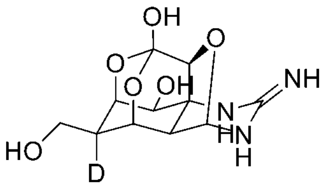
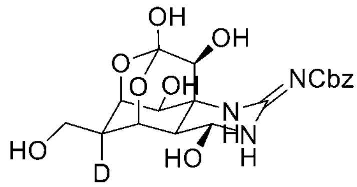

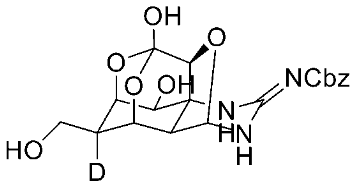
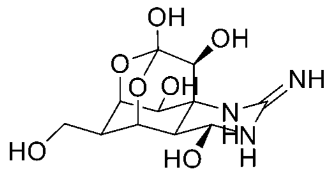
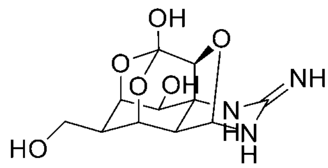

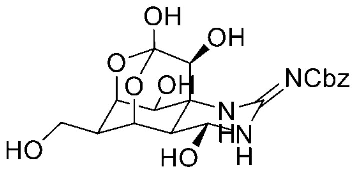
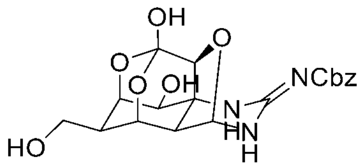
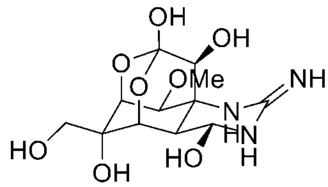

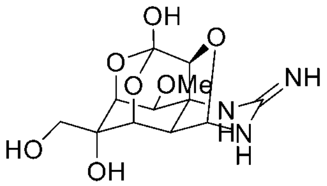
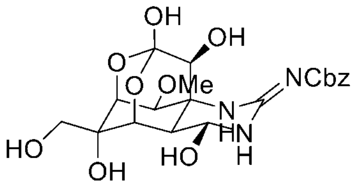
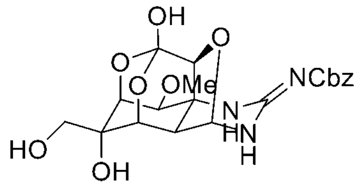

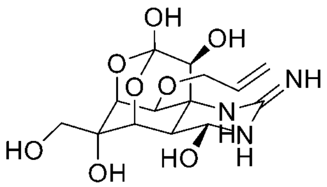
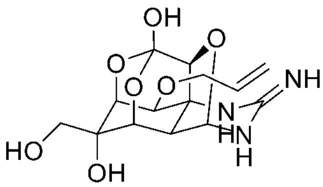
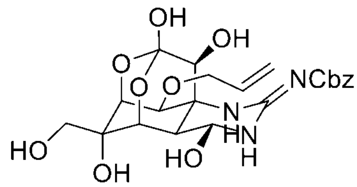

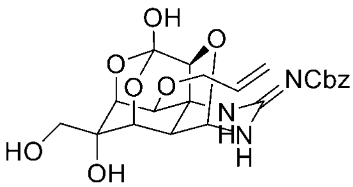
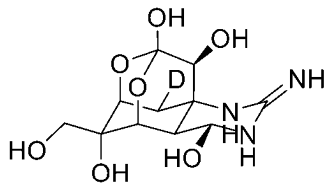
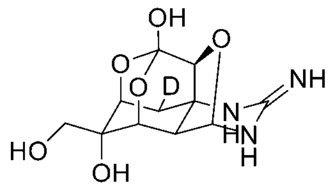

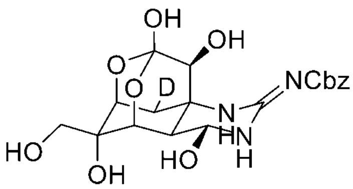
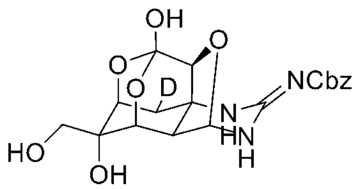
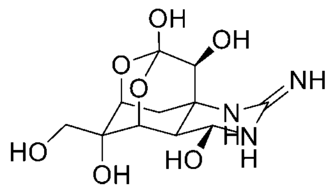

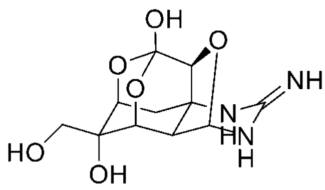
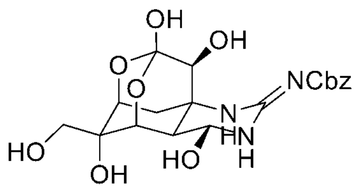
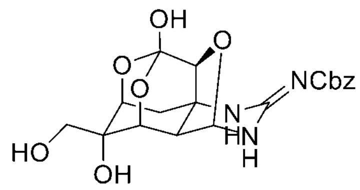

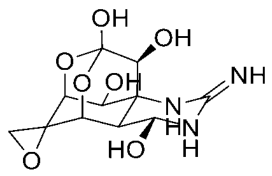
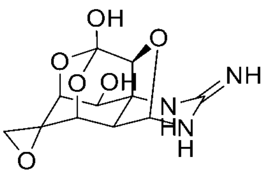
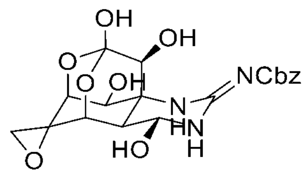
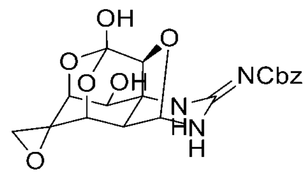

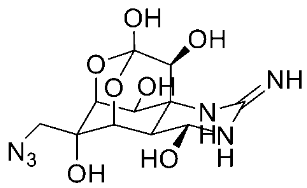
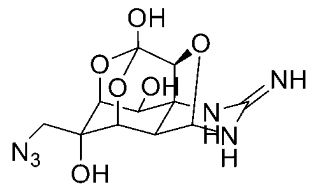
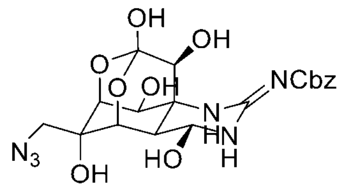

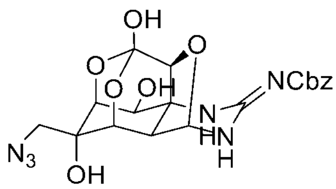
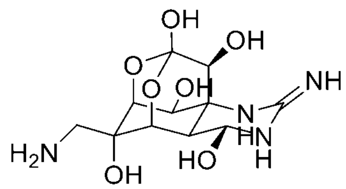
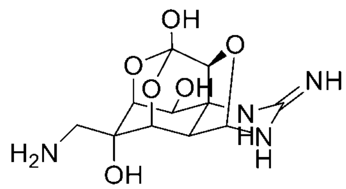

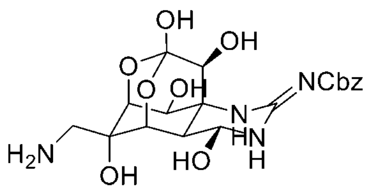
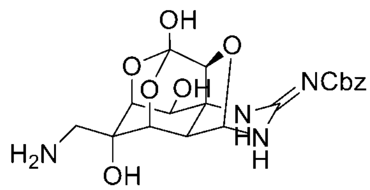
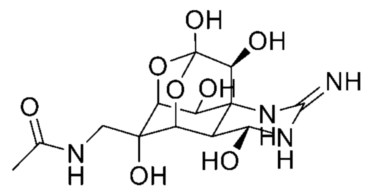

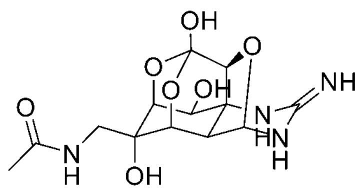
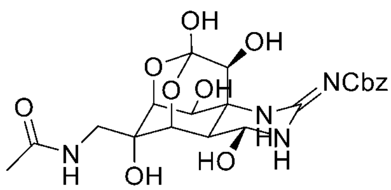
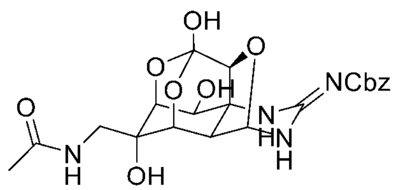

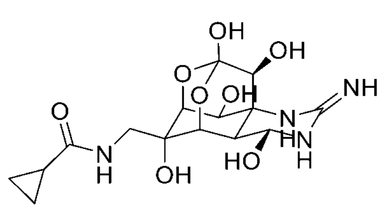
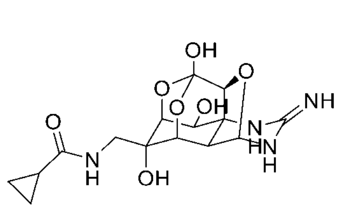
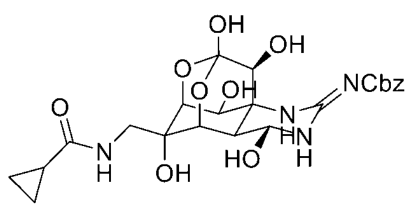

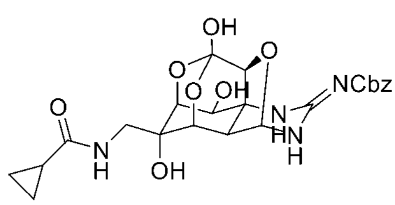
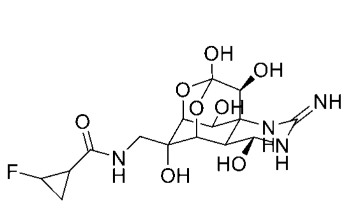
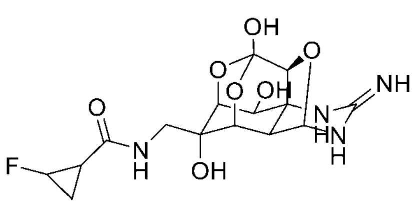

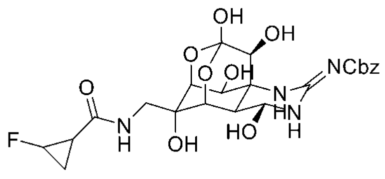 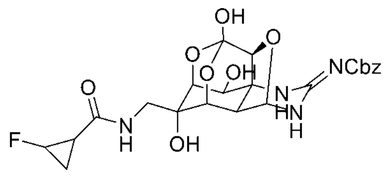 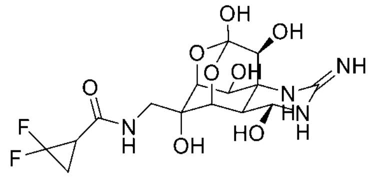

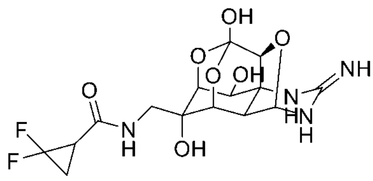 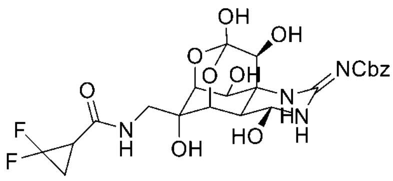 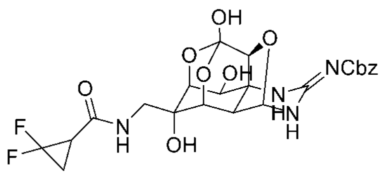

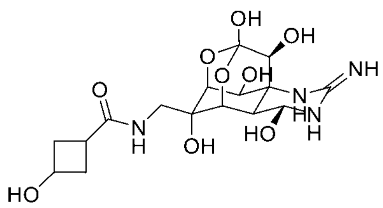 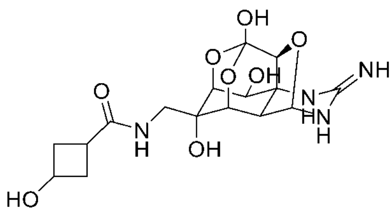 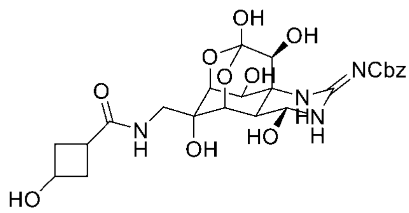

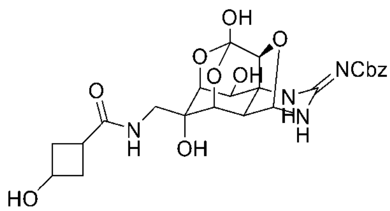 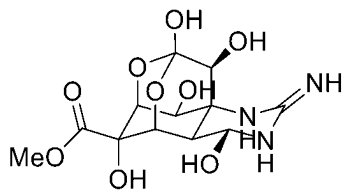 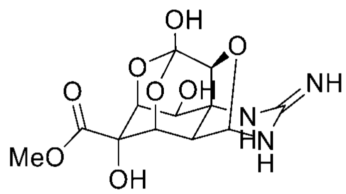

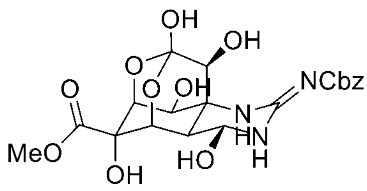 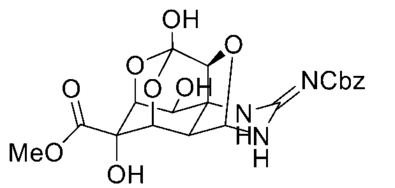 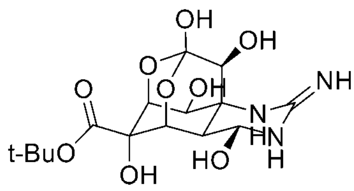

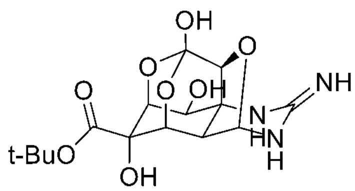 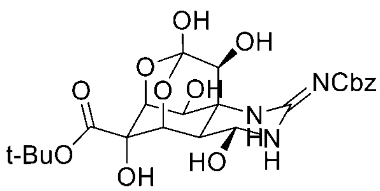 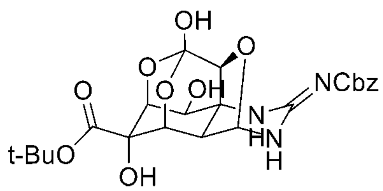

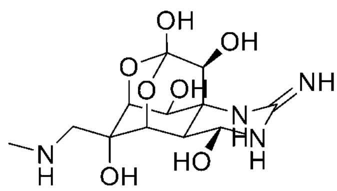 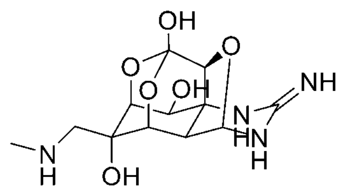 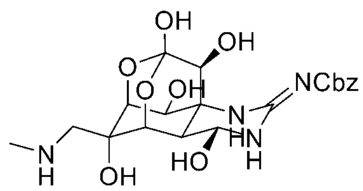

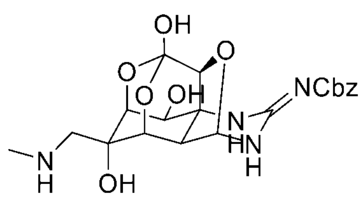 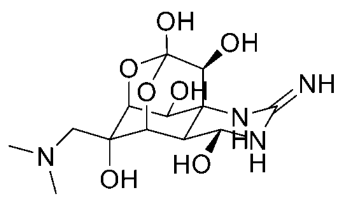 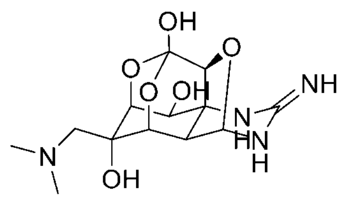

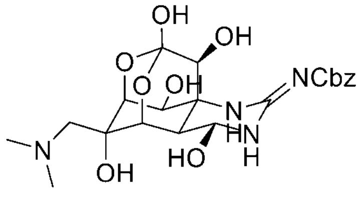
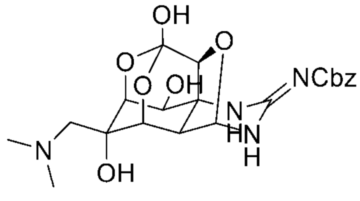
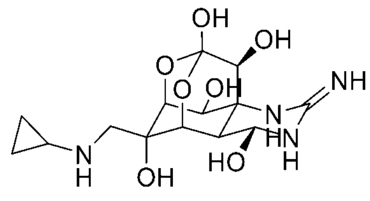

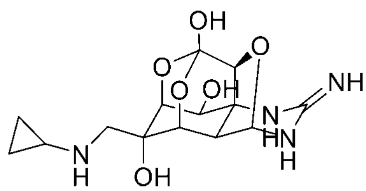
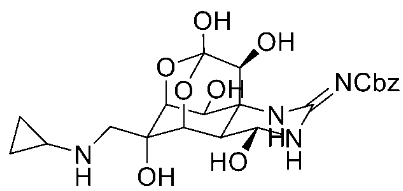
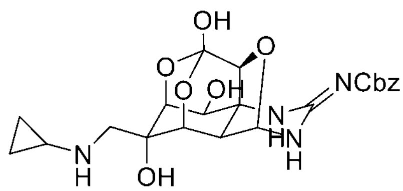

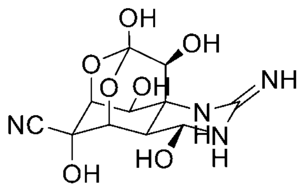
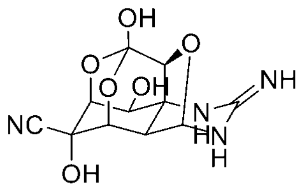
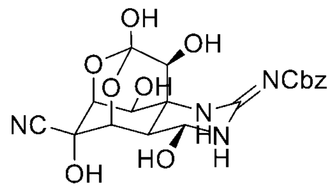

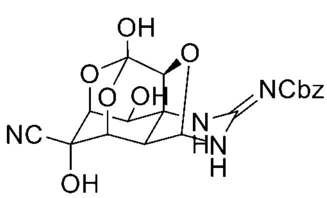
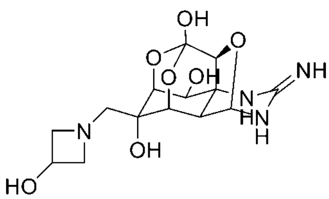
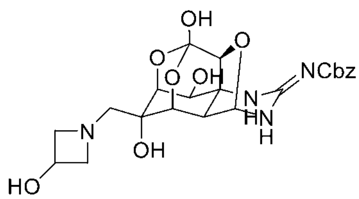

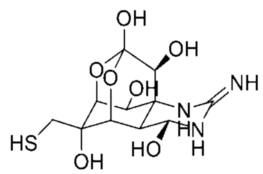
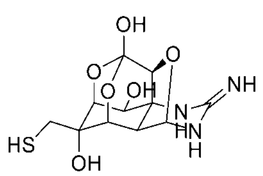
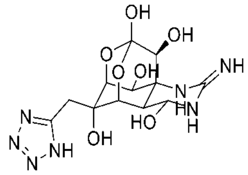
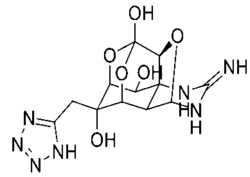

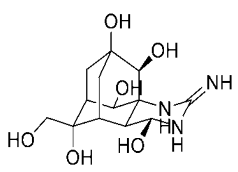
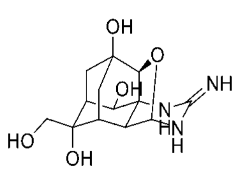
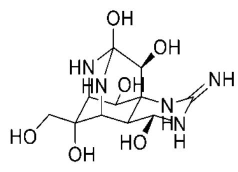
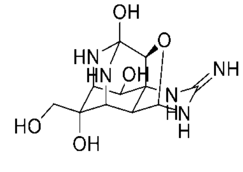

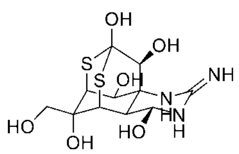
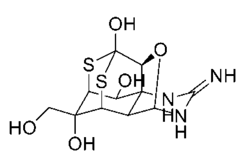
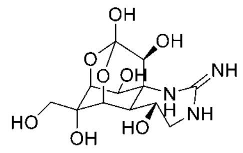
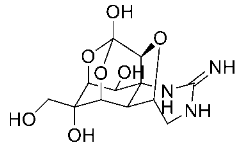

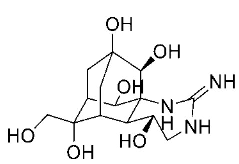
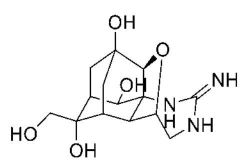
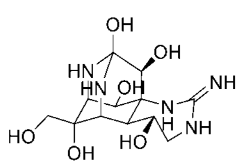
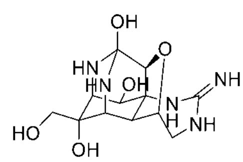

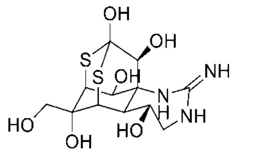
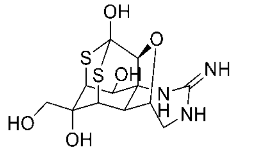
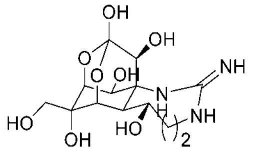
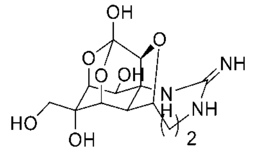
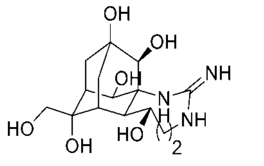
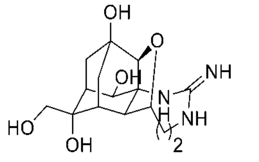
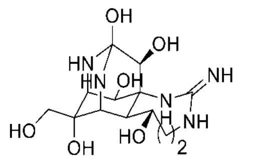
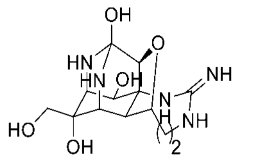
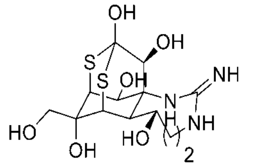
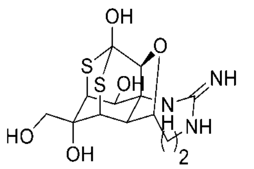
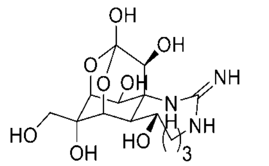
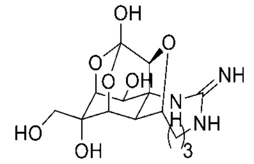
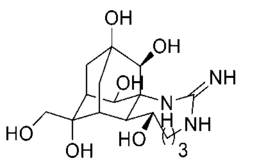
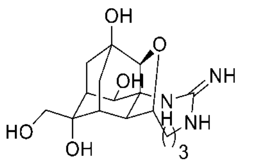
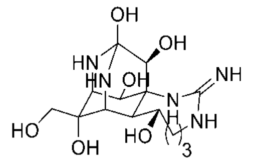
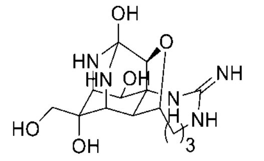
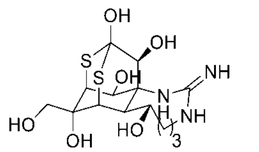
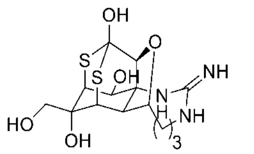
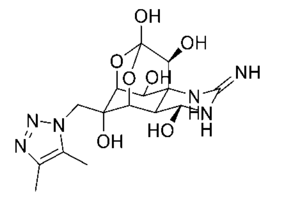
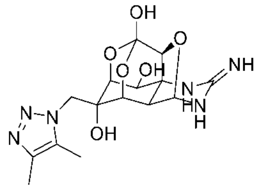
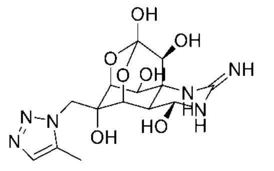
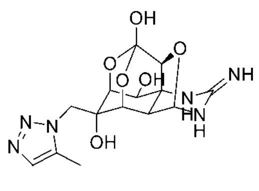
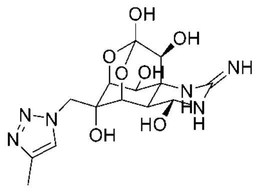
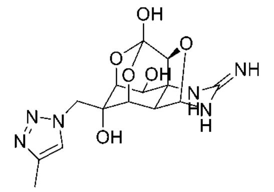
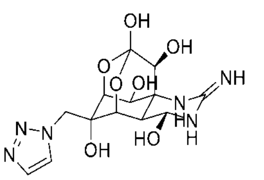
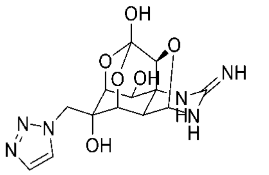
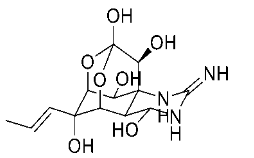
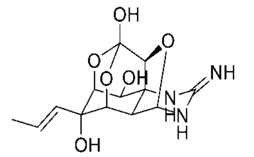
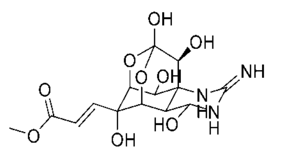
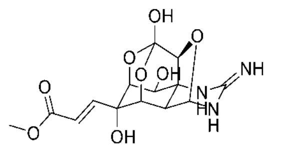
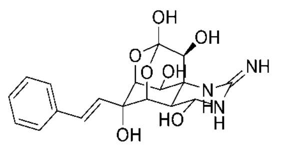
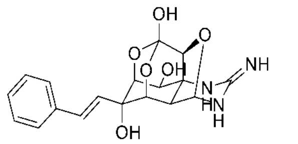

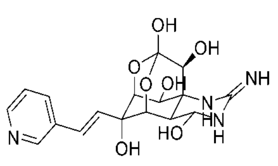 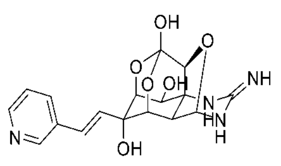 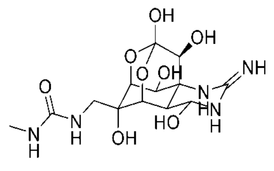 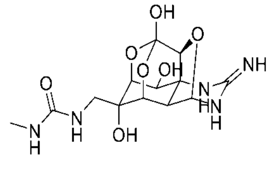

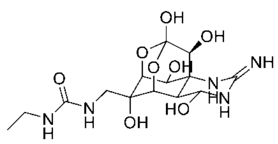 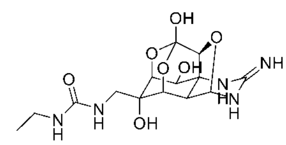 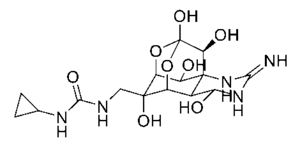 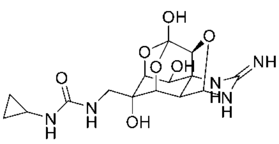

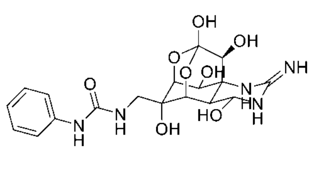 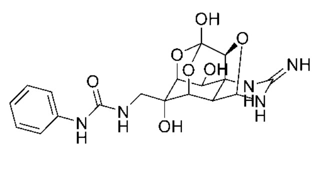 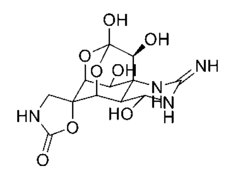 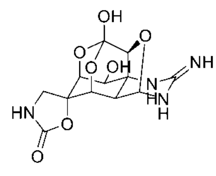

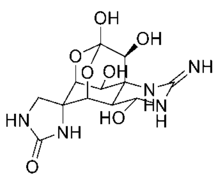 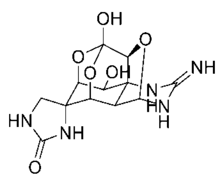 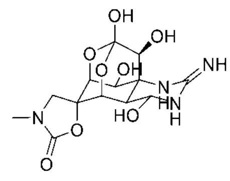 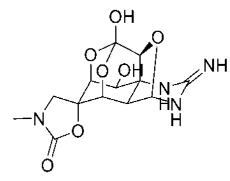

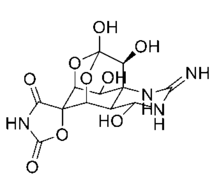 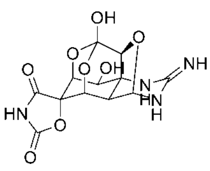 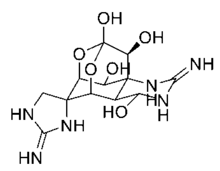 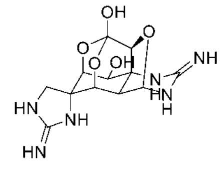

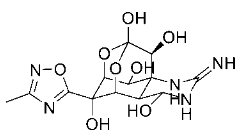 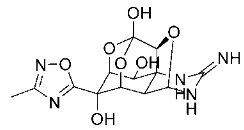 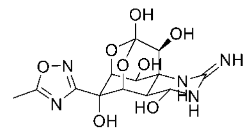 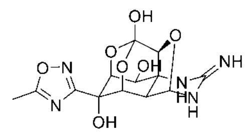

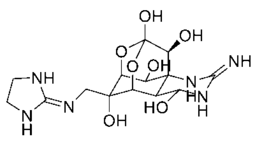 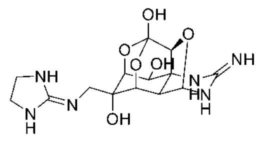 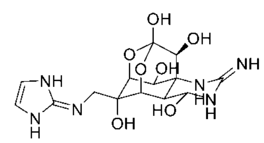 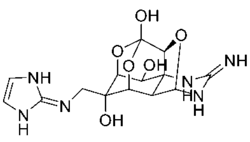

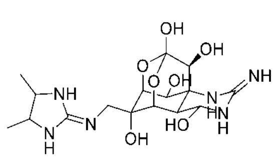 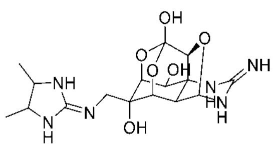 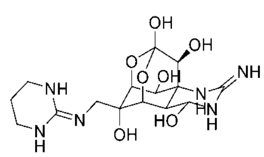 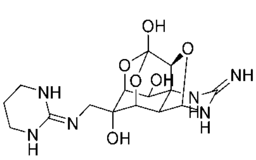

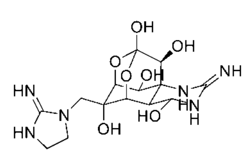 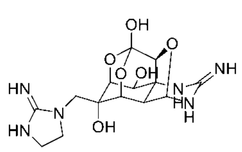 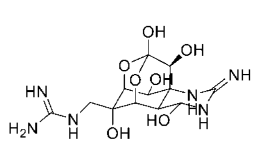 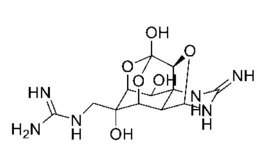

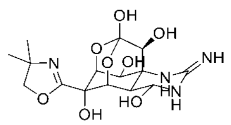
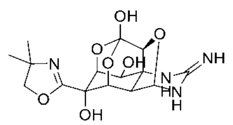
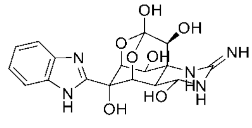
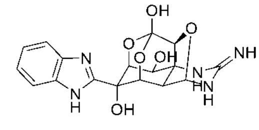
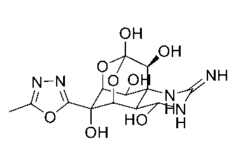
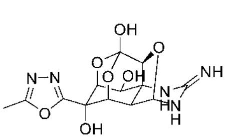
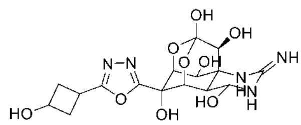
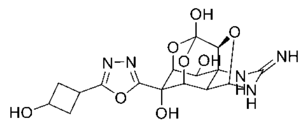
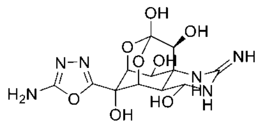
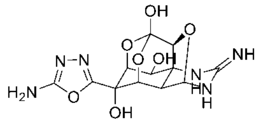
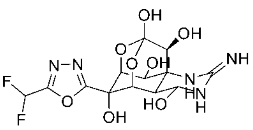
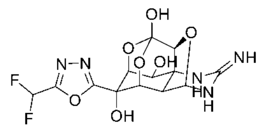
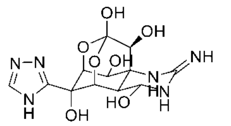
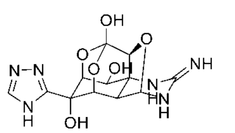
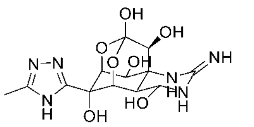
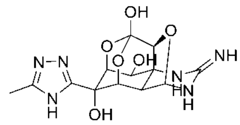
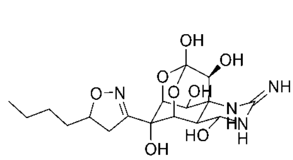
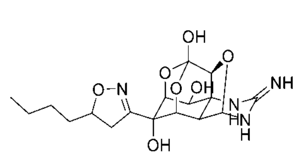
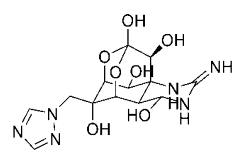
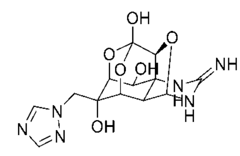
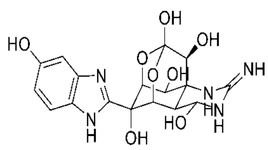
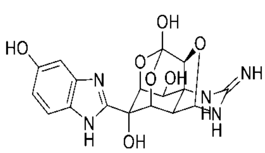
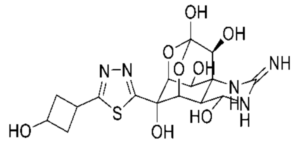
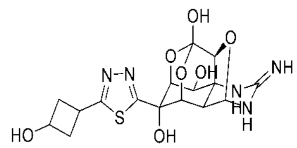
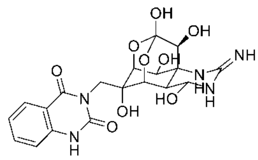
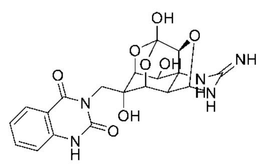
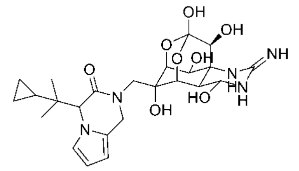
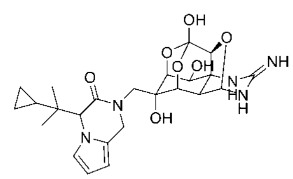
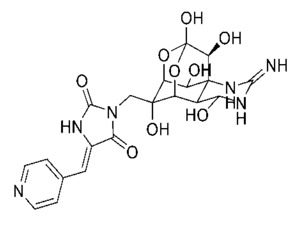
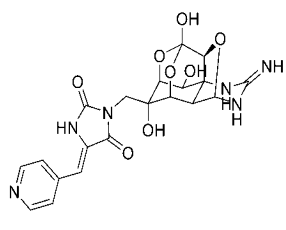
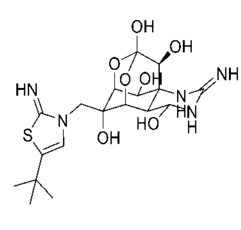
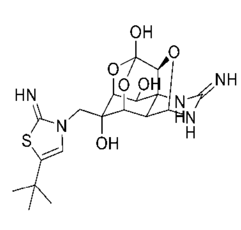
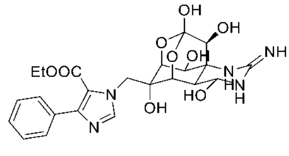
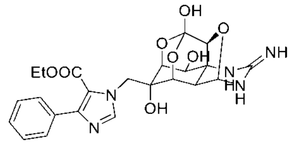
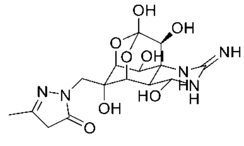
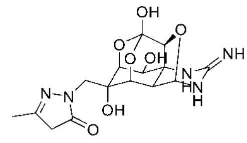

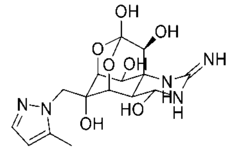 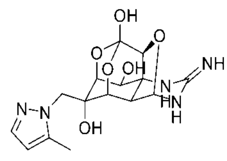 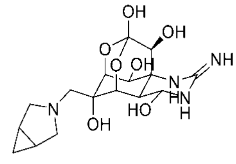 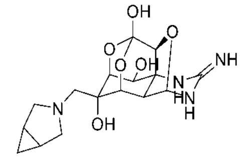

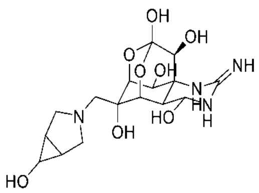 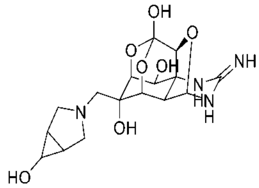 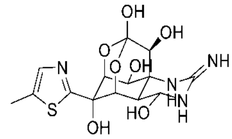 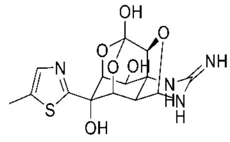

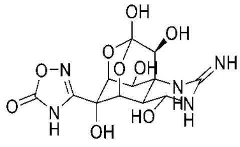 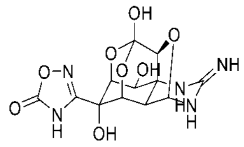 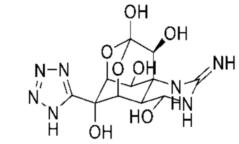 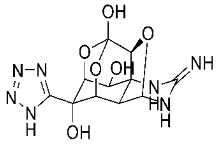

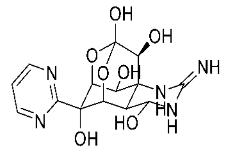 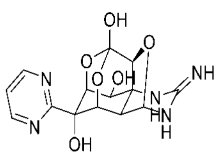 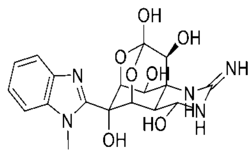 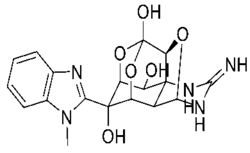

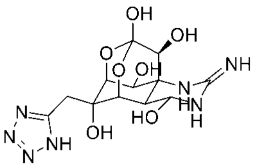 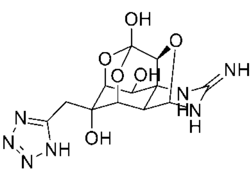 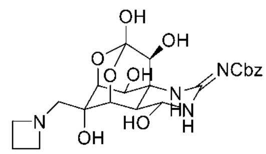 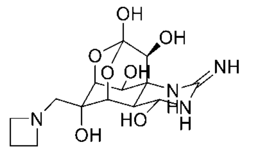

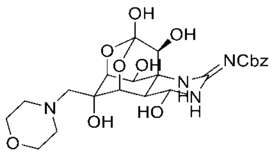 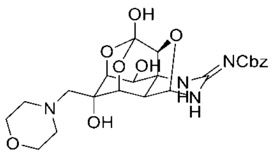 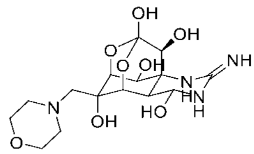 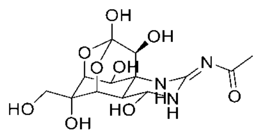

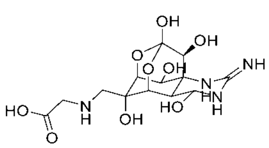 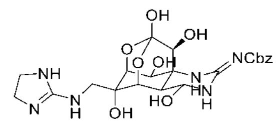 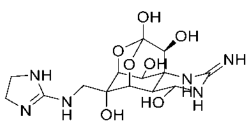 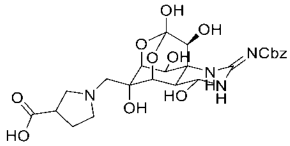

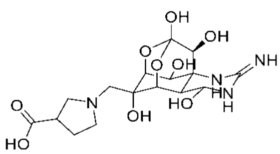 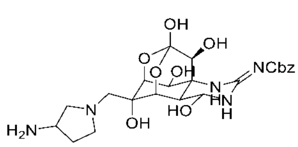 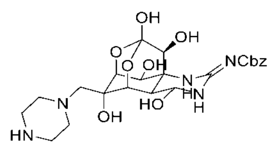 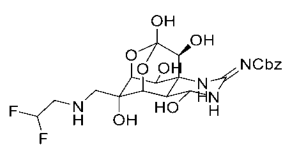

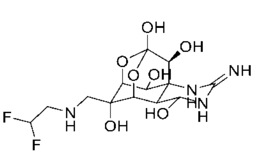 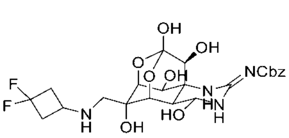 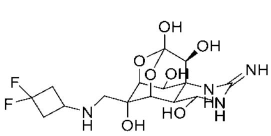 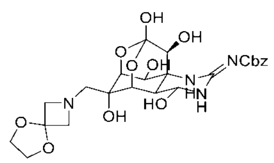

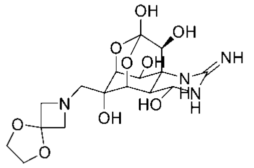 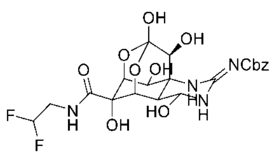 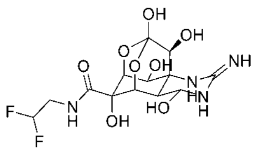 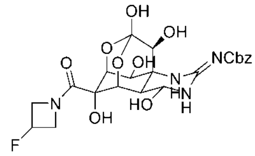

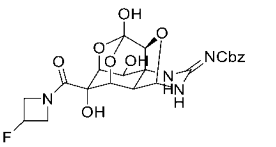 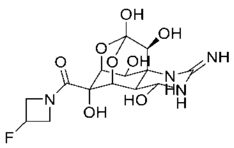 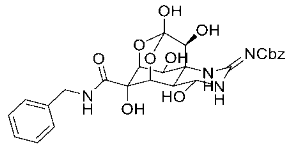 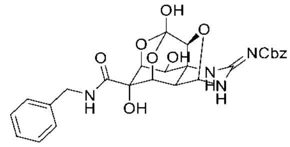

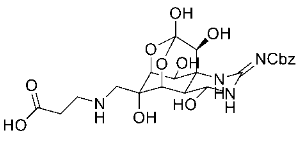 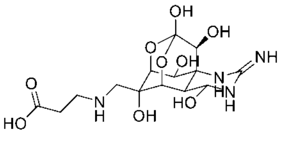 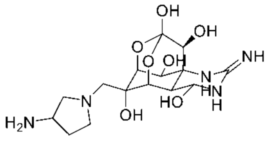 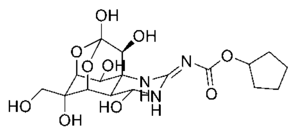

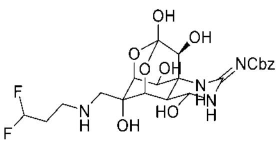 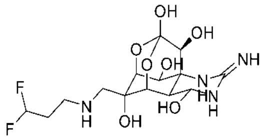 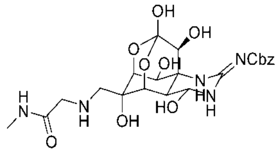 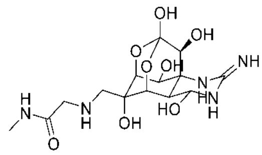

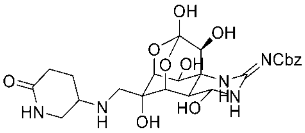 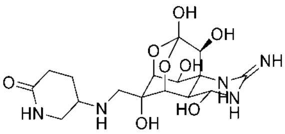 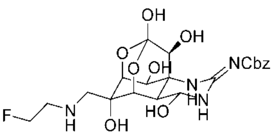 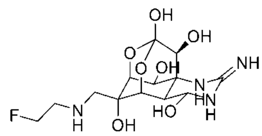

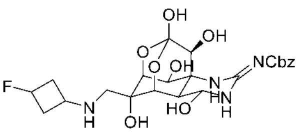 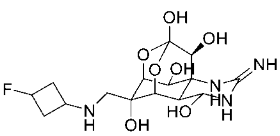 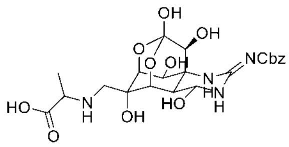 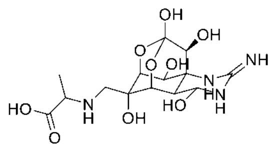

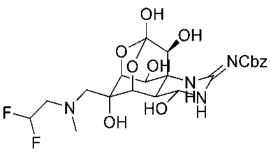 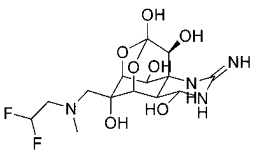 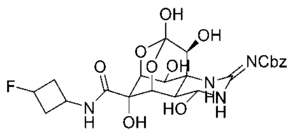 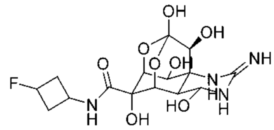

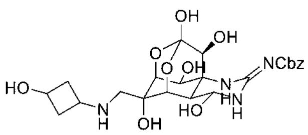 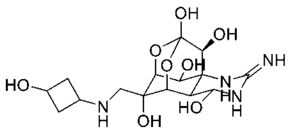 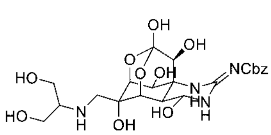 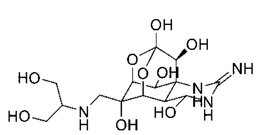

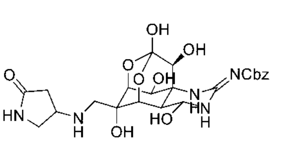 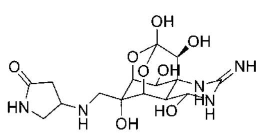 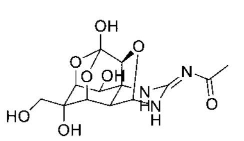 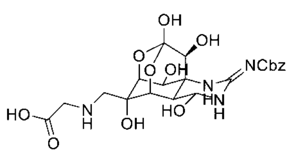

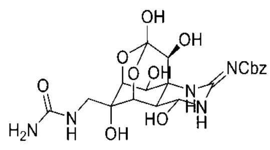 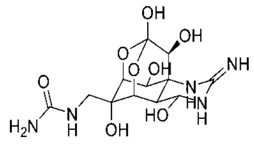 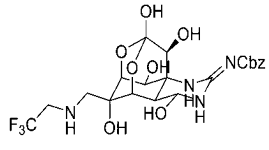 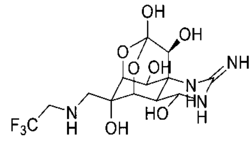

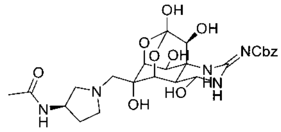 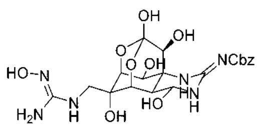 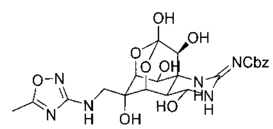

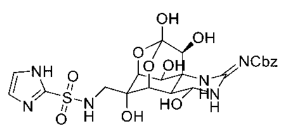 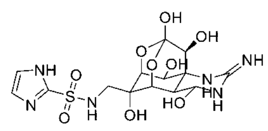 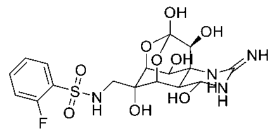 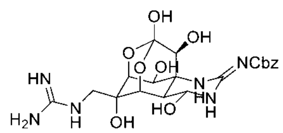

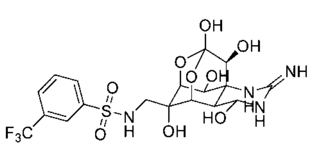 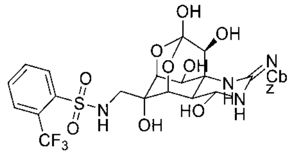 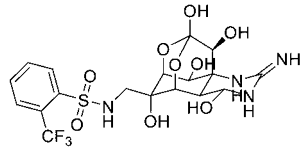

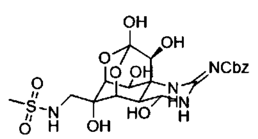 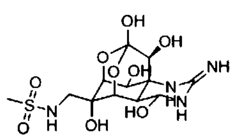 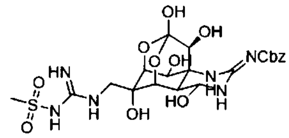 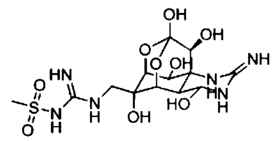

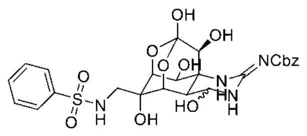 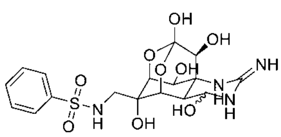 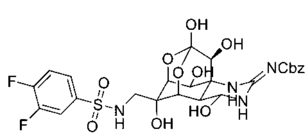 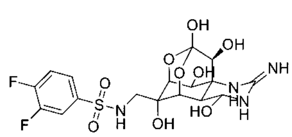

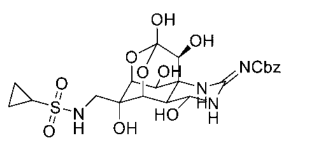 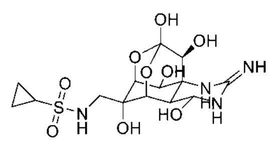 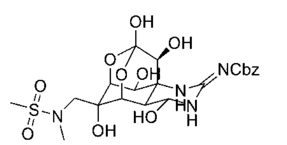 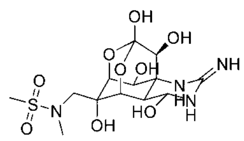

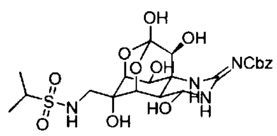 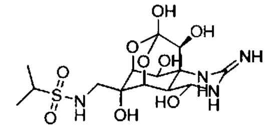 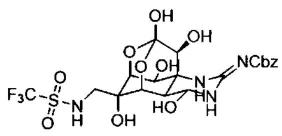 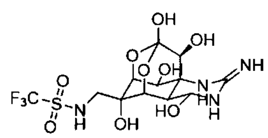

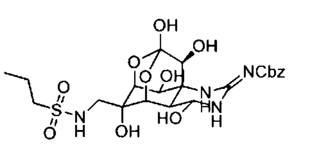 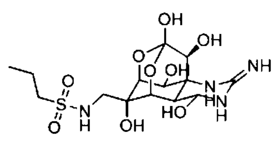 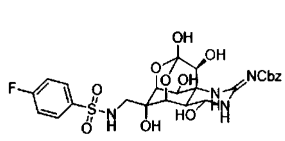 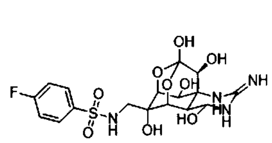

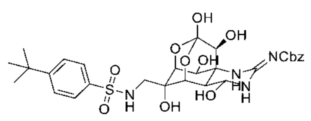 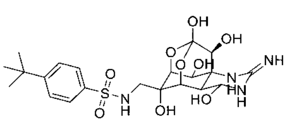 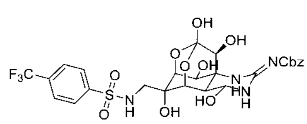 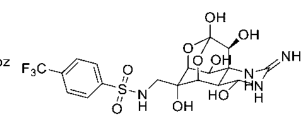

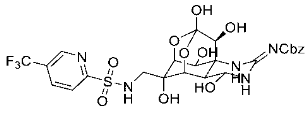 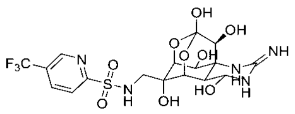 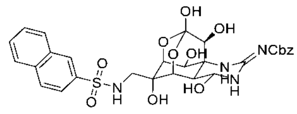 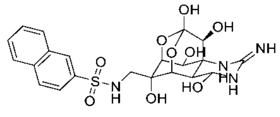

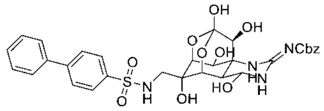 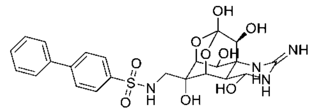 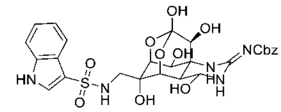 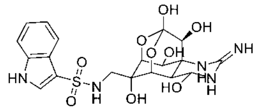

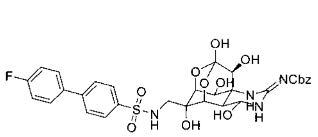 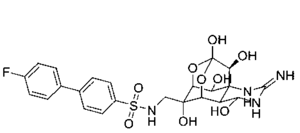 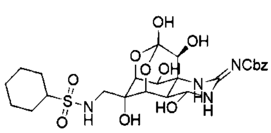 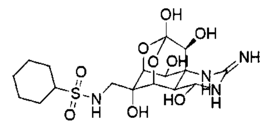

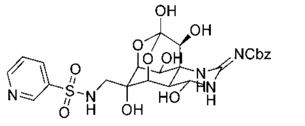 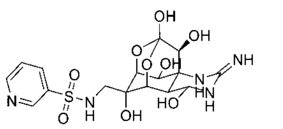 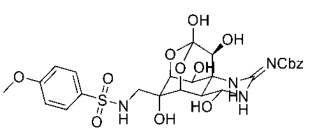 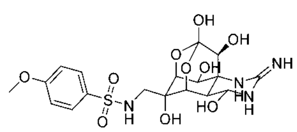

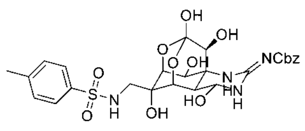 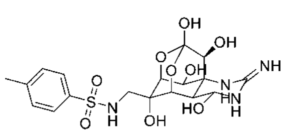 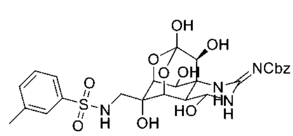 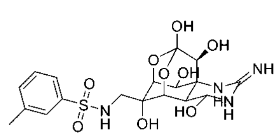

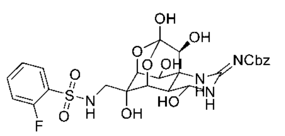 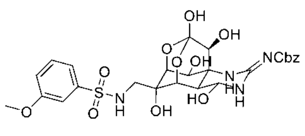 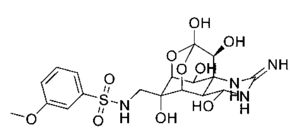 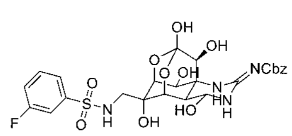

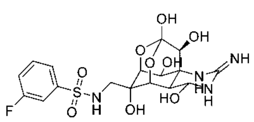 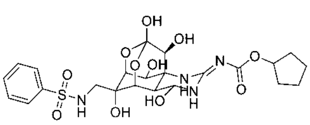 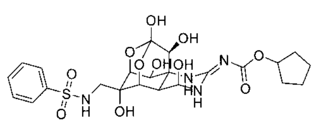 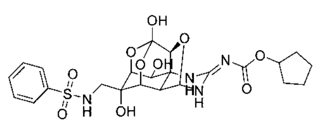

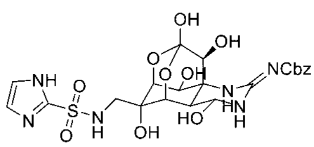 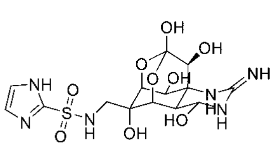 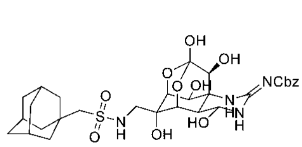 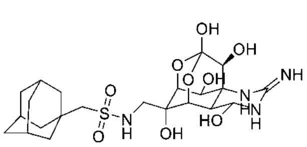

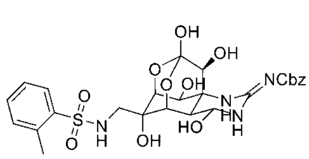 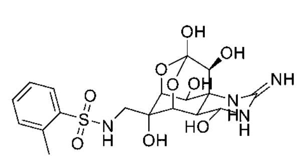 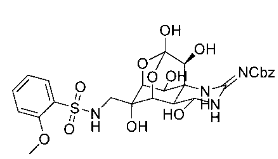 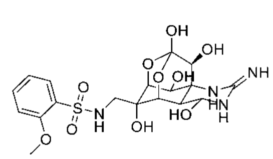

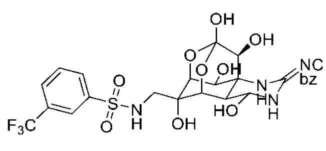 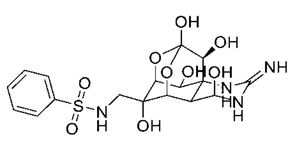 and 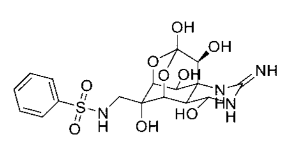 ;

preferably

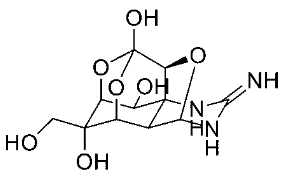 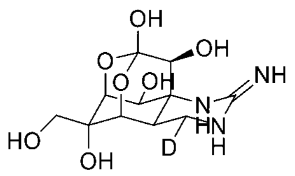 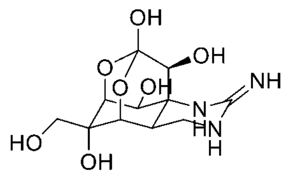 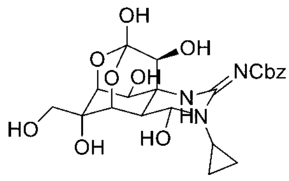

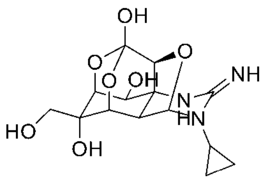 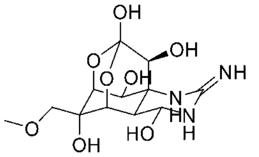 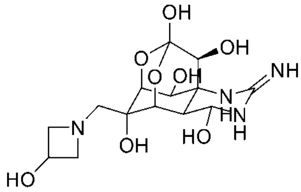 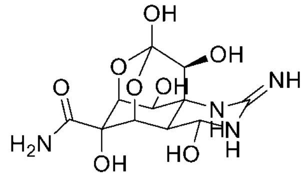

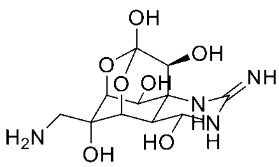 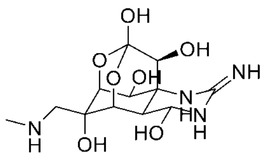 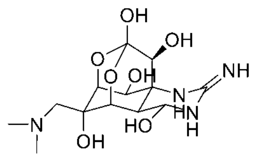 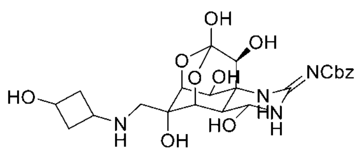

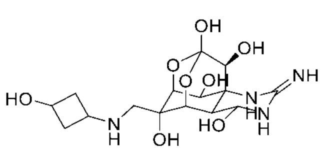 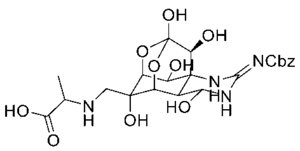 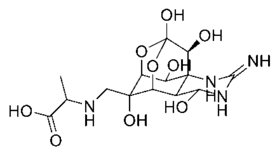 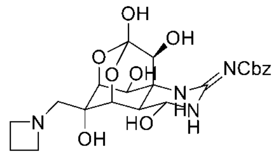

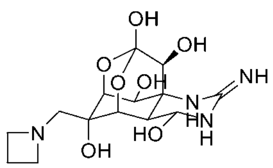 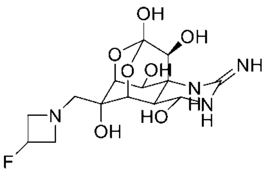 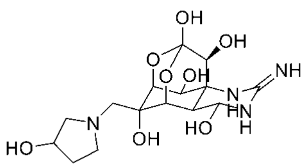 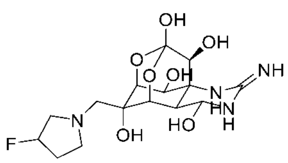

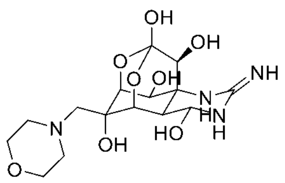 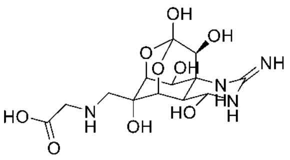 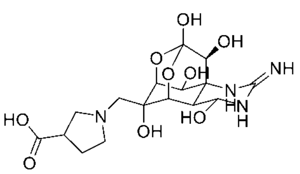 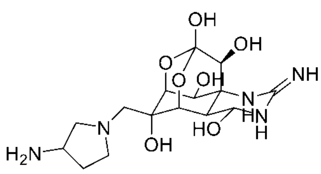

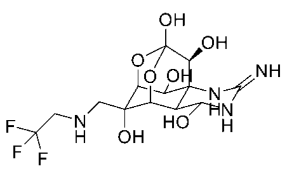 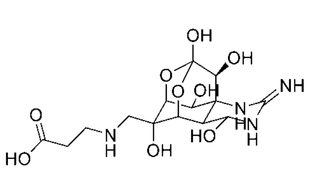 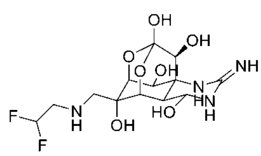 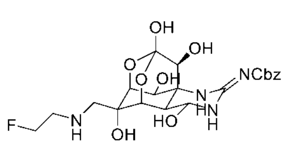

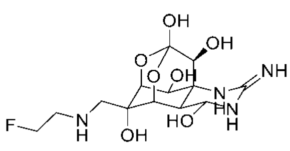 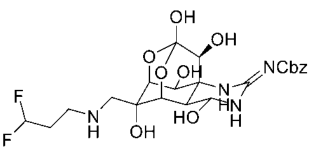 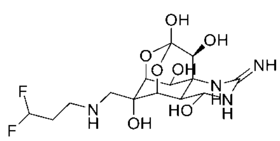 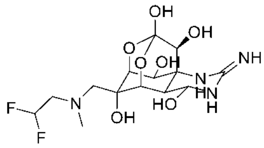

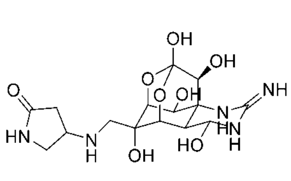 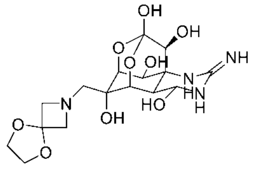 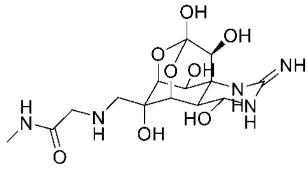 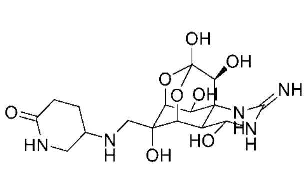

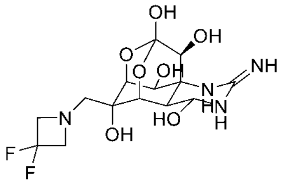 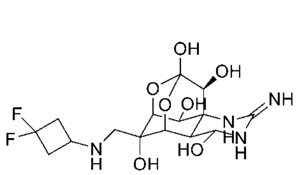 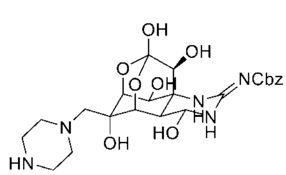 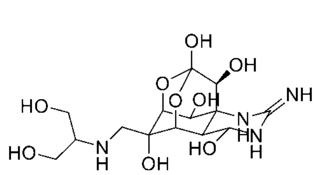

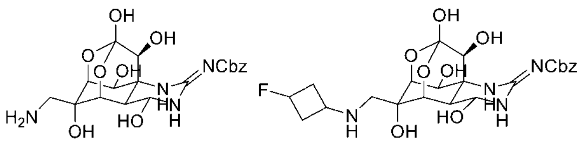 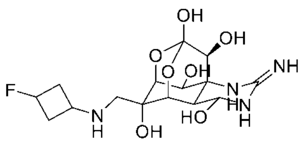 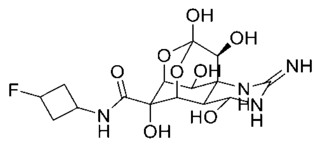

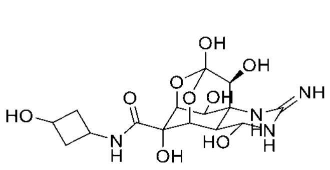 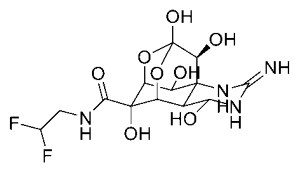 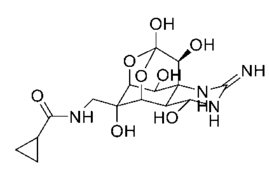 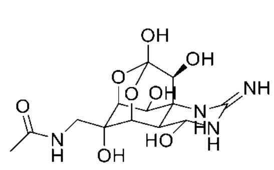

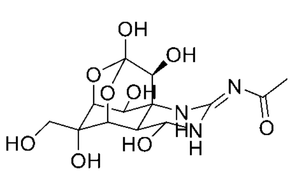 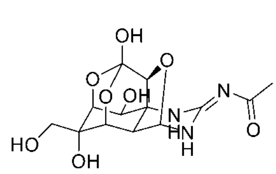 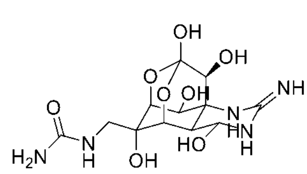 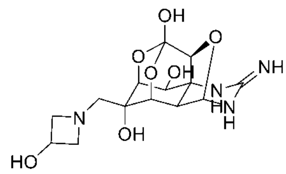

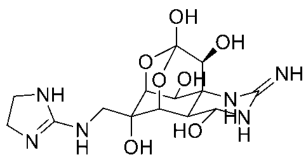 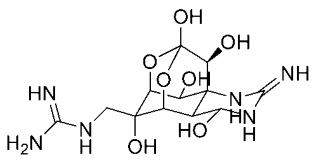 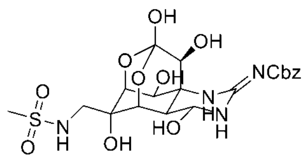 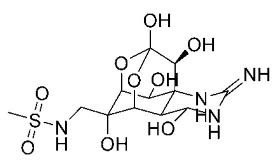

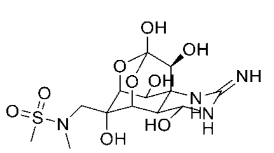 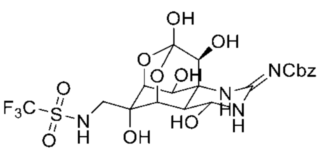 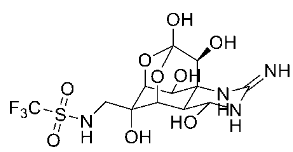 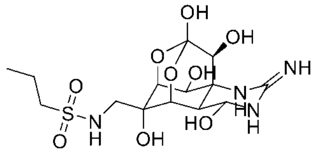

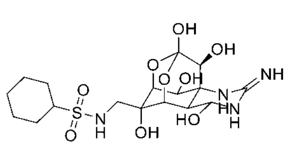 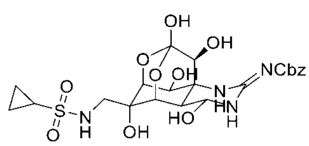 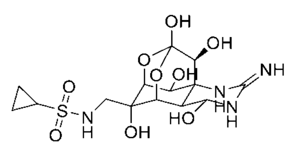 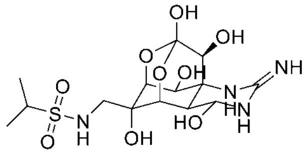

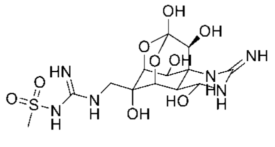 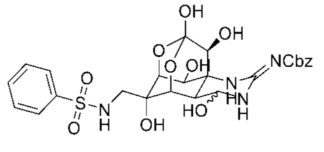 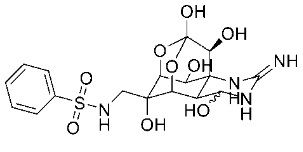 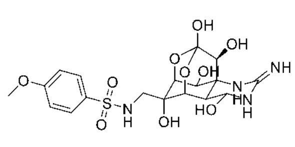

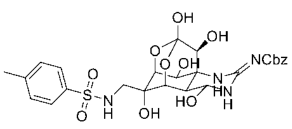
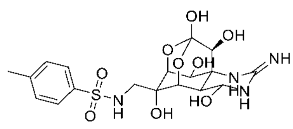
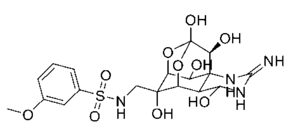
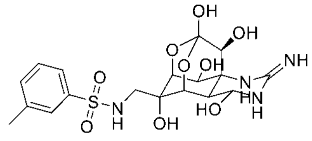

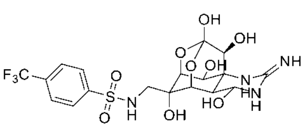
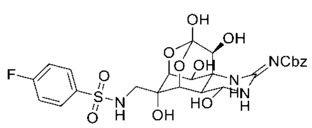
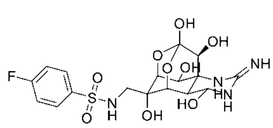
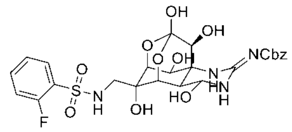

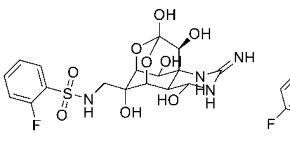
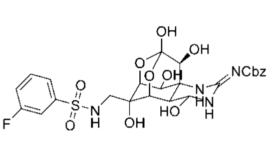
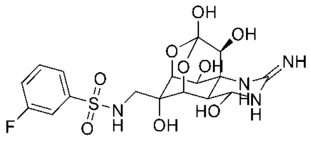
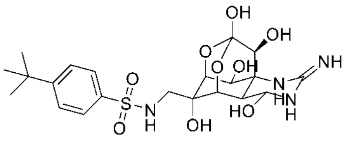

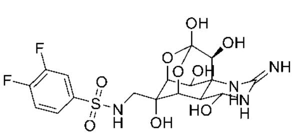
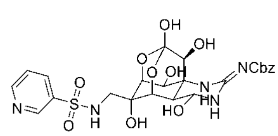
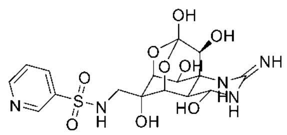
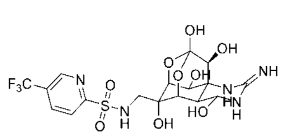

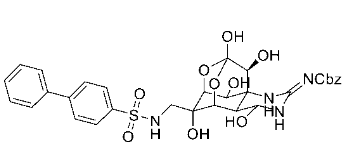
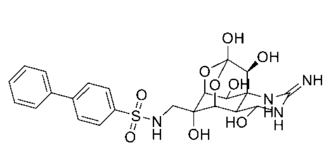
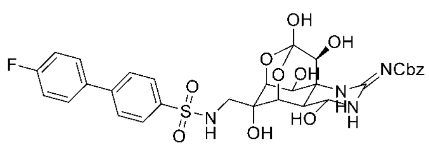

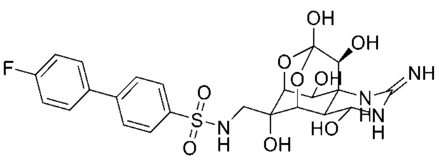
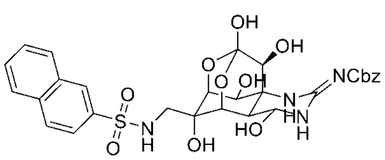
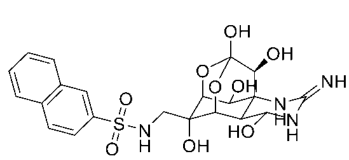

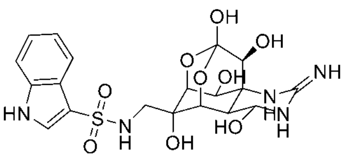
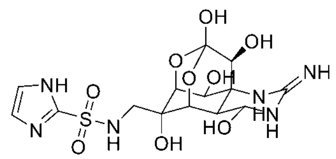
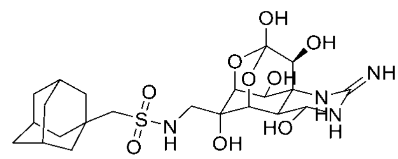

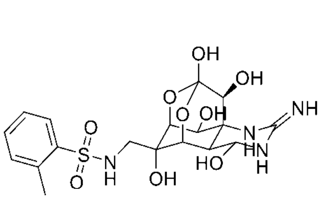
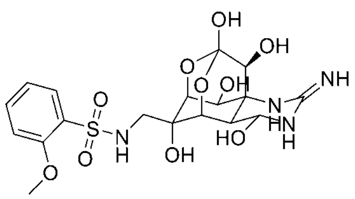
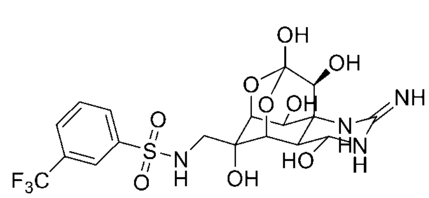

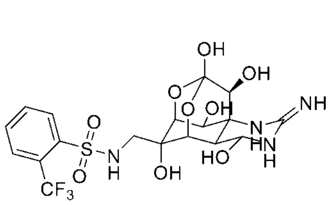
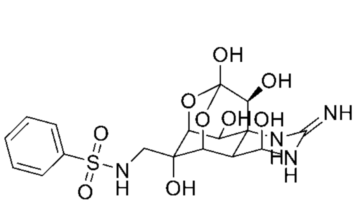
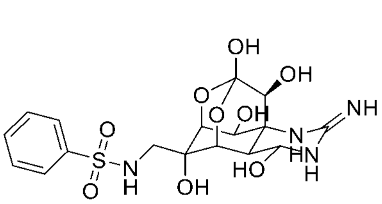

and

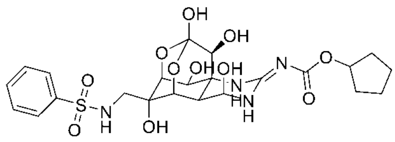;

more preferably

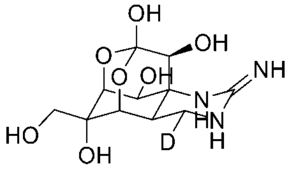
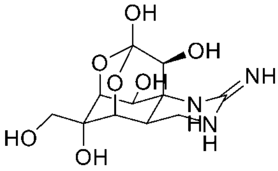
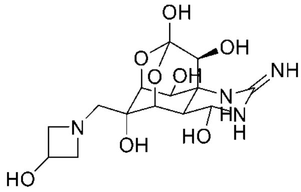
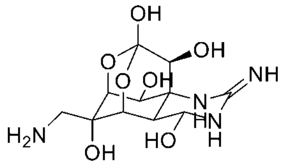
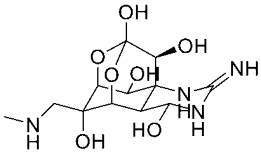
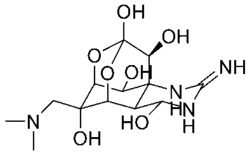
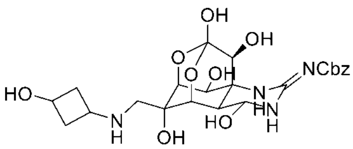
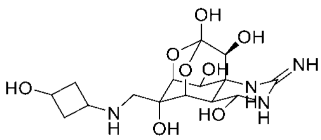
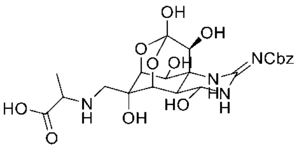
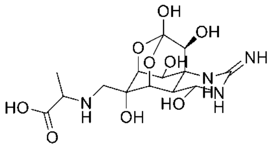
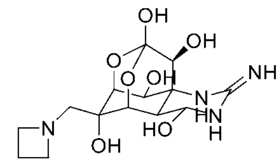
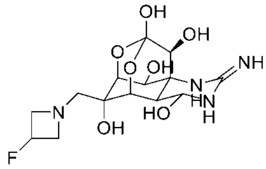
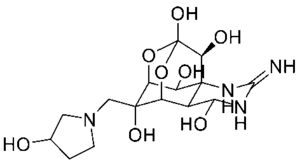
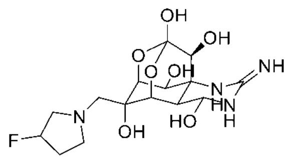
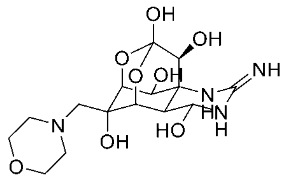
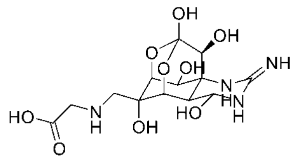
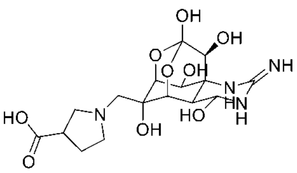
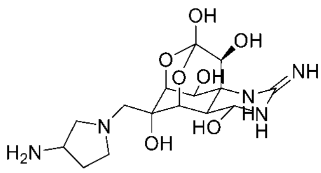
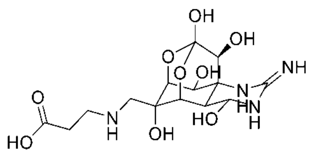
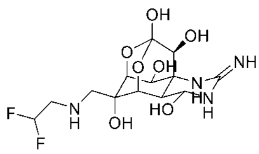
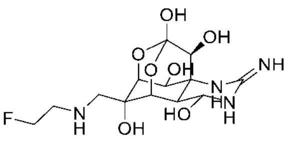
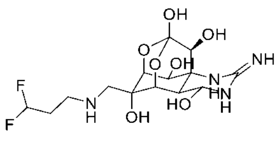
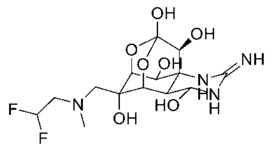
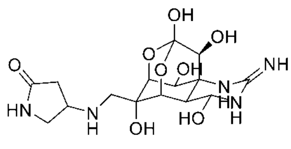
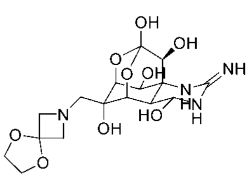
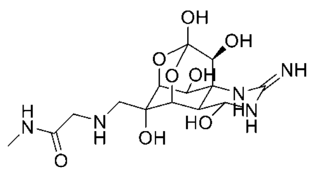
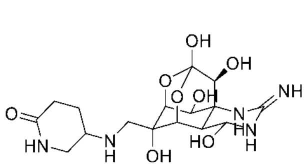
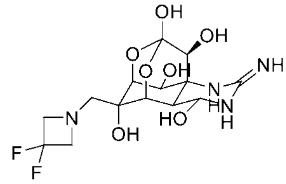

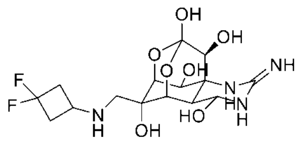 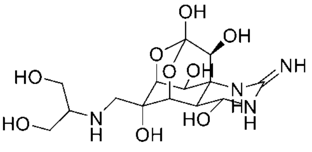 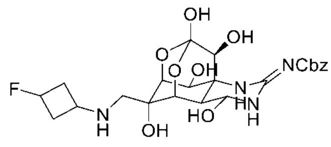

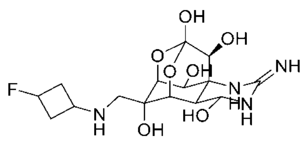 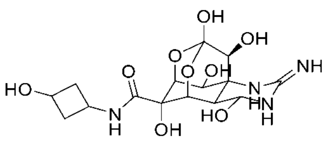 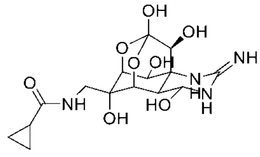 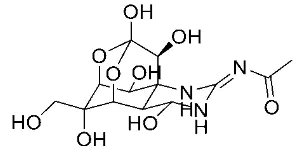

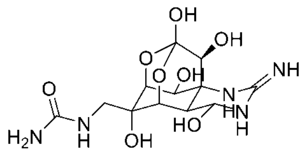 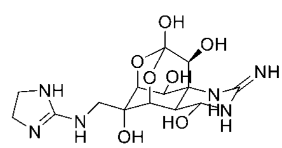 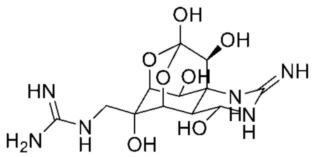 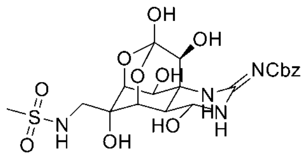

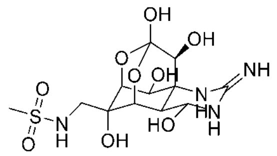 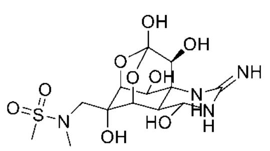 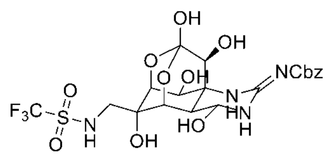 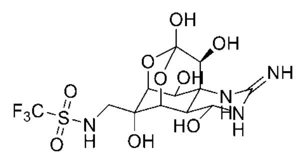

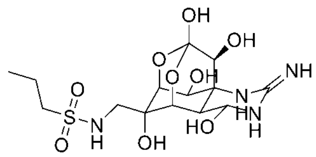 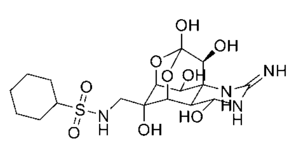 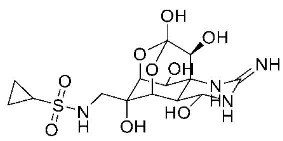 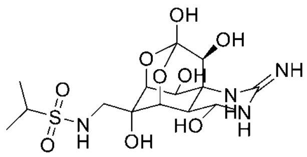

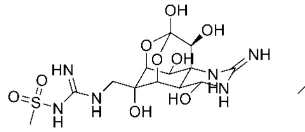 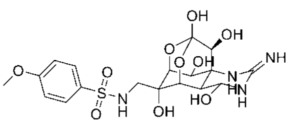 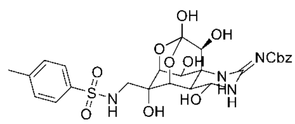 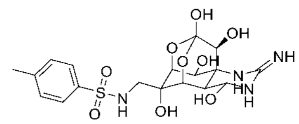

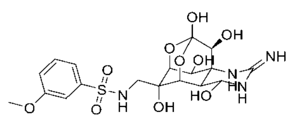 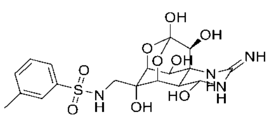 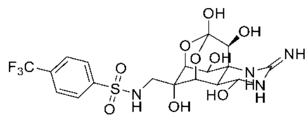 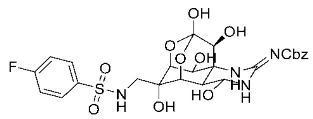

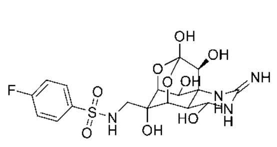 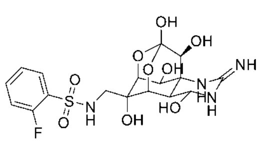 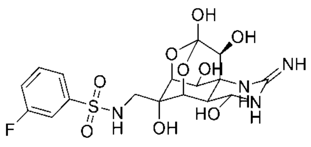 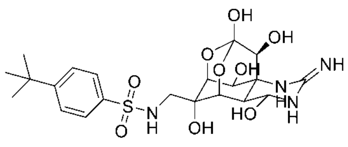

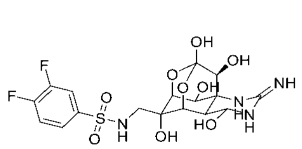 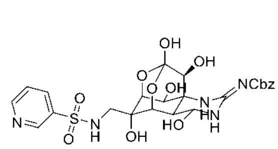 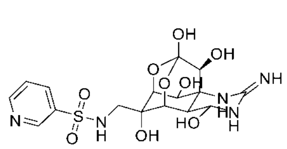 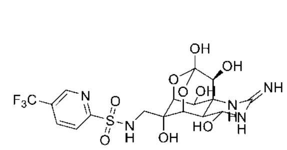

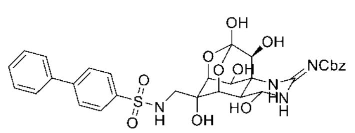 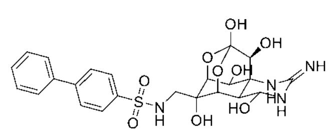 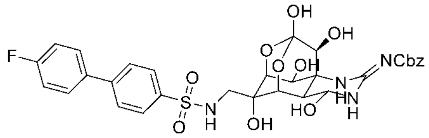

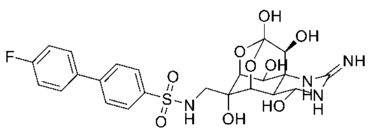 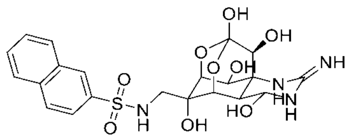 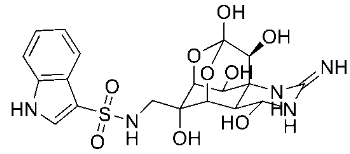

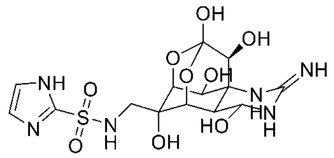 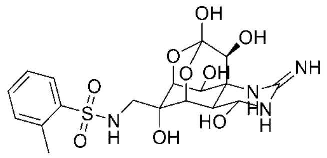 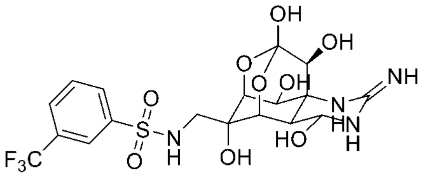

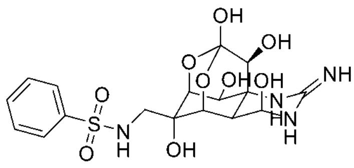 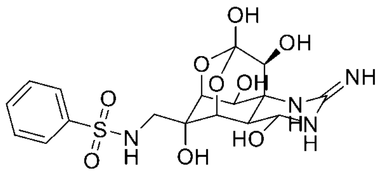 and 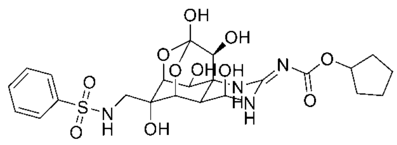 .

**28.** A pharmaceutical composition comprising a therapeutically effective amount of the compound according to any one of claims 1-27 or a pharmaceutically acceptable derivative thereof and one or more pharmaceutically acceptable carriers.

**29.** Use of the compound according to any one of claims 1-27 or a pharmaceutically acceptable derivative thereof or the pharmaceutical composition according to claim 28 in the manufacture of a medicament for treating a disease or condition associated with a sodium ion channel or for analgesia.

**30.** The use according to claim 29, wherein

the disease or condition associated with a sodium ion channel comprises pain, comprising neuropathic pain, inflammatory pain, visceral pain, cancer pain, chemotherapeutic pain, traumatic pain, surgical pain, post-surgical pain, childbirth pain, labor pain, chronic pain, persistent pain, peripherally mediated pain, centrally mediated pain, chronic headache, migraine, sinus headache, tension headache, phantom limb pain, dental pain, pain associated with HIV, acute pain, pain associated with multiple sclerosis (MS), familial rectal pain, fibromyalgia, or comprises pain caused by depression, cardiovascular disease, neurogenic cystitis, ulcerative colitis, respiratory disease, psychiatric disease, peripheral nerve damage, HIV treatment-induced neuropathy, thermosensitivity, sarcoidosis, irritable bowel syndrome, Crohn's disease, amyotrophic lateral sclerosis (ALS), diabetic neuropathy, peripheral neuropathy, arthritis, rheumatoid arthritis, osteoarthritis, atherosclerosis, paroxysmal dystonia, myasthenic syndrome, myotonic, malignant hyperthermia, cystic fibrosis, pseudohyperaldosteronism, rhabdomyolysis, hypothyroidism, bipolar depression, anxiety, schizophrenia, sodium channel toxin related disease, familial erythromelalgia, primary erythromelalgia, epilepsy, epileptic encephalopathy, focal and generalized tonic seizure, restless leg syndrome, arrhythmia, tachyarrhythmia, atrial fibrillation, or ventricular fibrillation;
the pain targeted by the analgesia comprises neuropathic pain, inflammatory pain, visceral pain, cancer pain, chemotherapeutic pain, traumatic pain, surgical pain, post-surgical pain, childbirth pain, labor pain, chronic pain, persistent pain, peripherally mediated pain, centrally mediated pain, chronic headache, migraine, sinus headache, tension headache, phantom limb pain, dental pain, pain associated with HIV, acute pain, pain associated with multiple sclerosis (MS), familial rectal pain, fibromyalgia, or comprises pain caused by depression, cardiovascular disease, neurogenic cystitis, ulcerative colitis, respiratory disease, psychiatric disease, peripheral nerve damage, HIV treatment-induced neuropathy, thermosensitivity, sarcoidosis, irritable bowel syndrome, Crohn's disease, amyotrophic lateral sclerosis (ALS), diabetic neuropathy, peripheral neuropathy, arthritis, rheumatoid arthritis, osteoarthritis, atherosclerosis, paroxysmal dystonia, myasthenic syndrome, myotonic, malignant hyperthermia, cystic fibrosis, pseudohyperaldosteronism, rhabdomyolysis, hypothyroidism, bipolar depression, anxiety, schizophrenia, sodium channel toxin related disease, familial erythromelalgia, primary erythromelalgia, epilepsy, epileptic encephalopathy, focal and generalized tonic seizure, restless leg syndrome, arrhythmia, tachyarrhythmia, atrial fibrillation, or ventricular fibrillation.

**31.** A compound having a structure of Formula (II):

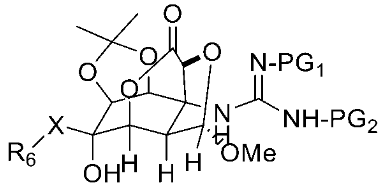

Formula (II)

wherein,

X, $R_6$ are as defined in any one of claims 1-27,
$Pg_1$, $Pg_2$ are each independently selected from the group consisting of acetyl, trityl, tert-butoxycarbonyl (Boc), benzyloxycarbonyl (Cbz), cyclopentyloxycarbonyl, and fluorenylmethoxycarbonyl (Fmoc).

**32.** The compound of claim 31, having the following structure:

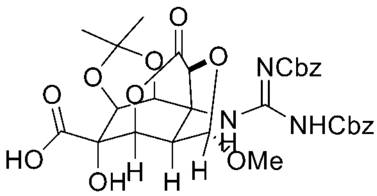
,
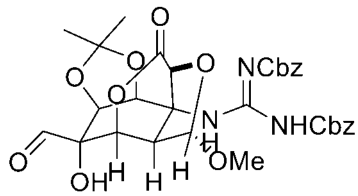
or
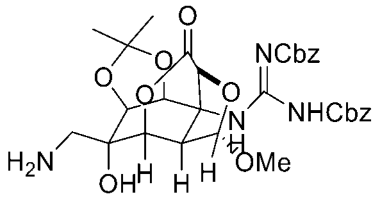
.

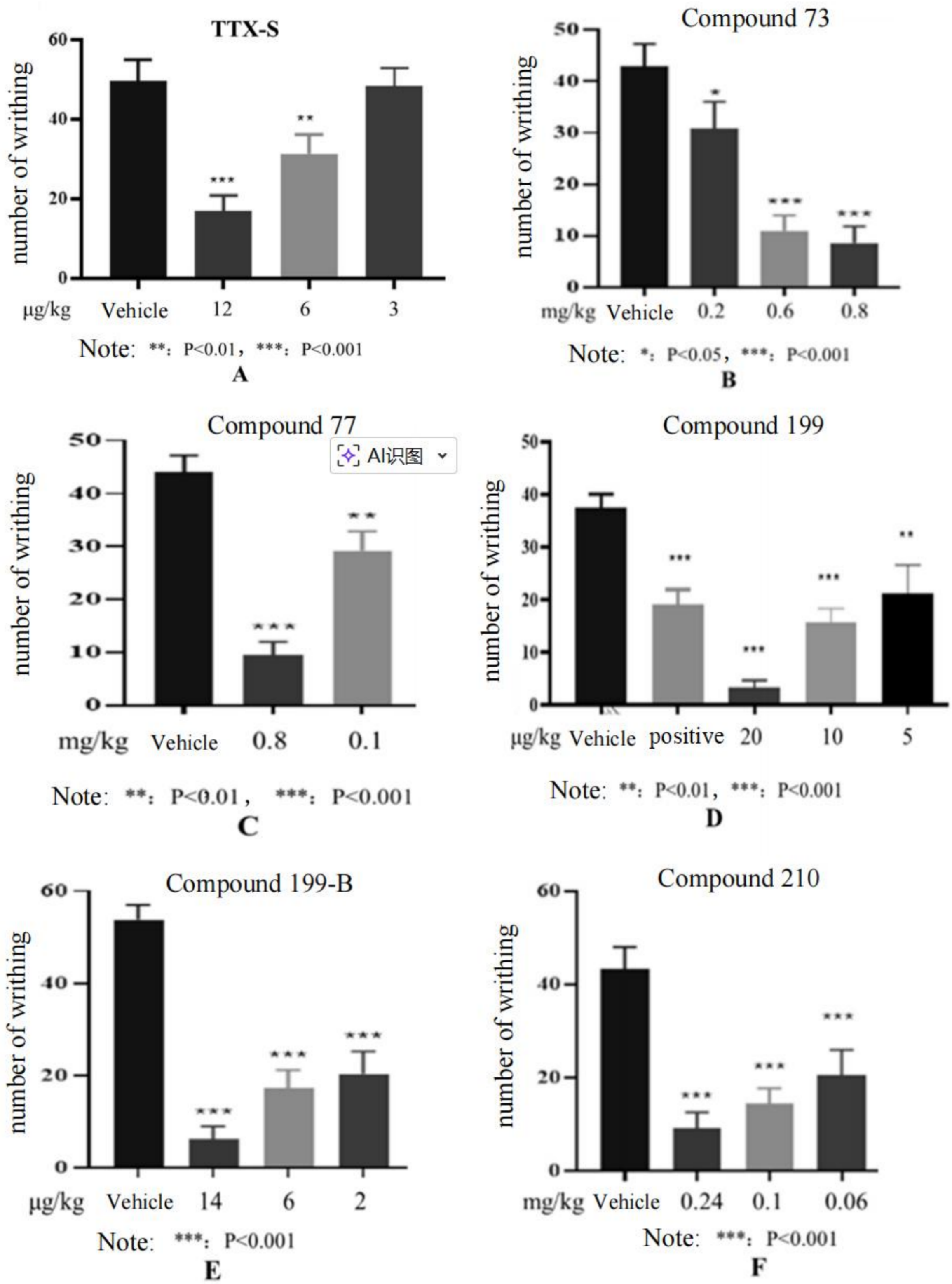
TTX-S
number of writhing
60
40
20
0
µg/kg Vehicle 12 6 3
***
**
Note: **: P<0.01, ***: P<0.001
A
Compound 73
number of writhing
50
40
30
20
10
0
mg/kg Vehicle 0.2 0.6 0.8
*
***
***
Note: *: P<0.05, ***: P<0.001
B
Compound 77
number of writhing
50
40
30
20
10
0
mg/kg Vehicle 0.8 0.1
***
**
Note: **: P<0.01, ***: P<0.001
C
Compound 199
number of writhing
50
40
30
20
10
0
µg/kg Vehicle positive 20 10 5
***
***
***
**
Note: **: P<0.01, ***: P<0.001
D
Compound 199-B
number of writhing
60
40
20
0
µg/kg Vehicle 14 6 2
***
***
***
Note: ***: P<0.001
E
Compound 210
number of writhing
60
40
20
0
mg/kg Vehicle 0.24 0.1 0.06
***
***
***
Note: ***: P<0.001
F

FIG. 1

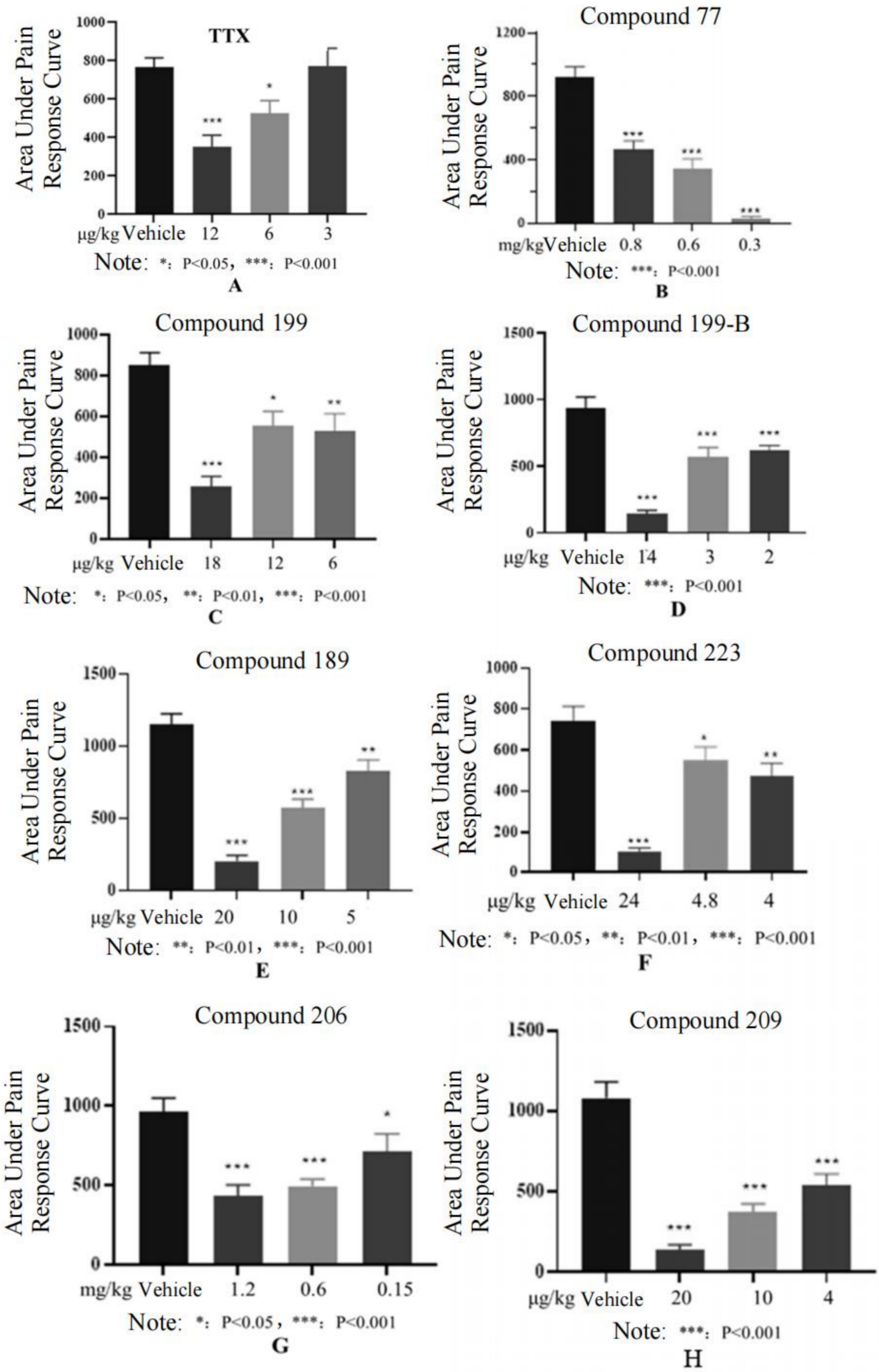
TTX
Area Under Pain Response Curve
μg/kg Vehicle 12 6 3
Note: *: P<0.05, ***: P<0.001
A
Compound 77
Area Under Pain Response Curve
mg/kg Vehicle 0.8 0.6 0.3
Note: ***: P<0.001
B
Compound 199
Area Under Pain Response Curve
μg/kg Vehicle 18 12 6
Note: *: P<0.05, **: P<0.01, ***: P<0.001
C
Compound 199-B
Area Under Pain Response Curve
μg/kg Vehicle 14 3 2
Note: ***: P<0.001
D
Compound 189
Area Under Pain Response Curve
μg/kg Vehicle 20 10 5
Note: **: P<0.01, ***: P<0.001
E
Compound 223
Area Under Pain Response Curve
μg/kg Vehicle 24 4.8 4
Note: *: P<0.05, **: P<0.01, ***: P<0.001
F
Compound 206
Area Under Pain Response Curve
mg/kg Vehicle 1.2 0.6 0.15
Note: *: P<0.05, ***: P<0.001
G
Compound 209
Area Under Pain Response Curve
μg/kg Vehicle 20 10 4
Note: ***: P<0.001
H

FIG. 2

## INTERNATIONAL SEARCH REPORT

International application No.
**PCT/CN2024/102404**

**A. CLASSIFICATION OF SUBJECT MATTER**

C07D 491/22(2006.01)i; A61K31/35(2006.01)i; A61K31/519(2006.01)i; A61P25/04(2006.01)i; A61P29/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, ENTXT, CNKI, STN (REGISTRY, MARPAT, CAPLUS): 河豚毒素, 钠离子通道, 钠通道, 阻滞剂, 衍生物, 疼痛, 镇痛, sodium channel, pain, Nav, analges, TTK, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| X | Registry. "RN 2387505-74-8 etc."<br>*STN*, 03 December 2019 (2019-12-03),<br>pp. 1-16 | 1-9, 12-27 |
| X | CHICHEPORTICHE, R. et al. "Synthesis of New, Highly Radioactive Tetrodotoxin Derivatives and Their Binding Properties to the Sodium Channel"<br>*European Journal of Biochemistry*, Vol. vol. 104, 31 December 1980 (1980-12-31), 617-625<br>page 623 | 1-30 |
| X | MAEHARA, Tomoaki et al. "Total Synthesis of (-)-Tetrodotoxin and 11-norTTX-6(R)-ol"<br>*Angewandte Chemie International Edition*, Vol. vol. 56, 31 December 2017 (2017-12-31), 1-5<br>page 1, Figure 1, and page 3, Scheme 5 | 1-32 |
| X | BANE, Vaishali et al. "High Resolution Mass Spectrometry Analysis of Tetrodotoxin (TTX) and its Analogues in Puffer Fish and Shellfish"<br>*Food Additives & Contaminants: Part A*, 10 August 2016 (2016-08-10), 1-43<br>pages 15-16, figure 1 | 1-30 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 September 2024** | **09 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/102404**

**C. DOCUMENTS CONSIDERED TO BE RELEVANT**

| Category* | Citation of document, with indication, where appropriate, of the relevant passages | Relevant to claim No. |
|---|---|---|
| X | SHOJI, Yuki et al. "Electrospray Ionization Mass Spectrometry of Tetrodotoxin and Its Analogs: Liquid Chromatography/Mass Spectrometry, Tandem Mass Spectrometry, and Liquid Chromatography/Tandem Mass Spectrometry" *Analytical Biochemistry,* Vol. vol. 290, 31 December 2001 (2001-12-31), 10-17<br>page 11, and figure 1 | 1-30 |
| A | YOSHIDA, Eiichi et al. "Photoaffinity Labeling of the Electroplax Sodium Channel with Tetrodotoxin Derivatives. II. Comparison of the Photoreactivity of Different Photoactivable Groups in the Tetrodotoxin Binding Site" *Chemical & Pharmaceutical Bulletin,* Vol. 38, No. (4), 31 December 1990 (1990-12-31), 982-987<br>page 982 | 1-32 |
| A | CN 116082354 A (FUJIAN ZHANGPING DAIXI BIOLOGICAL PHARMACEUTICAL CO., LTD.) 09 May 2023 (2023-05-09)<br>entire document | 1-32 |
| A | CN 113956266 A (SHANGHAI SHENGPING MEDICAL EQUIPMENT CO., LTD. et al.) 21 January 2022 (2022-01-21)<br>entire document | 1-32 |

Form PCT/ISA/210 (second sheet) (July 2022)

**INTERNATIONAL SEARCH REPORT**

International application No.
**PCT/CN2024/102404**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **1-31**
because they relate to parts of the international application that do not comply with the prescribed requirements to such an extent that no meaningful international search can be carried out, specifically:

The scope of the structure defined in claims 1-31 is very broad, and the related compounds cover a large number of known compounds in the prior art, which makes it difficult for the examiner to carry out an exhaustive search of the prior art with regard to the current scopes of the claims. In the present search report, according to an effect example, the search and examination are performed on the technical solutions involving that X in the compound of formula (I) is methylene or carbonyl, or does not exist, $R_6$ is selected from the group defined in claim 21, and $R_5$ is OH. Accordingly, the search and examination are only performed on the technical solutions of claims 2-31 relating to the compound described above.

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/102404**

| Patent document cited in search report | | | Publication date (day/month/year) | Patent family member(s) | Publication date (day/month/year) |
|---|---|---|---|---|---|
| CN | 116082354 | A | 09 May 2023 | None | |
| CN | 113956266 | A | 21 January 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 113956266 A

### Non-patent literature cited in the description


- **NAKAMURA M** ; **YASUMOTO T**. Tetrodotoxin years in puffer fish [J. *Toxicon*, 1985, vol. 232, 271-276
- **YASUMOTO T** ; **YOTSU M** ; **MURATA M et al.** New tetradotoxin analogs from the nenops ensauda [J. *j.am.chem.soc*, 1988, vol. 110 (7), 2344-2345
- *J. AM. CHEM. SOC.*, 2002, vol. 124, 7847-7852
- *Chem. Eur. J.*, 2004, vol. 10, 452-462
- **T. W. GREENE**. Protective Groups in Organic Synthesis. John Wiley & Sons, 1991